(19) Europäisches Patentamt
European Patent Office
Office européen des brevets

(11) Publication number: **0 165 753 B1**

(12) **EUROPEAN PATENT SPECIFICATION**

(45) Date of publication of patent specification: **11.12.91**

(51) Int. Cl.$^5$: **C07D 413/14**, C07D 409/14, C07D 409/12, C07D 401/14, C07D 403/14, A01N 47/36, //C07D413/04,C07D401/04

(21) Application number: **85304085.5**

(22) Date of filing: **10.06.85**

The file contains technical information submitted after the application was filed and not included in this specification

# (54) Herbicidal thiophenesulfonamides and pyridinesulfonamides.

(30) Priority: **11.06.84 US 619277**
**27.12.84 US 686834**
**19.04.85 US 724835**
**08.08.84 US 638964**
**22.04.85 US 724451**

(43) Date of publication of application:
**27.12.85 Bulletin 85/52**

(45) Publication of the grant of the patent:
**11.12.91 Bulletin 91/50**

(84) Designated Contracting States:
**BE CH DE FR GB IT LI LU NL SE**

(56) References cited:
**EP-A- 83 975**
**EP-A- 0 085 476**
**EP-A- 0 103 543**
**EP-A- 0 161 211**

(73) Proprietor: **E.I. DU PONT DE NEMOURS AND COMPANY**
**1007 Market Street**
**Wilmington Delaware 19898(US)**

(72) Inventor: **Shapiro, Rafael**
**1415 Fresno Road**
**Wilmington Delaware 19803(US)**
Inventor: **Rorer, Morris Padgett**
**64 Lower Valley Lane Green Valley**
**Newark Delaware 19711(US)**

(74) Representative: **Hildyard, Edward Martin et al**
**Frank B. Dehn & Co. European Patent Attorneys Imperial House 15-19 Kingsway**
**London WC2B 6UZ(GB)**

**EP 0 165 753 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

U.S. Patent 4,398,939, issued August 16, 1983 to Levitt, discloses herbicidal thiophenesulfonylureas such as

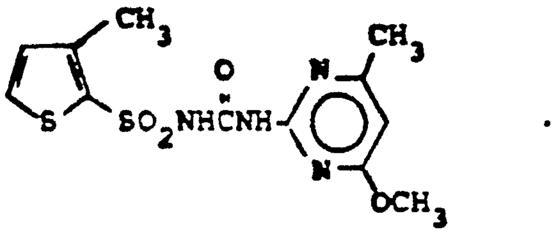

European Patent Application 30,142. published June 10, 1981, discloses herbicidal thiophene sulfonylureas such as

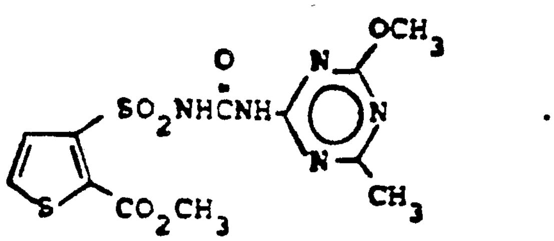

U.S. Patent 4,378,991, issued April 5, 1983 discloses o-phenyl sulfonylureas such as

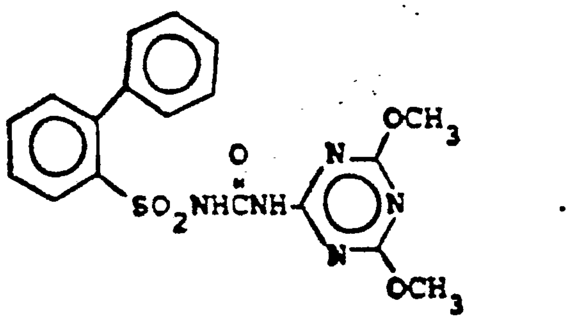

European Patent Application 83,975, published July 20, 1983, and European Patent Application 85,476, published August 10, 1983, disclose herbicidal benzenesulfonamides of the formula

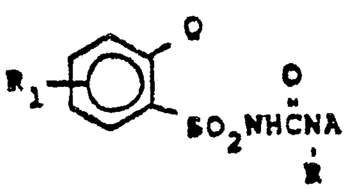

where Q is various 5- and 6-membered, saturated, unsaturated or partially unsaturated heterocyclic rings.

South African Patent Application 83/8416 (published May 12, 1984) discloses herbicidal sulfonamides of formula

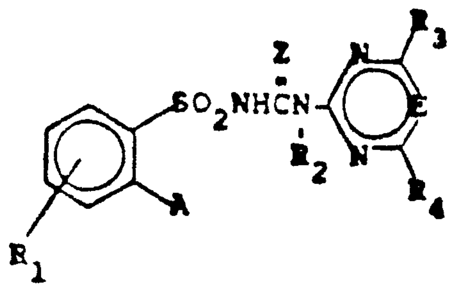

wherein

A is an unsaturated or only partially saturated 5- or 6-membered heterocyclic ring system which is bonded through a carbon atom and contains 1, 2 or 3 heteroatoms and which may be substituted by $C_1$-$C_4$ alkyl, $C_1$-$C_4$ haloalkyl, $C_1$-$C_4$ alkoxy, $C_1$-$C_4$ alkylthio, $C_2$-$C_8$ alkoxyalkyl, di($C_1$-$C_4$ alkyl)-amino, halogen, cyano or nitro.

Herbicidal pyridinesulfonamides of the formula

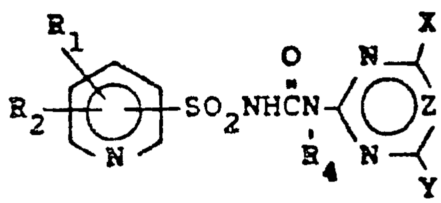

where

$R_1$ is H, Cl, Br, F, $C_1$-$C_4$ alkyl, $C_1$-$C_4$ alkoxy, $C_1$-$C_4$ alkylthio, $NO_2$ or $CO_2R_5$; and

$R_2$ is H, Cl, Br or $CH_3$;

are disclosed in European Patent Application (EP-A) 13,480.

EP-A-35,893 discloses herbicidal pyridinesulfonamides of formula

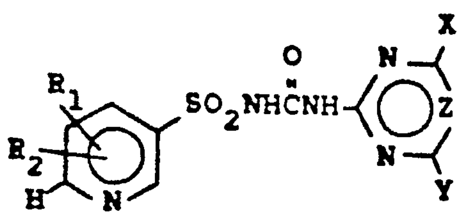

where

$R_1$ is $S(O)_nR_3$; and

$R_2$ is H, Cl, F, Br, $CH_3$, $OCH_3$, $CF_3$, $NO_2$, CN or $NH_2$.

EP-A-83,975 and EP-A-85,476 disclose herbicidal benzenesulfonamides of formula

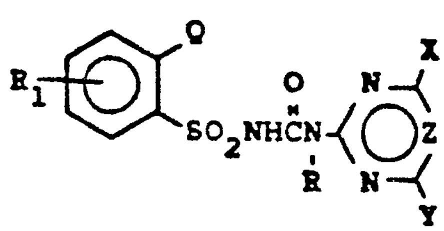

where

Q is various saturated and unsaturated 5- and 6-membered heterocycles.

U.S. 4,378,991 discloses herbicidal benzenesulfonamides of formula

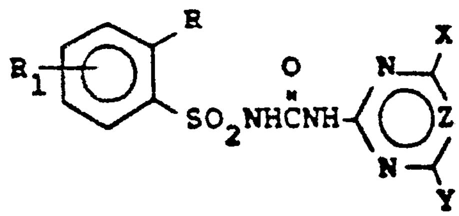

where
R is, among other values, phenyl; and
$R_1$ is H, F, Cl, Br, $NO_2$, $CF_3$, $C_1$-$C_4$ alkyl, $OCF_3$ or $C_1$-$C_4$ alkoxy.

EP-A-97,122, published December 28, 1983, discloses herbicidal sulfonamides of formula

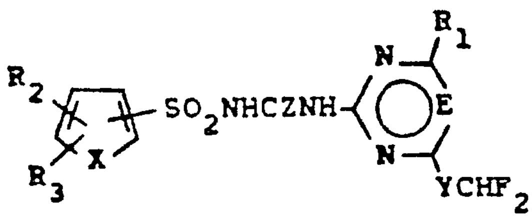

where
X is O, S, $NR_4$ or $CR_5$ = N; and
$R_2$ is H, $C_1$-$C_3$ alkyl, haloalkyl, alkoxy, alkylthio, alkylsulfinyl or alkylsulphonyl, halogen, $NO_2$, $CWR_8$, $SO_2NR_6R_7$ or $COR_9$.

South African Patent Application 836,639 discloses herbicidal sulfonamides of formula

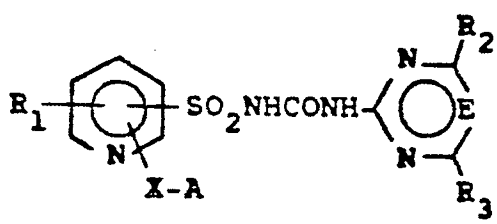

where
X is O, S, SO, $SO_2$ or XA may form an amino radical $NR_6R_7$; and
$R_1$ is H, halogen, $C_1$-$C_4$ alkyl, $C_1$-$C_4$ alkoxy.
$C_1$-$C_4$ haloalkyl, $C_1$-$C_4$ haloalkoxy, $C_2$-$C_5$ alkoxyalkoxy, $C_1$-$C_5$ alkylthio, $C_1$-$C_5$ alkylsulfinyl or $C_1$-$C_5$ alkylsulfonyl.

Summary of the Invention

This invention relates to novel compounds of Formulae Ia-Ie, agriculturally suitable compositions containing them and their method-of-use as general or selective preemergent and postemergent herbicides, or as plant growth regulants.

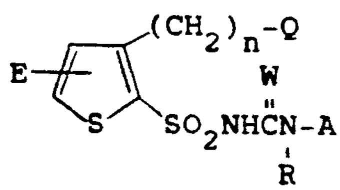

Ia

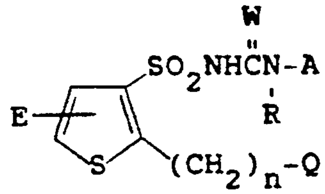

Ib

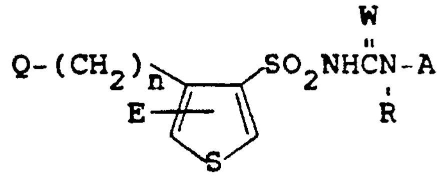

Ic

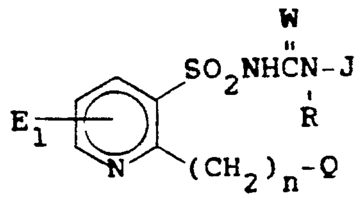

Id

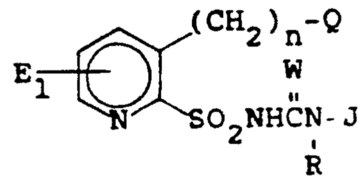

Ie

wherein

| | |
|---|---|
| R | is H or $CH_3$; |
| n | is 0. 1 or 2; |
| W | is O or S; |
| Q | is a saturated 5- or 6-membered ring containing 1 heteroatom selected from sulfur, oxygen or nitrogen, or an unsaturated 5- or 6-membered ring containing 1-3 hetero- atoms selected from 0-1 sulfur, 0-1 oxygen or 0-3 nitrogen; in compounds of Formulae Ia-Ic, Q may optionally be substituted by one or more groups selected from SH, $C_1$-$C_4$ alkyl, $C_3$-$C_4$ alkenyl, $C_1$-$C_3$ haloalkyl, halogen, $C_1$-$C_3$ alkoxy, $C_1$-$C_4$ alkylthio, $C_3$-$C_4$ alkenylthio, $C_3$-$C_4$ alkenyloxy, $C_1$-$C_2$ haloalkoxy, $C_1$-$C_2$ haloalkylthio, $C_3$-$C_4$ alkynylthio, $C_1$-$C_4$ cyanoalkylthio, $C_1$-$C_2$ alkoxycarbonylmethylthio or $C_1$-$C_2$ alkylcarbonylmethylthio; in compounds of Formulae Id and Ie, Q may optionally be substituted by one or more groups selected from $C_1$-$C_4$ alkyl, halogen, $C_3$-$C_4$ alkenyl, $C_1$-$C_3$ alkoxy, $C_1$-$C_3$ alkylthio $C_3$-$C_4$ alkenylthio, $C_1$-$C_2$ haloalkoxy or $C_1$-$C_2$ haloalkylthio; |
| E | is H, $C_1$-$C_2$ alkyl, $C_1$-$C_2$ alkoxy, halogen, $NO_2$, $C_1$-$C_2$ haloalkyl, $C_1$-$C_2$ alkylthio, $C_1$-$C_2$ alkylsulfonyl, $C_1$-$C_2$ alkoxycarbonyl, $C_1$-$C_2$ dialkylaminosulfamoyl; |
| $E_1$ | is H, Cl, Br, $CH_3$ or $SCH_3$; |
| A | is |

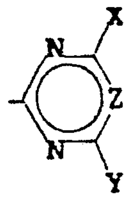,
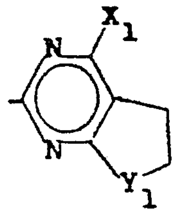,
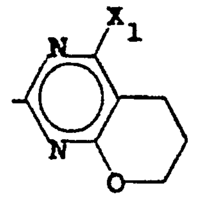,

A-1 A-2 A-3

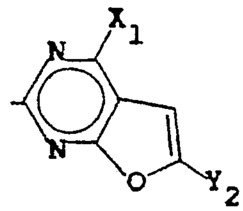,
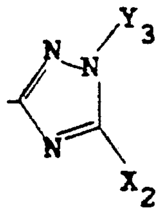
or
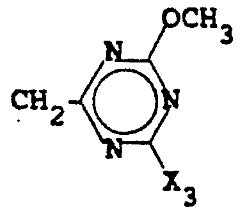;

A-4 A-5 A-6

J is

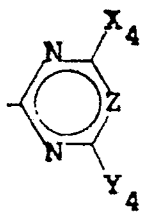,
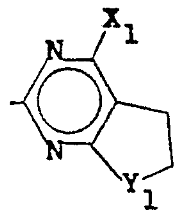,
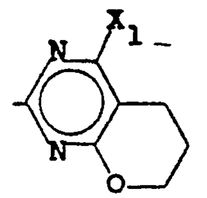,

J-1 J-2 J-3

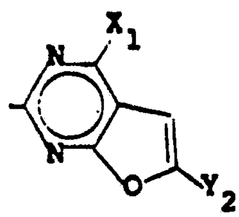,
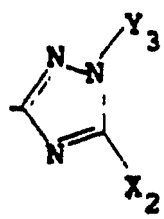
or
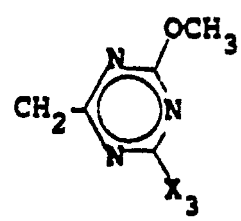;

J-4 J-5 J-6

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino or di($C_1$-$C_3$ alkyl)amino;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino, di($C_1$-$C_3$ alkyl)-amino, $C_3$-$C_4$ alkenyloxy, $C_3$-$C_4$ alkynyloxy, $C_2$-$C_5$ alkylthioalkyl, $C_2$-$C_5$ alkylsulfinylalkyl, $C_2$-$C_5$ alkylsulfonylalkyl, $C_1$-$C_4$ haloalkyl, $C_3$-$C_5$ cycloalkyl, $C_2$-$C_4$ alkynyl, C(O)$R_c$,

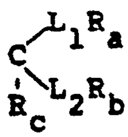, 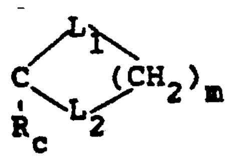, 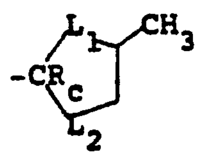

or $N(OCH_3)CH_3$;

m is 2 or 3;

$L_1$ and $L_2$ are independently O or S;

$R_a$ and $R_b$ are independently $C_1$-$C_2$ alkyl;

$R_c$ is H or $CH_3$;

Z is CH or N;

$Y_1$ is O or $CH_2$;

$X_1$ is $CH_3$, $OCH_3$, $OC_2H_5$ or $OCF_2H$;

$Y_2$ is H or $CH_3$;

$X_2$ is $CH_3$, $OCH_3$ or $SCH_3$;

$Y_3$ is $CH_3$, $CH_2CH_3$ or $CH_2CF_3$;

$X_3$ is $CH_3$ or $OCH_3$;

$X_4$ is $CH_3$, $OCH_3$, $OC_2H_5$, Cl, F, Br, I, $OCF_2H$, $CH_2F$, $OCH_2CH_2F$, $OCH_2CHF_2$, $OCH_2CF_3$ or $CF_3$;

$Y_4$ is H, $CH_3$, $OCH_3$, $OC_2H_5$, $CH_2OCH_3$, $NHCH_3$, $N(OCH_3)CH_3$, $N(CH_3)_2$, $CH_2CH_3$, $CF_3$, $SCH_3$, $OCH_2CH=CH_2$, $OCH_2C{\equiv}CH$, $CH_2OC_2H_5$, $OCH_2CH_2OCH_3$, $CH_2SCH_3$, $C(O)R_c$,

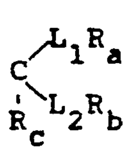, 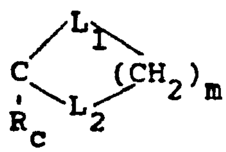, 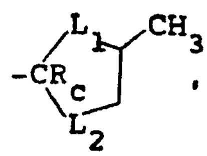, $OCF_2H$,

$SCF_2H$ or cyclopropyl;

and their agriculturally suitable salts; provided that

1) when X or $X_4$ is F, Cl, Br or I, then Z is CH, Y is $OCH_3$, $OC_2H_5$, $OCF_2H$, $NH_2$, $NHCH_3$, $N(OCH_3)CH_3$ or $N(CH_3)_2$, and $Y_4$ is $OCH_3$, $OC_2H_5$, $OCF_2H$, $NHCH_3$, $N(OCH_3)CH_3$ or $N(CH_3)_2$;

2) the total number of carbon atoms of Q must be less than or equal to 8;

3) when X, Y, $X_4$ or $Y_4$ is $OCF_2H$, then Z is CH;

4) when Q is a saturated 5- or 6-membered ring containing one nitrogen atom, it is bonded to the thiophene or pyridine ring through carbon;

5) in compounds of Formulae Id and Ie, when Q is 1H-1,2,4-triazol-1-yl, then Z is CH;

6) when $Y_4$ is cyclopropyl, $X_4$ is other than Cl, F, Br or I; and

7) when W is S, then R is H, A is A-1, J is J-1, and Y and $Y_4$ are $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$,

$CH(OCH_3)_2$ or 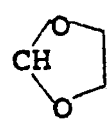.

In the above definitions, the term "alkyl", used either alone or in compound words such as "alkylthio" or "haloalkyl", denotes straight chain or branched alkyl, e.g. methyl, ethyl, n-propyl, isopropyl or the different butyl isomers.

Alkoxy denotes methoxy, ethoxy, n-propyloxy, isopropyloxy or the different butyl isomers.

Alkenyl denotes straight chain or branched alkenes, e.g. vinyl, 1-propenyl, 3-propenyl, isopropenyl or the different butenyl isomers.

Alkynyl denotes straight chain or branch alkynes, e.g., ethynyl, 1-propynyl, 2-propynyl or the different butynyl isomers.

Cycloalkyl denotes cyclopropyl, cyclobutyl or cyclopentyl.

The term "halogen", either alone or in compound words such as "haloalkyl", denotes fluorine, chlorine,

bromine or iodine.

Alkylcarbonyl denotes acetyl or propionyl.

Alkoxycarbonyl denotes methoxycarbonyl or ethoxycarbonyl.

Alkylsulfonyl denotes methylsulfonyl or ethylsulfonyl.

Alkylthio, alkylamino, alkylsulfamoyl, etc. are defined in an analogous manner.

In terms such as $C_2$-$C_3$ alkylthioalkyl, the specified number of carbon atoms is meant to define the total number of carbon atoms in that substituent group . For example, $C_2$-$C_3$ alkylthioalkyl would designate $CH_2SCH_3$, $CH_2SC_2H_5$, $CH_2CH_2SCH_3$ or $CH(CH_3)SCH_3$, and $C_2$-$C_5$ alkoxyalkoxy would represent $OCH_2OCH_3$, through $O(CH_2)_4OCH_3$ or $OCH_2O(CH_2)_3CH_3$ and the various structural isomers embraced therein.

Preferred for their higher herbicidal activity, greater plant growth regulant activity and/or more favorable ease of synthesis are:

1) Compounds of Formulas Ia-Ic where

Q is

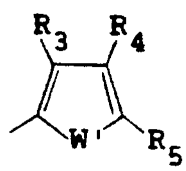 ,

Q-2

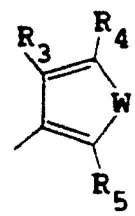 ,

Q-3

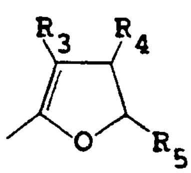 ,

Q-4

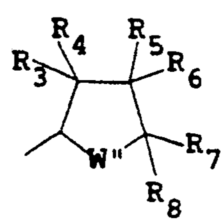 ,

Q-5

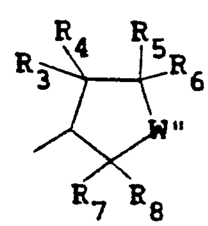 , ,

Q-6

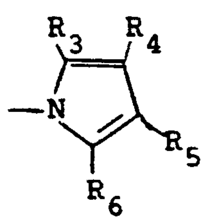 ,

Q-7

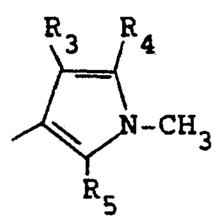 ,

Q-9

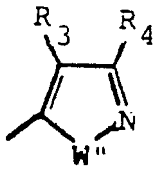 , 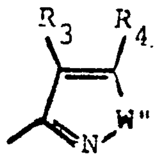 , 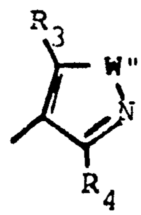 ,

Q-10 Q-11 Q-12

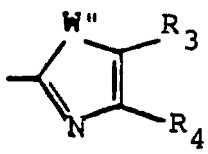 , 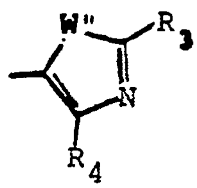 , 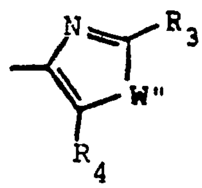 ,

Q-13 Q-14 Q-15

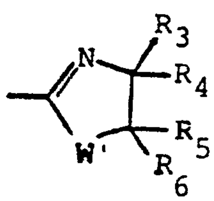 , 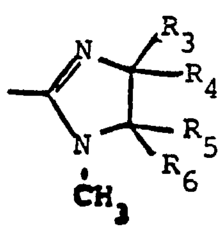 , 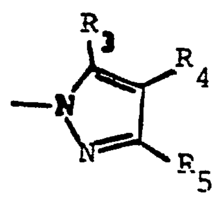 ,

Q-16 Q-17 Q-18

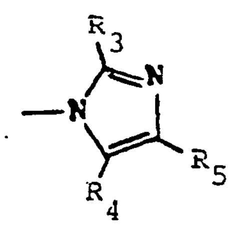 , 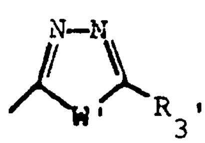 , 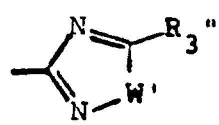 ,

Q-19 Q-20 Q-21

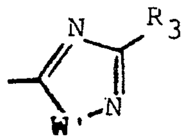 ,

Q-22

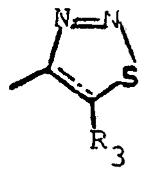 ,

Q-23

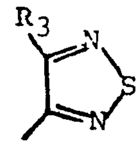

Q-24

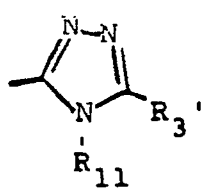 ,

Q-25

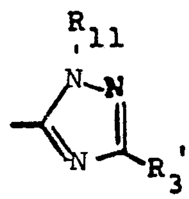 ,

Q-26

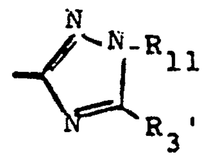 ,

Q-27

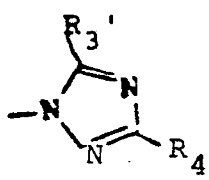 ,

Q-28

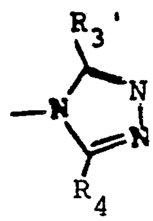 ,

Q-29

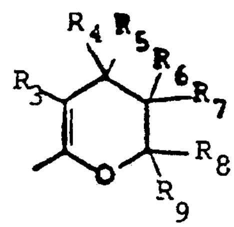

Q-30

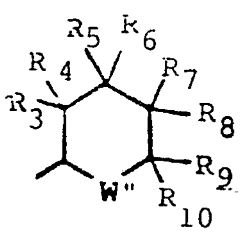 ,

Q-31

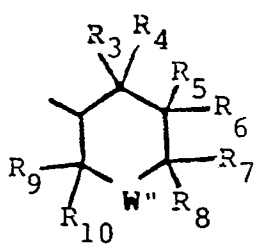 ,

Q-32

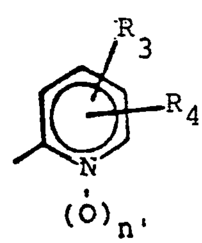 ,

Q-33

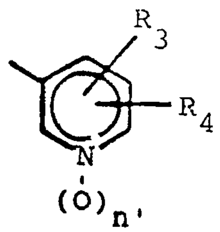

Q-34

,

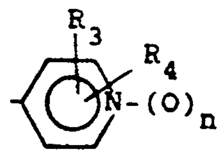

Q-35

,

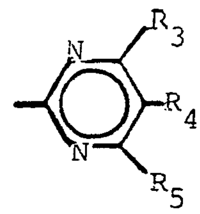

Q-36

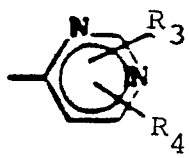

Q-37

,

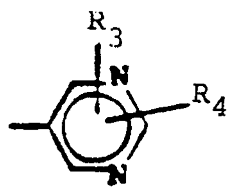

Q-38

,

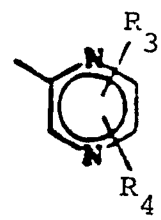

Q-39

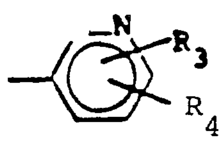

Q-40

,

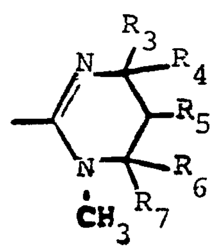

Q-41

,

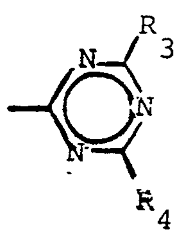

Q-43

,

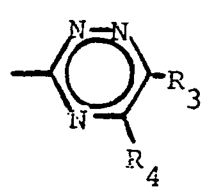

Q-44

or

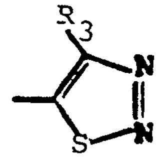

Q-45

;

| | |
|---|---|
| $R_3$, $R_4$, $R_5$, $R_6$, $R_7$, $R_8$, $R_9$ and $R_{10}$ | are independently H or $CH_3$; |
| n' | is 0 or 1; |
| $R_3'$ | is H, SH, $C_1$-$C_3$ alkyl, $C_1$-$C_4$ alkylthio, $C_3$-$C_4$ alkenylthio, $C_3$-$C_4$ alkynylthio, $C_1$-$C_3$ cyanoalkylthio, $SCH_2CO_2CH_3$, $SCH_2CO_2C_2H_5$, $SCH_2C(O)CH_3$, halogen, $C_1$-$C_3$ alkoxy or $OCH_2CH=CH_2$; |
| $R_3''$ | is H, $CH_3$, Cl or Br; |
| W' | is O or S; |
| W'' | is O or S or $NR_{11}$; |

$R_{11}$ is H, $C_1$-$C_3$ alkyl or $CH_2CH=CH_2$;

2) Compounds of Preferred 1 where E is H; A is A-1; X is $CH_3$, $OCH_3$, $OC_2H_5$, Cl or Br; Y is H, $CH_3$, $OCH_3$, $C_2H_5$, $OC_2H_5$, $CH_2OCH_3$, $CF_3$, $OCF_2H$, cyclopropyl, $OCH_2CF_3$, $NHCH_3$, $N(CH_3)_2$, $CH(OCH_3)_2$ or

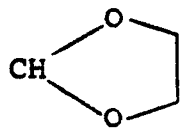 ;

n' is O; and

$R_3$' and $R_3$'' are independently H, $CH_3$ or Cl; R is H; and W is O;

3) Compounds of Preferred 2 where n is O;

4) Compounds of Preferred 3 where Y is $C_1$-$C_2$ alkyl, $OCH_3$, or $OCF_2H$;

5) Compounds of Preferred 4 of Formula Ia;

6) Compounds of Preferred 4 of Formula Ib;

7) Compounds of Preferred 4 of Formula Ic;

8) Compounds of Preferred 5 where Q is Q-2,

9) Compounds of Preferred 5 where Q is Q-3;

10) Compounds of Preferred 5 where Q is Q-5;

11) Compounds of Preferred 5 where Q is Q-7;

12) Compounds of Preferred 5 where Q is Q-10;

13) Compounds of Preferred 5 where Q is Q-11;

14) Compounds of Preferred 5 where Q is Q-15;

15) Compounds of Preferred 5 where Q is Q-16;

16) Compounds of Preferred 5 where Q is Q-20;

17) Compounds of Preferred 5 where Q is Q-23;

18) Compounds of Preferred 5 where Q is Q-28;

19) Compounds of Preferred 5 where Q is Q-36;

20) Compounds of Preferred 5 where Q is Q-39;

21) Compounds of Preferred 6 where Q is Q-2;

22) Compounds of Preferred 6 where Q is Q-3;

23) Compounds of Preferred 6 where Q is Q-5;

24) Compounds of Preferred 6 where Q is Q-7;

25) Compounds of Preferred 6 where Q is Q-10;

26) Compounds of Preferred 6 where Q is Q-11;

27) Compounds of Preferred 6 where Q is Q-15;

28) Compounds of Preferred 6 where Q is Q-16;

29) Compounds of Preferred 6 where Q is Q-20;

30) Compounds of Preferred 6 where Q is Q-23;

31) Compounds of Preferred 6 where Q is Q-28;

32) Compounds of Preferred 6 where Q is Q-36;

33) Compounds of Preferred 6 where Q is Q-39;

34) Compounds of Preferred 7 where Q is Q-2;

35) Compounds of Preferred 7 where Q is Q-3;

36) Compounds of Preferred 7 where Q is Q-5;

37) Compounds of Preferred 7 where Q is Q-7;

38) Compounds of Preferred 7 where Q is Q-10;

39) Compounds of Preferred 7 where Q is Q-11;

40) Compounds of Preferred 7 where Q is Q-15;

41) Compounds of Preferred 7 where Q is Q-16;

42) Compounds of Preferred 7 where Q is Q-20;

43) Compounds of Preferred 7 where Q is Q-23;

44) Compounds of Preferred 7 where Q is Q-28;

45) Compounds of Preferred 7 where Q is Q-36;

46) Compounds of Preferred 7 where Q is Q-39;

47) Compounds of Preferred 14 where W'' is S;

48) Compounds of Preferred 27 where W'' is S;

49) Compounds of Preferred 40 where W" is S;

50) Compounds of Formulae Id and Ie wherein

R is H;

W is O;

Q is selected from the group consisting of

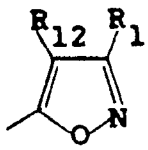 ,

Q-46

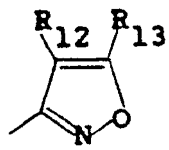 ,

Q-47

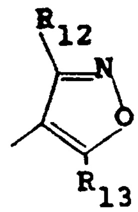 ,

Q-48

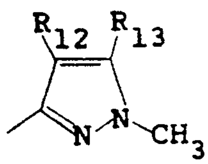 ,

Q-49

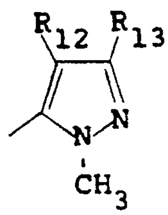 ,

Q-50

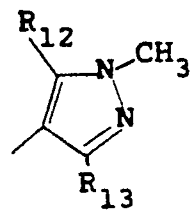 ,

Q-51

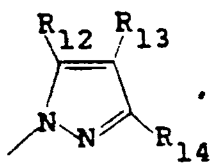 ,

Q-52

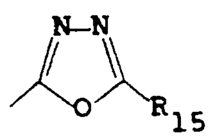 ,

Q-53

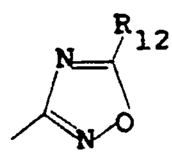 ,

Q-54

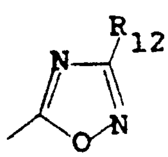 ,

Q-55

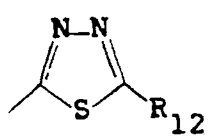 ,

Q-56

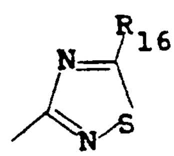 ,

Q-57

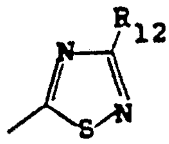,
Q-58

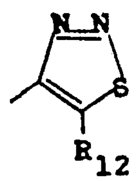,
Q-59

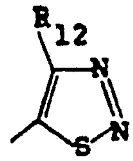,
Q-60

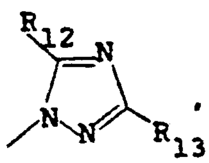,
Q-61

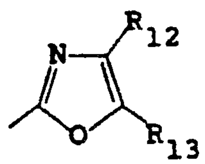,
Q-62

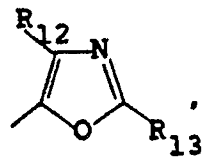,
Q-63

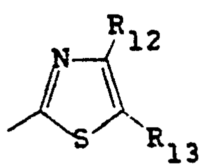,
Q-64

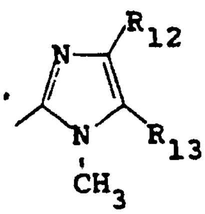,
Q-65

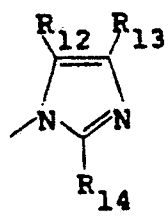,
Q-66

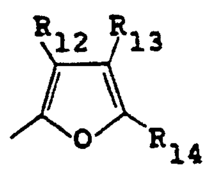,
Q-67

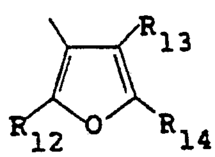,
Q-68

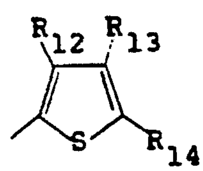,
Q-69

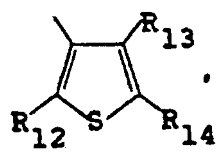,
Q-70

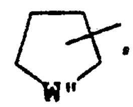,
Q-71

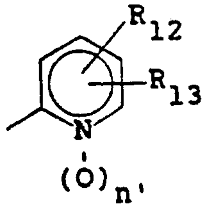,
Q-72

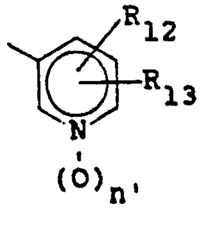,
Q-73

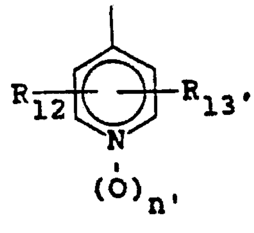,
Q-74

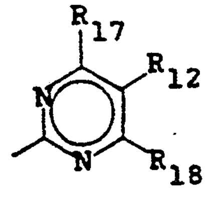,
Q-75

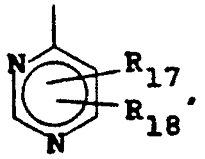

Q-76

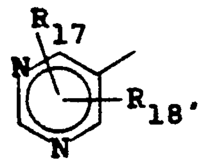

Q-77

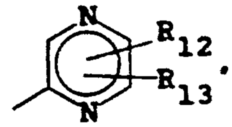

Q-78

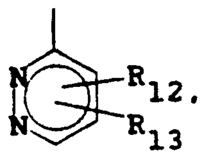

Q-79

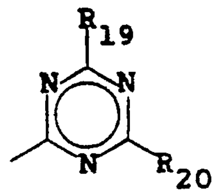

Q-80

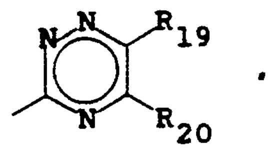

Q-81

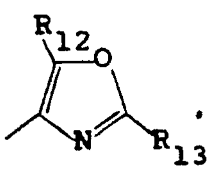

Q-82

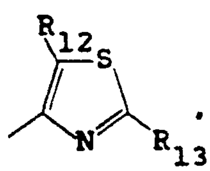

Q-83

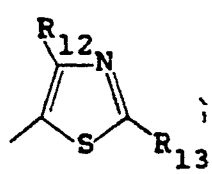

Q-84

| | |
|---|---|
| n' | is as defined previously; |
| $R_{12}$, $R_{13}$ and $R_{14}$ | are independently H or $CH_3$; |
| $R_{15}$ | is H, $CH_3$, $C_2H_5$, $C_1$-$C_3$ alkylthio, $SCH_2CH=CH_2$, $SCF_2H$, $OCH_3$ or $OCH_2CH_3$; |
| $R_{16}$ | is H or Cl; |
| $R_{17}$ and $R_{18}$ | are independently H, $CH_3$ or $OCH_3$; |
| $R_{19}$ and $R_{20}$ | are independently $CH_3$ or $OCH_3$; |
| W" | is O, S or $NR_{11}$; and |
| $R_{11}$ | is as defined previously; |

51) Compounds of Preferred 54 where J is J-1; $E_1$ is H; n' is O; and Q is selected from the group consisting of Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82 and Q-83;

52) Compounds of Preferred 51 where $X_4$ is $CH_3$, $OCH_3$, $OCH_2CH_3$ or Cl, and $Y_4$ is $CH_3$, $CH_2CH_3$, $OCH_3$, $CH(OCH_3)_2$ or $CH_2OCH_3$;

53) Compounds of Preferred 52 where n is O;

54) Compounds of Preferred 53 where $Y_4$ is $CH_3$, $CH_2CH_3$ or $OCH_3$;

55) Compounds of Preferred 54 of Formula Id;

56) Compounds of Preferred 54 of Formula Ie;

57) Compounds of Preferred 55 where Q is Q-46;

58) Compounds of Preferred 55 where Q is Q-47;

59) Compounds of Preferred 55 where Q is Q-48;

60) Compounds of Preferred 55 where Q is Q-49;

61) Compounds of Preferred 55 where Q is Q-52;

62) Compounds of Preferred 55 where Q is Q-53;

63) Compounds of Preferred 55 where Q is Q-54;

64) Compounds of Preferred 55 where Q is Q-55;

65) Compounds of Preferred 55 where Q is Q-56;

66) Compounds of Preferred 55 where Q is Q-59;

67) Compounds of Preferred 55 where Q is Q-61;

68) Compounds of Preferred 55 where Q is Q-62;
69) Compounds of Preferred 55 where Q is Q-63;
70) Compounds of Preferred 55 where Q is Q-66;
71) Compounds of Preferred 55 where Q is Q-67;
72) Compounds of Preferred 55 where Q is Q-69;
73) Compounds of Preferred 55 where Q is Q-72;
74) Compounds of Preferred 55 where Q is Q-75;
75) Compounds of Preferred 55 where Q is Q-78;
76) Compounds of Preferred 55 where Q is Q-82;
77) Compounds of Preferred 55 where Q is Q-83;
78) Compounds of Preferred 56 where Q is Q-46;
79) Compounds of Preferred 56 where Q is Q-47;
80) Compounds of Preferred 56 where Q is Q-48;
81) Compounds of Preferred 56 where Q is Q-49;
82) Compounds of Preferred 56 where Q is Q-52;
83) Compounds of Preferred 56 where Q is Q-53;
84) Compounds of Preferred 56 where Q is Q-54;
85) Compounds of Preferred 56 where Q is Q-55;
86) Compounds of Preferred 56 where Q is Q-56;
87) Compounds of Preferred 56 where Q is Q-59;
88) Compounds of Preferred 56 where Q is Q-61;
89) Compounds of Preferred 56 where Q is Q-62;
90) Compounds of Preferred 56 where Q is Q-63;
91) Compounds of Preferred 56 where Q is Q-66;
92) Compounds of Preferred 56 where Q is Q-67;
93) Compounds of Preferred 56 where Q is Q-69;
94) Compounds of Preferred 56 where Q is Q-72;
95) Compounds of Preferred 56 where Q is Q-75;
96) Compounds of Preferred 56 where Q is Q-78;
97) Compound of Preferred 56 where Q is Q-82;
98) Compounds of Preferred 56 where Q is Q-83;

Specifically Preferred for their highest herbicidal activity, greatest plant growth regulant activity and/or most favorable ease of synthesis are:

- N-[(4,6-dimethylpyrimidin-2-yl)aminocarbonyl]-3-(isoxazol-3-yl)-2-thiophenesulfonamide, m.p. 172.5-173 °C;
- 3-(isoxazol-3-yl)-N-[(4-methoxy-6-methylpyrimidin-2-yl)aminocarbonyl]-2-thiophenesulfonamide, m.p. 192 °C(d);
- N-[(4,6-dimethoxypyrimidin-2-yl)aminocarbonyl]-3-(isoxazol-3-yl)-2-thiophenesulfonamide, m.p. 157-158 °C;
- 3-(5-chloro-1H-1,2,4-triazol-1-yl)-N-[(4,6-dimethoxypyrimidin-2-yl)aminocarbonyl]-2-thiophenesulfonamide, m.p. 204-205 °C(d);
- N-[(4-methoxy-6-methylpyrimidin-2-yl)aminocarbonyl-3-(1H-pyrrol-1-yl)-2-thiophenesulfonamide, m.p. 152-155 °C;
- N-[(4-methoxy-6-methyl-1,3,5-triazin-2-yl)aminocarbonyl-3-(1H-pyrrol-1-yl)-2-thiophenesulfonamide, m.p. 144-146 °C;
- N-[(4,6-dimethoxypyrimidin-2-yl)aminocarbonyl]-3-(1H-pyrrol-1-yl)-2-thiophenesulfonamide, m.p. 142-146 °C;
- N-[(4,6-dimethylpyrimidin-2-yl)aminocarbonyl]-3-(1H-pyrrol-1-yl)-2-thiophenesulfonamide, m.p. 170-173 °C;
- N-[(4,6-dimethyl-1,3,5-triazin-2-yl)aminocarbonyl]-3-(1H-pyrrol-1-yl)-2-thiophenesulfonamide, m.p. 153-156 °C;
- N-[(4,6-dimethoxy-1,3,5-triazin-2-yl)aminocarbonyl]-3-(1H-pyrrol-1-yl)-2-thiophenesulfonamide, m.p. 156-159 °C;
- N-[(4-chloro-6-methoxypyridimin-2-yl)aminocarbonyl]-3-(2-methyl-4-thiazolyl)-2-thiophenesulfonamide, m.p. 146-149 °C;
- N-[(4-methoxy-6-methylpyrimidin-2-yl)aminocarbonyl]-3-(2-methyl-4-thiazolyl)-2-thiophenesulfonamide, m.p. 146-149 °C;
- N-[(4,6-dimethoxypyrimidin-2-yl)aminocarbonyl]-3-(2-methyl-4-thiazolyl)-2-thiophenesulfonamide, m.p.

164-168 °C; and

- N-[(4,6-dimethoxypyrimidin-2-yl)aminocarbonyl]-2-(1H-1,2,4-triazol-1-yl)-3-pyridinesulfonamide, m.p. 235-238 °C.

Other groups of compounds within the scope of Formula (I) are those disclosed in our U.S. Patent Applications Serial Nos. 619,277; 686,834; 724,835; 638,964 and 724,451. Copies of these specifications are available for inspection on the file of the present Application.

## Synthesis

The compounds of Formulae Ia-Ic can be prepared by one or more of the methods described below in Equations 1 through 4. These procedures are equally applicable for making compounds of Formulae Id and Ie.

Many of the compounds of Formula I can be prepared by reaction of a sulfonamide of Formula 2 with an appropriate methyl carbamate of Formula 3 in the presence of at least an equimolar amount of trimethylaluminum, according to Equation 1.

Equation 1

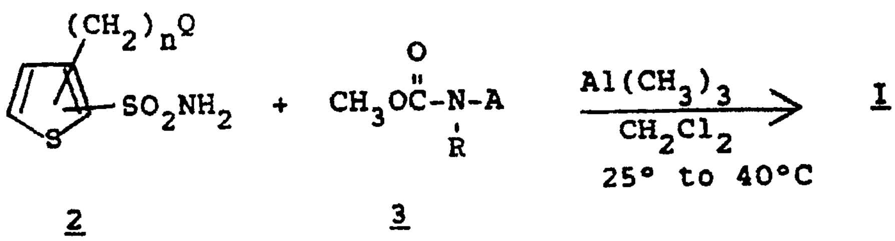


These reactions are carried out at 25° to 40°C under an inert atmosphere and in an inert, dipolar aprotic solvent such as methylene chloride for 10 to 96 hours. Details of this reaction as well as the preparation of the carbamates of Formula 3 can be found in EPO Publication No. 13,480.

Many of the compounds of Formula I also can be prepared by reacting a sulfonylcarbamate of Formula 4 with an appropriate heterocyclic amine of Formula 5, according to Equation 1a.

Equation 1a

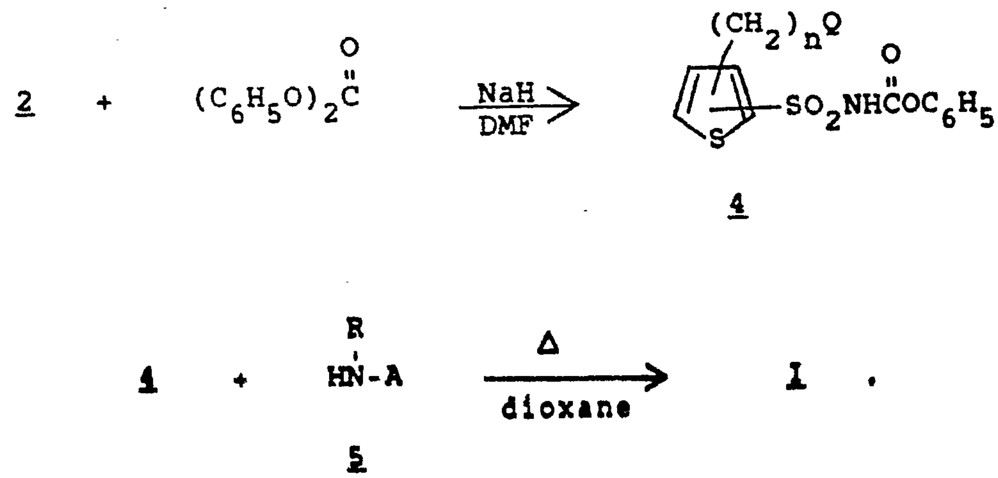


The reaction is carried out at 50° to 100°C in a solvent such as 1,4-dioxane for 0.5 to 24 hours according to EPO Publication No. 44807. The required carbamates of Formula 4 are prepared by reacting the appropriate sulfonamide, 2, with diphenylcarbonate in the presence of equimolar quantities of a strong base, such as sodium or potassium hydride.

Some of the compounds of Formula I also can be prepared as shown in Equation 2.

Equation 2

a)

2 + 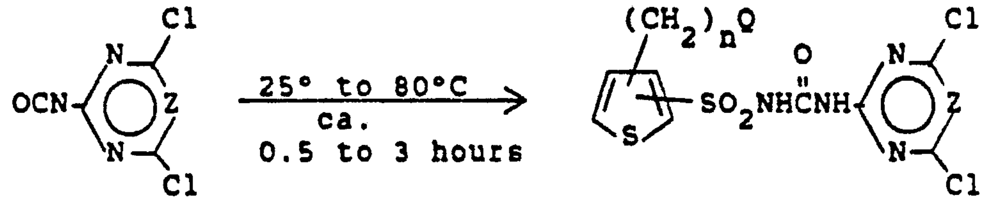


6 7

b)

7 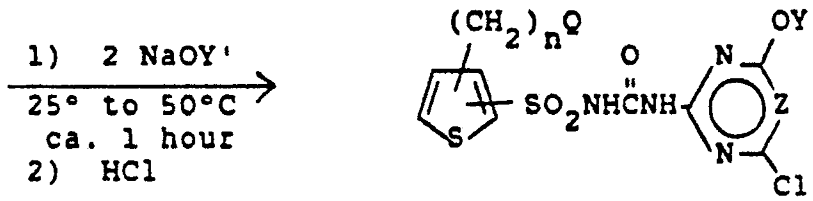


I'

c)

I' 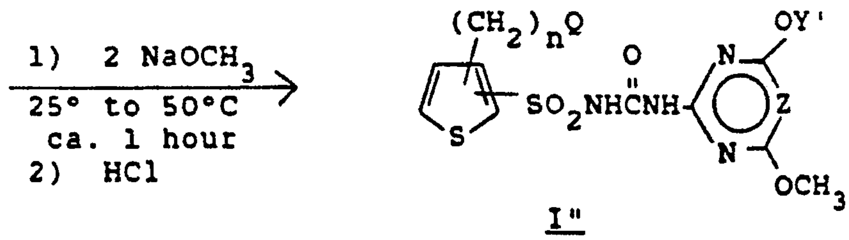 .


I''

wherein Y' is $CH_3$, $C_2H_5$ or $CH_2CF_3$. This reaction series is performed according to the procedures disclosed by EPO publication No. 30,140 and the requisite heterocyclic isocyanates of Formula 6 can be prepared according to methods described in Swiss 579,062, U.S. 3,919,228, U.S. 3,732,223 and U. von Gizycki, Angew. Chem. Int. Ed. Engl. 1976, 10, 402 and 403.

Compounds of Formula I also may be prepared by reaction of a thienylsulfonylisocyanate or thienylsulfonylisothiocyanate of Formula 8 with the appropriate heterocyclic amine, as shown in Equation 3.

Equation 3

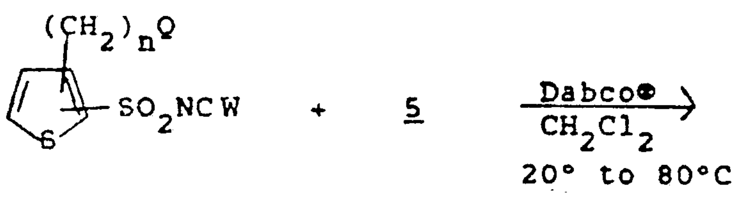

I .

8

The reaction of Equation 3 is most successful when performed in an inert dipolar aprotic solvent such as methylene chloride, tetrahydrofuran or acetonitrile at temperatures between 20° and 80°C. A catalytic amount of 1,4-diazabicyclo[2.2.2]octane (Dabco®) may be used to accelerate the reaction. In cases where the products are insoluble in the reaction solvent, isolation may be performed by simple filtration; when the products are soluble, isolation may be performed by evaporation of the solvent, trituration with a solvent

such as 1-chlorobutane, diethyl ether or methanol and filtration.

Thienylsulfonylisocyanates of Formula 8, can be prepared from sulfonamides of Formula 2 by methods described in U.S. 4,238,621, as indicated in Equation 3a. Alternatively, these sulfonylisocyanates can be synthesized via a two-step procedure, consisting of (1) reacting sulfonamide 2 with n-butylisocyanate in the presence of one molar equivalent of a base such as potassium carbonate in a solvent such as 2-butanone or acetonitrile, to form n-butylsulfonylureas of Formula 9 and (2) reaction of 9 with phosgene using Dabco® as a catalyst in refluxing xylene as solvent. This method is similar to the preparation found in "Newer Methods of Preparative Organic Chemistry," W. Forest, Ed., Vol. VI, Academic Press, NY, 1967, pp. 223-241. Equation 3b illustrates the procedure.

Equation 3a

2 $\xrightarrow[COCl_2/xylene]{C_4H_9NCO}$ 8 .

Equation 3b

(1)

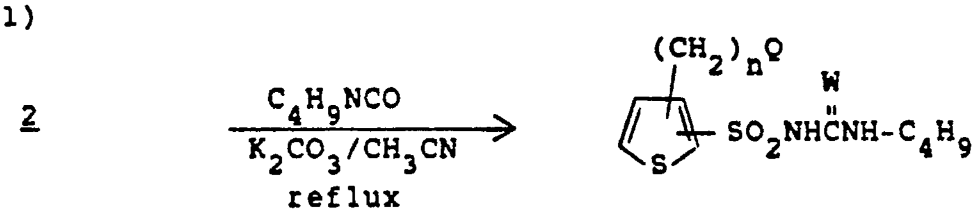


9

(2)

9 $\xrightarrow[xylene,\ reflux,\ Dabco®]{COCl_2}$ 8 .

wherein W is O.

Sulfonyl isothiocyanates of Formula 8, wherein W is S, can be prepared by treatment of sulfonamides of Formula 2 with carbon disulfide and potassium hydroxide followed by reaction of the dipotassium salt with phosgene according to K. Hartke, Arch. Pharm., 299, 174.

The compounds of Formula I also are available by the methodology described in South African Application No. 830441 and illustrated by Equation 4. Thienylsulfonamides of Formula 2 react with heterocyclic carbamates of Formula 10 in 1,4-dioxane at 20° to 80°C for periods of 1 to 24 hours when 1 equivalent of 1.8-diazabicyclo[5.4.0]-undec-7-ene (DBU) is added to the reaction mixture. The resultant products are isolated by dilution of the reaction mixture with water, acidification and subsequent filtration. Heterocyclic carbamates of Formula 10 in turn are synthesized by reaction of heterocyclic amines of Formula 5 with diphenyl carbonate or phenyl chloroformate in pyridine at temperatures ranging from 20° to 80°C, as indicated in Equation 4a.

Equation 4

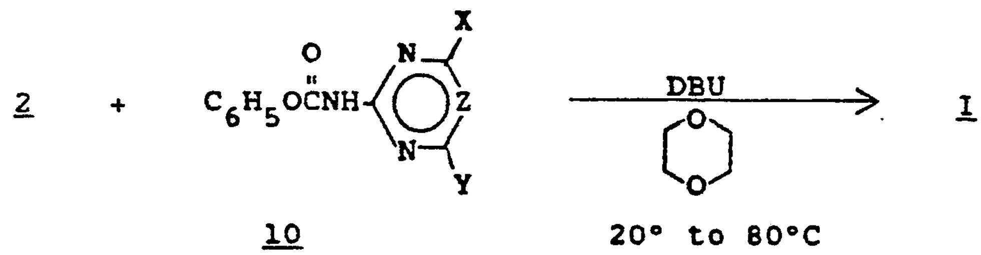


10

Equation 4a

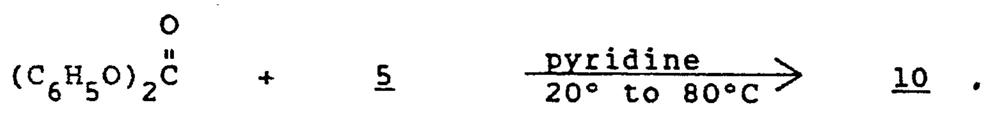


The synthesis of thienylsulfonylureas of Formulae Ia-Ic relies upon the requisite intermediate thiophenesulfonamides of Formula 2, whereas the synthesis of pyridine sulfonylureas of Formulae Id and Ie relies upon the intermediate pyridine-sulfonamide of Formula 196 below..

Some of the intermediate sulfonamides of Formula 2 described above can be prepared from amines of Formula 11 by a two-step procedure, as shown in Equation 5. This consists of (5a) diazotizing 11 and coupling the diazonium salt with sulfur dioxide to form a sulfonyl chloride of Formula 12; and (5b) aminating 12 with ammonium hydroxide or anhydrous ammonia to form 2.

Equation 5

a)

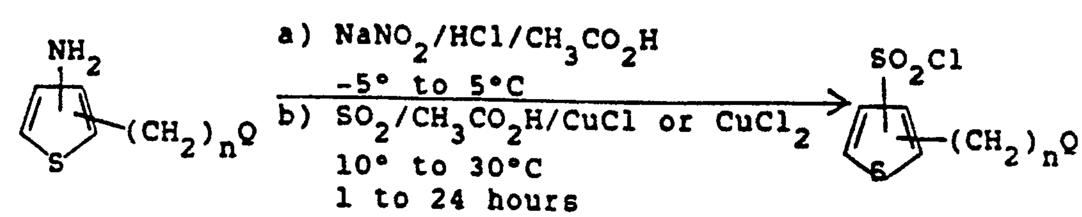


11 12

b)

12

$NH_4OH$ or $NH_3$
-20° to 40°C
0.5 to 10 hours

2

The reaction of Equation 5a is accomplished by treating a solution of amine 11 in a mixture of concentrated hydrochloric acid and glacial acetic acid with a solution of sodium nitrite in water at -5° to 5° C. After stirring for 10-30 minutes at about 0° C to insure complete diazotization, the solution is added to a mixture of an excess of sulfur dioxide and a catalytic amount of copper(I) chloride or copper(II) chloride in glacial acetic acid at about 10° C. The temperature is kept at about 10° C for 1/4 to 1 hour, then raised to 20° to 30° C and held at that temperature for 2 to about 24 hours. This solution is then poured into a large excess of ice water. The sulfonyl chloride 12 can be isolated by filtration or by extraction into a solvent such as ethyl ether, methylene chloride or, preferably, 1-chlorobutane followed by evaporation of the solvent.

The amination described in the reaction of Equation 5b above is carried out conveniently by treating a

solution of the sulfonyl chloride 12 with at least two mole equivalents of anhydrous ammonia in a solvent such as ethyl ether or methylene chloride at -20° to 30° C. If the sulfonamide product 2 is insoluble, it may be isolated by filtration followed by washing out the salts with water. If product 2 is soluble in the reaction solvent, it may be isolated by filtering off the precipitated ammonium chloride and evaporation of the solvent. Additionally, many sulfonamides 2 can be prepared by reaction of corresponding sulfonyl chlorides 12 with excess aqueous ammonium hydroxide in tetrahydrofuran at 0° to about 40° C for 0.5 to 10 hours. The sulfonamide product 2 is isolated by evaporation of the tetrahydrofuran solvent, addition of water to the residue and filtration.

Alternatively, the intermediate sulfonyl chloride 12 can be prepared as shown below in Equation 6.

Equation 6

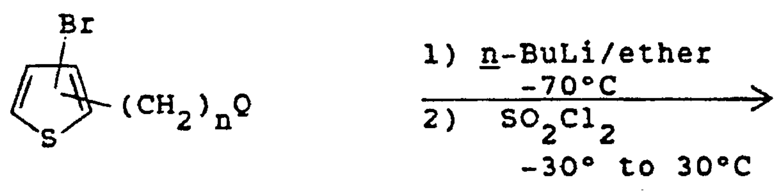


12

13

According to Equation 6, a lithium salt, prepared by reaction of 13 with n-butyllithium in ether at about -70° C, is added to sulfuryl chloride in hexane at about -30° to -20° C and stirred for 0.5 to 10 hours at -30° to 30° C to yield sulfonyl chloride 12, according to teachings of S. N. Bhattacharya, C. Earborn and D. R. M. Walsh, J. Chem Soc. (C) 1968, 1265 and H. Quast and F. Kee, Synthesis 1974, 489. Subsequent reaction of 12 with ammonia or ammonium hydroxide as described above provides the corresponding sulfonamide.

Starting with an appropriate bromothiophene, and carrying out the procedures described in Equation 6, or simple modifications thereof, one skilled in the art may prepare some of the other sulfonyl chlorides of Formula 12 described above. Of necessity, the reactions are limited to those cases in which the substituent $(CH_2)_nQ$ is inert to lithium reagents under the conditions of the reactions, which will be obvious to one skilled in the art. For a general review of metallation with lithium reagents, see H. W. Gschwend and H. R. Rodriguez, Org. Reactions 1979, 26, 1.

Some sulfonamides 2 are best prepared by the procedure of Equation 7 below.

Equation 7

13

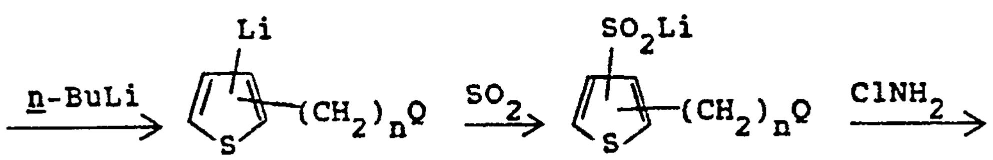


2

14

The preparation of sulfinic acid salts 14 by the procedure of Equation 7 is well known in the art; see U.S. 4,127,405 and H. W. Gschwend et al., loc. cit.. Sulfonamides 2 are best prepared by treatment of sulfinic acid salts with chloramine. In this procedure an ethereal solution or suspension of the salt 14 is treated at low temperature (25 to -30° C) with a dry ethereal solution of chloramine. The reaction is stirred for a period of several minutes to several hours. After filtration, the reaction mixture is washed with aqueous bisulfite, dried and the solvent removed on a rotary evaporator. The crude product is further purified by usual methods such as crystallization or chromatography.

In the reaction shown in Equation 8, a thienyl-copper compound of Formula 15 is reacted with an iodo- or bromoheterocycle ($X(CH_2)_nQ$ where X is I or Br and n is 0, 1 or 2) in a solvent such as pyridine or quinoline, The copper compounds of Formula 15 are prepared by reacting the corresponding lithium

compounds with cuprous iodide or cuprous bromide in a solvent such a diethyl ether. The detailed procedures for this type of reactions are described in the following references: M. Nilsson and C. Ullenius, Acta Chem. Scand. 1970, 24, 2379-2388; C. Ullenius, Acta Chem. Scand. 1972, 26, 3383-3386.

Equation 8

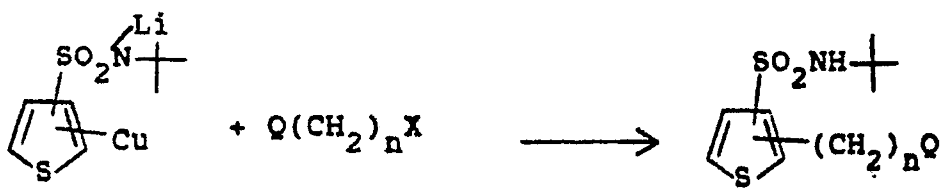

15

16

Treatment of the compounds of Formula 16 with an acid catalyst in an alcohol solvent or in trifluoroacetic acid removes the t-butyl group to yield compounds of Formula 2 as shown in Equation 9.

Equation 9

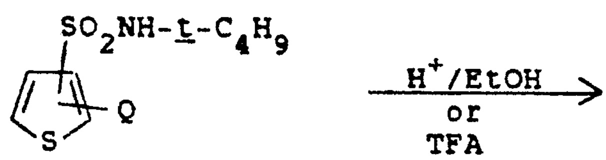


2

16

In the syntheses outlined above, the generalized designation of compounds of Formula I is meant to imply the $(CH_2)_nQ$ substituents and the sulfonylurea functional group are bonded to the thiophene ring in adjacent positions corresponding to one of the three possible combinations designated as Formula Ia, Ib, or Ic in the summary of the invention. It will be convenient for purposes of expediency and clarity of this teaching to use this generalized formula further in this disclosure. All values of Q, n, n', R, $R_1$, $R_2$, $R_3$, $R_3'$, $R_3''$, $R_4$, $R_5$, $R_6$, $R_7$, $R_8$, $R_9$, $R_{10}$, $R_{11}$, n', W, W', W'', A, X, Y, and Z are those described in the summary of the invention unless otherwise noted.

The heterocyclic thiophenesulfonamides of Formula 2 are important starting materials for the preparation of compounds of Formula I of this invention and specific examples are prepared by the following methods.

3-Bromo-2,2'-bithiophene (Q-2, W' is S, n is O) and 3-bromo-2,3'-bithiophene (Q-3, W' is S, n is O) may be prepared by the procedure of, for instance, S. Gronowitz, J. Skramstad and B. Eriksson, Ark. Kemi 1967, 28, 99.

When n is 1 or 2, application of organocopper methodologies known to one in the art yield substituted thiophenes of Formula 21a, as shown in Equation 11a. Thus, a dithienylcopperlithium of Formula 21b can be reacted with alkylhalides of Formula H to yield 21a; conversion to sulfonamide 17b can be accomplished by means of Equation 11.

Equation 11a

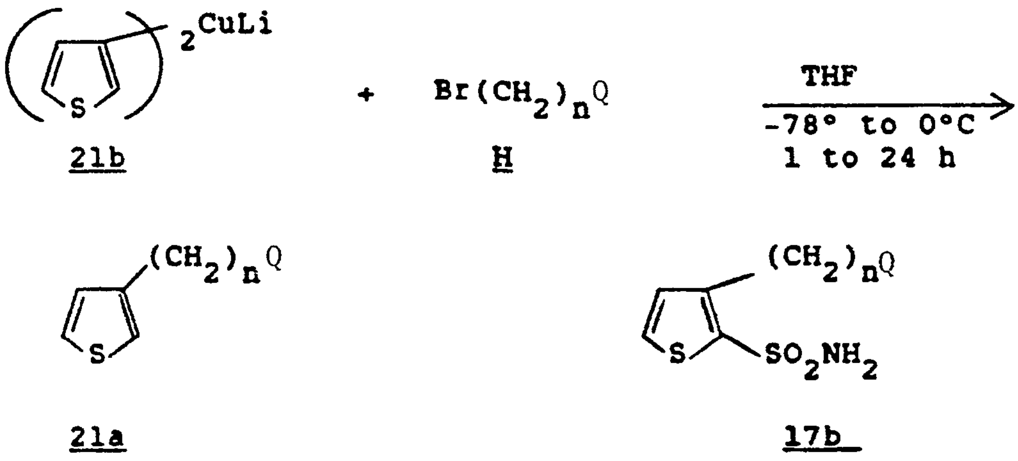


21b H 21a 17b

For the isomeric compounds of Formula 17b with generic structures of Formulae 2b and 2c, similar chemistry as in Equation 11a beginning with 2,3-dibromo- or 3,4-dibromothiophene will provide the requisite intermediates.

Equation 12 describes the synthesis of 3-(2-furanyl)- and 3-(2-thienyl)thiophenes of structure 24 (Q-2, n is O).

Equation 12

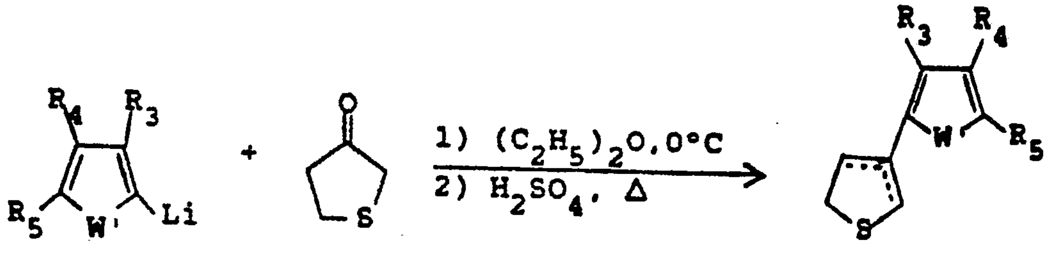


22

22

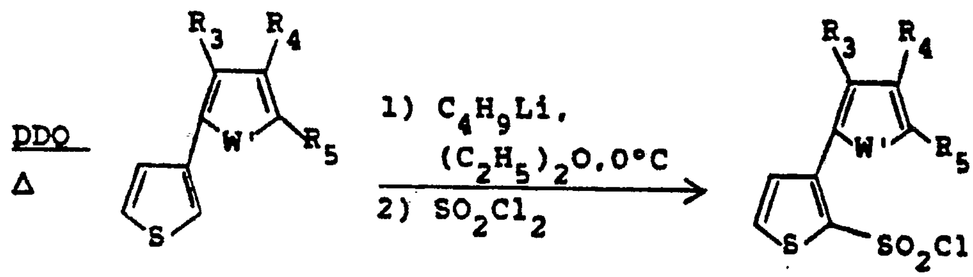


23

24a

24a

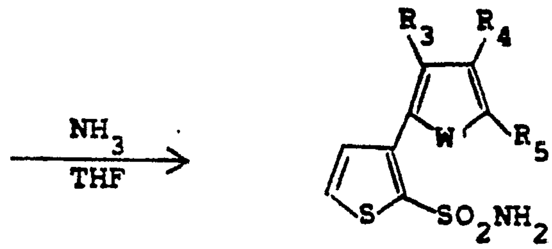


24

According to the work described by H. Wynberg, A. Logothetis, and D. Verploeg J. Am. Chem. Soc. 1957, 79, 1972, 2-lithiofuran or 2-lithiothiophene is added to 3-ketotetrahydrothiophene in diethyl ether at a temperature of 0° C. The reaction is quenched with and distilled from sulfuric acid to afford 3-substituted dihydrothiophenes of Formula 22, which are dehydrogenated using 2,3-dichloro-5,6-dicyano 1,4-benzoquinone (DDQ), giving thiophenes of Formula 23. These thiophenes can be metallated in the 2-position selectively and can be added to sulfuryl chloride in hexane solution, resulting in sulfonylchlorides of Formula 24a which are converted into the desired sulfonamides 24 by treatment with ammonia in THF solution.

For those cases in which n is 1 or 2, a procedure entirely analogous to that of Equation 12 may be used, beginning with Q-2 synthons of Formula 200 (available commercially or obtained by methods obvious to one skilled in the art);

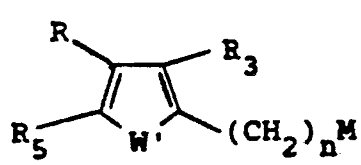

200

compounds of Formula 24b ultimately will be produced

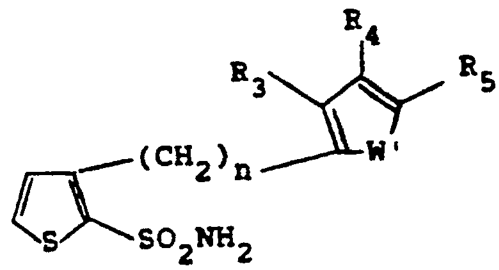

24b

Starting with the appropriate 3-substituted thiophene and following the metallation procedure indicated in Equation 12 or simple modifications thereof, one skilled in the art may prepare many of the sulfonamides of Formula 2a. Only those 3-substituents which are inert to metallating reagents relative to the desired 2-metallation of the thiophene nucleus are compatible with this synthetic strategy; such substituents will be obvious to one skilled in the art.

In complement to the metallation-sulfonation sequence illustrated by Equation 12, equivalent metallation-sulfination may be performed to obtain the sulfonamides of Formula 2a. The latter sequence is described by Equation 13 and is entirely analogous to that of Equation 7.

Equation 13

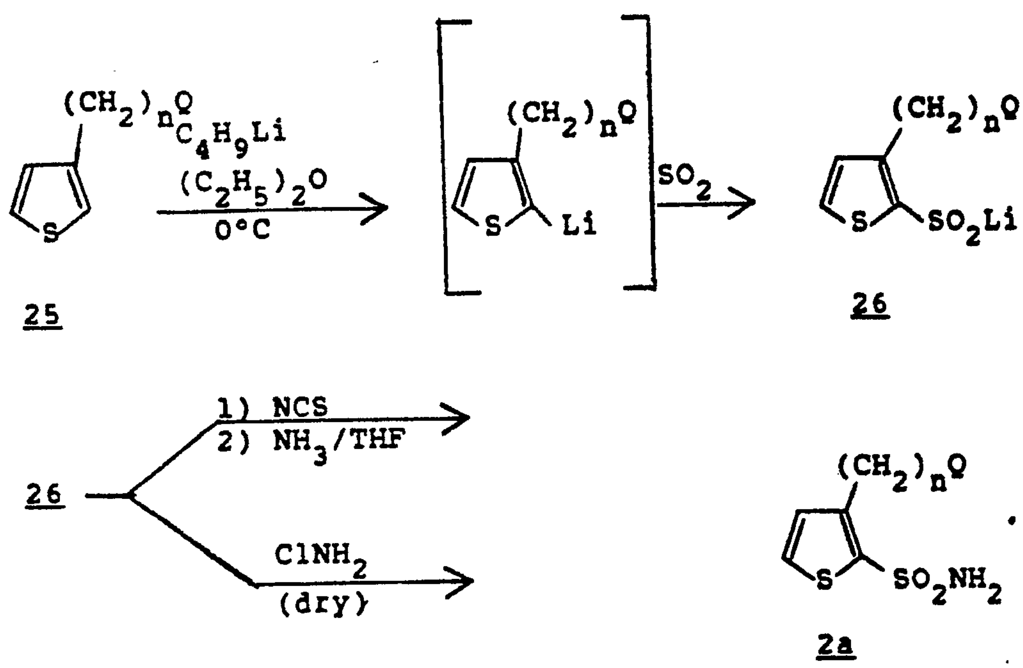


25, 26, 2a

Once sulfinate salts of Formula 26 have been made, they may be transformed into sulfonamides 2a directly by reaction with chloramine as described by G. H. Coleman and C. R. Hauser J. Am. Chem. Soc. 1928, 50, 1193. Sulfinate salts of Formula 26 also may be converted to 2a in a two-step process: chlorination to afford a sulfonyl chloride, as practiced by J. F. Sculley and E. V. Brown J. Org. Chem. 1954, 19, 894; W. E. Trull and E. Wellisch J. Am. Chem. Soc. 1952, 74, 5177 and Y. K. Yuriev and N. K. Sadavaya J. Gen. Chem. USSR 1964, 34, 1814 and treatment of that sulfonyl chloride with ammonia in an ethereal solvent such as THF. Any of these procedures also are compatible with those sulfinate salts of Formula 14 generated via Equation 7.

The use of the strategy of Equation 7 is especially appealing in those cases where metallation of the 3-substituted thiophene of Formula 25 in the 2-position is not possible due to the reactivity of the $(CH_2)_nQ$ substituent under the conditions of metallation. Formation of the 2-brominated thiophene 27 allows for exceedingly facile, mild halogen-metal exchange conditions to be used, as shown in Equation 14.

Equation 14

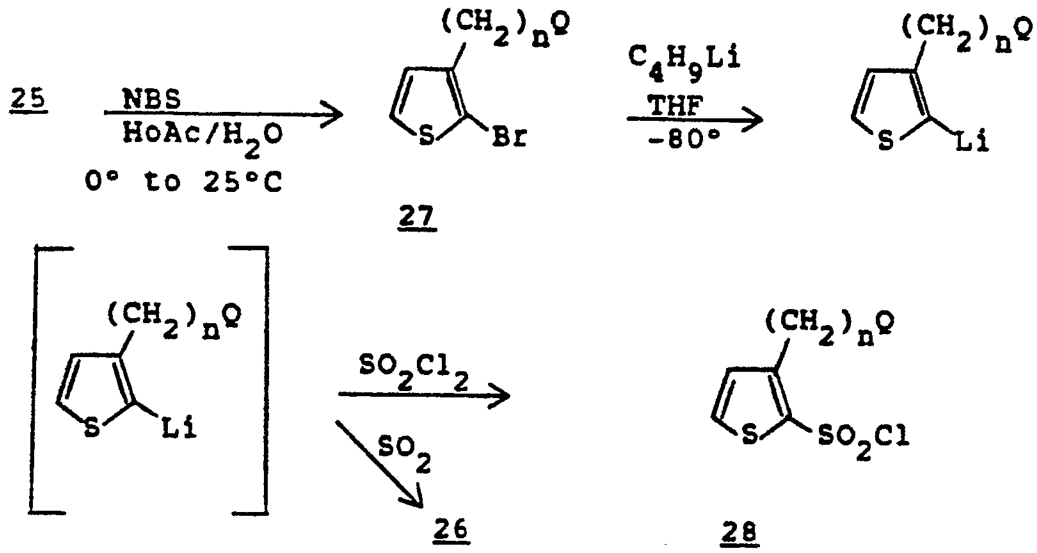


Electrophilic aromatic bromination of 3-substituted thiophenes of Formula 25 leads to the 2-brominated compound of Formula 27 selectively, as shown in the teachings of S. Gronowitz, P. Moses and A. B. Hornfeldt Ark. Kem. 1962, 17, 165. Subsequent halogen-metal exchange and sulfination with sulfur dioxide gives the sulfinate salt 26 or sulfonation with sulfuryl chloride leads to the sulfonyl chloride 28.

In still other cases where $(CH_2)_nQ$ will suffer addition of most nucleophiles, $(CH_2)_nQ$ can be placed at the 3-position of the thiophene nucleus after the sulfonamide moiety or its synthetic equivalent has been incorporated at the 2-position. This strategy is shown in Equation 15.

Equation 15

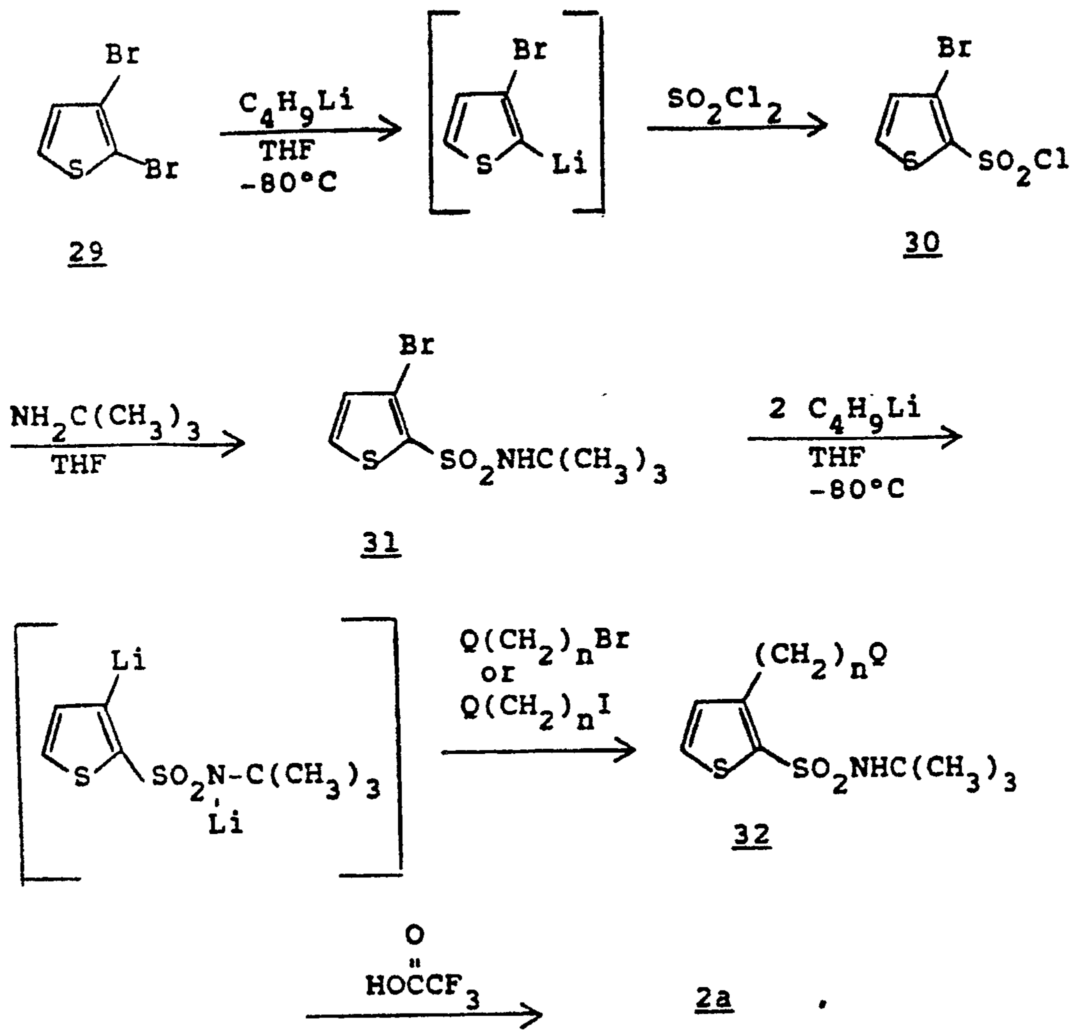


29 30 31 32 2a

2,3-Dibromothiophene, 29 can be lithiated preferentially at the 2-position and treated with sulfuryl chloride to give the sulfonyl halide 30. Treatment with tert-butylamine results in the tert-butyl-protected sulfonamide of Formula 31. A second lithium-halogen exchange reaction allows for an addition of the substituent $(CH_2)_nQ$, which may be added as an intact entity or as a synthetically equivalent substructure.

Given the general techniques of Equations 12 through 15, sulfonamides of Formula 2a may be prepared by the methods described below.

Compounds of Formula 35 can be prepared by use of the reaction sequence illustrated in Equation 16.

Equation 16

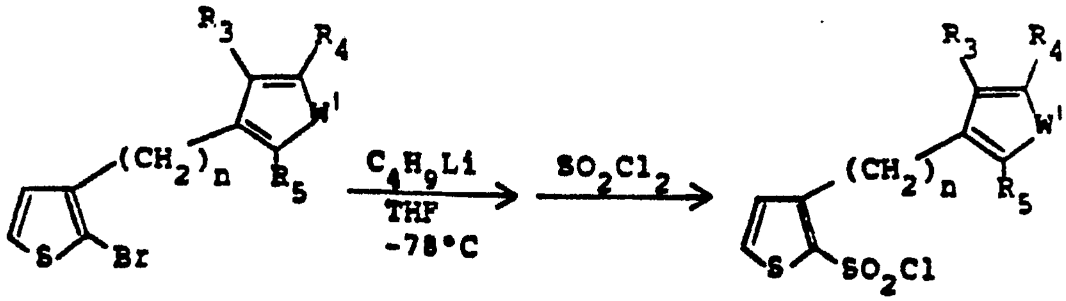


33

34

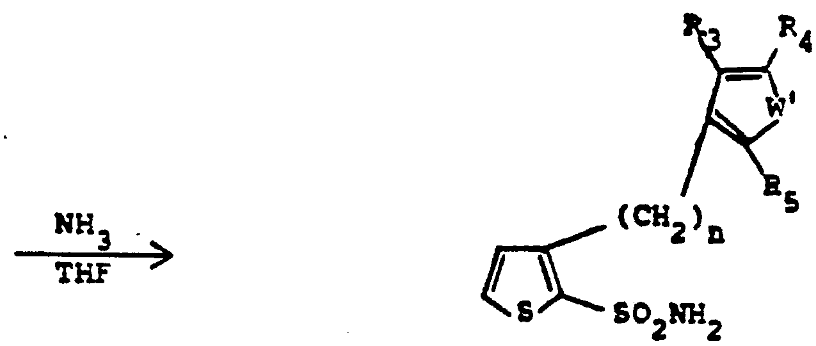


35

3(3-Furanyl)- and 3(3-thienyl)-2-bromothiophenes of Formula 33 may be prepared from the methods of Y. Tamaru, Y. Yoshimi and Z. Yoshida, Tetrahedron 1979, 35, 329 or straightforward modifications thereof. By route of Equation 14 sulfonyl chlorides of Formula 34, and consequently sulfonamides 35, are obtained.

Using the procedure of C. Ullenius, Acta. Chem. Scand. 1972, 26, 3383 and M. Nilsson and C. Ullenius, Acta. Chem. Scand. 1970, 24, 2379 compounds of Formula 36 (Q-2, W=O, n=O) and 37 (Q-3, W'=O, n=O) can be prepared as shown in Equation 17. For those cases in which n is 1 or 2, straightforward modification of Equation 17 using the appropriate furanylcopper species will result in the desired thienylbromide of Formula 37a.

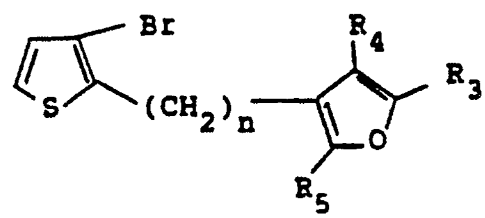

37a

Equation 17

29

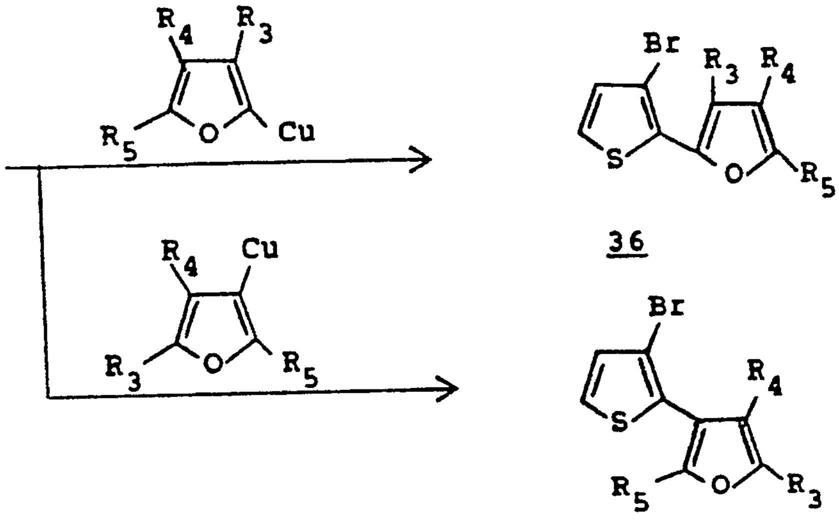

36

37

Sulfonamides containing a tetrahydrofuran or tetrahyrothiophene group (Q-4, Q-5, Q-6, W' = O,S) can be prepared by catalytic reduction of the corresponding furan or thiophene compounds as shown in Equation 18.

Equation 18

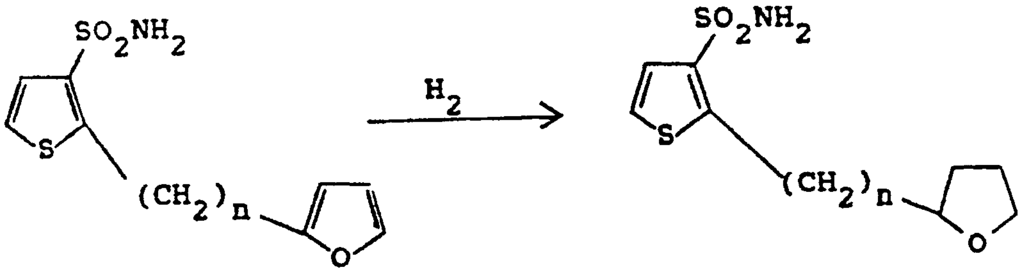

38

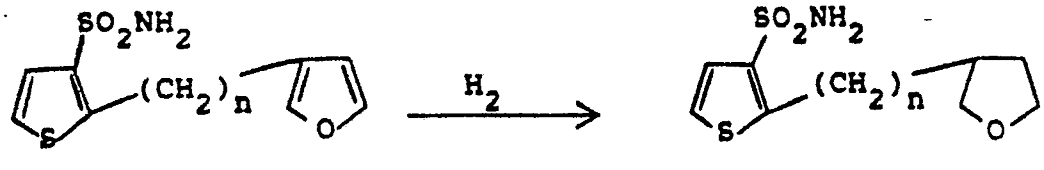


39

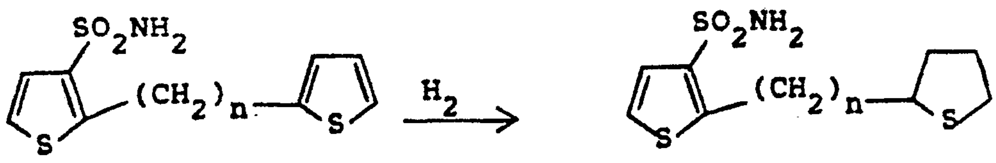


40

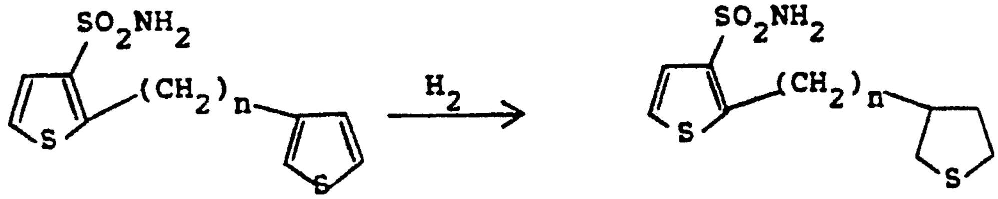


41

Selective reductions of the type shown in Equation 18 are well known in the literature. The choice of catalyst, solvent, pressure and temperature for reduction of furans has been reviewed by Samuel Sevadesh in "The Furans" by A. P. Dunlop and F. N. Peters, Reinhold Publishing Corporation, New York, N. Y. 1953, pp. 674-713; and by P. N. Rylander in "Catalytic Hydrogenation in Organic Synthesis"; Academic Press, 1979, pp. 227-234. The reduction of thiophenes is reviewed by H. D. Hartough in "Thiophene and Its Derivatives"; The Chemistry of Heterocyclic Compounds Series, Interscience Publishers, Inc., New York, N.Y. 1952, pp. 167-169.

Compounds of Formula 42 can be prepared as shown in Equation 19.

Equation 19

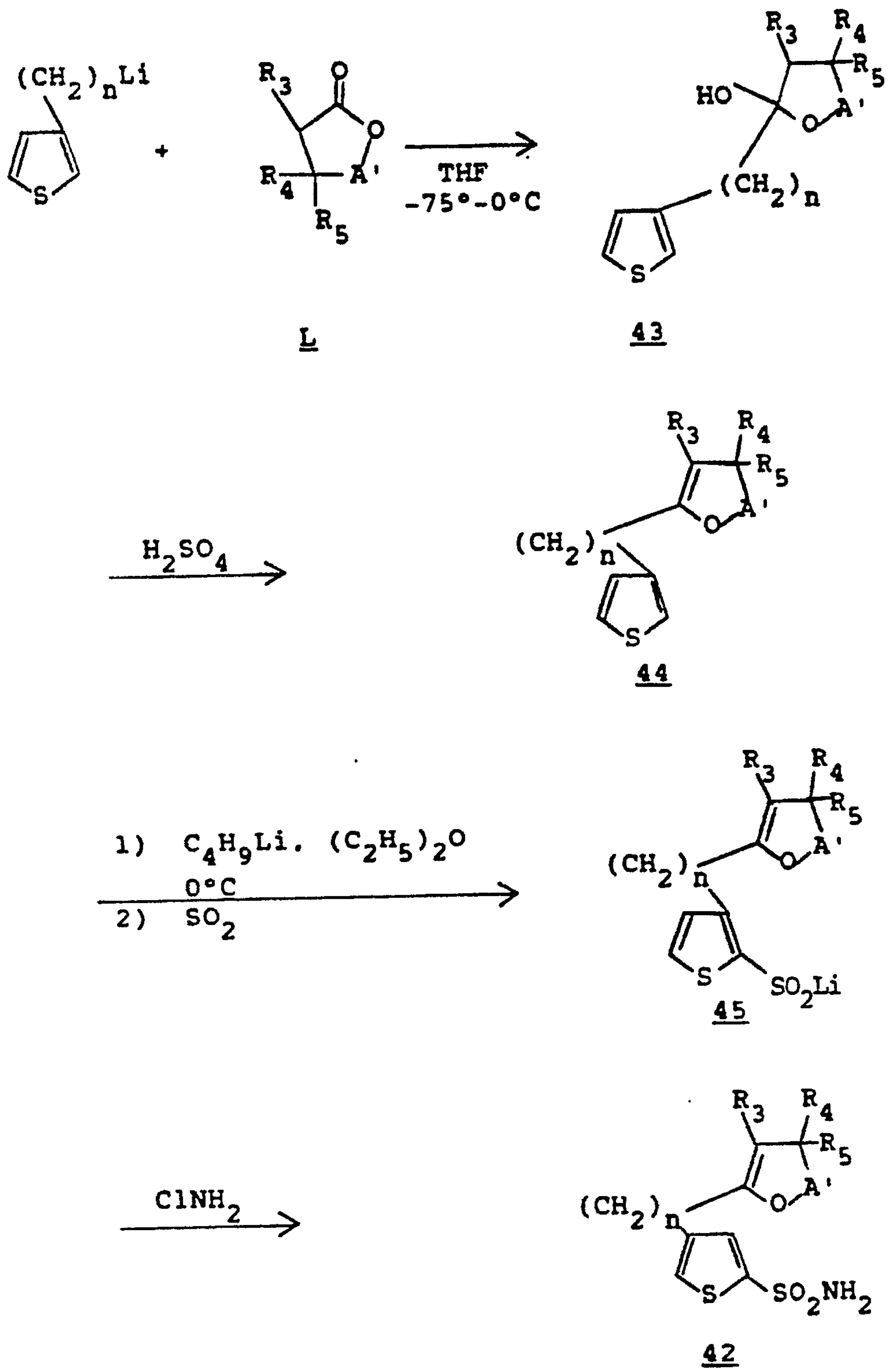


wherein

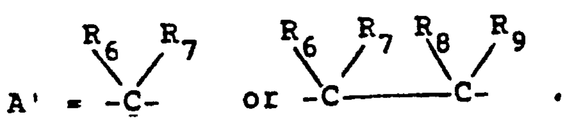

According to Equation 19, a lithiothiophene is reacted with lactone L at -78° to 0° C in an ethereal

solvent such as THF to form lactol 43. The lactol may be dehydrated using a strong acid such as sulfuric acid to form the thiophene of Formula 44. 44 may be lithiated by the route of Equation 13, using n-butyllithium as lithiating agent and diethyl ether as solvent at a temperature of about 0 ° C. Quenching of the reaction gives lithium sulfinates of Formula 45, which are isolated by evaporation of the reaction solvent. The sulfinate is treated with chloramine to yield 42. If desired, selective hydrogenation will yield compounds of Formula 46 ($R_4$ = H).

3-(2-Tetrahydrofuranyl)- and 3-(2-tetrahydrapyranyl)thiophenes of Formula 46 also can be prepared as illustrated in Equation 20.

Equation 20

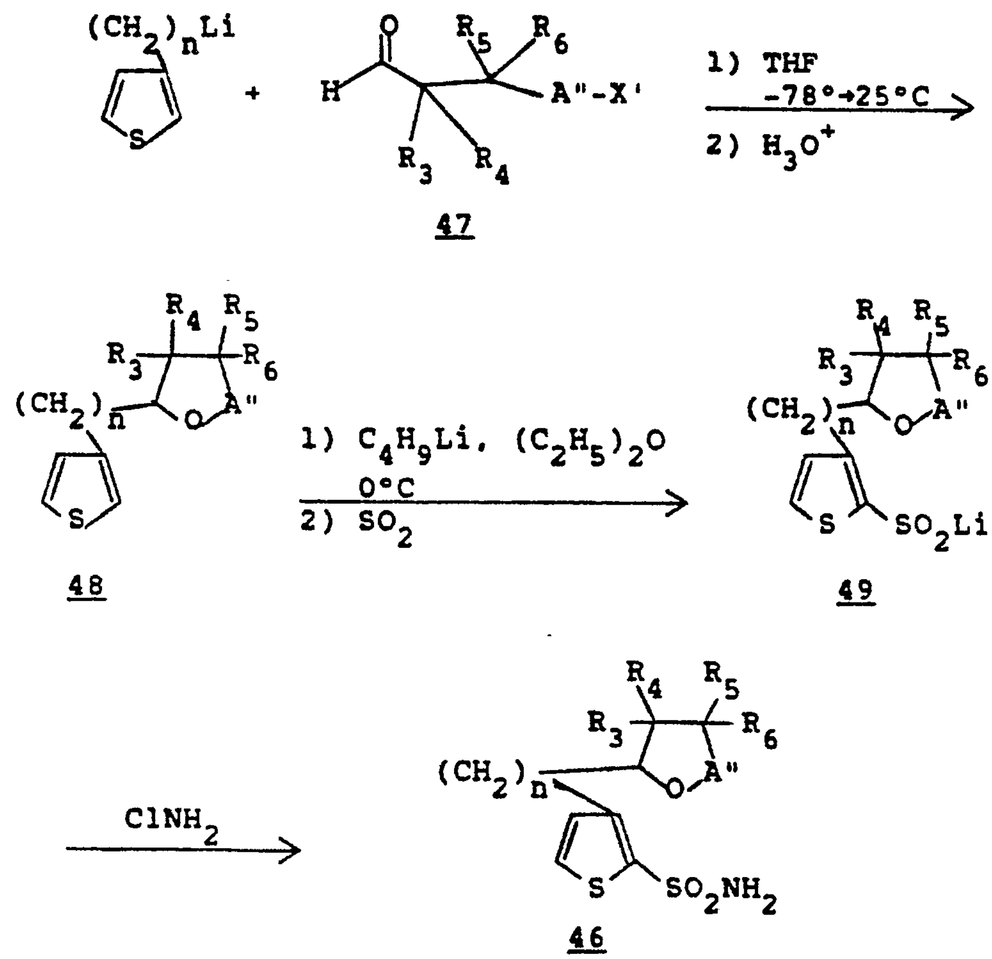


47

48

49

46

wherein

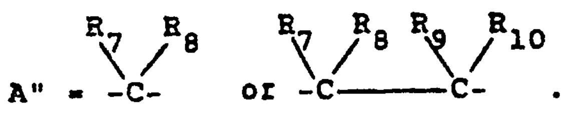

Reaction of a lithiothiophene with haloaldehydes of Formula 47 in inert solvents such as THF at temperatures of -78 ° to 25 ° C affords thienylalkoxides which undergo intramolecular cyclization yielding cyclic ethers 48. These in turn are converted to sulfonamides 46 via the methods of Equation 13: metallation in diethyl ether with n-butyllithium at a temperature of ca. 0 ° C, followed by sulfination using sulfur dioxide gives lithium sulfinate 49. 49 in turn is treated with chloramine to give the target compound 46.

Sulfonamides of Formula 50 are prepared in a manner analogous to that of Equation 12, as shown by Equation 21.

Equation 21

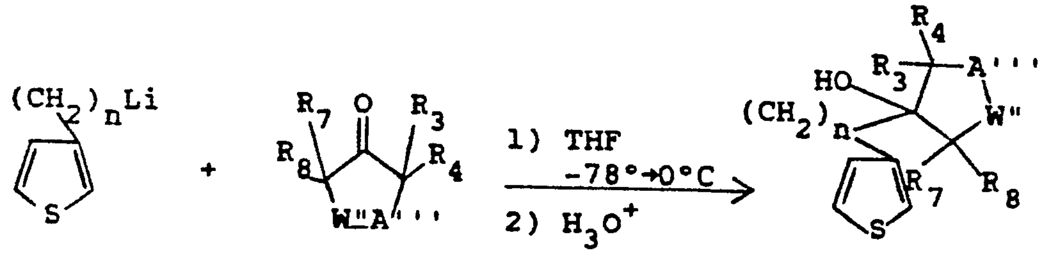


K 51

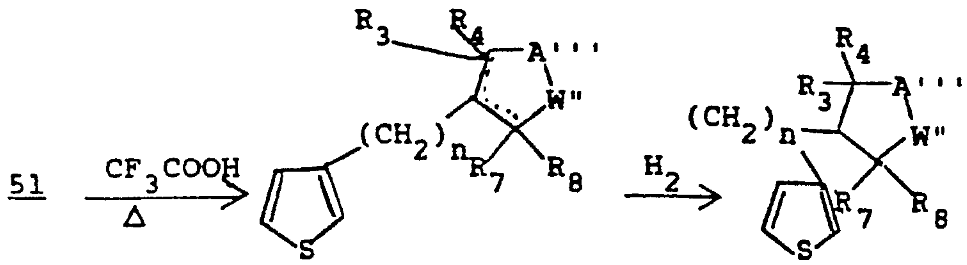


52 53

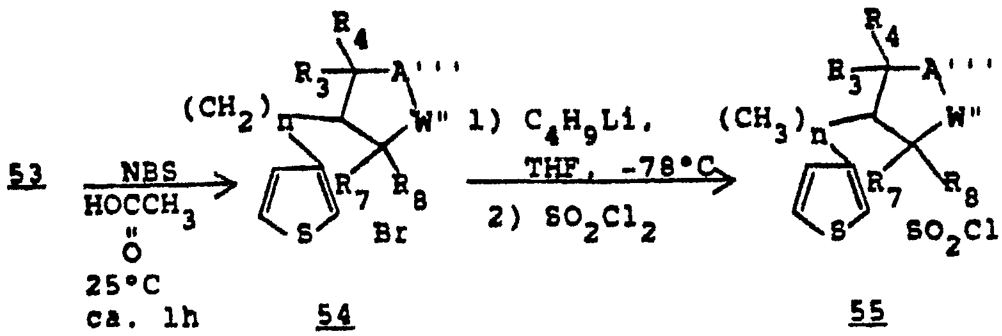


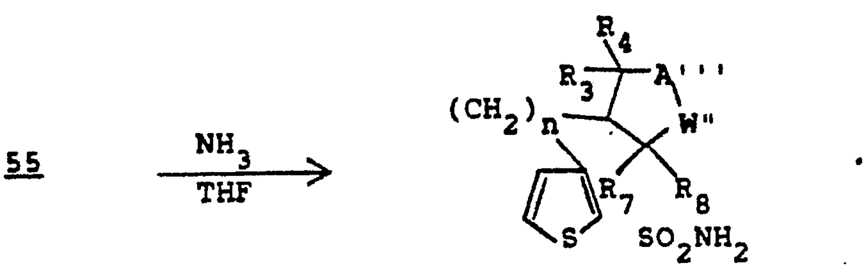


50

wherein

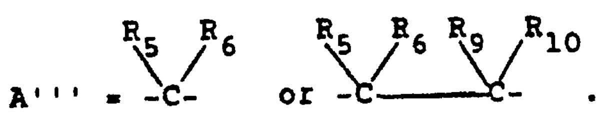


A lithiothiophene reacts with ketones of Formula K to give, after proton quench, tertiary alcohols of

Formula 51. 51 is dehydrated, using a strong acid such as trifluoroacetic acid, resulting in compounds of Formula 52 as mixtures of double bond isomers that are selectively hydrogenated to give 53. Bromination using N-bromosuccinimide in acetic acid for ca. 1 hour at ambient temperatures results in bromides of Formula 54; these bromides undergo halogen-metal exchange as described in Equation 14 to lead to sulfonamides of Formula 50.

Compounds of Formula 56 (Q-7, n is O) can be prepared as shown in Equation 22 by the reaction of the sodium salt of pyrrole with the bromothiophenesulfonamide in DMF with copper oxide catalyst.

Equation 22

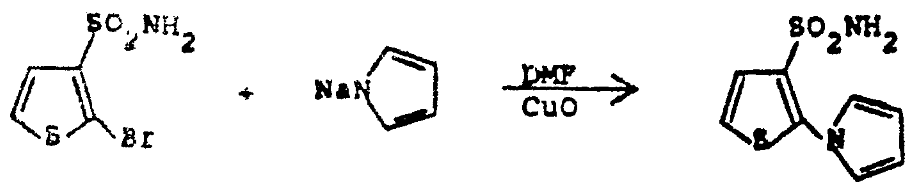


56 .

Alternatively, a 2-aminothiophene-3-sulfonamide can be prepared and reacted with 1,4-dicarbonyl compounds to give substituted compounds of Formula 60 as shown in Equation 23.

Equation 23

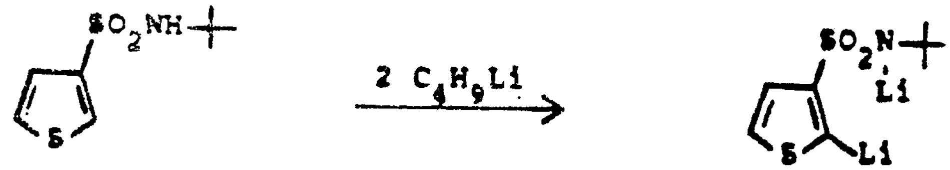


57 58

58

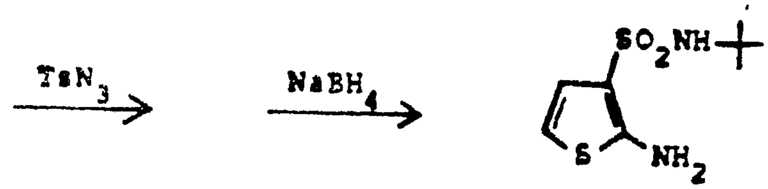


59

59

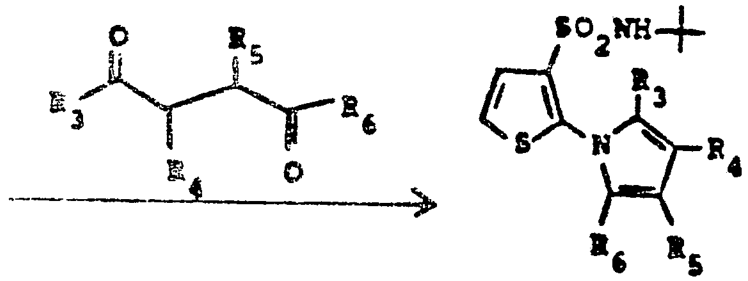

60

The tert-butyl sulfonamide of Formula 57 is treated with two equivalents of n-butyllithium at -50° to 80° C in tetrahydrofuran to give the dilithio salt 58 by analogy with the teaching of J. G. Lombardion, J. Org. Chem, 1971, 36 1843. Treatment of 58 with tosylazide followed by reduction with sodium borohydride gives the aminothiophenesulfonamide 59 by the procedure of J. N. Reedy and V. Smeckus Tetrahedron Lett. 1983, 3795. Condensation with a 1,4-dicarbonyl compound gives the compound of Formula 60. Removal of the t-butyl group affords the free sulfonamide.

3-(1-Pyrrolyl)thiophene 61 can be synthesized by the methods of V. Effi, M. Cugnonde Sevricourt, S. Rault and M. Robba, Heterocycles 1981, 16, 1519. Its conversion to the sulfonamide 62 is described in Equation 24.

Equation 24

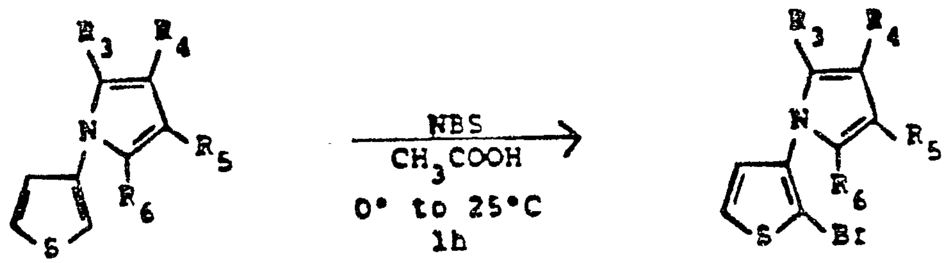


61 63

63 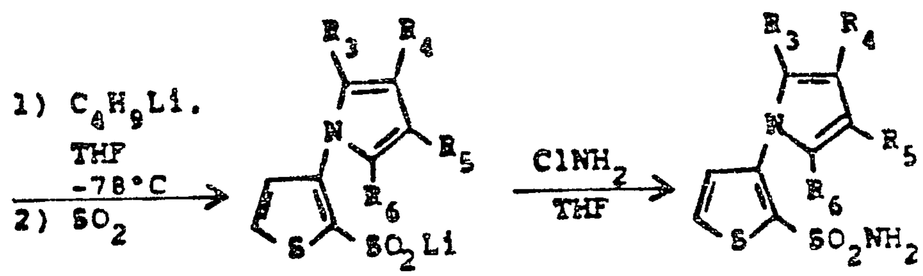


64 62

Selective 2-bromination of 61 may be performed by the use of NBS in acetic acid at temperatures of around 0° to 25° C for 1 hour, resulting in compound of Formula 63. Using the process described in Equation 14, halogen-metal exchange in THF at -78° C and quenching with sulfur dioxide gives lithium sulfinate 64. Amination using chloramine subsequently yields the sulfonamides of Formula 62.

Compounds of Formula 61a can be prepared using the route of Equation 24a.

Equation 24a

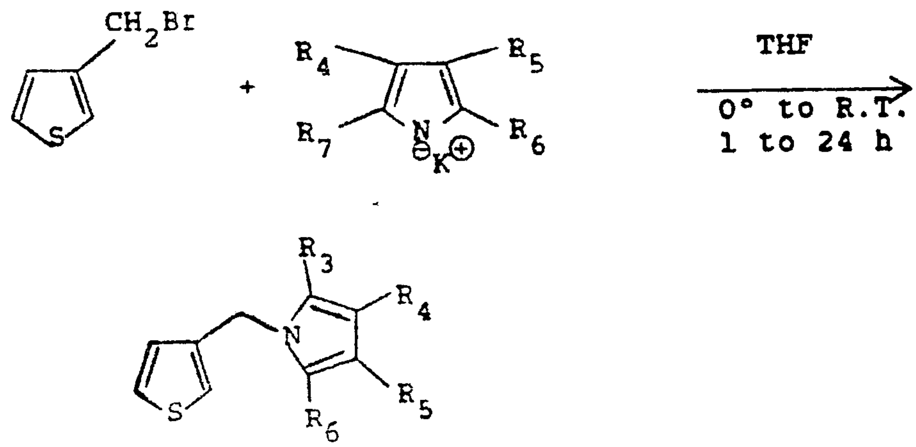


61a

Compounds of Formula 61b are prepared according to Equation 24b.

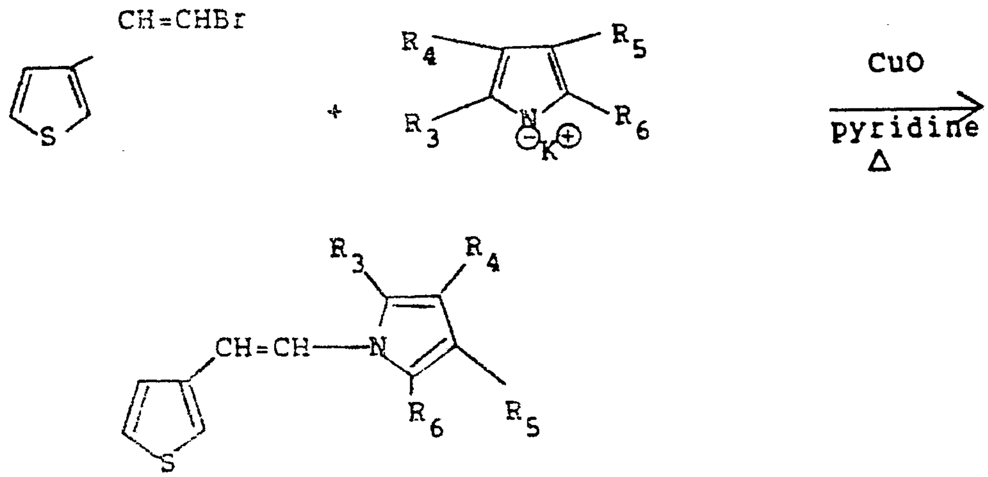


61c

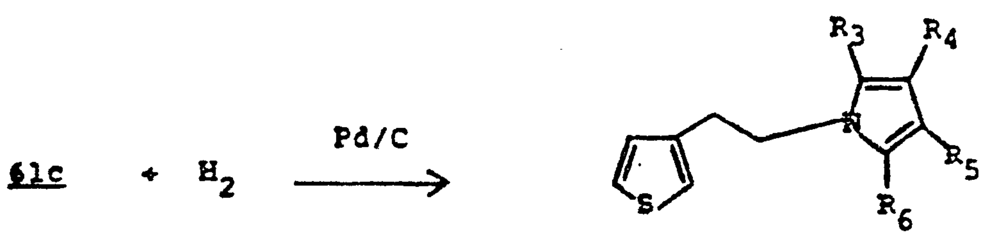


61b

Both 61b and 61a may be converted to sulfonamides of Formula 62a by means of Equation 24.

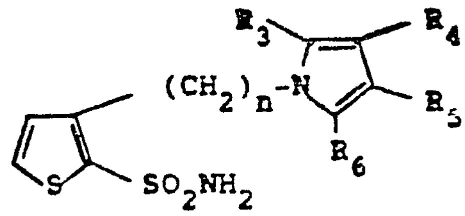

62a

The thiophene of Formula 70 (Q-9, n is O) can be prepared as shown in Equation 26.

Equation 26

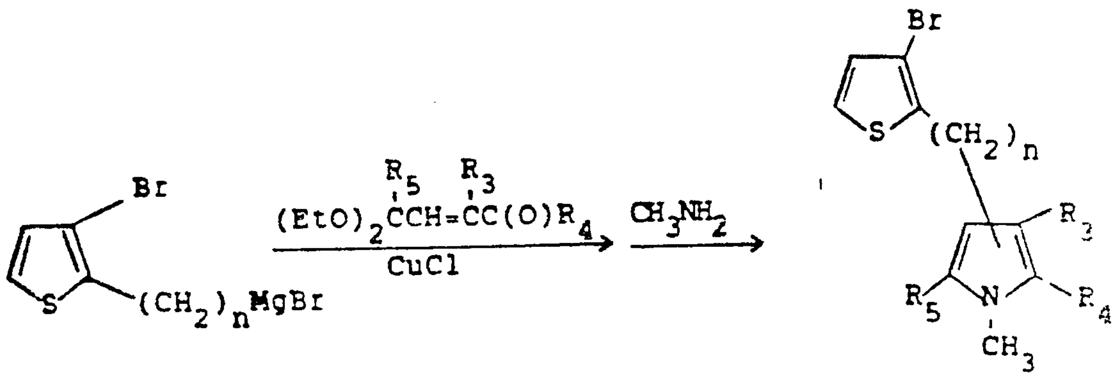


70

The condensation reaction of Equation 26 is well known in the art and has been reviewed by A. R. Jones and G. P. Bean in "The Chemistry of Pyrroles": Academic Press, New York, N.Y. 1977. 3-(1-Methylpyrrol-3-yl)-thiophenes of Formula 71 may be synthesized according to Equation 27.

Equation 27

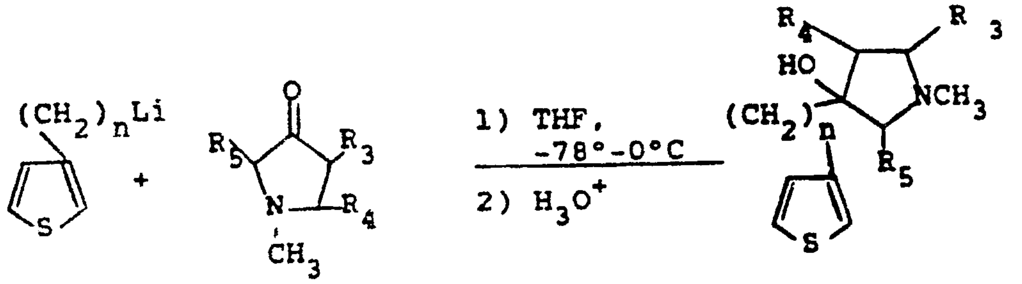


72 73

73 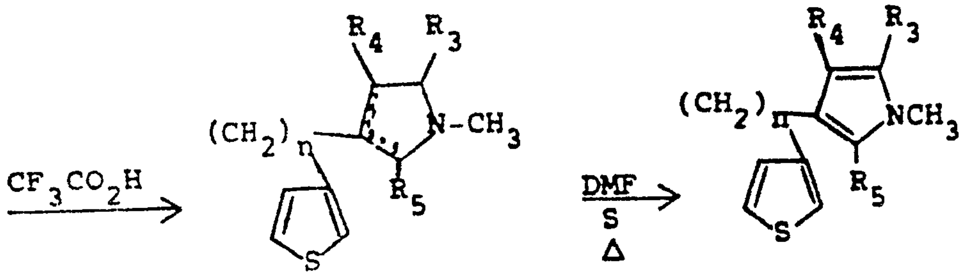


74 71

In a reaction similar to Equation 12, a lithiothiophene attacks the 1-methyl-3-pyrrolidinone, 72, in THF solution at temperatures of -78° to 0° C, resulting in tertiary alcohol 73. Dehydration of 73 by a strong acid such a trifluoroacetic acid results in dehydration products of Formula 74 as a mixture of regioisomers. Dehydrogenation of 74 in hot DMF with sulfur yields 71. Compounds of Formula 71 are converted to the respective sulfonamides of Formula 75 by route of the methods of Equation 14.

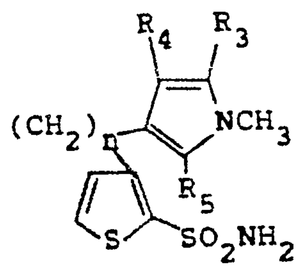

75

Certain methods of synthesis leading to the intermediate sulfonamides of this invention are similar for many values of Q in each of the three possible isomeric arrangements.

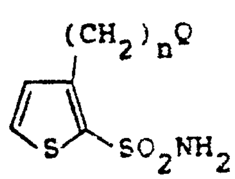

2a

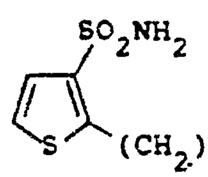

2b

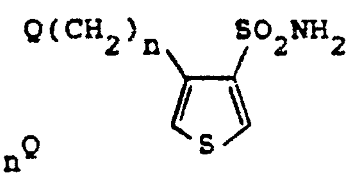

2c

It will be convenient to use the following generalized formulas in this disclosure when certain synthetic methods are applicable to all the necessary isomers.

In the outlined synthesis below, the use of $X^1$ and another substituent on the thiophene ring in undesignated positions is meant to imply that the values indicated are in adjacent positions and that when

$X^1$ is in the 2-position, it is hydrogen and when in the 3-position it is bromine.

An example showing the preparation of certain (5-isoxazolyl)thiophenes of Formula 76 (Q-10,W" = O, n is O) is shown in Equation 28.

Equation 28

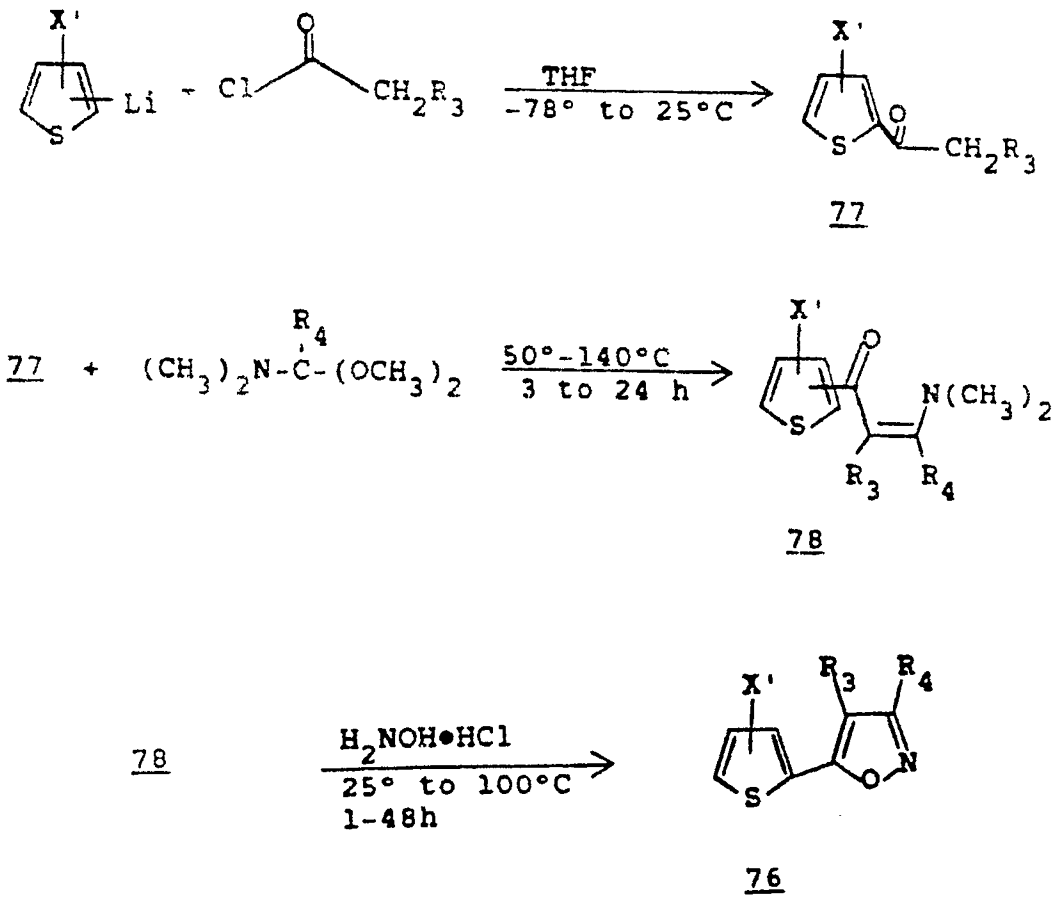


wherein

X' is 2-H or 3-Br

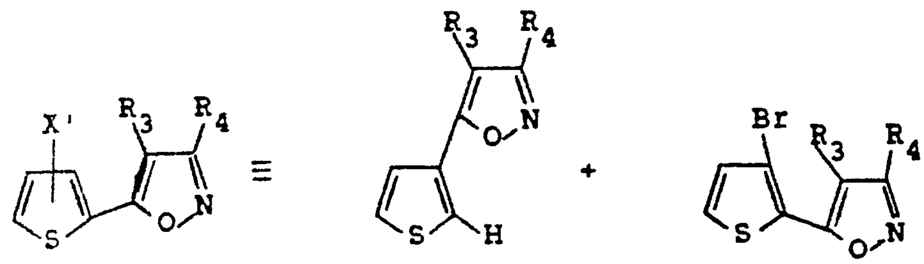

76a 76b

+

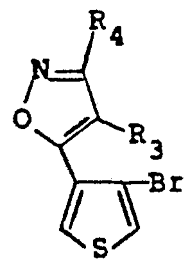

76c

The lithiothiophene is reacted with an acylchloride in THF solution at temperatures of -78° to 25° C, yielding ketones of Formula 77. Treatment of these ketones with dimethyl alkanamide dimethylacetals in solvents such as toluene or DMF at temperatures of 50° to 140° C for a period of 3 to 24 hours results in β-amino α,β-unsaturated ketones of Formula 78. Details of this class of reactions may be found in Technical Information Bulletin, "DMF Acetals"; Aldrich Chemical Co., Dec. 1973 and Y. Lin and S. A. Lang, J. Org. Chem. 1980, 45, 4857. 78 is treated with hydroxylamine hydrochloride in solvents such as ethanol or aqueous 1,4-dioxane at temperatures of 25° to 100° C for 1 to 48 hours and the product 76 is isolated by addition of water to the reaction medium and extraction into methylene chloride. Similar procedures are described by Y. Lin and S. A. Land, J. Heterocycl. Chem. 1977, 14, 345. Compounds of Formula 76 can be converted to the appropriate sulfonamides of Formula 79, by the procedures of Equation 13.

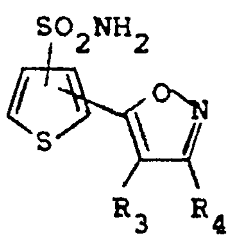

79

For those cases where n is 1 or 2, a modification of Equation 28 may be employed to yield sulfonamides of Formula 79a. Equation 28a illustrates this modification.

Equation 28a

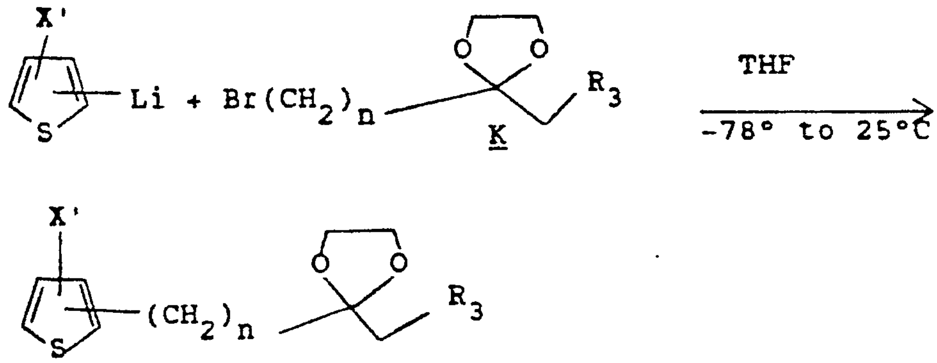


K

77a

77a
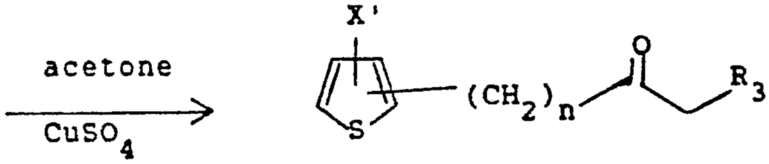


77b

77b
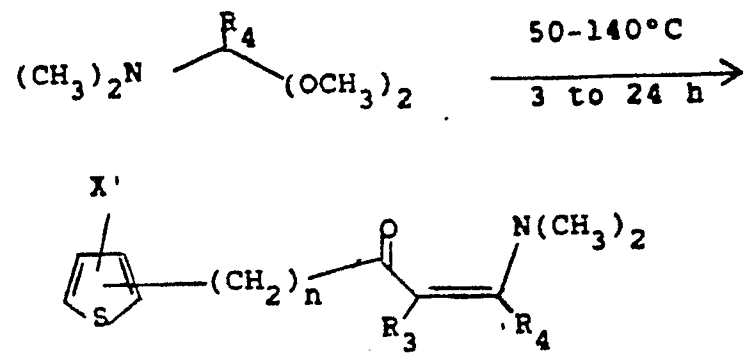


78a

78a
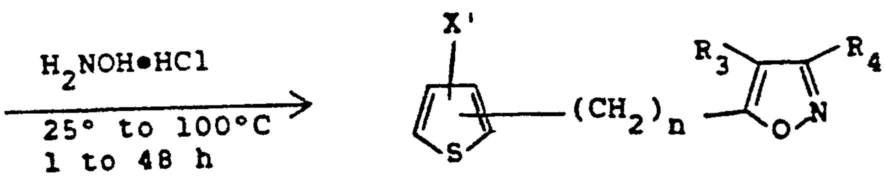


76d

The appropriate lithiothiophene is reacted with an alkyl bromide of Formula K to give the thienylketals of Formula 77a. Transketalization using acetone and anhydrous copper (II) sulfate yield ketones of Formula 77b, which are converted to isoxazoles of Formula 76d in a manner entirely analogous to that described for Equation 28. The compounds of Formula 76d are converted to the appropriate sulfonamides of Formula 79a by the procedures of Equation 13.

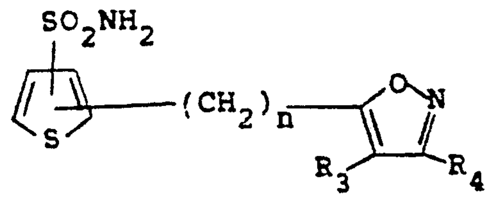

79a

Similarly, 5-isothiazolylthiophenes (Q-10, W' = S, n is O) of Formula 80 can be synthesized from the thioanalogues of 78, as shown by Equation 29; those isothiazolylthiophenes (Q-10, W' = S, n is 1 or 2) of Formula 80a can be prepared in a similar fashion, as illustrated in Equation 29a.

Equation 29

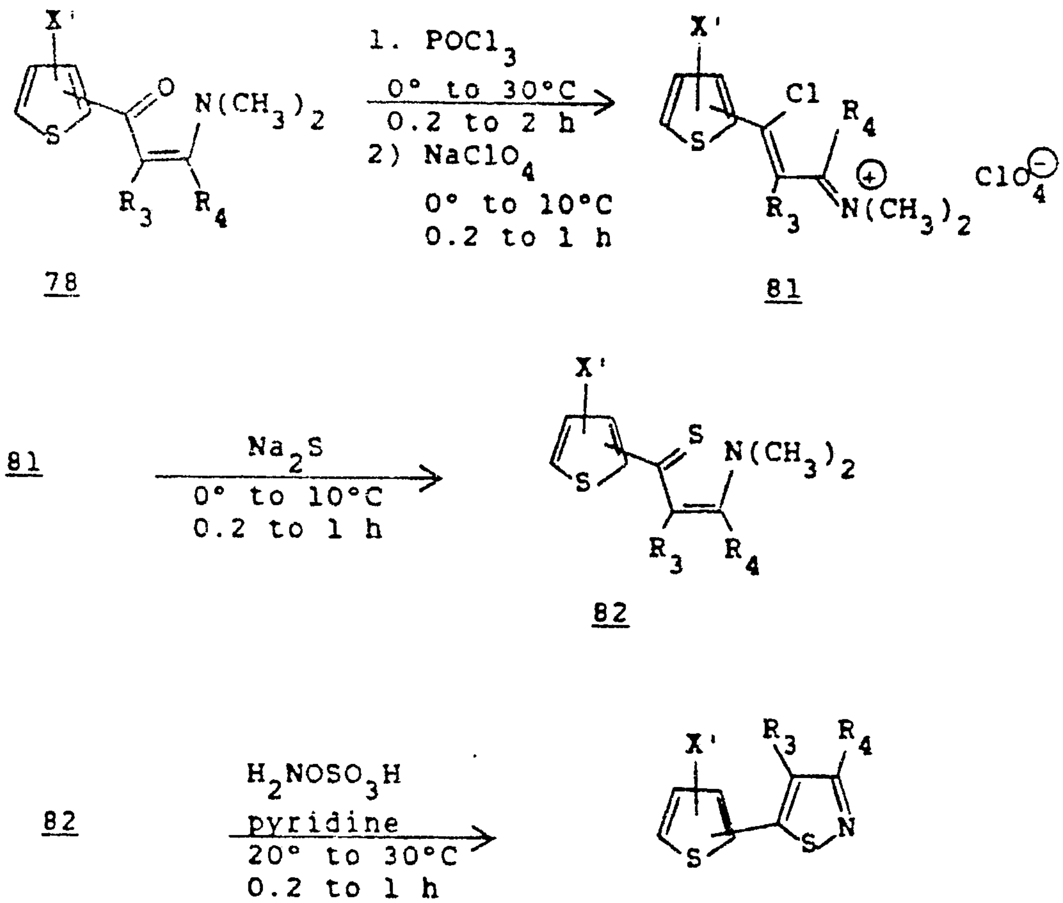


78 81 82 80

wherein

X' is 2-H or 3-Br.

Equation 29a

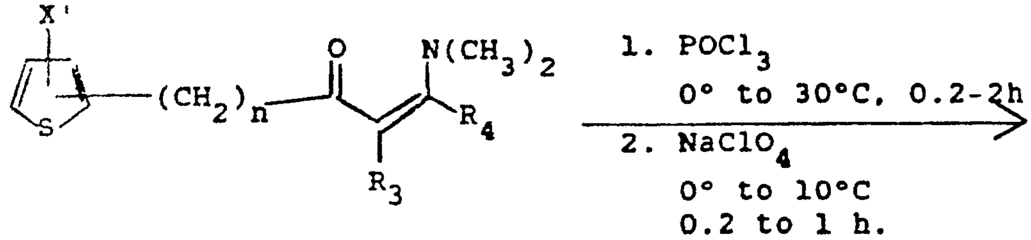


78a

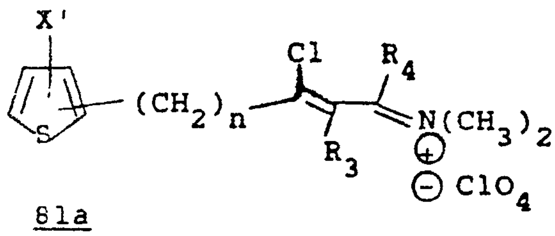

81a

81a

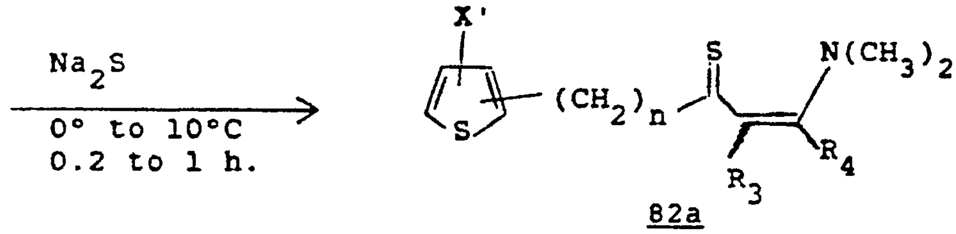


82a

82a

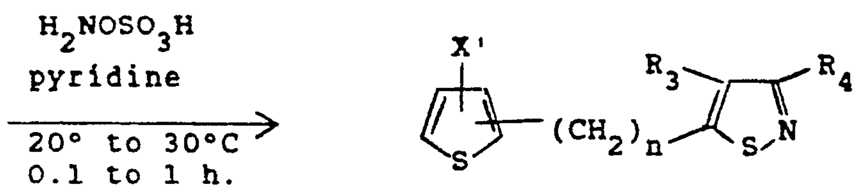


80a

Compounds of Formula 78 can be treated with phosphorous oxychloride in methylene chloride at 0° to 30°C for 0.2 to 2 hours and followed by addition of sodium perchlorate in water at 0° to 10°C for 0.2 to 1 hour to form perchlorate salts of Formula 81. Subsequently, 81 can be reacted with sodium sulfide monohydrate in DMF and water at 0° to 10°C for periods of 0.2 to 1 hour to form thiones of Formula 82. Finally, these thiones will react with hydroxylamine-O-sulfonic acid and 2 equivalents of pyridine in methanol at temperatures of 20° to 30°C for 0.2 to 1 hour to give 80. Thiophenes 80 and 80a may be converted to sulfonamides of Formula 83 and 83a by route of Equation 13.

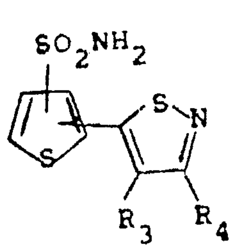

83

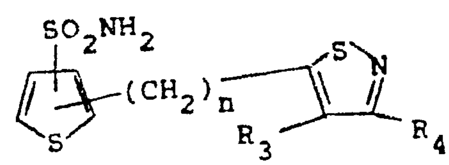

83a

The preparation of thiophenes of Formula 84 (Q = Q-11, W" is $NR_{11}$) and 85 (Q = Q-10, W" is $NR_{11}$) is

illustrated in Equation 30.

Equation 30

78 or 78a

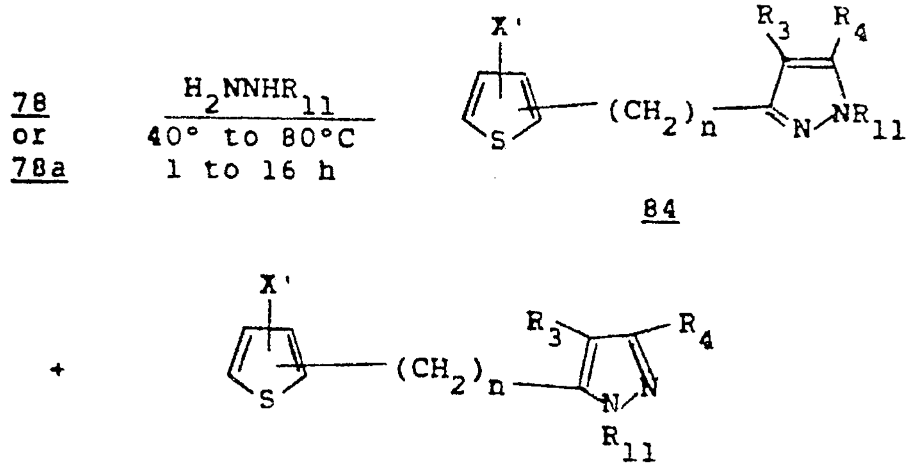


84

85

The α,β-unsaturated ketones of Formulae 78 and 78a from Equation 28 and 28a can be reacted with hydrazines in refluxing ethanol for periods of 1 to 16 hours. The product mixture of thiophenes of Formulae 84 and 85 are isolated by chromatographic methods which are well known to those skilled in the art. Conversion of either 84 or 85 to the corresponding sulfonamides of Formulae 86 and 87 proceeds by way of the processes described in Equation 14.

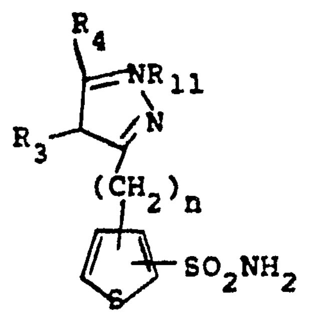

86

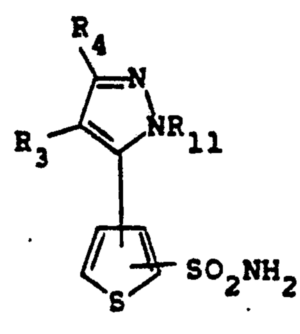

87

Equation 31 depicts the synthesis of 3-(3-isoxazolyl)thiophenes of Formula 91.

Equation 31

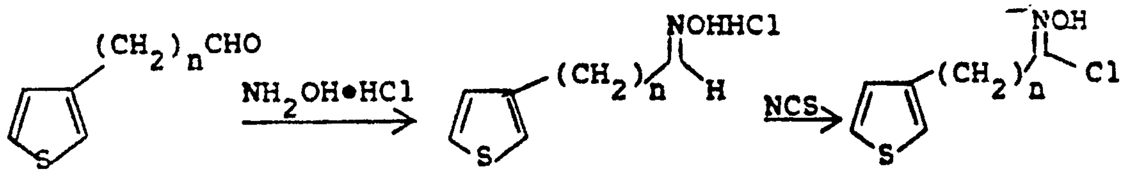


88 89

89 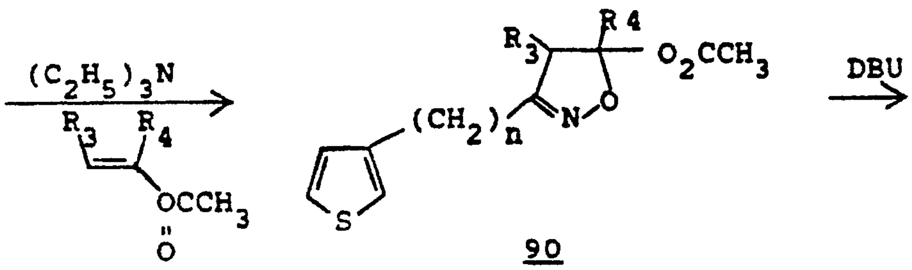


90

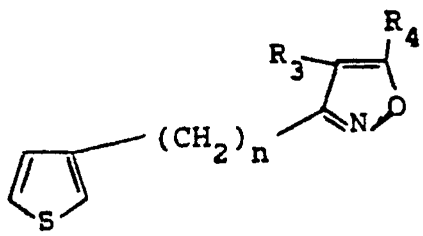

91

The 3-thienylaldehyde is reacted with hydroxylamine hydrochloride in ethanol to form oximes of Formula 88. These oximes are chlorinated by means of N-chlorosuccinimide (NCS), yielding chlorides of Formula 89 which, when reacted with vinyl acetates in the presence of one molar equivalent of triethylamine, undergo addition-cyclization to form acetates of Formula 90. Oxidative extrusion of acetic acid in hot DBU transforms 90 into thiophenes of Formula 91. Conversion to sulfonamides of Formula 92 can be accomplished by application of the chemistry described by Equation 13.

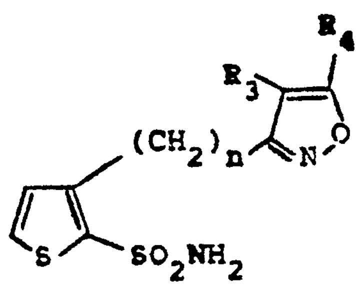

92

Equation 32 below illustrates a method for preparing 3-(3-bromothienyl)isoxazoles of Formula 93.

Equation 32

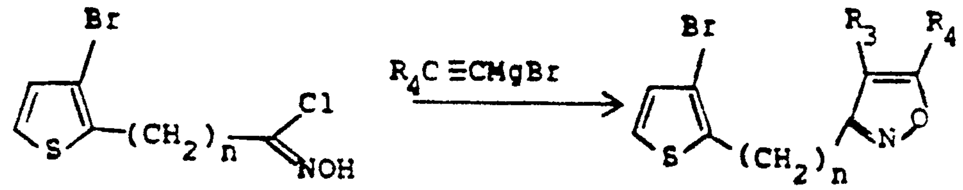


94 93

where $R_3$ = H.

The reaction of Equation 32 is run by procedures similar to those taught by M. Lanella et al., Chim. Ind. (Milan) 1965. 47, 996 and by G. Gaudiano et al., Gazz. Chim. Ital. 1959, 89, 2466. Thus, a 3-bromo-2-thiophenehydroxamic acid chloride of Formula 94 is reacted with an appropriate acetylenic Grignard reagent in tetrahydrofuran at 0° to 30°C for 1 to about 16 hours. The product is isolated by addition of water and ammonium chloride and extraction with methylene chloride. The acetylenic Grignard reagents are prepared from substituted acetylenes by procedures described in the cited references.

Another method of preparation of compounds of Formula 93 is shown in Equation 33.

Equation 33

94 +

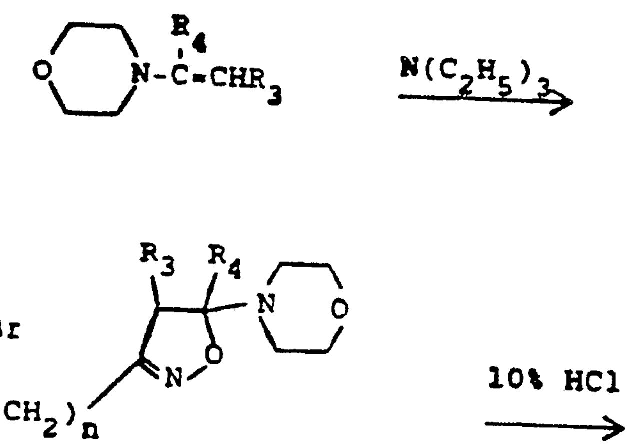


93

95

The reactions of Equation 33 above can be run by procedures similar to those described in G. Bianchetti et al., Gazz. Chim. Ital. 1963, 93, 1714. Thus, in Equation 33, 94 is reacted with an equimolar amount of triethylamine and a N-alkenylmorpholine in chloroform at reflux for 0.2 to about 1 hour to form a 5-(N-morpholino)-3-substituted-isoxazoline of Formula 95, which then is reacted with 10% hydrochloric acid at reflux for about 0.2 to 0.5 hour to from 93. The product 93 is isolated by extraction with methylene chloride.

Similarly, a reaction of the hydroxamic acid chloride with a vinyl acetate rather than an enamine can yield compounds of Formula 93 by the procedure of R. Micetich, Can. J. Chem. 1970, 48, 467.

(3-Isothiazolyl)thiophenes of Formula 96 can be prepared according to Equation 34.

Equation 34

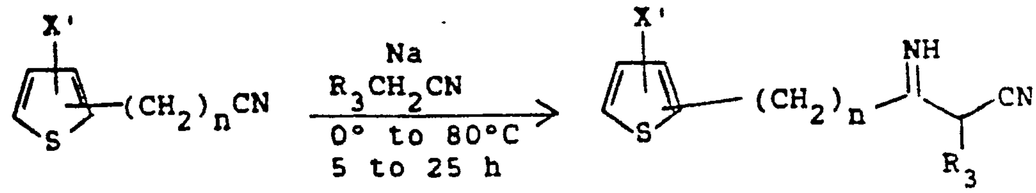


97

97

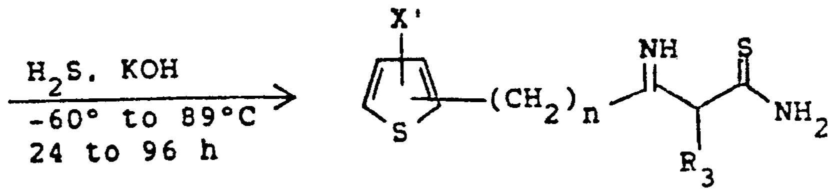


98

98

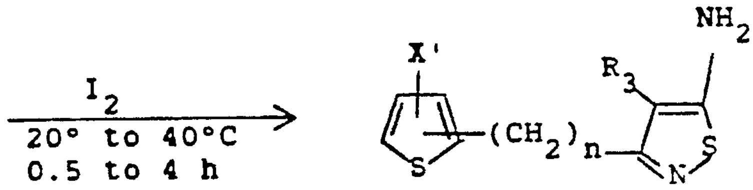


99

99

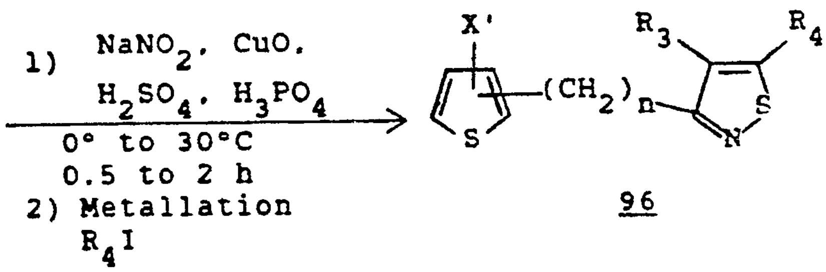


96

In Equation 34, the cyanothiophene can be reacted with an appropriate alkyl nitrile and sodium in a solvent such as ether or toluene at 0° to 80°C for approximately 5 to 25 hours, forming mononitriles of Formula 97. Similar transformations may be found in U.S. 3,479,365, Netherlands 6,608,094 and T. Naito, S. Nakagawa, J. Okumura, K. Takahashi, K. Masuka and Y. Narita. Bull. Chem. Soc. Japan 1968, 41, 965. 97 can be reacted with hydrogen sulfide in the presence of a catalytic amount of potassium hydroxide in methylene chloride at -60° to 80°C in a sealed vessel for 24 to 96 hours to give iminothioamides of Formula 98. Such a reaction parallels the work of T. Naito, S. Nakagawa and K. Takahashi, Chem. Pharm. Bull. 1968, 16, 148 and J. Goerdeles and H. Pohland, Chem. Ber. 1961, 94, 2950. Compounds of Formula 98 are cyclized by treatment with iodine in ether, chloroform or ethanol solution with potassium carbonate used as base at temperatures of 20° to 40°C for 0.5 to 4h, giving amines of Formula 99 in a manner similar to ibid., Netherlands 6,608,094 and J. Goerdeler and H. Pohland, Angew. Chem. 1962, 72, 77. Diazotization of 99 by nitrous acid generated in situ and reaction of the resultant diazonium salt with copper-(II) oxide in 50% phosphoric acid at 0°C for 2 hours followed by optional selective metallation and alkylation forms 96, in analogy to M. Beringer, B. Prijs and H. Erlenmeyer, Helv. Chim. Acta 1966, 49, 2466. Conversion to the corresponding sulfonamide 100 is accomplished using the synthetic scheme described in Equation 13.

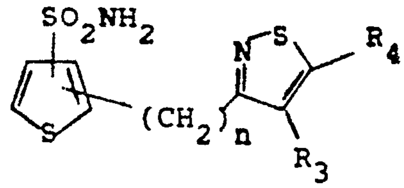

100

As shown in Equation 35 below, (3-bromothienyl)-isoxazoles of Formula 101 (Q-12,W" is O) can be prepared by reacting compounds of Formula 102 with hydroxylamine hydrochloride.

Equation 35

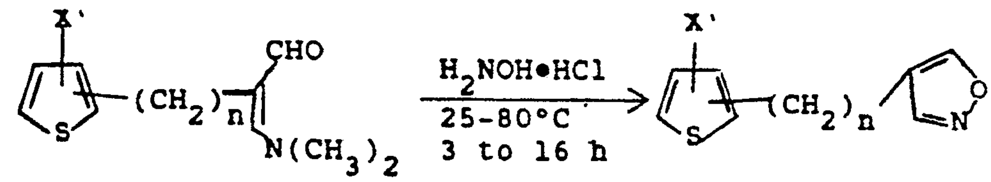


102 101

The reaction of Equation 35 is run in ethanol at 25° to 80° C for 3 to 16 hours. The product is isolated by addition of water and extraction with methylene chloride. The product is purified by recrystallization or column chromatography. The starting materials are prepared by known methods, e.g., V. Hengartner et al., J. Org. Chem., 1979, 44, 3748. For compounds of Formula 101a with substituents on the isoxazole ring, the reactions of Equation 36 illustrate a procedure similar to those described by H. Yasuda, Yakugaku Zuschi, 1959, 79, 623 and Bobranski and Wojtowski, Roczniki Chem., 1964, 38, 1327.

Equation 36

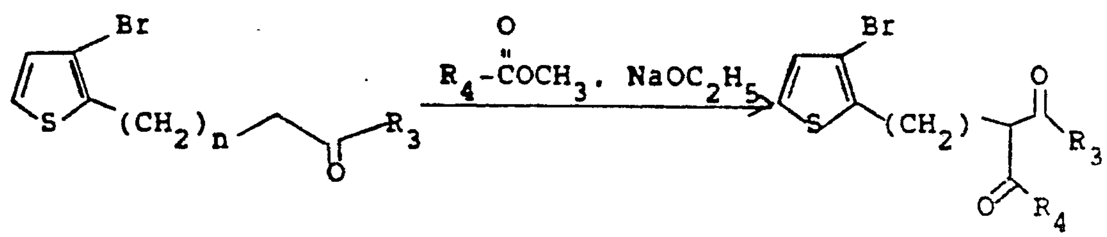

103 104

104

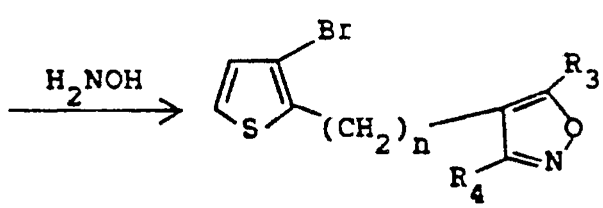


101a

4-Substituted isothiazoles of Formula 105 can be prepared in a manner analagous to that of Equation 29, beginning with those compounds of Formula 102. Subsequent conversion to sulfonamides of Formula

106 can be achieved by means of Equation 8.

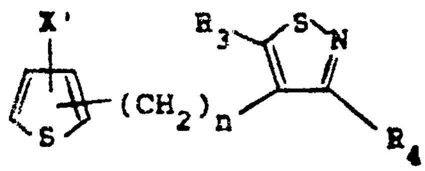

105

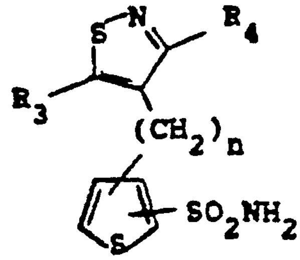

106

(1-Methylpyrazol-4-yl)thiophenes of Formula 107 (n = O) are prepared by the teachings of S. Liljefors and S. Gronowitz, Chem. Scr. 1979, 15, 102. The respective sulfonamides of Formula 108 may be synthesized by the methods described in Equation 8. When n is 1 or 2, the thiophenes of Formula 107, and their respective sulfonamides 108 are prepared in an analogous manner.

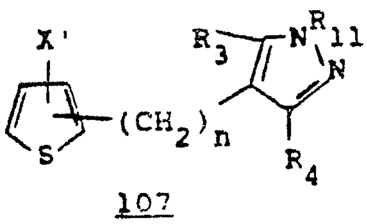

107

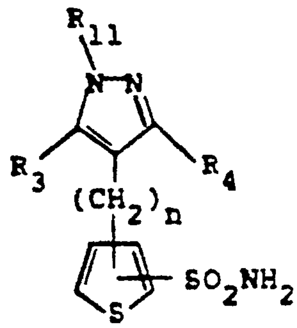

108

Equations 37 and 38 illustrate the preparation of (2-oxazolyl)thiophenes of Formula 109. Details of these preparations may be found in W. E. Cass, J. Am. Chem. Soc. 1942, 64, 785 and "Heterocyclic Compounds"; Vol. 5, R. C. Elderfield, Ed. J. Wiley and Sons, NY, 1957, Ch. 5.

Equation 37

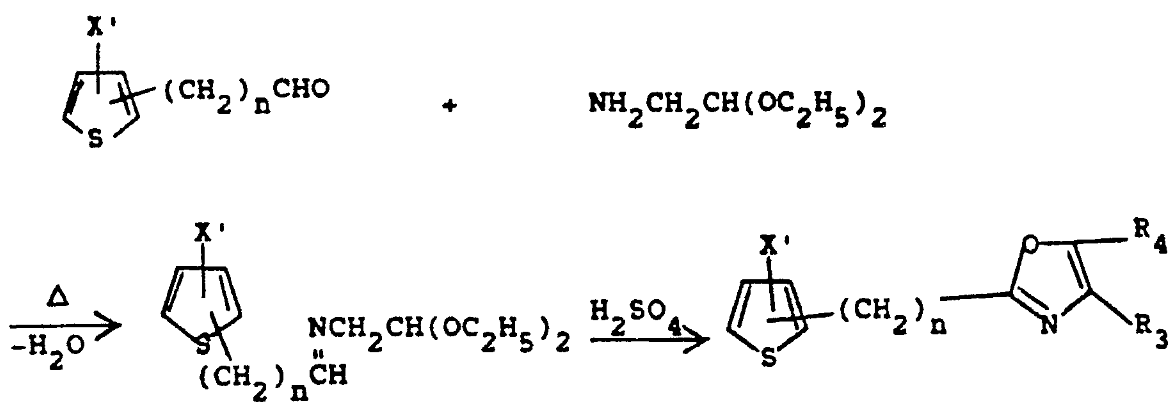


110

109

wherein $R_3$ and $R_4$ are H.

Equation 38

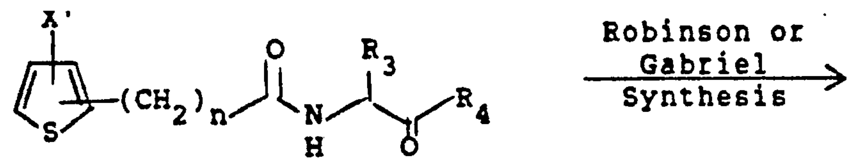


109

111

Thiophenes of Formula 109 can be converted to the corresponding sulfonamides of Formula 112 by the methods described in Equation 13.

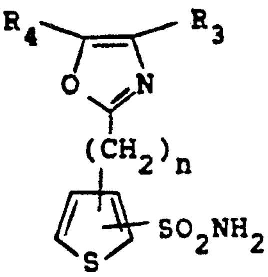

112

(2-Thiazolyl)thiophenes of Formula 113 can be prepared by the procedure of Equation 39.

Equation 39

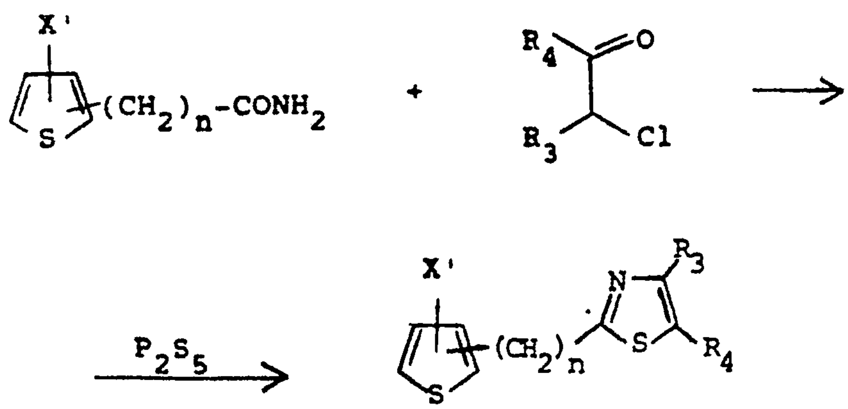


111

113

For details concerning the synthetic methods involved, see ibid. Conversion to sulfonamides of Formula 114 is accomplished using the techniques illustrated in Equation 13.

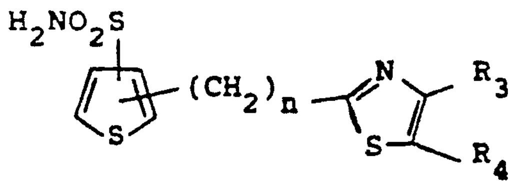

114

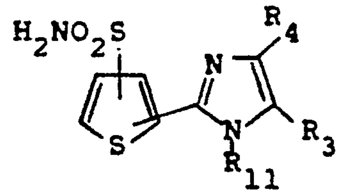

116

(1-Methylimidazol-2-yl)thiophenes of Formula 115 can be prepared by the method shown in Equation 40.

Equation 40

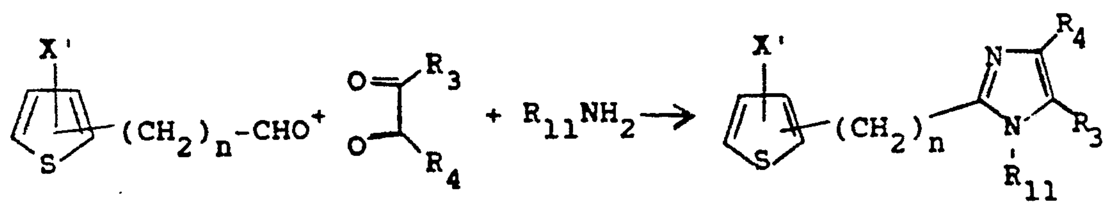

115

A condensation reaction between the thienylaldehyde, an α-diketone and an excess of an amine leads to imidazoles of Formula 115. For detailed discussion of such reactions, cf. ibid., Ch. 4, 5 and 8. Sulfonamides of Formula 116 can be prepared from 115 by means of Equation 13.

Equations 41, 42 and 43 show the synthetic approaches that can be taken to generate (5-oxazolyl)-thiophenes of Formula 117.

Equation 41

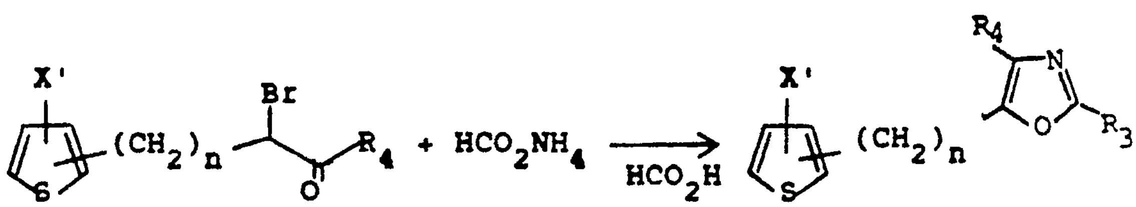


118 wherein $R_3$ is H. 117

**Equation 42**

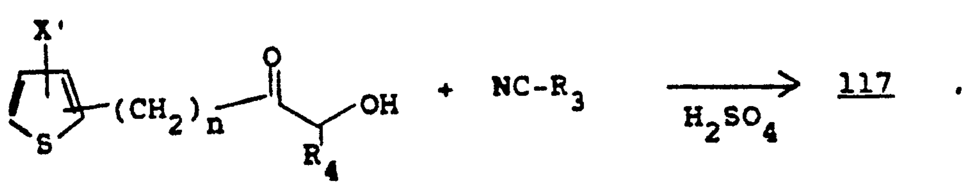


119

Equation 43

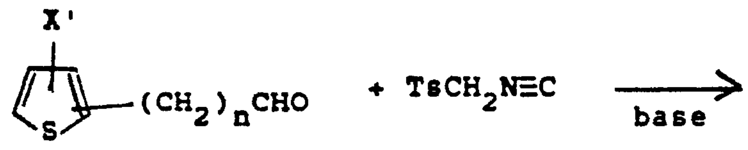


117 .

wherein

R3 and R4 are H and

Ts = 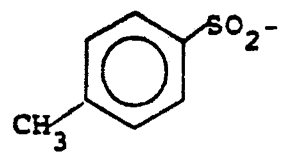

Details of the reactions depicted in Equations 39 and 40 are found in ibid., Ch. 5. The procedural details for Equation 41 are described by A. M. Van Leusen, B. E. Hogenboom and H. Serderius, Tetrahedron Lett. 1972, 2369. Compounds of Formula 118 are available by a route identical to or closely resembling the preparation of compounds of Formula 103 and subsequent bromination of the appropriate methylene moeity; details of such brominations have been recorded by S. Gronowitz, Ark. Kem. 1958, 13, 295. α-Hydroxyketones of Formula 119 in Equation 42 may be prepared from the appropriate thienylcarboxylic acid chlorides and appropriate 1,1,2,-tris[(trimethylsilyl)oxy]alkenes; cf. A. Wissner, J. E. Birnbaum and D. E. Wilson, J. Med. Chem. 1980, 23, 715. In all cases, transformation to sulfonamides of Formula 120 can be accomplished via Equation 13.

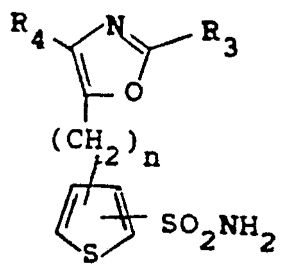

120

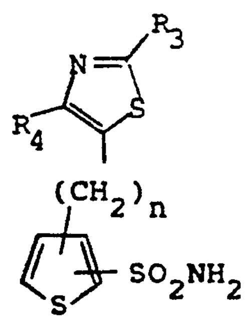

122

The analagous (5-thiazolyl)thiophenes of Formula 121 are prepared according to the process indicated in Equation 44, which is a straight-forward modification of Equation 41, above. Like synthons of Formula 117, those Formula 121 are converted to sulfonamides of Formula 122 by means of Equation 13.

Equation 44

118 + 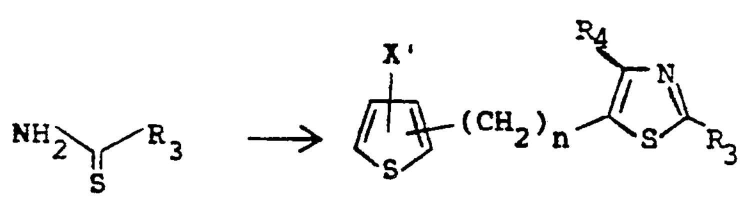

121

(5-Imidazolyl)thiophenes of Formula 123 are prepared according to Equation 45.

Equation 45

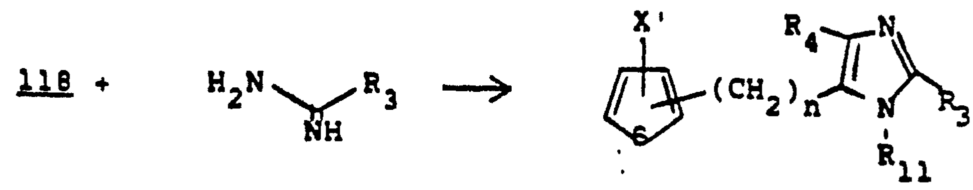

118 +

123

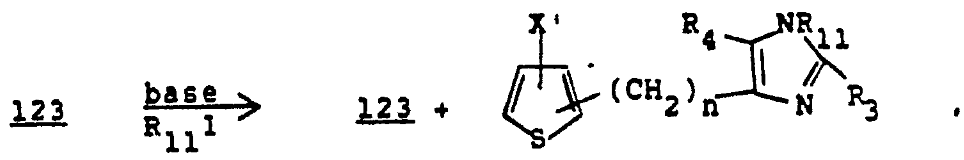


123

123 +

124

Reaction of the appropriate α-haloketone of Formula 119 with an amidine yields imidazoles of Formula 123 with $R_{11}$ = H. N-alkylation may be achieved using common procedures, evident to those skilled in the art, to give compounds of Formula 123 and 124. Sulfonamides of Formula 125 and 126 are prepared via Equation 14.

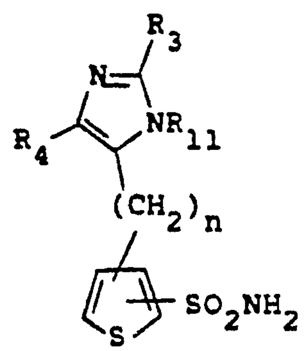

125

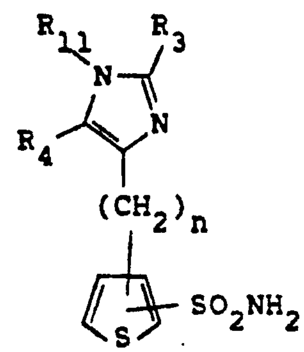

126

(4-Oxazolyl)thiophenes of Formula 127 can be prepared by procedures analogous to those described in Equations 41 or 42; substituent limitations and sulfonamide preparation to give compounds of Formula 128 via Equation 13 remain the same.

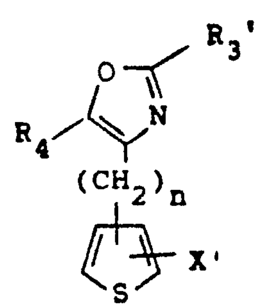

127

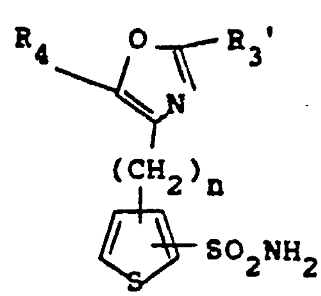

128

An alternative synthesis of the oxazoles of Formula 127 is shown in Equation 45a. It is analogous to that shown in Equation 41.

Equation 45a

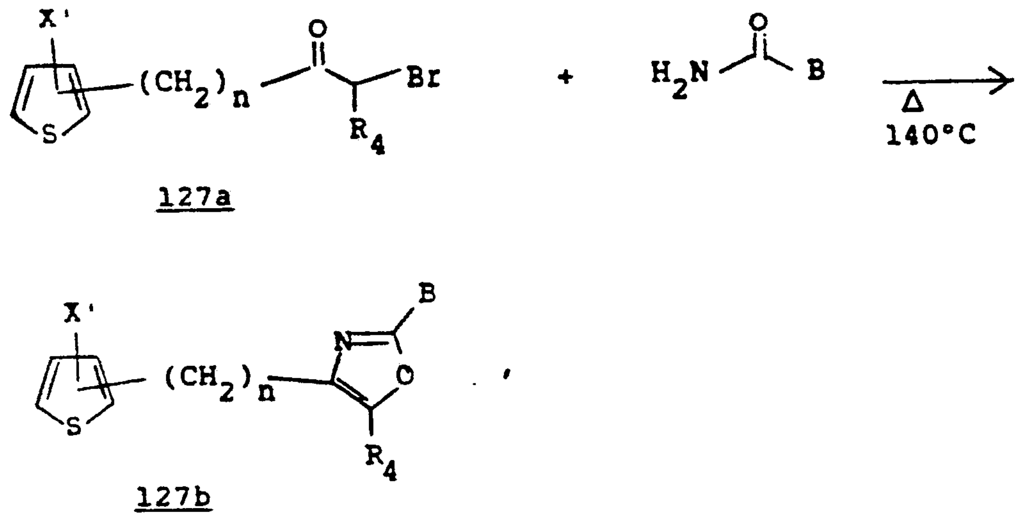


127a

127b

wherein B = $C_1$-$C_3$ alkyl, amino.

In the case where B = amino for compounds of Formula 127b, Sandmeyer-type reaction conditions afford new 2-substituted oxazoles, as shown in Equation 45b.

Equation 45b

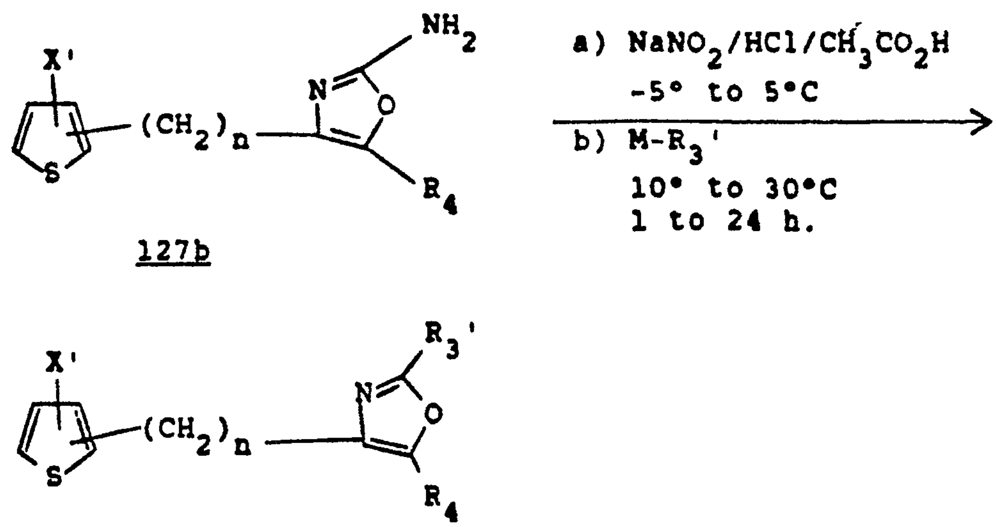


127b

127

The (4-thiazolyl)thiophenes of Formula 129 are prepared in a manner analogous to that shown in Equation 45 using the thioamide of Equation 44. These thiazoles are transformed into sulfonamides of Formula 130 using the method of Equation 7. Analogously, the procedures using Equations 45a and 45b also will give thaizoles of Formula 129.

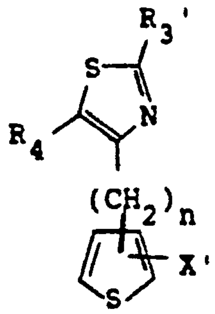

129

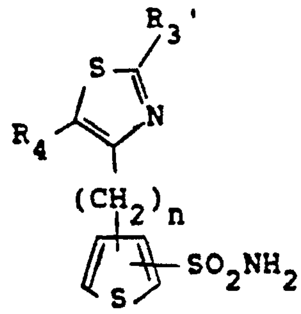

130

Compounds of Formula 131 or 132 (Q-16,W' = O) can be prepared by the procedures of the example shown in Equation 46.

Equation 46

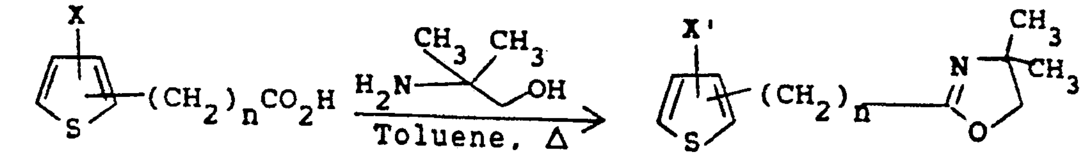


131

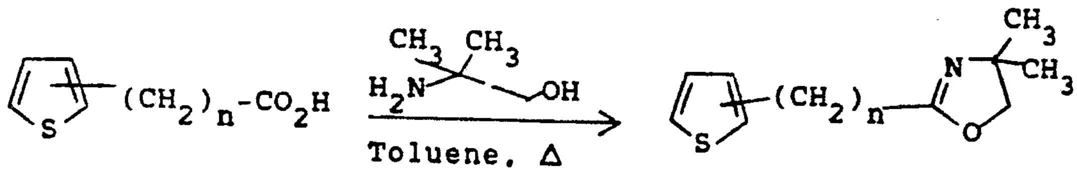


133

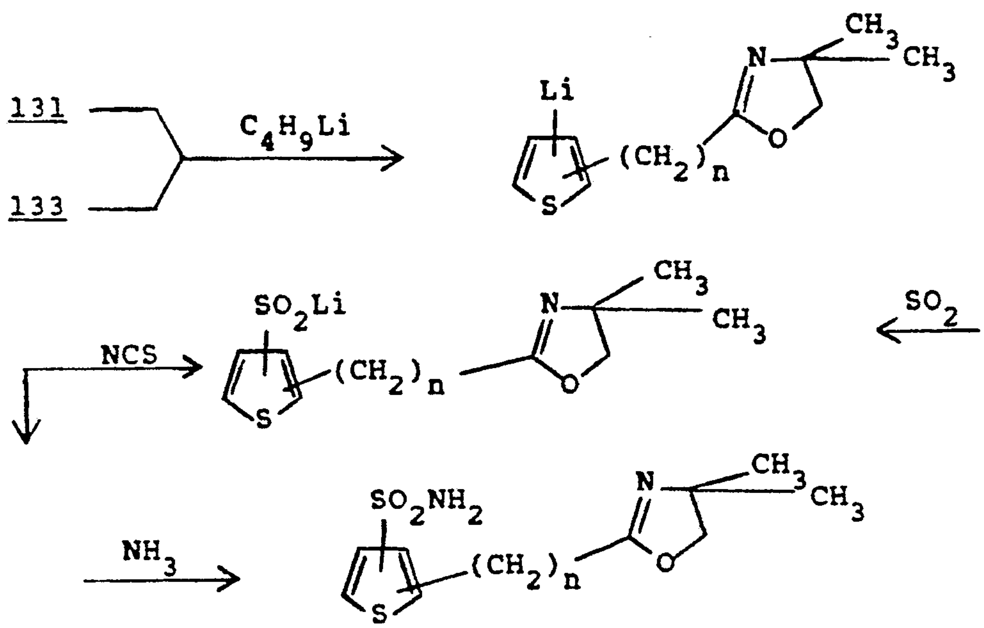


132

The formation of the 1,3-oxazolines of Equation 46 are prepared by heating the carboxylic acid and the appropriate amino alcohol in toluene to remove water by the procedure of H. L. Wehrmeister, J. Org. Chem., 1961, 26, 3821. The product oxazoline 133 or 131 can be lithiated with n-butyllithium in ether solvent. Treatment with $SO_2$ and conversion to compounds of Formula 132 is carried out by the procedures outlined in Equation 13 or as shown in Equation 46 by oxidation of the sulfinate salt with NCS (N-chlorosuccinimide) followed by amination using $NH_3$.

Synthesis of intermediate oxazolines, thiazolines and imidazolines where Q is Q-16 (W is S), Q-17 and Q-41 can be prepared by procedures similar to Equation 46 or by methods known in the art.

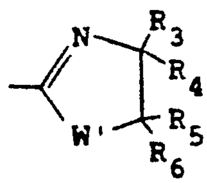

Q-16

W'=S

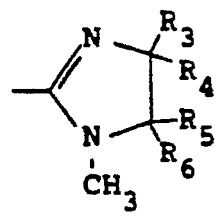

Q-17

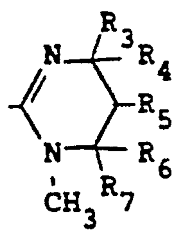

Q-41

For supplementary methods see:

R. C. Elderfield, op. cit.

R. J. Fern and J. C. Riebsomer, Chem. Rev. 1954, 54, 543.

R. H. Wiley and L. L. Bennett, Jr., Chem. Rev. 1944, 44, 447.

Compounds of Formula 134 (Q-18, n is 0) can be prepared by the procedure of S. Gronowitz and S. Liljefors, Chem. Scr. 1979, 13, 157 as shown in Equation 47.

Equation 47

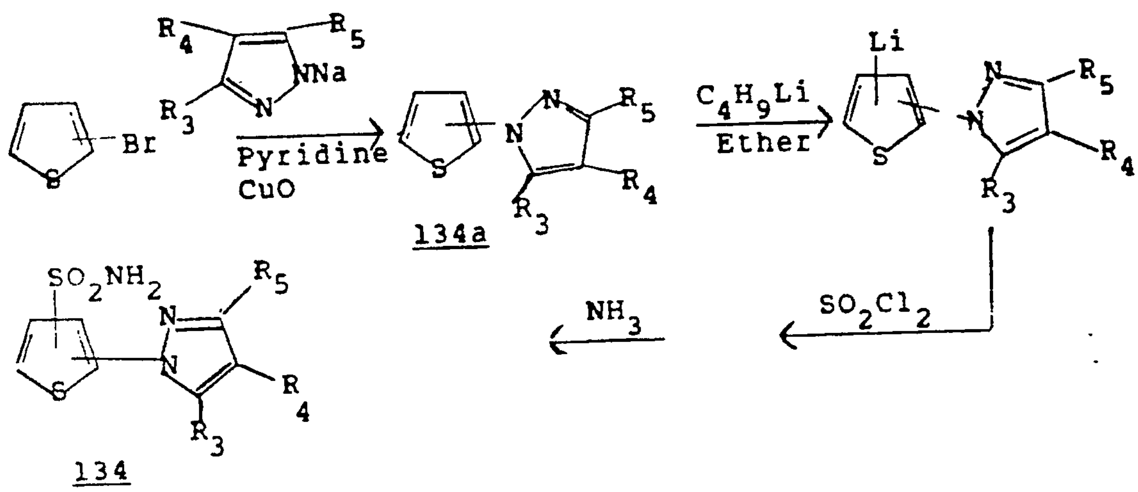


134a

134

According to Equation 47, a bromothiophene is reacted with the sodium salt of a pyrazole in pyridine containing a copper catalyst. The thienylpyrazole is lithiated and can be reacted with sulfuryl chloride by the procedures of S. N. Bhattacharya et al., J. Chem. Soc., C, 1968, 1265 to give compounds of Formula 134.

In those cases for Q-18 where n = 1 or 2, the appropriate thiophenes of Formula 134c are prepared according to Equation 47a.

Equation 47a

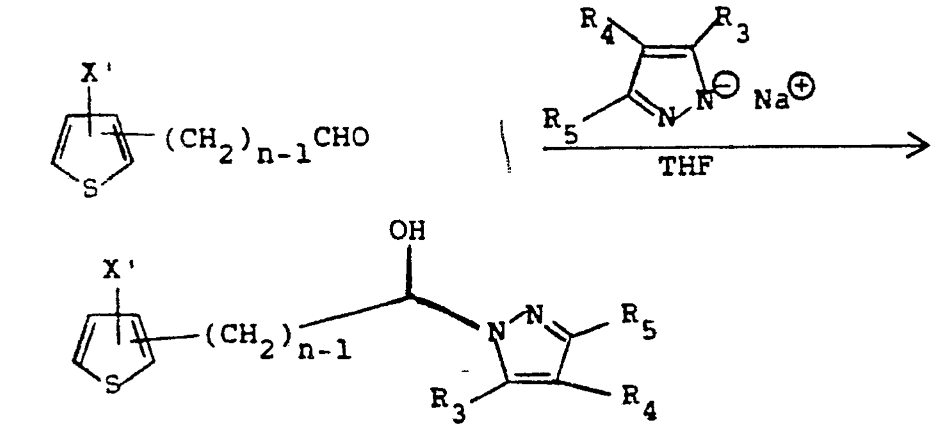


134b

134b

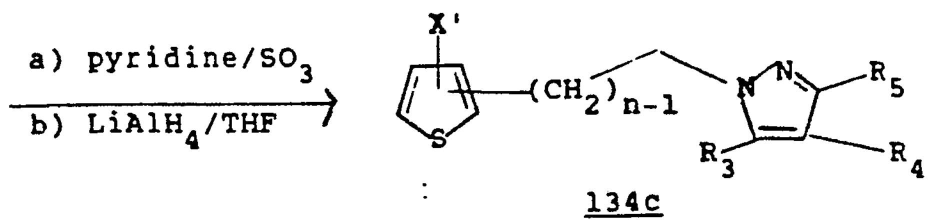


134c

Conversion to the sulfonamides of Formula 134d is accomplished in a way identical to that shown in Equation 13.

In an analogous manner, compounds of Formula 135 or 136b (Q-19) can be prepared by the procedures shown in Equations 48 and 49.

Equation 48

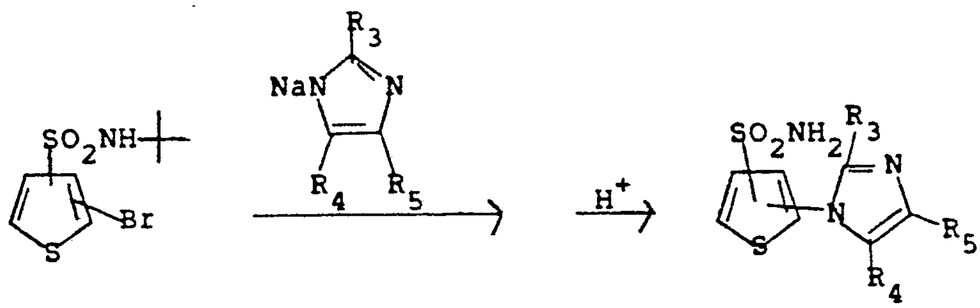


135

Equation 49

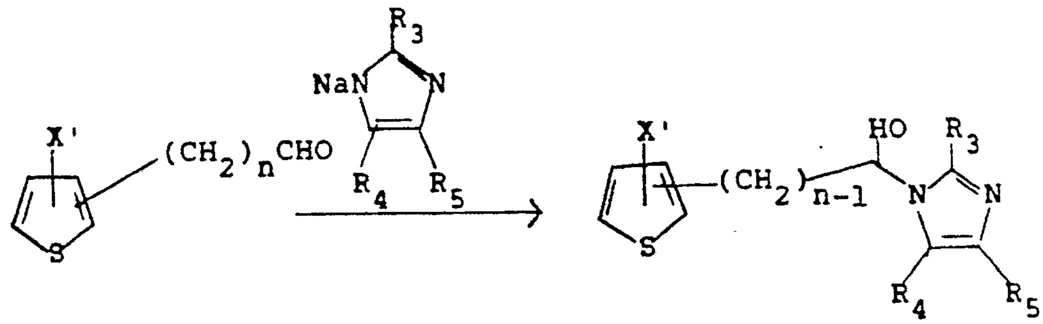

136

136

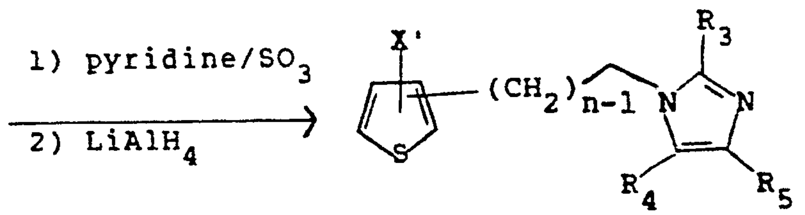


136b

The reactions are conducted in the presence of the appropriate solvent such a dimethylformamide, N-methylpyrolidone, THF or pyridine at temperatures from ambient to reflux for a period of one to twenty-four hours.

Equation 50 indicates the synthesis of thiophenes of Formula 137, bearing a Q-20 substituent.

Equation 50

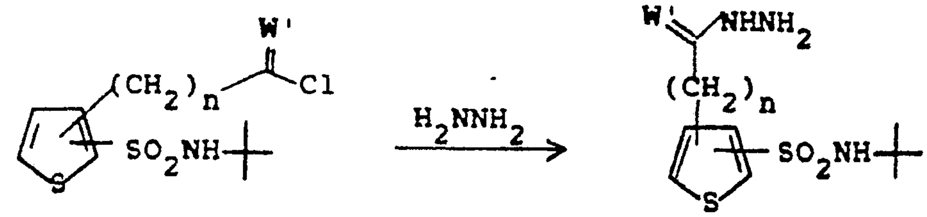


138 139

139

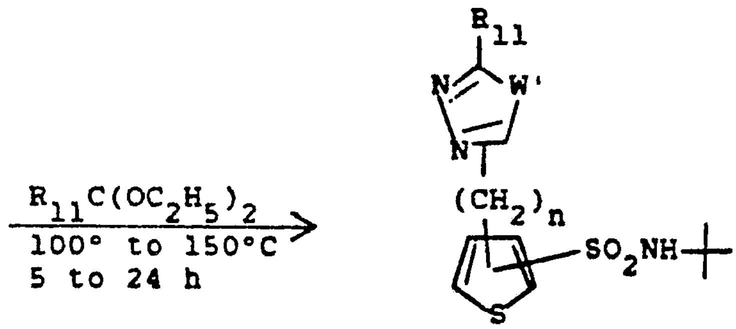


140

140

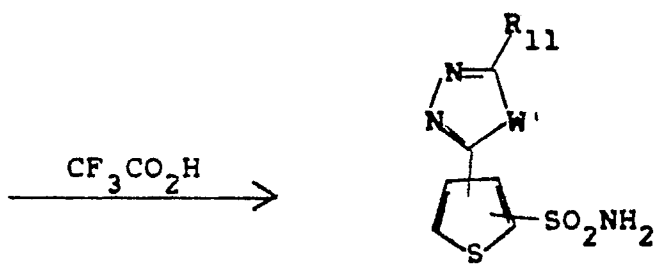


137

A chloride of Formula 138, when reacted with hydrazine, will result in a hydrazide of Formula 139. According to U.S. 3,808,223, reaction of 139 with an orthoester at 100° to 150° C for 5 to 24 hours will yield oxadiazoles of Formula 140. Deprotection of the sulfonamide moiety using a strong acid such as trifluoroacetic acid results in the desired sulfonamides of Formula 137. Synthesis of the starting thiophenes of Formula 138 can be accomplished by the methods taught in European Patent Application 30,142.

Equation 50a illustrates the preparation of thiophenes of Formula 137a where $R_3$ is $SR_{12}$.

Equation 50a

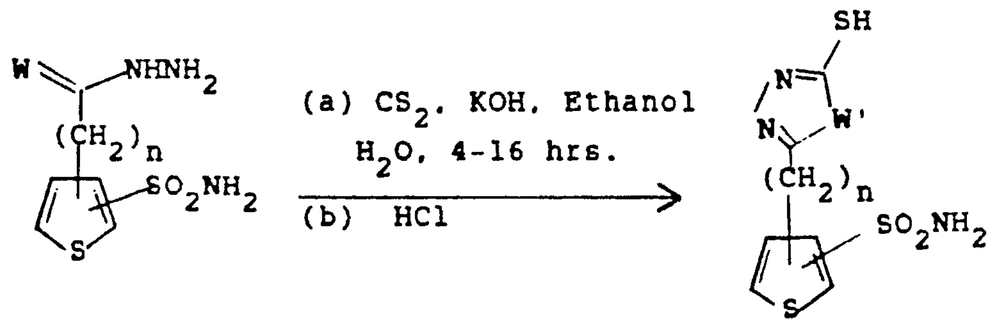


139a

140a

140a

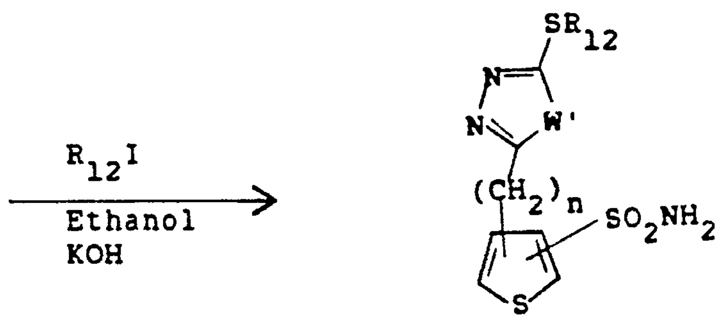


137a

wherein $R_{12}$ = $C_1$-$C_4$ alkyl, $C_3$-$C_4$ alkenyl, $C_3$-$C_4$ alkynyl, $C_1$-$C_3$ cyanoalkyl, methoxycarbonylmethyl, ethoxycarbonylmethyl, methylcarbonylmethyl, or halogen.

The reactions of Equation 50a are run according to similar procedures described in E. Hoggarth, J. Chem. Soc. 4811 (1952). Thus, the hydrazide is reacted with carbon disulfide in ethanol in the presence of a base, such as potassium hydroxide at reflux for 4-16 hours, followed by addition of water and acidification with hydrochloric acid to form compound 140a. Further reaction of compound 140a with $R_{12}I$ in ethanol in the presence of base gives compound 137a.

Compounds of Formula 141 (Q-21,W' = O) are prepared by the procedure of P. DuBus, B. Decroix, J. Moreland and P. Pastour, Ann. Chim. (Paris) 1975, S14 t10, p 331 as shown in Equation 51.

Equation 51

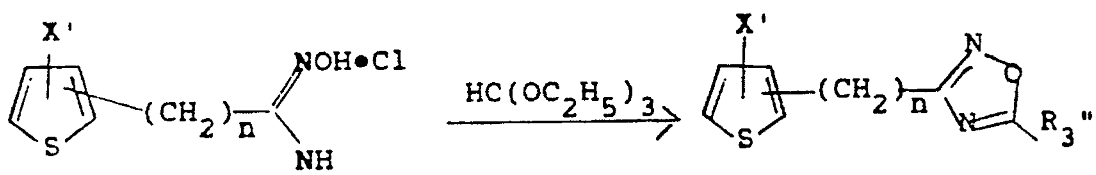


142

141

Compound 142 is reacted with excess triethylorthoformate at 100° to 150° C for 1 to 24 hours to afford 141 ($R_3''$ = H). $R_3''$ may be optionally substituted with Cl or Br by selective deprotonation and quenching with NCS or NBS, respectively.

For $R_3''CH_3$, Compound 142 is reacted with acetylchloride and $BF_3 \cdot Et_2O$ according to U.S. 3,270,029.

Analogously, 1,3,4-thiadiazol-5-ylthiophenes of Formula 143 may be prepared following the techniques described by C. Ainsworth and R. G. Jones, J. Am. Chem. Soc. 1955, 77, 621 and H. Weidinger and J. Kranz, Ber. 1963, 96 1059.

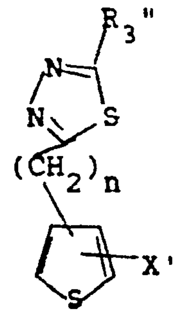

143

Equation 52 illustrates the preparation of 1,2,4-thiadiazol-3-ylthiophenes of Formula 144.

Equation 52

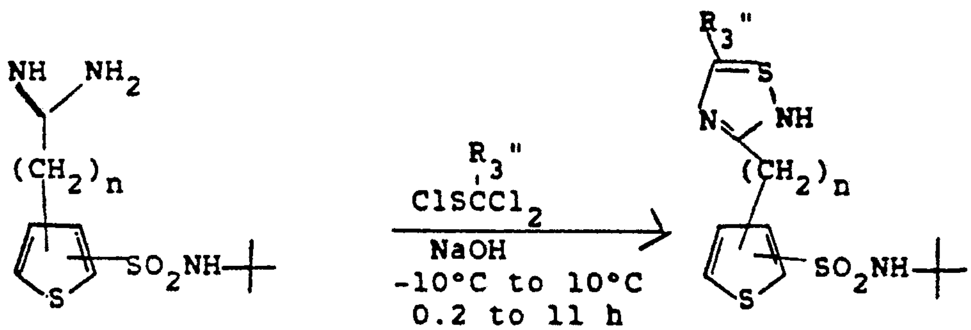


145 146

146

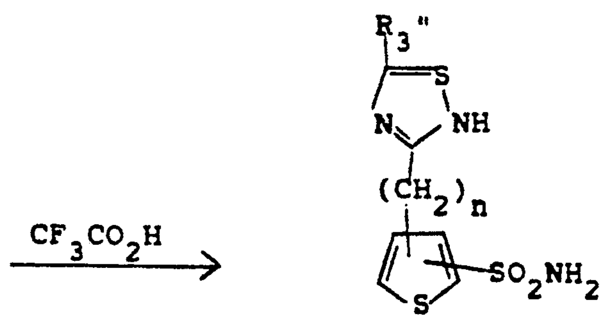


144

The reactions of Equation 52 may be run following similar methods found in J. Goerdeler and M. Budnowski, M. Chem. Ber. 1961, 94, 1682. Thus, an amidine of Formula 145, obtained via the corresponding carboxylate is treated with dichloromethylsulfenyl chloride and sodium hydroxide in a solvent such as aqueous 1,4-dioxane at temperatures of about 0° C for 0.2 to 11 hours to form thiophene of Formula 146. Deprotection with a strong acid affords sulfonamide of Formula 144.

Y. Lin, S. A. Land, M. F. Lovell and N. A. Perkinson, J. Org. Chem., 1979, 44, 4160 indicate methods for the formation of 1,2,4-oxadiazol-5-yl-thiophenes of Formula 148, as shown in Equation 53.

Equation 53

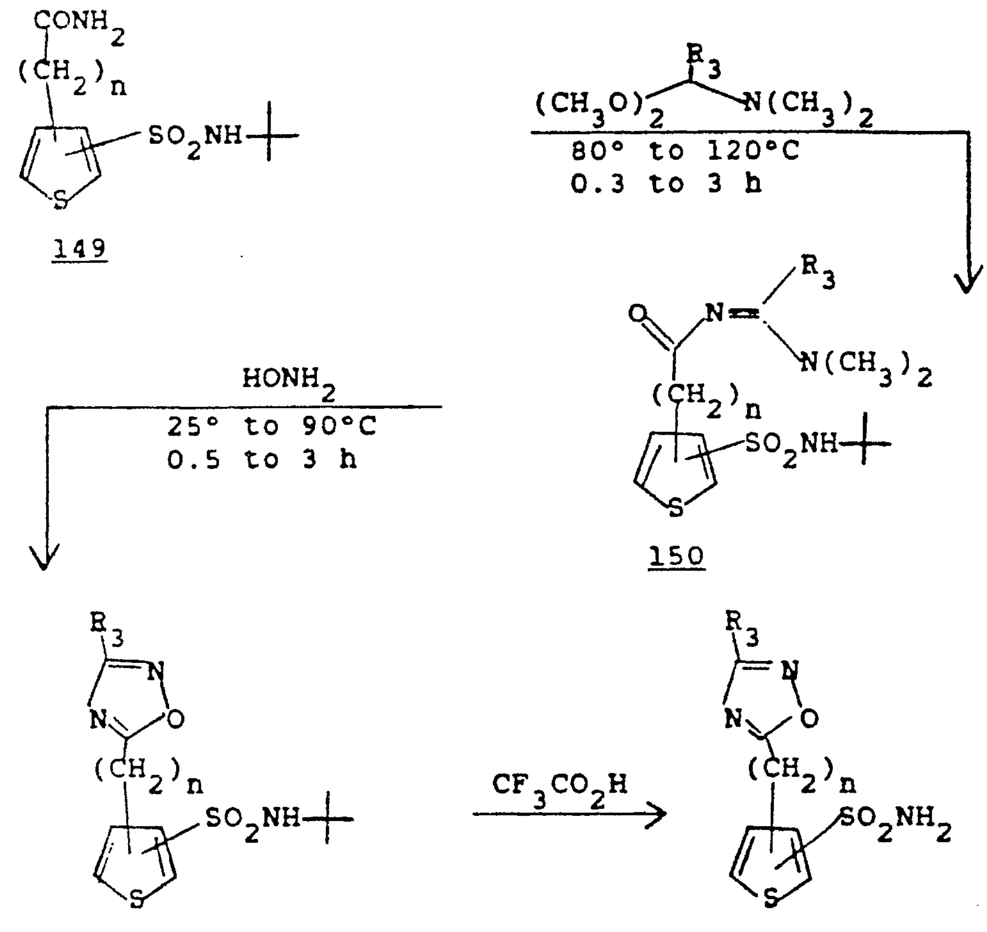


149 150 151 148

An amide of Formula 149, available by amination of the corresponding carboxylate is reacted with an excess of an alkanamide dimethylacetal at 80° to 120°C for 0.3 to 3 hours, forming compounds of Formula 150. These compounds can be dissolved in aqueous 1,4-dioxane-acetic acid and reacted with hydroxylamine at temperatures ranging between 25° to 90°C over 0.5 to 3 hours to form oxadiazoles of Formula 151, which are subsequently (or perhaps concomitantly) deprotected using a strong acid to give sulfonamides of Formula 148.

In a strictly analogous manner, using conditions similar to those described by Equation 53, 1,2,4-thiadiazol-5-ylthiophenes of Formula 152 can be prepared; cf. Y. Lin and S. A. Lang, J. Org. Chem. 1980, 45, 3750.

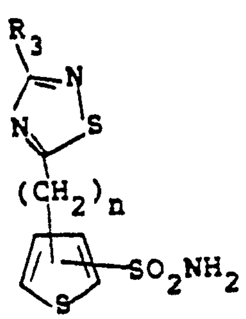

152

The 1,2,4-thiadiazol-4-ylthiophenes of Formula 153 can be prepared by the teachings of U.S. 3,940,407 and C. D. Hund and R. I. Mori. J. Am. Chem. Soc. 1955, 77, 5359. Equation 54 illustrates this process.

Equation 54

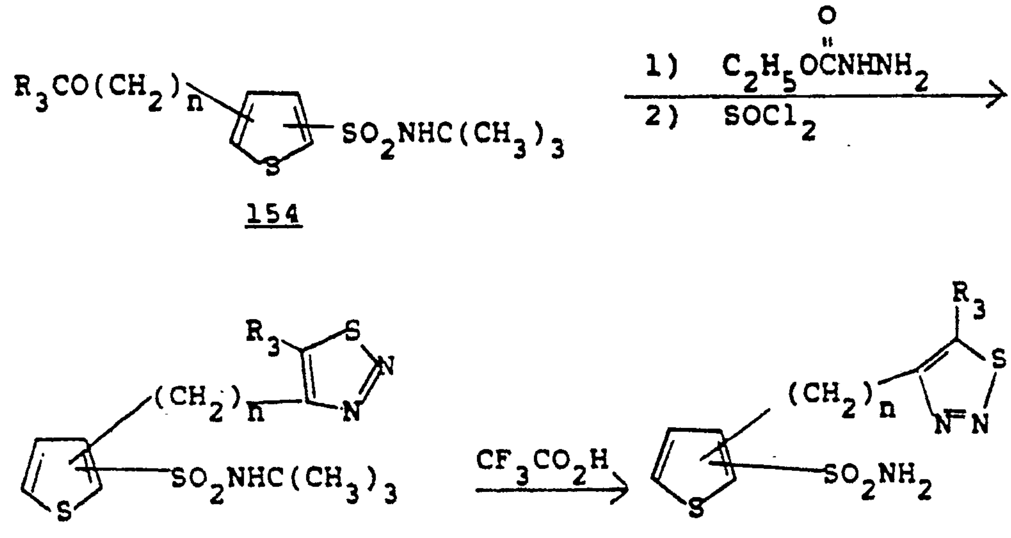


154

153

155

Ketones of Formula 154 react with ethyl carbazate to form the corresponding hydrazide; subsequent reaction with thionyl chloride results in compounds of Formula 155. Deprotection of these compounds using standard conditions leads to sulfonamides of Formula 153.

1,2,5-Thiadiozol-3-ylthiophenes of Formula 156 can be prepared from thiophenes of Formula 31 by several known methods, such as those of L. M. Weinstock, P. Davis, B. Handelsman and R. Tull, J. Org. Chem. 1967, 32, 2823; V. Bertini and P. Pino, Angew Chem. Int. Ed. Engl. 1966, 5, 514 and S. Mataka, A. Hosoki, K. Takahashi and M. Tashino, Synthesis 1979, 524.

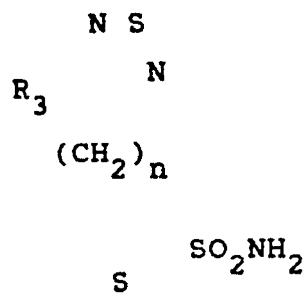

156

The methyl-1,3,4-triazol-2-ylthiophenes of Formula 157 can be prepared by the method shown in Equation 55.

Equation 55

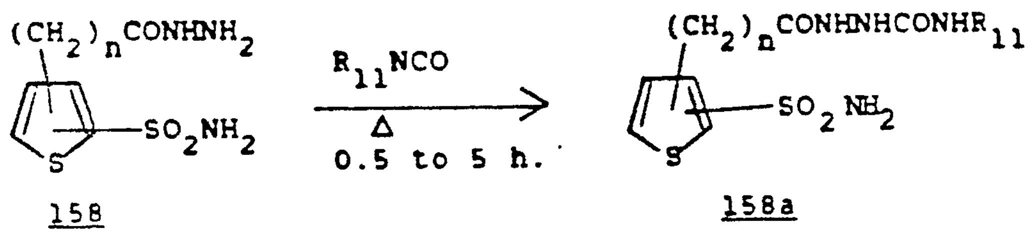


158 158a

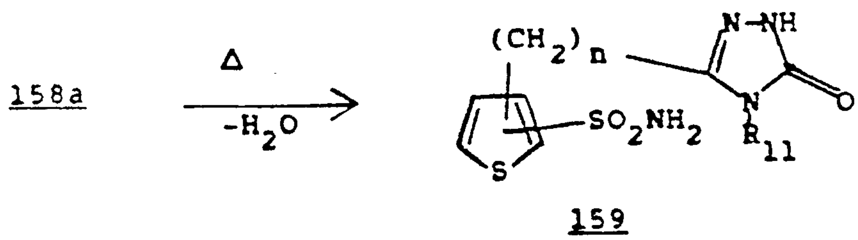


158a 159

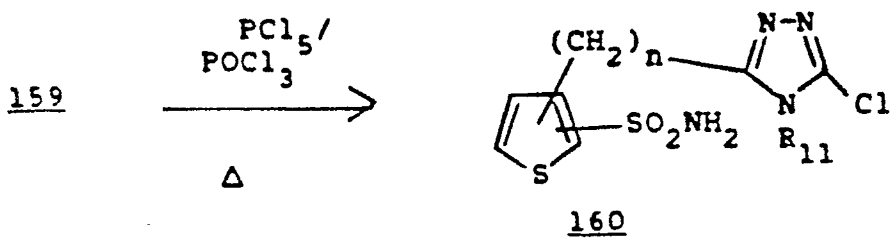


159 160

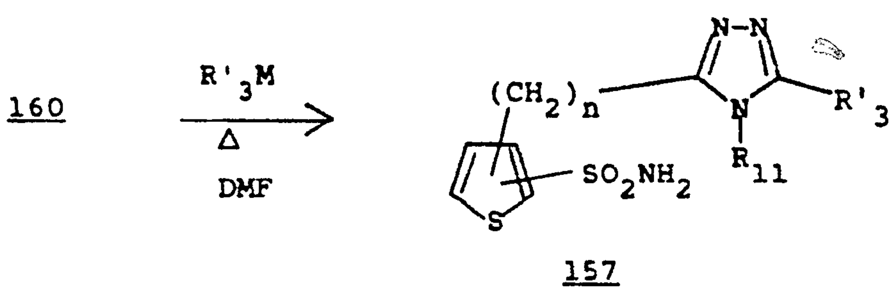


160 157

N-aminoamides of Formula 158 will react with isocyanates in solvents such as acetone or 2-butanone at reflux to afford adducts of Formula 158a. These in turn upon heating will cyclize with concomittant extrusion of water, yielding triazolones of Formula 159. Treatment with phosphorous pentachloride and phosphorous oxychloride at reflux gives 2-chlorotriazoles of Formula 160, which, upon treatment with a nuleophile $R_3$'M in a polar, aprotic solvent such as DMF, yields the substituted sulfonamide of Formula 157. Compounds of Formula 158 in turn are synthesized from the treatment of esters of Formula 161 with hydrazines.

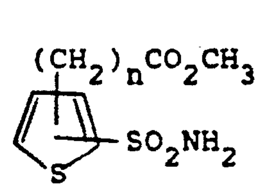

161

Methyl-1,2,4-triazol-5-ylthiophenes of Formula 162 can be synthesized by the route shown in Equation 56.

Equation 56

150

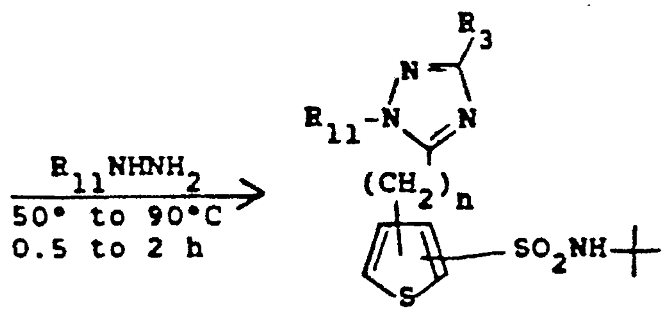


163

163

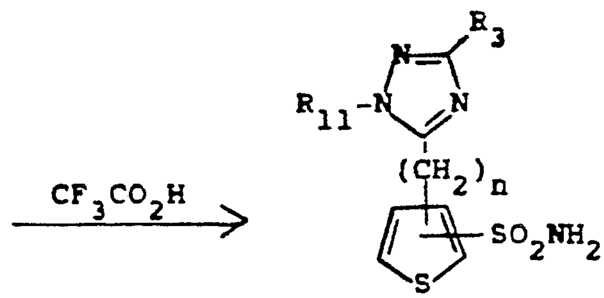


162

Y. Lin, S. A. Lang, M. F. Lovell and N. A. Perkinson, J. Org. Chem. 1979, 44, 460 have shown that reaction of a compound of Formula 150 with alkylhydrazines in acetic acid at temperatures of 50° to 90°C for 0.5 to 2 hours will give triazoles of Formula 163. Deprotection to form sulfonamides of Formula 162 may be accomplished during cyclization, or may be removed afterwards by use of a strong acid.

Additionally, compounds of Formula 162 may be prepared in a manner analogous to that of Equation 55, using the appropriate cyanate or thiocyanate to give the required intermediate compounds of Formula 158b.

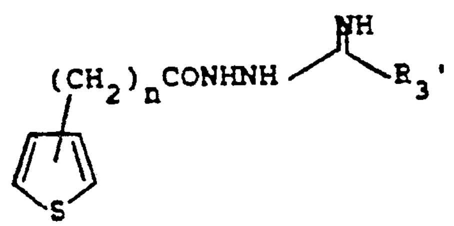

162

Methyl-1,2,4-triazol-3-ylthiophenes of Formula 164 where $R_3' = R_3$ are produced by the techniques illustrated in Equation 57 and similar to those described by M. Atkinson and J. Polya, J. Chem. Soc. 1954, 3319.

Equation 57

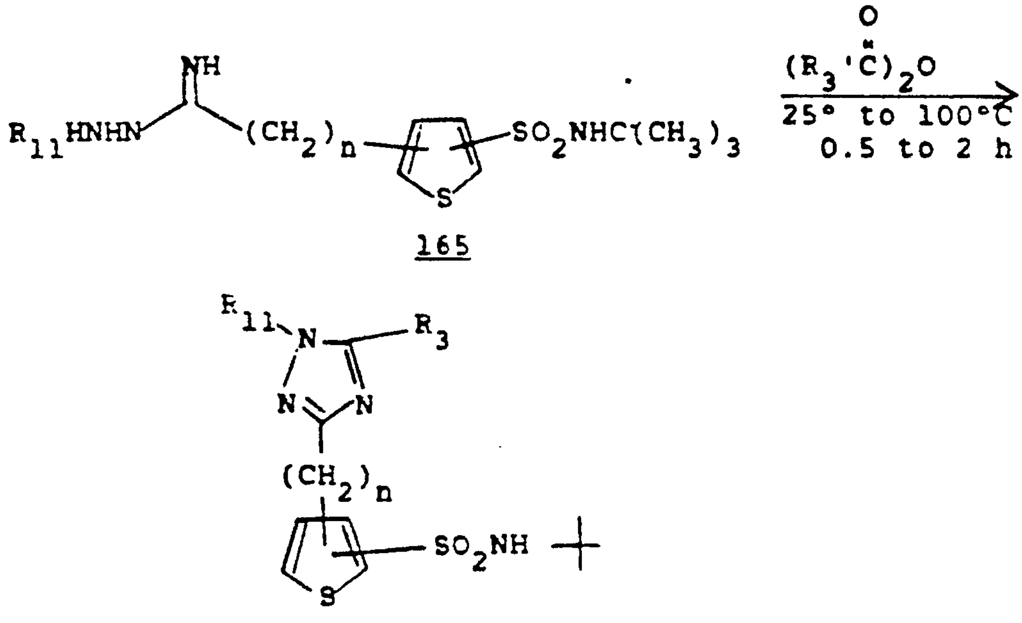


165

166

166

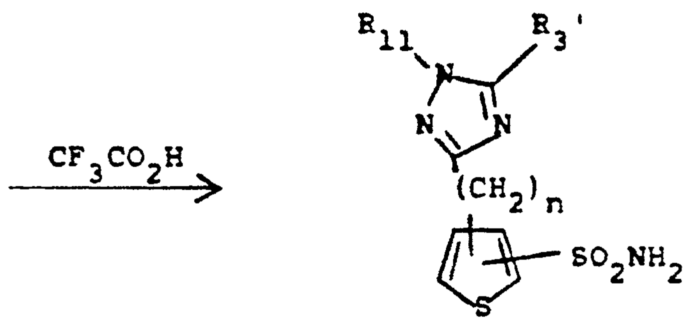


164

The hydrazine of Formula 165 reacts with an anhydride at temperatures ranging from 25° to 100°C for approximately 0.5 to 2 hours, forming triazoles of Formula 166. Standard deprotection affords sulfonamides of Formula 164. Hydrazine 165 may be prepared by reaction of nitrile 161 with alkylhydrazines.

Similar treatment of hydrazine of Formula 165 with diphenylcarbonate or phosgene will result in triazolones of Formula 166a, as indicated in Equation 57a. Conversion to sulfonamides of Formula 164b is performed in a manner analogous to that of Equation 57.

Equation 57a

165

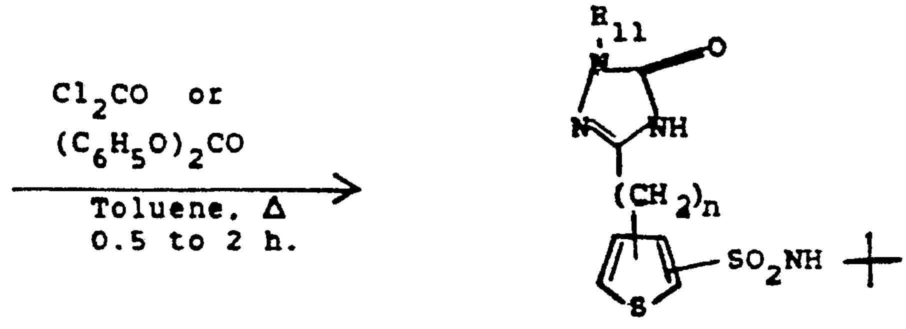


166a

1,2,4-Triazolylthiophenes of Formula 167 can be synthesized by the reaction sequence of Equation 58.

Equation 58

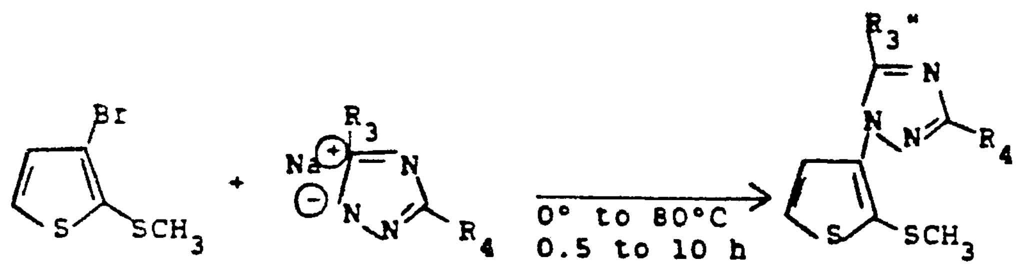


168 169 170

170

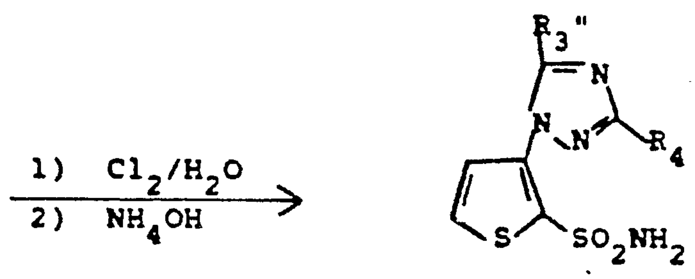


167

Similar to the preparation of thiophenes of Formula 134a, triazoles of Formula 170 can be formed by reaction of the bromothiophene of Formula 168 with a sodium 1,2,4-triazole salt of Formula 169. 170 then can be subjected to oxidative chlorination; amination of the intermediary sulfonyl chloride will result in sulfonamides of Formula 167.

Alternatively, sulfonamides of Formula 167 where $R_4$ = H can be prepared by the route illustrated in Equation 58a.

Equation 58a

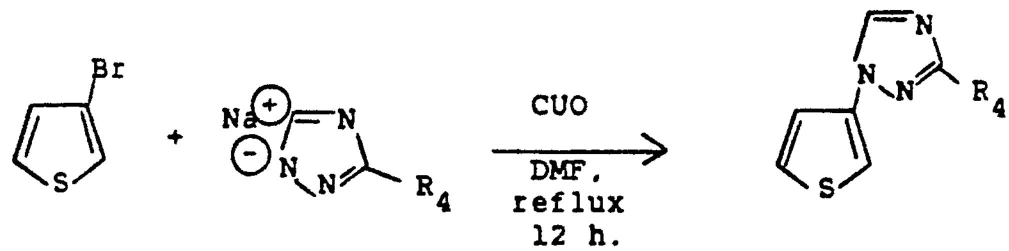


169

170a

170a

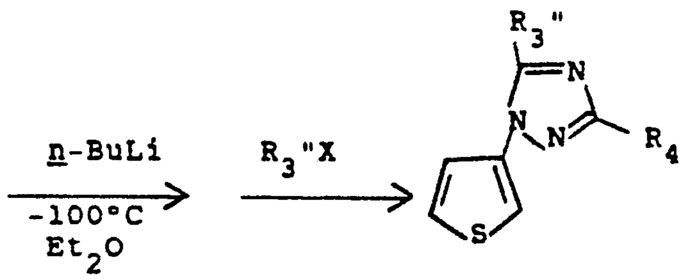


170b

170b

1) n-BuLi, -78°C

2) $SO_2Cl_2$

$NH_3$

THF

167

3-Bromothiophene is coupled with the sodium salt of a 1,2,4-triazole of Formula 169 in refluxing DMF using copper(II) catalysis. The resultant biaryl of Formula 170a then is regioselectively lithiated at -100° C using n-butyllithium in diethyl ether; quenching with an eletrophile, $R_3$"X, results in biaryls of Formula 170b. Sulfonamidation of 170b in a manner analogous to that described in Equation 12 gives the desired 167.

Those cases where n is 1 or 2, the sodio salt of the appropriate triazole may be added to aldehydes of Formula 170c, which can be converted by means of Equation 58b to sulfonamides of Formula 167a.

Equation 58b

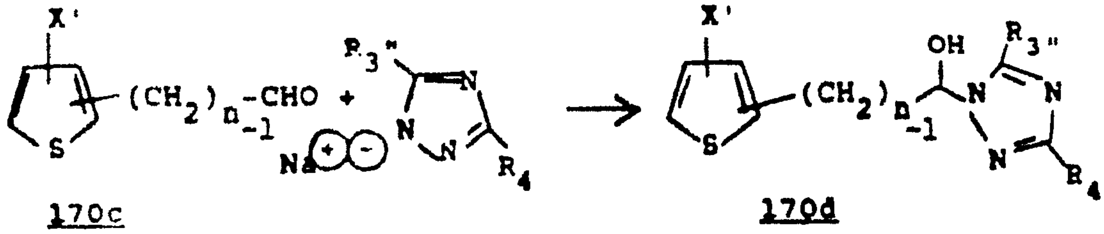

170c 170d

170d

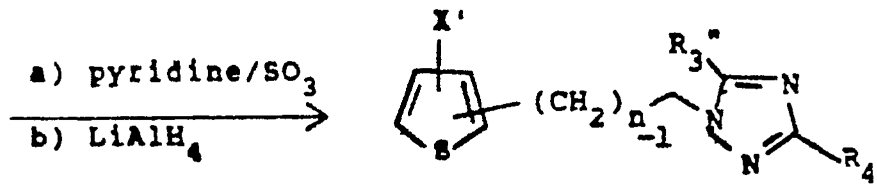


170e

170e

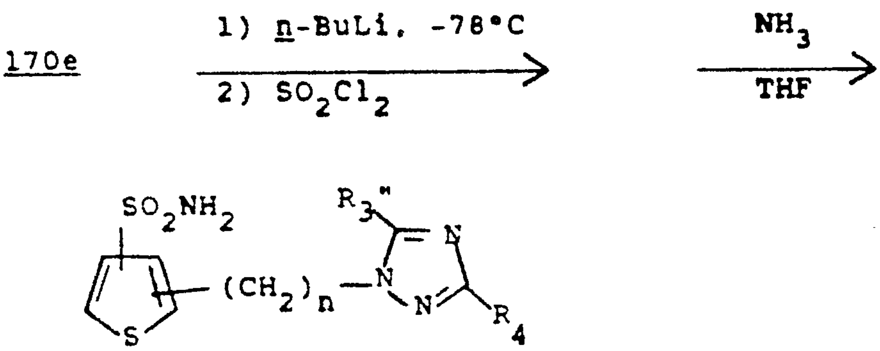


167a

Compounds of Formula 171 can be prepared by reacting a 1-formyl-2-(3'-bromothienyl)hydrazine of Formula 172 with excess formamide at reflux for about 1 to 6 hours, according to the procedures described by C. Ainsworth et al., J. Med. Pharm. Chem. 1962, 5, 353 as shown in Equation 59 below.

Equation 59

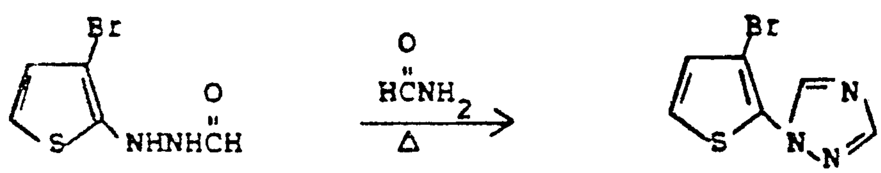


171

Alternatively, compounds of Formula 171 can be prepared from 2,3-dibromothiophene by reaction with the sodium salt of a 1,2,4-triazole of Formula 169 as shown in Equation 60.

Equation 60

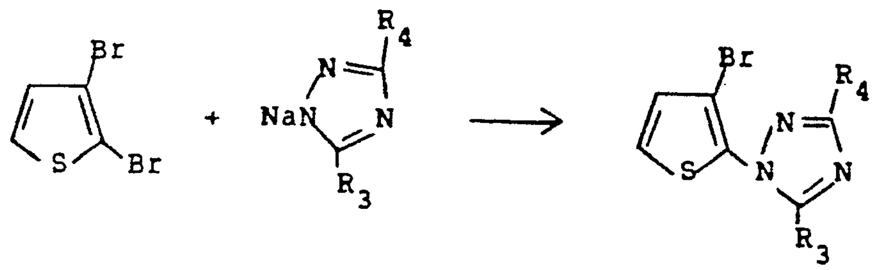

169 171a

The reaction can be run by the procedures described in Equations 47, 48, and 49.

Compounds of Formula 171 may also be prepared by a modified Ullmann reaction, according to the teachings of M. Kahn and J. Polya. J. Chem. Soc., C. 1970, 85.

Compounds of Formula 167a (Q-29) can be prepared by the procedures shown in Equation 61.

Equation 61

57 $\xrightarrow{2\underline{n}\text{-}C_4H_9Li}$ 58 $\xrightarrow[2)\ NaBH_4]{1)\ TsN_3}$ 59

59

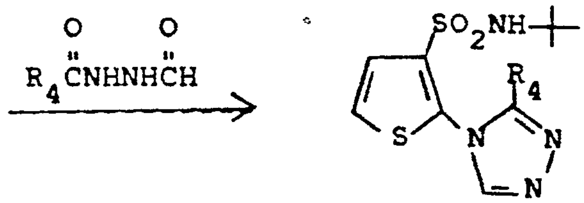


167a

167a

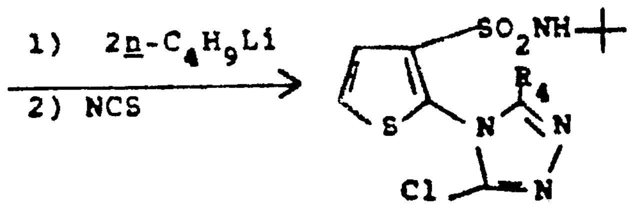


167c

167c

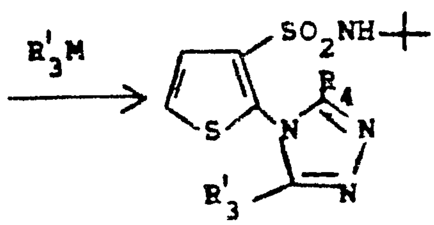


167d

The 2-amino-3-thiophene-t-butylsulfonamide 59, prepared by the procedures of Equation 23, is treated with N,N'-diacylhydrazine at 150° to 200° C for 0.5 to 2 hours, according to methods known in the art, e.g.,

C. Ainsworth et al., op. cit. 1,3,4-Triazol-3-ylthiophenes of Formula 167b are formed as shown in Equation 62. An aminothiophene of Formula 172 will react with diacylhydrozines at high temperatures (150° to 200°C) over a period of ca. 0.5 to 2 hours to result in triazoles of Formula 173. Conversion of these triazoles to sulfonamides of Formula 167b can be accomplished as shown in Equations 58 and 61.

Equation 62

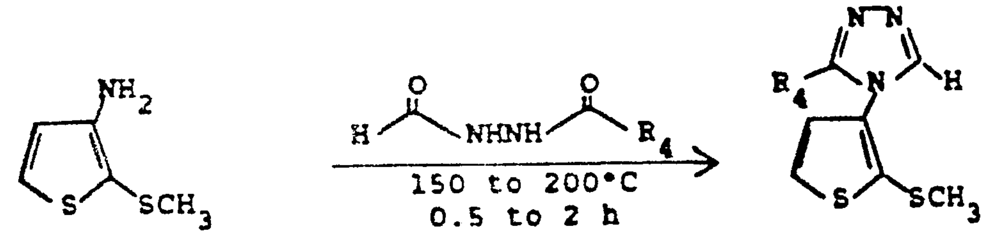


172 173

173 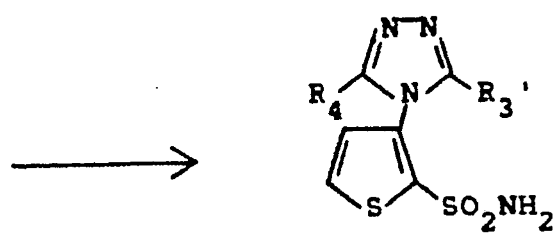

167b

In those cases where n is 1 or 2, a procedure entirely analogous to that of Equation 58b may be used to prepare sulfonamides of Formula 167e.

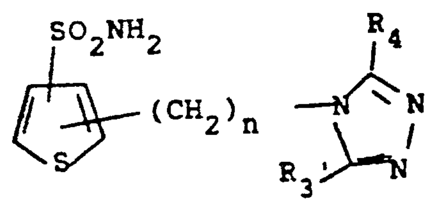

167c

3-Pyridinylthiophenes of Formulae 174, 175 and 176 are available by the methods of H. Wynberg, T. J. Van Bergen and R. M. Kellogg, J. Org. Chem. 1969, 34, 3175. In the case of 177, sulfonamides of this Formula can be prepared by route of Equation 7; for sulfonamides of Formulas 178 and 179, preparation will be better achieved by way of Equation 14.

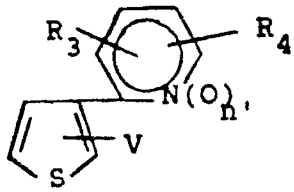

174. V=H
177. $V=SO_2NH_2$

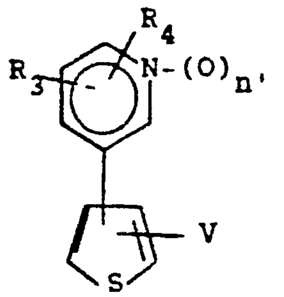

175. V=H
178. $V=SO_2NH_2$

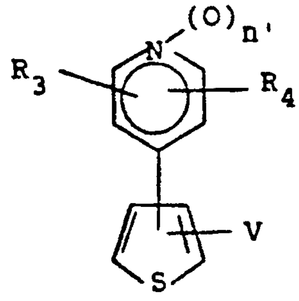

176. V=H
179. $V=SO_2NH_2$

Pyridinylthiophenesulfonamides where n' = 1 (pyridine N-oxides) can be prepared by treatment of the corresponding pyridinylthiophenesulfonamides with m-chloroperbenzoic acid in chloroform. The resulting N-oxide readily precipitates from solution.

In those cases where n is 1 or 2, a synthetic sequence similar to that depicted in Equation 58b will lead to the desired sulfonamides of Formulae 177-179.

3-Pyrimidin-2-ylthiophenes of Formula 180 are prepared according to the techniques described by S. Gronowitz and S. Liljefors, Acta Chem. Scand. 1977, B 31, 771 and straightforward modifications thereof. Sulfonamides of Formula 181 are formed by means of Equation 14.

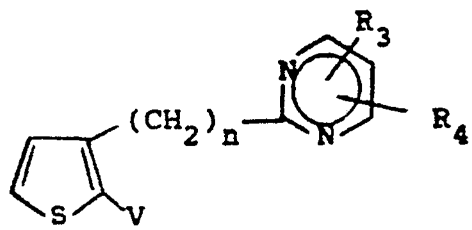

180. V=H

181. $V=SO_2NH_2$

3-Pyrimidin-4-ylthiophenes of Formula 182 are synthesized according to R. E. van der Stoel and H. C. van der Plas, J. Chem. Soc., Perkin I 1979, 2393. Conversion to sulfonamides of Formula 183 can be made by the synthetic sequence of Equation 14.

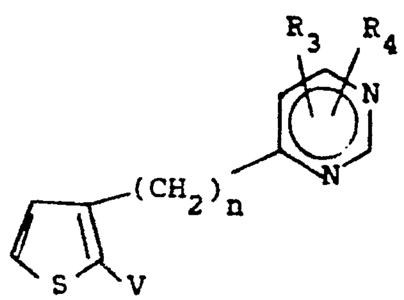

182. V=H

183. $V=SO_2NH_2$

3-Pyrimidin-5-ylthiophenes of Formula 184 are available by use of the synthesis described by S. Gronowitz and S. Liljefors, Chem. Scr. 1978, 13, 39 and J. Org. Chem. 1982, 47, 3177 and modifications thereof. Again, the preparation of the corresponding sulfonamides of Formula 185 can be accomplished by means of Equation 14.

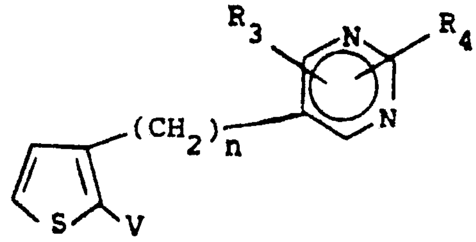

184. V=H
185. V=$SO_2NH_2$

3-Pyrazinylthiophenes of Formula 186 are prepared by the method of J. Bourguignon, J.-M. Bouchy, J.-C. Clinet and G. Queguiner, C. R. Acad. Sci. Paris C 1975, 281, 1019. The synthesis of the sulfonamides of Formula 187 can be achieved by use of the route described in Equation 14.

3-Pyridazin-3-ylthiophenes of Formula 188 are available by the method described by J. Bourguignon, C. Becue and G. Queguiner, J. Chem. Res., Synop. 1981, 4, 104. Conversion to the sulfonamides of Formula 189 is accomplished by means of Equation 14.

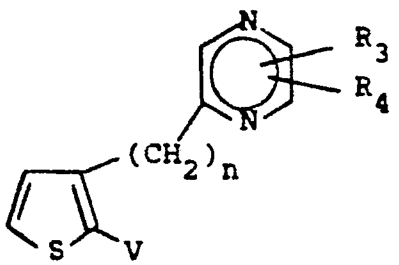

186. V=H
187. V=$SO_2NH_2$

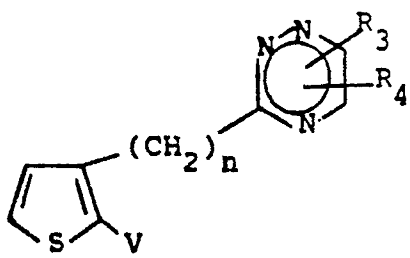

188. V=H
189. V=$SO_2NH_2$

3-(1,3,5-triazin-2-yl)thiophenes of Formula 190 can be prepared by the methods of J. K. Chakrabarti, R. W. Goulding and A. Todd, J. Chem. Soc., Perkin I 1973, 2499. The appropriate 1,3,5-triazines needed for the synthesis are described by Smolin, E. M. and Rapoport, L. "The Chemistry of Heterocyclic Compounds," Vol 13, Wily-Interscience, NY. The corresponding sulfonamides of Formula 191 are formed by application of the chemistry described in Equation 14.

3-(1,2,4-triazin-3-yl)thiophenes of Formula 192 are available by a route essentially similar to the preparation of thiophenes of Formula 190, using the appropriate 1,2,4-triazines as made by the methods of A. Rybowski and H. C. van der Plas, J. Org. Chem. 1980, 45, 881. Again, the sulfonamides of Formula 193 are available by use of the synthetic procedure described in Equation 14.

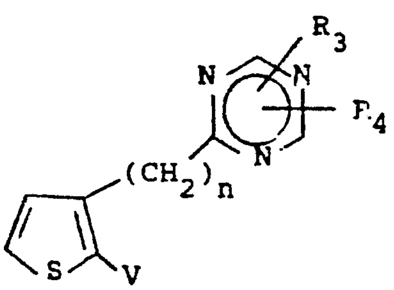

190. V=H
191. V=$SO_2NH_2$

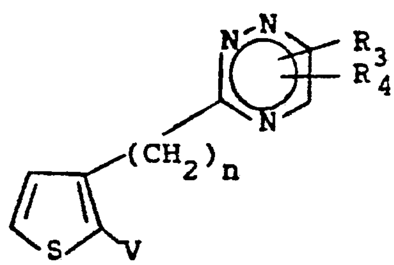

192. V=H
193. V=$SO_2NH_2$

3-(1,2,3-Thiadiazol-5-yl)thiophenes of Formula 195 can be prepared by a straightforward modification of the synthesis of 153 described by Equation 54, beginning with thiophenes of Formula 196. These

thiophenes in turn are available from the corresponding ketones.

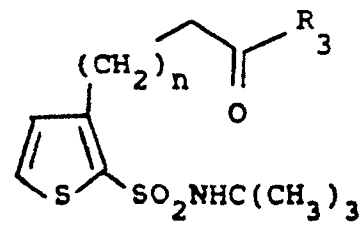

196

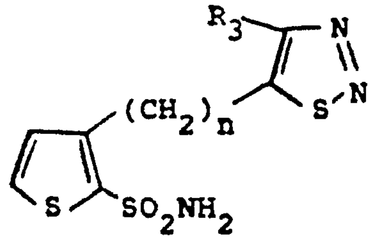

195

Compounds of Formula 2 where Q is Q-33, Q-34, Q-35, Q-37, Q-38, Q-39, Q-40, Q-43 and Q-44 can be prepared by the sequence of reactions outlined in Equations 8 and 9.

Pyrimidines and triazines of Formula 5 are necessary intermediates for the preparation of compounds of Formulas Ia, Ib and Ic of this invention. Such pyrimidines and triazines are either known compositions or may be prepared by methods obvious to those skilled in the art.

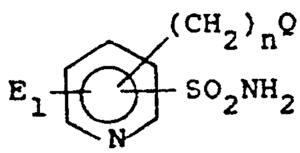

197a

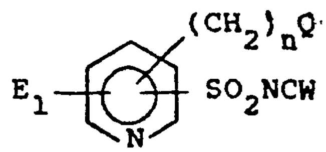

197b

2- and 3-Pyridinesulfonamides of Formula 197a above are important intermediates for the preparation of the compounds of this invention, and can be prepared by methods known in the literature, or simple modifications thereof, by those skilled in the art.

For example, 3-pyridinesulfonamides of Formula 197a, substituted in the 2-position with a pyrazol-1-yl, 1,2,4-triazol-1-yl or imidazol-1-yl group (Q is Q-52, Q-61 or Q-66) may be prepared by the sequence of reactions shown in Equation 63 below.

Equation 63

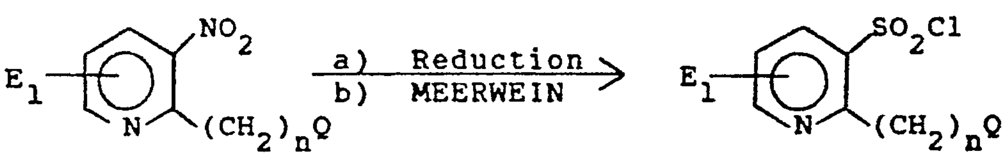


198

200

200

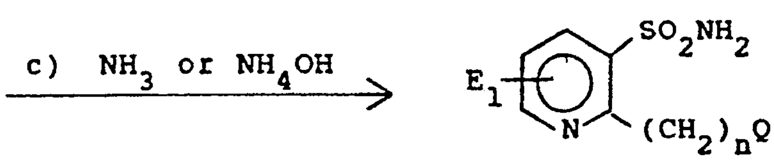


197c

wherein

- $E_1$ is as originally defined;
- Q is Q-52, Q-61 or Q-66; and
- n is 0, 1 or 2.

Reaction 63(a)

In this reaction 3-nitropyridines, 198, are reduced to corresponding 3-aminopyridines, for example, by reaction with stannous chloride in hydrochloric acid by conventional methods. Details and references for this and other methods for reducing nitropyridines to corresponding aminopyridines can be found in "Pyridine and Derivatives", Chapter IX, pp. 8-10, E. Klingsberg, Ed., a part of the series "The Chemistry of Heterocyclic Compounds", A. Weissberger, Ed. The 3-nitropyridines, 198, are prepared by reacting appropriate 2-chloro-3-nitropyridines with sodium salts of appropriate pyrazoles, 1,2,4-triazoles or imiazoles in an inert solvent such as N,N-dimethylformamide (DMF), according to the teachings of U.S. 3,489,761.

Reaction 63(b)

In this reaction 3-pyridinesulfonyl chlorides, 200, are prepared by the Meerwein reaction by diazotizing corresponding 3-aminopyridines with sodium nitrite in hydrochloric acid and acetic acid, and reacting the diazonium salts with excess sulfur dioxide in acetic acid in the presence of copper(I) chloride or copper(II) chloride catalyst. For details, refer to analogous reactions described in Y. Morisawa et al., J. Med. Chem., 23, 1376 (1980). EP-A-83,975 and H. L. Hale and F. Sowinski, J. Org. Chem., 25, 1824 (1960).

Reaction 63(c)

In this reaction sulfonyl chlorides 200 are transformed to sulfonamides 197c, by reaction with anhydrous ammonia in an inert solvent such as tetrahydrofuran, or by reaction with aqueous ammonium hydroxide in an inert solvent such as tetrahydrofuran by conventional methods. For details, refer to analogous reactions described in references cited above for Reaction 63(b).

2- and 3-Pyridinesulfonamides of Formula 197a, substituted in the 3- and 2-positions respectively with 5-thioxadiazol-2-yl groups (Q is Q-53), may be prepared from corresponding 2- and 3-mercaptopyridyl carboxylic acids, 201, by the sequence of reactions shown below in Equation 64.

Equation 64

(a)

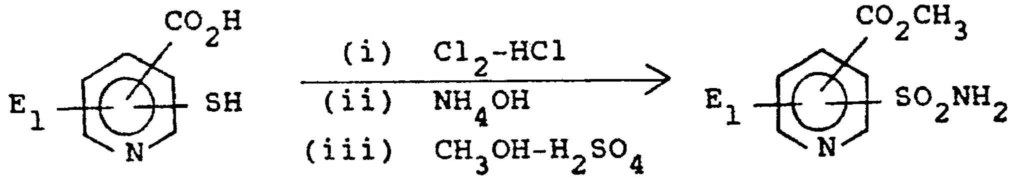


201 202

(b)

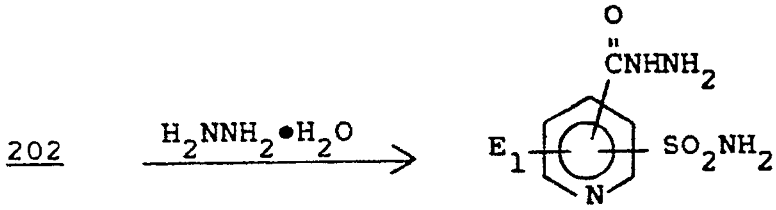


203

(c)

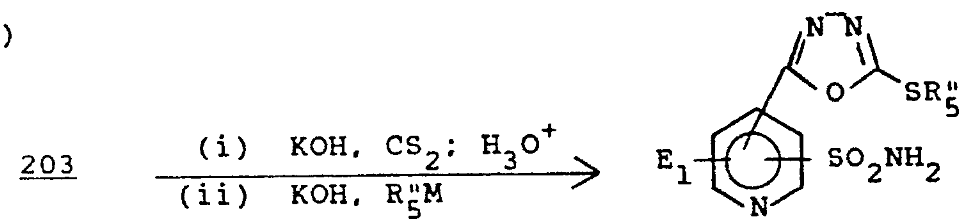


197d

wherein

| | |
|---|---|
| $E^1$ | is as previously defined; |
| $R_5''$ | is $C_1$-$C_3$ alkyl, $CH_2CH=CH_2$ or $CF_2H$; and |
| M | is Cl, Br or I. |

Reactions 64(a)

In these reactions pyridinesulfonamides, 202, are prepared by analogy with the teachings of Y. Morisawa et al., J. Med. Chem., 23, 1376 (1980). The method comprises (i) chlorinating appropriate 2-carboxy-3-mercaptopyridines or 3-carboxy-2-mercaptopyridines in concentrated hydrochloric acid and water at about -25 to 5°C to form corresponding carboxypyridyl sulfonyl chlorides; (ii) aminating the isolated sulfonyl chlorides with concentrated ammonium hydroxide to form the corresponding carboxypyridylsulfonamides; and (iii) esterifying these compounds by refluxing in methanol with sulfuric acid catalyst to form sulfonamides, 202.

Alternatively, mercaptopyridines, 201, may be chlorinated in the presence of potassium hydrogen difluoride in an inert solvent such as water and methanol at about -30° to 0°C to form the corresponding carboxypyridyl sulfonyl fluorides. Subsequent reaction of these compounds with ammonia can provide the corresponding sulfonamides. For further details refer to analogous reactions described in D. J. Brown and J. A. Hoskins, J. Chem. Soc. Perkin Trans I, 522 (1972).

Reaction 64(b)

This reaction is also run by analogy with the teachings of ibid. The method comprises reacting pyridyl esters, 202, with excess hydrazine monohydrate in methanol at reflux for several hours. Under certain conditions sulfonamides, 202, may ring-close to saccharin-like structures, either during their preparation in

Reaction 64(a) or during reflux with hydrazine in Reaction 64(b). In either case, subsequent reaction with hydrazine as described above may provide hydrazides, 203.

Reaction 64(c)

The conversion of hydrazides to 5-mercapto-oxadiazoles is well-known in the literature. e.g., R. W. Young and K. H. Wood, J. Am. Chem. Soc., 77, 400 (1955). In a typical procedure, hydrazides, 203, are heated at reflux with equimolar amounts of potassium hydroxide and an excess of carbon disulfide in methanol or ethanol solvent until the evolution of hydrogen sulfide has nearly stopped. Oxadiazoles, 197d - ($R_5''$ = H), are isolated by concentration of the solvent, addition of water to the residue, filtration of the aqueous suspension to remove insoluble impurities, acidification of the aqueous filtrate with hydrochloric acid and filtration.

Alkylation of 5-mercaptooxadiazoles is also well-known in the literature, e.g., S. Giri et al., Agr. Biol. Chem., 40, 17 (1976). Typically, oxadiazoles, 197d ($R_5''$ = H), are reacted with an equimolar amount of base such as potassium hydroxide and excess alkylating agent, $R_5''$ M, at reflux in an inert solvent such as methanol or ethanol for 0.5 to 24 hours. Sulfonamides, 197d, are isolated by concentration of the solvent, addition of water to the residue and filtration. For the case where $R_5''$ = $CF_2H$, the reaction is preferably run in DMF solvent at 60°-90°C with excess potassium carbonate as base. Following addition of water, sulfonamides, 197d, are isolated by filtration.

2- and 3-Pyridinesulfonamides of Formula 197a, substituted in the 3- and 2-positions respectively with a phenyl group, may be prepared from corresponding 3-phenyl-2-pyridinols and 2-phenyl-3-pyridinols, 204, by the sequence of reactions shown below in Equation 65.

Equation 65

(a)

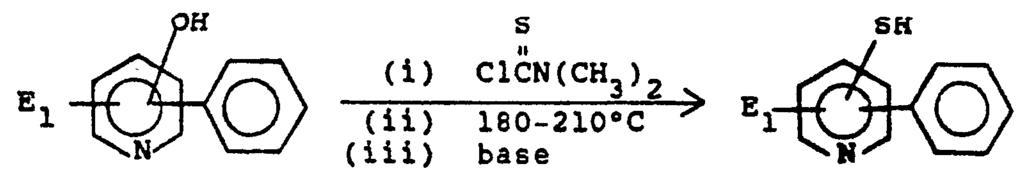


204 205

(b)

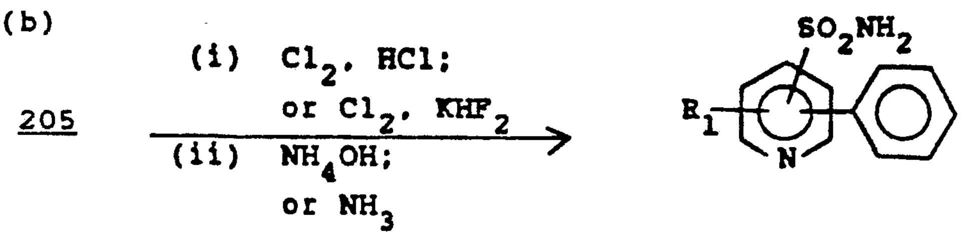


197e

wherein

$E_1$ is as previously defined.

Reaction 65(a)

In these reactions mercaptopyridines, 205, are prepared by analogy with the teachings of B. Blank et al., J. Med. Chem., 17, 1065 (1974). The method comprises (i) reacting pyridinols, 204, with dimethylthiocarbamoyl chloride and a base such as 1,4-diazabicyclo[2.2.2]octane (DABCO) in DMF to form the corresponding N,N-dimethyl-O-thiocarbamates; (ii) heating these compounds at elevated temperatures, e.g., 180°-210°C to form the corresponding N,N-dimethyl-S-carbamates; and (iii) heating these compounds with a base such as sodium carbonate or sodium hydroxide in an inert solvent such as methanol to form mercaptopyridines, 205.

Reaction 65(b)

These reactions are run by procedures analogous to those described above for the preparation of 202 - (Equation 64a).

2- and 3-Pyridinesulfonamides of Formula 197a, substituted in the 3- and 2-positions respectively with pyridinyl, pyrimidinyl, pyrazinyl, pyridazinyl or triazinyl groups (Q is Q-72 to Q-81) may be prepared by the sequence of reactions shown in Equation 66 below.

Equation 66

(a)

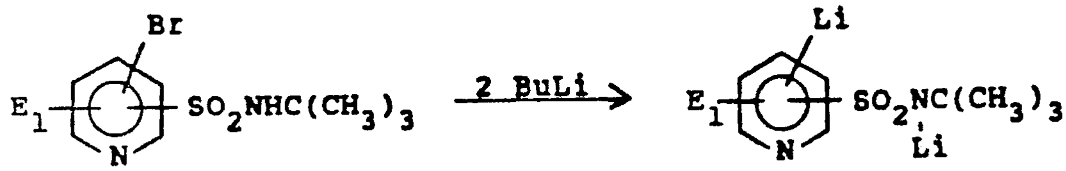


206 207

(b)

207 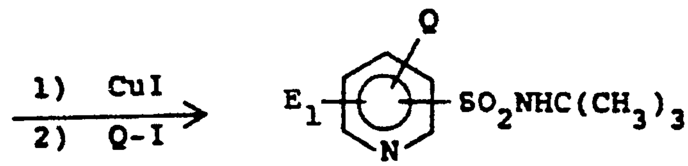


208 209

(c)

209 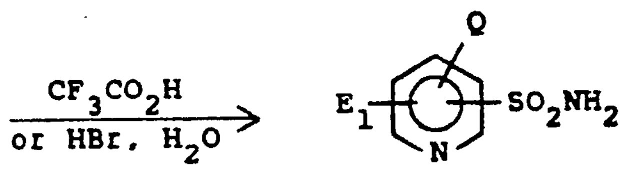


197f

wherein

E₁ is H or $CH_3$; and

Q is Q-72 to Q-81.

The compounds of Formula 197f are prepared by analogy with the teachings of EP-A No. 85,476 (published December 13, 1983).

Reaction 66(a,b)

An appropriate 2-bromo-3-(N-t-butyl)pyridinesulfonamide or 3-bromo-2-(N-t-butyl)pyridinesulfonamide is dissolved in an ethereal solvent, such as tetrahydrofuran, and two equivalents of n-butyllithium in hexanes are added at about -70° C. After 1-5 hours at about -70° C, the corresponding compound of Formula 207 is formed. One equivalent of copper(I) iodide is added at about -70° C, followed by 1-1.5 equivalents of an appropriately substituted heteroaromatic iodide of Formula 208. The reaction mixture is stirred at 0° to 70° C for 1-3 days, concentrated and poured onto aqueous ammonia. Compounds of Formula 209 are isolated by filtration if solids, or by extraction with methylene chloride and concentration if oils. The compounds, 209, may be further purified by recrystallization or chromatographic procedures. The compounds of Formula 206 and 208 may be prepared by methods known to those skilled in the art. Pertinent references for the preparation of compounds of Formula 208, are described in EP-A No. 85,476.

Reaction 66(c)

This reaction is conducted by stirring a compound of Formula 209 with 2-10 equivalents of trifluoroacetic acid or aqueous HBr with or without an inert solvent at 30°-70°C for 1-3 days. The product, 197f, may be isolated as a trifluoroacetate or hydrobromide salt by evaporation of solvent and excess acid and trituration with ether. The free base may be obtained by neutralization of the salt with aqueous base, extraction into an organic solvent, and concentration of the organic extracts.

2- and 3-Pyridinesulfonamides of Formula 197g can be prepared as shown in Equation 67 by procedures analogous to the preparation of Compounds 197d described in Equation 64.

Equation 67

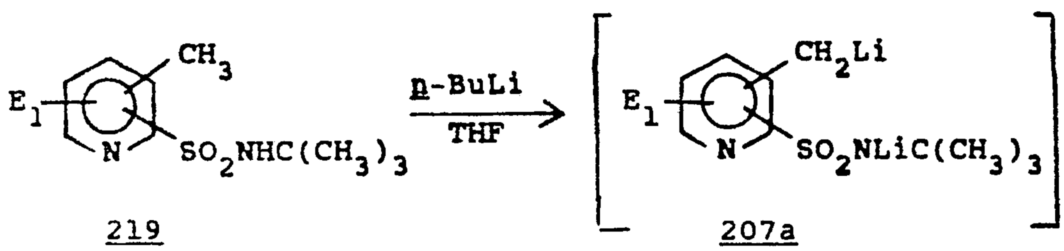


219 207a

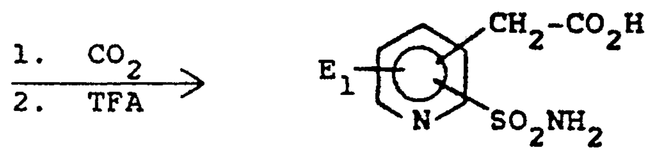


220

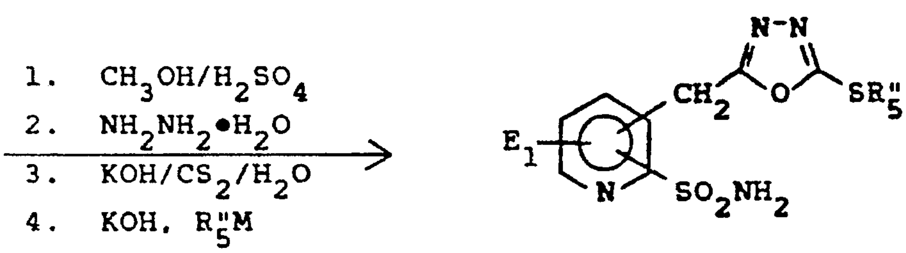


197q

wherein

$E_1$ is as previously defined;
$R''_5$ is $C_1$-$C_3$ alkyl or $CF_2H$; and
M is Cl, Br or I.

Compounds of Formula 197h in Equation 68a can be prepared from the lithium salt 207a by procedures described for the preparation of similar compounds in Equation 66 and Compound 197i can be prepared as shown in Equation 68b.

Equation 68a

207a

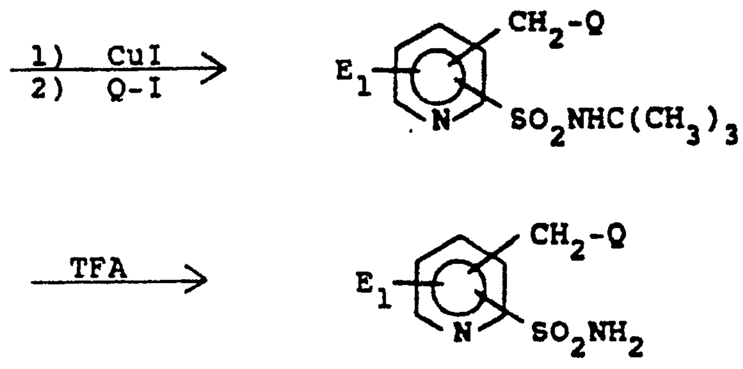


197h

wherein

E1 is H; and
Q is Q-72 to Q-81.

Equation 68b

207a

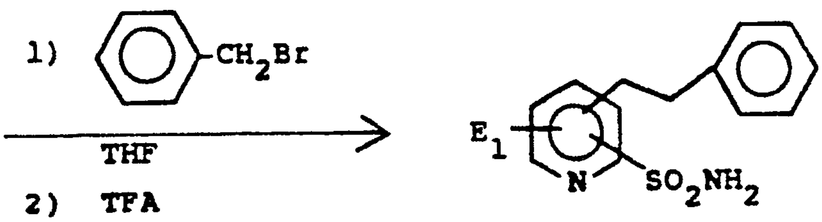


197i

The dilithio salt 207a can be prepared by lithiation of the appropriate picolinesulfonamides according to the teachings of R. E. Smith, S. Boatman and C. R Hauser; J. Org. Chem., 33, 2083 (1968).

Nitropyridines of Formula 197j containing an o-alkylfuran or thiophene group (Q is Q-67 to Q-70), can be prepared by analogy with the teachings in EP-A No. 85,476, and references cited therein, as illustrated in Equation 69.

Equation 69

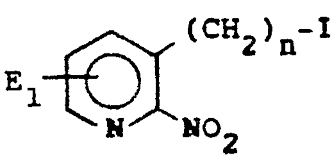

221

+ Cu-Q

222

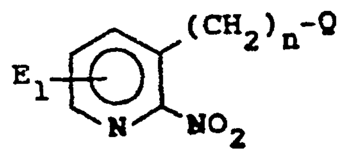

197j

wherein

E1 and n are as originally defined; and
Q is Q-67 to Q-70.

Thus, a furyl- or thienylcopper compound of Formula 222 is reacted with an o-(iodoalkyl)nitropyridine of Formula 221 in an inert solvent such as pyridine or quinoline at 0° to 60°C for 1-3 days. The product. 197i, is isolated by addition of acid such as acetic acid and water, extraction with methylene chloride, stripping of solvent and chromatographing the crude product. The copper compounds of Formula 222 are prepared by

reacting the corresponding lithium compounds with cuprous iodide or cuprous bromide in an inert solvent such as ethyl ether. Detailed procedures for analogous types of reactions are described in the following references: M. Nilsson and C. Ullenius, Acta. Chem. Scand., 24, 2379-2388 (1970); C. Ullenius, Acta. Chem. Scand., 26, 3383-3386 (1972).

2- and 3-Pyridinesulfonamides of Formula 197a substituted in the 3- and 2-positions, respectively, with group Q-71 may be prepared as shown in Equation 70.

Equation 70

(a)

207

or

207a

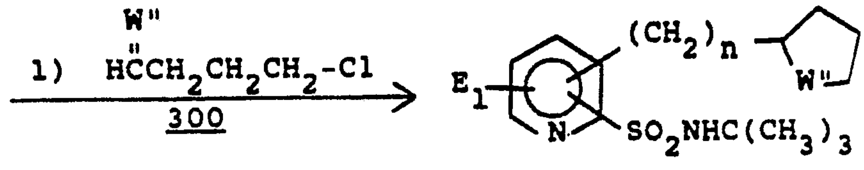


210

(b)

210

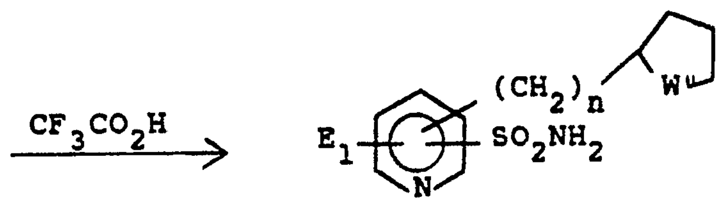


197a

wherein

$E_1$ is H or $CH_3$; and

n is 0 or 1.

The compounds of Formula 197a are prepared by analogy with the teachings in EP-A No. 85,476 (published August 10, 1983).

Reaction 70(a)

In this reaction dilithio salt, 207 or 207a, in tetrahydrofuran is treated with one equivalent of 300 at -70 °C to -80 °C. After stirring at about 25 °C for 1-3 days, the reaction is quenched by the addition of an acid such as acetic acid, and the product, 210, is isolated and purified by stripping the solvent and chromatographing the residue.

Reaction 70(b)

This reaction is run by procedures analogous to those described above in Equation 66(c).

3-Pyridinesulfonamides of Formula 197k, substituted in the 2-position by a furan or thiophene group (Q is Q-67 to Q-70) may be prepared as shown below in Equation 71.

Equation 71

(a)

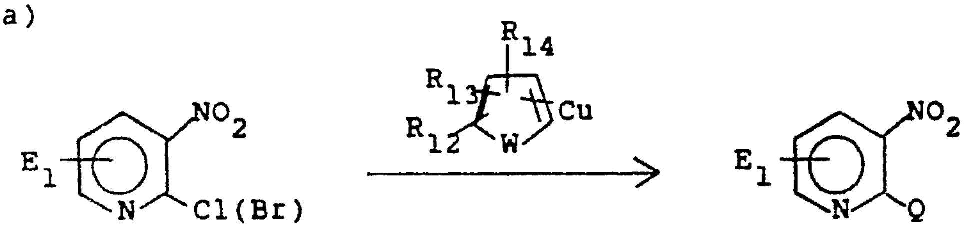

211 212 213

(b)

213

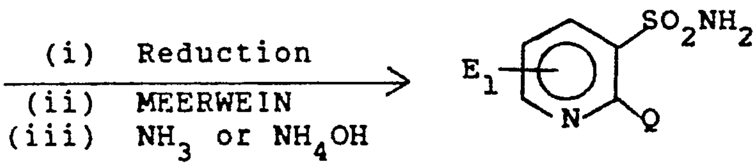


197k

wherein

| | |
|---|---|
| $E_1$, $R_{12}$, $R_{13}$ and $R_{14}$ | are as originally defined; |
| Q | is Q-67 to Q-70; and |
| W | is O or S. |

Reactions 71(a,b)

In Reaction 71(a) a furyl- or thienylcopper compound of Formula 212 is reacted with an appropriate chloro- or bromonitropyridine of Formula 211 in a solvent such as pyridine or quinoline. The copper compounds of Formula 212 are prepared by reacting the corresponding lithium compounds with cuprous iodide or cuprous bromide in a solvent such as diethyl ether. The detailed procedures for analogous types of reactions are described in the following references: M. Nilsson and C. Ullenius, Acta. Chem. Scand., 24, 2379-2388 (1970); C. Ullenius, Acta. Chem. Scand., 26, 3383-3386 (1972).

2-Pyridinesulfonamides of Formula 1971, substituted in the 3-position by a furan or thiophene group (Q is Q-67 to Q-70) may be prepared by the sequence of reactions shown in Equation 72 below.

Equation 72

(a)

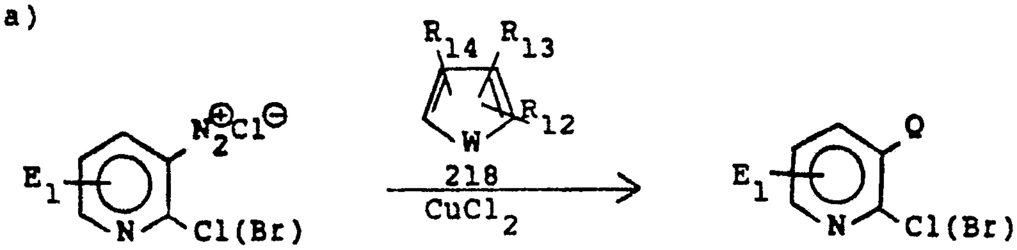


214 215

(b)

215 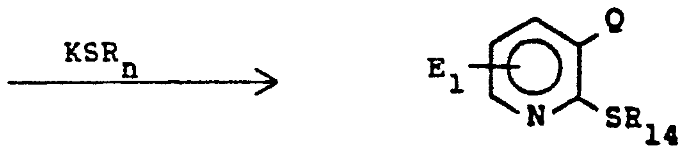


216

(c)

216 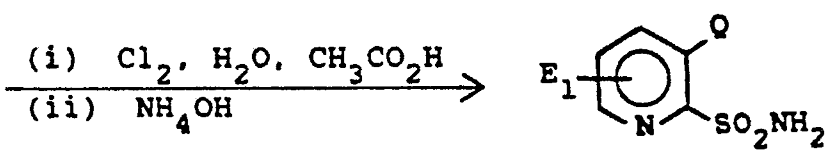


1971

wherein

| | |
|---|---|
| $E_1$, $R_{12}$, $R_{13}$ and $R_{14}$ | are as originally defined; |
| Rn | is $CH_2CH_2CH_3$ or $CH_2C_6H_5$; |
| Q | is Q-67 to Q-70; and |
| W | is O or S. |

Reaction 72(a)

In this reaction a 2-halo-3-pyridyl diazonium salt is coupled with an appropriately substituted thiophene or furan in the presence of a catalyst such as cupric chloride. This reaction may be run by procedures analogous to those described in Gomberg and Bachman, J. Am. Chem. Soc., 46, 2339 (1924); J. Johnson, J. Chem. Soc., 895 (1946); and in J. Pharm. Soc. (Japan), 90, 1150-1155 (1970), or simple modifications thereof, by those skilled in the art. In cases where both the α- and the β-position of the thiophene or furan are available for coupling, both isomers are usually obtained with the α-coupled product being the predominant isomer. These isomers may be separated by fractional crystallization or chromatographic procedures.

Reaction 72(b)

In this reaction thiopyridines of Formula 216 are prepared by conventional methods by treating 2-halopyridines, 215, with potassium benzyl or propylmercaptide in an inert solvent such as DMF at about 25° to 130° C for one to 24 hours. Following isolation by usual methods, the product, 216, may be purified by chromatographic procedures.

Reaction 72(c)

In this reaction the products of Reaction 72(b) are oxidatively chlorinated in a suitable inert solvent such as chloroform, methylene chloride or acetic acid, in the presence of water, to produce the corresponding sulfonyl chlorides. The reaction is carried out in the presence of at least 2.5 molar equivalents of water and at least three molar equivalents of chlorine at about -30° to 5°C for 1-5 hours. Following isolation, the sulfonyl chlorides are reacted with ammonia or ammonium hydroxide by conventional methods.

Other 2- and 3-pyridinesulfonamides of Formula 197a may also be prepared by oxidatively chlorinating appropriate 3-heterocyclic-2-thiopyridines and 2-heterocyclic-3-thiopyridines of Formula 217 to the corresponding sulfonyl chlorides, followed by amination to the corresponding sulfonamides, as shown below in Equation 73.

Equation 73

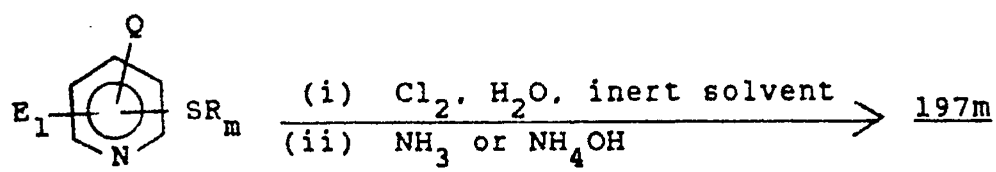


217

wherein

$E_1$ is as originally defined; and

$R_m$ is H, $C_2H_5$ or $CH_2C_6H_5$.

The reactions of Equation 73 are carried out by methods analogous to those described above in Equation 72(c) and for the preparation of 202 (Equation 5a). The compounds of Formula 217 may be prepared by those skilled in the art by the application of appropriate methods selected from the variety of known literature procedures for preparing substituted aromatic heterocycles. See, for example, EP-A No. 83,975 (published July 20, 1983), and references cited therein, which describe methods for transforming various o-(substituted)nitrobenzenes to corresponding o-(heterocyclic)-nitrobenzenes, in which the o-heterocyclic groups are Q-46 to Q-66. By carrying out similar reactions on appropriately substituted pyridines, or simple modifications thereof, those skilled in the art may prepare many of the compounds of Formula 217 above.

There exists a variety of known methods for incorporating a mercapto or alkylthio group into the 2- or 3-position of a pyridine ring, methods which are useful for the preparation of the compounds of the general Formula 217 described above in Equation 73. The choice of methods used depends in part on the reaction sequences used to prepare compounds 217, which would be obvious to those skilled in the art. These methods include (i) reacting 3-pyridyl diazonium salts with potassium ethyl xanthate to form corresponding 3-mercaptopyridines, which can be alkylated to 3-benzyl- or 3-propylthiopyridines by obvious methods; for details, refer to analogous reactions described in J. Pharm. Belg., 22, 213 (1967); ibid., 29, 281 (1974) and J. Org. Chem., 23, 1924 (1958); (ii) saponifying N,N-dimethyl-S-carbamates, prepared from 2- or 3-pyridinols, to form corresponding 2- or 3-mercaptopyridines; for general details see Equation 6(a) above; (iii) reacting 3-halopyridines, containing an activating ortho-group such as aldehyde, ketone, carboxylic ester, amide, nitrile or nitro group, with potassium propanethiol or benzylthiol in an inert solvent such as DMF or hexamethylphosphoramide by known methods to form corresponding 3-benzylthiol- or 3-propanethiol-pyridines; (iv) displacing a 2-halogen on a pyridine ring by sulfur nucleophiles such as potassium or sodium hydrosulfide, thiourea or potassium benzylthiol or propanethiol to form 2-thiosubstituted pyridines; for details, see analogous reactions described in J. Het. Chem., 3, 27 (1966); ibid., 17, 149 (1980); ibid., 5, 647 (1958); J. Am. Chem. Soc., 68, 342 (1946) and ibid., 70, 3908 (1948); and (v) diazotizing and converting 2-aminopyridines to 2-pyridinols which may be converted to 2-pyridinethiols with $P_4S_4$; for details see analogous reactions described in Farmaco Ed. Sci., 22, 1069 (1967).

The preparation of pyridinethiols is also reviewed in "Pyridine and Its Derivatives", Part 4, 1964, by H. L. Hale, a part of the series "The Chemistry of Heterocyclic Compounds", A. Weissberger, Ed., published by Interscience Publ., New York and London.

The heterocyclic amines of Formula 5 are also important intermediates for the preparation of the compounds of this invention, and can be prepared by methods known in the literature, or simple

modifications thereof, by those skilled in the art. For details, see, for example, EP-A No. 84,224 (published July 27, 1983) and W. Braker et al., J. Am. Chem. Soc., 69, 3072 (1947), which describe the synthesis of pyrimidine- and triazineamines substituted by acetals and thioacetals such as dialkoxymethyl or 1,3-dioxolan-2-yl. See also, for example, South African Patent Application Nos. 825,045 and 825,671 which describe the synthesis of aminopyrimidines and triazines substituted by such groups as haloalkoxy or haloalkylthio groups, e.g., $OCH_2CH_2F$, $OCH_2CF_3$, $OCF_2H$ or $SCF_2H$. Also, South African Patent Application No. 837,434 (published October 5, 1983) describes methods for the synthesis of cyclopropylpyrimidines and triazines substituted by such groups as alkyl, haloalkyl, alkoxy, haloalkoxy, alkylamino, dialkylamino or alkoxyalkyl.

Also, the 5,6-dihydrofuro[2,3-d]pyrimidin-2-amines, the cyclopenta[d]pyrimidin-2-amines (5, A = A-2) and the 6,7-dihydro-5H-pyrano[2,3-d]pyrimidin-2-amines (VII, A = A-3) can be prepared as described in EP-A No. 15,683. Also, the furo[2,3-d]pyrimidin-2-amines (VII, A = A-4) are described in EP-A No. 46,677.

Compounds of Formula 5, where A is A-5, are described in EP-A No. 73,562. Compounds of Formula 5, where A is A-6, are described in EP-A No. 94,260.

In addition, general methods for preparing aminopyrimidines and triazines have been reviewed in the following publications:

- "The Chemistry of Heterocyclic Compounds", a series published by Interscience Publishers, Inc., New York and London;
- "Pyrimidines", Vol. 16 of the same series, by D. J. Brown.
- "s-Triazines and Derivatives", Vol. 13 of the same series, by E. M. Smolin and L. Rappaport; and
- F. C. Schaefer, U.S. 3,154,547 and K. R. Huffman and F. C. Schaefer, J. Org. Chem., 28, 1812 (1963) which describe the synthesis of triazines.

Agriculturally suitable salts of compounds of Formulas Ia-Ie can be prepared by a number of ways known in the art. For example, some such ways include (1) metal and quarternary ammonium salts made by treatment of compounds of Formulas Ia-Ie with solutions of metal or ammonium hydroxides, alkoxides or carbonates that are suffiently basic with respect to the substrates Ia-Ie ; (2) cation exchange of those salts formed e.g. by (1) with an aqueous solution of a second cation; in particular, one whose resultant salt preferentially is sparingly soluble in aqueous solution and therefore precipitates from the solution; (3) cation exchange via passage through various cation exchange resins loaded with the cation to be exchanged; in particular, the exchange with cations whole resultant salts with Ia-Ie are water-soluble and (4) acid addition salts made by treatment of compounds of Formulas Ia-Ie with suitably strong acids such as trichloracetic acid or p-toluenesulfonic acid..

The compounds described in this invention are further illustrated by the following Examples 1 through 11.

Example 1

3-(3-Thienyl)-4,5-dihydro-5-acetoxyisoxazole

To an ice-cooled mixture of 100 g thiophene-3-carboxaldehyde, 100 mL methanol, 100 mL water, and 80 g hydroxylamine hydrochloride was added 50% aqueous NaOH to neutrality (ca. 40 mL). Methanol was removed by rotary evaporation and the mixture was extracted with ether. The ether extracts were washed with brine, dried ($MgSO_4$), and concentrated to afford 117 g of crude oxime. This was dissolved in 100 mL of DMF and 110 g of N-chlorosuccinimide was added in portions at 15°C. After the addition, the mixture was allowed to stir at 15°C for 1 h, and then the cooling bath was removed. A rapid exotherm caused the temperature to rise to 50°C and ice was immediately added to the reaction mixture, which then was partitioned between ether and water. The ether layer was washed several times with water, then washed with brine, dried ($MgSO_4$), and filtered.

Vinyl acetate (100 mL) then was added, and the solution was heated at reflux while adding a solution of 80 mL of triethylamine in 250 mL of ether over a 4 hour period. The mixture was cooled and filtered and the filter cake was washed several times with water and was air-dried. This material was combined with more product obtained by separating the filtrate, washing the $Et_2O$ layer with brine, drying ($MgSO_4$), and concentration and trituration with ether (total yield 88.6 g, mp 98-99°C).

Example 2

3-(3-Thienyl)isoxazole

To a solution of 88.6 g of the dihydroisoxazole in 300 mL of methylene chloride was added 88 g of 1,8-diazabicyclo[5.4.0]undec-7-ene (DBU) whereupon an exotherm caused the solvent to reflux. The solution was washed with aqueous HCl and with brine then was dried ($Na_2SO_4$), concentrated, and distilled to afford 52 g of the isoxazole, bp 75-80 °C/l mm.

Example 3

3-(3-Isoxazolyl)-2-thiophenesulfonyl chloride

To a solution of the thienylisoxazole in 300 mL of ether was added 60 mL of n-butyllithium as a 1.6M solution in hexane at -78 °C under nitrogen. After stirring for 10 min at -78 °C, liquid $SO_2$ (10 mL) was added and the mixture was allowed to stand overnight at room temperature. Filtration afforded the sulfinate salt which was hygroscopic. This was stirred in 100 mL of 50% aqueous acetic acid while adding 13 g of N-chlorosuccinimide in portions at 0 °C. After stirring for 30 minutes at room temperature, the mixture was diluted with 200 mL of ice water, filtered, washed with ice water, and air dried to afford 20 g of sulfonyl chloride, mp 92-92.5 °C.

Example 4

3-(3-Isoxazolyl)-2-thiophenesulfonamide

A solution of 19.5 g of the sulfonyl chloride in 150 mL of methylene chloride was saturated with gaseous ammonia and was stirred for 16 h at 25°. The precipitate was filtered and washed with $CH_2Cl_2$, then with water and air-dried to afford 11.7 g of the title sulfonamide, mp 163-164 °C. Another 1.5 g of product was obtained by washing the $CH_2Cl_2$-soluble fraction with brine, drying ($Na_2SO_4$), concentration, and trituration with ether.

Example 5

3-(3-Isoxazolyl)-2-thiophenesulfonylisocyanate

A mixture of 10 g of the sulfonamide, 100 mL of 2-butanone, 6 mL of n-butyl isocyanate, and 5 g of potassium carbonate was heated at reflux for 1 h, then was concentrated, acidified with cold, aqueous HCl, and filtered. The solid was dissolved in $CH_2Cl_2$, dried ($MgSO_4$) and concentrated to afford 12 g of the n-butyl urea. The butyl urea (11 g) was heated in 50 mL of xylene containing 0.2 g of 1,4-diazabicyclo[2.2.2.]-octane at 135 °C while adding a solution of 4 mL of phosgene in 6 mL of xylene over a 1 h period. The mixture was cooled, filtered, and concentrated to an orange oil which had a strong IR absorption at 2250 $cm^{-1}$ indicative of a sulfonylisocyanate. This oil (11 g) was dissolved in 40 mL of methylene chloride and was used for the next reaction.

Example 6

N-[(4,6-Dimethyl-pyrimidin-2-yl)aminocarbonyl]-3-(3-isoxazolyl)-2-thiophenesulfonamide

To 6 mL of the isocyanate/$CH_2Cl_2$ solution (1.1 g of contained isocyanate) was added 0.4 g of 2-amino-4,6-dimethylpyrimidine in 5 mL of methylene chloride. The solvent was removed and the crude product was triturated with ether and filtered. Recrystallization from $CH_3CN$/n-BuCl afforded 250 mg of product, mp 172.5-173°. NMR($CDCl_3$) δ2.4 (s,6H), 6.76(s,1H), 7.0(d,1H), 7.5(d,1H), 8.2(d,1H) 9.0(d,1H) 8.1(br s,1H), 10.6-(br s,1H).

Example 7

3-Nitro-2-(1H-1,2,4-triazol-1-yl)pyridine

A solution of 10 g of 2-chloro-3-nitropyridine dissolved in 30 mL of dry DMF was added dropwise to a suspension containing 6.9 g of 1,2,4-triazole sodium salt (90%, Aldrich Chemical Co.) in 40 mL of dry DMF. After a slow exotherm had subsided, the suspension was heated at 60 °C for three hours, then cooled to 25 °C and poured onto 500 mL of ice-water to yield a precipitate. After the mixture was filtered, the isolated

solid was washed 2x50 mL of water and suction-dried to yield 12 g of crude product. The product was recrystallized from 2-propanol to yield 8 g of the subject compound; m.p. 131-133°C.
Anal. calc. for $C_7H_5N_5O_2$: C, 43.9; H, 2.7; N, 36.6;
Found: C, 44.6; H, 2.7; N, 36.7.

Example 8

3-Amino-2-(1H-1,2,4-triazol-1-yl)pyridine

To a suspension containing 35.4 g of stannous chloride dihydrate in 100 mL of concentrated hydrochloric acid was added portionwise 10 g of the compound prepared in Example 7 over a 0.25 hour period. After the resulting exotherm (23°-79°) slowed, the suspension was heated at 85-90° for one hour, then cooled to 0°. The mixture was poured onto excess ice-water (about 700 mL), and the suspension was made strongly basic to litmus by addition of 50% aqueous NaOH to yield a precipitate. After filtering the mixture, the isolated solid was washed with water, suction-dried, then recrystallized from ethyl acetate-hexanes to yield 3 g of the subject compound; m.p. 103-105°C.
Anal. calc. for $C_7H_7N_5$: C, 52.2; H, 4.4; N, 43.4;
Found: C. 52.4; H, 4.3; N, 41.6.

Example 9

2-(1H-1,2,4-Triazol-1-yl)-3-pyridinesulfonyl chloride

A diazonium salt was prepared by adding a solution of 9 g of sodium nitrite in 30 mL of water to a suspension of 20 g of the compound prepared by the procedure of Example 8 in 45 mL of concentrated hydrochloric acid and 127 mL of glacial acetic acid at 0-25°C. After stirring about 0.4 hour, the diazonium suspension was poured slowly into a mixture consisting of 93 mL of acetic acid, 5.3 g of cupric chloride dihydrate and 37 mL of sulfur dioxide while cooling the reaction flask at 10-20°C in a dry ice-acetone bath. During the addition a delayed vigorous gas evolution with foaming occurred and was controlled by cooling and decreasing the rate of addition of the diazonium suspension. After addition was complete, the cooling bath was removed and the suspension was stirred at ambient temperature for four hours. The suspension was poured into ice-water (about 800 mL) and stirred to yield a solid. After the mixture was filtered, the isolated solid was washed 2x50 mL of water and suction-dried overnight to yield 17 g of the subject compound; m.p. 128-132°C.
Anal. calc. for $C_7H_5ClN_4O_2S$: C, 34.4; H, 2.1; N, 22.9;
Found: C, 34.4; H, 2.1; N, 23.2.

Example 10

2-(1H-1,2,4-Triazol-1-yl)-3-pyridinesulfonamide

To a suspension containing 15 g of the compound prepared in Example 9 in 125 mL of tetrahydrofuran was added dropwise 23 mL of concentrated aqueous ammonium hydroxide while maintaining the reaction temperature at 10-20°C with external ice-water cooling. After stirring at room temperature for three hours, the suspension was concentrated in vacuo to a water suspension. The suspension was poured into ice-water (about 200 mL) and stirred to yield a solid. The mixture was filtered to yield 12 g of crude product, which was recrystallized from acetonitrile to yield 8 g of the subject compound; m.p. 185-188°C.
Anal. calc. for $C_7H_7N_5O_2S$: C, 37.3; H, 3.2; N, 31.0;
Found: C, 37.4, H, 3.0; N, 30.9.

Example 11

N-[(4,6-Dimethoxypyrimidin-2-yl)aminocarbonyl]-2-(1H-1,2,4-triazol-1-yl)-3-pyridinesulfonamide

To a suspension containing 0.5 g of the sulfonamide prepared in Example 10 in 10 mL of p-dioxane was added 0.6 g of phenyl(4,6-dimethoxypyrimidin-2-yl)carbamate followed by 0.33 g of 1,8-diazabicyclo-[5.4.0]undec-7-ene (DBU). The suspension was stirred at room temperature for about two hours then diluted with about 75 mL of water to form a solution. After acidifying the solution with conc. hydrochloric acid (red

to litmus) and stirring 0.5 hour, a precipitate formed. The mixture was filtered and the isolated solid was washed with 10 mL water and suction-dried for 24 hours to yield 0.7 g of the subject compound; m.p. 226-230 °C.

Anal. calc. for $C_{14}H_{14}N_{8}O_{5}S$: C, 41.4; H, 3.5; N, 27.6;

Found: C, 41.3; H, 3.7; N, 27.4.

IR (nujol): 1715 cm-1 (C=O).

Using the techniques described in Equations 1-73 and Examples 1-11, or simple modifications thereof, the following compounds in Tables 1a-16g can be made by those skilled in the art.

In all Tables, W is O, unless indicated by * where W is S.

## General Structures for Tables

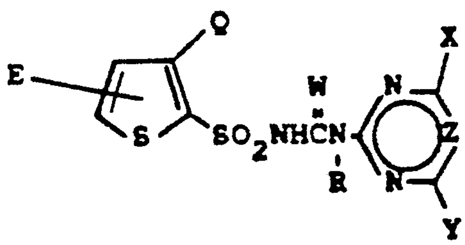

General Structure 1

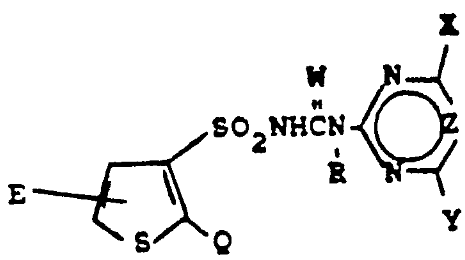

General Structure 2

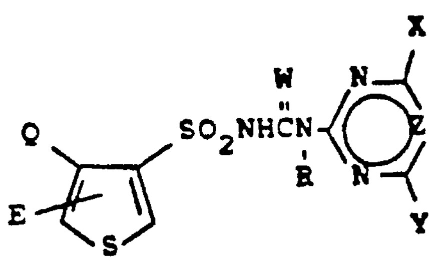

General Structure 3

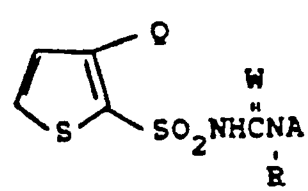

General Structure 4

General Structures for Tables (continued)

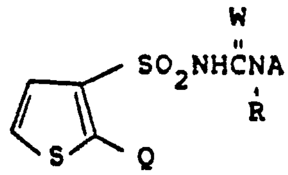

General Structure 5

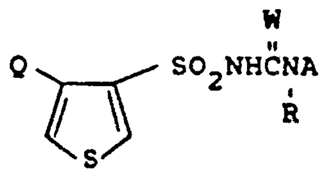

General Structure 6

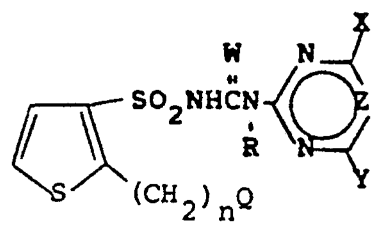

General Structure 7

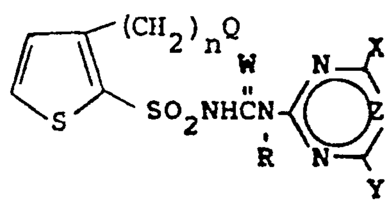

General Structure 8

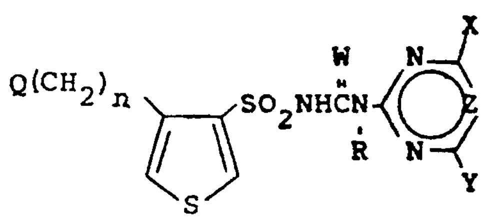

General Structure 9

**General Structures for Tables** (continued)

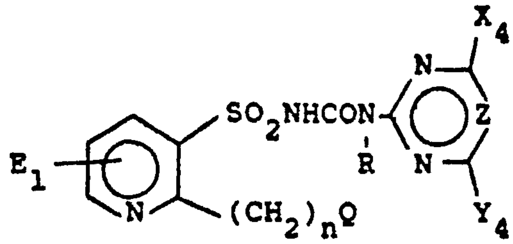

General Structure 9a

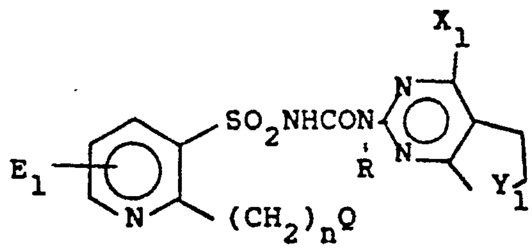

General Structure 9b

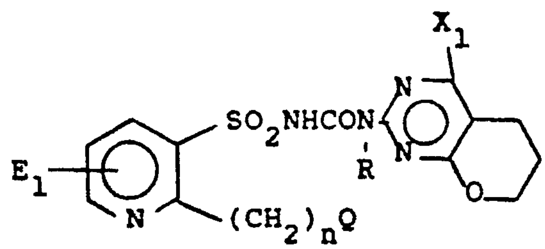

General Structure 9c

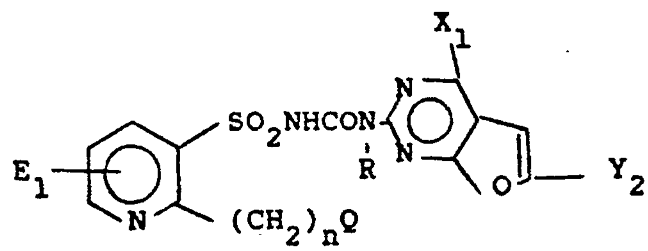

General Structure 9d

General Structures for Tables (continued)

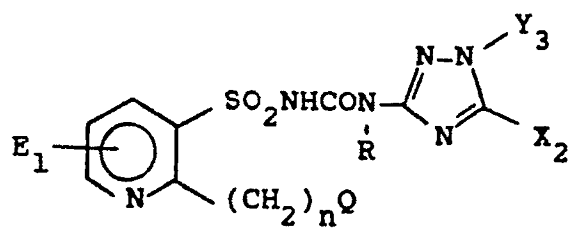

General Structure 9e

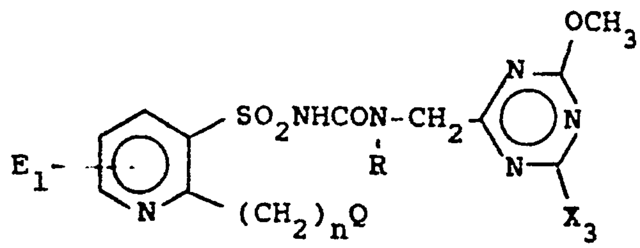

General Structure 9f

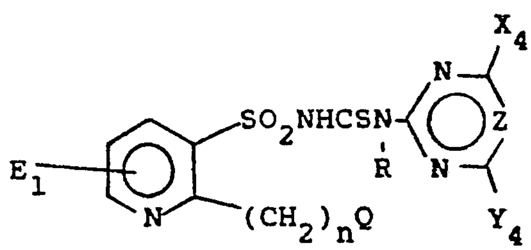

General Structure 9g

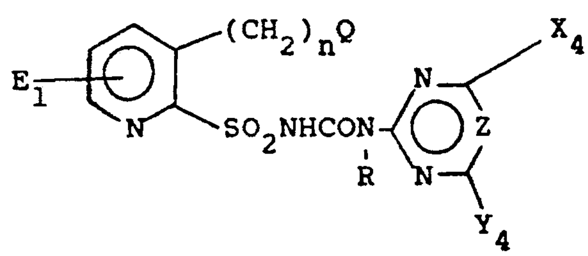

General Structure 10a

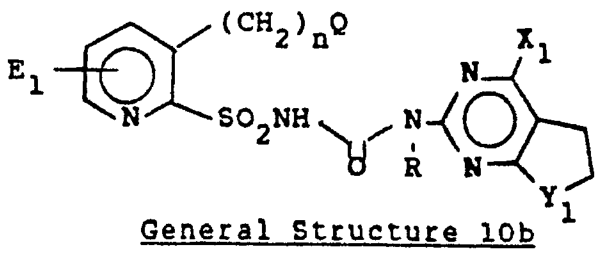

General Structure 10b

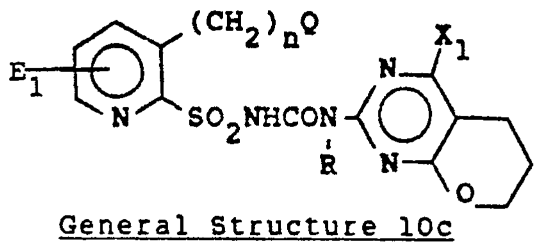

General Structure 10c

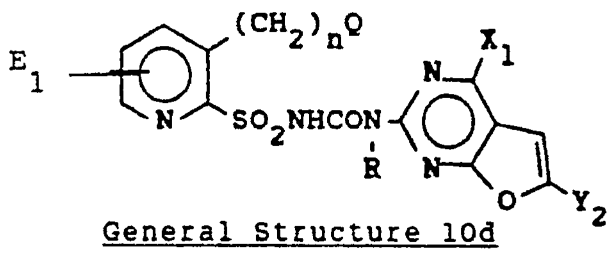

General Structure 10d

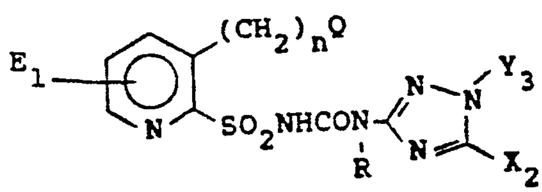

General Structure 10e

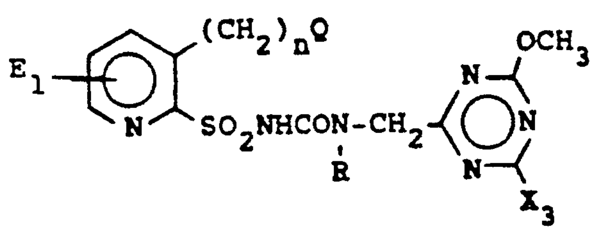

General Structure 10f

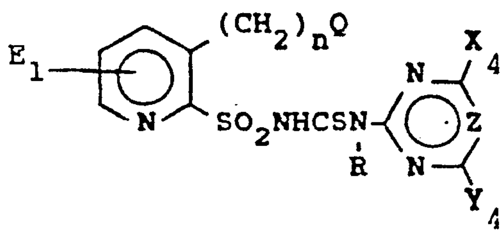

General Structure 10q

**Table 1a**

**General Structure 1**

| Q | R | $R_3$ | $R_4$ | $R_5$ | X | Y | Z | W' | m.p. °C |
|---|---|---|---|---|---|---|---|---|---|
| Q-2 | H | H | H | H | $CH_3$ | $OCH_3$ | CH | O | |
| Q-2 | H | H | H | H | $CH_3$ | $OCH_3$ | N | O | |
| Q-2 | H | H | H | H | $OCH_3$ | $OCH_3$ | CH | O | |
| Q-2 | H | H | H | H | $OCH_3$ | $OCH_3$ | N | O | |
| Q-2 | H | $CH_3$ | H | H | $CH_3$ | $CH_3$ | CH | O | |
| Q-2 | H | H | $CH_3$ | H | $CH_3$ | $OCH_3$ | N | O | |
| Q-2 | H | H | H | $CH_3$ | $CH_3$ | $OCH_3$ | CH | O | |
| Q-2 | $CH_3$ | H | H | H | CH | $CH_3$ | CH | O | |
| Q-2 | H | $CH_3$ | $CH_3$ | H | $OCH_3$ | $OCH_3$ | N | O | |
| Q-2 | H | H | H | H | Cl | $OCH_3$ | CH | S | |
| Q-2 | H | H | H | H | $CH_3$ | $OCH_3$ | CH | S | |
| Q-2 | H | H | H | H | $CH_3$ | $OCH_3$ | N | S | |
| Q-2 | H | H | H | H | $OCH_3$ | $OCH_3$ | CH | S | |
| Q-2 | H | H | H | H | $OCH_3$ | $OCH_3$ | N | S | |
| Q-2 | H | H | H | H | $CH_3$ | $CH_3$ | CH | S | |
| Q-2 | H | $CH_3$ | H | H | $CH_3$ | $CH_3$ | CH | S | |
| Q-2 | H | H | $CH_3$ | H | $CH_3$ | $CH_3$ | N | S | |
| Q-2 | H | H | H | $CH_3$ | $CH_3$ | $OCH_3$ | CH | S | |
| Q-2 | H | $CH_3$ | $CH_3$ | H | $CH_3$ | $OCH_3$ | N | S | |
| Q-2 | H | H | $CH_3$ | $CH_3$ | $OCH_3$ | $OCH_3$ | CH | S | |
| Q-2 | H | $CH_3$ | H | $CH_3$ | $OCH_3$ | $OCH_3$ | N | S | |
| Q-2 | H | $CH_3$ | $CH_3$ | $CH_3$ | $CH_3$ | $OCH_3$ | CH | S | |
| Q-2 | $CH_3$ | H | H | H | $CH_3$ | $CH_3$ | CH | S | |
| Q-2 | H | H | H | H | Cl | $OC_2H_5$ | CH | S | |
| Q-3 | H | H | H | H | $CH_3$ | $CH_3$ | CH | O | |
| Q-3 | H | H | H | H | $CH_3$ | $CH_3$ | N | O | |
| Q-3 | H | H | H | H | $CH_3$ | $OCH_3$ | CH | O | |
| Q-3 | H | H | H | H | $CH_3$ | $OCH_3$ | N | O | |
| Q-3 | H | H | H | H | $OCH_3$ | $OCH_3$ | CH | O | |
| Q-3 | H | H | H | H | $OCH_3$ | $OCH_3$ | N | O | |
| Q-3 | H | $CH_3$ | H | H | $CH_3$ | $CH_3$ | CH | O | |

Table 1a (continued)

| Q | R | $R_3$ | $R_4$ | $R_5$ | X | Y | Z | W' | m.p. °C |
|---|---|---|---|---|---|---|---|---|---|
| Q-3 | H | H | $CH_3$ | H | $CH_3$ | $CH_3$ | N | O | |
| Q-3 | H | H | H | $CH_3$ | $CH_3$ | $OCH_3$ | CH | O | |
| Q-3 | H | $CH_3$ | $CH_3$ | H | $CH_3$ | $OCH_3$ | N | O | |
| Q-3 | H | H | $CH_3$ | $CH_3$ | $OCH_3$ | $OCH_3$ | CH | O | |
| Q-3 | H | $CH_3$ | H | $CH_3$ | $OCH_3$ | $OCH_3$ | N | O | |
| Q-3 | H | H | H | H | $OCH_3$ | $OCH_2CF_3$ | CH | O | |
| Q-3 | H | H | H | H | $OCH_3$ | $OCH_2CF_3$ | CH | O | |
| Q-3 | $CH_3$ | H | H | H | Cl | $OCH_3$ | CH | O | |
| Q-3 | H | H | H | H | $CH_3$ | $OCH_3$ | CH | S | |
| Q-3 | H | H | H | H | $OCH_3$ | $OCH_3$ | CH | S | |
| Q-3 | H | H | H | H | $OCH_3$ | $OCH_3$ | N | S | |
| Q-3 | H | $CH_3$ | H | H | $CH_3$ | $CH_3$ | CH | S | |
| Q-3 | H | H | $CH_3$ | H | $CH_3$ | $OCH_3$ | N | S | |
| Q-3 | H | H | H | $CH_3$ | $CH_3$ | $OCH_3$ | CH | S | |
| Q-3 | $CH_3$ | H | H | H | $CH_3$ | $CH_3$ | CH | S | |
| Q-3 | H | H | $CH_3$ | $CH_3$ | $OCH_3$ | $OCH_3$ | N | S | |
| Q-3 | H | H | H | H | $OCH_3$ | $CF_3$ | N | S | |
| Q-4 | H | H | H | H | $CH_3$ | $OCH_3$ | CH | - | |
| Q-4 | H | H | H | H | $OCH_3$ | Cl | CH | - | |
| Q-4 | H | H | H | H | $CH_3$ | $OCH_3$ | N | - | |
| Q-4 | H | H | H | H | $CH_3$ | $CH_3$ | CH | - | |
| Q-4 | H | H | H | H | $OCH_3$ | $OCH_3$ | CH | - | |
| Q-4 | H | H | H | H | $OCH_3$ | $OCH_3$ | N | - | |
| Q-4 | H | $CH_3$ | H | H | $CH_3$ | $CH_3$ | CH | - | |
| Q-4 | H | H | $CH_3$ | H | $CH_3$ | $OCH_3$ | N | - | |
| Q-4 | H | H | H | $CH_3$ | $CH_3$ | $OCH_3$ | CH | - | |
| Q-4 | $CH_3$ | H | H | H | $OCH_3$ | $OCH_3$ | CH | - | |
| Q-4 | $CH_3$ | $CH_3$ | $CH_3$ | $CH_3$ | $CH_3$ | $OCH_3$ | N | - | |
| Q-4 | H | $CH_3$ | H | $CH_3$ | $CH_3$ | $OCH_3$ | CH | - | |
| Q-4 | H | H | H | H | $OCH_3$ | $OCH_2CF_3$ | N | - | |
| Q-4 | H | H | H | H | $CH_3$ | $NHCH_3$ | CH | - | |
| Q-4 | H | H | H | H | $CH_3$ | $CH_3$ | CH | - | |
| Q-4 | H | H | H | H | $CH_3$ | $CH_3$ | N | - | |

Table 1a (continued)

| Q | R | $R_3$ | $R_4$ | $R_5$ | X | Y | Z | W' | m.p. °C |
|---|---|---|---|---|---|---|---|---|---|
| Q-9 | H | H | H | H | $CH_3$ | $OCH_3$ | CH | - | |
| Q-9 | H | H | H | H | $CH_3$ | $OCH_3$ | N | - | |
| Q-9 | H | H | H | H | $OCH_3$ | $OCH_3$ | CH | - | |
| Q-9 | H | H | H | H | $OCH_3$ | $OCH_3$ | N | - | |
| Q-9 | $CH_3$ | $CH_3$ | H | H | $CH_3$ | $CH_3$ | CH | - | |
| Q-9 | H | H | $CH_3$ | H | $CH_3$ | $OCH_3$ | N | - | |
| Q-9 | H | H | H | $CH_3$ | $OCH_3$ | $OCH_3$ | CH | - | |
| Q-9 | H | H | $CH_3$ | $CH_3$ | $CH_3$ | $OCH_3$ | CH | - | |
| Q-9 | H | H | H | H | Cl | $OCH_2CH_3$ | CH | - | |
| Q-9 | H | H | H | H | $OCH_3$ | $CH(OCH_3)_2$ | CH | - | |
| Q-9 | H | H | H | H | $NH_2$ | $CH_3$ | CH | - | |
| Q-9 | H | H | H | H | $NHCH(CH_3)_2$ | $CH_3$ | CH | - | |
| Q-9 | H | H | H | H | $CH_2O$-n-$C_4H_9$ | $CH_3$ | CH | - | |
| Q-9 | H | H | H | H | S-n-$C_4H_9$ | $CH_3$ | CH | - | |
| Q-9 | H | H | H | H | S-n-$CH_2CH_2F$ | $CH_3$ | CH | - | |

**Table 1b**

**General Structure 1**

| Q | R | $R_3'$ | $R_4$ | X | Y | Z | W'' | m.p. °C |
|---|---|---|---|---|---|---|---|---|
| Q-10 | H | H | H | $CH_3$ | $OCH_3$ | CH | O | |
| Q-10 | H | H | H | $CH_3$ | $CH_3$ | CH | O | |
| Q-10 | H | H | H | $OCH_3$ | $OCH_3$ | N | O | |
| Q-10 | H | H | H | $OCH_3$ | Cl | CH | O | |
| Q-10 | H | H | H | $OCH_3$ | $OCH_3$ | CH | O | |
| Q-10 | H | H | H | $OCH_3$ | $OCH_3$ | N | O | |
| Q-10 | H | H | H | $CH_3$ | $OCH_3$ | CH | S | |
| Q-10 | H | H | H | $CH_3$ | $OCH_3$ | N | S | |
| Q-10 | H | H | H | $OCH_3$ | $OCH_3$ | CH | S | |
| Q-10 | H | H | H | $OCH_3$ | $OCH_3$ | N | S | |
| Q-10 | H | H | H | $OCH_3$ | Cl | CH | $NCH_3$ | |
| Q-10 | H | H | H | $CH_3$ | $CH_3$ | CH | $NCH_3$ | |
| Q-10 | H | H | H | $CH_3$ | $OCH_3$ | N | $NCH_3$ | |
| Q-10 | H | H | H | $CH_3$ | $CH_3$ | N | $NCH_3$ | |
| Q-10 | H | H | H | $OCH_3$ | $OCH_3$ | CH | $NCH_3$ | |
| Q-10 | H | H | H | $OCH_3$ | $OCH_3$ | N | $NCH_3$ | |
| Q-10 | H | $CH_3$ | H | $CH_3$ | $CH_3$ | CH | O | |
| Q-10 | H | H | $CH_3$ | $CH_3$ | $OCH_3$ | N | O | |
| Q-10 | H | $CH_3$ | $CH_3$ | $CH_3$ | $OCH_3$ | CH | O | |
| Q-10 | $CH_3$ | H | H | $OCH_3$ | $OCH_3$ | N | O | |
| Q-10 | H | H | H | $CH_3$ | 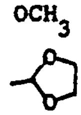 | CH | O | |
| Q-10 | H | $CH_3$ | H | $CH_3$ | $CH_3$ | CH | S | |
| Q-10 | H | H | $CH_3$ | $CH_3$ | $OCH_3$ | N | S | |
| Q-10 | H | $CH_3$ | $CH_3$ | $CH_3$ | $OCH_3$ | CH | S | |
| Q-10 | $CH_3$ | $CH_3$ | $CH_3$ | $CH_3$ | $OCH_3$ | CH | S | |
| Q-10 | $CH_3$ | H | H | $OCH_3$ | $OCH_3$ | N | S | |
| Q-10 | H | H | H | $CH_3$ | $OC_2H_5$ | CH | S | |
| Q-10 | H | H | $CH_3$ | $CH_3$ | $CH_3$ | N | $NCH_3$ | |
| Q-10 | H | H | $CH_3$ | $CH_3$ | $OCH_3$ | CH | $NCH_3$ | |
| Q-10 | H | $CH_3$ | $CH_3$ | $OCH_3$ | $OCH_3$ | N | $NCH_3$ | |
| Q-10 | $CH_3$ | H | H | $OCH_3$ | $OCH_3$ | N | $NCH_3$ | |
| Q-11 | H | H | H | $CH_3$ | $CH_3$ | N | O | |

**Table 1b (continued)**

| Q | R | R3' | R4 | X | Y | Z | W" | m.p. °C |
|---|---|---|---|---|---|---|---|---|
| Q-11 | H | H | H | $CH_3$ | $CH_3$ | CH | O | 172.5-173 |
| Q-11 | H | H | H | $CH_3$ | $OCH_3$ | N | O | |
| Q-11 | H | H | H | $CH_3$ | $OCH_3$ | CH | O | 192(d) |
| Q-11 | H | H | H | $OCH_3$ | $OCH_3$ | CH | O | 157-158 |
| Q-11 | H | H | H | $OCH_3$ | $OCH_3$ | N | O | |
| Q-11 | H | H | H | $CH_3$ | $CH_3$ | CH | S | |
| Q-11 | H | H | H | $CH_3$ | $OCH_3$ | CH | S | |
| Q-11 | H | H | H | $CH_3$ | $OCH_3$ | CH | S | |
| Q-11 | H | H | H | $OCH_3$ | $OCH_3$ | CH | S | |
| Q-11 | H | H | H | $CH_3$ | $CH_3$ | CH | $NCH_3$ | |
| Q-11 | H | H | H | $CH_3$ | $OCH_3$ | CH | $NCH_3$ | |
| Q-11 | H | H | H | $OCH_3$ | $OCH_3$ | CH | $NCH_3$ | |
| Q-11 | H | H | H | $CH_3$ | $OCH_3$ | N | $NCH_3$ | |
| Q-11 | H | $CH_3$ | H | $CH_3$ | $OCH_3$ | N | O | |
| Q-11 | H | H | $CH_3$ | $OCH_3$ | $OCH_3$ | N | O | |
| Q-11 | H | $CH_3$ | $CH_3$ | $CH_3$ | $OCH_3$ | CH | O | |
| Q-11 | $CH_3$ | H | H | $CH_3$ | $OCH_3$ | CH | O | |
| Q-11 | H | H | H | $CH_3$ | $C_2H_5$ | N | O | |
| Q-11 | H | H | H | $CH_3$ | $CH_2OCH_3$ | CH | O | |
| Q-11 | H | $CH_3$ | H | $CH_3$ | $OCH_3$ | N | S | |
| Q-11 | H | H | $CH_3$ | $OCH_3$ | $OCH_3$ | N | S | |
| Q-11 | H | $CH_3$ | $CH_3$ | $CH_3$ | $OCH_3$ | CH | S | |
| Q-11 | $CH_3$ | H | H | $CH_3$ | $OCH_3$ | CH | S | |
| Q-11 | H | H | H | $CH_3$ | $C_2H_5$ | N | S | |
| Q-11 | $CH_3$ | $CH_3$ | $CH_3$ | $CH_3$ | $OCH_3$ | N | S | |
| Q-11 | H | H | H | Cl | $OCH_3$ | CH | S | |
| Q-11 | H | $CH_3$ | H | $CH_3$ | $CH_3$ | CH | $NCH_3$ | |
| Q-11 | H | H | $CH_3$ | $CH_3$ | $OCH_3$ | N | $NCH_3$ | |
| Q-11 | H | $CH_3$ | $CH_3$ | $CH_3$ | $OCH_3$ | CH | $NCH_3$ | |
| Q-11 | $CH_3$ | H | H | $OCH_3$ | $OCH_3$ | N | NH | |
| Q-11 | H | H | H | $CH_3$ | $OCH_3$ | CH | NH | |
| Q-11 | H | H | H | $OCH_3$ | $OCH_3$ | CH | O | * |
| Q-11 | H | H | H | $OCH_3$ | CH | N | O | * |

Table 1b (continued)

| Q | R | $R_3$' | $R_4$ | X | Y | Z | W" | m.p. °C |
|---|---|---|---|---|---|---|---|---|
| Q-11 | H | H | H | $CH_3$ | $OCH_3$ | N | $NCH_3$ | |
| Q-11 | H | H | H | $CH_3$ | $OCF_2H$ | CH | $NCH_3$ | |
| Q-11 | H | $CH_3$ | H | $OCH_3$ | $OCH_2CF_3$ | N | $NCH_3$ | |
| Q-12 | H | H | H | $CH_3$ | $OCH_3$ | CH | O | |
| Q-12 | H | H | H | $CH_3$ | $OCH_3$ | N | O | |
| Q-12 | H | H | H | $OCH_3$ | $OCH_3$ | CH | O | |
| Q-12 | H | H | H | $OCH_3$ | $OCH_3$ | N | O | |
| Q-12 | H | H | H | $CH_3$ | $OCH_3$ | CH | S | |
| Q-12 | H | H | H | $CH_3$ | $OCH_3$ | N | S | |
| Q-12 | H | H | H | $OCH_3$ | $OCH_3$ | CH | S | |
| Q-12 | H | H | H | $OCH_3$ | $OCH_3$ | N | S | |
| Q-12 | H | H | H | $CH_3$ | $CH_3$ | N | $NCH_3$ | |
| Q-12 | H | H | H | $CH_3$ | $OCH_3$ | CH | $NCH_3$ | |
| Q-12 | H | H | H | $CH_3$ | $OCH_3$ | N | $NCH_3$ | |
| Q-12 | H | H | H | $OCH_3$ | $OCH_3$ | CH | $NCH_3$ | |
| Q-12 | H | H | H | $OCH_3$ | $OCH_3$ | N | $NCH_3$ | |
| Q-12 | H | H | H | $OCH_3$ | $OCH_3$ | N | NH | |
| Q-12 | H | $CH_3$ | H | $CH_3$ | $CH_3$ | CH | O | |
| Q-12 | H | H | $CH_3$ | $CH_3$ | $OCH_3$ | N | O | |
| Q-12 | H | $CH_3$ | $CH_3$ | $CH_3$ | $OCH_3$ | CH | O | |
| Q-12 | $CH_3$ | H | H | $OCH_3$ | $OCH_3$ | N | O | |
| Q-12 | $CH_3$ | H | H | $CH_3$ | $CF_3$ | CH | O | |
| Q-12 | H | H | H | $OCH_3$ | $OCH_2CF_3$ | N | | |
| Q-12 | H | $CH_3$ | H | $CH_3$ | $OCH_3$ | CH | S | |
| Q-12 | H | H | $CH_3$ | $CH_3$ | $OCH_3$ | N | S | |
| Q-12 | H | $CH_3$ | $CH_3$ | $OCH_3$ | $OCH_3$ | CH | S | |
| Q-12 | $CH_3$ | H | H | $OCH_3$ | $OCH_3$ | N | S | |
| Q-12 | $CH_3$ | $CH_3$ | H | $CH_3$ | $NHCH_3$ | CH | S | |
| Q-12 | H | H | H | Br | $OC_2H_5$ | CH | S | |
| Q-12 | H | $CH_3$ | H | $CH_3$ | $CH_3$ | CH | NH | |
| Q-12 | H | $CH_3$ | H | $CH_3$ | $CH_3$ | CH | $NCH_3$ | |
| Q-12 | H | H | $CH_3$ | $CH_3$ | $OCH_3$ | N | $NCH_3$ | |
| Q-12 | H | $CH_3$ | $CH_3$ | $CH_3$ | $CH_3$ | $CH_3$ | $NCH_3$ | |
| Q-12 | $CH_3$ | $CH_3$ | $CH_3$ | $CH_3$ | $OCH_3$ | N | $NCH_3$ | |

Table 1b (continued)

| Q | R | $R_3$' | $R_4$ | X | Y | Z | W" | m.p. °C |
|---|---|---|---|---|---|---|---|---|
| Q-12 | $CH_3$ | $CH_3$ | $CH_3$ | $CH_3$ | $OCH_3$ | CH | $NCH_3$ | |
| Q-12 | H | H | H | Br | $OCH_3$ | CH | $NCH_3$ | |
| Q-12 | H | H | H | $OCH_3$ | $N(CH_3)_2$ | CH | $NCH_3$ | |
| Q-13 | H | H | H | $CH_3$ | $OCH_3$ | CH | O | |
| Q-13 | H | H | H | $CH_3$ | $OCH_3$ | CH | O | |
| Q-13 | H | H | H | $CH_3$ | $CH_3$ | CH | O | |
| Q-13 | H | H | H | $CH_3$ | $OCH_3$ | N | O | |
| Q-13 | H | H | H | $OCH_3$ | $OCH_3$ | CH | O | |
| Q-13 | H | H | H | $CH_3$ | $CH_3$ | N | O | |
| Q-13 | H | H | H | $OCH_3$ | $OCH_3$ | N | O | |
| Q-13 | H | H | H | $CH_3$ | $OCH_3$ | CH | S | |
| Q-13 | H | H | H | $CH_3$ | $OCH_3$ | N | S | |
| Q-13 | H | H | H | $OCH_3$ | $OCH_3$ | CH | S | |
| Q-13 | H | H | H | $OCH_3$ | $OCH_3$ | N | S | |
| Q-13 | H | H | H | $CH_3$ | $OCH_3$ | CH | NH | |
| Q-13 | H | H | H | $CH_3$ | $OCH_3$ | N | NH | |
| Q-13 | H | H | H | $OCH_3$ | $OCH_3$ | CH | $NCH_3$ | 150-152 |
| Q-13 | H | H | H | $OCH_3$ | $OCH_3$ | N | NH | |
| Q-13 | H | $CH_3$ | H | $CH_3$ | $CH_3$ | CH | O | |
| Q-13 | H | H | $CH_3$ | $CH_3$ | $CH_3$ | N | O | |
| Q-13 | H | $CH_3$ | $CH_3$ | $CH_3$ | $OCH_3$ | CH | O | |
| Q-13 | $CH_3$ | H | H | $CH_3$ | $OCH_3$ | N | O | |
| Q-13 | H | H | H | $CH_3$ | $CH(OCH_3)_2$ | CH | O | |
| Q-13 | H | $CH_3$ | H | $CH_3$ | $OCH_3$ | CH | S | |
| Q-13 | H | H | $CH_3$ | $CH_3$ | $OCH_3$ | CH | S | |
| Q-13 | H | $CH_3$ | $CH_3$ | $CH_3$ | $OCH_3$ | N | S | |
| Q-13 | $CH_3$ | H | $CH_3$ | $OCH_3$ | $OCH_3$ | N | S | |
| Q-13 | $CH_3$ | $CH_3$ | $CH_3$ | $CH_3$ | $CH_3$ | N | S | |
| Q-13 | H | H | H | $OCH_3$ | 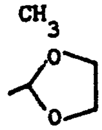 | CH | S | |
| Q-13 | H | $CH_3$ | H | $CH_3$ | $OCH_3$ | N | $NCH_3$ | |
| Q-13 | H | H | $CH_3$ | $CH_3$ | $OCH_3$ | N | $NCH_3$ | |
| Q-13 | H | $CH_3$ | $CH_3$ | $CH_3$ | $OCH_3$ | CH | $NCH_3$ | |

Table 1b (continued)

| Q | R | $R_{3}'$ | $R_4$ | X | Y | Z | W" | m.p. °C |
|---|---|---|---|---|---|---|---|---|
| Q-13 | $CH_3$ | H | H | $OCH_3$ | $OCH_3$ | CH | $NCH_3$ | |
| Q-13 | H | H | H | $CH_3$ | $OCH_3$ | N | NH | |
| Q-13 | H | H | H | Br | $OC_2H_5$ | CH | NH | |
| Q-13 | H | H | H | $OCH_3$ | $C_2H_5$ | N | NH | |
| Q-13 | H | H | H | $CH_3$ | $CH_3$ | CH | $NCH_3$ | |
| Q-13 | H | H | H | $CH_3$ | $CH_3$ | N | $NCH_3$ | |
| Q-13 | H | H | H | Cl | $OCH_3$ | CH | $NCH_3$ | |
| Q-14 | H | H | H | $CH_3$ | $CH_3$ | N | O | |
| Q-14 | H | H | H | $CH_3$ | $CH_3$ | CH | O | |
| Q-14 | H | H | H | $CH_3$ | $OCH_3$ | CH | O | |
| Q-14 | H | H | H | $CH_3$ | $OCH_3$ | CH | O | |
| Q-14 | H | H | H | $CH_3$ | $OCH_3$ | N | O | |
| Q-14 | H | H | H | $OCH_3$ | $OCH_3$ | CH | O | |
| Q-14 | H | H | H | $CH_3$ | $OCH_3$ | CH | S | |
| Q-14 | H | H | H | $CH_3$ | $OCH_3$ | N | S | |
| Q-14 | H | H | H | $OCH_3$ | $OCH_3$ | CH | S | |
| Q-14 | H | H | H | $OCH_3$ | $OCH_3$ | CH | S | |
| Q-14 | H | H | H | $OCH_3$ | $OCH_3$ | N | S | |
| Q-14 | H | H | H | $CH_3$ | $OCH_3$ | CH | $NCH_3$ | |
| Q-14 | H | H | H | $CH_3$ | $OCH_3$ | N | $NCH_3$ | |
| Q-14 | H | H | H | $OCH_3$ | $OCH_3$ | CH | NH | |
| Q-14 | H | H | H | $OCH_3$ | $OCH_3$ | N | $NCH_3$ | |
| Q-14 | H | $CH_3$ | H | $CH_3$ | $CH_3$ | CH | O | |
| Q-14 | H | H | $CH_3$ | $CH_3$ | $CH_3$ | N | O | |
| Q-14 | H | $CH_3$ | $CH_3$ | $CH_3$ | $OCH_3$ | CH | O | |
| Q-14 | $CH_3$ | $CH_3$ | $CH_3$ | $CH_3$ | $OCH_3$ | N | O | |
| Q-14 | H | H | H | $CH_3$ | $C_2H_5$ | N | O | |
| Q-14 | H | H | H | $CH_3$ | $OC_2H_5$ | N | O | |
| Q-14 | H | $CH_3$ | H | $CH_3$ | $OCH_3$ | CH | S | |
| Q-14 | H | H | $CH_3$ | $CH_3$ | $OCH_3$ | N | S | |
| Q-14 | H | $CH_3$ | $CH_3$ | $OCH_3$ | $OCH_3$ | CH | S | |
| Q-14 | $CH_3$ | $CH_3$ | $CH_3$ | $OCH_3$ | $OCH_3$ | CH | S | |
| Q-14 | H | H | H | $OCH_3$ | $C_2H_5$ | N | S | |
| Q-14 | H | H | H | $OCH_3$ | $OC_2H_5$ | N | S | |

Table 1b (continued)

| Q | R | $R_3'$ | $R_4$ | X | Y | Z | W" | m.p. °C |
|---|---|---|---|---|---|---|---|---|
| Q-14 | H | $CH_3$ | H | $CH_3$ | $OCH_3$ | CH | $NCH_3$ | |
| Q-14 | H | H | $CH_3$ | $CH_3$ | $OCH_3$ | N | $NCH_3$ | |
| Q-14 | H | $CH_3$ | $CH_3$ | $OCH_3$ | $OCH_3$ | CH | $NCH_3$ | |
| Q-14 | $CH_3$ | $CH_3$ | H | $OCH_3$ | $OCH_3$ | N | $NCH_3$ | |
| Q-14 | H | $CH_3$ | $CH_3$ | $CH_3$ | $OCH_3$ | CH | NH | |
| Q-14 | H | H | H | $CH_3$ | $OCH_3$ | N | NH | |
| Q-14 | H | H | H | $CH_3$ | $CH_2OCH_3$ | CH | $NCH_3$ | |
| Q-15 | H | H | H | $CH_3$ | $OCH_3$ | CH | O | |
| Q-15 | H | H | H | $CH_3$ | $OCH_3$ | N | O | |
| Q-15 | H | H | H | $OCH_3$ | $OCH_3$ | CH | O | |
| Q-15 | H | H | H | $OCH_3$ | $OCH_3$ | N | O | |
| Q-15 | H | $C_2H_5$ | H | $OCH_3$ | $OCH_3$ | CH | O | 134-136 |
| Q-15 | H | $CH_3$ | H | $CH_3$ | $OCH_3$ | CH | O | 150-152 |
| Q-15 | H | $CH_3$ | H | $CH_3$ | $CH_3$ | CH | O | 177-179 |
| Q-15 | H | $CH_3$ | H | $OCH_3$ | $OCH_3$ | CH | O | 133-134 |
| Q-15 | H | H | $CH_3$ | $CH_3$ | $CH_3$ | N | O | |
| Q-15 | H | $CH_3$ | H | $CH_3$ | $OCH_3$ | N | O | 158-160 |
| Q-15 | H | $CH_3$ | $CH_3$ | $CH_3$ | $OCH_3$ | CH | O | |
| Q-15 | H | $CH_3$ | H | $OCH_3$ | $OCH_3$ | N | O | 164-165 |
| Q-15 | $CH_3$ | H | H | $CH_3$ | $OCH_3$ | N | O | |
| Q-15 | H | $CH_3$ | H | $OCH_3$ | Cl | CH | O | 154-156 |
| Q-15 | $CH_3$ | $CH_3$ | $CH_3$ | $OCH_3$ | $OCH_3$ | CH | O | |
| Q-15 | H | H | H | $CH_3$ | $CF_3$ | CH | O | |
| Q-15 | H | H | H | $CH_3$ | $CF_3$ | N | O | |
| Q-15 | H | H | H | $OCH_3$ | $OCF_2H$ | CH | O | |
| Q-15 | $CH_3$ | H | H | $OCH_3$ | $OCF_2H$ | CH | O | |
| Q-15 | H | H | H | $CH_3$ | $CH_3$ | CH | O | |
| Q-15 | H | H | H | $CH_3$ | $CH_3$ | N | O | |
| Q-15 | H | H | H | $OCH_3$ | $OCH_3$ | CH | O * | |
| Q-15 | H | H | H | $OCH_3$ | $CH_3$ | N | O * | |
| Q-15 | H | H | H | $CH_3$ | $CH_3$ | N | S | |
| Q-15 | H | $CH_3$ | H | $CH_3$ | $OCH_3$ | CH | S | 146-149 |
| Q-15 | H | $CH_3$ | H | $CH_3$ | $OCH_3$ | N | S | 148-151 |

Table 1b (continued)

| Q | R | $R_3$, | $R_4$ | X | Y | Z | W" | m.p. °C |
|---|---|---|---|---|---|---|---|---|
| Q-15 | H | $CH_3$ | H | $OCH_3$ | $OCH_3$ | CH | S | 164-168 |
| Q-15 | H | $CH_3$ | H | $OCH_3$ | $OCH_3$ | N | S | 170-173 |
| Q-15 | H | $CH_3$ | H | Cl | $OCH_3$ | CH | S | 141-149 |
| Q-15 | H | H | H | $CH_3$ | $OCH_3$ | CH | $NCH_3$ | |
| Q-15 | H | H | H | $CH_3$ | $OCH_3$ | N | $NCH_3$ | |
| Q-15 | H | H | H | $OCH_3$ | $OCH_3$ | CH | $NCH_3$ | |
| Q-15 | H | H | H | $OCH_3$ | $OCH_3$ | N | $NCH_3$ | |
| Q-15 | H | $CH_3$ | H | $CH_3$ | $CH_3$ | CH | S | 172-175 |
| Q-15 | H | H | $CH_3$ | $CH_3$ | $CH_3$ | N | S | |
| Q-15 | H | $CH_3$ | $CH_3$ | $CH_3$ | $OCH_3$ | CH | S | |
| Q-15 | $CH_3$ | H | H | $CH_3$ | $OCH_3$ | N | S | |
| Q-15 | $CH_3$ | H | $CH_3$ | $OCH_3$ | $OCH_3$ | CH | S | |
| Q-15 | H | H | H | Cl | $OC_2H_5$ | CH | S | |
| Q-15 | H | H | H | $OCH_3$ | $OCH_2CF_3$ | CH | S | |
| Q-15 | H | $CH_3$ | H | $CH_3$ | $CH_3$ | CH | $NCH_3$ | |
| Q-15 | H | H | $CH_3$ | $CH_3$ | $OCH_3$ | CH | $NCH_3$ | |
| Q-15 | H | $CH_3$ | $CH_3$ | $CH_3$ | $OCH_3$ | N | $NCH_3$ | |
| Q-15 | $CH_3$ | $CH_3$ | $CH_3$ | $CH_3$ | $OCH_3$ | N | $NCH_3$ | |
| Q-15 | $CH_3$ | H | H | $CH_3$ | $OCH_3$ | CH | NH | |
| Q-15 | H | H | H | $OCH_3$ | $OCH_3$ | N | NH | |
| Q-15 | H | H | H | $CH_3$ | $NHCH_3$ | CH | $NCH_3$ | |
| Q-15 | H | H | H | $OCH_3$ | $N(CH_3)_2$ | CH | $NCH_3$ | |
| Q-15 | H | H | H | $CH_3$ | $OCH_2C\equiv CH$ | N | S | |
| Q-15 | H | H | H | $CH_3$ | $CH_2C\equiv CH_3$ | CH | S | |
| Q-15 | H | H | H | $CH_3$ | $C\equiv CH$ | N | S | |
| Q-15 | H | $C_2H_5$ | H | $OCH_3$ | $OCH_3$ | CH | S | 127-129 |
| Q-15 | H | H | H | $OCH_3$ | $(CH_2)_3CH_2F$ | CH | S | |
| Q-15 | H | H | H | $OCH_3$ | 2-methyl-1,3-dioxolan-2-yl | N | S | |
| Q-15 | H | H | H | $OCH_3$ | 2-methyl-1,3-dithiolan-2-yl | CH | S | |
| Q-15 | H | H | H | $OCH_3$ | 2-methyl-1,3-dithian-2-yl | N | S | |
| Q-15 | H | H | H | $OCH_3$ | $OCH_3$ | CH | S * | |
| Q-15 | H | H | H | $OCH_3$ | $CH_3$ | N | S * | |

Table Ic

General Structure 1

| Q | R | $R_3$ | $R_4$ | $R_5$ | X | Y | Z | m.p. °C |
|---|---|---|---|---|---|---|---|---|
| Q-18 | H | H | H | H | $CH_3$ | $CH_3$ | CH | 188-190 |
| Q-18 | H | H | H | H | $CH_3$ | $OCH_3$ | CH | 159-163 |
| Q-18 | H | H | H | H | $CH_3$ | $OCH_3$ | N | 133-136 |
| Q-18 | $CH_3$ | H | H | H | $CH_3$ | $OCH_3$ | N | |
| Q-18 | H | H | H | H | $OCH_3$ | $OCH_3$ | CH | 192-194 |
| Q-18 | H | H | H | H | $CH_3$ | $CH_3$ | CH | 165-167 |
| Q-18 | H | H | H | H | $OCH_3$ | $OCH_3$ | N | 163-166 |
| Q-18 | H | H | H | H | Cl | $OCH_3$ | CH | |
| Q-18 | H | $CH_3$ | H | H | $CH_3$ | $OCH_3$ | CH | |
| Q-18 | H | H | $CH_3$ | H | $CH_3$ | $OCH_3$ | N | |
| Q-18 | H | H | H | $CH_3$ | $CH_3$ | $CH_3$ | CH | |
| Q-18 | H | $CH_3$ | $CH_3$ | H | $CH_3$ | $CH_3$ | N | |
| Q-18 | H | H | $CH_3$ | $CH_3$ | $OCH_3$ | $OCH_3$ | CH | |
| Q-18 | H | $CH_3$ | H | $CH_3$ | $OCH_3$ | $OCH_3$ | N | |
| Q-18 | H | $CH_3$ | $CH_3$ | $CH_3$ | $CH_3$ | $OCH_3$ | CH | |
| Q-18 | H | H | H | H | $OCH_3$ | $CH(OCH_3)_2$ | CH | |
| Q-19 | $CH_3$ | H | H | H | Cl | $OCH_3$ | CH | |
| Q-19 | H | H | H | H | $CH_3$ | $OCH_3$ | CH | |
| Q-19 | H | H | H | H | $CH_3$ | $OCH_3$ | N | |
| Q-19 | H | H | H | H | $OCH_3$ | $OCH_3$ | CH | |
| Q-19 | H | H | H | H | $OCH_3$ | $OCH_3$ | N | |
| Q-19 | H | $CH_3$ | H | H | $CH_3$ | $CH_3$ | CH | |
| Q-19 | H | H | $CH_3$ | H | $CH_3$ | $CH_3$ | N | |
| Q-19 | H | H | H | $CH_3$ | $CH_3$ | $OCH_3$ | CH | |
| Q-19 | H | $CH_3$ | $CH_3$ | H | $CH_3$ | $OCH_3$ | N | |
| Q-19 | H | H | $CH_3$ | $CH_3$ | $OCH_3$ | $OCH_3$ | CH | |
| Q-19 | H | $CH_3$ | H | $CH_3$ | $OCH_3$ | $OCH_3$ | N | |
| Q-19 | H | H | H | H | $CH_3$ | $CH_3$ | CH | |
| Q-19 | H | $CH_3$ | $CH_3$ | $CH_3$ | $CH_3$ | $OCH_3$ | CH | |
| Q-19 | $CH_3$ | H | H | H | $CH_3$ | $OCH_3$ | N | |
| Q-19 | H | H | H | H | $OCH_3$ | 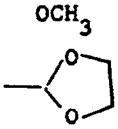 | CH | |

Table 1d

General Structure 1

| Q | R | $R_3'$," | X | Y | Z | W' | m.p. °C |
|---|---|---|---|---|---|---|---|
| Q-21 | H | H | $CH_3$ | $OCH_3$ | CH | O | |
| Q-21 | H | H | $CH_3$ | $OCH_3$ | N | O | |
| Q-21 | H | H | $OCH_3$ | $OCH_3$ | CH | O | |
| Q-21 | $CH_3$ | H | $OCH_3$ | $OCH_3$ | CH | O | |
| Q-21 | H | H | $OCH_3$ | $NHCH_3$ | CH | O | |
| Q-21 | H | H | $OCH_3$ | $OCH_3$ | N | O | |
| Q-21 | H | $CH_3$ | $CH_3$ | $OCH_3$ | CH | O | |
| Q-21 | $CH_3$ | $CH_3$ | $CH_3$ | $OCH_3$ | CH | O | |
| Q-21 | H | H | $CH_3$ | $CH_3$ | CH | O | |
| Q-21 | H | H | $CH_3$ | $CH_3$ | N | O | |
| Q-21 | H | H | $CH_3$ | $OCH_3$ | CH | S | |
| Q-21 | H | H | $CH_3$ | $OCH_3$ | N | S | |
| Q-21 | H | H | $OCH_3$ | $OCH_3$ | CH | S | |
| Q-21 | H | H | $OCH_3$ | $OCH_3$ | N | S | |
| Q-21 | H | $CH_3$ | $CH_3$ | $OCH_3$ | CH | S | |
| Q-21 | $CH_3$ | H | $CH_3$ | $OCH_3$ | CH | S | |
| Q-21 | H | H | $OCH_3$ | $N(CH_3)_2$ | N | S | |
| Q-22 | H | H | $CH_3$ | $OCH_3$ | CH | O | |
| Q-22 | H | H | $CH_3$ | $OCH_3$ | N | O | |
| Q-22 | H | H | $OCH_3$ | $OCH_3$ | CH | O | |
| Q-22 | H | H | $OCH_3$ | $OCH_3$ | N | O | |
| Q-22 | H | $CH_3$ | $CH_3$ | $OCH_3$ | CH | O | |
| Q-22 | H | $CH_3$ | $CH_3$ | $OCH_3$ | N | O | |
| Q-22 | $CH_3$ | $CH_3$ | $CH_3$ | $CH_3$ | CH | O | |
| Q-22 | H | H | $CH_3$ | $CH(OCH_3)_2$ | CH | O | |
| Q-22 | H | H | $CH_3$ | $C_2H_5$ | N | O | |
| Q-22 | H | H | $CH_3$ | $CH_3$ | CH | S | |
| Q-22 | H | H | $CH_3$ | $CH_3$ | N | S | |
| Q-22 | H | H | $CH_3$ | $OCH_3$ | CH | S | |
| Q-22 | H | H | $CH_3$ | $OCH_3$ | N | S | |

$R_{11}$ is -, unless indicated by ** where $R_{11}$ is $CH_3$ in all Tables.

Table 1d (continued)

| Q | R | $R_3'$," | X | Y | Z | W' | m.p. °C |
|---|---|---|---|---|---|---|---|
| Q-22 | H | H | $OCH_3$ | $OCH_3$ | CH | S | |
| Q-22 | H | H | $OCH_3$ | $OCH_3$ | N | S | |
| Q-22 | H | $CH_3$ | $CH_3$ | $OCH_3$ | CH | S | |
| Q-22 | $CH_3$ | $CH_3$ | $CH_3$ | $OCH_3$ | CH | S | |
| Q-22 | $CH_3$ | H | $CH_3$ | $OCH_3$ | N | S | |
| Q-22 | H | H | $CH_3$ | 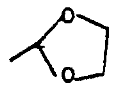 | CH | S | |
| Q-22 | H | H | $OCH_3$ | $C_2H_5$ | N | S | |
| Q-23 | H | H | $CH_3$ | $OCH_3$ | CH | - | |
| Q-23 | H | H | $CH_3$ | $OCH_3$ | N | - | |
| Q-23 | H | H | $OCH_3$ | $OCH_3$ | CH | - | |
| Q-23 | H | H | $OCH_3$ | $OCH_3$ | N | - | |
| Q-23 | H | $CH_3$ | $CH_3$ | $CH_3$ | CH | - | |
| Q-23 | H | $CH_3$ | $CH_3$ | $OCH_3$ | N | - | |
| Q-23 | $CH_3$ | H | $CH_3$ | $OCH_3$ | N | - | |
| Q-23 | $CH_3$ | $CH_3$ | $CH_3$ | $OCH_3$ | CH | - | |
| Q-23 | H | H | $CH_3$ | $OC_2H_5$ | N | - | |
| Q-23 | H | H | $CH_3$ | $CH_2OCH_3$ | CH | - | |
| Q-24 | H | H | $CH_3$ | $OCH_3$ | CH | - | |
| Q-24 | H | H | $CH_3$ | $OCH_3$ | N | - | |
| Q-24 | H | H | $OCH_3$ | $OCH_3$ | CH | - | |
| Q-24 | H | H | $OCH_3$ | $OCH_3$ | N | - | |
| Q-24 | H | $CH_3$ | $CH_3$ | $OCH_3$ | CH | - | |
| Q-24 | H | $CH_3$ | $CH_3$ | $CH_3$ | CH | - | |
| Q-24 | $CH_3$ | H | $CH_3$ | $CH_3$ | N | - | |
| Q-24 | H | H | $CH_3$ | $CF_3$ | CH | - | |
| Q-25 | H | H | $CH_3$ | $OCH_3$ | CH | - | ** |
| Q-25 | H | H | $CH_3$ | $OCH_3$ | N | - | ** |
| Q-25 | H | H | $OCH_3$ | $OCH_3$ | CH | - | ** |

Table 1d (continued)

| Q | R | $R_3'$," | X | Y | Z | W' | m.p. °C |
|---|---|---|---|---|---|---|---|
| Q-25 | H | H | $OCH_3$ | $OCH_3$ | N | - | ** |
| Q-25 | $CH_3$ | H | $OCH_3$ | $OCH_3$ | N | - | ** |
| Q-25 | H | H | $CH_3$ | $CH_3$ | CH | - | ** |
| Q-25 | H | H | $CH_3$ | $CH_3$ | N | - | ** |
| Q-25 | H | $CH_3$ | $CH_3$ | $OCH_3$ | CH | - | ** |
| Q-25 | H | $CH_3$ | $CH_3$ | $OCH_3$ | N | - | ** |
| Q-25 | $CH_3$ | $CH_3$ | $OCH_3$ | $OCH_3$ | N | - | ** |
| Q-25 | H | H | $CH_3$ | $OCH_2CF_3$ | CH | - | ** |
| Q-25 | H | H | $CH_3$ | $NHCH_3$ | N | - | ** |
| Q-26 | H | H | $CH_3$ | $OCH_3$ | CH | - | ** |
| Q-26 | H | H | $CH_3$ | $OCH_3$ | N | - | ** |
| Q-26 | H | H | $OCH_3$ | $OCH_3$ | CH | - | ** |
| Q-26 | H | $CH_3$ | $CH_3$ | $CH_3$ | N | - | ** |
| Q-26 | H | $CH_3$ | $CH_3$ | $CH_3$ | CH | - | ** |
| Q-26 | H | $CH_3$ | $CH_3$ | $OCH_3$ | N | - | ** |
| Q-26 | H | $CH_3$ | $CH_3$ | $OCH_3$ | CH | - | ** |
| Q-26 | H | $CH_3$ | $CH_3$ | $OCH_3$ | N | - | ** |
| Q-26 | H | H | $CH_3$ | $CH_3$ | CH | - | ** |
| Q-26 | H | H | $CH_3$ | $CH_3$ | N | - | ** |
| Q-26 | H | $CH_3$ | $OCH_3$ | $OCH_3$ | CH | - | ** |
| Q-26 | H | $CH_3$ | $OCH_3$ | $OCH_3$ | N | - | ** |
| Q-26 | $CH_3$ | H | $CH_3$ | $OCH_3$ | CH | - | ** |
| Q-26 | H | H | $CH_3$ | $N(CH_3)_2$ | CH | - | ** |
| Q-26 | H | H | $CH_3$ | $N(CH_3)_2$ | N | - | ** |
| Q-27 | H | H | $CH_3$ | $OCH_3$ | CH | - | ** |
| Q-27 | H | H | $CH_3$ | $OCH_3$ | N | - | ** |
| Q-27 | H | H | $OCH_3$ | $OCH_3$ | CH | - | ** |
| Q-27 | H | H | $OCH_3$ | $OCH_3$ | N | - | ** |
| Q-27 | H | H | $CH_3$ | $CH_3$ | CH | - | ** |
| Q-27 | H | H | $CH_3$ | $CH_3$ | N | - | ** |
| Q-27 | H | $CH_3$ | $CH_3$ | $CH_3$ | CH | - | ** |

Table 1d (continued)

| Q | R | $R_3'$," | X | Y | Z | W' | m.p. °C |
|---|---|---|---|---|---|---|---|
| Q-27 | H | H | Cl | $OC_2H_5$ | CH | - | ** |
| Q-27 | H | $CH_3$ | $CH_3$ | $CH_3$ | N | - | ** |
| Q-27 | H | $CH_3$ | $CH_3$ | $OCH_3$ | CH | - | ** |
| Q-27 | H | $CH_3$ | $CH_3$ | $OCH_3$ | N | - | ** |
| Q-27 | H | $CH_3$ | $OCH_3$ | $OCH_3$ | CH | - | ** |
| Q-27 | H | $CH_3$ | $OCH_3$ | $OCH_3$ | N | - | ** |
| Q-27 | $CH_3$ | $CH_3$ | $CH_3$ | $OCH_3$ | N | - | ** |
| Q-27 | $CH_3$ | H | $CH_3$ | $OCH_3$ | N | - | ** |
| Q-27 | H | H | $CH_3$ | $CH(OCH_3)_2$ | CH | - | ** |
| Q-27 | H | H | $CH_3$ | $O(CH_2)_3CH_2Cl$ | CH | - | ** |
| Q-25 | H | SH | $CH_3$ | $CH_3$ | CH | - | ** |
| Q-25 | H | SH | $CH_3$ | $OCH_3$ | CH | - | ** |
| Q-25 | H | SH | $OCH_3$ | $OCH_3$ | CH | - | ** |
| Q-25 | H | SH | $OCH_3$ | Cl | CH | - | ** |
| Q-25 | H | SH | $CH_3$ | $OCH_3$ | N | - | ** |
| Q-25 | H | SH | $OCH_3$ | $OCH_3$ | N | - | ** |
| Q-25 | H | $SCH_3$ | $CH_3$ | $CH_3$ | CH | - | ** |
| Q-25 | H | $SCH_3$ | $CH_3$ | $OCH_3$ | CH | - | ** |
| Q-25 | H | $SCH_3$ | $OCH_3$ | $OCH_3$ | CH | - | ** |
| Q-25 | H | $SCH_3$ | $OCH_3$ | Cl | CH | - | ** |
| Q-25 | H | $SCH_3$ | $CH_3$ | $OCH_3$ | N | - | ** |
| Q-25 | H | $SCH_3$ | $OCH_3$ | $OCH_3$ | N | - | ** |
| Q-25 | H | $SCH_2CH=CH_2$ | $CH_3$ | $CH_3$ | CH | - | ** |
| Q-25 | H | $SCH_2CH=CH_2$ | $CH_3$ | $OCH_3$ | CH | - | ** |
| Q-25 | H | $SCH_2CH=CH_2$ | $OCH_3$ | $OCH_3$ | CH | - | ** |
| Q-25 | H | $SCH_2CH=CH_2$ | $OCH_3$ | Cl | CH | - | ** |
| Q-25 | H | $SCH_2CH=CH_2$ | $CH_3$ | $OCH_3$ | N | - | ** |
| Q-25 | H | $SCH_2CH=CH_2$ | $OCH_3$ | $OCH_3$ | N | - | ** |
| Q-25 | $CH_3$ | $SCH_3$ | $OCH_3$ | $OCH_3$ | CH | - | ** |
| Q-25 | $CH_3$ | SH | $OCH_3$ | $OCH_3$ | CH | - | ** |
| Q-25 | $CH_3$ | $SCH_2CH=CH_2$ | $OCH_3$ | $OCH_3$ | CH | - | ** |

Table 1e

| Q | R | $R_3$ | $R_4$ | $R_5$ | X | Y | Z | m.p. °C |
|---|---|---|---|---|---|---|---|---|
| Q-37 | H | H | H | - | $CH_3$ | $OCH_3$ | N | |
| Q-37 | H | H | H | - | $OCH_3$ | $OCH_3$ | CH | |
| Q-37 | H | H | H | - | $OCH_3$ | $OCH_3$ | N | |
| Q-37 | H | H | H | - | $CH_3$ | $CH_3$ | CH | |
| Q-37 | H | 2-$CH_3$ | H | - | $CH_3$ | $CH_3$ | CH | |
| Q-37 | H | 4-$CH_3$ | H | - | $CH_3$ | $CH_3$ | N | |
| Q-37 | H | 5-$CH_3$ | H | - | $CH_3$ | OCH | CH | |
| Q-37 | H | H | H | - | $CH_3$ | $CH_3$ | N | |
| Q-37 | H | 2-$CH_3$ | 4-$CH_3$ | - | $CH_3$ | $OCH_3$ | N | |
| Q-37 | H | 2-$CH_3$ | 5-$CH_3$ | - | $OCH_3$ | $OCH_3$ | CH | |
| Q-37 | H | 4-$CH_3$ | 5-$CH_3$ | - | $OCH_3$ | $OCH_3$ | N | |
| Q-37 | $CH_3$ | H | H | - | $CH_3$ | $OCH_3$ | CH | |
| Q-37 | H | H | H | - | $CH_3$ | $CH(OCH_3)_2$ | CH | |
| Q-37 | H | H | H | - | Cl | $OCH_3$ | CH | |
| Q-38 | H | H | H | - | $CH_3$ | $OCH_3$ | CH | |
| Q-38 | H | H | H | - | $CH_3$ | $OCH_3$ | N | |
| Q-38 | H | H | H | - | $OCH_3$ | $OCH_3$ | CH | |
| Q-38 | H | H | H | - | $OCH_3$ | $OCH_3$ | N | |
| Q-38 | H | 2-$CH_3$ | H | - | $CH_3$ | $CH_3$ | CH | |
| Q-38 | H | 4-$CH_3$ | H | - | $CH_3$ | $CH_3$ | N | |
| Q-38 | H | 2-$CH_3$ | 4-$CH_3$ | - | $CH_3$ | $OCH_3$ | CH | |
| Q-38 | H | 4-$CH_3$ | 4-$CH_3$ | - | $CH_3$ | $OCH_3$ | N | |
| Q-38 | H | H | H | - | $CH_3$ | $CH_3$ | N | |
| Q-38 | $CH_3$ | 4-$CH_3$ | 6-$CH_3$ | - | $OCH_3$ | $OCH_3$ | CH | |
| Q-38 | H | H | H | - | $CH_3$ | 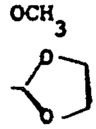 | CH | |
| Q-38 | H | H | H | - | $CH_3$ | $CH_3$ | CH | |
| Q-38 | H | H | H | - | $OCH_3$ | $C_2H_5$ | N | |
| Q-39 | H | H | H | - | $CH_3$ | $OCH_3$ | CH | |
| Q-39 | H | H | H | - | $CH_3$ | $OCH_3$ | N | |
| Q-39 | H | H | H | - | $CH_3$ | $CH_3$ | N | |

Table 1e (continued)

| Q | R | $R_3$ | $R_4$ | $R_5$ | X | Y | Z | m.p. °C |
|---|---|---|---|---|---|---|---|---|
| Q-39 | H | H | H | - | $OCH_3$ | $OCH_3$ | CH | |
| Q-39 | H | H | H | - | $OCH_3$ | $OCH_3$ | N | |
| Q-39 | $CH_3$ | H | H | - | $OCH_3$ | $OCH_3$ | N | |
| Q-39 | H | 3-$CH_3$ | H | - | $CH_3$ | $CH_3$ | CH | |
| Q-39 | H | H | H | - | $CH_3$ | $CH_3$ | CH | |
| Q-39 | H | 5-$CH_3$ | H | - | $CH_3$ | $CH_3$ | N | |
| Q-39 | H | H | H | - | Cl | $OC_2H_5$ | CH | |
| Q-39 | H | 6-$CH_3$ | H | - | $CH_3$ | $OCH_3$ | CH | |
| Q-39 | H | 3-$CH_3$ | 5-$CH_3$ | - | $CH_3$ | $OCH_3$ | N | |
| Q-39 | H | 3-$CH_3$ | 6-$CH_3$ | - | $OCH_3$ | $OCH_3$ | CH | |
| Q-39 | H | 5-$CH_3$ | 6-$CH_3$ | - | $OCH_3$ | $OCH_3$ | N | |
| Q-39 | H | H | H | - | $OCH_3$ | $OC_2H_5$ | N | |
| Q-39 | H | H | H | - | $OCH_3$ | $CH_2OCH_3$ | N | |
| Q-40 | H | H | H | - | $CH_3$ | $CH_3$ | CH | |
| Q-40 | H | H | H | - | $CH_3$ | $OCH_3$ | CH | |
| Q-40 | H | H | H | - | $CH_3$ | $OCH_3$ | N | |
| Q-40 | H | H | H | - | $OCH_3$ | $OCH_3$ | CH | |
| Q-40 | H | H | H | - | $OCH_3$ | $OCH_3$ | N | |
| Q-40 | H | H | H | - | $CH_3$ | $CH_3$ | N | |
| Q-40 | H | 4-$CH_3$ | H | - | $CH_3$ | $CH_3$ | CH | |
| Q-40 | H | 5-$CH_3$ | H | - | $CH_3$ | $CH_3$ | N | |
| Q-40 | H | 6-$CH_3$ | H | - | $CH_3$ | $OCH_3$ | CH | |
| Q-40 | H | 4-$CH_3$ | 5-$CH_3$ | - | $CH_3$ | $OCH_3$ | N | |
| Q-40 | H | 4-$CH_3$ | 6-$CH_3$ | - | $OCH_3$ | $OCH_3$ | CH | |
| Q-40 | H | 5-$CH_3$ | 6-$CH_3$ | - | $OCH_3$ | $OCH_3$ | N | |
| Q-40 | $CH_3$ | H | H | - | $CH_3$ | $CH_3$ | CH | |
| Q-40 | H | H | H | - | $OCH_3$ | $CF_3$ | N | |
| Q-40 | H | H | H | - | $OCH_3$ | $OCF_2H$ | N | |
| Q-43 | H | H | H | - | $CH_3$ | $CH_3$ | CH | |
| Q-43 | H | H | H | - | $CH_3$ | $OCH_3$ | CH | |
| Q-43 | H | H | H | - | $CH_3$ | $OCH_3$ | N | |

Table Ie(continued)

| Q | R | $R_3$ | $R_4$ | $R_5$ | X | Y | Z | m.p. °C |
|---|---|---|---|---|---|---|---|---|
| Q-43 | H | H | H | - | $OCH_3$ | $OCH_3$ | CH | |
| Q-43 | H | H | H | - | $OCH_3$ | $OCH_3$ | N | |
| Q-43 | $CH_3$ | H | H | - | $OCH_3$ | $OCH_3$ | CH | |
| Q-43 | H | $CH_3$ | $CH_3$ | - | $CH_3$ | $OCH_3$ | CH | |
| Q-43 | H | $CH_3$ | H | - | $CH_3$ | $CH_3$ | CH | |
| Q-43 | H | H | H | - | $CH_3$ | $CH_3$ | N | |
| Q-43 | H | H | H | - | $OCH_3$ | $OCH_2CF_3$ | N | |
| Q-43 | H | H | H | - | $OCH_3$ | $NHCH_3$ | N | |
| Q-44 | H | H | H | - | $CH_3$ | $OCH_3$ | CH | |
| Q-44 | H | H | H | - | $CH_3$ | $OCH_3$ | N | |
| Q-44 | H | H | H | - | $OCH_3$ | $OCH_3$ | CH | |
| Q-44 | H | H | H | - | $OCH_3$ | $OCH_3$ | N | |
| Q-44 | H | $CH_3$ | H | - | $CH_3$ | $CH_3$ | CH | |
| Q-44 | H | H | $CH_3$ | - | $CH_3$ | $CH_3$ | N | |
| Q-44 | H | $CH_3$ | $CH_3$ | - | $CH_3$ | $OCH_3$ | N | |
| Q-44 | $CH_3$ | H | H | - | $CH_3$ | $OCH_3$ | N | |
| Q-44 | H | H | H | - | $OCH_3$ | $N(CH_3)_2$ | N | |
| Q-44 | H | H | H | - | Br | $OCH_3$ | CH | |
| Q-44 | H | H | H | - | $CH_3$ | $CH_3$ | CH | |
| Q-44 | H | H | H | - | $CH_3$ | $CH_3$ | N | |
| Q-45 | H | H | - | - | $CH_3$ | $OCH_3$ | CH | |
| Q-45 | H | H | - | - | $CH_3$ | $OCH_3$ | N | |
| Q-45 | H | H | - | - | $OCH_3$ | $OCH_3$ | CH | |
| Q-45 | H | H | - | - | $OCH_3$ | $OCH_3$ | N | |
| Q-45 | H | $CH_3$ | - | - | $CH_3$ | $CH_3$ | CH | |
| Q-45 | $CH_3$ | H | - | - | $CH_3$ | $CH_3$ | N | |
| Q-45 | H | H | - | - | $OCH_3$ | $CH(OCH_3)_2$ | N | |
| Q-45 | H | H | - | - | $CH_3$ | $CH_3$ | CH | |
| Q-45 | H | H | - | - | $CH_3$ | $CH_3$ | N | |
| Q-45 | H | H | - | - | Cl | $OCH_3$ | CH | |

Table 2a

General Structure 1 wherein Q is Q-5.

| R | $R_3$ | $R_4$ | $R_5$ | $R_6$ | $R_7$ | $R_8$ | X | Y | Z | m.p. °C |
|---|---|---|---|---|---|---|---|---|---|---|
| H | H | H | H | H | H | H | $CH_3$ | $CH_3$ | N | |
| H | H | H | H | H | H | H | $CH_3$ | $OCH_3$ | CH | |
| H | H | H | H | H | H | H | $CH_3$ | $OCH_3$ | N | |
| H | H | H | H | H | H | H | $OCH_3$ | $OCH_3$ | CH | |
| H | H | H | H | H | H | H | $OCH_3$ | $OCH_3$ | N | |
| $CH_3$ | H | H | H | H | H | H | $OCH_3$ | $OCH_3$ | CH | |
| H | $CH_3$ | H | H | H | H | H | $CH_3$ | $CH_3$ | CH | |
| H | H | H | $CH_3$ | H | H | H | $CH_3$ | $CH_3$ | N | |
| H | H | H | H | H | $CH_3$ | H | $CH_3$ | $OCH_3$ | CH | |
| H | $CH_3$ | $CH_3$ | H | H | $CH_3$ | H | $CH_3$ | $OCH_3$ | N | |
| H | $CH_3$ | H | $CH_3$ | H | H | H | $OCH_3$ | $OCH_3$ | CH | |
| H | $CH_3$ | H | H | H | $CH_3$ | H | $OCH_3$ | $OCH_3$ | N | |
| H | H | H | $CH_3$ | H | $CH_3$ | H | $CH_3$ | $CH_3$ | CH | |
| H | $CH_3$ | H | $CH_3$ | H | $CH_3$ | H | $CH_3$ | $CH_3$ | N | |
| H | $CH_3$ | $CH_3$ | H | H | H | H | $CH_3$ | $OCH_3$ | CH | |
| H | H | H | H | $CH_3$ | H | H | $CH_3$ | $OCH_3$ | N | |
| H | H | H | H | H | $CH_3$ | $CH_3$ | $OCH_3$ | $OCH_3$ | CH | |
| H | $CH_3$ | $CH_3$ | $CH_3$ | H | H | H | $OCH_3$ | $OCH_3$ | N | |
| H | $CH_3$ | $CH_3$ | H | H | $CH_3$ | H | $CH_3$ | $CH_3$ | CH | |
| H | H | H | $CH_3$ | $CH_3$ | $CH_3$ | H | $CH_3$ | $CH_3$ | N | |
| H | $CH_3$ | H | $CH_3$ | $CH_3$ | $CH_3$ | H | $CH_3$ | $OCH_3$ | CH | |
| H | $CH_3$ | $CH_3$ | $CH_3$ | H | $CH_3$ | H | $CH_3$ | $OCH_3$ | N | |
| H | $CH_3$ | H | $CH_3$ | H | $CH_3$ | $CH_3$ | $OCH_3$ | $OCH_3$ | CH | |
| H | $CH_3$ | H | $CH_3$ | $CH_3$ | $CH_3$ | $CH_3$ | $CH_3$ | $CH_3$ | CH | |
| H | $CH_3$ | H | $CH_3$ | $CH_3$ | $CH_3$ | $CH_3$ | $CH_3$ | $CH_3$ | CH | |
| H | $CH_3$ | $CH_3$ | $CH_3$ | $CH_3$ | $CH_3$ | H | $CH_3$ | $CH_3$ | N | |
| H | $CH_3$ | $CH_3$ | $CH_3$ | $CH_3$ | $CH_3$ | $CH_3$ | $CH_3$ | $OCH_3$ | CH | |
| $CH_3$ | H | H | H | H | H | H | $CH_3$ | $OCH_3$ | N | |

Wherein W" is O.

Table 2a (continued)

| R | $R_3$ | $R_4$ | $R_5$ | $R_6$ | $R_7$ | $R_8$ | X | Y | Z | m.p. °C |
|---|---|---|---|---|---|---|---|---|---|---|
| H | H | H | H | H | H | H | $OCH_3$ | $CH(OCH_3)_2$ | N | |
| H | H | H | H | H | H | H | $OCH_3$ | 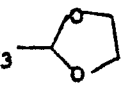 | N | |
| H | H | H | H | H | H | H | Cl | $OCH_3$ | CH | |
| H | H | H | H | H | H | H | $CH_3$ | $CH_3$ | CH | |

wherein W" is O

**Table 2b**

**General Structure 1 wherein Q is Q-6.**

| Q | R | $R_3$ | $R_4$ | $R_5$ | $R_6$ | $R_7$ | $R_8$ | X | Y | Z | W" | m.p. °C |
|---|---|---|---|---|---|---|---|---|---|---|---|---|
| Q-6 | H | H | H | H | H | H | H | $CH_3$ | $CH_3$ | CH | O | |
| Q-6 | H | H | H | H | H | H | H | $CH_3$ | $CH_3$ | N | O | |
| Q-6 | H | $CH_3$ | H | H | H | H | H | $CH_3$ | $OCH_3$ | CH | O | |
| Q-6 | H | H | H | $CH_3$ | H | H | H | $CH_3$ | $OCH_3$ | N | O | |
| Q-6 | H | H | H | H | H | $CH_3$ | H | $OCH_3$ | $OCH_3$ | CH | O | |
| Q-6 | H | $CH_3$ | H | $CH_3$ | H | H | H | $OCH_3$ | $OCH_3$ | N | O | |
| Q-6 | H | $CH_3$ | H | H | H | $CH_3$ | H | $CH_3$ | $CH_3$ | CH | O | |
| Q-6 | H | H | H | $CH_3$ | H | $CH_3$ | H | $CH_3$ | $CH_3$ | N | O | |
| Q-6 | H | $CH_3$ | $CH_3$ | H | H | H | H | $CH_3$ | $OCH_3$ | CH | O | |
| Q-6 | H | H | H | $CH_3$ | $CH_3$ | H | H | $CH_3$ | $OCH_3$ | N | O | |
| Q-6 | H | H | H | H | H | $CH_3$ | $CH_3$ | $OCH_3$ | $OCH_3$ | CH | O | |
| Q-6 | H | $CH_3$ | H | H | H | $CH_3$ | $CH_3$ | $OCH_3$ | $OCH_3$ | N | O | |
| Q-6 | H | $CH_3$ | $CH_3$ | H | H | $CH_3$ | H | $CH_3$ | $CH_3$ | CH | O | |
| Q-6 | H | $CH_3$ | H | $CH_3$ | $CH_3$ | H | H | $CH_3$ | $CH_3$ | N | O | |
| Q-6 | H | H | H | $CH_3$ | $CH_3$ | $CH_3$ | H | $CH_3$ | $OCH_3$ | CH | O | |
| Q-6 | H | $CH_3$ | $CH_3$ | $CH_3$ | H | H | H | $CH_3$ | $OCH_3$ | N | O | |
| Q-6 | H | H | H | $CH_3$ | $CH_3$ | $CH_3$ | H | $OCH_3$ | $OCH_3$ | CH | O | |
| Q-6 | H | $CH_3$ | H | $CH_3$ | $CH_3$ | $CH_3$ | H | $OCH_3$ | $OCH_3$ | N | O | |
| Q-6 | H | $CH_3$ | $CH_3$ | $CH_3$ | H | $CH_3$ | H | $CH_3$ | $CH_3$ | CH | O | |
| Q-6 | H | $CH_3$ | H | $CH_3$ | H | $CH_3$ | $CH_3$ | $CH_3$ | $CH_3$ | N | O | |
| Q-6 | H | $CH_3$ | $CH_3$ | $CH_3$ | $CH_3$ | H | H | $CH_3$ | $OCH_3$ | CH | O | |
| Q-6 | H | $CH_3$ | $CH_3$ | H | H | $CH_3$ | $CH_3$ | $CH_3$ | $OCH_3$ | N | O | |
| Q-6 | H | H | H | $CH_3$ | $CH_3$ | $CH_3$ | $CH_3$ | $OCH_3$ | $OCH_3$ | CH | O | |
| Q-6 | $CH_3$ | H | H | H | H | H | H | $CH_3$ | $OCH_3$ | N | O | |
| Q-6 | H | H | H | H | H | H | H | Br | $OCH_3$ | CH | O | |
| Q-6 | H | H | H | H | H | H | H | Cl | $OC_2H_5$ | CH | O | |

Table 2b (continued)

| Q | R | $R_3$ | $R_4$ | $R_5$ | $R_6$ | $R_7$ | $R_8$ | X | Y | Z | W" | m.p. °C |
|---|---|---|---|---|---|---|---|---|---|---|---|---|
| Q-6 | H | H | H | H | H | H | H | $OCH_3$ | $C_2H_5$ | CH | O | |
| Q-6 | H | H | H | H | H | H | H | $OCH_3$ | $OC_2H_5$ | CH | O | |
| Q-6 | H | H | H | H | H | H | H | $CH_3$ | $OCH_3$ | CH | O | |
| Q-6 | H | H | H | H | H | H | H | $CH_3$ | $OCH_3$ | N | O | |
| Q-6 | H | H | H | H | H | H | H | $OCH_3$ | $OCH_3$ | CH | O | |
| Q-6 | H | H | H | H | H | H | H | $OCH_3$ | $OCH_3$ | N | O | |
| Q-6 | $CH_3$ | H | H | H | H | H | H | $OCH_3$ | $OCH_3$ | CH | O | |
| Q-6 | H | H | H | H | H | H | H | $CH_3$ | $OCH_3$ | CH | S | |
| Q-6 | H | H | H | H | H | H | H | $CH_3$ | $OCH_3$ | N | S | |
| Q-6 | H | H | H | H | H | H | H | $OCH_3$ | $OCH_3$ | CH | S | |
| Q-6 | H | H | H | H | H | H | H | $OCH_3$ | $OCH_3$ | N | S | |
| Q-6 | H | H | H | H | H | H | H | $OCH_3$ | $C_2H_5$ | CH | S | |
| Q-6 | H | H | H | H | H | H | H | $CH_3$ | $CH_3$ | CH | S | |
| Q-6 | H | H | H | H | H | H | H | $CH_3$ | $CH_3$ | N | S | |
| Q-6 | H | $CH_3$ | H | H | H | H | H | $CH_3$ | $OCH_3$ | CH | S | |
| Q-6 | H | H | H | $CH_3$ | H | H | H | $CH_3$ | $OCH_3$ | N | S | |
| Q-6 | H | H | H | H | H | $CH_3$ | H | $OCH_3$ | $OCH_3$ | CH | S | |
| Q-6 | H | $CH_3$ | H | $CH_3$ | H | H | H | $OCH_3$ | $OCH_3$ | N | S | |
| Q-6 | H | $CH_3$ | H | H | H | $CH_3$ | H | $CH_3$ | $CH_3$ | CH | S | |
| Q-6 | H | H | H | $CH_3$ | H | $CH_3$ | H | $CH_3$ | $CH_3$ | N | S | |
| Q-6 | H | $CH_3$ | $CH_3$ | H | H | H | H | $CH_3$ | $OCH_3$ | CH | S | |
| Q-6 | H | H | H | $CH_3$ | $CH_3$ | H | H | $CH_3$ | $OCH_3$ | N | S | |
| Q-6 | H | H | H | H | H | H | H | $OCH_3$ | $CH_2OCH_3$ | CH | S | |
| Q-6 | H | H | H | H | H | H | H | $OCH_3$ | $CF_3$ | CH | S | |
| Q-6 | H | H | H | H | H | H | H | $OCH_3$ | $OCF_2H$ | CH | S | |
| Q-6 | H | H | H | H | H | $CH_3$ | $CH_3$ | $OCH_3$ | $OCH_3$ | CH | S | |
| Q-6 | H | $CH_3$ | H | H | H | $CH_3$ | $CH_3$ | $OCH_3$ | $OCH_3$ | N | S | |
| Q-6 | H | $CH_3$ | $CH_3$ | H | H | $CH_3$ | H | $CH_3$ | $CH_3$ | CH | S | |
| Q-6 | H | $CH_3$ | H | $CH_3$ | $CH_3$ | H | H | $CH_3$ | $CH_3$ | N | S | |
| Q-6 | H | H | H | $CH_3$ | $CH_3$ | $CH_3$ | H | $CH_3$ | $OCH_3$ | CH | S | |
| Q-6 | H | $CH_3$ | $CH_3$ | $CH_3$ | H | H | H | $CH_3$ | $OCH_3$ | N | S | |

Table 2b (continued)

| Q | R | $R_3$ | $R_4$ | $R_5$ | $R_6$ | $R_7$ | $R_8$ | X | Y | Z | W'' | m.p. °C |
|---|---|---|---|---|---|---|---|---|---|---|---|---|
| Q-6 | H | H | H | $CH_3$ | $CH_3$ | $CH_3$ | H | $OCH_3$ | $OCH_3$ | CH | S | |
| Q-6 | H | $CH_3$ | H | $CH_3$ | $CH_3$ | $CH_3$ | H | $OCH_3$ | $OCH_3$ | N | S | |
| Q-6 | H | $CH_3$ | $CH_3$ | $CH_3$ | H | $CH_3$ | H | $CH_3$ | $CH_3$ | CH | S | |
| Q-6 | H | $CH_3$ | H | $CH_3$ | H | $CH_3$ | $CH_3$ | $CH_3$ | $CH_3$ | N | S | |
| Q-6 | H | $CH_3$ | $CH_3$ | $CH_3$ | $CH_3$ | H | H | $CH_3$ | $OCH_3$ | CH | S | |
| Q-6 | H | $CH_3$ | $CH_3$ | H | H | $CH_3$ | $CH_3$ | $CH_3$ | $OCH_3$ | N | S | |
| Q-6 | H | H | H | $CH_3$ | $CH_3$ | $CH_3$ | $CH_3$ | $OCH_3$ | $OCH_3$ | CH | S | |
| Q-6 | $CH_3$ | H | H | H | H | H | H | $CH_3$ | $OCH_3$ | N | S | |

Table 2c

General Structure 1

| Q | R | $R_3$ | $R_4$ | $R_5$ | $R_6$ | X | Y | Z | W' | m.p. °C |
|---|---|---|---|---|---|---|---|---|---|---|
| Q-7 | H | H | H | H | H | $CH_3$ | $OCH_3$ | CH | - | 152-155 |
| Q-7 | H | H | H | H | H | $CH_3$ | $OCH_3$ | N | - | 144-146 |
| Q-7 | H | H | H | H | H | $OCH_3$ | $OCH_3$ | CH | - | 167-170 |
| Q-7 | H | H | H | H | H | $OCH_3$ | $OCH_3$ | N | - | 156-159 |
| Q-7 | H | H | H | H | H | $CH_3$ | $CH_3$ | CH | - | 170-173 |
| Q-7 | H | H | H | H | H | $CH_3$ | $CH_3$ | N | - | 153-156 |
| Q-7 | H | $CH_3$ | H | H | H | $CH_3$ | $OCH_3$ | CH | - | |
| Q-7 | H | H | $CH_3$ | H | H | $CH_3$ | $OCH_3$ | N | - | |
| Q-7 | H | $CH_3$ | H | H | $CH_3$ | $OCH_3$ | $OCH_3$ | CH | - | |
| Q-7 | H | H | $CH_3$ | $CH_3$ | H | $OCH_3$ | $OCH_3$ | N | - | |
| Q-7 | H | $CH_3$ | $CH_3$ | H | H | $CH_3$ | $CH_3$ | CH | - | |
| Q-7 | H | $CH_3$ | $CH_3$ | $CH_3$ | H | $CH_3$ | $CH_3$ | N | - | |
| Q-7 | H | $CH_3$ | H | $CH_3$ | $CH_3$ | $CH_3$ | $OCH_3$ | CH | - | |
| Q-7 | H | $CH_3$ | $CH_3$ | $CH_3$ | $CH_3$ | $CH_3$ | $OCH_3$ | N | - | |
| Q-7 | $CH_3$ | H | H | H | H | $OCH_3$ | $OCH_3$ | CH | - | |
| Q-7 | $CH_3$ | $CH_3$ | $CH_3$ | $CH_3$ | $CH_3$ | $OCH_3$ | $OCH_3$ | CH | - | |
| Q-7 | H | H | H | H | H | $OCH_3$ | $OCH_2CF_3$ | CH | - | |
| Q-7 | H | H | H | H | H | $OCH_3$ | $NHCH_3$ | CH | - | |
| Q-7 | H | H | H | H | H | Cl | $OCH_3$ | CH | - | 157-159 |
| Q-7 | H | H | H | H | H | $OCH_3$ | $OCH_3$ | CH | - | * |
| Q-7 | H | H | H | H | H | $OCH_3$ | $CH_3$ | N | - | * |
| Q-7 | H | H | H | H | H | $OCH_3$ | CH | CH | - | * |
| Q-16 | H | $CH_3$ | $CH_3$ | H | H | $CH_3$ | $OCH_3$ | CH | O | 157-160 |
| Q-16 | H | $CH_3$ | $CH_3$ | H | H | $CH_3$ | $OCH_3$ | N | O | 137-140 |
| Q-16 | H | $CH_3$ | $CH_3$ | H | H | $OCH_3$ | $OCH_3$ | CH | O | |
| Q-16 | H | $CH_3$ | $CH_3$ | H | H | $OCH_3$ | $OCH_3$ | N | O | 138-141 |
| Q-16 | $CH_3$ | $CH_3$ | $CH_3$ | H | H | $CH_3$ | $OCH_3$ | N | O | |
| Q-16 | $CH_3$ | $CH_3$ | $CH_3$ | H | H | Cl | $OCH_3$ | CH | O | |
| Q-16 | H | $CH_3$ | $CH_3$ | H | H | $CH_3$ | $CH_3$ | CH | O | 185-188 |
| Q-16 | H | $CH_3$ | $CH_3$ | H | H | $CH_3$ | $CH_3$ | N | O | 163-167 |
| Q-16 | H | H | H | H | H | $CH_3$ | $OCH_3$ | CH | O | |
| Q-16 | H | H | H | H | H | $CH_3$ | $OCH_3$ | N | O | |

Table 2c (continued)

| Q | R | $R_3$ | $R_4$ | $R_5$ | $R_6$ | X | Y | Z | W' | m.p. °C |
|---|---|---|---|---|---|---|---|---|---|---|
| Q-16 | H | H | H | H | H | $OCH_3$ | $OCH_3$ | CH | O | |
| Q-16 | H | H | H | H | H | $OCH_3$ | $OCH_3$ | N | O | |
| Q-16 | H | $CH_3$ | $CH_3$ | H | H | $OCH_3$ | $N(CH_3)_2$ | CH | O | |
| Q-16 | H | $CH_3$ | $CH_3$ | H | H | $OCH_3$ | $CH(OCH_3)_2$ | CH | O | |
| Q-16 | H | $CH_3$ | $CH_3$ | H | H | Cl | $OCH_3$ | CH | O | 158-161 |
| Q-16 | H | $CH_3$ | $CH_3$ | H | H | $CH_3$ | $CH_2OCH_3$ | N | O | |
| Q-16 | H | $CH_3$ | H | H | H | $CH_3$ | $CH_3$ | CH | O | |
| Q-16 | H | H | H | $CH_3$ | H | $CH_3$ | $CH_3$ | N | O | |
| Q-16 | H | $CH_3$ | H | $CH_3$ | H | $CH_3$ | $OCH_3$ | N | O | |
| Q-16 | H | H | H | $CH_3$ | $CH_3$ | $CH_3$ | $OCH_3$ | CH | O | |
| Q-16 | H | $CH_3$ | H | $CH_3$ | $CH_3$ | $OCH_3$ | $OCH_3$ | CH | O | |
| Q-16 | H | $CH_3$ | $CH_3$ | $CH_3$ | H | $OCH_3$ | $OCH_3$ | N | O | |
| Q-16 | H | $CH_3$ | $CH_3$ | $CH_3$ | H | $CH_3$ | $CH_3$ | CH | O | |
| Q-16 | H | $CH_3$ | $CH_3$ | $CH_3$ | $CH_3$ | $CH_3$ | $CH_3$ | N | O | |
| Q-16 | H | $CH_3$ | $CH_3$ | H | H | $CH_3$ | $CH_3$ | CH | S | |
| Q-16 | H | $CH_3$ | $CH_3$ | H | H | $CH_3$ | $CH_3$ | N | S | |
| Q-16 | H | $CH_3$ | $CH_3$ | H | H | $CH_3$ | $OCH_3$ | CH | S | |
| Q-16 | H | $CH_3$ | $CH_3$ | H | H | $CH_3$ | $OCH_3$ | N | S | |
| Q-16 | H | $CH_3$ | $CH_3$ | H | H | $OCH_3$ | $OCH_3$ | CH | S | |
| Q-16 | H | $CH_3$ | $CH_3$ | H | H | $OCH_3$ | $OCH_3$ | N | S | |
| Q-16 | H | $CH_3$ | $CH_3$ | H | H | Cl | $OCH_3$ | CH | S | |
| Q-16 | H | $CH_3$ | $CH_3$ | H | H | $CH_3$ | $NHCH_3$ | N | S | |
| Q-16 | H | $CH_3$ | $CH_3$ | H | H | $CH_3$ | $N(CH_3)_2$ | N | S | |
| Q-16 | H | H | H | H | H | $CH_3$ | $OCH_3$ | CH | S | |
| Q-16 | H | H | H | H | H | $CH_3$ | $OCH_3$ | N | S | |
| Q-16 | H | H | H | H | H | $OCH_3$ | $OCH_3$ | CH | S | |
| Q-16 | H | H | H | H | H | $OCH_3$ | $OCH_3$ | N | S | |
| Q-16 | $CH_3$ | H | H | H | H | $CH_3$ | $CH_3$ | CH | S | |
| Q-16 | H | $CH_3$ | H | H | H | $CH_3$ | $CH_3$ | N | S | |
| Q-16 | H | H | H | $CH_3$ | H | $CH_3$ | $OCH_3$ | N | S | |
| Q-16 | H | $CH_3$ | H | $CH_3$ | H | $CH_3$ | $OCH_3$ | CH | S | |
| Q-16 | H | H | H | $CH_3$ | $CH_3$ | $OCH_3$ | $OCH_3$ | CH | S | |

Table 2c (continued)

| Q | R | $R_3$ | $R_4$ | $R_5$ | $R_6$ | X | Y | Z | W' | m.p. °C |
|---|---|---|---|---|---|---|---|---|---|---|
| Q-16 | H | $CH_3$ | $CH_3$ | $CH_3$ | H | $OCH_3$ | $OCH_3$ | N | S | |
| Q-16 | H | $CH_3$ | $CH_3$ | $CH_3$ | H | $CH_3$ | $CH_3$ | CH | S | |
| Q-16 | H | $CH_3$ | H | $CH_3$ | $CH_3$ | $CH_3$ | $CH_3$ | N | S | |
| Q-16 | H | $CH_3$ | $CH_3$ | $CH_3$ | $CH_3$ | $CH_3$ | $OCH_3$ | CH | S | |
| Q-17 | H | H | H | H | H | Cl | $OCH_3$ | CH | - | |
| Q-17 | H | H | H | H | H | $CH_3$ | $CH_3$ | CH | - | |
| Q-17 | H | H | H | H | H | Cl | $OC_2H_5$ | CH | - | |
| Q-17 | H | H | H | H | H | $CH_3$ | $CH_3$ | N | - | |
| Q-17 | H | H | H | H | H | $OCH_3$ | $OCH_3$ | CH | - | |
| Q-17 | H | H | H | H | H | $OCH_3$ | $OCH_3$ | N | - | |
| Q-17 | H | H | H | H | H | $OCH_3$ | $OC_2H_5$ | CH | - | |
| Q-17 | H | H | H | H | H | $OCH_3$ | $OC_2H_5$ | N | - | |
| Q-17 | H | $CH_3$ | H | H | H | $CH_3$ | $CH_3$ | CH | - | |
| Q-17 | H | H | H | H | H | $CH_3$ | $CH(OCH_3)_2$ | N | - | |
| Q-17 | H | H | H | H | H | $CH_3$ | 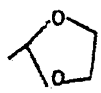 | N | - | |
| Q-17 | H | H | H | $CH_3$ | H | $CH_3$ | $CH_3$ | N | - | |
| Q-17 | H | $CH_3$ | $CH_3$ | H | H | $CH_3$ | $OCH_3$ | CH | - | |
| Q-17 | H | $CH_3$ | $CH_3$ | H | H | $CH_3$ | $OCH_3$ | N | - | |
| Q-17 | H | H | H | $CH_3$ | $CH_3$ | $OCH_3$ | $OCH_3$ | CH | - | |
| Q-17 | H | $CH_3$ | H | $CH_3$ | $CH_3$ | $OCH_3$ | $OCH_3$ | N | - | |
| Q-17 | H | $CH_3$ | $CH_3$ | $CH_3$ | H | $CH_3$ | $CH_3$ | CH | - | |
| Q-17 | H | $CH_3$ | $CH_3$ | $CH_3$ | H | $CH_3$ | $CH_3$ | N | - | |
| Q-17 | H | $CH_3$ | $CH_3$ | $CH_3$ | $CH_3$ | $CH_3$ | $OCH_3$ | CH | - | |
| Q-17 | $CH_3$ | H | H | H | H | $CH_3$ | $OCH_3$ | N | - | |

## Table 2d

### General Structure 1 wherein Q is Q-30

| R | $R_3$ | $R_4$ | $R_5$ | $R_6$ | $R_7$ | $R_8$ | $R_9$ | X | Y | Z | m.p. °C |
|---|---|---|---|---|---|---|---|---|---|---|---|
| H | H | H | H | H | H | H | H | $CH_3$ | $OCH_3$ | CH | |
| H | H | H | H | H | H | H | H | $CH_3$ | $OCH_3$ | N | |
| H | H | H | H | H | H | H | H | $OCH_3$ | $OCH_3$ | CH | |
| H | H | H | H | H | H | H | H | $OCH_3$ | $OCH_3$ | N | |
| H | H | H | H | H | H | H | H | $CH_3$ | $CH_3$ | N | |
| H | H | H | H | H | H | H | H | $CH_3$ | $CH_3$ | CH | |
| $CH_3$ | H | H | H | H | H | H | H | $CH_3$ | $OCH_3$ | CH | |
| H | $CH_3$ | H | H | H | H | H | H | $CH_3$ | $OCH_3$ | N | |
| H | H | H | H | H | H | H | H | Cl | $OCH_3$ | CH | |
| H | H | $CH_3$ | H | H | H | H | H | $OCH_3$ | $OCH_3$ | CH | |
| H | H | H | H | $CH_3$ | H | H | H | $OCH_3$ | $OCH_3$ | N | |
| H | H | H | H | H | H | $CH_3$ | H | $CH_3$ | $CH_3$ | CH | |
| H | $CH_3$ | $CH_3$ | H | H | H | H | H | $CH_3$ | $CH_3$ | N | |
| H | $CH_3$ | H | H | $CH_3$ | H | H | H | $CH_3$ | $OCH_3$ | CH | |
| H | $CH_3$ | H | H | H | H | $CH_3$ | H | $CH_3$ | $OCH_3$ | N | |
| H | H | $CH_3$ | $CH_3$ | H | H | H | H | $OCH_3$ | $OCH_3$ | CH | |
| H | H | $CH_3$ | H | $CH_3$ | H | H | H | $OCH_3$ | $OCH_3$ | N | |
| H | H | $CH_3$ | H | H | H | $CH_3$ | H | $CH_3$ | $CH_3$ | CH | |
| H | H | H | H | $CH_3$ | $CH_3$ | H | H | $CH_3$ | $CH_3$ | N | |
| H | H | H | H | $CH_3$ | H | $CH_3$ | H | $CH_3$ | $OCH_3$ | CH | |
| H | H | H | H | H | H | $CH_3$ | $CH_3$ | $CH_3$ | $OCH_3$ | N | |
| H | $CH_3$ | $CH_3$ | $CH_3$ | H | H | H | H | $OCH_3$ | $OCH_3$ | CH | |
| H | $CH_3$ | $CH_3$ | H | $CH_3$ | H | H | H | $OCH_3$ | $OCH_3$ | N | |
| H | $CH_3$ | $CH_3$ | H | H | H | $CH_3$ | H | $CH_3$ | $CH_3$ | CH | |
| H | $CH_3$ | H | H | H | $CH_3$ | H | H | $CH_3$ | $CH_3$ | N | |
| H | $CH_3$ | H | H | $CH_3$ | H | $CH_3$ | H | $CH_3$ | $OCH_3$ | CH | |
| H | $CH_3$ | H | H | H | H | $CH_3$ | $CH_3$ | $CH_3$ | $OCH_3$ | N | |
| H | H | $CH_3$ | $CH_3$ | $CH_3$ | H | H | H | $OCH_3$ | $OCH_3$ | CH | |
| H | H | $CH_3$ | $CH_3$ | H | H | $CH_3$ | H | $OCH_3$ | $OCH_3$ | N | |
| H | H | $CH_3$ | H | $CH_3$ | $CH_3$ | H | H | $CH_3$ | $CH_3$ | CH | |
| H | H | $CH_3$ | H | $CH_3$ | H | $CH_3$ | H | $CH_3$ | $CH_3$ | N | |
| H | H | $CH_3$ | H | H | H | $CH_3$ | $CH_3$ | $CH_3$ | $OCH_3$ | CH | |

Table 2d (continued)

| R | $R_3$ | $R_4$ | $R_5$ | $R_6$ | $R_7$ | $R_8$ | $R_9$ | X | Y | Z | m.p. °C |
|---|---|---|---|---|---|---|---|---|---|---|---|
| H | H | H | H | $CH_3$ | $CH_3$ | $CH_3$ | H | $CH_3$ | $OCH_3$ | N | |
| H | H | H | H | $CH_3$ | H | $CH_3$ | $CH_3$ | $OCH_3$ | $OCH_3$ | CH | |
| H | H | H | H | H | H | H | H | $CH_3$ | $OC_2H_5$ | CH | |
| H | H | H | H | H | H | H | H | $CH_3$ | $CH_2OCH_3$ | CH | |

Table 2e

General Structure 1 wherein Q is Q-31.

| R | $R_3$ | $R_4$ | $R_5$ | $R_6$ | $R_7$ | $R_8$ | $R_9$ | $R_{10}$ | X | Y | Z | m.p. °C |
|---|---|---|---|---|---|---|---|---|---|---|---|---|
| H | H | H | H | H | H | H | H | H | $CH_3$ | $OCH_3$ | CH | |
| H | H | H | H | H | H | H | H | H | $CH_3$ | $OCH_3$ | N | |
| H | H | H | H | H | H | H | H | H | $OCH_3$ | $OCH_3$ | CH | |
| H | H | H | H | H | H | H | H | H | $OCH_3$ | $OCH_3$ | N | |
| H | H | H | H | H | H | H | H | H | $CH_3$ | $CH_3$ | CH | |
| H | H | H | H | H | H | H | H | H | $CH_3$ | $CH_3$ | N | |
| H | H | H | H | H | H | H | H | H | Cl | $OCH_3$ | CH | |
| H | H | H | H | H | H | H | H | H | $CH_3$ | $C_2H_5$ | CH | |
| H | H | H | H | H | H | H | H | H | $CH_3$ | $CF_3$ | CH | |
| H | H | H | H | H | H | H | H | H | $CH_3$ | $OCF_2H$ | CH | |
| H | H | H | H | H | H | H | H | H | $CH_3$ | $OCH_2CF_3$ | CH | |
| H | $CH_3$ | H | H | H | H | H | H | H | $CH_3$ | $CH_3$ | CH | |
| H | H | H | $CH_3$ | H | H | H | H | H | $CH_3$ | $CH_3$ | N | |
| H | H | H | H | H | $CH_3$ | H | H | H | $CH_3$ | $OCH_3$ | CH | |
| H | H | H | H | H | H | H | $CH_3$ | H | $CH_3$ | $OCH_3$ | N | |
| H | $CH_3$ | $CH_3$ | H | H | H | H | H | H | $OCH_3$ | $OCH_3$ | CH | |
| H | H | H | $CH_3$ | $CH_3$ | H | H | H | H | $OCH_3$ | $OCH_3$ | N | |
| H | H | H | H | H | $CH_3$ | $CH_3$ | H | H | $CH_3$ | $CH_3$ | CH | |
| H | H | H | H | H | H | H | $CH_3$ | $CH_3$ | $CH_3$ | $CH_3$ | N | |
| H | H | H | $CH_3$ | H | H | H | $CH_3$ | H | $CH_3$ | $OCH_3$ | CH | |
| H | $CH_3$ | H | H | H | $CH_3$ | H | H | H | $CH_3$ | $OCH_3$ | N | |
| H | $CH_3$ | H | $CH_3$ | H | H | H | H | H | $OCH_3$ | $OCH_3$ | CH | |
| H | H | H | H | H | $CH_3$ | H | $CH_3$ | H | $OCH_3$ | $OCH_3$ | N | |
| H | $CH_3$ | $CH_3$ | $CH_3$ | H | H | H | H | H | $CH_3$ | $CH_3$ | CH | |
| H | $CH_3$ | H | H | H | $CH_3$ | $CH_3$ | H | H | $CH_3$ | $CH_3$ | N | |
| H | H | H | $CH_3$ | $CH_3$ | H | H | $CH_3$ | H | $CH_3$ | $OCH_3$ | CH | |
| H | H | H | $CH_3$ | $CH_3$ | $CH_3$ | H | H | H | $CH_3$ | $OCH_3$ | N | |
| H | $CH_3$ | H | H | H | $CH_3$ | H | $CH_3$ | H | $OCH_3$ | $OCH_3$ | CH | |

Table 2e (continued)

| R | $R_3$ | $R_4$ | $R_5$ | $R_6$ | $R_7$ | $R_8$ | $R_9$ | $R_{10}$ | X | Y | Z | m.p. °C |
|---|---|---|---|---|---|---|---|---|---|---|---|---|
| $CH_3$ | H | H | H | H | H | H | H | H | $OCH_3$ | $OCH_3$ | N | |
| H | H | H | H | H | H | H | H | H | $CH_3$ | $OCH_3$ | CH | * |
| H | H | H | H | H | H | H | H | H | $CH_3$ | $OCH_3$ | N | * |
| H | H | H | H | H | H | H | H | H | $OCH_3$ | $OCH_3$ | CH | * |
| H | H | H | H | H | H | H | H | H | $OCH_3$ | $OCH_3$ | N | * |
| H | H | H | H | H | H | H | H | H | $CH_3$ | $CH_3$ | CH | * |
| H | H | H | H | H | H | H | H | H | $CH_3$ | $CH_3$ | N | * |
| H | H | H | H | H | H | H | H | H | Cl | $OCH_3$ | CH | * |

***Wherein W" is O, unless indicated by **** where W" is S in all Tables.

Table 2f

General Structure 1 wherein Q is Q-32.

| R | $R_3$ | $R_4$ | $R_5$ | $R_6$ | $R_7$ | $R_8$ | $R_9$ | $R_{10}$ | X | Y | Z | W'' |
|---|---|---|---|---|---|---|---|---|---|---|---|---|
| H | H | H | H | H | H | H | H | H | $CH_3$ | $CH_3$ | CH | O |
| H | H | H | H | H | H | H | H | H | $CH_3$ | $CH_3$ | N | O |
| H | H | H | H | H | H | H | H | H | Cl | $OCH_3$ | CH | O |
| H | H | H | H | H | H | H | H | H | $CH_3$ | $NHCH_3$ | CH | O |
| H | H | H | H | H | H | H | H | H | $CH_3$ | $N(CH_3)_2$ | CH | O |
| H | H | H | H | H | H | H | H | H | $CH_3$ | $CH(OCH_3)_2$ | CH | O |
| H | H | H | H | H | H | H | H | H | Br | $OCH_3$ | CH | O |
| H | $CH_3$ | H | H | H | H | H | H | H | $CH_3$ | $CH_3$ | CH | O |
| H | H | H | $CH_3$ | H | H | H | H | H | $CH_3$ | $CH_3$ | N | O |
| H | H | H | H | H | $CH_3$ | H | H | H | $CH_3$ | $OCH_3$ | CH | O |
| H | H | H | H | H | H | H | $CH_3$ | H | $CH_3$ | $OCH_3$ | N | O |
| H | $CH_3$ | $CH_3$ | H | H | H | H | H | H | $OCH_3$ | $OCH_3$ | CH | O |
| H | H | H | $CH_3$ | $CH_3$ | H | H | H | H | $OCH_3$ | $OCH_3$ | N | O |
| H | H | H | H | H | $CH_3$ | $CH_3$ | H | H | $CH_3$ | $CH_3$ | CH | O |
| H | H | H | H | H | H | H | $CH_3$ | $CH_3$ | $CH_3$ | $CH_3$ | N | O |
| H | H | H | $CH_3$ | H | H | H | $CH_3$ | H | $CH_3$ | $OCH_3$ | CH | O |
| H | $CH_3$ | H | H | H | $CH_3$ | H | H | H | $CH_3$ | $OCH_3$ | N | O |
| H | $CH_3$ | H | $CH_3$ | H | H | H | H | H | $OCH_3$ | $OCH_3$ | CH | O |
| H | H | H | H | H | $CH_3$ | H | $CH_3$ | H | $OCH_3$ | $OCH_3$ | N | O |
| H | $CH_3$ | $CH_3$ | $CH_3$ | H | H | H | H | H | $CH_3$ | $CH_3$ | CH | O |
| H | $CH_3$ | H | H | H | $CH_3$ | $CH_3$ | H | H | $CH_3$ | $CH_3$ | N | O |
| H | H | H | $CH_3$ | $CH_3$ | H | H | $CH_3$ | H | $CH_3$ | $OCH_3$ | CH | O |
| H | H | H | $CH_3$ | $CH_3$ | $CH_3$ | H | H | H | $CH_3$ | $OCH_3$ | N | O |
| H | $CH_3$ | H | H | H | $CH_3$ | H | $CH_3$ | H | $OCH_3$ | $OCH_3$ | CH | O |

**Table 2f (continued)**

| R | $R_3$ | $R_4$ | $R_5$ | $R_6$ | $R_7$ | $R_8$ | $R_9$ | $R_{10}$ | X | Y | Z | W" |
|---|---|---|---|---|---|---|---|---|---|---|---|---|
| $CH_3$ | H | H | H | H | H | H | H | H | $OCH_3$ | $OCH_3$ | N | O |
| H | $CH_3$ | H | H | H | H | H | $CH_3$ | H | $CH_3$ | $OCH_3$ | CH | O |
| H | H | H | H | H | H | H | H | H | $CH_3$ | $OCH_3$ | CH | O |
| H | H | H | H | H | H | H | H | H | $CH_3$ | $OCH_3$ | N | O |
| H | H | H | H | H | H | H | H | H | $OCH_3$ | $OCH_3$ | CH | O |
| H | H | H | H | H | H | H | H | H | $OCH_3$ | $OCH_3$ | N | O |
| H | H | H | H | H | H | H | H | H | $CH_3$ | $OCH_3$ | CH | S |
| H | H | H | H | H | H | H | H | H | $CH_3$ | $OCH_3$ | N | S |
| H | H | H | H | H | H | H | H | H | $OCH_3$ | $OCH_3$ | CH | S |
| H | H | H | H | H | H | H | H | H | $OCH_3$ | $OCH_3$ | N | S |
| H | H | H | H | H | H | H | H | H | $CH_3$ | $CH_3$ | CH | S |
| H | H | H | H | H | H | H | H | H | $CH_3$ | $CH_3$ | N | S |
| H | H | H | H | H | H | H | H | H | Cl | $OCH_3$ | CH | S |
| H | H | H | H | H | H | H | H | H | $CH_3$ | 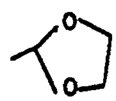 | CH | S |
| H | H | H | H | H | H | H | H | H | Cl | $OC_2H_5$ | CH | S |
| H | H | H | H | H | H | H | H | H | $CH_3$ | $C_2H_5$ | N | S |
| H | H | H | H | H | H | H | H | H | $CH_3$ | $OC_2H_5$ | N | S |
| H | $CH_3$ | H | H | H | H | H | H | H | $CH_3$ | $CH_3$ | CH | S |
| H | H | H | $CH_3$ | H | H | H | H | H | $CH_3$ | $CH_3$ | N | S |
| H | H | H | H | H | $CH_3$ | H | H | H | $CH_3$ | $OCH_3$ | CH | S |
| H | H | H | H | H | H | H | $CH_3$ | H | $CH_3$ | $OCH_3$ | N | S |
| H | $CH_3$ | $CH_3$ | H | H | H | H | H | H | $OCH_3$ | $OCH_3$ | CH | S |
| H | H | H | $CH_3$ | $CH_3$ | H | H | H | H | $OCH_3$ | $OCH_3$ | N | S |
| H | H | H | H | H | $CH_3$ | $CH_3$ | H | H | $CH_3$ | $CH_3$ | CH | S |

Table 2f (continued)

| R | $R_3$ | $R_4$ | $R_5$ | $R_6$ | $R_7$ | $R_8$ | $R_9$ | $R_{10}$ | X | Y | Z | W" |
|---|---|---|---|---|---|---|---|---|---|---|---|---|
| H | H | H | H | H | H | H | $CH_3$ | $CH_3$ | $CH_3$ | $CH_3$ | N | S |
| H | H | H | $CH_3$ | H | H | H | $CH_3$ | H | $CH_3$ | $OCH_3$ | CH | S |
| H | $CH_3$ | H | H | H | $CH_3$ | H | H | H | $CH_3$ | $OCH_3$ | N | S |
| H | $CH_3$ | H | $CH_3$ | H | H | H | H | H | $OCH_3$ | $OCH_3$ | CH | S |
| H | H | H | H | H | $CH_3$ | H | $CH_3$ | H | $OCH_3$ | $OCH_3$ | N | S |
| H | $CH_3$ | $CH_3$ | $CH_3$ | H | H | H | H | H | $CH_3$ | $CH_3$ | CH | S |
| H | $CH_3$ | H | H | H | $CH_3$ | $CH_3$ | H | H | $CH_3$ | $CH_3$ | N | S |
| H | H | H | $CH_3$ | $CH_3$ | H | H | $CH_3$ | H | $CH_3$ | $OCH_3$ | CH | S |
| H | H | H | $CH_3$ | $CH_3$ | $CH_3$ | H | H | H | $CH_3$ | $OCH_3$ | N | S |
| H | $CH_3$ | H | H | H | $CH_3$ | H | $CH_3$ | H | $OCH_3$ | $OCH_3$ | CH | S |
| $CH_3$ | H | H | H | H | H | H | H | H | $OCH_3$ | $OCH_3$ | N | S |
| H | $CH_3$ | H | H | H | H | H | $CH_3$ | H | $CH_3$ | $OCH_3$ | CH | S |

Table 2g

General Structure 1

| Q | R | $R_3$ | $R_4$ | $R_5$ | $R_6$ | $R_7$ | X | Y | Z | m.p. °C |
|---|---|---|---|---|---|---|---|---|---|---|
| Q-41 | H | H | H | H | H | H | Cl | $OCH_3$ | CH | |
| Q-41 | H | H | H | H | H | H | Br | $OCH_3$ | CH | |
| Q-41 | H | H | H | H | H | H | Cl | $OC_2H_5$ | CH | |
| Q-41 | H | H | H | H | H | H | $CH_3$ | $CH_3$ | N | |
| Q-41 | H | H | H | H | H | H | $OCH_3$ | $OCH_3$ | CH | |
| Q-41 | H | H | H | H | H | H | $OCH_3$ | $OCH_3$ | N | |
| Q-41 | H | H | H | H | H | H | $OCH_3$ | $OC_2H_5$ | CH | |
| Q-41 | H | H | H | H | H | H | $OCH_3$ | $OC_2H_5$ | N | |
| Q-41 | H | H | H | H | H | H | $CH_3$ | $CH_3$ | CH | |
| Q-41 | H | H | H | H | H | H | $CH_3$ | $OCH_3$ | N | |
| Q-41 | H | H | H | H | H | H | $CH_3$ | $OCH_3$ | CH | |
| Q-41 | H | H | H | H | H | H | $CH_3$ | $CH_2OCH_3$ | CH | |
| Q-41 | H | H | H | H | H | H | $CH_3$ | $CF_3$ | N | |
| Q-41 | H | $CH_3$ | H | H | H | H | $CH_3$ | $CH_3$ | CH | |
| Q-41 | H | H | H | $CH_3$ | H | H | $CH_3$ | $CH_3$ | N | |
| Q-41 | H | H | H | H | $CH_3$ | H | $CH_3$ | $OCH_3$ | CH | |
| Q-41 | H | $CH_3$ | $CH_3$ | H | H | H | $CH_3$ | $OCH_3$ | N | |
| Q-41 | H | $CH_3$ | $CH_3$ | H | H | H | $OCH_3$ | $OCH_3$ | CH | |
| Q-41 | H | $CH_3$ | $CH_3$ | H | H | H | $OCH_3$ | $OCH_3$ | N | |
| Q-41 | H | $CH_3$ | H | $CH_3$ | H | H | $CH_3$ | $CH_3$ | CH | |
| Q-41 | H | $CH_3$ | H | H | $CH_3$ | H | $CH_3$ | $CH_3$ | N | |
| Q-41 | H | H | H | $CH_3$ | $CH_3$ | H | $CH_3$ | $OCH_3$ | CH | |
| Q-41 | H | H | H | H | $CH_3$ | $CH_3$ | $CH_3$ | $OCH_3$ | N | |
| Q-41 | H | $CH_3$ | $CH_3$ | $CH_3$ | H | H | $OCH_3$ | $OCH_3$ | CH | |
| Q-41 | H | $CH_3$ | $CH_3$ | H | $CH_3$ | H | $OCH_3$ | $OCH_3$ | N | |
| Q-41 | H | $CH_3$ | H | $CH_3$ | $CH_3$ | H | $CH_3$ | $CH_3$ | CH | |
| Q-41 | H | $CH_3$ | H | H | $CH_3$ | $CH_3$ | $CH_3$ | $CH_3$ | N | |

Table 2g (continued)

| Q | R | $R_3$ | $R_4$ | $R_5$ | $R_6$ | $R_7$ | X | Y | Z | m.p. °C |
|---|---|---|---|---|---|---|---|---|---|---|
| Q-41 | $CH_3$ | H | H | H | H | H | $CH_3$ | $CH_3$ | CH | |
| Q-41 | $CH_3$ | H | H | H | H | H | $CH_3$ | $OCH_3$ | CH | |

**Table 3a**

General Structure 1 wherein Q is Q-20.

| R | $R'_3$ | X | Y | Z | W' | m.p. °C |
|---|---|---|---|---|---|---|
| H | H | $CH_3$ | $OCH_3$ | CH | O | |
| H | H | $CH_3$ | $OCH_3$ | N | O | |
| H | H | $OCH_3$ | $OCH_3$ | CH | O | |
| H | H | $OCH_3$ | Cl | CH | O | |
| H | H | $OCH_3$ | $OCH_3$ | N | O | |
| H | H | $CH_3$ | $CH_3$ | CH | O | |
| H | $CH_3$ | $CH_3$ | $OCH_3$ | CH | O | |
| $CH_3$ | H | $CH_3$ | $OCH_3$ | N | O | |
| $CH_3$ | $CH_3$ | $OCH_3$ | $OCH_3$ | CH | O | |
| H | H | $CH_3$ | $OC_2H_5$ | N | O | |
| H | H | $CH_3$ | $CH_3$ | CH | S | |
| H | H | $CH_3$ | $CH_3$ | N | S | |
| H | H | $CH_3$ | $OCH_3$ | CH | S | |
| H | H | $CH_3$ | $OCH_3$ | N | S | |
| H | H | $OCH_3$ | $OCH_3$ | CH | S | |
| H | H | $OCH_3$ | $OCH_3$ | N | S | |
| $CH_3$ | H | $CH_3$ | $OCH_3$ | CH | S | |
| H | $CH_3$ | $CH_3$ | $OCH_3$ | N | S | |
| H | $CH_3$ | $CH_3$ | $CH_2OCH_3$ | N | S | |
| H | $C_2H_5$ | $CH_3$ | $CH_3$ | CH | O | |
| H | $C_2H_5$ | $CH_3$ | $CH_3$ | N | O | |
| H | $C_2H_5$ | $CH_3$ | $OCH_3$ | CH | O | |
| H | $C_2H_5$ | $CH_3$ | $OCH_3$ | N | O | |
| H | $C_2H_5$ | $OCH_3$ | $OCH_3$ | CH | O | |
| H | $C_2H_5$ | $OCH_3$ | $OCH_3$ | N | O | |
| H | $C_2H_5$ | $OCH_3$ | Cl | CH | O | |
| H | $C_2H_5$ | $CH_3$ | $CH_3$ | CH | S | |
| H | $C_2H_5$ | $CH_3$ | $CH_3$ | N | S | |
| H | $C_2H_5$ | $CH_3$ | $OCH_3$ | CH | S | |
| H | $C_2H_5$ | $CH_3$ | $OCH_3$ | N | S | |
| H | $C_2H_5$ | $OCH_3$ | $OCH_3$ | CH | S | |
| H | $C_2H_5$ | $OCH_3$ | $OCH_3$ | N | S | |

**Table 3a (continued)**

| R | R'3 | X | Y | Z | W' | m.p. °C |
|---|---|---|---|---|---|---|
| H | $C_2H_5$ | $OCH_3$ | Cl | CH | S | |
| H | $C_2H_5$ | $OCH_3$ | $N(CH_3)_2$ | N | S | |
| $CH_3$ | n-$C_3H_7$ | $OCH_3$ | $OCH_3$ | CH | O | |
| $CH_3$ | n-$C_3H_7$ | $CH_3$ | $CH_3$ | CH | O | |
| $CH_3$ | n-$C_3H_7$ | $CH_3$ | $OCH_3$ | CH | O | |
| $CH_3$ | n-$C_3H_7$ | $OCH_3$ | $OCH_3$ | CH | O | |
| $CH_3$ | n-$C_3H_7$ | $OCH_3$ | $OCH_3$ | N | O | |
| $CH_3$ | n-$C_3H_7$ | $OCH_3$ | Cl | CH | O | |
| $CH_3$ | n-$C_3H_7$ | $CH_3$ | $OCH_2CF_3$ | CH | r | |
| H | n-$C_3H_7$ | $CH_3$ | $CH_3$ | CH | S | |
| H | n-$C_3H_7$ | $CH_3$ | $OCH_3$ | CH | S | |
| H | n-$C_3H_7$ | $CH_3$ | $OCH_3$ | N | S | |
| H | n-$C_3H_7$ | $OCH_3$ | $OCH_3$ | CH | S | |
| H | n-$C_3H_7$ | $OCH_3$ | $OCH_3$ | N | S | |
| H | n-$C_3H_7$ | $OCH_3$ | Cl | CH | S | |
| H | n-$C_3H_7$ | $CH_3$ | $CH(OCH_3)_2$ | CH | S | |
| H | i-$C_3H_7$ | $CH_3$ | $CH_3$ | CH | O | |
| H | i-$C_3H_7$ | $CH_3$ | $OCH_3$ | CH | O | |
| H | i-$C_3H_7$ | $CH_3$ | $OCH_3$ | N | O | |
| H | i-$C_3H_7$ | $OCH_3$ | $OCH_3$ | CH | O | |
| H | i-$C_3H_7$ | $OCH_3$ | $OCH_3$ | N | O | |
| H | i-$C_3H_7$ | $OCH_3$ | Cl | CH | O | |
| H | i-$C_3H_7$ | $CH_3$ | $CH_3$ | N | O | |
| H | i-$C_3H_7$ | $OCH_3$ | 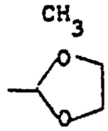 | CH | O | |
| $CH_3$ | i-$C_3H_7$ | $OCH_3$ | $OCH_3$ | CH | S | |
| H | i-$C_3H_7$ | $CH_3$ | $CH_3$ | CH | S | |
| H | i-$C_3H_7$ | $CH_3$ | $OCH_3$ | CH | S | |
| H | i-$C_3H_7$ | $CH_3$ | $OCH_3$ | N | S | |
| H | i-$C_3H_7$ | $OCH_3$ | $OCH_3$ | CH | S | |
| H | i-$C_3H_7$ | $OCH_3$ | $OCH_3$ | N | S | |

Table 3a (continued)

| R | $R'_3$ | X | Y | Z | W' | m.p. °C |
|---|---|---|---|---|---|---|
| H | i-$C_3H_7$ | $OCH_3$ | Cl | CH | S | |
| H | i-$C_3H_7$ | $CH_3$ | $OC_2H_5$ | N | S | |
| H | $SCH_3$ | $CH_3$ | $CH_3$ | CH | O | |
| H | $SCH_3$ | $CH_3$ | $OCH_3$ | CH | O | |
| H | $SCH_3$ | $CH_3$ | $OCH_3$ | N | O | |
| H | $SCH_3$ | $OCH_3$ | $OCH_3$ | CH | O | |
| H | $SCH_3$ | $OCH_3$ | $OCH_3$ | N | O | |
| H | $SCH_3$ | $OCH_3$ | Cl | CH | O | |
| H | $SCH_3$ | $OCH_3$ | $NHCH_3$ | CH | O | |
| $CH_3$ | $SCH_3$ | $OCH_3$ | $OCH_3$ | CH | S | |
| H | $SCH_3$ | $CH_3$ | $CH_3$ | CH | S | |
| H | $SCH_3$ | $CH_3$ | $CH_3$ | N | S | |
| H | $SCH_3$ | $CH_3$ | $OCH_3$ | CH | S | |
| H | $SCH_3$ | $CH_3$ | $OCH_3$ | N | S | |
| H | $SCH_3$ | $OCH_3$ | $OCH_3$ | CH | S | |
| H | $SCH_3$ | $OCH_3$ | $OCH_3$ | N | S | |
| H | $SCH_3$ | $OCH_3$ | Cl | CH | S | |
| $CH_3$ | $SC_2H_5$ | $OCH_3$ | $OCH_3$ | CH | O | |
| H | $SC_2H_5$ | $CH_3$ | $CH_3$ | CH | O | |
| H | $SC_2H_5$ | $CH_3$ | $CH_3$ | N | O | |
| H | $SC_2H_5$ | $CH_3$ | $OCH_3$ | CH | O | |
| H | $SC_2H_5$ | $CH_3$ | $OCH_3$ | N | O | |
| H | $SC_2H_5$ | $OCH_3$ | $OCH_3$ | CH | O | |
| H | $SC_2H_5$ | $OCH_3$ | $OCH_3$ | N | O | |
| H | $SC_2H_5$ | $OCH_3$ | Cl | CH | O | |
| H | $SC_2H_5$ | $CH_3$ | $CH_3$ | CH | S | |
| H | $SC_2H_5$ | $CH_3$ | $OCH_3$ | CH | S | |
| H | $SC_2H_5$ | $CH_3$ | $OCH_3$ | N | S | |
| H | $SC_2H_5$ | $OCH_3$ | $OCH_3$ | CH | S | |
| H | $SC_2H_5$ | $CH_3$ | $OCH_3$ | N | S | |
| H | $SC_2H_5$ | $OCH_3$ | Cl | CH | S | |

Table 3a (continued)

| R | $R'_3$ | X | Y | Z | W' | m.p. °C |
|---|---|---|---|---|---|---|
| H | $SC_2H_5$ | $CH_3$ | $OC_2H_5$ | N | S | |
| H | S-n-$C_3H_7$ | $CH_3$ | $CH_3$ | CH | O | |
| H | S-n-$C_3H_7$ | $CH_3$ | $CH_2OCH_3$ | CH | O | |
| H | S-n-$C_3H_7$ | $CH_3$ | $OCH_3$ | CH | O | |
| H | S-n-$C_3H_7$ | $CH_3$ | $OCH_3$ | N | O | |
| H | S-n-$C_3H_7$ | $OCH_3$ | $OCH_3$ | CH | O | |
| H | S-n-$C_3H_7$ | $OCH_3$ | $OCH_3$ | N | O | |
| H | S-n-$C_3H_7$ | $OCH_3$ | Cl | CH | O | |
| $CH_3$ | S-n-$C_3H_7$ | $OCH_3$ | $OCH_3$ | CH | S | |
| H | S-n-$C_3H_7$ | $CH_3$ | $CH_3$ | CH | S | |
| H | S-n-$C_3H_7$ | $CH_3$ | $CH_3$ | N | S | |
| H | S-n-$C_3H_7$ | $CH_3$ | $OCH_3$ | CH | S | |
| H | S-n-$C_3H_7$ | $OCH_3$ | $OCH_3$ | CH | S | |
| H | S-n-$C_3H_7$ | $OCH_3$ | $OCH_3$ | N | S | |
| H | S-n-$C_3H_7$ | $CH_3$ | Cl | CH | S | |
| H | S-n-$C_3H_7$ | $CH_3$ | $OCH_3$ | CH | O | |
| H | S-n-$C_3H_7$ | $OCH_3$ | $OCH_3$ | CH | S | |
| H | S-n-$C_3H_7$ | $CH_3$ | $OCH_3$ | N | S | |
| $CH_3$ | S-n-$C_4H_9$ | $OCH_3$ | $OCH_3$ | CH | O | |
| H | S-n-$C_4H_9$ | $CH_3$ | $CH_3$ | CH | O | |
| H | S-n-$C_4H_9$ | $CH_3$ | $CH_3$ | N | O | |
| H | S-n-$C_4H_9$ | $CH_3$ | $OCH_3$ | CH | O | |
| H | S-n-$C_4H_9$ | $CH_3$ | $OCH_3$ | N | O | |
| H | S-n-$C_4H_9$ | $OCH_3$ | $OCH_3$ | CH | O | |
| H | S-n-$C_4H_9$ | $OCH_3$ | $OCH_3$ | N | O | |
| H | S-n-$C_4H_9$ | $OCH_3$ | Cl | CH | O | |
| H | S-n-$C_4H_9$ | $CH_3$ | $CH_3$ | CH | S | |
| H | S-n-$C_4H_9$ | $CH_3$ | $OCH_3$ | N | S | |
| H | S-n-$C_4H_9$ | $CH_3$ | $OCH_3$ | CH | S | |
| H | S-n-$C_4H_9$ | $OCH_3$ | $OCH_3$ | N | S | |
| H | S-n-$C_4H_9$ | $OCH_3$ | $OCH_3$ | CH | S | |
| H | S-n-$C_4H_9$ | $OCH_3$ | $OCH_3$ | N | S | |

Table 3a (continued)

| R | $R'_3$ | X | Y | Z | W' | m.p. °C |
|---|---|---|---|---|---|---|
| H | S-n-$C_4H_9$ | $OCH_3$ | $OCF_2H$ | N | S | |
| H | S-n-$C_4H_9$ | $OCH_3$ | $OCH_3$ | CH | O | |
| H | S-n-$C_4H_9$ | $CH_3$ | $OCH_3$ | N | O | |
| H | S-n-$C_4H_9$ | $OCH_3$ | $OCH_3$ | CH | S | |
| H | S-n-$C_4H_9$ | $CH_3$ | $OCH_3$ | CH | S | |
| H | S-n-$C_4H_9$ | $CH_3$ | $OCH_3$ | N | O | |
| H | S-n-$C_4H_9$ | $CH_3$ | $OCH_3$ | CH | O | |
| H | S-n-$C_4H_9$ | $OCH_3$ | $OCH_3$ | CH | S | |
| H | S-n-$C_4H_9$ | $OCH_3$ | $OCH_3$ | N | S | |
| H | S-n-$C_4H_9$ | $CH_3$ | $OCH_3$ | CH | O | |
| H | S-n-$C_4H_9$ | $OCH_3$ | $OCH_3$ | N | O | |
| H | S-n-$C_4H_9$ | $OCH_3$ | $OCH_3$ | CH | S | |
| H | S-n-$C_4H_9$ | $CH_3$ | $OCH_3$ | N | S | |
| H | $SCH_2CH=CH_2$ | $CH_3$ | $CH_3$ | CH | O | |
| H | $SCH_2CH=CH_2$ | $CH_3$ | $OCH_3$ | N | O | |
| H | $SCH_2CH=CH_2$ | $CH_3$ | $OCH_3$ | CH | O | |
| H | $SCH_2CH=CH_2$ | $OCH_3$ | $OCH_3$ | N | O | |
| H | $SCH_2CH=CH_2$ | $OCH_3$ | $OCH_3$ | CH | O | |
| H | $SCH_2CH=CH_2$ | $OCH_3$ | $OCH_3$ | N | O | |
| H | $SCH_2CH=CH_2$ | $OCH_3$ | Cl | CH | O | |
| H | $SCH_2CH=CH_2$ | $CH_3$ | $OCH_2CF_3$ | CH | O | |
| $CH_3$ | $SCH_2CH=CH_2$ | $OCH_3$ | $OCH_3$ | CH | S | |
| H | $SCH_2CH=CH_2$ | $CH_3$ | $CH_3$ | CH | S | |
| H | $SCH_2CH=CH_2$ | $CH_3$ | $CH_3$ | N | S | |
| H | $SCH_2CH=CH_2$ | $CH_3$ | $OCH_3$ | CH | S | |
| H | $SCH_2CH=CH_2$ | $CH_3$ | $OCH_3$ | N | S | |
| H | $SCH_2CH=CH_2$ | $OCH_3$ | $OCH_3$ | CH | S | |
| H | $SCH_2CH=CH_2$ | $OCH_3$ | $OCH_3$ | N | S | |
| H | $SCH_2CH=CH_2$ | $OCH_3$ | Cl | CH | S | |
| H | E-$SCH=CHCH_3$ | $CH_3$ | $OCH_3$ | CH | O | |
| H | E-$SCH=CHCH_3$ | $CH_3$ | $OCH_3$ | N | O | |

Table 3a (continued)

| R | R'3 | X | Y | Z | W' | m.p. °C |
|---|---|---|---|---|---|---|
| H | E-$SCH=CHCH_3$ | $OCH_3$ | $OCH_3$ | CH | O | |
| H | E-$SCH=CHCH_3$ | $OCH_3$ | $OCH_3$ | N | O | |
| H | E-$SCH=CHCH_3$ | $CH_3$ | $OCH_3$ | N | S | |
| H | E-$SCH=CHCH_3$ | $CH_3$ | $OCH_3$ | CH | S | |
| H | E-$SCH=CHCH_3$ | $OCH_3$ | $OCH_3$ | CH | S | |
| H | E-$SCH=CHCH_3$ | $OCH_3$ | Cl | CH | S | |
| H | Z-$SCH=CHCH_3$ | $CH_3$ | $OCH_3$ | N | O | |
| H | Z-$SCH=CHCH_3$ | $OCH_3$ | $OCH_3$ | CH | O | |
| H | Z-$SCH=CHCH_3$ | $OCH_3$ | $OCH_3$ | N | O | |
| H | Z-$SCH=CHCH_3$ | $OCH_3$ | Cl | CH | O | |
| H | Z-$SCH=CHCH_3$ | $CH_3$ | $CH_3$ | CH | S | |
| H | Z-$SCH=CHCH_3$ | $OCH_3$ | $OCH_3$ | CH | S | |
| H | Z-$SCH=CHCH_3$ | $CH_3$ | $OCH_3$ | N | S | |
| H | Z-$SCH=CHCH_3$ | $OCH_3$ | $OCH_3$ | N | S | |
| H | $S(CH_2)_2CH=CH_2$ | $OCH_3$ | $OCH_3$ | CH | O | |
| H | $S(CH_2)_2CH=CH_2$ | $OCH_3$ | $OCH_3$ | N | O | |
| H | $S(CH_2)_2CH=CH_2$ | $CH_3$ | $OCH_3$ | CH | O | |
| H | $S(CH_2)_2CH=CH_2$ | $OCH_3$ | Cl | CH | O | |
| H | $S(CH_2)_2CH=CH_2$ | $OCH_3$ | $OCH_3$ | CH | S | |
| H | $S(CH_2)_2CH=CH_2$ | $CH_3$ | $OCH_3$ | N | S | |
| H | $S(CH_2)_2CH=CH_2$ | $OCH_3$ | $OCH_3$ | N | S | |
| H | $S(CH_2)_2CH=CH_2$ | $CH_3$ | $CH_3$ | CH | S | |
| H | E-$SCH_2CH=CHCH_3$ | $OCH_3$ | $OCH_3$ | CH | O | |
| H | E-$SCH_2CH=CHCH_3$ | $OCH_3$ | $OCH_3$ | N | O | |
| H | E-$SCH_2CH=CHCH_3$ | $CH_3$ | $OCH_3$ | CH | O | |
| H | E-$SCH_2CH=CHCH_3$ | $OCH_3$ | Cl | CH | O | |
| H | E-$SCH_2CH=CHCH_3$ | $OCH_3$ | $OCH_3$ | CH | S | |
| H | E-$SCH_2CH=CHCH_3$ | $CH_3$ | $OCH_3$ | N | S | |
| H | E-$SCH_2CH=CHCH_3$ | $CH_3$ | $OCH_3$ | CH | S | |
| H | E-$SCH_2CH=CHCH_3$ | $CH_3$ | $CH_3$ | CH | S | |
| H | Z-$SCH_2CH=CHCH_3$ | $CH_3$ | $OCH_3$ | CH | O | |

Table 3a (continued)

| R | $R'_3$ | X | Y | Z | W' | m.p. °C |
|---|---|---|---|---|---|---|
| H | Z-$SCH_2CH=CHCH_3$ | $CH_3$ | $OCH_3$ | N | O | |
| H | Z-$SCH_2CH=CHCH_3$ | $OCH_3$ | $OCH_3$ | CH | O | |
| H | Z-$SCH_2CH=CHCH_3$ | $OCH_3$ | Cl | CH | O | |
| H | Z-$SCH_2CH=CHCH_3$ | $CH_3$ | $OCH_3$ | CH | S | |
| H | Z-$SCH_2CH=CHCH_3$ | $CH_3$ | $OCH_3$ | N | S | |
| H | Z-$SCH_2CH=CHCH_3$ | $OCH_3$ | $OCH_3$ | CH | S | |
| H | Z-$SCH_2CH=CHCH_3$ | $OCH_3$ | $OCH_3$ | N | S | |
| H | E-$SCH=CHC_2H_5$ | $OCH_3$ | $OCH_3$ | CH | O | |
| H | E-$SCH=CHC_2H_5$ | $OCH_3$ | $OCH_3$ | N | O | |
| H | E-$SCH=CHC_2H_5$ | $CH_3$ | $OCH_3$ | CH | O | |
| H | E-$SCH=CHC_2H_5$ | $OCH_3$ | Cl | CH | O | |
| H | E-$SCH=CHC_2H_5$ | $OCH_3$ | $OCH_3$ | CH | S | |
| H | E-$SCH=CHC_2H_5$ | $CH_3$ | $CH_3$ | CH | S | |
| H | E-$SCH=CHC_2H_5$ | $CH_3$ | $OCH_3$ | N | S | |
| H | E-$SCH=CHC_2H_5$ | $CH_3$ | $OCH_3$ | CH | S | |
| H | Z-$SCH=CHC_2H_5$ | $OCH_3$ | $OCH_3$ | CH | O | |
| H | Z-$SCH=CHC_2H_5$ | $OCH_3$ | $OCH_3$ | N | O | |
| H | Z-$SCH=CHC_2H_5$ | $CH_3$ | $CH_3$ | CH | O | |
| H | Z-$SCH=CHC_2H_5$ | $CH_3$ | $OCH_3$ | N | O | |
| H | Z-$SCH=CHC_2H_5$ | $OCH_3$ | $OCH_3$ | CH | S | |
| H | Z-$SCH=CHC_2H_5$ | $CH_3$ | $OCH_3$ | CH | S | |
| H | Z-$SCH=CHC_2H_5$ | $CH_3$ | $OCH_3$ | N | S | |
| H | Z-$SCH=CHC_2H_5$ | $OCH_3$ | Cl | CH | S | |
| H | $SCH_2C≡CH$ | $CH_3$ | $OCH_3$ | CH | O | |
| H | $SCH_2C≡CH$ | $CH_3$ | $OCH_3$ | N | O | |
| H | $SCH_2C≡CH$ | $OCH_3$ | $OCH_3$ | CH | O | |
| H | $SCH_2C≡CH$ | $OCH_3$ | $OCH_3$ | N | O | |
| H | $SCH_2C≡CH$ | $CH_3$ | $OCH_3$ | CH | S | |
| H | $SCH_2C≡CH$ | $OCH_3$ | $OCH_3$ | N | S | |
| H | $SCH_2C≡CH$ | $OCH_3$ | $OCH_3$ | CH | S | |
| H | $SCH_2C≡CH$ | $OCH_3$ | Cl | CH | S | |

Table 3a (continued)

| R | $R'_3$ | X | Y | Z | W' | m.p. °C |
|---|---|---|---|---|---|---|
| $CH_3$ | $SCH_2C{\equiv}CH$ | $OCH_3$ | $OCH_3$ | CH | S | |
| H | $SC{\equiv}CCH_3$ | $CH_3$ | $CH_3$ | CH | O | |
| H | $SC{\equiv}CCH_3$ | $CH_3$ | $OCH_3$ | N | O | |
| H | $SC{\equiv}CCH_3$ | $CH_3$ | $OCH_3$ | CH | O | |
| H | $SC{\equiv}CCH_3$ | $OCH_3$ | $OCH_3$ | N | O | |
| H | $SC{\equiv}CCH_3$ | $OCH_3$ | $OCH_3$ | CH | S | |
| H | $SC{\equiv}CCH_3$ | $CH_3$ | $OCH_3$ | N | S | |
| H | $SC{\equiv}CCH_3$ | $CH_3$ | $OCH_3$ | CH | S | |
| H | $SC{\equiv}CCH_3$ | $OCH_3$ | $OCH_3$ | N | S | |
| H | $S(CH_2)_2C{\equiv}CH$ | $CH_3$ | $OCH_3$ | CH | O | |
| H | $S(CH_2)_2C{\equiv}CH$ | $CH_3$ | $OCH_3$ | N | O | |
| H | $S(CH_2)_2C{\equiv}CH$ | $OCH_3$ | $OCH_3$ | CH | O | |
| H | $S(CH_2)_2C{\equiv}CH$ | $OCH_3$ | $OCH_3$ | N | O | |
| H | $S(CH_2)_2C{\equiv}CH$ | $CH_3$ | $OCH_3$ | CH | S | |
| H | $S(CH_2)_2C{\equiv}CH$ | $CH_3$ | $OCH_3$ | N | S | |
| H | $S(CH_2)_2C{\equiv}CH$ | $OCH_3$ | $OCH_3$ | CH | S | |
| H | $S(CH_2)_2C{\equiv}CH$ | $OCH_3$ | $OCH_3$ | N | S | |
| H | $SCH_2C{\equiv}CCH_3$ | $CH_3$ | $CH_3$ | CH | O | |
| H | $SCH_2C{\equiv}CCH_3$ | $CH_3$ | $OCH_3$ | N | O | |
| H | $SCH_2C{\equiv}CCH_3$ | $CH_3$ | $OCH_3$ | CH | O | |
| H | $SCH_2C{\equiv}CCH_3$ | $OCH_3$ | $OCH_3$ | CH | O | |
| H | $SCH_2C{\equiv}CCH_3$ | $OCH_3$ | $OCH_3$ | CH | S | |
| H | $SCH_2C{\equiv}CCH_3$ | $OCH_3$ | $OCH_3$ | N | S | |
| H | $SCH_2C{\equiv}CCH_3$ | $CH_3$ | $OCH_3$ | CH | S | |
| H | $SCH_2C{\equiv}CCH_3$ | $OCH_3$ | Cl | CH | S | |
| H | $SC{\equiv}CC_2H_5$ | $CH_3$ | $OCH_3$ | N | O | |
| H | $SC{\equiv}CC_2H_5$ | $OCH_3$ | $OCH_3$ | CH | O | |
| H | $SC{\equiv}CC_2H_5$ | $OCH_3$ | $OCH_3$ | CH | S | |
| H | $SC{\equiv}CC_2H_5$ | $CH_3$ | $CH_3$ | CH | S | |
| $CH_3$ | $SC{\equiv}CC_2H_5$ | $CH_3$ | $OCH_3$ | CH | O | |
| H | $CH_2CN$ | $CH_3$ | $CH_3$ | CH | O | |

Table 3a (continued)

| R | R'3 | X | Y | Z | W' | m.p. °C |
|---|---|---|---|---|---|---|
| H | $CH_2CN$ | $CH_3$ | $OCH_3$ | N | O | |
| H | $CH_2CN$ | $CH_3$ | $OCH_3$ | CH | O | |
| H | $CH_2CN$ | $OCH_3$ | $OCH_3$ | N | O | |
| H | $CH_2CN$ | $OCH_3$ | $OCH_3$ | CH | O | |
| H | $CH_2CN$ | $OCH_3$ | Cl | CH | O | |
| H | $CH_2CN$ | $CH_3$ | $NHCH_3$ | N | O | |
| $CH_3$ | $CH_2CN$ | $OCH_3$ | $OCH_3$ | CH | S | |
| H | $CH_2CN$ | $CH_3$ | $CH_3$ | CH | S | |
| H | $CH_2CN$ | $CH_3$ | $CH_3$ | N | S | |
| H | $CH_2CN$ | $CH_3$ | $OCH_3$ | CH | S | |
| H | $CH_2CN$ | $CH_3$ | $OCH_3$ | N | S | |
| H | $CH_2CN$ | $OCH_3$ | $OCH_3$ | CH | S | |
| H | $CH_2CN$ | $OCH_3$ | $OCH_3$ | N | S | |
| H | $CH_2CN$ | $OCH_3$ | Cl | CH | S | |
| $CH_3$ | $CH_2CO_2CH_3$ | $CH_3$ | $OCH_3$ | CH | O | |
| H | $CH_2CO_2CH_3$ | $CH_3$ | $CH_3$ | CH | O | |
| H | $CH_2CO_2CH_3$ | $CH_3$ | $OCH_3$ | CH | O | |
| H | $CH_2CO_2CH_3$ | $CH_3$ | $OCH_3$ | N | O | |
| H | $CH_2CO_2CH_3$ | $OCH_3$ | $OCH_3$ | CH | O | |
| H | $CH_2CO_2CH_3$ | $OCH_3$ | $OCH_3$ | N | O | |
| H | $CH_2CO_2CH_3$ | $OCH_3$ | Cl | CH | O | |
| H | $CH_2CO_2CH_3$ | $CH_3$ | $CH_3$ | CH | S | |
| H | $CH_2CO_2CH_3$ | $CH_3$ | $OCH_3$ | N | S | |
| H | $CH_2CO_2CH_3$ | $CH_3$ | $OCH_3$ | CH | S | |
| H | $CH_2CO_2CH_3$ | $OCH_3$ | $OCH_3$ | N | S | |
| H | $CH_2CO_2CH_3$ | $OCH_3$ | $OCH_3$ | CH | S | |
| H | $CH_2CO_2CH_3$ | $OCH_3$ | Cl | CH | S | |
| H | $CH_2CO_2CH_3$ | $CH_3$ | $N(CH_3)_2$ | N | S | |
| H | $CH_2CO_2C_2H_5$ | $CH_3$ | $CH_3$ | CH | O | |
| H | $CH_2CO_2C_2H_5$ | $CH_3$ | $OCH_3$ | N | O | |
| H | $CH_2CO_2C_2H_5$ | $CH_3$ | $OCH_3$ | CH | O | |

Table 3a (continued)

| R | $R'_3$ | X | Y | Z | W' | m.p. °C |
|---|---|---|---|---|---|---|
| H | $CH_2CO_2C_2H_5$ | $OCH_3$ | $OCH_3$ | N | O | |
| H | $CH_2CO_2C_2H_5$ | $OCH_3$ | $OCH_3$ | CH | O | |
| H | $CH_2CO_2C_2H_5$ | $OCH_3$ | Cl | CH | O | |
| H | $CH_2CO_2C_2H_5$ | $OC_2H_5$ | Cl | CH | O | |
| $CH_3$ | $CH_2CO_2C_2H_5$ | $OCH_3$ | $OCH_3$ | CH | S | |
| H | $CH_2CO_2C_2H_5$ | $CH_3$ | $CH_3$ | CH | S | |
| H | $CH_2CO_2C_2H_5$ | $CH_3$ | $CH_3$ | N | S | |
| H | $CH_2CO_2C_2H_5$ | $CH_3$ | $OCH_3$ | CH | S | |
| H | $CH_2CO_2C_2H_5$ | $CH_3$ | $OCH_3$ | N | S | |
| H | $CH_2CO_2C_2H_5$ | $OCH_3$ | $OCH_3$ | CH | S | |
| H | $CH_2CO_2C_2H_5$ | $OCH_3$ | $OCH_3$ | N | S | |
| H | $CH_2CO_2C_2H_5$ | $OCH_3$ | Cl | CH | S | |
| $CH_3$ | $CH_2OCH_3$ | $OCH_3$ | $OCH_3$ | CH | O | |
| H | $CH_2OCH_3$ | $CH_3$ | $CH_3$ | CH | O | |
| H | $CH_2OCH_3$ | $CH_3$ | $CH_3$ | N | O | |
| H | $CH_2OCH_3$ | $CH_3$ | $OCH_3$ | CH | O | |
| H | $CH_2OCH_3$ | $CH_3$ | $OCH_3$ | N | O | |
| H | $CH_2OCH_3$ | $OCH_3$ | $OCH_3$ | CH | O | |
| H | $CH_2OCH_3$ | $OCH_3$ | $OCH_3$ | N | O | |
| H | $CH_2OCH_3$ | $OCH_3$ | Cl | CH | O | |
| H | $CH_2OCH_3$ | $CH_3$ | $CH_3$ | CH | S | |
| H | $CH_2OCH_3$ | $CH_3$ | $OCH_3$ | N | S | |
| H | $CH_2OCH_3$ | $CH_3$ | $OCH_3$ | CH | S | |
| H | $CH_2OCH_3$ | $OCH_3$ | $OCH_3$ | N | S | |
| H | $CH_2OCH_3$ | $OCH_3$ | $OCH_3$ | CH | S | |
| H | $CH_2OCH_3$ | $OCH_3$ | Cl | CH | S | |
| H | $CH_2OCH_3$ | $CH_3$ | $CH(OCH_3)_2$ | CH | S | |
| H | $CH_2OC_2H_5$ | $CH_3$ | $CH_3$ | CH | O | |
| H | $CH_2OC_2H_5$ | $CH_3$ | $OCH_3$ | N | O | |
| H | $CH_2OC_2H_5$ | $CH_3$ | $OCH_3$ | CH | O | |
| H | $CH_2OC_2H_5$ | $OCH_3$ | $OCH_3$ | N | O | |

Table 3a (continued)

| R | R'3 | X | Y | Z | W' | m.p. °C |
|---|---|---|---|---|---|---|
| H | $CH_2OC_2H_5$ | $OCH_3$ | $OCH_3$ | CH | O | |
| H | $CH_2OC_2H_5$ | $OCH_3$ | Cl | CH | O | |
| H | $CH_2OC_2H_5$ | $OCH_3$ | Br | CH | O | |
| $CH_3$ | $CH_2OC_2H_5$ | $CH_3$ | $OCH_3$ | CH | S | |
| H | $CH_2OC_2H_5$ | $CH_3$ | $CH_3$ | CH | S | |
| H | $CH_2OC_2H_5$ | $CH_3$ | $CH_3$ | N | S | |
| H | $CH_2OC_2H_5$ | $CH_3$ | $OCH_3$ | CH | S | |
| H | $CH_2OC_2H_5$ | $CH_3$ | $OCH_3$ | N | S | |
| H | $CH_2OC_2H_5$ | $OCH_3$ | $OCH_3$ | CH | S | |
| H | $CH_2OC_2H_5$ | $OCH_3$ | $OCH_3$ | N | S | |
| H | $CH_2OC_2H_5$ | $OCH_3$ | Cl | CH | S | |
| $CH_3$ | $(CH_2)_2COCH_3$ | $OCH_3$ | $OCH_3$ | CH | O | |
| H | $(CH_2)_2COCH_3$ | $CH_3$ | $CH_3$ | CH | O | |
| H | $(CH_2)_2COCH_3$ | $CH_3$ | $CH_3$ | N | O | |
| H | $(CH_2)_2COCH_3$ | $CH_3$ | $OCH_3$ | CH | O | |
| H | $(CH_2)_2COCH_3$ | $CH_3$ | $OCH_3$ | N | O | |
| H | $(CH_2)_2COCH_3$ | $OCH_3$ | $OCH_3$ | CH | O | |
| H | $(CH_2)_2COCH_3$ | $OCH_3$ | $OCH_3$ | N | O | |
| H | $(CH_2)_2COCH_3$ | $OCH_3$ | Cl | CH | O | |
| H | $(CH_2)_2COCH_3$ | $CH_3$ | $CH_3$ | CH | S | |
| H | $(CH_2)_2COCH_3$ | $CH_3$ | $OCH_3$ | N | S | |
| H | $(CH_2)_2COCH_3$ | $CH_3$ | $OCH_3$ | CH | S | |
| H | $(CH_2)_2COCH_3$ | $OCH_3$ | $OCH_3$ | N | S | |
| H | $(CH_2)_2COCH_3$ | $OCH_3$ | $OCH_3$ | CH | S | |
| H | $(CH_2)_2COCH_3$ | $OCH_3$ | Cl | CH | S | |
| H | $(CH_2)_2COCH_3$ | $CH_3$ | 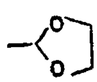 | CH | S | |
| H | $(CH_2)_2CN$ | $CH_3$ | $OCH_3$ | CH | O | |
| H | $(CH_2)_2CN$ | $CH_3$ | $OCH_3$ | N | O | |
| H | $(CH_2)_2CN$ | $OCH_3$ | $OCH_3$ | CH | O | |
| H | $(CH_2)_2CN$ | $OCH_3$ | $OCH_3$ | N | O | |

Table 3a (continued)

| R | $R'_3$ | X | Y | Z | W' | m.p. °C |
|---|---|---|---|---|---|---|
| H | $(CH_2)_2CN$ | $CH_3$ | $CH_3$ | CH | S | |
| H | $(CH_2)_2CN$ | $CH_3$ | $OCH_3$ | N | S | |
| H | $(CH_2)_2CN$ | $OCH_3$ | $OCH_3$ | CH | S | |
| H | $(CH_2)_2CN$ | $OCH_3$ | Cl | CH | S | |
| H | $CH(CH_3)CN$ | $OCH_3$ | $OCH_3$ | CH | O | |
| H | $CH(CH_3)CN$ | $OCH_3$ | $OCH_3$ | N | O | |
| H | $CH(CH_3)CN$ | $CH_3$ | $OCH_3$ | CH | S | |
| H | $CH(CH_3)CN$ | $CH_3$ | $OCH_3$ | N | S | |
| H | $(CH_2)_3CN$ | $CH_3$ | $OCH_3$ | CH | O | |
| H | $(CH_2)_3CN$ | $CH_3$ | $OCH_3$ | N | O | |
| H | $(CH_2)_3CN$ | $OCH_3$ | $OCH_3$ | CH | O | |
| H | $(CH_2)_3CN$ | $OCH_3$ | $OCH_3$ | N | O | |
| H | $(CH_2)_3CN$ | $CH_3$ | $CH_3$ | CH | S | |
| H | $(CH_2)_3CN$ | $CH_3$ | $OCH_3$ | N | S | |
| H | $(CH_2)_3CN$ | $OCH_3$ | $OCH_3$ | $CH_3$ | S | |
| H | $(CH_2)_3CN$ | $OCH_3$ | $OCH_3$ | N | S | |
| H | $CH_2CH(CH_3)CN$ | $CH_3$ | $OCH_3$ | CH | O | |
| H | $CH_2CH(CH_3)CN$ | $CH_3$ | $OCH_3$ | N | O | |
| H | $CH_2CH(CH_3)CN$ | $OCH_3$ | $OCH_3$ | CH | S | |
| H | $CH_2CH(CH_3)CN$ | $OCH_3$ | Cl | CH | S | |
| H | $CH_2CH(CH_3)CN$ | $CH_3$ | $CH_3$ | CH | O | |
| H | $CH_2CH(CH_3)CN$ | $CH_3$ | $OCH_3$ | N | O | |
| H | $CH_2CH(CH_3)CN$ | $OCH_3$ | $OCH_3$ | CH | S | |
| H | $CH_2CH(CH_3)CN$ | $OCH_3$ | $CH_3$ | CH | S | |
| $CH_3$ | $CH_2CH(CH_3)CN$ | $OCH_3$ | $OCH_3$ | CH | S | |
| H | $CH(CN)C_2H_5$ | $CH_3$ | $OCH_3$ | N | O | |
| H | $CH(CN)C_2H_5$ | $OCH_3$ | $OCH_3$ | CH | O | |
| H | $CH(CN)C_2H_5$ | $OCH_3$ | $OCH_3$ | N | S | |
| H | $CH(CN)C_2H_5$ | $OCH_3$ | Cl | CH | S | |
| H | $C(CH_3)_2CN$ | $CH_3$ | $CH_3$ | CH | O | |
| H | $C(CH_3)_2CN$ | $OCH_3$ | $OCH_3$ | CH | O | |

Table 3a (continued)

| R | $R'_3$ | X | Y | Z | W' | m.p. °C |
|---|---|---|---|---|---|---|
| H | $C(CH_3)_2CN$ | $OCH_3$ | $OCH_3$ | N | S | |
| H | $C(CH_3)_2CN$ | $OCH_3$ | $OCH_3$ | CH | S | |
| H | $CH(CH_3)CO_2CH_3$ | $OCH_3$ | $OCH_3$ | CH | O | |
| H | $CH(CH_3)CO_2CH_3$ | $OCH_3$ | $OCH_3$ | N | O | |
| H | $CH(CH_3)CO_2CH_3$ | $CH_3$ | $OCH_3$ | CH | S | |
| H | $CH(CH_3)CO_2CH_3$ | $CH_3$ | $OCH_3$ | N | S | |
| H | $(CH_2)_3CO_2CH_3$ | $CH_3$ | $OCH_3$ | CH | O | |
| H | $(CH_2)_3CO_2CH_3$ | $CH_3$ | $OCH_3$ | N | O | |
| H | $(CH_2)_3CO_2CH_3$ | $OCH_3$ | $OCH_3$ | CH | O | |
| H | $(CH_2)_3CO_2CH_3$ | $OCH_3$ | $OCH_3$ | N | O | |
| H | $(CH_2)_3CO_2CH_3$ | $CH_3$ | $OCH_3$ | CH | S | |
| H | $(CH_2)_3CO_2CH_3$ | $CH_3$ | $OCH_3$ | N | S | |
| H | $(CH_2)_3CO_2CH_3$ | $OCH_3$ | $OCH_3$ | CH | S | |
| H | $(CH_2)_3CO_2CH_3$ | $OCH_3$ | $OCH_3$ | N | S | |
| H | $CH_2CH(CH_3)CO_2CH_3$ | $CH_3$ | $OCH_3$ | CH | O | |
| H | $CH_2CH(CH_3)CO_2CH_3$ | $CH_3$ | $OCH_3$ | N | O | |
| H | $CH_2CH(CH_3)CO_2CH_3$ | $OCH_3$ | $OCH_3$ | CH | S | |
| H | $CH_2CH(CH_3)CO_2CH_3$ | $OCH_3$ | $OCH_3$ | N | S | |
| H | $CH_2CH(CH_3)CO_2CH_3$ | $CH_3$ | $OCH_3$ | CH | O | |
| H | $CH_2CH(CH_3)CO_2CH_3$ | $CH_3$ | $OCH_3$ | N | O | |
| H | $CH_2CH(CH_3)CO_2CH_3$ | $OCH_3$ | $OCH_3$ | CH | S | |
| H | $CH_2CH(CH_3)CO_2CH_3$ | $OCH_3$ | $OCH_3$ | N | S | |
| H | $CH(CO_2CH_3)C_2H_5$ | $CH_3$ | $OCH_3$ | N | O | |
| H | $CH(CO_2CH_3)C_2H_5$ | $OCH_3$ | $OCH_3$ | CH | O | |
| H | $CH(CO_2CH_3)C_2H_5$ | $OCH_3$ | $OCH_3$ | N | S | |
| H | $CH(CO_2CH_3)C_2H_5$ | $OCH_3$ | $OCH_3$ | CH | S | |
| H | $C(CH_3)_2CO_3CH_3$ | $CH_3$ | $OCH_3$ | N | O | |
| H | $C(CH_3)_2CO_3CH_3$ | $OCH_3$ | $OCH_3$ | CH | O | |
| H | $C(CH_3)_2CO_3CH_3$ | $CH_3$ | $OCH_3$ | N | S | |
| H | $C(CH_3)_2CO_3CH_3$ | $OCH_3$ | $OCH_3$ | CH | S | |

Table 3a (continued)

| R | $R'_3$ | X | Y | Z | W' | m.p. °C |
|---|---|---|---|---|---|---|
| H | $(CH_2)_2CO_2C_2H_5$ | $CH_3$ | $CH_3$ | CH | O | |
| H | $(CH_2)_2CO_2C_2H_5$ | $CH_3$ | $OCH_3$ | N | O | |
| H | $(CH_2)_2CO_2C_2H_5$ | $OCH_3$ | $OCH_3$ | CH | O | |
| H | $(CH_2)_2CO_2C_2H_5$ | $OCH_3$ | $OCH_3$ | N | O | |
| H | $(CH_2)_2CO_2C_2H_5$ | $OCH_3$ | $OCH_3$ | CH | S | |
| H | $(CH_2)_2CO_2C_2H_5$ | $CH_3$ | $OCH_3$ | N | S | |
| H | $(CH_2)_2CO_2C_2H_5$ | $OCH_3$ | $OCH_3$ | CH | S | |
| H | $(CH_2)_2CO_2C_2H_5$ | $OCH_3$ | $OCH_3$ | N | S | |
| H | $CH(CH_3)CO_2C_2H_5$ | $OCH_3$ | $OCH_3$ | CH | O | |
| H | $CH(CH_3)CO_2C_2H_5$ | $OCH_3$ | $OCH_3$ | N | O | |
| H | $CH(CH_3)CO_2C_2H_5$ | $CH_3$ | $OCH_3$ | CH | S | |
| H | $CH(CH_3)CO_2C_2H_5$ | $CH_3$ | $OCH_3$ | N | S | |
| H | $(CH_2)_3CO_2C_2H_5$ | $CH_3$ | $OCH_3$ | CH | O | |
| H | $(CH_2)_3CO_2C_2H_5$ | $CH_3$ | $OCH_3$ | N | O | |
| H | $(CH_2)_3CO_2C_2H_5$ | $OCH_3$ | $OCH_3$ | CH | O | |
| H | $(CH_2)_3CO_2C_2H_5$ | $OCH_3$ | $OCH_3$ | N | O | |
| H | $(CH_2)_3CO_2C_2H_5$ | $CH_3$ | $OCH_3$ | CH | S | |
| H | $(CH_2)_3CO_2C_2H_5$ | $CH_3$ | $OCH_3$ | N | S | |
| H | $(CH_2)_3CO_2C_2H_5$ | $OCH_3$ | $OCH_3$ | CH | S | |
| H | $(CH_2)_3CO_2C_2H_5$ | $OCH_3$ | $OCH_3$ | N | S | |
| H | $CH_2CH(CH_3)CO_2C_2H_5$ | $CH_3$ | $OCH_3$ | CH | O | |
| H | $CH_2CH(CH_3)CO_2C_2H_5$ | $CH_3$ | $OCH_3$ | N | O | |
| H | $CH_2CH(CH_3)CO_2C_2H_5$ | $OCH_3$ | $OCH_3$ | CH | S | |
| H | $CH_2CH(CH_3)CO_2C_2H_5$ | $OCH_3$ | $OCH_3$ | N | S | |
| H | $CH_2CH(CH_3)CO_2C_2H_5$ | $CH_3$ | $OCH_3$ | CH | O | |
| H | $CH_2CH(CH_3)CO_2C_2H_5$ | $CH_3$ | $OCH_3$ | N | O | |
| H | $CH_2CH(CH_3)CO_2C_2H_5$ | $CH_3$ | $OCH_3$ | CH | S | |
| H | $CH_2CH(CH_3)CO_2C_2H_5$ | $OCH_3$ | $OCH_3$ | N | S | |
| H | $CH(CO_2C_2H_5)C_2H_5$ | $CH_3$ | $OCH_3$ | N | O | |
| H | $CH(CO_2C_2H_5)C_2H_5$ | $OCH_3$ | $OCH_3$ | CH | O | |
| H | $CH(CO_2C_2H_5)C_2H_5$ | $OCH_3$ | $OCH_3$ | N | S | |

Table 3a (continued)

| R | $R'_3$ | X | Y | Z | W' | m.p. °C |
|---|---|---|---|---|---|---|
| H | $CH(CO_2C_2H_5)C_2H_5$ | $OCH_3$ | $OCH_3$ | CH | S | |
| H | $C(CH_3)_2CO_2C_2H_5$ | $CH_3$ | $OCH_3$ | N | O | |
| H | $C(CH_3)_2CO_2C_2H_5$ | $OCH_3$ | $OCH_3$ | CH | O | |
| H | $C(CH_3)_2CO_2C_2H_5$ | $CH_3$ | $OCH_3$ | N | O | |
| H | $C(CH_3)_2CO_2C_2H_5$ | $OCH_3$ | $OCH_3$ | CH | S | |
| H | $(CH_2)_2OCH_3$ | $CH_3$ | $CH_3$ | CH | O | |
| H | $(CH_2)_2OCH_3$ | $CH_3$ | $OCH_3$ | N | O | |
| H | $(CH_2)_2OCH_3$ | $OCH_3$ | $OCH_3$ | CH | O | |
| H | $(CH_2)_2OCH_3$ | $OCH_3$ | $OCH_3$ | N | O | |
| H | $(CH_2)_2OCH_3$ | $OCH_3$ | $OCH_3$ | CH | S | |
| H | $(CH_2)_2OCH_3$ | $CH_3$ | $OCH_3$ | N | S | |
| H | $(CH_2)_2OCH_3$ | $OCH_3$ | $OCH_3$ | CH | S | |
| H | $(CH_2)_2OCH_3$ | $OCH_3$ | $OCH_3$ | N | S | |
| H | $CH(CH_3)OCH_3$ | $OCH_3$ | $OCH_3$ | CH | O | |
| H | $CH(CH_3)OCH_3$ | $OCH_3$ | $OCH_3$ | N | O | |
| H | $CH(CH_3)OCH_3$ | $CH_3$ | $OCH_3$ | CH | S | |
| H | $CH(CH_3)OCH_3$ | $CH_3$ | $OCH_3$ | N | S | |
| H | $(CH_2)_3OCH_3$ | $CH_3$ | $OCH_3$ | CH | O | |
| H | $(CH_2)_3OCH_3$ | $CH_3$ | $OCH_3$ | N | O | |
| H | $(CH_2)_3OCH_3$ | $OCH_3$ | $OCH_3$ | CH | O | |
| H | $(CH_2)_3OCH_3$ | $OCH_3$ | $OCH_3$ | N | O | |
| H | $(CH_2)_3OCH_3$ | $CH_3$ | $OCH_3$ | CH | S | |
| H | $(CH_2)_3OCH_3$ | $CH_3$ | $OCH_3$ | N | S | |
| H | $(CH_2)_3OCH_3$ | $OCH_3$ | $OCH_3$ | CH | S | |
| H | $(CH_2)_3OCH_3$ | $OCH_3$ | $OCH_3$ | N | S | |
| H | $CH_2CH(CH_3)OCH_3$ | $CH_3$ | $OCH_3$ | CH | O | |
| H | $CH_2CH(CH_3)OCH_3$ | $CH_3$ | $OCH_3$ | N | O | |
| H | $CH_2CH(CH_3)OCH_3$ | $OCH_3$ | $OCH_3$ | CH | S | |
| H | $CH_2CH(CH_3)OCH_3$ | $OCH_3$ | $OCH_3$ | N | S | |
| H | $CH_2CH(CH_3)OCH_3$ | $CH_3$ | $OCH_3$ | CH | O | |
| H | $CH_2CH(CH_3)OCH_3$ | $CH_3$ | $OCH_3$ | N | O | |

**Table 3a (continued)**

| R | $R'_3$ | X | Y | Z | W' | m.p. °C |
|---|---|---|---|---|---|---|
| H | $CH_2CH(CH_3)OCH_3$ | $CH_3$ | $OCH_3$ | CH | S | |
| H | $CH_2CH(CH_3)OCH_3$ | $OCH_3$ | $OCH_3$ | N | S | |
| H | $CH(OCH_3)C_2H_5$ | $CH_3$ | $OCH_3$ | N | O | |
| H | $CH(OCH_3)C_2H_5$ | $OCH_3$ | $OCH_3$ | CH | O | |
| H | $CH(OCH_3)C_2H_5$ | $OCH_3$ | $OCH_3$ | N | S | |
| H | $CH(OCH_3)C_2H_5$ | $OCH_3$ | $OCH_3$ | CH | S | |
| H | $C(CH_3)_2OCH_3$ | $CH_3$ | $OCH_3$ | N | O | |
| H | $C(CH_3)_2OCH_3$ | $OCH_3$ | $OCH_3$ | CH | O | |
| H | $C(CH_3)_2OCH_3$ | $CH_3$ | $OCH_3$ | N | r | |
| H | $C(CH_3)_2OCH_3$ | $OCH_3$ | $OCH_3$ | CH | S | |
| H | $(CH_2)_2OC_2H_5$ | $CH_3$ | $CH_3$ | CH | O | |
| H | $(CH_2)_2OC_2H_5$ | $CH_3$ | $OCH_3$ | N | O | |
| H | $(CH_2)_2OC_2H_5$ | $OCH_3$ | $OCH_3$ | CH | O | |
| H | $(CH_2)_2OC_2H_5$ | $OCH_3$ | $OCH_3$ | N | O | |
| H | $(CH_2)_2OC_2H_5$ | $OCH_3$ | $OCH_3$ | CH | S | |
| H | $(CH_2)_2OC_2H_5$ | $CH_3$ | $OCH_3$ | N | S | |
| H | $(CH_2)_2OC_2H_5$ | $OCH_3$ | $OCH_3$ | CH | S | |
| H | $(CH_2)_2OC_2H_5$ | $OCH_3$ | $OCH_3$ | N | S | |
| H | $CH(CH_3)OC_2H_5$ | $OCH_3$ | $OCH_3$ | CH | O | |
| H | $CH(CH_3)OC_2H_5$ | $OCH_3$ | $OCH_3$ | N | O | |
| H | $CH(CH_3)OC_2H_5$ | $CH_3$ | $OCH_3$ | CH | S | |
| H | $CH(CH_3)OC_2H_5$ | $CH_3$ | $OCH_3$ | N | S | |
| H | $(CH_2)_3OC_2H_5$ | $CH_3$ | $OCH_3$ | CH | O | |
| H | $(CH_2)_3OC_2H_5$ | $CH_3$ | $OCH_3$ | N | O | |
| H | $(CH_2)_3OC_2H_5$ | $OCH_3$ | $OCH_3$ | CH | O | |
| H | $(CH_2)_3OC_2H_5$ | $OCH_3$ | $OCH_3$ | N | O | |
| H | $(CH_2)_3OC_2H_5$ | $CH_3$ | $OCH_3$ | CH | S | |
| H | $(CH_2)_3OC_2H_5$ | $CH_3$ | $OCH_3$ | N | S | |
| H | $(CH_2)_3OC_2H_5$ | $OCH_3$ | $OCH_3$ | CH | S | |
| H | $(CH_2)_3OC_2H_5$ | $OCH_3$ | $OCH_3$ | N | S | |
| H | $CH_2CH(CH_3)OC_2H_5$ | $CH_3$ | $OCH_3$ | CH | O | |

Table 3a (continued)

| R | R'3 | X | Y | Z | W' | m.p. °C |
|---|---|---|---|---|---|---|
| H | $CH_2CH(CH_3)OC_2H_5$ | $CH_3$ | $OCH_3$ | N | O | |
| H | $CH_2CH(CH_3)OC_2H_5$ | $OCH_3$ | $OCH_3$ | CH | S | |
| H | $CH_2CH(CH_3)OC_2H_5$ | $OCH_3$ | $OCH_3$ | N | S | |
| H | $CH(CH_3)CH_2OC_2H_5$ | $CH_3$ | $OCH_3$ | CH | O | |
| H | $CH(CH_3)CH_2OC_2H_5$ | $CH_3$ | $OCH_3$ | N | O | |
| H | $CH(CH_3)CH_2OC_2H_5$ | $CH_3$ | $OCH_3$ | CH | S | |
| H | $CH(CH_3)CH_2OC_2H_5$ | $OCH_3$ | $OCH_3$ | N | S | |
| H | $CH(OC_2H_5)C_2H_5$ | $CH_3$ | $OCH_3$ | N | O | |
| H | $CH(OC_2H_5)C_2H_5$ | $OCH_3$ | $OCH_3$ | CH | O | |
| H | $CH(OC_2H_5)C_2H_5$ | $OCH_3$ | $OCH_3$ | N | S | |
| H | $CH(OC_2H_5)C_2H_5$ | $OCH_3$ | $OCH_3$ | CH | S | |
| H | $CH(CH_3)_2OC_2H_5$ | $CH_3$ | $OCH_3$ | N | O | |
| H | $CH(CH_3)_2OC_2H_5$ | $OCH_3$ | $OCH_3$ | CH | O | |
| H | $CH(CH_3)_2OC_2H_5$ | $CH_3$ | $OCH_3$ | N | O | |
| H | $C(CH_3)_2OC_2H_5$ | $OCH_3$ | $OCH_3$ | CH | S | |
| H | $(CH_2)CH_2COCH_3$ | $CH_3$ | $CH_3$ | CH | O | |
| H | $(CH_2)CH_2COCH_3$ | $CH_3$ | $OCH_3$ | N | O | |
| H | $(CH_2)CH_2COCH_3$ | $OCH_3$ | $OCH_3$ | CH | O | |
| H | $(CH_2)CH_2COCH_3$ | $OCH_3$ | $OCH_3$ | N | O | |
| H | $(CH_2)CH_2COCH_3$ | $OCH_3$ | $OCH_3$ | CH | S | |
| H | $(CH_2)CH_2COCH_3$ | $CH_3$ | $OCH_3$ | N | S | |
| H | $(CH_2)CH_2COCH_3$ | $OCH_3$ | $OCH_3$ | CH | S | |
| H | $(CH_2)CH_2COCH_3$ | $OCH_3$ | $OCH_3$ | N | S | |
| H | $CH(CH_3)CH_2COCH_3$ | $OCH_3$ | $OCH_3$ | CH | O | |
| H | $CH(CH_3)CH_2COCH_3$ | $OCH_3$ | $OCH_3$ | N | O | |
| H | $CH(CH_3)CH_2COCH_3$ | $CH_3$ | $OCH_3$ | CH | S | |
| H | $CH(CH_3)CH_2COCH_3$ | $CH_3$ | $OCH_3$ | N | S | |
| H | $(CH_2)_3CH_2COCH_3$ | $CH_3$ | $OCH_3$ | CH | O | |
| H | $(CH_2)_3CH_2COCH_3$ | $CH_3$ | $OCH_3$ | N | O | |
| H | $(CH_2)_3CH_2COCH_3$ | $OCH_3$ | $OCH_3$ | CH | O | |
| H | $(CH_2)_3CH_2COCH_3$ | $OCH_3$ | $OCH_3$ | N | O | |

Table 3a (continued)

| R | $R'_3$ | X | Y | Z | W' | m.p. °C |
|---|---|---|---|---|---|---|
| H | $(CH_2)_3CH_2COCH_3$ | $CH_3$ | $OCH_3$ | CH | S | |
| H | $(CH_2)_3CH_2COCH_3$ | $CH_3$ | $OCH_3$ | N | S | |
| H | $(CH_2)_3CH_2COCH_3$ | $OCH_3$ | $OCH_3$ | CH | S | |
| H | $(CH_2)_3CH_2COCH_3$ | $OCH_3$ | $OCH_3$ | N | S | |
| H | $CH_2CH(CH_3)CH_2COCH_3$ | $CH_3$ | $OCH_3$ | CH | O | |
| H | $CH_2CH(CH_3)CH_2COCH_3$ | $CH_3$ | $OCH_3$ | N | O | |
| H | $CH_2CH(CH_3)CH_2COCH_3$ | $OCH_3$ | $OCH_3$ | CH | S | |
| H | $CH_2CH(CH_3)CH_2COCH_3$ | $OCH_3$ | $OCH_3$ | N | S | |
| H | $CH(CH_3)CH_2CH_2COCH_3$ | $CH_3$ | $OCH_3$ | CH | O | |
| H | $CH(CH_3)CH_2CH_2COCH_3$ | $CH_3$ | $OCH_3$ | N | O | |
| H | $CH(CH_3)CH_2CH_2COCH_3$ | $CH_3$ | $OCH_3$ | CH | S | |
| H | $CH(CH_3)CH_2CH_2COCH_3$ | $OCH_3$ | $OCH_3$ | N | S | |
| H | $CH(C_2H_5)CH_2COCH_3$ | $CH_3$ | $OCH_3$ | N | O | |
| H | $CH(C_2H_5)CH_2OCH_3$ | $OCH_3$ | $OCH_3$ | CH | O | |
| H | $CH(C_2H_5)CH_2OCH_3$ | $OCH_3$ | $OCH_3$ | N | S | |
| H | $CH(C_2H_5)CH_2OCH_3$ | $OCH_3$ | $OCH_3$ | CH | S | |
| H | $CH(CH_3)_2CH_2COCH_3$ | $CH_3$ | $OCH_3$ | N | O | |
| H | $CH(CH_3)_2CH_2COCH_3$ | $OCH_3$ | $OCH_3$ | CH | O | |
| H | $CH(CH_3)_2CH_2COCH_3$ | $CH_3$ | $OCH_3$ | N | O | |
| H | $C(CH_3)_2CH_2COCH_3$ | $OCH_3$ | $OCH_3$ | CH | S | |
| H | H | $OCH_3$ | $OCH_3$ | CH | O * | |
| H | H | $OCH_3$ | $OCH_3$ | CH | S * | |
| H | $CH_3$ | $OCH_3$ | $OCH_3$ | CH | O * | |
| H | $CH_3$ | $OCH_3$ | $OCH_3$ | CH | S * | |
| H | $SCH_3$ | $OCH_3$ | $OCH_3$ | CH | O * | |
| H | $SCH_3$ | $OCH_3$ | $OCH_3$ | CH | S * | |
| H | $SCH_2CH=CH_2$ | $OCH_3$ | $OCH_3$ | CH | O * | |
| H | $SCH_3CH=CH_2$ | $OCH_3$ | $OCH_3$ | CH | S * | |
| H | SH | $OCH_3$ | $OCH_3$ | CH | O * | |
| H | SH | $OCH_3$ | $OCH_3$ | CH | S * | |

W is O, unless indicated by * where W is S in all Tables.

Table 3b

General Structure 1 wherein Q is Q-28.

| R | R3' | R4 | X | Y | Z | m.p. °C |
|---|---|---|---|---|---|---|
| H | H | H | $CH_3$ | $OCH_3$ | CH | |
| H | H | H | $CH_3$ | $OCH_3$ | N | |
| H | H | H | $OCH_3$ | $OCH_3$ | CH | |
| H | H | H | $OCH_3$ | $OCH_3$ | N | |
| H | H | H | $CH_3$ | $CH_3$ | CH | |
| H | H | H | $CH_3$ | $CH_3$ | N | |
| H | $CH_3$ | H | $CH_3$ | $OCH_3$ | CH | |
| H | H | H | Br | $OCH_3$ | CH | |
| H | H | $CH_3$ | $CH_3$ | $OCH_3$ | N | |
| H | $CH_3$ | $CH_3$ | $OCH_3$ | $OCH_3$ | CH | |
| $CH_3$ | $CH_3$ | $CH_3$ | $OCH_3$ | $OCH_3$ | N | |
| H | H | H | $CH_3$ | 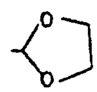 | CH | |
| H | H | H | $CH_3$ | $C_2H_5$ | CH | |
| H | $CH_3$ | H | $CH_3$ | $CH_3$ | CH | 220-222 |
| H | $CH_3$ | H | $CH_3$ | $OCH_3$ | N | 189-192 |
| H | $CH_3$ | H | $OCH_3$ | $OCH_3$ | CH | 198-207 |
| H | $CH_3$ | H | $OCH_3$ | $OCH_3$ | N | |
| H | $CH_3$ | H | $OCH_3$ | Cl | CH | 217-218 |
| H | $CH_3$ | H | $CH_3$ | $CH_3$ | N | |
| H | Cl | H | $CH_3$ | $CH_3$ | CH | 211-213 |
| H | Cl | H | $CH_3$ | $CH_3$ | N | 195-197 |
| H | Cl | H | $CH_3$ | $OCH_3$ | CH | |
| H | Cl | H | $CH_3$ | $OCH_3$ | N | 175-177 |
| H | Cl | H | $OCH_3$ | $OCH_3$ | CH | 204-205 |
| H | Cl | H | $OCH_3$ | $OCH_3$ | N | 187-188 |
| H | Cl | H | $OCH_3$ | Cl | CH | 205-206 |
| H | Cl | $CH_3$ | $CH_3$ | $OCH_3$ | CH | |
| H | Cl | $CH_3$ | $CH_3$ | $OCH_3$ | N | |
| H | Cl | $CH_3$ | $OCH_3$ | $OCH_3$ | CH | |

Table 3b (continued)

| R | R3' | R4 | X | Y | Z | m.p. °C |
|---|---|---|---|---|---|---|
| H | Cl | $CH_3$ | $OCH_3$ | $OCH_3$ | N | |
| H | Br | H | $OCH_3$ | $OCH_3$ | CH | |
| H | Br | H | $CH_3$ | $CH_3$ | CH | |
| H | Br | H | $CH_3$ | $OCH_3$ | N | |
| H | Br | H | $CH_3$ | $OCH_3$ | CH | |
| H | Br | H | $OCH_3$ | $OCH_3$ | N | |
| H | Br | H | $OCH_3$ | $OCH_3$ | CH | |
| H | Br | H | $OCH_3$ | Cl | CH | |
| H | H | H | $OCH_3$ | $OCH_3$ | CH * | |
| H | $CH_3$ | H | $OCH_3$ | $OCH_3$ | CH * | |
| H | H | $CH_3$ | $OCH_3$ | $OCH_3$ | CH * | |
| H | $CH_3$ | $CH_3$ | $OCH_3$ | $OCH_3$ | CH * | |
| H | Cl | H | $OCH_3$ | $OCH_3$ | CH * | |
| H | Cl | $CH_3$ | $OCH_3$ | $OCH_3$ | CH * | |
| H | Br | H | $OCH_3$ | $OCH_3$ | CH * | |
| H | Br | $CH_3$ | $OCH_3$ | $OCH_3$ | CH * | |

*Wherein W is O, unless indicated by * where W is S in all Tables.

Table 3c

General Structure 1

| Q | R | $R_3$ | $R_4$ | X | Y | Z | n' | m.p. °C |
|---|---|---|---|---|---|---|---|---|
| Q-33 | H | H | H | $CH_3$ | $OCH_3$ | CH | 0 | |
| Q-33 | H | H | H | $CH_3$ | $OCH_3$ | N | 0 | 173-176(d) |
| Q-33 | H | H | H | $OCH_3$ | $OCH_3$ | CH | 0 | 198-203(d) |
| Q-33 | H | H | H | $OCH_3$ | $OCH_3$ | N | 0 | 172-174 |
| Q-33 | H | H | H | $CH_3$ | $CH_3$ | CH | 0 | 130-133 |
| Q-33 | H | 3-$CH_3$ | H | $CH_3$ | $OCH_3$ | CH | 0 | |
| Q-33 | H | 4-$CH_3$ | H | $CH_3$ | $OCH_3$ | CH | 0 | |
| Q-33 | H | 5-$CH_3$ | H | $CH_3$ | $OCH_3$ | CH | 0 | |
| Q-33 | H | H | H | $CH_3$ | $CH_3$ | N | 0 | |
| Q-33 | H | 6-$CH_3$ | H | $CH_3$ | $OCH_3$ | CH | 0 | |
| Q-33 | H | 3-$CH_3$ | 5-$CH_3$ | $CH_3$ | $OCH_3$ | N | 0 | |
| Q-33 | H | 4-$CH_3$ | 6-$CH_3$ | $CH_3$ | $OCH_3$ | N | 0 | |
| Q-33 | $CH_3$ | H | H | $OCH_3$ | $OCH_3$ | CH | 0 | |
| Q-33 | H | H | H | $CH_3$ | $CH_2OCH_3$ | CH | 0 | |
| Q-33 | H | H | H | Cl | $OC_2H_5$ | CH | 0 | |
| Q-33 | H | H | H | $CH_3$ | $CF_3$ | CH | 0 | |
| Q-33 | H | H | H | Cl | $OCH_3$ | CH | 1 | 180 |
| Q-33 | H | H | H | $CH_3$ | $OCH_3$ | CH | 1 | 160-162 |
| Q-33 | H | H | H | $CH_3$ | $OCH_3$ | N | 1 | 223-226 |
| Q-33 | H | H | H | $OCH_3$ | $OCH_3$ | CH | 1 | 182-185 |
| Q-33 | H | H | H | $OCH_3$ | $OCH_3$ | N | 1 | 218-220 |
| Q-33 | H | H | H | $CH_3$ | $CH_3$ | CH | 1 | 159-161 |
| Q-33 | H | 3-$CH_3$ | H | $CH_3$ | $OCH_3$ | CH | 1 | |
| Q-33 | H | 4-$CH_3$ | H | $CH_3$ | $OCH_3$ | CH | 1 | |
| Q-33 | H | 5-$CH_3$ | H | $CH_3$ | $OCH_3$ | CH | 1 | |
| Q-33 | H | H | H | $CH_3$ | $CH_3$ | N | 1 | |
| Q-33 | H | 6-$CH_3$ | H | $CH_3$ | $OCH_3$ | CH | 1 | |
| Q-33 | H | 3-$CH_3$ | 5-$CH_3$ | $CH_3$ | $OCH_3$ | N | 1 | |
| Q-33 | H | 4-$CH_3$ | 6-$CH_3$ | $CH_3$ | $OCH_3$ | N | 1 | |
| Q-33 | $CH_3$ | H | H | $OCH_3$ | $OCH_3$ | CH | 1 | |
| Q-33 | H | H | H | $CH_3$ | $CH_2OCH_3$ | CH | 1 | |

Table 3c (continued)

| Q | R | $R_3$ | $R_4$ | X | Y | Z | n' | m.p. °C |
|---|---|---|---|---|---|---|---|---|
| Q-33 | H | H | H | Cl | $OC_2H_5$ | CH | 1 | |
| Q-33 | H | H | H | $CH_3$ | $CF_3$ | CH | 1 | |
| Q-34 | H | H | H | $CH_3$ | $OCH_3$ | CH | 0 | |
| Q-34 | H | H | H | $CH_3$ | $OCH_3$ | N | 0 | |
| Q-34 | H | H | H | $OCH_3$ | $OCH_3$ | CH | 0 | |
| Q-34 | H | H | H | $OCH_3$ | $OCH_3$ | N | 0 | |
| Q-34 | H | 2-$CH_3$ | H | $CH_3$ | $CH_3$ | CH | 0 | |
| Q-34 | H | 4-$CH_3$ | H | $CH_3$ | $CH_3$ | N | 0 | |
| Q-34 | H | 5-$CH_3$ | H | $CH_3$ | $OCH_3$ | CH | 0 | |
| Q-34 | H | 6-$CH_3$ | H | $CH_3$ | $OCH_3$ | N | 0 | |
| Q-34 | H | 2-$CH_3$ | 6-$CH_3$ | $OCH_3$ | $OCH_3$ | CH | 0 | |
| Q-34 | H | 4-$CH_3$ | 6-$CH_3$ | $OCH_3$ | $OCH_3$ | N | 0 | |
| Q-34 | H | H | H | $CH_3$ | $CH_3$ | N | 0 | |
| Q-34 | $CH_3$ | H | H | $CH_3$ | $CH_3$ | CH | 0 | |
| Q-34 | H | H | H | $CH_3$ | $CH_3$ | CH | 0 | |
| Q-34 | H | H | H | $CH_3$ | $OCF_2H$ | CH | 0 | |
| Q-34 | H | H | H | $CH_3$ | $OCH_3$ | CH | 1 | |
| Q-34 | H | H | H | $CH_3$ | $OCH_3$ | N | 1 | |
| Q-34 | H | H | H | $OCH_3$ | $OCH_3$ | CH | 1 | |
| Q-34 | H | H | H | $OCH_3$ | $OCH_3$ | N | 1 | |
| Q-34 | H | 2-$CH_3$ | H | $CH_3$ | $CH_3$ | CH | 1 | |
| Q-34 | H | 4-$CH_3$ | H | $CH_3$ | $CH_3$ | N | 1 | |
| Q-34 | H | 5-$CH_3$ | H | $CH_3$ | $OCH_3$ | CH | 1 | |
| Q-34 | H | 6-$CH_3$ | H | $CH_3$ | $OCH_3$ | N | 1 | |
| Q-34 | H | 2-$CH_3$ | 6-$CH_3$ | $OCH_3$ | $OCH_3$ | CH | 1 | |
| Q-34 | H | 4-$CH_3$ | 6-$CH_3$ | $OCH_3$ | $OCH_3$ | N | 1 | |
| Q-34 | H | H | H | $CH_3$ | $CH_3$ | N | 1 | |
| Q-34 | $CH_3$ | H | H | $CH_3$ | $CH_3$ | CH | 1 | |
| Q-34 | H | H | H | $CH_3$ | $CH_3$ | CH | 1 | |
| Q-34 | H | H | H | $CH_3$ | $OCF_2H$ | CH | 1 | |
| Q-35 | H | H | H | $CH_3$ | $OCH_3$ | CH | 0 | |

Table 3c (continued)

| Q | R | $R_3$ | $R_4$ | X | Y | Z | n' | m.p. °C |
|---|---|---|---|---|---|---|---|---|
| Q-35 | H | H | H | $CH_3$ | $OCH_3$ | N | 0 | |
| Q-35 | H | H | H | $OCH_3$ | $OCH_3$ | CH | 0 | |
| Q-35 | H | H | H | $OCH_3$ | $OCH_3$ | N | 0 | |
| Q-35 | H | 2-$CH_3$ | H | $CH_3$ | $CH_3$ | CH | 0 | |
| Q-35 | H | H | H | Cl | $OCH_3$ | CH | 0 | |
| Q-35 | H | 3-$CH_3$ | H | $CH_3$ | $CH_3$ | N | 0 | |
| Q-35 | H | 2-$CH_3$ | 6-$CH_3$ | $CH_3$ | $OCH_3$ | CH | 0 | |
| Q-35 | H | 2-$CH_3$ | 3-$CH_3$ | $CH_3$ | $OCH_3$ | N | 0 | |
| Q-35 | H | 3-$CH_3$ | 5-$CH_3$ | $CH_3$ | $OCH_3$ | N | 0 | |
| Q-35 | H | H | H | $CH_3$ | $CH_3$ | N | 0 | |
| Q-35 | $CH_3$ | H | H | $OCH_3$ | $OCH_3$ | CH | 0 | |
| Q-35 | H | H | H | $CH_3$ | $OCH_2CF_3$ | CH | 0 | |
| Q-35 | H | H | H | $CH_3$ | $CH_3$ | CH | 0 | |
| Q-35 | H | H | H | $CH_3$ | $OCH_3$ | CH | 1 | |
| Q-35 | H | H | H | $CH_3$ | $OCH_3$ | N | 1 | |
| Q-35 | H | H | H | $OCH_3$ | $OCH_3$ | CH | 1 | |
| Q-35 | H | H | H | $OCH_3$ | $OCH_3$ | N | 1 | |
| Q-35 | H | 2-$CH_3$ | H | $CH_3$ | $CH_3$ | CH | 1 | |
| Q-35 | H | H | H | Cl | $OCH_3$ | CH | 1 | |
| Q-35 | H | 3-$CH_3$ | H | $CH_3$ | $CH_3$ | N | 1 | |
| Q-35 | H | 2-$CH_3$ | 6-$CH_3$ | $CH_3$ | $OCH_3$ | CH | 1 | |
| Q-35 | H | 2-$CH_3$ | 3-$CH_3$ | $CH_3$ | $OCH_3$ | N | 1 | |
| Q-35 | H | 3-$CH_3$ | 5-$CH_3$ | $CH_3$ | $OCH_3$ | N | 1 | |
| Q-35 | H | H | H | $CH_3$ | $CH_3$ | N | 1 | |
| Q-35 | $CH_3$ | H | H | $OCH_3$ | $OCH_3$ | CH | 1 | |
| Q-35 | H | H | H | $CH_3$ | $OCH_2CF_3$ | CH | 1 | |
| Q-35 | H | H | H | $CH_3$ | $CH_3$ | CH | 1 | |

**Table 4a**

**General Structure 2**

| Q | R | $R_3$ | $R_4$ | $R_5$ | X | Y | Z | W' | m.p. °C |
|---|---|---|---|---|---|---|---|---|---|
| Q-2 | H | H | H | H | $CH_3$ | $OCH_3$ | CH | O | |
| Q-2 | H | H | H | H | $CH_3$ | $OCH_3$ | N | O | |
| Q-2 | H | H | H | H | $OCH_3$ | $OCH_3$ | CH | O | |
| Q-2 | H | H | H | H | $OCH_3$ | $OCH_3$ | N | O | |
| Q-2 | H | $CH_3$ | H | H | $CH_3$ | $CH_3$ | CH | O | |
| Q-2 | H | H | $CH_3$ | H | $CH_3$ | $OCH_3$ | N | O | |
| Q-2 | H | H | H | $CH_3$ | $CH_3$ | $OCH_3$ | CH | O | |
| Q-2 | $CH_3$ | H | H | H | CH | $CH_3$ | CH | O | |
| Q-2 | H | $CH_3$ | $CH_3$ | H | $OCH_3$ | $OCH_3$ | N | O | |
| Q-2 | H | H | H | H | Cl | $OCH_3$ | CH | S | |
| Q-2 | H | H | H | H | $CH_3$ | $OCH_3$ | CH | S | |
| Q-2 | H | H | H | H | $CH_3$ | $OCH_3$ | N | S | |
| Q-2 | H | H | H | H | $OCH_3$ | $OCH_3$ | CH | S | |
| Q-2 | H | H | H | H | $OCH_3$ | $OCH_3$ | N | S | |
| Q-2 | H | H | H | H | $CH_3$ | $CH_3$ | CH | S | |
| Q-2 | H | $CH_3$ | H | H | $CH_3$ | $CH_3$ | CH | S | |
| Q-2 | H | H | $CH_3$ | H | $CH_3$ | $CH_3$ | N | S | |
| Q-2 | H | H | H | $CH_3$ | $CH_3$ | $OCH_3$ | CH | S | |
| Q-2 | H | $CH_3$ | $CH_3$ | H | $CH_3$ | $OCH_3$ | N | S | |
| Q-2 | H | H | $CH_3$ | $CH_3$ | $OCH_3$ | $OCH_3$ | CH | S | |
| Q-2 | H | $CH_3$ | H | $CH_3$ | $OCH_3$ | $OCH_3$ | N | S | |
| Q-2 | H | $CH_3$ | $CH_3$ | $CH_3$ | $CH_3$ | $OCH_3$ | CH | S | |
| Q-2 | $CH_3$ | H | H | H | $CH_3$ | $CH_3$ | CH | S | |
| Q-2 | H | H | H | H | Cl | $OC_2H_5$ | CH | S | |
| Q-3 | H | H | H | H | $CH_3$ | $CH_3$ | CH | O | |
| Q-3 | H | H | H | H | $CH_3$ | $CH_3$ | N | O | |
| Q-3 | H | H | H | H | $CH_3$ | $OCH_3$ | CH | O | |
| Q-3 | H | H | H | H | $CH_3$ | $OCH_3$ | N | O | |
| Q-3 | H | H | H | H | $OCH_3$ | $OCH_3$ | CH | O | |
| Q-3 | H | H | H | H | $OCH_3$ | $OCH_3$ | N | O | |
| Q-3 | H | $CH_3$ | H | H | $CH_3$ | $CH_3$ | CH | O | |

Table 4a (continued)

| Q | R | $R_3$ | $R_4$ | $R_5$ | X | Y | Z | W' | m.p. °C |
|---|---|---|---|---|---|---|---|---|---|
| Q-3 | H | H | $CH_3$ | H | $CH_3$ | $CH_3$ | N | O | |
| Q-3 | H | H | H | $CH_3$ | $CH_3$ | $OCH_3$ | CH | O | |
| Q-3 | H | $CH_3$ | $CH_3$ | H | $CH_3$ | $OCH_3$ | N | O | |
| Q-3 | H | H | $CH_3$ | $CH_3$ | $OCH_3$ | $OCH_3$ | CH | O | |
| Q-3 | H | $CH_3$ | H | $CH_3$ | $OCH_3$ | $OCH_3$ | N | O | |
| Q-3 | H | H | H | H | $OCH_3$ | $OCH_2CF_3$ | CH | O | |
| Q-3 | H | H | H | H | $OCH_3$ | $OCH_2CF_3$ | CH | O | |
| Q-3 | $CH_3$ | H | H | H | Cl | $OCH_3$ | CH | O | |
| Q-3 | H | H | H | H | $CH_3$ | $OCH_3$ | CH | S | |
| Q-3 | H | H | H | H | $OCH_3$ | $OCH_3$ | CH | S | 133-135 |
| Q-3 | H | H | H | H | $OCH_3$ | $OCH_3$ | N | S | |
| Q-3 | H | $CH_3$ | H | H | $CH_3$ | $CH_3$ | CH | S | |
| Q-3 | H | H | $CH_3$ | H | $CH_3$ | $OCH_3$ | N | S | |
| Q-3 | H | H | H | $CH_3$ | $CH_3$ | $OCH_3$ | CH | S | |
| Q-3 | $CH_3$ | H | H | H | $CH_3$ | $CH_3$ | CH | S | |
| Q-3 | H | H | $CH_3$ | $CH_3$ | $OCH_3$ | $OCH_3$ | N | S | |
| Q-3 | H | H | H | H | $OCH_3$ | $CF_3$ | N | S | |
| Q-4 | H | H | H | H | $CH_3$ | $OCH_3$ | CH | - | |
| Q-4 | H | H | H | H | $OCH_3$ | Cl | CH | - | |
| Q-4 | H | H | H | H | $CH_3$ | $OCH_3$ | N | - | 192-199 |
| Q-4 | H | H | H | H | $CH_3$ | $CH_3$ | CH | - | |
| Q-4 | H | H | H | H | $OCH_3$ | $OCH_3$ | CH | - | 140-144 |
| Q-4 | H | H | H | H | $OCH_3$ | $OCH_3$ | N | - | |
| Q-4 | H | $CH_3$ | H | H | $CH_3$ | $CH_3$ | CH | - | |
| Q-4 | H | H | $CH_3$ | H | $CH_3$ | $OCH_3$ | N | - | |
| Q-4 | H | H | H | $CH_3$ | $CH_3$ | $OCH_3$ | CH | - | |
| Q-4 | $CH_3$ | H | H | H | $OCH_3$ | $OCH_3$ | CH | - | |
| Q-4 | $CH_3$ | $CH_3$ | $CH_3$ | $CH_3$ | $CH_3$ | $OCH_3$ | N | - | |
| Q-4 | H | $CH_3$ | H | $CH_3$ | $CH_3$ | $OCH_3$ | CH | - | |
| Q-4 | H | H | H | H | $OCH_3$ | $OCH_2CF_3$ | N | - | |
| Q-4 | H | H | H | H | $CH_3$ | $NHCH_3$ | CH | - | |
| Q-4 | H | H | H | H | $CH_3$ | $CH_3$ | CH | - | |
| Q-4 | H | H | H | H | $CH_3$ | $CH_3$ | N | - | |

**Table 4a (continued)**

| Q | R | $R_3$ | $R_4$ | $R_5$ | X | Y | Z | W' | m.p. °C |
|---|---|---|---|---|---|---|---|---|---|
| Q-9 | H | H | H | H | $CH_3$ | $OCH_3$ | CH | - | |
| Q-9 | H | H | H | H | $CH_3$ | $OCH_3$ | N | - | |
| Q-9 | H | H | H | H | $OCH_3$ | $OCH_3$ | CH | - | |
| Q-9 | H | H | H | H | $OCH_3$ | $OCH_3$ | N | - | |
| Q-9 | $CH_3$ | $CH_3$ | H | H | $CH_3$ | $CH_3$ | CH | - | |
| Q-9 | H | H | $CH_3$ | H | $CH_3$ | $OCH_3$ | N | - | |
| Q-9 | H | H | H | $CH_3$ | $OCH_3$ | $OCH_3$ | CH | - | |
| Q-9 | H | H | $CH_3$ | $CH_3$ | $CH_3$ | $OCH_3$ | CH | - | |
| Q-9 | H | H | H | H | Cl | $OCH_2CH_3$ | CH | - | |
| Q-9 | H | H | H | H | $OCH_3$ | $CH(OCH_3)_2$ | CH | - | |
| Q-9 | H | H | H | H | $NH_2$ | $CH_3$ | CH | - | |
| Q-9 | H | H | H | H | $NHCH(CH_3)_2$ | $CH_3$ | CH | - | |
| Q-9 | H | H | H | H | $CH_2O$-n-$C_4H_9$ | $CH_3$ | CH | - | |
| Q-9 | H | H | H | H | S-n-$C_4H_9$ | $CH_3$ | CH | - | |
| Q-9 | H | H | H | H | S-n-$CH_2CH_2F$ | $CH_3$ | CH | - | |

**Table 4b**

**General Structure 2**

| Q | R | $R_3$' | $R_4$ | X | Y | Z | W' | m.p. °C |
|---|---|---|---|---|---|---|---|---|
| Q-10 | H | H | H | $CH_3$ | $OCH_3$ | CH | O | |
| Q-10 | H | H | H | $CH_3$ | $CH_3$ | CH | O | |
| Q-10 | H | H | H | $OCH_3$ | $OCH_3$ | N | O | |
| Q-10 | H | H | H | $OCH_3$ | Cl | CH | O | |
| Q-10 | H | H | H | $OCH_3$ | $OCH_3$ | CH | O | 157-161 |
| Q-10 | H | H | H | $OCH_3$ | $OCH_3$ | N | O | |
| Q-10 | H | H | H | $CH_3$ | $OCH_3$ | CH | S | |
| Q-10 | H | H | H | $CH_3$ | $OCH_3$ | N | S | |
| Q-10 | H | H | H | $OCH_3$ | $OCH_3$ | CH | S | |
| Q-10 | H | H | H | $OCH_3$ | $OCH_3$ | N | S | |
| Q-10 | H | H | H | $OCH_3$ | Cl | CH | $NCH_3$ | 193-195 |
| Q-10 | H | H | H | $CH_3$ | $CH_3$ | CH | $NCH_3$ | 112-115 |
| Q-10 | H | H | H | $CH_3$ | $OCH_3$ | N | $NCH_3$ | 190-192 |
| Q-10 | H | H | H | $CH_3$ | $CH_3$ | N | $NCH_3$ | 185-189 |
| Q-10 | H | H | H | $OCH_3$ | $OCH_3$ | CH | $NCH_3$ | 202-204 |
| Q-10 | H | H | H | $OCH_3$ | $CH_3$ | CH | $NCH_3$ | 171-174 |
| Q-10 | H | $CH_3$ | H | $CH_3$ | $CH_3$ | CH | O | |
| Q-10 | H | H | $CH_3$ | $CH_3$ | $OCH_3$ | N | O | |
| Q-10 | H | $CH_3$ | $CH_3$ | $CH_3$ | $OCH_3$ | CH | O | |
| Q-10 | $CH_3$ | H | H | $OCH_3$ | $OCH_3$ | N | O | |
| Q-10 | H | H | H | $CH_3$ | 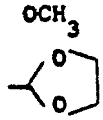 | CH | O | |
| Q-10 | H | $CH_3$ | H | $CH_3$ | $CH_3$ | CH | S | |
| Q-10 | H | H | $CH_3$ | $CH_3$ | $OCH_3$ | N | S | |
| Q-10 | H | $CH_3$ | $CH_3$ | $CH_3$ | $OCH_3$ | CH | S | |
| Q-10 | $CH_3$ | $CH_3$ | $CH_3$ | $CH_3$ | $OCH_3$ | CH | S | |
| Q-10 | $CH_3$ | H | H | $OCH_3$ | $OCH_3$ | N | S | |
| Q-10 | H | H | H | $CH_3$ | $OC_2H_5$ | CH | S | |
| Q-10 | H | H | $CH_3$ | $CH_3$ | $CH_3$ | N | $NCH_3$ | |
| Q-10 | H | H | $CH_3$ | $CH_3$ | $OCH_3$ | CH | $NCH_3$ | |
| Q-10 | H | $CH_3$ | $CH_3$ | $OCH_3$ | $OCH_3$ | N | $NCH_3$ | |
| Q-10 | $CH_3$ | H | H | $OCH_3$ | $OCH_3$ | N | $NCH_3$ | |
| Q-11 | H | H | H | $CH_3$ | $CH_3$ | N | O | |

Table 4b (continued)

| Q | R | $R_3'$ | $R_4$ | X | Y | Z | W" | m.p. °C |
|---|---|---|---|---|---|---|---|---|
| Q-11 | H | H | H | $CH_3$ | $CH_3$ | CH | O | |
| Q-11 | H | H | H | $CH_3$ | $OCH_3$ | N | O | |
| Q-11 | H | H | H | $CH_3$ | $OCH_3$ | CH | O | |
| Q-11 | H | H | H | $OCH_3$ | $OCH_3$ | CH | O | |
| Q-11 | H | H | H | $OCH_3$ | $OCH_3$ | N | O | |
| Q-11 | H | H | H | $CH_3$ | $CH_3$ | CH | S | |
| Q-11 | H | H | H | $CH_3$ | $OCH_3$ | CH | S | |
| Q-11 | H | H | H | $CH_3$ | $OCH_3$ | CH | S | |
| Q-11 | H | H | H | $OCH_3$ | $OCH_3$ | CH | S | |
| Q-11 | H | H | H | $CH_3$ | $CH_3$ | CH | $NCH_3$ | |
| Q-11 | H | H | H | $CH_3$ | $OCH_3$ | CH | $NCH_3$ | |
| Q-11 | H | H | H | $OCH_3$ | $OCH_3$ | CH | $NCH_3$ | |
| Q-11 | H | H | H | $CH_3$ | $OCH_3$ | N | $NCH_3$ | |
| Q-11 | H | $CH_3$ | H | $CH_3$ | $OCH_3$ | N | O | |
| Q-11 | H | H | $CH_3$ | $OCH_3$ | $OCH_3$ | N | O | |
| Q-11 | H | $CH_3$ | $CH_3$ | $CH_3$ | $OCH_3$ | CH | O | |
| Q-11 | $CH_3$ | H | H | $CH_3$ | $OCH_3$ | CH | O | |
| Q-11 | H | H | H | $CH_3$ | $C_2H_5$ | N | O | |
| Q-11 | H | H | H | $CH_3$ | $CH_2OCH_3$ | CH | O | |
| Q-11 | H | $CH_3$ | H | $CH_3$ | $OCH_3$ | N | S | |
| Q-11 | H | H | $CH_3$ | $OCH_3$ | $OCH_3$ | N | S | |
| Q-11 | H | $CH_3$ | $CH_3$ | $CH_3$ | $OCH_3$ | CH | S | |
| Q-11 | $CH_3$ | H | H | $CH_3$ | $OCH_3$ | CH | S | |
| Q-11 | H | H | H | $CH_3$ | $C_2H_5$ | N | S | |
| Q-11 | $CH_3$ | $CH_3$ | $CH_3$ | $CH_3$ | $OCH_3$ | N | S | |
| Q-11 | H | H | H | Cl | $OCH_3$ | CH | S | |
| Q-11 | H | $CH_3$ | H | $CH_3$ | $CH_3$ | CH | $NCH_3$ | |
| Q-11 | H | H | $CH_3$ | $CH_3$ | $OCH_3$ | N | $NCH_3$ | |
| Q-11 | H | $CH_3$ | $CH_3$ | $CH_3$ | $OCH_3$ | CH | $NCH_3$ | |
| Q-11 | $CH_3$ | H | H | $OCH_3$ | $OCH_3$ | N | NH | |
| Q-11 | H | H | H | $CH_3$ | $OCH_3$ | CH | NH | |
| Q-11 | H | H | H | $OCH_3$ | $OCH_3$ | CH | O | * |
| Q-11 | H | H | H | $OCH_3$ | CH | N | O | * |

Table 4b (continued)

| Q | R | $R_3$ | $R_4$ | X | Y | Z | W" | m.p. °C |
|---|---|---|---|---|---|---|---|---|
| Q-11 | H | H | H | $CH_3$ | $OCH_3$ | N | $NCH_3$ | |
| Q-11 | H | H | H | $CH_3$ | $OCF_2H$ | CH | $NCH_3$ | |
| Q-11 | H | $CH_3$ | H | $OCH_3$ | $OCH_2CF_3$ | N | $NCH_3$ | |
| Q-12 | H | H | H | $CH_3$ | $OCH_3$ | CH | O | |
| Q-12 | H | H | H | $CH_3$ | $OCH_3$ | N | O | |
| Q-12 | H | H | H | $OCH_3$ | $OCH_3$ | CH | O | |
| Q-12 | H | H | H | $OCH_3$ | $OCH_3$ | N | O | |
| Q-12 | H | H | H | $CH_3$ | $OCH_3$ | CH | S | |
| Q-12 | H | H | H | $CH_3$ | $OCH_3$ | N | S | |
| Q-12 | H | H | H | $OCH_3$ | $OCH_3$ | CH | S | |
| Q-12 | H | H | H | $OCH_3$ | $OCH_3$ | N | S | |
| Q-12 | H | H | H | $CH_3$ | $CH_3$ | N | $NCH_3$ | |
| Q-12 | H | H | H | $CH_3$ | $OCH_3$ | CH | $NCH_3$ | |
| Q-12 | H | H | H | $CH_3$ | $OCH_3$ | N | $NCH_3$ | |
| Q-12 | H | H | H | $OCH_3$ | $OCH_3$ | CH | $NCH_3$ | |
| Q-12 | H | H | H | $OCH_3$ | $OCH_3$ | N | $NCH_3$ | |
| Q-12 | H | H | H | $OCH_3$ | $OCH_3$ | N | NH | |
| Q-12 | H | $CH_3$ | H | $CH_3$ | $CH_3$ | CH | O | |
| Q-12 | H | H | $CH_3$ | $CH_3$ | $OCH_3$ | N | O | |
| Q-12 | H | $CH_3$ | $CH_3$ | $CH_3$ | $OCH_3$ | CH | O | |
| Q-12 | $CH_3$ | H | H | $OCH_3$ | $OCH_3$ | N | O | |
| Q-12 | $CH_3$ | H | H | $CH_3$ | $CF_3$ | CH | O | |
| Q-12 | H | H | H | $OCH_3$ | $OCH_2CF_3$ | N | | |
| Q-12 | H | $CH_3$ | H | $CH_3$ | $OCH_3$ | CH | S | |
| Q-12 | H | H | $CH_3$ | $CH_3$ | $OCH_3$ | N | S | |
| Q-12 | H | $CH_3$ | $CH_3$ | $OCH_3$ | $OCH_3$ | CH | S | |
| Q-12 | $CH_3$ | H | H | $OCH_3$ | $OCH_3$ | N | S | |
| Q-12 | $CH_3$ | $CH_3$ | H | $CH_3$ | $NHCH_3$ | CH | S | |
| Q-12 | H | H | H | Br | $OC_2H_5$ | CH | S | |
| Q-12 | H | $CH_3$ | H | $CH_3$ | $CH_3$ | CH | NH | |
| Q-12 | H | $CH_3$ | H | $CH_3$ | $CH_3$ | CH | $NCH_3$ | |
| Q-12 | H | H | $CH_3$ | $CH_3$ | $OCH_3$ | N | $NCH_3$ | |
| Q-12 | H | $CH_3$ | $CH_3$ | $CH_3$ | $CH_3$ | $CH_3$ | $NCH_3$ | |
| Q-12 | $CH_3$ | $CH_3$ | $CH_3$ | $CH_3$ | $OCH_3$ | N | $NCH_3$ | |

Table 4b (continued)

| Q | R | $R_3$' | $R_4$ | X | Y | Z | W" | m.p. °C |
|---|---|---|---|---|---|---|---|---|
| Q-12 | $CH_3$ | $CH_3$ | $CH_3$ | $CH_3$ | $OCH_3$ | CH | $NCH_3$ | |
| Q-12 | H | H | H | Br | $OCH_3$ | CH | $NCH_3$ | |
| Q-12 | H | H | H | $OCH_3$ | $N(CH_3)_2$ | CH | $NCH_3$ | |
| Q-13 | H | H | H | $CH_3$ | $OCH_3$ | CH | O | |
| Q-13 | H | H | H | $CH_3$ | $OCH_3$ | CH | O | |
| Q-13 | H | H | H | $CH_3$ | $CH_3$ | CH | O | |
| Q-13 | H | H | H | $CH_3$ | $OCH_3$ | N | O | |
| Q-13 | H | H | H | $OCH_3$ | $OCH_3$ | CH | O | |
| Q-13 | H | H | H | $CH_3$ | $CH_3$ | N | O | |
| Q-13 | H | H | H | $OCH_3$ | $OCH_3$ | N | O | |
| Q-13 | H | H | H | $CH_3$ | $OCH_3$ | CH | S | |
| Q-13 | H | H | H | $CH_3$ | $OCH_3$ | N | S | |
| Q-13 | H | H | H | $OCH_3$ | $OCH_3$ | CH | S | |
| Q-13 | H | H | H | $OCH_3$ | $OCH_3$ | N | S | |
| Q-13 | H | H | H | $CH_3$ | $OCH_3$ | CH | NH | |
| Q-13 | H | H | H | $CH_3$ | $OCH_3$ | N | NH | |
| Q-13 | H | H | H | $OCH_3$ | $OCH_3$ | CH | $NCH_3$ | |
| Q-13 | H | H | H | $OCH_3$ | $OCH_3$ | N | NH | |
| Q-13 | H | $CH_3$ | H | $CH_3$ | $CH_3$ | CH | O | |
| Q-13 | H | H | $CH_3$ | $CH_3$ | $CH_3$ | N | O | |
| Q-13 | H | $CH_3$ | $CH_3$ | $CH_3$ | $OCH_3$ | CH | O | |
| Q-13 | $CH_3$ | H | H | $CH_3$ | $OCH_3$ | N | O | |
| Q-13 | H | H | H | $CH_3$ | $CH(OCH_3)_2$ | CH | O | |
| Q-13 | H | $CH_3$ | H | $CH_3$ | $OCH_3$ | CH | S | |
| Q-13 | H | H | $CH_3$ | $CH_3$ | $OCH_3$ | CH | S | |
| Q-13 | H | $CH_3$ | $CH_3$ | $CH_3$ | $OCH_3$ | N | S | |
| Q-13 | $CH_3$ | H | $CH_3$ | $OCH_3$ | $OCH_3$ | N | S | |
| Q-13 | $CH_3$ | $CH_3$ | $CH_3$ | $CH_3$ | $CH_3$ | N | S | |
| Q-13 | H | H | H | $OCH_3$ | 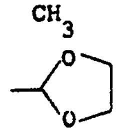 | CH | S | |
| Q-13 | H | $CH_3$ | H | $CH_3$ | $OCH_3$ | N | $NCH_3$ | |
| Q-13 | H | H | $CH_3$ | $CH_3$ | $OCH_3$ | N | $NCH_3$ | |
| Q-13 | H | $CH_3$ | $CH_3$ | $CH_3$ | $OCH_3$ | CH | $NCH_3$ | |

Table 4b (continued)

| Q | R | $R_3$' | $R_4$ | X | Y | Z | W" | m.p. °C |
|---|---|---|---|---|---|---|---|---|
| Q-13 | $CH_3$ | H | H | $OCH_3$ | $OCH_3$ | CH | $NCH_3$ | |
| Q-13 | H | H | H | $CH_3$ | $OCH_3$ | N | NH | |
| Q-13 | H | H | H | Br | $OC_2H_5$ | CH | NH | |
| Q-13 | H | H | H | $OCH_3$ | $C_2H_5$ | N | NH | |
| Q-13 | H | H | H | $CH_3$ | $CH_3$ | CH | $NCH_3$ | |
| Q-13 | H | H | H | $CH_3$ | $CH_3$ | N | $NCH_3$ | |
| Q-13 | H | H | H | Cl | $OCH_3$ | CH | $NCH_3$ | |
| Q-14 | H | H | H | $CH_3$ | $CH_3$ | N | O | |
| Q-14 | H | H | H | $CH_3$ | $CH_3$ | CH | O | |
| Q-14 | H | H | H | $CH_3$ | $OCH_3$ | CH | O | |
| Q-14 | H | H | H | $CH_3$ | $OCH_3$ | CH | O | |
| Q-14 | H | H | H | $CH_3$ | $OCH_3$ | N | O | |
| Q-14 | H | H | H | $OCH_3$ | $OCH_3$ | CH | O | |
| Q-14 | H | H | H | $CH_3$ | $OCH_3$ | CH | S | |
| Q-14 | H | H | H | $CH_3$ | $OCH_3$ | N | S | |
| Q-14 | H | H | H | $OCH_3$ | $OCH_3$ | CH | S | |
| Q-14 | H | H | H | $OCH_3$ | $OCH_3$ | CH | S | |
| Q-14 | H | H | H | $OCH_3$ | $OCH_3$ | N | S | |
| Q-14 | H | H | H | $CH_3$ | $OCH_3$ | CH | $NCH_3$ | |
| Q-14 | H | H | H | $CH_3$ | $OCH_3$ | N | $NCH_3$ | |
| Q-14 | H | H | H | $OCH_3$ | $OCH_3$ | CH | NH | |
| Q-14 | H | H | H | $OCH_3$ | $OCH_3$ | N | $NCH_3$ | |
| Q-14 | H | $CH_3$ | H | $CH_3$ | $CH_3$ | CH | O | |
| Q-14 | H | H | $CH_3$ | $CH_3$ | $CH_3$ | N | O | |
| Q-14 | H | $CH_3$ | $CH_3$ | $CH_3$ | $OCH_3$ | CH | O | |
| Q-14 | $CH_3$ | $CH_3$ | $CH_3$ | $CH_3$ | $OCH_3$ | N | O | |
| Q-14 | H | H | H | $CH_3$ | $C_2H_5$ | N | O | |
| Q-14 | H | H | H | $CH_3$ | $OC_2H_5$ | N | O | |
| Q-14 | H | $CH_3$ | H | $CH_3$ | $OCH_3$ | CH | S | |
| Q-14 | H | H | $CH_3$ | $CH_3$ | $OCH_3$ | N | S | |
| Q-14 | H | $CH_3$ | $CH_3$ | $OCH_3$ | $OCH_3$ | CH | S | |
| Q-14 | $CH_3$ | $CH_3$ | $CH_3$ | $OCH_3$ | $OCH_3$ | CH | S | |
| Q-14 | H | H | H | $OCH_3$ | $C_2H_5$ | N | S | |
| Q-14 | H | H | H | $OCH_3$ | $OC_2H_5$ | N | S | |

Table 4b (continued)

| Q | R | $R_3$' | $R_4$ | X | Y | Z | W" | m.p. °C |
|---|---|---|---|---|---|---|---|---|
| Q-14 | H | $CH_3$ | H | $CH_3$ | $OCH_3$ | CH | $NCH_3$ | |
| Q-14 | H | H | $CH_3$ | $CH_3$ | $OCH_3$ | N | $NCH_3$ | |
| Q-14 | H | $CH_3$ | $CH_3$ | $OCH_3$ | $OCH_3$ | CH | $NCH_3$ | |
| Q-14 | $CH_3$ | $CH_3$ | H | $OCH_3$ | $OCH_3$ | N | $NCH_3$ | |
| Q-14 | H | $CH_3$ | $CH_3$ | $CH_3$ | $OCH_3$ | CH | NH | |
| Q-14 | H | H | H | $CH_3$ | $OCH_3$ | N | NH | |
| Q-14 | H | H | H | $CH_3$ | $CH_2OCH_3$ | CH | $NCH_3$ | |
| Q-15 | H | H | H | $CH_3$ | $OCH_3$ | CH | O | |
| Q-15 | H | H | H | $CH_3$ | $OCH_3$ | N | O | |
| Q-15 | H | H | H | $OCH_3$ | $OCH_3$ | CH | O | |
| Q-15 | H | H | H | $OCH_3$ | $OCH_3$ | N | O | |
| Q-15 | H | $C_2H_5$ | H | $OCH_3$ | $OCH_3$ | CH | O | |
| Q-15 | H | $CH_3$ | H | $CH_3$ | $OCH_3$ | CH | O | |
| Q-15 | H | $CH_3$ | H | $CH_3$ | $CH_3$ | CH | O | |
| Q-15 | H | $CH_3$ | H | $OCH_3$ | $OCH_3$ | CH | O | |
| Q-15 | H | H | $CH_3$ | $CH_3$ | $CH_3$ | N | O | |
| Q-15 | H | $CH_3$ | H | $CH_3$ | $OCH_3$ | N | O | |
| Q-15 | H | $CH_3$ | $CH_3$ | $CH_3$ | $OCH_3$ | CH | O | |
| Q-15 | H | $CH_3$ | H | $OCH_3$ | $OCH_3$ | N | O | |
| Q-15 | $CH_3$ | H | H | $CH_3$ | $OCH_3$ | N | O | |
| Q-15 | H | $CH_3$ | H | $OCH_3$ | Cl | CH | O | |
| Q-15 | $CH_3$ | $CH_3$ | $CH_3$ | $OCH_3$ | $OCH_3$ | CH | O | |
| Q-15 | H | H | H | $CH_3$ | $CF_3$ | CH | O | |
| Q-15 | H | H | H | $CH_3$ | $CF_3$ | N | O | |
| Q-15 | H | H | H | $OCH_3$ | $OCF_2H$ | CH | O | |
| Q-15 | $CH_3$ | H | H | $OCH_3$ | $OCF_2H$ | CH | O | |
| Q-15 | H | H | H | $CH_3$ | $CH_3$ | CH | O | |
| Q-15 | H | H | H | $CH_3$ | $CH_3$ | N | O | |
| Q-15 | H | H | H | $OCH_3$ | $OCH_3$ | CH | O * | |
| Q-15 | H | H | H | $OCH_3$ | $CH_3$ | N | O * | |
| Q-15 | H | H | H | $CH_3$ | $CH_3$ | N | S | |
| Q-15 | H | $CH_3$ | H | $CH_3$ | $OCH_3$ | CH | S | |
| Q-15 | H | $CH_3$ | H | $CH_3$ | $OCH_3$ | N | S | |

Table 4b (continued)

| Q | R | $R_3$, | $R_4$ | X | Y | Z | W" | m.p. °C |
|---|---|---|---|---|---|---|---|---|
| Q-15 | H | $CH_3$ | H | $OCH_3$ | $OCH_3$ | CH | S | |
| Q-15 | H | $CH_3$ | H | $OCH_3$ | $OCH_3$ | N | S | |
| Q-15 | H | $CH_3$ | H | Cl | $OCH_3$ | CH | S | |
| Q-15 | H | H | H | $CH_3$ | $OCH_3$ | CH | $NCH_3$ | |
| Q-15 | H | H | H | $CH_3$ | $OCH_3$ | N | $NCH_3$ | |
| Q-15 | H | H | H | $OCH_3$ | $OCH_3$ | CH | $NCH_3$ | |
| Q-15 | H | H | H | $OCH_3$ | $OCH_3$ | N | $NCH_3$ | |
| Q-15 | H | $CH_3$ | H | $CH_3$ | $CH_3$ | CH | S | |
| Q-15 | H | H | $CH_3$ | $CH_3$ | $CH_3$ | N | S | |
| Q-15 | H | $CH_3$ | $CH_3$ | $CH_3$ | $OCH_3$ | CH | S | |
| Q-15 | $CH_3$ | H | H | $CH_3$ | $OCH_3$ | N | S | |
| Q-15 | $CH_3$ | H | $CH_3$ | $OCH_3$ | $OCH_3$ | CH | S | |
| Q-15 | H | H | H | Cl | $OC_2H_5$ | CH | S | |
| Q-15 | H | H | H | $OCH_3$ | $OCH_2CF_3$ | CH | S | |
| Q-15 | H | $CH_3$ | H | $CH_3$ | $CH_3$ | CH | $NCH_3$ | |
| Q-15 | H | H | $CH_3$ | $CH_3$ | $OCH_3$ | CH | $NCH_3$ | |
| Q-15 | H | $CH_3$ | $CH_3$ | $CH_3$ | $OCH_3$ | N | $NCH_3$ | |
| Q-15 | $CH_3$ | $CH_3$ | $CH_3$ | $CH_3$ | $OCH_3$ | N | $NCH_3$ | |
| Q-15 | $CH_3$ | H | H | $CH_3$ | $OCH_3$ | CH | NH | |
| Q-15 | H | H | H | $OCH_3$ | $OCH_3$ | N | NH | |
| Q-15 | H | H | H | $CH_3$ | $NHCH_3$ | CH | $NCH_3$ | |
| Q-15 | H | H | H | $OCH_3$ | $N(CH_3)_2$ | CH | $NCH_3$ | |
| Q-15 | H | H | H | $CH_3$ | $OCH_2C{\equiv}CH$ | N | S | |
| Q-15 | H | H | H | $CH_3$ | $CH_2C{\equiv}CH_3$ | CH | S | |
| Q-15 | H | H | H | $CH_3$ | $C{\equiv}CH$ | N | S | |
| Q-15 | H | $C_2H_5$ | H | $OCH_3$ | $OCH_3$ | CH | S | |
| Q-15 | H | H | H | $OCH_3$ | $(CH_2)_3CH_2F$ | CH | S | |
| Q-15 | H | H | H | $OCH_3$ | 2-methyl-1,3-dioxolan-2-yl | N | S | |
| Q-15 | H | H | H | $OCH_3$ | 2-methyl-1,3-dithiolan-2-yl | CH | S | |
| Q-15 | H | H | H | $OCH_3$ | 2-methyl-1,3-dithian-2-yl | N | S | |
| Q-15 | H | H | H | $OCH_3$ | $OCH_3$ | CH | S * | |
| Q-15 | H | H | H | $OCH_3$ | $CH_3$ | N | S * | |

Table 4c

General Structure 2

| Q | R | $R_3$ | $R_4$ | $R_5$ | X | Y | Z | m.p. °C |
|---|---|---|---|---|---|---|---|---|
| Q-18 | H | H | H | H | $CH_3$ | $CH_3$ | CH | 198-200 |
| Q-18 | H | H | H | H | $CH_3$ | $OCH_3$ | CH | |
| Q-18 | H | H | H | H | $CH_3$ | $OCH_3$ | N | 177-179 |
| Q-18 | $CH_3$ | H | H | H | $CH_3$ | $OCH_3$ | N | |
| Q-18 | H | H | H | H | $OCH_3$ | $OCH_3$ | CH | 178-182 |
| Q-18 | H | H | H | H | $CH_3$ | $CH_3$ | CH | |
| Q-18 | H | H | H | H | $OCH_3$ | $OCH_3$ | N | 185-188 |
| Q-18 | H | H | H | H | Cl | $OCH_3$ | CH | |
| Q-18 | H | $CH_3$ | H | H | $CH_3$ | $OCH_3$ | CH | |
| Q-18 | H | H | $CH_3$ | H | $CH_3$ | $OCH_3$ | N | |
| Q-18 | H | H | H | $CH_3$ | $CH_3$ | $CH_3$ | CH | |
| Q-18 | H | $CH_3$ | $CH_3$ | H | $CH_3$ | $CH_3$ | N | |
| Q-18 | H | H | $CH_3$ | $CH_3$ | $OCH_3$ | $OCH_3$ | CH | |
| Q-18 | H | $CH_3$ | H | $CH_3$ | $OCH_3$ | $OCH_3$ | N | |
| Q-18 | H | $CH_3$ | $CH_3$ | $CH_3$ | $CH_3$ | $OCH_3$ | CH | |
| Q-18 | H | H | H | H | $OCH_3$ | $CH(OCH_3)_2$ | CH | |
| Q-19 | $CH_3$ | H | H | H | Cl | $OCH_3$ | CH | |
| Q-19 | H | H | H | H | $CH_3$ | $OCH_3$ | CH | |
| Q-19 | H | H | H | H | $CH_3$ | $OCH_3$ | N | |
| Q-19 | H | H | H | H | $OCH_3$ | $OCH_3$ | CH | |
| Q-19 | H | H | H | H | $OCH_3$ | $OCH_3$ | N | |
| Q-19 | H | $CH_3$ | H | H | $CH_3$ | $CH_3$ | CH | |
| Q-19 | H | H | $CH_3$ | H | $CH_3$ | $CH_3$ | N | |
| Q-19 | H | H | H | $CH_3$ | $CH_3$ | $OCH_3$ | CH | |
| Q-19 | H | $CH_3$ | $CH_3$ | H | $CH_3$ | $OCH_3$ | N | |
| Q-19 | H | H | $CH_3$ | $CH_3$ | $OCH_3$ | $OCH_3$ | CH | |
| Q-19 | H | $CH_3$ | H | $CH_3$ | $OCH_3$ | $OCH_3$ | N | |
| Q-19 | H | H | H | H | $CH_3$ | $CH_3$ | CH | |
| Q-19 | H | $CH_3$ | $CH_3$ | $CH_3$ | $CH_3$ | $OCH_3$ | CH | |
| Q-19 | $CH_3$ | H | H | H | $CH_3$ | $OCH_3$ | N | |
| Q-19 | H | H | H | H | $OCH_3$ | 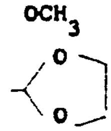 | CH | |

Table 4d

General Structure 2

| Q | R | $R_3'$," | X | Y | Z | W' | m.p. °C |
|---|---|---|---|---|---|---|---|
| Q-21 | H | H | $CH_3$ | $OCH_3$ | CH | O | |
| Q-21 | H | H | $CH_3$ | $OCH_3$ | N | O | |
| Q-21 | H | H | $OCH_3$ | $OCH_3$ | CH | O | |
| Q-21 | $CH_3$ | H | $OCH_3$ | $OCH_3$ | CH | O | |
| Q-21 | H | H | $OCH_3$ | $NHCH_3$ | CH | O | |
| Q-21 | H | H | $OCH_3$ | $OCH_3$ | N | O | |
| Q-21 | H | $CH_3$ | $CH_3$ | $OCH_3$ | CH | O | |
| Q-21 | $CH_3$ | $CH_3$ | $CH_3$ | $OCH_3$ | CH | O | |
| Q-21 | H | H | $CH_3$ | $CH_3$ | CH | O | |
| Q-21 | H | H | $CH_3$ | $CH_3$ | N | O | |
| Q-21 | H | H | $CH_3$ | $OCH_3$ | CH | S | |
| Q-21 | H | H | $CH_3$ | $OCH_3$ | N | S | |
| Q-21 | H | H | $OCH_3$ | $OCH_3$ | CH | S | |
| Q-21 | H | H | $OCH_3$ | $OCH_3$ | N | S | |
| Q-21 | H | $CH_3$ | $CH_3$ | $OCH_3$ | CH | S | |
| Q-21 | $CH_3$ | H | $CH_3$ | $OCH_3$ | CH | S | |
| Q-21 | H | H | $OCH_3$ | $N(CH_3)_2$ | N | S | |
| Q-22 | H | H | $CH_3$ | $OCH_3$ | CH | O | |
| Q-22 | H | H | $CH_3$ | $OCH_3$ | N | O | |
| Q-22 | H | H | $OCH_3$ | $OCH_3$ | CH | O | |
| Q-22 | H | H | $OCH_3$ | $OCH_3$ | N | O | |
| Q-22 | H | $CH_3$ | $CH_3$ | $OCH_3$ | CH | O | |
| Q-22 | H | $CH_3$ | $CH_3$ | $OCH_3$ | N | O | |
| Q-22 | $CH_3$ | $CH_3$ | $CH_3$ | $CH_3$ | CH | O | |
| Q-22 | H | H | $CH_3$ | $CH(OCH_3)_2$ | CH | O | |
| Q-22 | H | H | $CH_3$ | $C_2H_5$ | N | O | |
| Q-22 | H | H | $CH_3$ | $CH_3$ | CH | S | |
| Q-22 | H | H | $CH_3$ | $CH_3$ | N | S | |
| Q-22 | H | H | $CH_3$ | $OCH_3$ | CH | S | |
| Q-22 | H | H | $CH_3$ | $OCH_3$ | N | S | |

$R_{11}$ is -, unless indicated by ** where $R_{11}$ is $CH_3$ in all Tables.

Table 4d (continued)

| Q | R | $R'_3$," | X | Y | Z | W' | m.p. °C |
|---|---|---|---|---|---|---|---|
| Q-22 | H | H | $OCH_3$ | $OCH_3$ | CH | S | |
| Q-22 | H | H | $OCH_3$ | $OCH_3$ | N | S | |
| Q-22 | H | $CH_3$ | $CH_3$ | $OCH_3$ | CH | S | |
| Q-22 | $CH_3$ | $CH_3$ | $CH_3$ | $OCH_3$ | CH | S | |
| Q-22 | $CH_3$ | H | $CH_3$ | $OCH_3$ | N | S | |
| Q-22 | H | H | $CH_3$ | 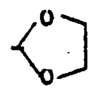 | CH | S | |
| Q-22 | H | H | $OCH_3$ | $C_2H_5$ | N | S | |
| Q-23 | H | H | $CH_3$ | $OCH_3$ | CH | - | |
| Q-23 | H | H | $CH_3$ | $OCH_3$ | N | - | |
| Q-23 | H | H | $OCH_3$ | $OCH_3$ | CH | - | |
| Q-23 | H | H | $OCH_3$ | $OCH_3$ | N | - | |
| Q-23 | H | $CH_3$ | $CH_3$ | $CH_3$ | CH | - | |
| Q-23 | H | $CH_3$ | $CH_3$ | $OCH_3$ | N | - | |
| Q-23 | $CH_3$ | H | $CH_3$ | $OCH_3$ | N | - | |
| Q-23 | $CH_3$ | $CH_3$ | $CH_3$ | $OCH_3$ | CH | - | |
| Q-23 | H | H | $CH_3$ | $OC_2H_5$ | N | - | |
| Q-23 | H | H | $CH_3$ | $CH_2OCH_3$ | CH | - | |
| Q-24 | H | H | $CH_3$ | $OCH_3$ | CH | - | |
| Q-24 | H | H | $CH_3$ | $OCH_3$ | N | - | |
| Q-24 | H | H | $OCH_3$ | $OCH_3$ | CH | - | |
| Q-24 | H | H | $OCH_3$ | $OCH_3$ | N | - | |
| Q-24 | H | $CH_3$ | $CH_3$ | $OCH_3$ | CH | - | |
| Q-24 | H | $CH_3$ | $CH_3$ | $CH_3$ | CH | - | |
| Q-24 | $CH_3$ | H | $CH_3$ | $CH_3$ | N | - | |
| Q-24 | H | H | $CH_3$ | $CF_3$ | CH | - | |
| Q-25 | H | H | $CH_3$ | $OCH_3$ | CH | - | ** |
| Q-25 | H | H | $CH_3$ | $OCH_3$ | N | - | ** |
| Q-25 | H | H | $OCH_3$ | $OCH_3$ | CH | - | ** |

Table 4d (continued)

| Q | R | $R_3'$," | X | Y | Z | W' | m.p. °C |
|---|---|---|---|---|---|---|---|
| Q-25 | H | H | $OCH_3$ | $OCH_3$ | N | - | ** |
| Q-25 | $CH_3$ | H | $OCH_3$ | $OCH_3$ | N | - | ** |
| Q-25 | H | H | $CH_3$ | $CH_3$ | CH | - | ** |
| Q-25 | H | H | $CH_3$ | $CH_3$ | N | - | ** |
| Q-25 | H | $CH_3$ | $CH_3$ | $OCH_3$ | CH | - | ** |
| Q-25 | H | $CH_3$ | $CH_3$ | $OCH_3$ | N | - | ** |
| Q-25 | $CH_3$ | $CH_3$ | $OCH_3$ | $OCH_3$ | N | - | ** |
| Q-25 | H | H | $CH_3$ | $OCH_2CF_3$ | CH | - | ** |
| Q-25 | H | H | $CH_3$ | $NHCH_3$ | N | - | ** |
| Q-26 | H | H | $CH_3$ | $OCH_3$ | CH | - | ** |
| Q-26 | H | H | $CH_3$ | $OCH_3$ | N | - | ** |
| Q-26 | H | H | $OCH_3$ | $OCH_3$ | CH | - | ** |
| Q-26 | H | $CH_3$ | $CH_3$ | $CH_3$ | N | - | ** |
| Q-26 | H | $CH_3$ | $CH_3$ | $CH_3$ | CH | - | ** |
| Q-26 | H | $CH_3$ | $CH_3$ | $OCH_3$ | N | - | ** |
| Q-26 | H | $CH_3$ | $CH_3$ | $OCH_3$ | CH | - | ** |
| Q-26 | H | $CH_3$ | $CH_3$ | $OCH_3$ | N | - | ** |
| Q-26 | H | H | $CH_3$ | $CH_3$ | CH | - | ** |
| Q-26 | H | H | $CH_3$ | $CH_3$ | N | - | ** |
| Q-26 | H | $CH_3$ | $OCH_3$ | $OCH_3$ | CH | - | ** |
| Q-26 | H | $CH_3$ | $OCH_3$ | $OCH_3$ | N | - | ** |
| Q-26 | $CH_3$ | H | $CH_3$ | $OCH_3$ | CH | - | ** |
| Q-26 | H | H | $CH_3$ | $N(CH_3)_2$ | CH | - | ** |
| Q-26 | H | H | $CH_3$ | $N(CH_3)_2$ | N | - | ** |
| Q-27 | H | H | $CH_3$ | $OCH_3$ | CH | - | ** |
| Q-27 | H | H | $CH_3$ | $OCH_3$ | N | - | ** |
| Q-27 | H | H | $OCH_3$ | $OCH_3$ | CH | - | ** |
| Q-27 | H | H | $OCH_3$ | $OCH_3$ | N | - | ** |
| Q-27 | H | H | $CH_3$ | $CH_3$ | CH | - | ** |
| Q-27 | H | H | $CH_3$ | $CH_3$ | N | - | ** |
| Q-27 | H | $CH_3$ | $CH_3$ | $CH_3$ | CH | - | ** |

Table 4d (continued)

| Q | R | $R_3$'," | X | Y | Z | W' | | m.p. °C |
|---|---|---|---|---|---|---|---|---|
| Q-27 | H | H | Cl | $OC_2H_5$ | CH | - | ** | |
| Q-27 | H | $CH_3$ | $CH_3$ | $CH_3$ | N | - | ** | |
| Q-27 | H | $CH_3$ | $CH_3$ | $OCH_3$ | CH | - | ** | |
| Q-27 | H | $CH_3$ | $CH_3$ | $OCH_3$ | N | - | ** | |
| Q-27 | H | $CH_3$ | $OCH_3$ | $OCH_3$ | CH | - | ** | |
| Q-27 | H | $CH_3$ | $OCH_3$ | $OCH_3$ | N | - | ** | |
| Q-27 | $CH_3$ | $CH_3$ | $CH_3$ | $OCH_3$ | N | - | ** | |
| Q-27 | $CH_3$ | H | $CH_3$ | $OCH_3$ | N | - | ** | |
| Q-27 | H | H | $CH_3$ | $CH(OCH_3)_2$ | CH | - | ** | |
| Q-27 | H | H | $CH_3$ | $O(CH_2)_3CH_2Cl$ | CH | - | ** | |
| Q-25 | H | SH | $CH_3$ | $CH_3$ | CH | - | ** | 210-219 |
| Q-25 | H | SH | $CH_3$ | $OCH_3$ | CH | - | ** | 220-222 |
| Q-25 | H | SH | $OCH_3$ | $OCH_3$ | CH | - | ** | 171-173 |
| Q-25 | H | SH | $OCH_3$ | Cl | CH | - | ** | 180-182 |
| Q-25 | H | SH | $CH_3$ | $OCH_3$ | N | - | ** | 169-171 |
| Q-25 | H | SH | $OCH_3$ | $OCH_3$ | N | - | ** | 209-213 |
| Q-25 | H | $SCH_3$ | $CH_3$ | $CH_3$ | CH | - | ** | 154-160 |
| Q-25 | H | $SCH_3$ | $CH_3$ | $OCH_3$ | CH | - | ** | 142-145 |
| Q-25 | H | $SCH_3$ | $OCH_3$ | $OCH_3$ | CH | - | ** | 178-180 |
| Q-25 | H | $SCH_3$ | $OCH_3$ | Cl | CH | - | ** | 192-195 |
| Q-25 | H | $SCH_3$ | CH | $OCH_3$ | N | - | ** | 198-200 |
| Q-25 | H | $SCH_3$ | $OCH_3$ | $OCH_3$ | N | - | ** | 152-153 |
| Q-25 | H | $SCH_2CH{=}CH_2$ | $CH_3$ | $CH_3$ | CH | - | ** | 173-175 |
| Q-25 | H | $SCH_2CH{=}CH_2$ | $CH_3$ | $OCH_3$ | CH | - | ** | 154-159 |
| Q-25 | H | $SCH_2CH{=}CH_2$ | $OCH_3$ | $OCH_3$ | CH | - | ** | 115-117 |
| Q-25 | H | $SCH_2CH{=}CH_2$ | $OCH_3$ | Cl | CH | - | ** | 114-116 |
| Q-25 | H | $SCH_2CH{=}CH_2$ | $CH_3$ | $OCH_3$ | N | - | ** | 142-145 |
| Q-25 | H | $SCH_2CH{=}CH_2$ | $OCH_3$ | $OCH_3$ | N | - | ** | 91-93 |
| Q-25 | $CH_3$ | $SCH_3$ | $OCH_3$ | $OCH_3$ | CH | - | ** | |
| Q-25 | $CH_3$ | SH | $OCH_3$ | $OCH_3$ | CH | - | ** | |
| Q-25 | $CH_3$ | $SCH_2CH{=}CH_2$ | $OCH_3$ | $OCH_3$ | CH | - | ** | |

Table 4e

General Structure 2

| Q | R | $R_3$ | $R_4$ | $R_5$ | X | Y | Z | m.p. °C |
|---|---|---|---|---|---|---|---|---|
| Q-29 | H | H | H | - | $CH_3$ | $OCH_3$ | CH | |
| Q-29 | H | H | H | - | $CH_3$ | $OCH_3$ | N | |
| Q-29 | H | H | H | - | $OCH_3$ | $OCH_3$ | CH | |
| Q-29 | H | H | H | - | $OCH_3$ | $OCH_3$ | N | |
| Q-29 | H | H | H | - | $CH_3$ | $CH_3$ | CH | |
| Q-29 | H | H | H | - | $CH_3$ | $CH_3$ | N | |
| Q-29 | H | $CH_3$ | H | - | $CH_3$ | $OCH_3$ | CH | |
| Q-29 | H | $CH_3$ | H | - | $CH_3$ | $CH_3$ | CH | |
| Q-29 | H | $CH_3$ | $CH_3$ | - | $CH_3$ | $OCH_3$ | N | |
| Q-29 | $CH_3$ | $CH_3$ | $CH_3$ | - | $CH_3$ | $CH_3$ | N | |
| Q-29 | $CH_3$ | H | H | - | $OCH_3$ | $OCH_3$ | CH | |
| Q-29 | H | H | H | - | $CH_3$ | $OC_2H_5$ | N | |
| Q-29 | H | H | H | - | Cl | $OCH_3$ | CH | |
| Q-36 | H | H | H | H | $CH_3$ | $OCH_3$ | CH | |
| Q-36 | $CH_3$ | H | H | H | $CH_3$ | $OCH_3$ | CH | |
| Q-36 | H | H | H | H | $CH_3$ | $OCH_3$ | N | |
| Q-36 | H | H | H | H | $OCH_3$ | $OCH_3$ | CH | |
| Q-36 | H | H | H | H | $OCH_3$ | $OCH_3$ | N | |
| Q-36 | H | H | H | H | $OCH_3$ | $C_2H_5$ | CH | |
| Q-36 | H | H | H | H | $CH_3$ | $CH_3$ | CH | |
| Q-36 | H | H | H | H | $CH_3$ | $CH_3$ | N | |
| Q-36 | H | $CH_3$ | H | H | $CH_3$ | $CH_3$ | CH | |
| Q-36 | H | H | $CH_3$ | H | $CH_3$ | $CH_3$ | N | |
| Q-36 | H | $CH_3$ | H | $CH_3$ | $CH_3$ | $OCH_3$ | CH | |
| Q-36 | H | $CH_3$ | $CH_3$ | H | $CH_3$ | $OCH_3$ | N | |
| Q-36 | H | $CH_3$ | $CH_3$ | $CH_3$ | $OCH_3$ | $OCH_3$ | CH | |
| Q-36 | $CH_3$ | H | H | H | $OCH_3$ | $OCH_3$ | N | |
| Q-36 | H | H | H | H | $CH_3$ | $NHCH_3$ | CH | |
| Q-36 | H | H | H | H | $CH_3$ | $N(CH_3)_2$ | CH | |
| Q-37 | H | H | H | - | $CH_3$ | $OCH_3$ | CH | |

wherein W' is O.

Table 4e (continued)

| Q | R | $R_3$ | $R_4$ | $R_5$ | X | Y | Z | m.p. °C |
|---|---|---|---|---|---|---|---|---|
| Q-37 | H | H | H | - | $CH_3$ | $OCH_3$ | N | |
| Q-37 | H | H | H | - | $OCH_3$ | $OCH_3$ | CH | |
| Q-37 | H | H | H | - | $OCH_3$ | $OCH_3$ | N | |
| Q-37 | H | H | H | - | $CH_3$ | $CH_3$ | CH | |
| Q-37 | H | 2-$CH_3$ | H | - | $CH_3$ | $CH_3$ | CH | |
| Q-37 | H | 4-$CH_3$ | H | - | $CH_3$ | $CH_3$ | N | |
| Q-37 | H | 5-$CH_3$ | H | - | $CH_3$ | OCH | CH | |
| Q-37 | H | H | H | - | $CH_3$ | $CH_3$ | N | |
| Q-37 | H | 2-$CH_3$ | 4-$CH_3$ | - | $CH_3$ | $OCH_3$ | N | |
| Q-37 | H | 2-$CH_3$ | 5-$CH_3$ | - | $OCH_3$ | $OCH_3$ | CH | |
| Q-37 | H | 4-$CH_3$ | 5-$CH_3$ | - | $OCH_3$ | $OCH_3$ | N | |
| Q-37 | $CH_3$ | H | H | - | $CH_3$ | $OCH_3$ | CH | |
| Q-37 | H | H | H | - | $CH_3$ | $CH(OCH_3)_2$ | CH | |
| Q-37 | H | H | H | - | Cl | $OCH_3$ | CH | |
| Q-38 | H | H | H | - | $CH_3$ | $OCH_3$ | CH | |
| Q-38 | H | H | H | - | $CH_3$ | $OCH_3$ | N | |
| Q-38 | H | H | H | - | $OCH_3$ | $OCH_3$ | CH | |
| Q-38 | H | H | H | - | $OCH_3$ | $OCH_3$ | N | |
| Q-38 | H | 2-$CH_3$ | H | - | $CH_3$ | $CH_3$ | CH | |
| Q-38 | H | 4-$CH_3$ | H | - | $CH_3$ | $CH_3$ | N | |
| Q-38 | H | 2-$CH_3$ | 4-$CH_3$ | - | $CH_3$ | $OCH_3$ | CH | |
| Q-38 | H | 4-$CH_3$ | 4-$CH_3$ | - | $CH_3$ | $OCH_3$ | N | |
| Q-38 | H | H | H | - | $CH_3$ | $CH_3$ | N | |
| Q-38 | $CH_3$ | 4-$CH_3$ | 6-$CH_3$ | - | $OCH_3$ | $OCH_3$ | CH | |
| Q-38 | H | H | H | - | $CH_3$ | 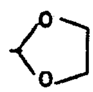 | CH | |
| Q-38 | H | H | H | - | $CH_3$ | $CH_3$ | CH | |
| Q-38 | H | H | H | - | $OCH_3$ | $C_2H_5$ | N | |
| Q-39 | H | H | H | - | $CH_3$ | $OCH_3$ | CH | |
| Q-39 | H | H | H | - | $CH_3$ | $OCH_3$ | N | |
| Q-39 | H | H | H | - | $CH_3$ | $CH_3$ | N | |

Table 4e(continued)

| Q | R | $R_3$ | $R_4$ | $R_5$ | X | Y | Z | m.p. °C |
|---|---|---|---|---|---|---|---|---|
| Q-39 | H | H | H | - | $OCH_3$ | $OCH_3$ | CH | |
| Q-39 | H | H | H | - | $OCH_3$ | $OCH_3$ | N | |
| Q-39 | $CH_3$ | H | H | - | $OCH_3$ | $OCH_3$ | N | |
| Q-39 | H | 3-$CH_3$ | H | - | $CH_3$ | $CH_3$ | CH | |
| Q-39 | H | H | H | - | $CH_3$ | $CH_3$ | CH | |
| Q-39 | H | 5-$CH_3$ | H | - | $CH_3$ | $CH_3$ | N | |
| Q-39 | H | H | H | - | Cl | $OC_2H_5$ | CH | |
| Q-39 | H | 6-$CH_3$ | H | - | $CH_3$ | $OCH_3$ | CH | |
| Q-39 | H | 3-$CH_3$ | 5-$CH_3$ | - | $CH_3$ | $OCH_3$ | N | |
| Q-39 | H | 3-$CH_3$ | 6-$CH_3$ | - | $OCH_3$ | $OCH_3$ | CH | |
| Q-39 | H | 5-$CH_3$ | 6-$CH_3$ | - | $OCH_3$ | $OCH_3$ | N | |
| Q-39 | H | H | H | - | $OCH_3$ | $OC_2H_5$ | N | |
| Q-39 | H | H | H | - | $OCH_3$ | $CH_2OCH_3$ | N | |
| Q-40 | H | H | H | - | $CH_3$ | $CH_3$ | CH | |
| Q-40 | H | H | H | - | $CH_3$ | $OCH_3$ | CH | |
| Q-40 | H | H | H | - | $CH_3$ | $OCH_3$ | N | |
| Q-40 | H | H | H | - | $OCH_3$ | $OCH_3$ | CH | |
| Q-40 | H | H | H | - | $OCH_3$ | $OCH_3$ | N | |
| Q-40 | H | H | H | - | $CH_3$ | $CH_3$ | N | |
| Q-40 | H | 4-$CH_3$ | H | - | $CH_3$ | $CH_3$ | CH | |
| Q-40 | H | 5-$CH_3$ | H | - | $CH_3$ | $CH_3$ | N | |
| Q-40 | H | 6-$CH_3$ | H | - | $CH_3$ | $OCH_3$ | CH | |
| Q-40 | H | 4-$CH_3$ | 5-$CH_3$ | - | $CH_3$ | $OCH_3$ | N | |
| Q-40 | H | 4-$CH_3$ | 6-$CH_3$ | - | $OCH_3$ | $OCH_3$ | CH | |
| Q-40 | H | 5-$CH_3$ | 6-$CH_3$ | - | $OCH_3$ | $OCH_3$ | N | |
| Q-40 | $CH_3$ | H | H | - | $CH_3$ | $CH_3$ | CH | |
| Q-40 | H | H | H | - | $OCH_3$ | $CF_3$ | N | |
| Q-43 | H | H | H | - | $CH_3$ | $CH_3$ | CH | |
| Q-43 | H | H | H | - | $CH_3$ | $OCH_3$ | CH | |
| Q-43 | H | H | H | - | $CH_3$ | $OCH_3$ | N | |

Table 4e (continued)

| Q | R | $R_3$ | $R_4$ | $R_5$ | X | Y | Z | m.p. °C |
|---|---|---|---|---|---|---|---|---|
| Q-43 | H | H | H | - | $OCH_3$ | $OCH_3$ | CH | |
| Q-43 | H | H | H | - | $OCH_3$ | $OCH_3$ | N | |
| Q-43 | $CH_3$ | H | H | - | $OCH_3$ | $OCH_3$ | CH | |
| Q-43 | H | $CH_3$ | $CH_3$ | - | $CH_3$ | $OCH_3$ | CH | |
| Q-43 | H | $CH_3$ | H | - | $CH_3$ | $CH_3$ | CH | |
| Q-43 | H | H | H | - | $CH_3$ | $CH_3$ | N | |
| Q-43 | H | H | H | - | $OCH_3$ | $OCH_2CF_3$ | N | |
| Q-43 | H | H | H | - | $OCH_3$ | $NHCH_3$ | N | |
| Q-44 | H | H | H | - | $CH_3$ | $OCH_3$ | CH | |
| Q-44 | H | H | H | - | $CH_3$ | $OCH_3$ | N | |
| Q-44 | H | H | H | - | $OCH_3$ | $OCH_3$ | CH | |
| Q-44 | H | H | H | - | $OCH_3$ | $OCH_3$ | N | |
| Q-44 | H | $CH_3$ | H | - | $CH_3$ | $CH_3$ | CH | |
| Q-44 | H | H | $CH_3$ | - | $CH_3$ | $CH_3$ | N | |
| Q-44 | H | $CH_3$ | $CH_3$ | - | $CH_3$ | $OCH_3$ | N | |
| Q-44 | $CH_3$ | H | H | - | $CH_3$ | $OCH_3$ | N | |
| Q-44 | H | H | H | - | $OCH_3$ | $N(CH_3)_2$ | N | |
| Q-44 | H | H | H | - | Br | $OCH_3$ | CH | |
| Q-44 | H | H | H | - | $CH_3$ | $CH_3$ | CH | |
| Q-44 | H | H | H | - | $CH_3$ | $CH_3$ | N | |
| Q-45 | H | H | - | - | $CH_3$ | $OCH_3$ | CH | |
| Q-45 | H | H | - | - | $CH_3$ | $OCH_3$ | N | |
| Q-45 | H | H | - | - | $OCH_3$ | $OCH_3$ | CH | |
| Q-45 | H | H | - | - | $OCH_3$ | $OCH_3$ | N | |
| Q-45 | H | $CH_3$ | - | - | $CH_3$ | $CH_3$ | CH | |
| Q-45 | $CH_3$ | H | - | - | $CH_3$ | $CH_3$ | N | |
| Q-45 | H | H | - | - | $OCH_3$ | $CH(OCH_3)_2$ | N | |
| Q-45 | H | H | - | - | $CH_3$ | $CH_3$ | CH | |
| Q-45 | H | H | - | - | $CH_3$ | $CH_3$ | N | |
| Q-45 | H | H | - | - | Cl | $OCH_3$ | CH | |

Table 5a

General Structure 2 wherein Q is Q-5.

| R | $R_3$ | $R_4$ | $R_5$ | $R_6$ | $R_7$ | $R_8$ | X | Y | Z | m.p. °C |
|---|---|---|---|---|---|---|---|---|---|---|
| H | H | H | H | H | H | H | $CH_3$ | $CH_3$ | N | |
| H | H | H | H | H | H | H | $CH_3$ | $OCH_3$ | CH | |
| H | H | H | H | H | H | H | $CH_3$ | $OCH_3$ | N | |
| H | H | H | H | H | H | H | $OCH_3$ | $OCH_3$ | CH | |
| H | H | H | H | H | H | H | $OCH_3$ | $OCH_3$ | N | |
| $CH_3$ | H | H | H | H | H | H | $OCH_3$ | $OCH_3$ | CH | |
| H | $CH_3$ | H | H | H | H | H | $CH_3$ | $CH_3$ | CH | |
| H | H | H | $CH_3$ | H | H | H | $CH_3$ | $CH_3$ | N | |
| H | H | H | H | H | $CH_3$ | H | $CH_3$ | $OCH_3$ | CH | |
| H | $CH_3$ | $CH_3$ | H | H | $CH_3$ | H | $CH_3$ | $OCH_3$ | N | |
| H | $CH_3$ | H | $CH_3$ | H | H | H | $OCH_3$ | $OCH_3$ | CH | |
| H | $CH_3$ | H | H | H | $CH_3$ | H | $OCH_3$ | $OCH_3$ | N | |
| H | H | H | $CH_3$ | H | $CH_3$ | H | $CH_3$ | $CH_3$ | CH | |
| H | $CH_3$ | H | $CH_3$ | H | $CH_3$ | H | $CH_3$ | $CH_3$ | N | |
| H | $CH_3$ | $CH_3$ | H | H | H | H | $CH_3$ | $OCH_3$ | CH | |
| H | H | H | H | $CH_3$ | H | H | $CH_3$ | $OCH_3$ | N | |
| H | H | H | H | H | $CH_3$ | $CH_3$ | $OCH_3$ | $OCH_3$ | CH | |
| H | $CH_3$ | $CH_3$ | $CH_3$ | H | H | H | $OCH_3$ | $OCH_3$ | N | |
| H | $CH_3$ | $CH_3$ | H | H | $CH_3$ | H | $CH_3$ | $CH_3$ | CH | |
| H | H | H | $CH_3$ | $CH_3$ | $CH_3$ | H | $CH_3$ | $CH_3$ | N | |
| H | $CH_3$ | H | $CH_3$ | $CH_3$ | $CH_3$ | H | $CH_3$ | $OCH_3$ | CH | |
| H | $CH_3$ | $CH_3$ | $CH_3$ | H | $CH_3$ | H | $CH_3$ | $OCH_3$ | N | |
| H | $CH_3$ | H | $CH_3$ | H | $CH_3$ | $CH_3$ | $OCH_3$ | $OCH_3$ | CH | |
| H | $CH_3$ | H | $CH_3$ | $CH_3$ | $CH_3$ | $CH_3$ | $CH_3$ | $CH_3$ | CH | |
| H | $CH_3$ | H | $CH_3$ | $CH_3$ | $CH_3$ | $CH_3$ | $CH_3$ | $CH_3$ | CH | |
| H | $CH_3$ | $CH_3$ | $CH_3$ | $CH_3$ | $CH_3$ | H | $CH_3$ | $CH_3$ | N | |
| H | $CH_3$ | $CH_3$ | $CH_3$ | $CH_3$ | $CH_3$ | $CH_3$ | $CH_3$ | $OCH_3$ | CH | |
| $CH_3$ | H | H | H | H | H | H | $CH_3$ | $OCH_3$ | N | |

Wherein W" is O.

Table 5a (continued)

| R | $R_3$ | $R_4$ | $R_5$ | $R_6$ | $R_7$ | $R_8$ | X | Y | Z | m.p. °C |
|---|---|---|---|---|---|---|---|---|---|---|
| H | H | H | H | H | H | H | $OCH_3$ | $CH(OCH_3)_2$ | N | |
| H | H | H | H | H | H | H | $OCH_3$ | 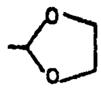 | N | |
| H | H | H | H | H | H | H | Cl | $OCH_3$ | CH | |
| H | H | H | H | H | H | H | $CH_3$ | $CH_3$ | CH | |

**Table 5b**

**General Structure 2 wherein Q is Q-6.**

| Q | R | $R_3$ | $R_4$ | $R_5$ | $R_6$ | $R_7$ | $R_8$ | X | Y | Z | W" | m.p. °C |
|---|---|---|---|---|---|---|---|---|---|---|---|---|
| Q-6 | H | H | H | H | H | H | H | $CH_3$ | $CH_3$ | CH | O | |
| Q-6 | H | H | H | H | H | H | H | $CH_3$ | $CH_3$ | N | O | |
| Q-6 | H | $CH_3$ | H | H | H | H | H | $CH_3$ | $OCH_3$ | CH | O | |
| Q-6 | H | H | H | $CH_3$ | H | H | H | $CH_3$ | $OCH_3$ | N | O | |
| Q-6 | H | H | H | H | H | $CH_3$ | H | $OCH_3$ | $OCH_3$ | CH | O | |
| Q-6 | H | $CH_3$ | H | $CH_3$ | H | H | H | $OCH_3$ | $OCH_3$ | N | O | |
| Q-6 | H | $CH_3$ | H | H | H | $CH_3$ | H | $CH_3$ | $CH_3$ | CH | O | |
| Q-6 | H | H | H | $CH_3$ | H | $CH_3$ | H | $CH_3$ | $CH_3$ | N | O | |
| Q-6 | H | $CH_3$ | $CH_3$ | H | H | H | H | $CH_3$ | $OCH_3$ | CH | O | |
| Q-6 | H | H | H | $CH_3$ | $CH_3$ | H | H | $CH_3$ | $OCH_3$ | N | O | |
| Q-6 | H | H | H | H | H | $CH_3$ | $CH_3$ | $OCH_3$ | $OCH_3$ | CH | O | |
| Q-6 | H | $CH_3$ | H | H | H | $CH_3$ | $CH_3$ | $OCH_3$ | $OCH_3$ | N | O | |
| Q-6 | H | $CH_3$ | $CH_3$ | H | H | $CH_3$ | H | $CH_3$ | $CH_3$ | CH | O | |
| Q-6 | H | $CH_3$ | H | $CH_3$ | $CH_3$ | H | H | $CH_3$ | $CH_3$ | N | O | |
| Q-6 | H | H | H | $CH_3$ | $CH_3$ | $CH_3$ | H | $CH_3$ | $OCH_3$ | CH | O | |
| Q-6 | H | $CH_3$ | $CH_3$ | $CH_3$ | H | H | H | $CH_3$ | $OCH_3$ | N | O | |
| Q-6 | H | H | H | $CH_3$ | $CH_3$ | $CH_3$ | H | $OCH_3$ | $OCH_3$ | CH | O | |
| Q-6 | H | $CH_3$ | H | $CH_3$ | $CH_3$ | $CH_3$ | H | $OCH_3$ | $OCH_3$ | N | O | |
| Q-6 | H | $CH_3$ | $CH_3$ | $CH_3$ | H | $CH_3$ | H | $CH_3$ | $CH_3$ | CH | O | |
| Q-6 | H | $CH_3$ | H | $CH_3$ | H | $CH_3$ | $CH_3$ | $CH_3$ | $CH_3$ | N | O | |
| Q-6 | H | $CH_3$ | $CH_3$ | $CH_3$ | $CH_3$ | H | H | $CH_3$ | $OCH_3$ | CH | O | |
| Q-6 | H | $CH_3$ | $CH_3$ | H | H | $CH_3$ | $CH_3$ | $CH_3$ | $OCH_3$ | N | O | |
| Q-6 | H | H | H | $CH_3$ | $CH_3$ | $CH_3$ | $CH_3$ | $OCH_3$ | $OCH_3$ | CH | O | |
| Q-6 | $CH_3$ | H | H | H | H | H | H | $CH_3$ | $OCH_3$ | N | O | |
| Q-6 | H | H | H | H | H | H | H | Br | $OCH_3$ | CH | O | |
| Q-6 | H | H | H | H | H | H | H | Cl | $OC_2H_5$ | CH | O | |

Table 5b (continued)

| Q | R | R3 | R4 | R5 | R6 | R7 | R8 | X | Y | Z | W" | m.p. °C |
|---|---|---|---|---|---|---|---|---|---|---|---|---|
| Q-6 | H | H | H | H | H | H | H | $OCH_3$ | $C_2H_5$ | CH | O | |
| Q-6 | H | H | H | H | H | H | H | $OCH_3$ | $OC_2H_5$ | CH | O | |
| Q-6 | H | H | H | H | H | H | H | $CH_3$ | $OCH_3$ | CH | O | |
| Q-6 | H | H | H | H | H | H | H | $CH_3$ | $OCH_3$ | N | O | |
| Q-6 | H | H | H | H | H | H | H | $OCH_3$ | $OCH_3$ | CH | O | |
| Q-6 | H | H | H | H | H | H | H | $OCH_3$ | $OCH_3$ | N | O | |
| Q-6 | $CH_3$ | H | H | H | H | H | H | $OCH_3$ | $OCH_3$ | CH | O | |
| Q-6 | H | H | H | H | H | H | H | $CH_3$ | $OCH_3$ | CH | S | |
| Q-6 | H | H | H | H | H | H | H | $CH_3$ | $OCH_3$ | N | S | |
| Q-6 | H | H | H | H | H | H | H | $OCH_3$ | $OCH_3$ | CH | S | |
| Q-6 | H | H | H | H | H | H | H | $OCH_3$ | $OCH_3$ | N | S | |
| Q-6 | H | H | H | H | H | H | H | $OCH_3$ | $C_2H_5$ | CH | S | |
| Q-6 | H | H | H | H | H | H | H | $CH_3$ | $CH_3$ | CH | S | |
| Q-6 | H | H | H | H | H | H | H | $CH_3$ | $CH_3$ | N | S | |
| Q-6 | H | $CH_3$ | H | H | H | H | H | $CH_3$ | $OCH_3$ | CH | S | |
| Q-6 | H | H | H | $CH_3$ | H | H | H | $CH_3$ | $OCH_3$ | N | S | |
| Q-6 | H | H | H | H | H | $CH_3$ | H | $OCH_3$ | $OCH_3$ | CH | S | |
| Q-6 | H | $CH_3$ | H | $CH_3$ | H | H | H | $OCH_3$ | $OCH_3$ | N | S | |
| Q-6 | H | $CH_3$ | H | H | H | $CH_3$ | H | $CH_3$ | $CH_3$ | CH | S | |
| Q-6 | H | H | H | $CH_3$ | H | $CH_3$ | H | $CH_3$ | $CH_3$ | N | S | |
| Q-6 | H | $CH_3$ | $CH_3$ | H | H | H | H | $CH_3$ | $OCH_3$ | CH | S | |
| Q-6 | H | H | H | $CH_3$ | $CH_3$ | H | H | $CH_3$ | $OCH_3$ | N | S | |
| Q-6 | H | H | H | H | H | H | H | $OCH_3$ | $CH_2OCH_3$ | CH | S | |
| Q-6 | H | H | H | H | H | H | H | $OCH_3$ | $CF_3$ | CH | S | |
| Q-6 | H | H | H | H | H | H | H | $OCH_3$ | $OCF_2H$ | CH | S | |
| Q-6 | H | H | H | H | H | $CH_3$ | $CH_3$ | $OCH_3$ | $OCH_3$ | CH | S | |
| Q-6 | H | $CH_3$ | H | H | H | $CH_3$ | $CH_3$ | $OCH_3$ | $OCH_3$ | N | S | |
| Q-6 | H | $CH_3$ | $CH_3$ | H | H | $CH_3$ | H | $CH_3$ | $CH_3$ | CH | S | |
| Q-6 | H | $CH_3$ | H | $CH_3$ | $CH_3$ | H | H | $CH_3$ | $CH_3$ | N | S | |
| Q-6 | H | H | H | $CH_3$ | $CH_3$ | $CH_3$ | H | $CH_3$ | $OCH_3$ | CH | S | |
| Q-6 | H | $CH_3$ | $CH_3$ | $CH_3$ | H | H | H | $CH_3$ | $OCH_3$ | N | S | |

Table 5b (continued)

| Q | R | $R_3$ | $R_4$ | $R_5$ | $R_6$ | $R_7$ | $R_8$ | X | Y | Z | W" | m.p. °C |
|---|---|---|---|---|---|---|---|---|---|---|---|---|
| Q-6 | H | H | H | $CH_3$ | $CH_3$ | $CH_3$ | H | $OCH_3$ | $OCH_3$ | CH | S | |
| Q-6 | H | $CH_3$ | H | $CH_3$ | $CH_3$ | $CH_3$ | H | $OCH_3$ | $OCH_3$ | N | S | |
| Q-6 | H | $CH_3$ | $CH_3$ | $CH_3$ | H | $CH_3$ | H | $CH_3$ | $CH_3$ | CH | S | |
| Q-6 | H | $CH_3$ | H | $CH_3$ | H | $CH_3$ | $CH_3$ | $CH_3$ | $CH_3$ | N | S | |
| Q-6 | H | $CH_3$ | $CH_3$ | $CH_3$ | $CH_3$ | H | H | $CH_3$ | $OCH_3$ | CH | S | |
| Q-6 | H | $CH_3$ | $CH_3$ | H | H | $CH_3$ | $CH_3$ | $CH_3$ | $OCH_3$ | N | S | |
| Q-6 | H | H | H | $CH_3$ | $CH_3$ | $CH_3$ | $CH_3$ | $OCH_3$ | $OCH_3$ | CH | S | |
| Q-6 | $CH_3$ | H | H | H | H | H | H | $CH_3$ | $OCH_3$ | N | S | |

**Table 5c**

**General Structure 2**

| Q | R | $R_3$ | $R_4$ | $R_5$ | $R_6$ | X | Y | Z | W' | m.p. °C |
|---|---|---|---|---|---|---|---|---|---|---|
| Q-7 | H | H | H | H | H | $CH_3$ | $OCH_3$ | CH | - | |
| Q-7 | H | H | H | H | H | $CH_3$ | $OCH_3$ | N | - | |
| Q-7 | H | H | H | H | H | $OCH_3$ | $OCH_3$ | CH | - | |
| Q-7 | H | H | H | H | H | $OCH_3$ | $OCH_3$ | N | - | |
| Q-7 | H | H | H | H | H | $CH_3$ | $CH_3$ | CH | - | |
| Q-7 | H | H | H | H | H | $CH_3$ | $CH_3$ | N | - | |
| Q-7 | H | $CH_3$ | H | H | H | $CH_3$ | $OCH_3$ | CH | - | |
| Q-7 | H | H | $CH_3$ | H | H | $CH_3$ | $OCH_3$ | N | - | |
| Q-7 | H | $CH_3$ | H | H | $CH_3$ | $OCH_3$ | $OCH_3$ | CH | - | |
| Q-7 | H | H | $CH_3$ | $CH_3$ | H | $OCH_3$ | $OCH_3$ | N | - | |
| Q-7 | H | $CH_3$ | $CH_3$ | H | H | $CH_3$ | $CH_3$ | CH | - | |
| Q-7 | H | $CH_3$ | $CH_3$ | $CH_3$ | H | $CH_3$ | $CH_3$ | N | - | |
| Q-7 | H | $CH_3$ | H | $CH_3$ | $CH_3$ | $CH_3$ | $OCH_3$ | CH | - | |
| Q-7 | H | $CH_3$ | $CH_3$ | $CH_3$ | $CH_3$ | $CH_3$ | $OCH_3$ | N | - | |
| Q-7 | $CH_3$ | H | H | H | H | $OCH_3$ | $OCH_3$ | CH | - | |
| Q-7 | $CH_3$ | $CH_3$ | $CH_3$ | $CH_3$ | $CH_3$ | $OCH_3$ | $OCH_3$ | CH | - | |
| Q-7 | H | H | H | H | H | $OCH_3$ | $OCH_2CF_3$ | CH | - | |
| Q-7 | H | H | H | H | H | $OCH_3$ | $NHCH_3$ | CH | - | |
| Q-7 | H | H | H | H | H | Cl | $OCH_3$ | CH | - | |
| Q-7 | H | H | H | H | H | $OCH_3$ | $OCH_3$ | CH | - | * |
| Q-7 | H | H | H | H | H | $OCH_3$ | $CH_3$ | N | - | * |
| Q-7 | H | H | H | H | H | $OCH_3$ | CH | CH | - | * |
| Q-16 | H | $CH_3$ | $CH_3$ | H | H | $CH_3$ | $OCH_3$ | CH | O | 181-183 |
| Q-16 | H | $CH_3$ | $CH_3$ | H | H | $CH_3$ | $OCH_3$ | N | O | 175-176.5 |
| Q-16 | H | $CH_3$ | $CH_3$ | H | H | $OCH_3$ | $OCH_3$ | CH | O | 176-178 |
| Q-16 | H | $CH_3$ | $CH_3$ | H | H | $OCH_3$ | $OCH_3$ | N | O | 174-176 |
| Q-16 | $CH_3$ | $CH_3$ | $CH_3$ | H | H | $CH_3$ | $OCH_3$ | N | O | |
| Q-16 | $CH_3$ | $CH_3$ | $CH_3$ | H | H | Cl | $OCH_3$ | CH | O | |
| Q-16 | H | $CH_3$ | $CH_3$ | H | H | $CH_3$ | $CH_3$ | CH | O | 175-182 |
| Q-16 | H | $CH_3$ | $CH_3$ | H | H | $CH_3$ | $CH_3$ | N | O | |
| Q-16 | H | H | H | H | H | $CH_3$ | $OCH_3$ | CH | O | |
| Q-16 | H | H | H | H | H | $CH_3$ | $OCH_3$ | N | O | |

Table 5c (continued)

| Q | R | $R_3$ | $R_4$ | $R_5$ | $R_6$ | X | Y | Z | W' | m.p. °C |
|---|---|---|---|---|---|---|---|---|---|---|
| Q-16 | H | H | H | H | H | $OCH_3$ | $OCH_3$ | CH | O | |
| Q-16 | H | H | H | H | H | $OCH_3$ | $OCH_3$ | N | O | |
| Q-16 | H | $CH_3$ | $CH_3$ | H | H | $OCH_3$ | $N(CH_3)_2$ | CH | O | |
| Q-16 | H | $CH_3$ | $CH_3$ | H | H | $OCH_3$ | $CH(OCH_3)_2$ | CH | O | |
| Q-16 | H | $CH_3$ | $CH_3$ | H | H | Cl | $OCH_3$ | CH | O | 168-171 |
| Q-16 | H | $CH_3$ | $CH_3$ | H | H | $CH_3$ | $CH_2OCH_3$ | N | O | |
| Q-16 | H | $CH_3$ | H | H | H | $CH_3$ | $CH_3$ | CH | O | |
| Q-16 | H | H | H | $CH_3$ | H | $CH_3$ | $CH_3$ | N | O | |
| Q-16 | H | $CH_3$ | H | $CH_3$ | H | $CH_3$ | $OCH_3$ | N | O | |
| Q-16 | H | H | H | $CH_3$ | $CH_3$ | $CH_3$ | $OCH_3$ | CH | O | |
| Q-16 | H | $CH_3$ | H | $CH_3$ | $CH_3$ | $OCH_3$ | $OCH_3$ | CH | O | |
| Q-16 | H | $CH_3$ | $CH_3$ | $CH_3$ | H | $OCH_3$ | $OCH_3$ | N | O | |
| Q-16 | H | $CH_3$ | $CH_3$ | $CH_3$ | H | $CH_3$ | $CH_3$ | CH | O | |
| Q-16 | H | $CH_3$ | $CH_3$ | $CH_3$ | $CH_3$ | $CH_3$ | $CH_3$ | N | O | |
| Q-16 | H | $CH_3$ | $CH_3$ | H | H | $CH_3$ | $CH_3$ | CH | S | |
| Q-16 | H | $CH_3$ | $CH_3$ | H | H | $CH_3$ | $CH_3$ | N | S | |
| Q-16 | H | $CH_3$ | $CH_3$ | H | H | $CH_3$ | $OCH_3$ | CH | S | |
| Q-16 | H | $CH_3$ | $CH_3$ | H | H | $CH_3$ | $OCH_3$ | N | S | |
| Q-16 | H | $CH_3$ | $CH_3$ | H | H | $OCH_3$ | $OCH_3$ | CH | S | |
| Q-16 | H | $CH_3$ | $CH_3$ | H | H | $OCH_3$ | $OCH_3$ | N | S | |
| Q-16 | H | $CH_3$ | $CH_3$ | H | H | Cl | $OCH_3$ | CH | S | |
| Q-16 | H | $CH_3$ | $CH_3$ | H | H | $CH_3$ | $NHCH_3$ | N | S | |
| Q-16 | H | $CH_3$ | $CH_3$ | H | H | $CH_3$ | $N(CH_3)_2$ | N | S | |
| Q-16 | H | H | H | H | H | $CH_3$ | $OCH_3$ | CH | S | |
| Q-16 | H | H | H | H | H | $CH_3$ | $OCH_3$ | N | S | |
| Q-16 | H | H | H | H | H | $OCH_3$ | $OCH_3$ | CH | S | |
| Q-16 | H | H | H | H | H | $OCH_3$ | $OCH_3$ | N | S | |
| Q-16 | $CH_3$ | H | H | H | H | $CH_3$ | $CH_3$ | CH | S | |
| Q-16 | H | $CH_3$ | H | H | H | $CH_3$ | $CH_3$ | N | S | |
| Q-16 | H | H | H | $CH_3$ | H | $CH_3$ | $OCH_3$ | N | S | |
| Q-16 | H | $CH_3$ | H | $CH_3$ | H | $CH_3$ | $OCH_3$ | CH | S | |
| Q-16 | H | H | H | $CH_3$ | $CH_3$ | $OCH_3$ | $OCH_3$ | CH | S | |

## Table 5c (continued)

| Q | R | $R_3$ | $R_4$ | $R_5$ | $R_6$ | X | Y | Z | W' | m.p. °C |
|---|---|---|---|---|---|---|---|---|---|---|
| Q-16 | H | $CH_3$ | $CH_3$ | $CH_3$ | H | $OCH_3$ | $OCH_3$ | N | S | |
| Q-16 | H | $CH_3$ | $CH_3$ | $CH_3$ | H | $CH_3$ | $CH_3$ | CH | S | |
| Q-16 | H | $CH_3$ | H | $CH_3$ | $CH_3$ | $CH_3$ | $CH_3$ | N | S | |
| Q-16 | H | $CH_3$ | $CH_3$ | $CH_3$ | $CH_3$ | $CH_3$ | $OCH_3$ | CH | S | |
| Q-17 | H | H | H | H | H | Cl | $OCH_3$ | CH | - | |
| Q-17 | H | H | H | H | H | $CH_3$ | $CH_3$ | CH | - | |
| Q-17 | H | H | H | H | H | Cl | $OC_2H_5$ | CH | - | |
| Q-17 | H | H | H | H | H | $CH_3$ | $CH_3$ | N | - | |
| Q-17 | H | H | H | H | H | $OCH_3$ | $OCH_3$ | CH | - | |
| Q-17 | H | H | H | H | H | $OCH_3$ | $OCH_3$ | N | - | |
| Q-17 | H | H | H | H | H | $OCH_3$ | $OC_2H_5$ | CH | - | |
| Q-17 | H | H | H | H | H | $OCH_3$ | $OC_2H_5$ | N | - | |
| Q-17 | H | $CH_3$ | H | H | H | $CH_3$ | $CH_3$ | CH | - | |
| Q-17 | H | H | H | H | H | $CH_3$ | $CH(OCH_3)_2$ | N | - | |
| Q-17 | H | H | H | H | H | $CH_3$ | 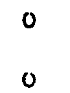 | N | - | |
| Q-17 | H | H | H | $CH_3$ | H | $CH_3$ | $CH_3$ | N | - | |
| Q-17 | H | $CH_3$ | $CH_3$ | H | H | $CH_3$ | $OCH_3$ | CH | - | |
| Q-17 | H | $CH_3$ | $CH_3$ | H | H | $CH_3$ | $OCH_3$ | N | - | |
| Q-17 | H | H | H | $CH_3$ | $CH_3$ | $OCH_3$ | $OCH_3$ | CH | - | |
| Q-17 | H | $CH_3$ | H | $CH_3$ | $CH_3$ | $OCH_3$ | $OCH_3$ | N | - | |
| Q-17 | H | $CH_3$ | $CH_3$ | $CH_3$ | H | $CH_3$ | $CH_3$ | CH | - | |
| Q-17 | H | $CH_3$ | $CH_3$ | $CH_3$ | H | $CH_3$ | $CH_3$ | N | - | |
| Q-17 | H | $CH_3$ | $CH_3$ | $CH_3$ | $CH_3$ | $CH_3$ | $OCH_3$ | CH | - | |
| Q-17 | $CH_3$ | H | H | H | H | $CH_3$ | $OCH_3$ | N | - | |

Table 5d

General Structure 2 wherein Q is Q-30

| R | $R_3$ | $R_4$ | $R_5$ | $R_6$ | $R_7$ | $R_8$ | $R_9$ | X | Y | Z | m.p. °C |
|---|---|---|---|---|---|---|---|---|---|---|---|
| H | H | H | H | H | H | H | H | $CH_3$ | $OCH_3$ | CH | |
| H | H | H | H | H | H | H | H | $CH_3$ | $OCH_3$ | N | |
| H | H | H | H | H | H | H | H | $OCH_3$ | $OCH_3$ | CH | |
| H | H | H | H | H | H | H | H | $OCH_3$ | $OCH_3$ | N | |
| H | H | H | H | H | H | H | H | $CH_3$ | $CH_3$ | N | |
| H | H | H | H | H | H | H | H | $CH_3$ | $CH_3$ | CH | |
| $CH_3$ | H | H | H | H | H | H | H | $CH_3$ | $OCH_3$ | CH | |
| H | $CH_3$ | H | H | H | H | H | H | $CH_3$ | $OCH_3$ | N | |
| H | H | H | H | H | H | H | H | Cl | $OCH_3$ | CH | |
| H | H | $CH_3$ | H | H | H | H | H | $OCH_3$ | $OCH_3$ | CH | |
| H | H | H | H | $CH_3$ | H | H | H | $OCH_3$ | $OCH_3$ | N | |
| H | H | H | H | H | H | $CH_3$ | H | $CH_3$ | $CH_3$ | CH | |
| H | $CH_3$ | $CH_3$ | H | H | H | H | H | $CH_3$ | $CH_3$ | N | |
| H | $CH_3$ | H | H | $CH_3$ | H | H | H | $CH_3$ | $OCH_3$ | CH | |
| H | $CH_3$ | H | H | H | H | $CH_3$ | H | $CH_3$ | $OCH_3$ | N | |
| H | H | $CH_3$ | $CH_3$ | H | H | H | H | $OCH_3$ | $OCH_3$ | CH | |
| H | H | $CH_3$ | H | $CH_3$ | H | H | H | $OCH_3$ | $OCH_3$ | N | |
| H | H | $CH_3$ | H | H | H | $CH_3$ | H | $CH_3$ | $CH_3$ | CH | |
| H | H | H | H | $CH_3$ | $CH_3$ | H | H | $CH_3$ | $CH_3$ | N | |
| H | H | H | H | $CH_3$ | H | $CH_3$ | H | $CH_3$ | $OCH_3$ | CH | |
| H | H | H | H | H | H | $CH_3$ | $CH_3$ | $CH_3$ | $OCH_3$ | N | |
| H | $CH_3$ | $CH_3$ | $CH_3$ | H | H | H | H | $OCH_3$ | $OCH_3$ | CH | |
| H | $CH_3$ | $CH_3$ | H | $CH_3$ | H | H | H | $OCH_3$ | $OCH_3$ | N | |
| H | $CH_3$ | $CH_3$ | H | H | H | $CH_3$ | H | $CH_3$ | $CH_3$ | CH | |
| H | $CH_3$ | H | H | H | $CH_3$ | H | H | $CH_3$ | $CH_3$ | N | |
| H | $CH_3$ | H | H | $CH_3$ | H | $CH_3$ | H | $CH_3$ | $OCH_3$ | CH | |
| H | $CH_3$ | H | H | H | H | $CH_3$ | $CH_3$ | $CH_3$ | $OCH_3$ | N | |
| H | H | $CH_3$ | $CH_3$ | $CH_3$ | H | H | H | $OCH_3$ | $OCH_3$ | CH | |
| H | H | $CH_3$ | $CH_3$ | H | H | $CH_3$ | H | $OCH_3$ | $OCH_3$ | N | |
| H | H | $CH_3$ | H | $CH_3$ | $CH_3$ | H | H | $CH_3$ | $CH_3$ | CH | |
| H | H | $CH_3$ | H | $CH_3$ | H | $CH_3$ | H | $CH_3$ | $CH_3$ | N | |
| H | H | $CH_3$ | H | H | H | $CH_3$ | $CH_3$ | $CH_3$ | $OCH_3$ | CH | |

Table 5d (continued)

| R | $R_3$ | $R_4$ | $R_5$ | $R_6$ | $R_7$ | $R_8$ | $R_9$ | X | Y | Z | m.p. °C |
|---|---|---|---|---|---|---|---|---|---|---|---|
| H | H | H | H | $CH_3$ | $CH_3$ | $CH_3$ | H | $CH_3$ | $OCH_3$ | N | |
| H | H | H | H | $CH_3$ | H | $CH_3$ | $CH_3$ | $OCH_3$ | $OCH_3$ | CH | |
| H | $CH_3$ | $CH_3$ | $CH_3$ | $CH_3$ | H | H | H | $OCH_3$ | $OCH_3$ | N | |
| H | $CH_3$ | $CH_3$ | H | $CH_3$ | H | $CH_3$ | H | $CH_3$ | $CH_3$ | CH | |
| H | $CH_3$ | H | H | $CH_3$ | $CH_3$ | $CH_3$ | H | $CH_3$ | $OCH_3$ | N | |
| H | $CH_3$ | $CH_3$ | $CH_3$ | H | H | $CH_3$ | H | $CH_3$ | $OCH_3$ | CH | |
| H | $CH_3$ | H | H | $CH_3$ | H | $CH_3$ | $CH_3$ | $CH_3$ | $OCH_3$ | N | |
| H | $CH_3$ | $CH_3$ | H | $CH_3$ | $CH_3$ | H | H | $OCH_3$ | $OCH_3$ | CH | |
| H | $CH_3$ | $CH_3$ | H | H | H | $CH_3$ | $CH_3$ | $OCH_3$ | $OCH_3$ | N | |
| H | H | $CH_3$ | $CH_3$ | $CH_3$ | $CH_3$ | H | H | $CH_3$ | $CH_3$ | CH | |
| H | H | $CH_3$ | $CH_3$ | $CH_3$ | H | $CH_3$ | H | $CH_3$ | $CH_3$ | N | |
| H | H | $CH_3$ | H | $CH_3$ | $CH_3$ | $CH_3$ | H | $CH_3$ | $OCH_3$ | CH | |
| H | H | $CH_3$ | H | $CH_3$ | H | $CH_3$ | $CH_3$ | $CH_3$ | $OCH_3$ | N | |
| H | H | H | H | $CH_3$ | $CH_3$ | $CH_3$ | $CH_3$ | $OCH_3$ | $OCH_3$ | CH | |
| H | $CH_3$ | $CH_3$ | $CH_3$ | $CH_3$ | $CH_3$ | H | H | $OCH_3$ | $OCH_3$ | N | |
| H | $CH_3$ | $CH_3$ | H | $CH_3$ | $CH_3$ | $CH_3$ | H | $CH_3$ | $CH_3$ | CH | |
| H | $CH_3$ | $CH_3$ | H | $CH_3$ | H | $CH_3$ | $CH_3$ | $CH_3$ | $CH_3$ | N | |
| H | $CH_3$ | H | H | $CH_3$ | $CH_3$ | $CH_3$ | $CH_3$ | $CH_3$ | $OCH_3$ | CH | |
| H | $CH_3$ | $CH_3$ | $CH_3$ | H | H | $CH_3$ | $CH_3$ | $CH_3$ | $OCH_3$ | N | |
| H | H | $CH_3$ | $CH_3$ | $CH_3$ | H | $CH_3$ | $CH_3$ | $OCH_3$ | $OCH_3$ | CH | |
| H | H | $CH_3$ | H | $CH_3$ | $CH_3$ | $CH_3$ | $CH_3$ | $OCH_3$ | $OCH_3$ | N | |
| H | H | $CH_3$ | $CH_3$ | $CH_3$ | $CH_3$ | $CH_3$ | H | $CH_3$ | $CH_3$ | CH | |
| H | $CH_3$ | $CH_3$ | H | $CH_3$ | $CH_3$ | $CH_3$ | $CH_3$ | $CH_3$ | $CH_3$ | N | |
| H | $CH_3$ | $CH_3$ | $CH_3$ | $CH_3$ | H | $CH_3$ | $CH_3$ | $CH_3$ | $OCH_3$ | CH | |
| H | $CH_3$ | $CH_3$ | $CH_3$ | $CH_3$ | $CH_3$ | $CH_3$ | H | $CH_3$ | $OCH_3$ | N | |
| H | H | $CH_3$ | $CH_3$ | $CH_3$ | $CH_3$ | $CH_3$ | $CH_3$ | $OCH_3$ | $OCH_3$ | CH | |
| H | $CH_3$ | $CH_3$ | $CH_3$ | $CH_3$ | $CH_3$ | $CH_3$ | $CH_3$ | $OCH_3$ | $OCH_3$ | N | |
| $CH_3$ | $CH_3$ | $CH_3$ | $CH_3$ | $CH_3$ | $CH_3$ | $CH_3$ | $CH_3$ | $CH_3$ | $CH_3$ | CH | |
| H | H | H | H | H | H | H | H | $CH_3$ | $OC_2H_5$ | CH | |
| H | H | H | H | H | H | H | H | $CH_3$ | $CH_2OCH_3$ | CH | |

Table 5e

General Structure 2 wherein Q is Q-31.

| R | $R_3$ | $R_4$ | $R_5$ | $R_6$ | $R_7$ | $R_8$ | $R_9$ | $R_{10}$ | X | Y | Z | m.p °C |
|---|---|---|---|---|---|---|---|---|---|---|---|---|
| H | H | H | H | H | H | H | H | H | $CH_3$ | $OCH_3$ | CH | |
| H | H | H | H | H | H | H | H | H | $CH_3$ | $OCH_3$ | N | |
| H | H | H | H | H | H | H | H | H | $OCH_3$ | $OCH_3$ | CH | |
| H | H | H | H | H | H | H | H | H | $OCH_3$ | $OCH_3$ | N | |
| H | H | H | H | H | H | H | H | H | $CH_3$ | $CH_3$ | CH | |
| H | H | H | H | H | H | H | H | H | $CH_3$ | $CH_3$ | N | |
| H | H | H | H | H | H | H | H | H | Cl | $OCH_3$ | CH | |
| H | H | H | H | H | H | H | H | H | $CH_3$ | $C_2H_5$ | CH | |
| H | H | H | H | H | H | H | H | H | $CH_3$ | $CF_3$ | CH | |
| H | H | H | H | H | H | H | H | H | $CH_3$ | $OCF_2H$ | CH | |
| H | H | H | H | H | H | H | H | H | $CH_3$ | $OCH_2CF_3$ | CH | |
| H | $CH_3$ | H | H | H | H | H | H | H | $CH_3$ | $CH_3$ | CH | |
| H | H | H | $CH_3$ | H | H | H | H | H | $CH_3$ | $CH_3$ | N | |
| H | H | H | H | H | $CH_3$ | H | H | H | $CH_3$ | $OCH_3$ | CH | |
| H | H | H | H | H | H | H | $CH_3$ | H | $CH_3$ | $OCH_3$ | N | |
| H | $CH_3$ | $CH_3$ | H | H | H | H | H | H | $OCH_3$ | $OCH_3$ | CH | |
| H | H | H | $CH_3$ | $CH_3$ | H | H | H | H | $OCH_3$ | $OCH_3$ | N | |
| H | H | H | H | H | $CH_3$ | $CH_3$ | H | H | $CH_3$ | $CH_3$ | CH | |
| H | H | H | H | H | H | H | $CH_3$ | $CH_3$ | $CH_3$ | $CH_3$ | N | |
| H | H | H | $CH_3$ | H | H | H | $CH_3$ | H | $CH_3$ | $OCH_3$ | CH | |
| H | $CH_3$ | H | H | H | $CH_3$ | H | H | H | $CH_3$ | $OCH_3$ | N | |
| H | $CH_3$ | H | $CH_3$ | H | H | H | H | H | $OCH_3$ | $OCH_3$ | CH | |
| H | H | H | H | H | $CH_3$ | H | $CH_3$ | H | $OCH_3$ | $OCH_3$ | N | |
| H | $CH_3$ | $CH_3$ | $CH_3$ | H | H | H | H | H | $CH_3$ | $CH_3$ | CH | |
| H | $CH_3$ | H | H | H | $CH_3$ | $CH_3$ | H | H | $CH_3$ | $CH_3$ | N | |
| H | H | H | $CH_3$ | $CH_3$ | H | H | $CH_3$ | H | $CH_3$ | $OCH_3$ | CH | |
| H | H | H | $CH_3$ | $CH_3$ | $CH_3$ | H | H | H | $CH_3$ | $OCH_3$ | N | |
| H | $CH_3$ | H | H | H | $CH_3$ | H | $CH_3$ | H | $OCH_3$ | $OCH_3$ | CH | |
| H | $CH_3$ | $CH_3$ | H | H | H | H | $CH_3$ | $CH_3$ | $OCH_3$ | $OCH_3$ | N | |
| H | $CH_3$ | H | H | H | $CH_3$ | $CH_3$ | $CH_3$ | H | $CH_3$ | $CH_3$ | CH | |

Table 5e (continued)

| R | R3 | R4 | R5 | R6 | R7 | R8 | R9 | R10 | X | Y | Z | | m.p. °C |
|---|---|---|---|---|---|---|---|---|---|---|---|---|---|
| H | $CH_3$ | H | $CH_3$ | H | $CH_3$ | H | $CH_3$ | H | $CH_3$ | $CH_3$ | N | | |
| H | H | H | $CH_3$ | $CH_3$ | $CH_3$ | $CH_3$ | H | H | $CH_3$ | $OCH_3$ | CH | | |
| H | $CH_3$ | $CH_3$ | $CH_3$ | H | H | H | $CH_3$ | $CH_3$ | $CH_3$ | $OCH_3$ | N | | |
| H | $CH_3$ | H | $CH_3$ | $CH_3$ | $CH_3$ | H | $CH_3$ | H | $OCH_3$ | $OCH_3$ | CH | | |
| H | $CH_3$ | H | $CH_3$ | H | $CH_3$ | $CH_3$ | $CH_3$ | H | $OCH_3$ | $OCH_3$ | N | | |
| H | $CH_3$ | $CH_3$ | $CH_3$ | H | $CH_3$ | H | $CH_3$ | H | $CH_3$ | $CH_3$ | CH | | |
| H | $CH_3$ | H | $CH_3$ | $CH_3$ | $CH_3$ | $CH_3$ | $CH_3$ | H | $CH_3$ | $CH_3$ | N | | |
| H | $CH_3$ | H | $CH_3$ | $CH_3$ | $CH_3$ | H | $CH_3$ | $CH_3$ | $CH_3$ | $OCH_3$ | CH | | |
| H | $CH_3$ | H | $CH_3$ | $CH_3$ | $CH_3$ | $CH_3$ | $CH_3$ | $CH_3$ | $CH_3$ | $OCH_3$ | N | | |
| H | $CH_3$ | $CH_3$ | $CH_3$ | $CH_3$ | $CH_3$ | $CH_3$ | $CH_3$ | H | $OCH_3$ | $OCH_3$ | CH | | |
| $CH_3$ | H | H | H | H | H | H | H | H | $OCH_3$ | $OCH_3$ | N | | |
| H | H | H | H | H | H | H | H | H | $CH_3$ | $OCH_3$ | CH | * | |
| H | H | H | H | H | H | H | H | H | $CH_3$ | $OCH_3$ | N | * | |
| H | H | H | H | H | H | H | H | H | $OCH_3$ | $OCH_3$ | CH | * | |
| H | H | H | H | H | H | H | H | H | $OCH_3$ | $OCH_3$ | N | * | |
| H | H | H | H | H | H | H | H | H | $CH_3$ | $CH_3$ | CH | * | |
| H | H | H | H | H | H | H | H | H | $CH_3$ | $CH_3$ | N | * | |
| H | H | H | H | H | H | H | H | H | Cl | $OCH_3$ | CH | * | |

***Wherein W" is O, unless indicated by **** where W" is S in all Tables.

**Table 5f**

**General Structure 2 wherein Q is Q-32.**

| R | $R_3$ | $R_4$ | $R_5$ | $R_6$ | $R_7$ | $R_8$ | $R_9$ | $R_{10}$ | X | Y | Z | W" |
|---|---|---|---|---|---|---|---|---|---|---|---|---|
| H | H | H | H | H | H | H | H | H | $CH_3$ | $CH_3$ | CH | O |
| H | H | H | H | H | H | H | H | H | $CH_3$ | $CH_3$ | N | O |
| H | H | H | H | H | H | H | H | H | Cl | $OCH_3$ | CH | O |
| H | H | H | H | H | H | H | H | H | $CH_3$ | $NHCH_3$ | CH | O |
| H | H | H | H | H | H | H | H | H | $CH_3$ | $N(CH_3)_2$ | CH | O |
| H | H | H | H | H | H | H | H | H | $CH_3$ | $CH(OCH_3)_2$ | CH | O |
| H | H | H | H | H | H | H | H | H | Br | $OCH_3$ | CH | O |
| H | $CH_3$ | H | H | H | H | H | H | H | $CH_3$ | $CH_3$ | CH | O |
| H | H | H | $CH_3$ | H | H | H | H | H | $CH_3$ | $CH_3$ | N | O |
| H | H | H | H | H | $CH_3$ | H | H | H | $CH_3$ | $OCH_3$ | CH | O |
| H | H | H | H | H | H | H | $CH_3$ | H | $CH_3$ | $OCH_3$ | N | O |
| H | $CH_3$ | $CH_3$ | H | H | H | H | H | H | $OCH_3$ | $OCH_3$ | CH | O |
| H | H | H | $CH_3$ | $CH_3$ | H | H | H | H | $OCH_3$ | $OCH_3$ | N | O |
| H | H | H | H | H | $CH_3$ | $CH_3$ | H | H | $CH_3$ | $CH_3$ | CH | O |
| H | H | H | H | H | H | H | $CH_3$ | $CH_3$ | $CH_3$ | $CH_3$ | N | O |
| H | H | H | $CH_3$ | H | H | H | $CH_3$ | H | $CH_3$ | $OCH_3$ | CH | O |
| H | $CH_3$ | H | H | H | $CH_3$ | H | H | H | $CH_3$ | $OCH_3$ | N | O |
| H | $CH_3$ | H | $CH_3$ | H | H | H | H | H | $OCH_3$ | $OCH_3$ | CH | O |
| H | H | H | H | H | $CH_3$ | H | $CH_3$ | H | $OCH_3$ | $OCH_3$ | N | O |
| H | $CH_3$ | $CH_3$ | $CH_3$ | H | H | H | H | H | $CH_3$ | $CH_3$ | CH | O |
| H | $CH_3$ | H | H | H | $CH_3$ | $CH_3$ | H | H | $CH_3$ | $CH_3$ | N | O |
| H | H | H | $CH_3$ | $CH_3$ | H | H | $CH_3$ | H | $CH_3$ | $OCH_3$ | CH | O |
| H | H | H | $CH_3$ | $CH_3$ | $CH_3$ | H | H | H | $CH_3$ | $OCH_3$ | N | O |
| H | $CH_3$ | H | H | H | $CH_3$ | H | $CH_3$ | H | $OCH_3$ | $OCH_3$ | CH | O |
| H | $CH_3$ | $CH_3$ | H | H | H | H | $CH_3$ | $CH_3$ | $OCH_3$ | $OCH_3$ | N | O |
| H | $CH_3$ | H | H | H | $CH_3$ | $CH_3$ | $CH_3$ | H | $CH_3$ | $CH_3$ | CH | O |
| H | $CH_3$ | H | $CH_3$ | H | $CH_3$ | H | $CH_3$ | H | $CH_3$ | $CH_3$ | N | O |
| H | H | H | $CH_3$ | $CH_3$ | $CH_3$ | $CH_3$ | H | H | $CH_3$ | $OCH_3$ | CH | O |
| H | $CH_3$ | $CH_3$ | $CH_3$ | H | H | H | $CH_3$ | $CH_3$ | $CH_3$ | $OCH_3$ | N | O |
| H | $CH_3$ | H | $CH_3$ | $CH_3$ | $CH_3$ | H | $CH_3$ | H | $OCH_3$ | $OCH_3$ | CH | O |

Table 5f (continued)

| R | $R_3$ | $R_4$ | $R_5$ | $R_6$ | $R_7$ | $R_8$ | $R_9$ | $R_{10}$ | X | Y | Z | W" |
|---|---|---|---|---|---|---|---|---|---|---|---|---|
| H | $CH_3$ | H | $CH_3$ | H | $CH_3$ | $CH_3$ | $CH_3$ | H | $OCH_3$ | $OCH_3$ | N | O |
| H | $CH_3$ | $CH_3$ | $CH_3$ | H | $CH_3$ | H | $CH_3$ | H | $CH_3$ | $CH_3$ | CH | O |
| H | $CH_3$ | H | $CH_3$ | $CH_3$ | $CH_3$ | $CH_3$ | $CH_3$ | H | $CH_3$ | $CH_3$ | N | O |
| H | $CH_3$ | H | $CH_3$ | $CH_3$ | $CH_3$ | H | $CH_3$ | $CH_3$ | $CH_3$ | $OCH_3$ | CH | O |
| H | $CH_3$ | H | $CH_3$ | $CH_3$ | $CH_3$ | $CH_3$ | $CH_3$ | $CH_3$ | $CH_3$ | $OCH_3$ | N | O |
| H | $CH_3$ | $CH_3$ | $CH_3$ | $CH_3$ | $CH_3$ | $CH_3$ | $CH_3$ | H | $OCH_3$ | $OCH_3$ | CH | O |
| $CH_3$ | H | H | H | H | H | H | H | H | $OCH_3$ | $OCH_3$ | N | O |
| H | $CH_3$ | H | H | H | H | H | $CH_3$ | H | $CH_3$ | $OCH_3$ | CH | O |
| H | H | H | H | H | H | H | H | H | $CH_3$ | $OCH_3$ | CH | O |
| H | H | H | H | H | H | H | H | H | $CH_3$ | $OCH_3$ | N | O |
| H | H | H | H | H | H | H | H | H | $OCH_3$ | $OCH_3$ | CH | O |
| H | H | H | H | H | H | H | H | H | $OCH_3$ | $OCH_3$ | N | O |
| H | H | H | H | H | H | H | H | H | $CH_3$ | $OCH_3$ | CH | S |
| H | H | H | H | H | H | H | H | H | $CH_3$ | $OCH_3$ | N | S |
| H | H | H | H | H | H | H | H | H | $OCH_3$ | $OCH_3$ | CH | S |
| H | H | H | H | H | H | H | H | H | $OCH_3$ | $OCH_3$ | N | S |
| H | H | H | H | H | H | H | H | H | $CH_3$ | $CH_3$ | CH | S |
| H | H | H | H | H | H | H | H | H | $CH_3$ | $CH_3$ | N | S |
| H | H | H | H | H | H | H | H | H | Cl | $OCH_3$ | CH | S |
| H | H | H | H | H | H | H | H | H | $CH_3$ | O<br>O | CH | S |
| H | H | H | H | H | H | H | H | H | Cl | $OC_2H_5$ | CH | S |
| H | H | H | H | H | H | H | H | H | $CH_3$ | $C_2H_5$ | N | S |
| H | H | H | H | H | H | H | H | H | $CH_3$ | $OC_2H_5$ | N | S |
| H | $CH_3$ | H | H | H | H | H | H | H | $CH_3$ | $CH_3$ | CH | S |
| H | H | H | $CH_3$ | H | H | H | H | H | $CH_3$ | $CH_3$ | N | S |
| H | H | H | H | H | $CH_3$ | H | H | H | $CH_3$ | $OCH_3$ | CH | S |
| H | H | H | H | H | H | H | $CH_3$ | H | $CH_3$ | $OCH_3$ | N | S |
| H | $CH_3$ | $CH_3$ | H | H | H | H | H | H | $OCH_3$ | $OCH_3$ | CH | S |
| H | H | H | $CH_3$ | $CH_3$ | H | H | H | H | $OCH_3$ | $OCH_3$ | N | S |
| H | H | H | H | H | $CH_3$ | $CH_3$ | H | H | $CH_3$ | $CH_3$ | CH | S |

Table 5f (continued)

| R | $R_3$ | $R_4$ | $R_5$ | $R_6$ | $R_7$ | $R_8$ | $R_9$ | $R_{10}$ | X | Y | Z | W'' |
|---|---|---|---|---|---|---|---|---|---|---|---|---|
| H | H | H | H | H | H | H | $CH_3$ | $CH_3$ | $CH_3$ | $CH_3$ | N | S |
| H | H | H | $CH_3$ | H | H | H | $CH_3$ | H | $CH_3$ | $OCH_3$ | CH | S |
| H | $CH_3$ | H | H | H | $CH_3$ | H | H | H | $CH_3$ | $OCH_3$ | N | S |
| H | $CH_3$ | H | $CH_3$ | H | H | H | H | H | $OCH_3$ | $OCH_3$ | CH | S |
| H | H | H | H | H | $CH_3$ | H | $CH_3$ | H | $OCH_3$ | $OCH_3$ | N | S |
| H | $CH_3$ | $CH_3$ | $CH_3$ | H | H | H | H | H | $CH_3$ | $CH_3$ | CH | S |
| H | $CH_3$ | H | H | H | $CH_3$ | $CH_3$ | H | H | $CH_3$ | $CH_3$ | N | S |
| H | H | H | $CH_3$ | $CH_3$ | H | H | $CH_3$ | H | $CH_3$ | $OCH_3$ | CH | S |
| H | H | H | $CH_3$ | $CH_3$ | $CH_3$ | H | H | H | $CH_3$ | $OCH_3$ | N | S |
| H | $CH_3$ | H | H | H | $CH_3$ | H | $CH_3$ | H | $OCH_3$ | $OCH_3$ | CH | S |
| H | $CH_3$ | $CH_3$ | H | H | H | H | $CH_3$ | $CH_3$ | $OCH_3$ | $OCH_3$ | N | S |
| H | $CH_3$ | H | H | H | $CH_3$ | $CH_3$ | $CH_3$ | H | $CH_3$ | $CH_3$ | CH | S |
| H | $CH_3$ | H | $CH_3$ | H | $CH_3$ | H | $CH_3$ | H | $CH_3$ | $CH_3$ | N | S |
| H | H | H | $CH_3$ | $CH_3$ | $CH_3$ | $CH_3$ | H | H | $CH_3$ | $OCH_3$ | N | S |
| H | $CH_3$ | $CH_3$ | $CH_3$ | H | H | H | $CH_3$ | $CH_3$ | $CH_3$ | $OCH_3$ | N | S |
| H | $CH_3$ | H | $CH_3$ | $CH_3$ | $CH_3$ | H | $CH_3$ | H | $OCH_3$ | $OCH_3$ | CH | S |
| H | $CH_3$ | H | $CH_3$ | H | $CH_3$ | $CH_3$ | $CH_3$ | H | $OCH_3$ | $OCH_3$ | N | S |
| H | $CH_3$ | $CH_3$ | $CH_3$ | H | $CH_3$ | H | $CH_3$ | H | $CH_3$ | $CH_3$ | CH | S |
| H | $CH_3$ | H | $CH_3$ | $CH_3$ | $CH_3$ | $CH_3$ | $CH_3$ | H | $CH_3$ | $CH_3$ | N | S |
| H | $CH_3$ | H | $CH_3$ | $CH_3$ | $CH_3$ | H | $CH_3$ | $CH_3$ | $CH_3$ | $OCH_3$ | CH | S |
| H | $CH_3$ | H | $CH_3$ | $CH_3$ | $CH_3$ | $CH_3$ | $CH_3$ | $CH_3$ | $CH_3$ | $OCH_3$ | N | S |
| H | $CH_3$ | $CH_3$ | $CH_3$ | $CH_3$ | $CH_3$ | $CH_3$ | $CH_3$ | H | $OCH_3$ | $OCH_3$ | CH | S |
| $CH_3$ | H | H | H | H | H | H | H | H | $OCH_3$ | $OCH_3$ | N | S |
| H | $CH_3$ | H | H | H | H | H | $CH_3$ | H | $CH_3$ | $OCH_3$ | CH | S |

Table 5g

General Structure 2

| Q | R | $R_3$ | $R_4$ | $R_5$ | $R_6$ | $R_7$ | X | Y | Z | m.p. °C |
|---|---|---|---|---|---|---|---|---|---|---|
| Q-41 | H | H | H | H | H | H | Cl | $OCH_3$ | CH | |
| Q-41 | H | H | H | H | H | H | Br | $OCH_3$ | CH | |
| Q-41 | H | H | H | H | H | H | Cl | $OC_2H_5$ | CH | |
| Q-41 | H | H | H | H | H | H | $CH_3$ | $CH_3$ | N | |
| Q-41 | H | H | H | H | H | H | $OCH_3$ | $OCH_3$ | CH | |
| Q-41 | H | H | H | H | H | H | $OCH_3$ | $OCH_3$ | N | |
| Q-41 | H | H | H | H | H | H | $OCH_3$ | $OC_2H_5$ | CH | |
| Q-41 | H | H | H | H | H | H | $OCH_3$ | $OC_2H_5$ | N | |
| Q-41 | H | H | H | H | H | H | $CH_3$ | $CH_3$ | CH | |
| Q-41 | H | H | H | H | H | H | $CH_3$ | $OCH_3$ | N | |
| Q-41 | H | H | H | H | H | H | $CH_3$ | $OCH_3$ | CH | |
| Q-41 | H | H | H | H | H | H | $CH_3$ | $CH_2OCH_3$ | CH | |
| Q-41 | H | H | H | H | H | H | $CH_3$ | $CF_3$ | N | |
| Q-41 | H | H | H | H | H | H | $CH_3$ | $OCF_2H$ | N | |
| Q-41 | H | $CH_3$ | H | H | H | H | $CH_3$ | $CH_3$ | CH | |
| Q-41 | H | H | H | $CH_3$ | H | H | $CH_3$ | $CH_3$ | N | |
| Q-41 | H | H | H | H | $CH_3$ | H | $CH_3$ | $OCH_3$ | CH | |
| Q-41 | H | $CH_3$ | $CH_3$ | H | H | H | $CH_3$ | $OCH_3$ | N | |
| Q-41 | H | $CH_3$ | $CH_3$ | H | H | H | $OCH_3$ | $OCH_3$ | CH | |
| Q-41 | H | $CH_3$ | $CH_3$ | H | H | H | $OCH_3$ | $OCH_3$ | N | |
| Q-41 | H | $CH_3$ | H | $CH_3$ | H | H | $CH_3$ | $CH_3$ | CH | |
| Q-41 | H | $CH_3$ | H | H | $CH_3$ | H | $CH_3$ | $CH_3$ | N | |
| Q-41 | H | H | H | $CH_3$ | $CH_3$ | H | $CH_3$ | $OCH_3$ | CH | |
| Q-41 | H | H | H | H | $CH_3$ | $CH_3$ | $CH_3$ | $OCH_3$ | N | |
| Q-41 | H | $CH_3$ | $CH_3$ | $CH_3$ | H | H | $OCH_3$ | $OCH_3$ | CH | |
| Q-41 | H | $CH_3$ | $CH_3$ | H | $CH_3$ | H | $OCH_3$ | $OCH_3$ | N | |
| Q-41 | H | $CH_3$ | H | $CH_3$ | $CH_3$ | H | $CH_3$ | $CH_3$ | CH | |
| Q-41 | H | $CH_3$ | H | H | $CH_3$ | $CH_3$ | $CH_3$ | $CH_3$ | N | |
| Q-41 | H | $CH_3$ | $CH_3$ | $CH_3$ | $CH_3$ | H | $CH_3$ | $OCH_3$ | CH | |
| Q-41 | H | $CH_3$ | $CH_3$ | H | $CH_3$ | $CH_3$ | $CH_3$ | $OCH_3$ | N | |

Table 5g (continued)

| Q | R | $R_3$ | $R_4$ | $R_5$ | $R_6$ | $R_7$ | X | Y | Z | W | m.p. °C |
|---|---|---|---|---|---|---|---|---|---|---|---|
| Q-41 | H | $CH_3$ | H | $CH_3$ | $CH_3$ | $CH_3$ | $OCH_3$ | $OCH_3$ | CH | - | |
| Q-41 | H | $CH_3$ | $CH_3$ | $CH_3$ | $CH_3$ | $CH_3$ | $OCH_3$ | $OCH_3$ | N | - | |
| Q-41 | $CH_3$ | H | H | H | H | H | $CH_3$ | $CH_3$ | CH | - | |
| Q-41 | $CH_3$ | H | H | H | H | H | $CH_3$ | $OCH_3$ | CH | - | |
| Q-42 | H | H | H | H | H | H | $CH_3$ | $CH_3$ | CH | - | |
| Q-42 | H | H | H | H | H | H | $CH_3$ | $CH_3$ | N | O | |
| Q-42 | H | H | H | H | H | H | $CH_3$ | $OCH_2CF_3$ | N | O | |
| Q-42 | H | H | H | H | H | H | $CH_3$ | $NHCH_3$ | N | O | |
| Q-42 | H | H | H | H | H | H | $CH_3$ | $N(CH_3)_2$ | N | O | |
| Q-42 | H | H | H | H | H | H | $CH_3$ | $CH(OCH_3)_2$ | N | O | |
| Q-42 | $CH_3$ | H | H | H | H | H | $CH_3$ | $OCH_3$ | CH | O | |
| Q-42 | $CH_3$ | H | H | H | H | H | $CH_3$ | $OCH_3$ | N | O | |
| Q-42 | H | $CH_3$ | H | H | H | H | $CH_3$ | $CH_3$ | CH | O | |
| Q-42 | H | H | H | $CH_3$ | H | H | $CH_3$ | $CH_3$ | N | O | |
| Q-42 | H | H | H | H | $CH_3$ | H | $CH_3$ | $OCH_3$ | CH | O | |
| Q-42 | H | $CH_3$ | $CH_3$ | H | H | H | $CH_3$ | $OCH_3$ | N | O | |
| Q-42 | H | $CH_3$ | $CH_3$ | H | H | H | $OCH_3$ | $OCH_3$ | CH | O | |
| Q-42 | H | $CH_3$ | H | $CH_3$ | H | H | $OCH_3$ | $OCH_3$ | N | O | |
| Q-42 | H | $CH_3$ | H | H | $CH_3$ | H | $CH_3$ | $CH_3$ | CH | O | |
| Q-42 | H | H | H | $CH_3$ | $CH_3$ | H | $CH_3$ | $CH_3$ | N | O | |
| Q-42 | H | H | H | H | $CH_3$ | $CH_3$ | $CH_3$ | $OCH_3$ | CH | O | |
| Q-42 | H | $CH_3$ | $CH_3$ | $CH_3$ | H | H | $CH_3$ | $OCH_3$ | N | O | |
| Q-42 | H | $CH_3$ | $CH_3$ | H | $CH_3$ | H | $OCH_3$ | $OCH_3$ | CH | O | |
| Q-42 | H | $CH_3$ | H | $CH_3$ | $CH_3$ | H | $OCH_3$ | $OCH_3$ | N | O | |
| Q-42 | H | H | H | $CH_3$ | $CH_3$ | $CH_3$ | $CH_3$ | $CH_3$ | CH | O | |
| Q-42 | H | $CH_3$ | H | H | $CH_3$ | $CH_3$ | $CH_3$ | $CH_3$ | N | O | |
| Q-42 | H | $CH_3$ | $CH_3$ | $CH_3$ | $CH_3$ | H | $CH_3$ | $OCH_3$ | CH | O | |
| Q-42 | H | $CH_3$ | $CH_3$ | H | $CH_3$ | $CH_3$ | $CH_3$ | $OCH_3$ | N | O | |
| Q-42 | H | $CH_3$ | H | $CH_3$ | $CH_3$ | $CH_3$ | $OCH_3$ | $OCH_3$ | CH | O | |
| Q-42 | H | $CH_3$ | $CH_3$ | $CH_3$ | $CH_3$ | $CH_3$ | $OCH_3$ | $OCH_3$ | N | O | |
| Q-42 | H | H | H | H | H | H | $CH_3$ | $OCH_3$ | CH | O | |

Table 5g (continued)

| Q | R | $R_3$ | $R_4$ | $R_5$ | $R_6$ | $R_7$ | X | Y | Z | W | m.p. °C |
|---|---|---|---|---|---|---|---|---|---|---|---|
| Q-42 | H | H | H | H | H | H | $CH_3$ | $OCH_3$ | N | O | |
| Q-42 | H | H | H | H | H | H | $OCH_3$ | $OCH_3$ | CH | O | |
| Q-42 | H | H | H | H | H | H | $OCH_3$ | $OCH_3$ | N | O | |
| Q-42 | H | H | H | H | H | H | $CH_3$ | $OCH_3$ | CH | S | |
| Q-42 | H | H | H | H | H | H | $CH_3$ | $OCH_3$ | N | S | |
| Q-42 | H | H | H | H | H | H | $OCH_3$ | $OCH_3$ | CH | S | |
| Q-42 | H | H | H | H | H | H | $OCH_3$ | $OCH_3$ | N | S | |
| Q-42 | H | H | H | H | H | H | $CH_3$ | $CH_3$ | CH | S | |
| Q-42 | H | H | H | H | H | H | $CH_3$ | $CH_3$ | N | S | |
| Q-42 | H | H | H | H | H | H | $CH_3$ | 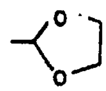 | N | S | |
| Q-42 | H | H | H | H | H | H | Br | $OC_2H_5$ | CH | S | |
| Q-42 | H | H | H | H | H | H | $OCH_3$ | $CH_2OCH_3$ | N | S | |
| Q-42 | $CH_3$ | H | H | H | H | H | $CH_3$ | $OCH_3$ | CH | S | |
| Q-42 | $CH_3$ | H | H | H | H | H | $CH_3$ | $OCH_3$ | N | S | |
| Q-42 | H | $CH_3$ | H | H | H | H | $CH_3$ | $CH_3$ | CH | S | |
| Q-42 | H | H | H | $CH_3$ | H | H | $CH_3$ | $CH_3$ | N | S | |
| Q-42 | H | H | H | H | $CH_3$ | H | $CH_3$ | $OCH_3$ | CH | S | |
| Q-42 | H | $CH_3$ | $CH_3$ | H | H | H | $CH_3$ | $OCH_3$ | N | S | |
| Q-42 | H | $CH_3$ | $CH_3$ | H | H | H | $OCH_3$ | $OCH_3$ | CH | S | |
| Q-42 | H | $CH_3$ | H | $CH_3$ | H | H | $OCH_3$ | $OCH_3$ | N | S | |
| Q-42 | H | $CH_3$ | H | H | $CH_3$ | H | $CH_3$ | $CH_3$ | CH | S | |
| Q-42 | H | H | H | $CH_3$ | $CH_3$ | H | $CH_3$ | $CH_3$ | N | S | |
| Q-42 | H | H | H | H | $CH_3$ | $CH_3$ | $CH_3$ | $OCH_3$ | CH | S | |
| Q-42 | H | $CH_3$ | $CH_3$ | $CH_3$ | H | H | $CH_3$ | $OCH_3$ | N | S | |
| Q-42 | H | $CH_3$ | $CH_3$ | H | $CH_3$ | H | $OCH_3$ | $OCH_3$ | CH | S | |
| Q-42 | H | $CH_3$ | H | $CH_3$ | $CH_3$ | H | $OCH_3$ | $OCH_3$ | N | S | |
| Q-42 | H | H | H | $CH_3$ | $CH_3$ | $CH_3$ | $CH_3$ | $CH_3$ | CH | S | |
| Q-42 | H | $CH_3$ | H | H | $CH_3$ | $CH_3$ | $CH_3$ | $CH_3$ | N | S | |
| Q-42 | H | $CH_3$ | $CH_3$ | $CH_3$ | $CH_3$ | H | $CH_3$ | $OCH_3$ | CH | S | |
| Q-42 | H | $CH_3$ | $CH_3$ | H | $CH_3$ | $CH_3$ | $CH_3$ | $OCH_3$ | N | S | |
| Q-42 | H | $CH_3$ | H | $CH_3$ | $CH_3$ | $CH_3$ | $OCH_3$ | $OCH_3$ | CH | S | |
| Q-42 | H | $CH_3$ | $CH_3$ | $CH_3$ | $CH_3$ | $CH_3$ | $OCH_3$ | $OCH_3$ | N | S | |

**Table 6a**

General Structure 2 wherein Q is Q-20.

| R | $R'_3$ | X | Y | Z | W' | m.p. °C |
|---|---|---|---|---|---|---|
| H | H | $CH_3$ | $OCH_3$ | CH | O | |
| H | H | $CH_3$ | $OCH_3$ | N | O | 150-153 |
| H | H | $OCH_3$ | $OCH_3$ | CH | O | 186-188 |
| H | H | $OCH_3$ | Cl | CH | O | |
| H | H | $OCH_3$ | $OCH_3$ | N | O | |
| H | H | $CH_3$ | $CH_3$ | CH | O | |
| H | $CH_3$ | $CH_3$ | $OCH_3$ | CH | O | |
| $CH_3$ | H | $CH_3$ | $OCH_3$ | N | O | |
| $CH_3$ | $CH_3$ | $OCH_3$ | $OCH_3$ | CH | O | |
| H | H | $CH_3$ | $OC_2H_5$ | N | O | |
| H | H | $CH_3$ | $CH_3$ | CH | S | |
| H | H | $CH_3$ | $CH_3$ | N | S | |
| H | H | $CH_3$ | $OCH_3$ | CH | S | |
| H | H | $CH_3$ | $OCH_3$ | N | S | |
| H | H | $OCH_3$ | $OCH_3$ | CH | S | |
| H | H | $OCH_3$ | $OCH_3$ | N | S | |
| $CH_3$ | H | $CH_3$ | $OCH_3$ | CH | S | |
| H | $CH_3$ | $CH_3$ | $OCH_3$ | N | S | |
| H | $CH_3$ | $CH_3$ | $CH_2OCH_3$ | N | S | |
| H | $C_2H_5$ | $CH_3$ | $CH_3$ | CH | O | |
| H | $C_2H_5$ | $CH_3$ | $CH_3$ | N | O | |
| H | $C_2H_5$ | $CH_3$ | $OCH_3$ | CH | O | |
| H | $C_2H_5$ | $CH_3$ | $OCH_3$ | N | O | |
| H | $C_2H_5$ | $OCH_3$ | $OCH_3$ | CH | O | |
| H | $C_2H_5$ | $OCH_3$ | $OCH_3$ | N | O | |
| H | $C_2H_5$ | $OCH_3$ | Cl | CH | O | |
| H | $C_2H_5$ | $CH_3$ | $CH_3$ | CH | S | |
| H | $C_2H_5$ | $CH_3$ | $CH_3$ | N | S | |
| H | $C_2H_5$ | $CH_3$ | $OCH_3$ | CH | S | |
| H | $C_2H_5$ | $CH_3$ | $OCH_3$ | N | S | |
| H | $C_2H_5$ | $OCH_3$ | $OCH_3$ | CH | S | |
| H | $C_2H_5$ | $OCH_3$ | $OCH_3$ | N | S | |

Table 6a (continued)

| R | $R'_3$ | X | Y | Z | W' | m.p. °C |
|---|---|---|---|---|---|---|
| H | $C_2H_5$ | $OCH_3$ | Cl | CH | S | |
| H | $C_2H_5$ | $OCH_3$ | $N(CH_3)_2$ | N | S | |
| $CH_3$ | n-$C_3H_7$ | $OCH_3$ | $OCH_3$ | CH | O | |
| $CH_3$ | n-$C_3H_7$ | $CH_3$ | $CH_3$ | CH | O | |
| $CH_3$ | n-$C_3H_7$ | $CH_3$ | $OCH_3$ | CH | O | |
| $CH_3$ | n-$C_3H_7$ | $OCH_3$ | $OCH_3$ | CH | O | |
| $CH_3$ | n-$C_3H_7$ | $OCH_3$ | $OCH_3$ | N | O | |
| $CH_3$ | n-$C_3H_7$ | $OCH_3$ | Cl | CH | O | |
| $CH_3$ | n-$C_3H_7$ | $CH_3$ | $OCH_2CF_3$ | CH | O | |
| H | n-$C_3H_7$ | $CH_3$ | $CH_3$ | CH | S | |
| H | n-$C_3H_7$ | $CH_3$ | $OCH_3$ | CH | S | |
| H | n-$C_3H_7$ | $CH_3$ | $OCH_3$ | N | S | |
| H | n-$C_3H_7$ | $OCH_3$ | $OCH_3$ | CH | S | |
| H | n-$C_3H_7$ | $OCH_3$ | $OCH_3$ | N | S | |
| H | n-$C_3H_7$ | $OCH_3$ | Cl | CH | S | |
| H | n-$C_3H_7$ | $CH_3$ | $CH(OCH_3)_2$ | CH | S | |
| H | i-$C_3H_7$ | $CH_3$ | $CH_3$ | CH | O | |
| H | i-$C_3H_7$ | $CH_3$ | $OCH_3$ | CH | O | |
| H | i-$C_3H_7$ | $CH_3$ | $OCH_3$ | N | O | |
| H | i-$C_3H_7$ | $OCH_3$ | $OCH_3$ | CH | O | |
| H | i-$C_3H_7$ | $OCH_3$ | $OCH_3$ | N | O | |
| H | i-$C_3H_7$ | $OCH_3$ | Cl | CH | O | |
| H | i-$C_3H_7$ | $CH_3$ | $CH_3$ | N | O | |
| H | i-$C_3H_7$ | $OCH_3$ | 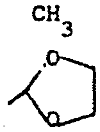 | CH | O | |
| $CH_3$ | i-$C_3H_7$ | $OCH_3$ | $OCH_3$ | CH | S | |
| H | i-$C_3H_7$ | $CH_3$ | $CH_3$ | CH | S | |
| H | i-$C_3H_7$ | $CH_3$ | $OCH_3$ | CH | S | |
| H | i-$C_3H_7$ | $CH_3$ | $OCH_3$ | N | S | |
| H | i-$C_3H_7$ | $OCH_3$ | $OCH_3$ | CH | S | |
| H | i-$C_3H_7$ | $OCH_3$ | $OCH_3$ | N | S | |

Table 6a (continued)

| R | R'3 | X | Y | Z | W' | m.p. °C |
|---|---|---|---|---|---|---|
| H | i-$C_3H_7$ | $OCH_3$ | Cl | CH | S | |
| H | i-$C_3H_7$ | $CH_3$ | $OC_2H_5$ | N | S | |
| H | $SCH_3$ | $CH_3$ | $CH_3$ | CH | O | 175-177 |
| H | $SCH_3$ | $CH_3$ | $OCH_3$ | CH | O | 148-149 |
| H | $SCH_3$ | $CH_3$ | $OCH_3$ | N | O | 167-170 |
| H | $SCH_3$ | $OCH_3$ | $OCH_3$ | CH | O | 178-180 |
| H | $SCH_3$ | $OCH_3$ | $OCH_3$ | N | O | 146-151 |
| H | $SCH_3$ | $OCH_3$ | Cl | CH | O | 163-169 |
| H | $SCH_3$ | $OCH_3$ | $NHCH_3$ | CH | O | |
| $CH_3$ | $SCH_3$ | $OCH_3$ | $OCH_3$ | CH | S | |
| H | $SCH_3$ | $CH_3$ | $CH_3$ | CH | S | |
| H | $SCH_3$ | $CH_3$ | $CH_3$ | N | S | |
| H | $SCH_3$ | $CH_3$ | $OCH_3$ | CH | S | |
| H | $SCH_3$ | $CH_3$ | $OCH_3$ | N | S | |
| H | $SCH_3$ | $OCH_3$ | $OCH_3$ | CH | S | |
| H | $SCH_3$ | $OCH_3$ | $OCH_3$ | N | S | |
| H | $SCH_3$ | $OCH_3$ | Cl | CH | S | |
| $CH_3$ | $SC_2H_5$ | $OCH_3$ | $OCH_3$ | CH | O | |
| H | $SC_2H_5$ | $CH_3$ | $CH_3$ | CH | O | 168-177 |
| H | $SC_2H_5$ | $CH_3$ | $CH_3$ | N | O | |
| H | $SC_2H_5$ | $CH_3$ | $OCH_3$ | CH | O | |
| H | $SC_2H_5$ | $CH_3$ | $OCH_3$ | N | O | |
| H | $SC_2H_5$ | $OCH_3$ | $OCH_3$ | CH | O | |
| H | $SC_2H_5$ | $OCH_3$ | $OCH_3$ | N | O | |
| H | $SC_2H_5$ | $OCH_3$ | Cl | CH | O | |
| H | $SC_2H_5$ | $CH_3$ | $CH_3$ | CH | S | |
| H | $SC_2H_5$ | $CH_3$ | $OCH_3$ | CH | S | |
| H | $SC_2H_5$ | $CH_3$ | $OCH_3$ | N | S | |
| H | $SC_2H_5$ | $OCH_3$ | $OCH_3$ | CH | S | |
| H | $SC_2H_5$ | $CH_3$ | $OCH_3$ | N | S | |
| H | $SC_2H_5$ | $OCH_3$ | Cl | CH | S | |

Table 6a (continued)

| R | R'3 | X | Y | Z | W' | m.p. °C |
|---|---|---|---|---|---|---|
| H | $SC_2H_5$ | $CH_3$ | $OC_2H_5$ | N | S | |
| H | S-n-$C_3H_7$ | $CH_3$ | $CH_3$ | CH | O | |
| H | S-n-$C_3H_7$ | $CH_3$ | $CH_2OCH_3$ | CH | O | |
| H | S-n-$C_3H_7$ | $CH_3$ | $OCH_3$ | CH | O | |
| H | S-n-$C_3H_7$ | $CH_3$ | $OCH_3$ | N | O | |
| H | S-n-$C_3H_7$ | $OCH_3$ | $OCH_3$ | CH | O | |
| H | S-n-$C_3H_7$ | $OCH_3$ | $OCH_3$ | N | O | |
| H | S-n-$C_3H_7$ | $OCH_3$ | Cl | CH | O | |
| $CH_3$ | S-n-$C_3H_7$ | $OCH_3$ | $OCH_3$ | CH | S | |
| H | S-n-$C_3H_7$ | $CH_3$ | $CH_3$ | CH | S | |
| H | S-n-$C_3H_7$ | $CH_3$ | $CH_3$ | N | S | |
| H | S-n-$C_3H_7$ | $CH_3$ | $OCH_3$ | CH | S | |
| H | S-n-$C_3H_7$ | $OCH_3$ | $OCH_3$ | CH | S | |
| H | S-n-$C_3H_7$ | $OCH_3$ | $OCH_3$ | N | S | |
| H | S-n-$C_3H_7$ | $CH_3$ | Cl | CH | S | |
| H | S-n-$C_3H_7$ | $CH_3$ | $OCH_3$ | CH | O | |
| H | S-n-$C_3H_7$ | $OCH_3$ | $OCH_3$ | CH | S | |
| H | S-n-$C_3H_7$ | $CH_3$ | $OCH_3$ | N | S | |
| $CH_3$ | S-n-$C_4H_9$ | $OCH_3$ | $OCH_3$ | CH | O | |
| H | S-n-$C_4H_9$ | $CH_3$ | $CH_3$ | CH | O | |
| H | S-n-$C_4H_9$ | $CH_3$ | $CH_3$ | N | O | |
| H | S-n-$C_4H_9$ | $CH_3$ | $OCH_3$ | CH | O | |
| H | S-n-$C_4H_9$ | $CH_3$ | $OCH_3$ | N | O | |
| H | S-n-$C_4H_9$ | $OCH_3$ | $OCH_3$ | CH | O | |
| H | S-n-$C_4H_9$ | $OCH_3$ | $OCH_3$ | N | O | |
| H | S-n-$C_4H_9$ | $OCH_3$ | Cl | CH | O | |
| H | S-n-$C_4H_9$ | $CH_3$ | $CH_3$ | CH | S | |
| H | S-n-$C_4H_9$ | $CH_3$ | $OCH_3$ | N | S | |
| H | S-n-$C_4H_9$ | $CH_3$ | $OCH_3$ | CH | S | |
| H | S-n-$C_4H_9$ | $OCH_3$ | $OCH_3$ | N | S | |
| H | S-n-$C_4H_9$ | $OCH_3$ | $OCH_3$ | CH | S | |
| H | S-n-$C_4H_9$ | $OCH_3$ | $OCH_3$ | N | S | |

Table 6a (continued)

| R | $R'_3$ | X | Y | Z | W' | m.p. °C |
|---|---|---|---|---|---|---|
| H | S-n-$C_4H_9$ | $OCH_3$ | $OCH_3$ | CH | O | |
| H | S-n-$C_4H_9$ | $CH_3$ | $OCH_3$ | N | O | |
| H | S-n-$C_4H_9$ | $OCH_3$ | $OCH_3$ | CH | S | |
| H | S-n-$C_4H_9$ | $CH_3$ | $OCH_3$ | CH | S | |
| H | S-n-$C_4H_9$ | $CH_3$ | $OCH_3$ | N | O | |
| H | S-n-$C_4H_9$ | $CH_3$ | $OCH_3$ | CH | O | |
| H | S-n-$C_4H_9$ | $OCH_3$ | $OCH_3$ | CH | S | |
| H | S-n-$C_4H_9$ | $OCH_3$ | $OCH_3$ | N | S | |
| H | S-n-$C_4H_9$ | $CH_3$ | $OCH_3$ | CH | O | |
| H | S-n-$C_4H_9$ | $OCH_3$ | $OCH_3$ | N | O | |
| H | S-n-$C_4H_9$ | $OCH_3$ | $OCH_3$ | CH | S | |
| H | S-n-$C_4H_9$ | $CH_3$ | $OCH_3$ | N | S | |
| H | $SCH_2CH=CH_2$ | $CH_3$ | $CH_3$ | CH | O | 169-170 |
| H | $SCH_2CH=CH_2$ | $CH_3$ | $OCH_3$ | N | O | 148-151 |
| H | $SCH_2CH=CH_2$ | $CH_3$ | $OCH_3$ | CH | O | 134-138 |
| H | $SCH_2CH=CH_2$ | $OCH_3$ | $OCH_3$ | N | O | 149-152 |
| H | $SCH_2CH=CH_2$ | $OCH_3$ | $OCH_3$ | CH | O | 165-168 |
| H | $SCH_2CH=CH_2$ | $OCH_3$ | $OCH_3$ | N | O | |
| H | $SCH_2CH=CH_2$ | $OCH_3$ | Cl | CH | O | 144-146 |
| H | $SCH_2CH=CH_2$ | $CH_3$ | $OCH_2CF_3$ | CH | O | |
| $CH_3$ | $SCH_2CH=CH_2$ | $OCH_3$ | $OCH_3$ | CH | S | |
| H | $SCH_2CH=CH_2$ | $CH_3$ | $CH_3$ | CH | S | |
| H | $SCH_2CH=CH_2$ | $CH_3$ | $CH_3$ | N | S | |
| H | $SCH_2CH=CH_2$ | $CH_3$ | $OCH_3$ | CH | S | |
| H | $SCH_2CH=CH_2$ | $CH_3$ | $OCH_3$ | N | S | |
| H | $SCH_2CH=CH_2$ | $OCH_3$ | $OCH_3$ | CH | S | |
| H | $SCH_2CH=CH_2$ | $OCH_3$ | $OCH_3$ | N | S | |
| H | $SCH_2CH=CH_2$ | $OCH_3$ | Cl | CH | S | |
| H | E-$SCH=CHCH_3$ | $CH_3$ | $OCH_3$ | CH | O | |
| H | E-$SCH=CHCH_3$ | $CH_3$ | $OCH_3$ | N | O | |

Table 6a (continued)

| R | $R'_3$ | X | Y | Z | W' | m.p. °C |
|---|---|---|---|---|---|---|
| H | E-SCH=CHCH$_3$ | OCH$_3$ | OCH$_3$ | CH | O | |
| H | E-SCH=CHCH$_3$ | OCH$_3$ | OCH$_3$ | N | O | |
| H | E-SCH=CHCH$_3$ | CH$_3$ | OCH$_3$ | N | S | |
| H | E-SCH=CHCH$_3$ | CH$_3$ | OCH$_3$ | CH | S | |
| H | E-SCH=CHCH$_3$ | OCH$_3$ | OCH$_3$ | CH | S | |
| H | E-SCH=CHCH$_3$ | OCH$_3$ | Cl | CH | S | |
| H | Z-SCH=CHCH$_3$ | CH$_3$ | OCH$_3$ | N | O | |
| H | Z-SCH=CHCH$_3$ | OCH$_3$ | OCH$_3$ | CH | O | |
| H | Z-SCH=CnCH$_3$ | OCH$_3$ | OCH$_3$ | N | O | |
| H | Z-SCH=CHCH$_3$ | OCH$_3$ | Cl | CH | O | |
| H | Z-SCH=CHCH$_3$ | CH$_3$ | CH$_3$ | CH | S | |
| H | Z-SCH=CHCH$_3$ | OCH$_3$ | OCH$_3$ | CH | S | |
| H | Z-SCH=CHCH$_3$ | CH$_3$ | OCH$_3$ | N | S | |
| H | Z-SCH=CHCH$_3$ | OCH$_3$ | OCH$_3$ | N | S | |
| H | S(CH$_2$)$_2$CH=CH$_2$ | OCH$_3$ | OCH$_3$ | CH | O | |
| H | S(CH$_2$)$_2$CH=CH$_2$ | OCH$_3$ | OCH$_3$ | N | O | |
| H | S(CH$_2$)$_2$CH=CH$_2$ | CH$_3$ | OCH$_3$ | CH | O | |
| H | S(CH$_2$)$_2$CH=CH$_2$ | OCH$_3$ | Cl | CH | O | |
| H | S(CH$_2$)$_2$CH=CH$_2$ | OCH$_3$ | OCH$_3$ | CH | S | |
| H | S(CH$_2$)$_2$CH=CH$_2$ | CH$_3$ | OCH$_3$ | N | S | |
| H | S(CH$_2$)$_2$CH=CH$_2$ | OCH$_3$ | OCH$_3$ | N | S | |
| H | S(CH$_2$)$_2$CH=CH$_2$ | CH$_3$ | CH$_3$ | CH | S | |
| H | E-SCH$_2$CH=CHCH$_3$ | OCH$_3$ | OCH$_3$ | CH | O | |
| H | E-SCH$_2$CH=CHCH$_3$ | OCH$_3$ | OCH$_3$ | N | O | |
| H | E-SCH$_2$CH=CHCH$_3$ | CH$_3$ | OCH$_3$ | CH | O | |
| H | E-SCH$_2$CH=CHCH$_3$ | OCH$_3$ | Cl | CH | O | |
| H | E-SCH$_2$CH=CHCH$_3$ | OCH$_3$ | OCH$_3$ | CH | S | |
| H | E-SCH$_2$CH=CHCH$_3$ | CH$_3$ | OCH$_3$ | N | S | |
| H | E-SCH$_2$CH=CHCH$_3$ | CH$_3$ | OCH$_3$ | CH | S | |
| H | E-SCH$_2$CH=CHCH$_3$ | CH$_3$ | CH$_3$ | CH | S | |
| H | Z-SCH$_2$CH=CHCH$_3$ | CH$_3$ | OCH$_3$ | CH | O | |

Table 6a (continued)

| R | $R'_3$ | X | Y | Z | W' | m.p. °C |
|---|---|---|---|---|---|---|
| H | Z-$SCH_2CH=CHCH_3$ | $CH_3$ | $OCH_3$ | N | O | |
| H | Z-$SCH_2CH=CHCH_3$ | $OCH_3$ | $OCH_3$ | CH | O | |
| H | Z-$SCH_2CH=CHCH_3$ | $OCH_3$ | Cl | CH | O | |
| H | Z-$SCH_2CH=CHCH_3$ | $CH_3$ | $OCH_3$ | CH | S | |
| H | Z-$SCH_2CH=CHCH_3$ | $CH_3$ | $OCH_3$ | N | S | |
| H | Z-$SCH_2CH=CHCH_3$ | $OCH_3$ | $OCH_3$ | CH | S | |
| H | Z-$SCH_2CH=CHCH_3$ | $OCH_3$ | $OCH_3$ | N | S | |
| H | E-$SCH=CHC_2H_5$ | $OCH_3$ | $OCH_3$ | CH | O | |
| H | E-$SCH=CHC_2H_5$ | $OCH_3$ | $OCH_3$ | N | O | |
| H | E-$SCH=CHC_2H_5$ | $CH_3$ | $OCH_3$ | CH | O | |
| H | E-$SCH=CHC_2H_5$ | $OCH_3$ | Cl | CH | O | |
| H | E-$SCH=CHC_2H_5$ | $OCH_3$ | $OCH_3$ | CH | S | |
| H | E-$SCH=CHC_2H_5$ | $CH_3$ | $CH_3$ | CH | S | |
| H | E-$SCH=CHC_2H_5$ | $CH_3$ | $OCH_3$ | N | S | |
| H | E-$SCH=CHC_2H_5$ | $CH_3$ | $OCH_3$ | CH | S | |
| H | Z-$SCH=CHC_2H_5$ | $OCH_3$ | $OCH_3$ | CH | O | |
| H | Z-$SCH=CHC_2H_5$ | $OCH_3$ | $OCH_3$ | N | O | |
| H | Z-$SCH=CHC_2H_5$ | $CH_3$ | $CH_3$ | CH | O | |
| H | Z-$SCH=CHC_2H_5$ | $CH_3$ | $OCH_3$ | N | O | |
| H | Z-$SCH=CHC_2H_5$ | $OCH_3$ | $OCH_3$ | CH | S | |
| H | Z-$SCH=CHC_2H_5$ | $CH_3$ | $OCH_3$ | CH | S | |
| H | Z-$SCH=CHC_2H_5$ | $CH_3$ | $OCH_3$ | N | S | |
| H | Z-$SCH=CHC_2H_5$ | $OCH_3$ | Cl | CH | S | |
| H | $SCH_2C\equiv CH$ | $CH_3$ | $OCH_3$ | CH | O | |
| H | $SCH_2C\equiv CH$ | $CH_3$ | $OCH_3$ | N | O | |
| H | $SCH_2C\equiv CH$ | $OCH_3$ | $OCH_3$ | CH | O | |
| H | $SCH_2C\equiv CH$ | $OCH_3$ | $OCH_3$ | N | O | |
| H | $SCH_2C\equiv CH$ | $CH_3$ | $OCH_3$ | CH | S | |
| H | $SCH_2C\equiv CH$ | $OCH_3$ | $OCH_3$ | N | S | |
| H | $SCH_2C\equiv CH$ | $OCH_3$ | $OCH_3$ | CH | S | |
| H | $SCH_2C\equiv CH$ | $OCH_3$ | Cl | CH | S | |

Table 6a (continued)

| R | $R'_3$ | X | Y | Z | W' | m.p. °C |
|---|---|---|---|---|---|---|
| $CH_3$ | $SCH_2C{\equiv}CH$ | $OCH_3$ | $OCH_3$ | CH | S | |
| H | $SC{\equiv}CCH_3$ | $CH_3$ | $CH_3$ | CH | O | |
| H | $SC{\equiv}CCH_3$ | $CH_3$ | $OCH_3$ | N | O | |
| H | $SC{\equiv}CCH_3$ | $CH_3$ | $OCH_3$ | CH | O | |
| H | $SC{\equiv}CCH_3$ | $OCH_3$ | $OCH_3$ | N | O | |
| H | $SC{\equiv}CCH_3$ | $OCH_3$ | $OCH_3$ | CH | S | |
| H | $SC{\equiv}CCH_3$ | $CH_3$ | $OCH_3$ | N | S | |
| H | $SC{\equiv}CCH_3$ | $CH_3$ | $OCH_3$ | CH | S | |
| H | $SC{\equiv}CCH_3$ | $OCH_3$ | $OCH_3$ | N | S | |
| H | $S(CH_2)_2C{\equiv}CH$ | $CH_3$ | $OCH_3$ | CH | O | |
| H | $S(CH_2)_2C{\equiv}CH$ | $CH_3$ | $OCH_3$ | N | O | |
| H | $S(CH_2)_2C{\equiv}CH$ | $OCH_3$ | $OCH_3$ | CH | O | |
| H | $S(CH_2)_2C{\equiv}CH$ | $OCH_3$ | $OCH_3$ | N | O | |
| H | $S(CH_2)_2C{\equiv}CH$ | $CH_3$ | $OCH_3$ | CH | S | |
| H | $S(CH_2)_2C{\equiv}CH$ | $CH_3$ | $OCH_3$ | N | S | |
| H | $S(CH_2)_2C{\equiv}CH$ | $OCH_3$ | $OCH_3$ | CH | S | |
| H | $S(CH_2)_2C{\equiv}CH$ | $OCH_3$ | $OCH_3$ | N | S | |
| H | $SCH_2C{\equiv}CCH_3$ | $CH_3$ | $CH_3$ | CH | O | |
| H | $SCH_2C{\equiv}CCH_3$ | $CH_3$ | $OCH_3$ | N | O | |
| H | $SCH_2C{\equiv}CCH_3$ | $CH_3$ | $OCH_3$ | CH | O | |
| H | $SCH_2C{\equiv}CCH_3$ | $OCH_3$ | $OCH_3$ | CH | O | |
| H | $SCH_2C{\equiv}CCH_3$ | $OCH_3$ | $OCH_3$ | CH | S | |
| H | $SCH_2C{\equiv}CCH_3$ | $OCH_3$ | $OCH_3$ | N | S | |
| H | $SCH_2C{\equiv}CCH_3$ | $CH_3$ | $OCH_3$ | CH | S | |
| H | $SCH_2C{\equiv}CCH_3$ | $OCH_3$ | Cl | CH | S | |
| H | $SC{\equiv}CC_2H_5$ | $CH_3$ | $OCH_3$ | N | O | |
| H | $SC{\equiv}CC_2H_5$ | $OCH_3$ | $OCH_3$ | CH | O | |
| H | $SC{\equiv}CC_2H_5$ | $OCH_3$ | $OCH_3$ | CH | S | |
| H | $SC{\equiv}CC_2H_5$ | $CH_3$ | $CH_3$ | CH | S | |
| $CH_3$ | $SC{\equiv}CC_2H_5$ | $CH_3$ | $OCH_3$ | CH | O | |
| H | $CH_2CN$ | $CH_3$ | $CH_3$ | CH | O | |

Table 6a (continued)

| R | R'3 | X | Y | Z | W' | m.p. °C |
|---|---|---|---|---|---|---|
| H | $CH_2CN$ | $CH_3$ | $OCH_3$ | N | O | |
| H | $CH_2CN$ | $CH_3$ | $OCH_3$ | CH | O | |
| H | $CH_2CN$ | $OCH_3$ | $OCH_3$ | N | O | |
| H | $CH_2CN$ | $OCH_3$ | $OCH_3$ | CH | O | |
| H | $CH_2CN$ | $OCH_3$ | Cl | CH | O | |
| H | $CH_2CN$ | $CH_3$ | $NHCH_3$ | N | O | |
| $CH_3$ | $CH_2CN$ | $OCH_3$ | $OCH_3$ | CH | S | |
| H | $CH_2CN$ | $CH_3$ | $CH_3$ | CH | S | |
| H | $CH_2CN$ | $CH_3$ | $CH_3$ | N | S | |
| H | $CH_2CN$ | $CH_3$ | $OCH_3$ | CH | S | |
| H | $CH_2CN$ | $CH_3$ | $OCH_3$ | N | S | |
| H | $CH_2CN$ | $OCH_3$ | $OCH_3$ | CH | S | |
| H | $CH_2CN$ | $OCH_3$ | $OCH_3$ | N | S | |
| H | $CH_2CN$ | $OCH_3$ | Cl | CH | S | |
| $CH_3$ | $CH_2CO_2CH_3$ | $CH_3$ | $OCH_3$ | CH | O | |
| H | $CH_2CO_2CH_3$ | $CH_3$ | $CH_3$ | CH | O | |
| H | $CH_2CO_2CH_3$ | $CH_3$ | $OCH_3$ | CH | O | |
| H | $CH_2CO_2CH_3$ | $CH_3$ | $OCH_3$ | N | O | |
| H | $CH_2CO_2CH_3$ | $OCH_3$ | $OCH_3$ | CH | O | |
| H | $CH_2CO_2CH_3$ | $OCH_3$ | $OCH_3$ | N | O | |
| H | $CH_2CO_2CH_3$ | $OCH_3$ | Cl | CH | O | |
| H | $CH_2CO_2CH_3$ | $CH_3$ | $CH_3$ | CH | S | |
| H | $CH_2CO_2CH_3$ | $CH_3$ | $OCH_3$ | N | S | |
| H | $CH_2CO_2CH_3$ | $CH_3$ | $OCH_3$ | CH | S | |
| H | $CH_2CO_2CH_3$ | $OCH_3$ | $OCH_3$ | N | S | |
| H | $CH_2CO_2CH_3$ | $OCH_3$ | $OCH_3$ | CH | S | |
| H | $CH_2CO_2CH_3$ | $OCH_3$ | Cl | CH | S | |
| H | $CH_2CO_2CH_3$ | $CH_3$ | $N(CH_3)_2$ | N | S | |
| H | $CH_2CO_2C_2H_5$ | $CH_3$ | $CH_3$ | CH | O | |
| H | $CH_2CO_2C_2H_5$ | $CH_3$ | $OCH_3$ | N | O | |
| H | $CH_2CO_2C_2H_5$ | $CH_3$ | $OCH_3$ | CH | O | |

Table 6a (continued)

| R | $R'_3$ | X | Y | Z | W' | m.p. °C |
|---|---|---|---|---|---|---|
| H | $CH_2CO_2C_2H_5$ | $OCH_3$ | $OCH_3$ | N | O | |
| H | $CH_2CO_2C_2H_5$ | $OCH_3$ | $OCH_3$ | CH | O | |
| H | $CH_2CO_2C_2H_5$ | $OCH_3$ | Cl | CH | O | |
| H | $CH_2CO_2C_2H_5$ | $OC_2H_5$ | Cl | CH | O | |
| $CH_3$ | $CH_2CO_2C_2H_5$ | $OCH_3$ | $OCH_3$ | CH | S | |
| H | $CH_2CO_2C_2H_5$ | $CH_3$ | $CH_3$ | CH | S | |
| H | $CH_2CO_2C_2H_5$ | $CH_3$ | $CH_3$ | N | S | |
| H | $CH_2CO_2C_2H_5$ | $CH_3$ | $OCH_3$ | CH | S | |
| H | $CH_2CO_2C_2H_5$ | $CH_3$ | $OCH_3$ | N | S | |
| H | $CH_2CO_2C_2H_5$ | $OCH_3$ | $OCH_3$ | CH | S | |
| H | $CH_2CO_2C_2H_5$ | $OCH_3$ | $OCH_3$ | N | S | |
| H | $CH_2CO_2C_2H_5$ | $OCH_3$ | Cl | CH | S | |
| $CH_3$ | $CH_2OCH_3$ | $OCH_3$ | $OCH_3$ | CH | O | |
| H | $CH_2OCH_3$ | $CH_3$ | $CH_3$ | CH | O | |
| H | $CH_2OCH_3$ | $CH_3$ | $CH_3$ | N | O | |
| H | $CH_2OCH_3$ | $CH_3$ | $OCH_3$ | CH | O | |
| H | $CH_2OCH_3$ | $CH_3$ | $OCH_3$ | N | O | |
| H | $CH_2OCH_3$ | $OCH_3$ | $OCH_3$ | CH | O | |
| H | $CH_2OCH_3$ | $OCH_3$ | $OCH_3$ | N | O | |
| H | $CH_2OCH_3$ | $OCH_3$ | Cl | CH | O | |
| H | $CH_2OCH_3$ | $CH_3$ | $CH_3$ | CH | S | |
| H | $CH_2OCH_3$ | $CH_3$ | $OCH_3$ | N | S | |
| H | $CH_2OCH_3$ | $CH_3$ | $OCH_3$ | CH | S | |
| H | $CH_2OCH_3$ | $OCH_3$ | $OCH_3$ | N | S | |
| H | $CH_2OCH_3$ | $OCH_3$ | $OCH_3$ | CH | S | |
| H | $CH_2OCH_3$ | $OCH_3$ | Cl | CH | S | |
| H | $CH_2OCH_3$ | $CH_3$ | $CH(OCH_3)_2$ | CH | S | |
| H | $CH_2OC_2H_5$ | $CH_3$ | $CH_3$ | CH | O | |
| H | $CH_2OC_2H_5$ | $CH_3$ | $OCH_3$ | N | O | |
| H | $CH_2OC_2H_5$ | $CH_3$ | $OCH_3$ | CH | O | |
| H | $CH_2OC_2H_5$ | $OCH_3$ | $OCH_3$ | N | O | |

Table 6a (continued)

| R | R'3 | X | Y | Z | W' | m.p. °C |
|---|---|---|---|---|---|---|
| H | $CH_2OC_2H_5$ | $OCH_3$ | $OCH_3$ | CH | O | |
| H | $CH_2OC_2H_5$ | $OCH_3$ | Cl | CH | O | |
| H | $CH_2OC_2H_5$ | $OCH_3$ | Br | CH | O | |
| $CH_3$ | $CH_2OC_2H_5$ | $CH_3$ | $OCH_3$ | CH | S | |
| H | $CH_2OC_2H_5$ | $CH_3$ | $CH_3$ | CH | S | |
| H | $CH_2OC_2H_5$ | $CH_3$ | $CH_3$ | N | S | |
| H | $CH_2OC_2H_5$ | $CH_3$ | $OCH_3$ | CH | S | |
| H | $CH_2OC_2H_5$ | $CH_3$ | $OCH_3$ | N | S | |
| H | $CH_2OC_2H_5$ | $OCH_3$ | $OCH_3$ | CH | S | |
| H | $CH_2OC_2H_5$ | $OCH_3$ | $OCH_3$ | N | S | |
| H | $CH_2OC_2H_5$ | $OCH_3$ | Cl | CH | S | |
| $CH_3$ | $(CH_2)_2COCH_3$ | $OCH_3$ | $OCH_3$ | CH | O | |
| H | $(CH_2)_2COCH_3$ | $CH_3$ | $CH_3$ | CH | O | |
| H | $(CH_2)_2COCH_3$ | $CH_3$ | $CH_3$ | N | O | |
| H | $(CH_2)_2COCH_3$ | $CH_3$ | $OCH_3$ | CH | O | |
| H | $(CH_2)_2COCH_3$ | $CH_3$ | $OCH_3$ | N | O | |
| H | $(CH_2)_2COCH_3$ | $OCH_3$ | $OCH_3$ | CH | O | |
| H | $(CH_2)_2COCH_3$ | $OCH_3$ | $OCH_3$ | N | O | |
| H | $(CH_2)_2COCH_3$ | $OCH_3$ | Cl | CH | O | |
| H | $(CH_2)_2COCH_3$ | $CH_3$ | $CH_3$ | CH | S | |
| H | $(CH_2)_2COCH_3$ | $CH_3$ | $OCH_3$ | N | S | |
| H | $(CH_2)_2COCH_3$ | $CH_3$ | $OCH_3$ | CH | S | |
| H | $(CH_2)_2COCH_3$ | $OCH_3$ | $OCH_3$ | N | S | |
| H | $(CH_2)_2COCH_3$ | $OCH_3$ | $OCH_3$ | CH | S | |
| H | $(CH_2)_2COCH_3$ | $OCH_3$ | Cl | CH | S | |
| H | $(CH_2)_2COCH_3$ | $CH_3$ | 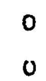 | CH | S | |
| H | $(CH_2)_2CN$ | $CH_3$ | $OCH_3$ | CH | O | |
| H | $(CH_2)_2CN$ | $CH_3$ | $OCH_3$ | N | O | |
| H | $(CH_2)_2CN$ | $OCH_3$ | $OCH_3$ | CH | O | |
| H | $(CH_2)_2CN$ | $OCH_3$ | $OCH_3$ | N | O | |

Table 6a (continued)

| R | $R'_3$ | X | Y | Z | W' | m.p. °C |
|---|---|---|---|---|---|---|
| H | $(CH_2)_2CN$ | $CH_3$ | $CH_3$ | CH | S | |
| H | $(CH_2)_2CN$ | $CH_3$ | $OCH_3$ | N | S | |
| H | $(CH_2)_2CN$ | $OCH_3$ | $OCH_3$ | CH | S | |
| H | $(CH_2)_2CN$ | $OCH_3$ | Cl | CH | S | |
| H | $CH(CH_3)CN$ | $OCH_3$ | $OCH_3$ | CH | O | |
| H | $CH(CH_3)CN$ | $OCH_3$ | $OCH_3$ | N | O | |
| H | $CH(CH_3)CN$ | $CH_3$ | $OCH_3$ | CH | S | |
| H | $CH(CH_3)CN$ | $CH_3$ | $OCH_3$ | N | S | |
| H | $(CH_2)_3CN$ | $CH_3$ | $OCH_3$ | CH | O | |
| H | $(CH_2)_3CN$ | $CH_3$ | $OCH_3$ | N | O | |
| H | $(CH_2)_3CN$ | $OCH_3$ | $OCH_3$ | CH | O | |
| H | $(CH_2)_3CN$ | $OCH_3$ | $OCH_3$ | N | O | |
| H | $(CH_2)_3CN$ | $CH_3$ | $CH_3$ | CH | S | |
| H | $(CH_2)_3CN$ | $CH_3$ | $OCH_3$ | N | S | |
| H | $(CH_2)_3CN$ | $OCH_3$ | $OCH_3$ | $CH_3$ | S | |
| H | $(CH_2)_3CN$ | $OCH_3$ | $OCH_3$ | N | S | |
| H | $CH_2CH(CH_3)CN$ | $CH_3$ | $OCH_3$ | CH | O | |
| H | $CH_2CH(CH_3)CN$ | $CH_3$ | $OCH_3$ | N | O | |
| H | $CH_2CH(CH_3)CN$ | $OCH_3$ | $OCH_3$ | CH | S | |
| H | $CH_2CH(CH_3)CN$ | $OCH_3$ | Cl | CH | S | |
| H | $CH_2CH(CH_3)CN$ | $CH_3$ | $CH_3$ | CH | O | |
| H | $CH_2CH(CH_3)CN$ | $CH_3$ | $OCH_3$ | N | O | |
| H | $CH_2CH(CH_3)CN$ | $OCH_3$ | $OCH_3$ | CH | S | |
| H | $CH_2CH(CH_3)CN$ | $OCH_3$ | $CH_3$ | CH | S | |
| $CH_3$ | $CH_2CH(CH_3)CN$ | $OCH_3$ | $OCH_3$ | CH | S | |
| H | $CH(CN)C_2H_5$ | $CH_3$ | $OCH_3$ | N | O | |
| H | $CH(CN)C_2H_5$ | $OCH_3$ | $OCH_3$ | CH | O | |
| H | $CH(CN)C_2H_5$ | $OCH_3$ | $OCH_3$ | N | S | |
| H | $CH(CN)C_2H_5$ | $OCH_3$ | Cl | CH | S | |
| H | $C(CH_3)_2CN$ | $CH_3$ | $CH_3$ | CH | O | |
| H | $C(CH_3)_2CN$ | $OCH_3$ | $OCH_3$ | CH | O | |

Table 6a (continued)

| R | $R'_3$ | X | Y | Z | W' | m.p. °C |
|---|---|---|---|---|---|---|
| H | $C(CH_3)_2CN$ | $OCH_3$ | $OCH_3$ | N | S | |
| H | $C(CH_3)_2CN$ | $OCH_3$ | $OCH_3$ | CH | S | |
| H | $CH(CH_3)CO_2CH_3$ | $OCH_3$ | $OCH_3$ | CH | O | |
| H | $CH(CH_3)CO_2CH_3$ | $OCH_3$ | $OCH_3$ | N | O | |
| H | $CH(CH_3)CO_2CH_3$ | $CH_3$ | $OCH_3$ | CH | S | |
| H | $CH(CH_3)CO_2CH_3$ | $CH_3$ | $OCH_3$ | N | S | |
| H | $(CH_2)_3CO_2CH_3$ | $CH_3$ | $OCH_3$ | CH | O | |
| H | $(CH_2)_3CO_2CH_3$ | $CH_3$ | $OCH_3$ | N | O | |
| H | $(CH_2)_3CO_2CH_3$ | $OCH_3$ | $OCH_3$ | CH | O | |
| H | $(CH_2)_3CO_2CH_3$ | $OCH_3$ | $OCH_3$ | N | O | |
| H | $(CH_2)_3CO_2CH_3$ | $CH_3$ | $OCH_3$ | CH | S | |
| H | $(CH_2)_3CO_2CH_3$ | $CH_3$ | $OCH_3$ | N | S | |
| H | $(CH_2)_3CO_2CH_3$ | $OCH_3$ | $OCH_3$ | CH | S | |
| H | $(CH_2)_3CO_2CH_3$ | $OCH_3$ | $OCH_3$ | N | S | |
| H | $CH_2CH(CH_3)CO_2CH_3$ | $CH_3$ | $OCH_3$ | CH | O | |
| H | $CH_2CH(CH_3)CO_2CH_3$ | $CH_3$ | $OCH_3$ | N | O | |
| H | $CH_2CH(CH_3)CO_2CH_3$ | $OCH_3$ | $OCH_3$ | CH | S | |
| H | $CH_2CH(CH_3)CO_2CH_3$ | $OCH_3$ | $OCH_3$ | N | S | |
| H | $CH_2CH(CH_3)CO_2CH_3$ | $CH_3$ | $OCH_3$ | CH | O | |
| H | $CH_2CH(CH_3)CO_2CH_3$ | $CH_3$ | $OCH_3$ | N | O | |
| H | $CH_2CH(CH_3)CO_2CH_3$ | $OCH_3$ | $OCH_3$ | CH | S | |
| H | $CH_2CH(CH_3)CO_2CH_3$ | $OCH_3$ | $OCH_3$ | N | S | |
| H | $CH(CO_2CH_3)C_2H_5$ | $CH_3$ | $OCH_3$ | N | O | |
| H | $CH(CO_2CH_3)C_2H_5$ | $OCH_3$ | $OCH_3$ | CH | O | |
| H | $CH(CO_2CH_3)C_2H_5$ | $OCH_3$ | $OCH_3$ | N | S | |
| H | $CH(CO_2CH_3)C_2H_5$ | $OCH_3$ | $OCH_3$ | CH | S | |
| H | $C(CH_3)_2CO_3CH_3$ | $CH_3$ | $OCH_3$ | N | O | |
| H | $C(CH_3)_2CO_3CH_3$ | $OCH_3$ | $OCH_3$ | CH | O | |
| H | $C(CH_3)_2CO_3CH_3$ | $CH_3$ | $OCH_3$ | N | S | |
| H | $C(CH_3)_2CO_3CH_3$ | $OCH_3$ | $OCH_3$ | CH | S | |

Table 6a (continued)

| R | $R'_3$ | X | Y | Z | W' | m.p. °C |
|---|---|---|---|---|---|---|
| H | $(CH_2)_2CO_2C_2H_5$ | $CH_3$ | $CH_3$ | CH | O | |
| H | $(CH_2)_2CO_2C_2H_5$ | $CH_3$ | $OCH_3$ | N | O | |
| H | $(CH_2)_2CO_2C_2H_5$ | $OCH_3$ | $OCH_3$ | CH | O | |
| H | $(CH_2)_2CO_2C_2H_5$ | $OCH_3$ | $OCH_3$ | N | O | |
| H | $(CH_2)_2CO_2C_2H_5$ | $OCH_3$ | $OCH_3$ | CH | S | |
| H | $(CH_2)_2CO_2C_2H_5$ | $CH_3$ | $OCH_3$ | N | S | |
| H | $(CH_2)_2CO_2C_2H_5$ | $OCH_3$ | $OCH_3$ | CH | S | |
| H | $(CH_2)_2CO_2C_2H_5$ | $OCH_3$ | $OCH_3$ | N | S | |
| H | $CH(CH_3)CO_2C_2H_5$ | $OCH_3$ | $OCH_3$ | CH | O | |
| H | $CH(CH_3)CO_2C_2H_5$ | $OCH_3$ | $OCH_3$ | N | O | |
| H | $CH(CH_3)CO_2C_2H_5$ | $CH_3$ | $OCH_3$ | CH | S | |
| H | $CH(CH_3)CO_2C_2H_5$ | $CH_3$ | $OCH_3$ | N | S | |
| H | $(CH_2)_3CO_2C_2H_5$ | $CH_3$ | $OCH_3$ | CH | O | |
| H | $(CH_2)_3CO_2C_2H_5$ | $CH_3$ | $OCH_3$ | N | O | |
| H | $(CH_2)_3CO_2C_2H_5$ | $OCH_3$ | $OCH_3$ | CH | O | |
| H | $(CH_2)_3CO_2C_2H_5$ | $OCH_3$ | $OCH_3$ | N | O | |
| H | $(CH_2)_3CO_2C_2H_5$ | $CH_3$ | $OCH_3$ | CH | S | |
| H | $(CH_2)_3CO_2C_2H_5$ | $CH_3$ | $OCH_3$ | N | S | |
| H | $(CH_2)_3CO_2C_2H_5$ | $OCH_3$ | $OCH_3$ | CH | S | |
| H | $(CH_2)_3CO_2C_2H_5$ | $OCH_3$ | $OCH_3$ | N | S | |
| H | $CH_2CH(CH_3)CO_2C_2H_5$ | $CH_3$ | $OCH_3$ | CH | O | |
| H | $CH_2CH(CH_3)CO_2C_2H_5$ | $CH_3$ | $OCH_3$ | N | O | |
| H | $CH_2CH(CH_3)CO_2C_2H_5$ | $OCH_3$ | $OCH_3$ | CH | S | |
| H | $CH_2CH(CH_3)CO_2C_2H_5$ | $OCH_3$ | $OCH_3$ | N | S | |
| H | $CH_2CH(CH_3)CO_2C_2H_5$ | $CH_3$ | $OCH_3$ | CH | O | |
| H | $CH_2CH(CH_3)CO_2C_2H_5$ | $CH_3$ | $OCH_3$ | N | O | |
| H | $CH_2CH(CH_3)CO_2C_2H_5$ | $CH_3$ | $OCH_3$ | CH | S | |
| H | $CH_2CH(CH_3)CO_2C_2H_5$ | $OCH_3$ | $OCH_3$ | N | S | |
| H | $CH(CO_2C_2H_5)C_2H_5$ | $CH_3$ | $OCH_3$ | N | O | |
| H | $CH(CO_2C_2H_5)C_2H_5$ | $OCH_3$ | $OCH_3$ | CH | O | |
| H | $CH(CO_2C_2H_5)C_2H_5$ | $OCH_3$ | $OCH_3$ | N | S | |

Table 6a (continued)

| R | $R'_3$ | X | Y | Z | W' | m.p. °C |
|---|---|---|---|---|---|---|
| H | $CH(CO_2C_2H_5)C_2H_5$ | $OCH_3$ | $OCH_3$ | CH | S | |
| H | $C(CH_3)_2CO_2C_2H_5$ | $CH_3$ | $OCH_3$ | N | O | |
| H | $C(CH_3)_2CO_2C_2H_5$ | $OCH_3$ | $OCH_3$ | CH | O | |
| H | $C(CH_3)_2CO_2C_2H_5$ | $CH_3$ | $OCH_3$ | N | O | |
| H | $C(CH_3)_2CO_2C_2H_5$ | $OCH_3$ | $OCH_3$ | CH | S | |
| H | $(CH_2)_2OCH_3$ | $CH_3$ | $CH_3$ | CH | O | |
| H | $(CH_2)_2OCH_3$ | $CH_3$ | $OCH_3$ | N | O | |
| H | $(CH_2)_2OCH_3$ | $OCH_3$ | $OCH_3$ | CH | O | |
| H | $(CH_2)_2OCH_3$ | $OCH_3$ | $OCH_3$ | N | O | |
| H | $(CH_2)_2OCH_3$ | $OCH_3$ | $OCH_3$ | CH | S | |
| H | $(CH_2)_2OCH_3$ | $CH_3$ | $OCH_3$ | N | S | |
| H | $(CH_2)_2OCH_3$ | $OCH_3$ | $OCH_3$ | CH | S | |
| H | $(CH_2)_2OCH_3$ | $OCH_3$ | $OCH_3$ | N | S | |
| H | $CH(CH_3)OCH_3$ | $OCH_3$ | $OCH_3$ | CH | O | |
| H | $CH(CH_3)OCH_3$ | $OCH_3$ | $OCH_3$ | N | O | |
| H | $CH(CH_3)OCH_3$ | $CH_3$ | $OCH_3$ | CH | S | |
| H | $CH(CH_3)OCH_3$ | $CH_3$ | $OCH_3$ | N | S | |
| H | $(CH_2)_3OCH_3$ | $CH_3$ | $OCH_3$ | CH | O | |
| H | $(CH_2)_3OCH_3$ | $CH_3$ | $OCH_3$ | N | O | |
| H | $(CH_2)_3OCH_3$ | $OCH_3$ | $OCH_3$ | CH | O | |
| H | $(CH_2)_3OCH_3$ | $OCH_3$ | $OCH_3$ | N | O | |
| H | $(CH_2)_3OCH_3$ | $CH_3$ | $OCH_3$ | CH | S | |
| H | $(CH_2)_3OCH_3$ | $CH_3$ | $OCH_3$ | N | S | |
| H | $(CH_2)_3OCH_3$ | $OCH_3$ | $OCH_3$ | CH | S | |
| H | $(CH_2)_3OCH_3$ | $OCH_3$ | $OCH_3$ | N | S | |
| H | $CH_2CH(CH_3)OCH_3$ | $CH_3$ | $OCH_3$ | CH | O | |
| H | $CH_2CH(CH_3)OCH_3$ | $CH_3$ | $OCH_3$ | N | O | |
| H | $CH_2CH(CH_3)OCH_3$ | $OCH_3$ | $OCH_3$ | CH | S | |
| H | $CH_2CH(CH_3)OCH_3$ | $OCH_3$ | $OCH_3$ | N | S | |
| H | $CH_2CH(CH_3)OCH_3$ | $CH_3$ | $OCH_3$ | CH | O | |
| H | $CH_2CH(CH_3)OCH_3$ | $CH_3$ | $OCH_3$ | N | O | |

**Table 6a (continued)**

| R | $R'_3$ | X | Y | Z | W' | m.p. °C |
|---|---|---|---|---|---|---|
| H | $CH_2CH(CH_3)OCH_3$ | $CH_3$ | $OCH_3$ | CH | S | |
| H | $CH_2CH(CH_3)OCH_3$ | $OCH_3$ | $OCH_3$ | N | S | |
| H | $CH(OCH_3)C_2H_5$ | $CH_3$ | $OCH_3$ | N | O | |
| H | $CH(OCH_3)C_2H_5$ | $OCH_3$ | $OCH_3$ | CH | O | |
| H | $CH(OCH_3)C_2H_5$ | $OCH_3$ | $OCH_3$ | N | S | |
| H | $CH(OCH_3)C_2H_5$ | $OCH_3$ | $OCH_3$ | CH | S | |
| H | $C(CH_3)_2OCH_3$ | $CH_3$ | $OCH_3$ | N | O | |
| H | $C(CH_3)_2OCH_3$ | $OCH_3$ | $OCH_3$ | CH | O | |
| H | $C(CH_3)_2OCH_3$ | $CH_3$ | $OCH_3$ | N | O | |
| H | $C(CH_3)_2OCH_3$ | $OCH_3$ | $OCH_3$ | CH | S | |
| H | $(CH_2)_2OC_2H_5$ | $CH_3$ | $CH_3$ | CH | O | |
| H | $(CH_2)_2OC_2H_5$ | $CH_3$ | $OCH_3$ | N | O | |
| H | $(CH_2)_2OC_2H_5$ | $OCH_3$ | $OCH_3$ | CH | O | |
| H | $(CH_2)_2OC_2H_5$ | $OCH_3$ | $OCH_3$ | N | O | |
| H | $(CH_2)_2OC_2H_5$ | $OCH_3$ | $OCH_3$ | CH | S | |
| H | $(CH_2)_2OC_2H_5$ | $CH_3$ | $OCH_3$ | N | S | |
| H | $(CH_2)_2OC_2H_5$ | $OCH_3$ | $OCH_3$ | CH | S | |
| H | $(CH_2)_2OC_2H_5$ | $OCH_3$ | $OCH_3$ | N | S | |
| H | $CH(CH_3)OC_2H_5$ | $OCH_3$ | $OCH_3$ | CH | O | |
| H | $CH(CH_3)OC_2H_5$ | $OCH_3$ | $OCH_3$ | N | O | |
| H | $CH(CH_3)OC_2H_5$ | $CH_3$ | $OCH_3$ | CH | S | |
| H | $CH(CH_3)OC_2H_5$ | $CH_3$ | $OCH_3$ | N | S | |
| H | $(CH_2)_3OC_2H_5$ | $CH_3$ | $OCH_3$ | CH | O | |
| H | $(CH_2)_3OC_2H_5$ | $CH_3$ | $OCH_3$ | N | O | |
| H | $(CH_2)_3OC_2H_5$ | $OCH_3$ | $OCH_3$ | CH | O | |
| H | $(CH_2)_3OC_2H_5$ | $OCH_3$ | $OCH_3$ | N | O | |
| H | $(CH_2)_3OC_2H_5$ | $CH_3$ | $OCH_3$ | CH | S | |
| H | $(CH_2)_3OC_2H_5$ | $CH_3$ | $OCH_3$ | N | S | |
| H | $(CH_2)_3OC_2H_5$ | $OCH_3$ | $OCH_3$ | CH | S | |
| H | $(CH_2)_3OC_2H_5$ | $OCH_3$ | $OCH_3$ | N | S | |
| H | $CH_2CH(CH_3)OC_2H_5$ | $CH_3$ | $OCH_3$ | CH | O | |

Table 6a (continued)

| R | $R'_3$ | X | Y | Z | W' | m.p. °C |
|---|---|---|---|---|---|---|
| H | $CH_2CH(CH_3)OC_2H_5$ | $CH_3$ | $OCH_3$ | N | O | |
| H | $CH_2CH(CH_3)OC_2H_5$ | $OCH_3$ | $OCH_3$ | CH | S | |
| H | $CH_2CH(CH_3)OC_2H_5$ | $OCH_3$ | $OCH_3$ | N | S | |
| H | $CH(CH_3)CH_2OC_2H_5$ | $CH_3$ | $OCH_3$ | CH | O | |
| H | $CH(CH_3)CH_2OC_2H_5$ | $CH_3$ | $OCH_3$ | N | O | |
| H | $CH(CH_3)CH_2OC_2H_5$ | $CH_3$ | $OCH_3$ | CH | S | |
| H | $CH(CH_3)CH_2OC_2H_5$ | $OCH_3$ | $OCH_3$ | N | S | |
| H | $CH(OC_2H_5)C_2H_5$ | $CH_3$ | $OCH_3$ | N | O | |
| H | $CH(OC_2H_5)C_2H_5$ | $OCH_3$ | $OCH_3$ | CH | O | |
| H | $CH(OC_2H_5)C_2H_5$ | $OCH_3$ | $OCH_3$ | N | S | |
| H | $CH(OC_2H_5)C_2H_5$ | $OCH_3$ | $OCH_3$ | CH | S | |
| H | $CH(CH_3)_2OC_2H_5$ | $CH_3$ | $OCH_3$ | N | O | |
| H | $CH(CH_3)_2OC_2H_5$ | $OCH_3$ | $OCH_3$ | CH | O | |
| H | $CH(CH_3)_2OC_2H_5$ | $CH_3$ | $OCH_3$ | N | O | |
| H | $C(CH_3)_2OC_2H_5$ | $OCH_3$ | $OCH_3$ | CH | S | |
| H | $(CH_2)CH_2COCH_3$ | $CH_3$ | $CH_3$ | CH | O | |
| H | $(CH_2)CH_2COCH_3$ | $CH_3$ | $OCH_3$ | N | O | |
| H | $(CH_2)CH_2COCH_3$ | $OCH_3$ | $OCH_3$ | CH | O | |
| H | $(CH_2)CH_2COCH_3$ | $OCH_3$ | $OCH_3$ | N | O | |
| H | $(CH_2)CH_2COCH_3$ | $OCH_3$ | $OCH_3$ | CH | S | |
| H | $(CH_2)CH_2COCH_3$ | $CH_3$ | $OCH_3$ | N | S | |
| H | $(CH_2)CH_2COCH_3$ | $OCH_3$ | $OCH_3$ | CH | S | |
| H | $(CH_2)CH_2COCH_3$ | $OCH_3$ | $OCH_3$ | N | S | |
| H | $CH(CH_3)CH_2COCH_3$ | $OCH_3$ | $OCH_3$ | CH | O | |
| H | $CH(CH_3)CH_2COCH_3$ | $OCH_3$ | $OCH_3$ | N | O | |
| H | $CH(CH_3)CH_2COCH_3$ | $CH_3$ | $OCH_3$ | CH | S | |
| H | $CH(CH_3)CH_2COCH_3$ | $CH_3$ | $OCH_3$ | N | S | |
| H | $(CH_2)_3CH_2COCH_3$ | $CH_3$ | $OCH_3$ | CH | O | |
| H | $(CH_2)_3CH_2COCH_3$ | $CH_3$ | $OCH_3$ | N | O | |
| H | $(CH_2)_3CH_2COCH_3$ | $OCH_3$ | $OCH_3$ | CH | O | |
| H | $(CH_2)_3CH_2COCH_3$ | $OCH_3$ | $OCH_3$ | N | O | |

Table 6a (continued)

| R | R'3 | X | Y | Z | W' | m.p. °C |
|---|---|---|---|---|---|---|
| H | $(CH_2)_3CH_2COCH_3$ | $CH_3$ | $OCH_3$ | CH | S | |
| H | $(CH_2)_3CH_2COCH_3$ | $CH_3$ | $OCH_3$ | N | S | |
| H | $(CH_2)_3CH_2COCH_3$ | $OCH_3$ | $OCH_3$ | CH | S | |
| H | $(CH_2)_3CH_2COCH_3$ | $OCH_3$ | $OCH_3$ | N | S | |
| H | $CH_2CH(CH_3)CH_2COCH_3$ | $CH_3$ | $OCH_3$ | CH | O | |
| H | $CH_2CH(CH_3)CH_2COCH_3$ | $CH_3$ | $OCH_3$ | N | O | |
| H | $CH_2CH(CH_3)CH_2COCH_3$ | $OCH_3$ | $OCH_3$ | CH | S | |
| H | $CH_2CH(CH_3)CH_2COCH_3$ | $OCH_3$ | $OCH_3$ | N | S | |
| H | $CH(CH_3)CH_2CH_2COCH_3$ | $CH_3$ | $OCH_3$ | CH | O | |
| H | $CH(CH_3)CH_2CH_2COCH_3$ | $CH_3$ | $OCH_3$ | N | O | |
| H | $CH(CH_3)CH_2CH_2COCH_3$ | $CH_3$ | $OCH_3$ | CH | S | |
| H | $CH(CH_3)CH_2CH_2COCH_3$ | $OCH_3$ | $OCH_3$ | N | S | |
| H | $CH(C_2H_5)CH_2COCH_3$ | $CH_3$ | $OCH_3$ | N | O | |
| H | $CH(C_2H_5)CH_2OCH_3$ | $OCH_3$ | $OCH_3$ | CH | O | |
| H | $CH(C_2H_5)CH_2OCH_3$ | $OCH_3$ | $OCH_3$ | N | S | |
| H | $CH(C_2H_5)CH_2OCH_3$ | $OCH_3$ | $OCH_3$ | CH | S | |
| H | $CH(CH_3)_2CH_2COCH_3$ | $CH_3$ | $OCH_3$ | N | O | |
| H | $CH(CH_3)_2CH_2COCH_3$ | $OCH_3$ | $OCH_3$ | CH | O | |
| H | $CH(CH_3)_2CH_2COCH_3$ | $CH_3$ | $OCH_3$ | N | O | |
| H | $C(CH_3)_2CH_2COCH_3$ | $OCH_3$ | $OCH_3$ | CH | S | |
| H | H | $OCH_3$ | $OCH_3$ | CH | O * | |
| H | H | $OCH_3$ | $OCH_3$ | CH | S * | |
| H | $CH_3$ | $OCH_3$ | $OCH_3$ | CH | O * | |
| H | $CH_3$ | $OCH_3$ | $OCH_3$ | CH | S * | |
| H | $SCH_3$ | $OCH_3$ | $OCH_3$ | CH | O * | |
| H | $SCH_3$ | $OCH_3$ | $OCH_3$ | CH | S * | |
| H | $SCH_2CH=CH_2$ | $OCH_3$ | $OCH_3$ | CH | O * | |
| H | $SCH_3CH=CH_2$ | $OCH_3$ | $OCH_3$ | CH | S * | |
| H | SH | $OCH_3$ | $OCH_3$ | CH | O * | |
| H | SH | $OCH_3$ | $OCH_3$ | CH | S * | |

W is O, unless indicated by * where W is S in all Tables.

**Table 6b**

**General Structure 2 wherein Q is Q-28.**

| R | $R_3'$ | $R_4$ | X | Y | Z | m.p. °C |
|---|---|---|---|---|---|---|
| H | H | H | $CH_3$ | $OCH_3$ | CH | 216-218 |
| H | H | H | $CH_3$ | $OCH_3$ | N | 197-200 |
| H | H | H | $OCH_3$ | $OCH_3$ | CH | 180-186 |
| H | H | H | $OCH_3$ | $OCH_3$ | N | 196-198 |
| H | H | H | $CH_3$ | $CH_3$ | CH | 242-244 |
| H | H | H | $CH_3$ | $CH_3$ | N | |
| H | $CH_3$ | H | $CH_3$ | $OCH_3$ | CH | |
| H | H | H | Cl | $OCH_3$ | CH | 206-208 |
| H | H | $CH_3$ | $CH_3$ | $OCH_3$ | N | |
| H | $CH_3$ | $CH_3$ | $OCH_3$ | $OCH_3$ | CH | |
| $CH_3$ | $CH_3$ | $CH_3$ | $OCH_3$ | $OCH_3$ | N | |
| H | H | H | $CH_3$ | 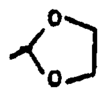 | CH | |
| H | H | H | $CH_3$ | $C_2H_5$ | CH | |
| H | $CH_3$ | H | $CH_3$ | $CH_3$ | CH | |
| H | $CH_3$ | H | $CH_3$ | $OCH_3$ | N | |
| H | $CH_3$ | H | $OCH_3$ | $OCH_3$ | CH | |
| H | $CH_3$ | H | $OCH_3$ | $OCH_3$ | N | |
| H | $CH_3$ | H | $OCH_3$ | Cl | CH | |
| H | $CH_3$ | H | $CH_3$ | $CH_3$ | N | |
| H | Cl | H | $CH_3$ | $CH_3$ | CH | |
| H | Cl | H | $CH_3$ | $CH_3$ | N | |
| H | Cl | H | $CH_3$ | $OCH_3$ | CH | |
| H | Cl | H | $CH_3$ | $OCH_3$ | N | |
| H | Cl | H | $OCH_3$ | $OCH_3$ | CH | |
| H | Cl | H | $OCH_3$ | $OCH_3$ | N | |
| H | Cl | H | $OCH_3$ | Cl | CH | |
| H | Cl | $CH_3$ | $CH_3$ | $OCH_3$ | CH | |
| H | Cl | $CH_3$ | $CH_3$ | $OCH_3$ | N | |
| H | Cl | $CH_3$ | $OCH_3$ | $OCH_3$ | CH | |

Table 6b (continued)

| R | $R_3'$ | $R_4$ | X | Y | Z | m.p. °C |
|---|---|---|---|---|---|---|
| H | Cl | $CH_3$ | $OCH_3$ | $OCH_3$ | N | |
| H | Br | H | $OCH_3$ | $OCH_3$ | CH | |
| H | Br | H | $CH_3$ | $CH_3$ | CH | |
| H | Br | H | $CH_3$ | $OCH_3$ | N | |
| H | Br | H | $CH_3$ | $OCH_3$ | CH | |
| H | Br | H | $OCH_3$ | $OCH_3$ | N | |
| H | Br | H | $OCH_3$ | $OCH_3$ | CH | |
| H | Br | H | $OCH_3$ | Cl | CH | |
| H | H | H | $OCH_3$ | $OCH_3$ | CH * | |
| H | $CH_3$ | H | $OCH_3$ | $OCH_3$ | CH * | |
| H | H | $CH_3$ | $OCH_3$ | $OCH_3$ | CH * | |
| H | $CH_3$ | $CH_3$ | $OCH_3$ | $OCH_3$ | CH * | |
| H | Cl | H | $OCH_3$ | $OCH_3$ | CH * | |
| H | Cl | $CH_3$ | $OCH_3$ | $OCH_3$ | CH * | |
| H | Br | H | $OCH_3$ | $OCH_3$ | CH * | |
| H | Br | $CH_3$ | $OCH_3$ | $OCH_3$ | CH * | |

*Wherein W is O, unless indicated by * where W is S in all Tables.

**Table 6c**

**General Structure 2**

| Q | R | $R_3$ | $R_4$ | X | Y | Z | n' | m.p. °C |
|---|---|---|---|---|---|---|---|---|
| Q-33 | H | H | H | $CH_3$ | $OCH_3$ | CH | 0 | 168-173 |
| Q-33 | H | H | H | $CH_3$ | $OCH_3$ | N | 0 | 189-190 |
| Q-33 | H | H | H | $OCH_3$ | $OCH_3$ | CH | 0 | 195-196 |
| Q-33 | H | H | H | $OCH_3$ | $OCH_3$ | N | 0 | 183-185 |
| Q-33 | H | H | H | $CH_3$ | $CH_3$ | CH | 0 | 164-167 |
| Q-33 | H | 3-$CH_3$ | H | $CH_3$ | $OCH_3$ | CH | 0 | |
| Q-33 | H | 4-$CH_3$ | H | $CH_3$ | $OCH_3$ | CH | 0 | |
| Q-33 | H | 5-$CH_3$ | H | $CH_3$ | $OCH_3$ | CH | 0 | |
| Q-33 | H | H | H | $CH_3$ | $CH_3$ | N | 0 | |
| Q-33 | H | 6-$CH_3$ | H | $CH_3$ | $OCH_3$ | CH | 0 | |
| Q-33 | H | 3-$CH_3$ | 5-$CH_3$ | $CH_3$ | $OCH_3$ | N | 0 | |
| Q-33 | H | 4-$CH_3$ | 6-$CH_3$ | $CH_3$ | $OCH_3$ | N | 0 | |
| Q-33 | $CH_3$ | H | H | $OCH_3$ | $OCH_3$ | CH | 0 | |
| Q-33 | H | H | H | $CH_3$ | $CH_2OCH_3$ | CH | 0 | |
| Q-33 | H | H | H | Cl | $OCH_3$ | CH | 0 | 183-184 |
| Q-33 | H | H | H | $CH_3$ | $CF_3$ | CH | 0 | |
| Q-33 | H | H | H | Cl | $OCH_3$ | CH | 1 | 184-187 |
| Q-33 | H | H | H | $CH_3$ | $OCH_3$ | CH | 1 | 185-187 |
| Q-33 | H | H | H | $CH_3$ | $OCH_3$ | N | 1 | 200 |
| Q-33 | H | H | H | $OCH_3$ | $OCH_3$ | CH | 1 | 191-192 |
| Q-33 | H | H | H | $OCH_3$ | $OCH_3$ | N | 1 | 190 |
| Q-33 | H | H | H | $CH_3$ | $CH_3$ | CH | 1 | 171-174 |
| Q-33 | H | 3-$CH_3$ | H | $CH_3$ | $OCH_3$ | CH | 1 | |
| Q-33 | H | 4-$CH_3$ | H | $CH_3$ | $OCH_3$ | CH | 1 | |
| Q-33 | H | 5-$CH_3$ | H | $CH_3$ | $OCH_3$ | CH | 1 | |
| Q-33 | H | H | H | $CH_3$ | $CH_3$ | N | 1 | |
| Q-33 | H | 6-$CH_3$ | H | $CH_3$ | $OCH_3$ | CH | 1 | |
| Q-33 | H | 3-$CH_3$ | 5-$CH_3$ | $CH_3$ | $OCH_3$ | N | 1 | |
| Q-33 | H | 4-$CH_3$ | 6-$CH_3$ | $CH_3$ | $OCH_3$ | N | 1 | |
| Q-33 | $CH_3$ | H | H | $OCH_3$ | $OCH_3$ | CH | 1 | |
| Q-33 | H | H | H | $CH_3$ | $CH_2OCH_3$ | CH | 1 | |

Table 6c (continued)

| Q | R | $R_3$ | $R_4$ | X | Y | Z | n' | m.p. °C |
|---|---|---|---|---|---|---|---|---|
| Q-33 | H | H | H | Cl | $OC_2H_5$ | CH | 1 | |
| Q-33 | H | H | H | $CH_3$ | $CF_3$ | CH | 1 | |
| Q-34 | H | H | H | $CH_3$ | $OCH_3$ | CH | 0 | |
| Q-34 | H | H | H | $CH_3$ | $OCH_3$ | N | 0 | |
| Q-34 | H | H | H | $OCH_3$ | $OCH_3$ | CH | 0 | |
| Q-34 | H | H | H | $OCH_3$ | $OCH_3$ | N | 0 | |
| Q-34 | H | 2-$CH_3$ | H | $CH_3$ | $CH_3$ | CH | 0 | |
| Q-34 | H | 4-$CH_3$ | H | $CH_3$ | $CH_3$ | N | 0 | |
| Q-34 | H | 5-$CH_3$ | H | $CH_3$ | $OCH_3$ | CH | 0 | |
| Q-34 | H | 6-$CH_3$ | H | $CH_3$ | $OCH_3$ | N | 0 | |
| Q-34 | H | 2-$CH_3$ | 6-$CH_3$ | $OCH_3$ | $OCH_3$ | CH | 0 | |
| Q-34 | H | 4-$CH_3$ | 6-$CH_3$ | $OCH_3$ | $OCH_3$ | N | 0 | |
| Q-34 | H | H | H | $CH_3$ | $CH_3$ | N | 0 | |
| Q-34 | $CH_3$ | H | H | $CH_3$ | $CH_3$ | CH | 0 | |
| Q-34 | H | H | H | $CH_3$ | $CH_3$ | CH | 0 | |
| Q-34 | H | H | H | $CH_3$ | $OCF_2H$ | CH | 0 | |
| Q-34 | H | H | H | $CH_3$ | $OCH_3$ | CH | 1 | |
| Q-34 | H | H | H | $CH_3$ | $OCH_3$ | N | 1 | |
| Q-34 | H | H | H | $OCH_3$ | $OCH_3$ | CH | 1 | |
| Q-34 | H | H | H | $OCH_3$ | $OCH_3$ | N | 1 | |
| Q-34 | H | 2-$CH_3$ | H | $CH_3$ | $CH_3$ | CH | 1 | |
| Q-34 | H | 4-$CH_3$ | H | $CH_3$ | $CH_3$ | N | 1 | |
| Q-34 | H | 5-$CH_3$ | H | $CH_3$ | $OCH_3$ | CH | 1 | |
| Q-34 | H | 6-$CH_3$ | H | $CH_3$ | $OCH_3$ | N | 1 | |
| Q-34 | H | 2-$CH_3$ | 6-$CH_3$ | $OCH_3$ | $OCH_3$ | CH | 1 | |
| Q-34 | H | 4-$CH_3$ | 6-$CH_3$ | $OCH_3$ | $OCH_3$ | N | 1 | |
| Q-34 | H | H | H | $CH_3$ | $CH_3$ | N | 1 | |
| Q-34 | $CH_3$ | H | H | $CH_3$ | $CH_3$ | CH | 1 | |
| Q-34 | H | H | H | $CH_3$ | $CH_3$ | CH | 1 | |
| Q-34 | H | H | H | $CH_3$ | $OCF_2H$ | CH | 1 | |
| Q-35 | H | H | H | $CH_3$ | $OCH_3$ | CH | 0 | |

Table 6c (continued)

| Q | R | $R_3$ | $R_4$ | X | Y | Z | n' | m.p. °C |
|---|---|---|---|---|---|---|---|---|
| Q-35 | H | H | H | $CH_3$ | $OCH_3$ | N | 0 | |
| Q-35 | H | H | H | $OCH_3$ | $OCH_3$ | CH | 0 | |
| Q-35 | H | H | H | $OCH_3$ | $OCH_3$ | N | 0 | |
| Q-35 | H | 2-$CH_3$ | H | $CH_3$ | $CH_3$ | CH | 0 | |
| Q-35 | H | H | H | Cl | $OCH_3$ | CH | 0 | |
| Q-35 | H | 3-$CH_3$ | H | $CH_3$ | $CH_3$ | N | 0 | |
| Q-35 | H | 2-$CH_3$ | 6-$CH_3$ | $CH_3$ | $OCH_3$ | CH | 0 | |
| Q-35 | H | 2-$CH_3$ | 3-$CH_3$ | $CH_3$ | $OCH_3$ | N | 0 | |
| Q-35 | H | 3-$CH_3$ | 5-$CH_3$ | $CH_3$ | $OCH_3$ | N | 0 | |
| Q-35 | H | H | H | $CH_3$ | $CH_3$ | N | 0 | |
| Q-35 | $CH_3$ | H | H | $OCH_3$ | $OCH_3$ | CH | 0 | |
| Q-35 | H | H | H | $CH_3$ | $OCH_2CF_3$ | CH | 0 | |
| Q-35 | H | H | H | $CH_3$ | $CH_3$ | CH | 0 | |
| Q-35 | H | H | H | $CH_3$ | $OCH_3$ | CH | 1 | |
| Q-35 | H | H | H | $CH_3$ | $OCH_3$ | N | 1 | |
| Q-35 | H | H | H | $OCH_3$ | $OCH_3$ | CH | 1 | |
| Q-35 | H | H | H | $OCH_3$ | $OCH_3$ | N | 1 | |
| Q-35 | H | 2-$CH_3$ | H | $CH_3$ | $CH_3$ | CH | 1 | |
| Q-35 | H | H | H | Cl | $OCH_3$ | CH | 1 | |
| Q-35 | H | 3-$CH_3$ | H | $CH_3$ | $CH_3$ | N | 1 | |
| Q-35 | H | 2-$CH_3$ | 6-$CH_3$ | $CH_3$ | $OCH_3$ | CH | 1 | |
| Q-35 | H | 2-$CH_3$ | 3-$CH_3$ | $CH_3$ | $OCH_3$ | N | 1 | |
| Q-35 | H | 3-$CH_3$ | 5-$CH_3$ | $CH_3$ | $OCH_3$ | N | 1 | |
| Q-35 | H | H | H | $CH_3$ | $CH_3$ | N | 1 | |
| Q-35 | $CH_3$ | H | H | $OCH_3$ | $OCH_3$ | CH | 1 | |
| Q-35 | H | H | H | $CH_3$ | $OCH_2CF_3$ | CH | 1 | |
| Q-35 | H | H | H | $CH_3$ | $CH_3$ | CH | 1 | |

Table 7a

General Structure 3

| Q | R | $R_3$ | $R_4$ | $R_5$ | X | Y | Z | W' | m.p. °C |
|---|---|---|---|---|---|---|---|---|---|
| Q-2 | H | H | H | H | $CH_3$ | $OCH_3$ | CH | O | |
| Q-2 | H | H | H | H | $CH_3$ | $OCH_3$ | N | O | |
| Q-2 | H | H | H | H | $OCH_3$ | $OCH_3$ | CH | O | |
| Q-2 | H | H | H | H | $OCH_3$ | $OCH_3$ | N | O | |
| Q-2 | H | $CH_3$ | H | H | $CH_3$ | $CH_3$ | CH | O | |
| Q-2 | H | H | $CH_3$ | H | $CH_3$ | $OCH_3$ | N | O | |
| Q-2 | H | H | H | $CH_3$ | $CH_3$ | $OCH_3$ | CH | O | |
| Q-2 | $CH_3$ | H | H | H | CH | $CH_3$ | CH | O | |
| Q-2 | H | $CH_3$ | $CH_3$ | H | $OCH_3$ | $OCH_3$ | N | O | |
| Q-2 | H | H | H | H | Cl | $OCH_3$ | CH | S | |
| Q-2 | H | H | H | H | $CH_3$ | $OCH_3$ | CH | S | |
| Q-2 | H | H | H | H | $CH_3$ | $OCH_3$ | N | S | |
| Q-2 | H | H | H | H | $OCH_3$ | $OCH_3$ | CH | S | |
| Q-2 | H | H | H | H | $OCH_3$ | $OCH_3$ | N | S | |
| Q-2 | H | H | H | H | $CH_3$ | $CH_3$ | CH | S | |
| Q-2 | H | $CH_3$ | H | H | $CH_3$ | $CH_3$ | CH | S | |
| Q-2 | H | H | $CH_3$ | H | $CH_3$ | $CH_3$ | N | S | |
| Q-2 | H | H | H | $CH_3$ | $CH_3$ | $OCH_3$ | CH | S | |
| Q-2 | H | $CH_3$ | $CH_3$ | H | $CH_3$ | $OCH_3$ | N | S | |
| Q-2 | H | H | $CH_3$ | $CH_3$ | $OCH_3$ | $OCH_3$ | CH | S | |
| Q-2 | H | $CH_3$ | H | $CH_3$ | $OCH_3$ | $OCH_3$ | N | S | |
| Q-2 | H | $CH_3$ | $CH_3$ | $CH_3$ | $CH_3$ | $OCH_3$ | CH | S | |
| Q-2 | $CH_3$ | H | H | H | $CH_3$ | $CH_3$ | CH | S | |
| Q-2 | H | H | H | H | Cl | $OC_2H_5$ | CH | S | |
| Q-3 | H | H | H | H | $CH_3$ | $CH_3$ | CH | O | |
| Q-3 | H | H | H | H | $CH_3$ | $CH_3$ | N | O | |
| Q-3 | H | H | H | H | $CH_3$ | $OCH_3$ | CH | O | |
| Q-3 | H | H | H | H | $CH_3$ | $OCH_3$ | N | O | |
| Q-3 | H | H | H | H | $OCH_3$ | $OCH_3$ | CH | O | |
| Q-3 | H | H | H | H | $OCH_3$ | $OCH_3$ | N | O | |
| Q-3 | H | $CH_3$ | H | H | $CH_3$ | $CH_3$ | CH | O | |

Table 7a (continued)

| Q | R | $R_3$ | $R_4$ | $R_5$ | X | Y | Z | W' | m.p. °C |
|---|---|---|---|---|---|---|---|---|---|
| Q-3 | H | H | $CH_3$ | H | $CH_3$ | $CH_3$ | N | O | |
| Q-3 | H | H | H | $CH_3$ | $CH_3$ | $OCH_3$ | CH | O | |
| Q-3 | H | $CH_3$ | $CH_3$ | H | $CH_3$ | $OCH_3$ | N | O | |
| Q-3 | H | H | $CH_3$ | $CH_3$ | $OCH_3$ | $OCH_3$ | CH | O | |
| Q-3 | H | $CH_3$ | H | $CH_3$ | $OCH_3$ | $OCH_3$ | N | O | |
| Q-3 | H | H | H | H | $OCH_3$ | $OCH_2CF_3$ | CH | O | |
| Q-3 | H | H | H | H | $OCH_3$ | $OCH_2CF_3$ | CH | O | |
| Q-3 | $CH_3$ | H | H | H | Cl | $OCH_3$ | CH | O | |
| Q-3 | H | H | H | H | $CH_3$ | $OCH_3$ | CH | S | |
| Q-3 | H | H | H | H | $OCH_3$ | $OCH_3$ | CH | S | |
| Q-3 | H | H | H | H | $OCH_3$ | $OCH_3$ | N | S | |
| Q-3 | H | $CH_3$ | H | H | $CH_3$ | $CH_3$ | CH | S | |
| Q-3 | H | H | $CH_3$ | H | $CH_3$ | $OCH_3$ | N | S | |
| Q-3 | H | H | H | $CH_3$ | $CH_3$ | $OCH_3$ | CH | S | |
| Q-3 | $CH_3$ | H | H | H | $CH_3$ | $CH_3$ | CH | S | |
| Q-3 | H | H | $CH_3$ | $CH_3$ | $OCH_3$ | $OCH_3$ | N | S | |
| Q-3 | H | H | H | H | $OCH_3$ | $CF_3$ | N | S | |
| Q-4 | H | H | H | H | $CH_3$ | $OCH_3$ | CH | – | |
| Q-4 | H | H | H | H | $OCH_3$ | Cl | CH | – | |
| Q-4 | H | H | H | H | $CH_3$ | $OCH_3$ | N | – | |
| Q-4 | H | H | H | H | $CH_3$ | $CH_3$ | CH | – | |
| Q-4 | H | H | H | H | $OCH_3$ | $OCH_3$ | CH | – | |
| Q-4 | H | H | H | H | $OCH_3$ | $OCH_3$ | N | – | |
| Q-4 | H | $CH_3$ | H | H | $CH_3$ | $CH_3$ | CH | – | |
| Q-4 | H | H | $CH_3$ | H | $CH_3$ | $OCH_3$ | N | – | |
| Q-4 | H | H | H | $CH_3$ | $CH_3$ | $OCH_3$ | CH | – | |
| Q-4 | $CH_3$ | H | H | H | $OCH_3$ | $OCH_3$ | CH | – | |
| Q-4 | $CH_3$ | $CH_3$ | $CH_3$ | $CH_3$ | $CH_3$ | $OCH_3$ | N | – | |
| Q-4 | H | $CH_3$ | H | $CH_3$ | $CH_3$ | $OCH_3$ | CH | – | |
| Q-4 | H | H | H | H | $OCH_3$ | $OCH_2CF_3$ | N | – | |
| Q-4 | H | H | H | H | $CH_3$ | $NHCH_3$ | CH | – | |
| Q-4 | H | H | H | H | $CH_3$ | $CH_3$ | CH | – | |
| Q-4 | H | H | H | H | $CH_3$ | $CH_3$ | N | – | |

Table 7a (continued)

| Q | R | $R_3$ | $R_4$ | $R_5$ | X | Y | Z | W' | m.p. °C |
|---|---|---|---|---|---|---|---|---|---|
| Q-9 | H | H | H | H | $CH_3$ | $OCH_3$ | CH | - | |
| Q-9 | H | H | H | H | $CH_3$ | $OCH_3$ | N | - | |
| Q-9 | H | H | H | H | $OCH_3$ | $OCH_3$ | CH | - | |
| Q-9 | H | H | H | H | $OCH_3$ | $OCH_3$ | N | - | |
| Q-9 | $CH_3$ | $CH_3$ | H | H | $CH_3$ | $CH_3$ | CH | - | |
| Q-9 | H | H | $CH_3$ | H | $CH_3$ | $OCH_3$ | N | - | |
| Q-9 | H | H | H | $CH_3$ | $OCH_3$ | $OCH_3$ | CH | - | |
| Q-9 | H | H | $CH_3$ | $CH_3$ | $CH_3$ | $OCH_3$ | CH | - | |
| Q-9 | H | H | H | H | Cl | $OCH_2CH_3$ | CH | - | |
| Q-9 | H | H | H | H | $OCH_3$ | $CH(OCH_3)_2$ | CH | - | |
| Q-9 | H | H | H | H | $NH_2$ | $CH_3$ | CH | - | |
| Q-9 | H | H | H | H | $NHCH(CH_3)_2$ | $CH_3$ | CH | - | |
| Q-9 | H | H | H | H | $CH_2O$-n-$C_4H_9$ | $CH_3$ | CH | - | |
| Q-9 | H | H | H | H | S-n-$C_4H_9$ | $CH_3$ | CH | - | |
| Q-9 | H | H | H | H | S-n-$CH_2CH_2F$ | $CH_3$ | CH | - | |

**Table 7b**

**General Structure 3**

| Q | R | $R_{3'}$ | $R_4$ | X | Y | Z | W" | m.p. °C |
|---|---|---|---|---|---|---|---|---|
| Q-10 | H | H | H | $CH_3$ | $OCH_3$ | CH | O | |
| Q-10 | H | H | H | $CH_3$ | $CH_3$ | CH | O | |
| Q-10 | H | H | H | $OCH_3$ | $OCH_3$ | N | O | |
| Q-10 | H | H | H | $OCH_3$ | Cl | CH | O | |
| Q-10 | H | H | H | $OCH_3$ | $OCH_3$ | CH | O | |
| Q-10 | H | H | H | $OCH_3$ | $OCH_3$ | N | O | |
| Q-10 | H | H | H | $CH_3$ | $OCH_3$ | CH | S | |
| Q-10 | H | H | H | $CH_3$ | $OCH_3$ | N | S | |
| Q-10 | H | H | H | $OCH_3$ | $OCH_3$ | CH | S | |
| Q-10 | H | H | H | $OCH_3$ | $OCH_3$ | N | S | |
| Q-10 | H | H | H | $OCH_3$ | Cl | CH | $NCH_3$ | |
| Q-10 | H | H | H | $CH_3$ | $CH_3$ | CH | $NCH_3$ | |
| Q-10 | H | H | H | $CH_3$ | $OCH_3$ | N | $NCH_3$ | |
| Q-10 | H | H | H | $CH_3$ | $CH_3$ | N | $NCH_3$ | |
| Q-10 | H | H | H | $OCH_3$ | $OCH_3$ | CH | $NCH_3$ | |
| Q-10 | H | H | H | $OCH_3$ | $OCH_3$ | N | $NCH_3$ | |
| Q-10 | H | $CH_3$ | H | $CH_3$ | $CH_3$ | CH | O | |
| Q-10 | H | H | $CH_3$ | $CH_3$ | $OCH_3$ | N | O | |
| Q-10 | H | $CH_3$ | $CH_3$ | $CH_3$ | $OCH_3$ | CH | O | |
| Q-10 | $CH_3$ | H | H | $OCH_3$ | $OCH_3$ | N | O | |
| Q-10 | H | H | H | $CH_3$ | 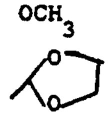 | CH | O | |
| Q-10 | H | $CH_3$ | H | $CH_3$ | $CH_3$ | CH | S | |
| Q-10 | H | H | $CH_3$ | $CH_3$ | $OCH_3$ | N | S | |
| Q-10 | H | $CH_3$ | $CH_3$ | $CH_3$ | $OCH_3$ | CH | S | |
| Q-10 | $CH_3$ | $CH_3$ | $CH_3$ | $CH_3$ | $OCH_3$ | CH | S | |
| Q-10 | $CH_3$ | H | H | $OCH_3$ | $OCH_3$ | N | S | |
| Q-10 | H | H | H | $CH_3$ | $OC_2H_5$ | CH | S | |
| Q-10 | H | H | $CH_3$ | $CH_3$ | $CH_3$ | N | $NCH_3$ | |
| Q-10 | H | H | $CH_3$ | $CH_3$ | $OCH_3$ | CH | $NCH_3$ | |
| Q-10 | H | $CH_3$ | $CH_3$ | $OCH_3$ | $OCH_3$ | N | $NCH_3$ | |
| Q-10 | $CH_3$ | H | H | $OCH_3$ | $OCH_3$ | N | $NCH_3$ | |
| Q-11 | H | H | H | $CH_3$ | $CH_3$ | N | O | |

Table 7b (continued)

| Q | R | $R_3$' | $R_4$ | X | Y | Z | W" | m.p. °C |
|---|---|---|---|---|---|---|---|---|
| Q-11 | H | H | H | $CH_3$ | $CH_3$ | CH | O | |
| Q-11 | H | H | H | $CH_3$ | $OCH_3$ | N | O | |
| Q-11 | H | H | H | $CH_3$ | $OCH_3$ | CH | O | |
| Q-11 | H | H | H | $OCH_3$ | $OCH_3$ | CH | O | |
| Q-11 | H | H | H | $OCH_3$ | $OCH_3$ | N | O | |
| Q-11 | H | H | H | $CH_3$ | $CH_3$ | CH | S | |
| Q-11 | H | H | H | $CH_3$ | $OCH_3$ | CH | S | |
| Q-11 | H | H | H | $CH_3$ | $OCH_3$ | CH | S | |
| Q-11 | H | H | H | $OCH_3$ | $OCH_3$ | CH | S | |
| Q-11 | H | H | H | $CH_3$ | $CH_3$ | CH | $NCH_3$ | |
| Q-11 | H | H | H | $CH_3$ | $OCH_3$ | CH | $NCH_3$ | |
| Q-11 | H | H | H | $OCH_3$ | $OCH_3$ | CH | $NCH_3$ | |
| Q-11 | H | H | H | $CH_3$ | $OCH_3$ | N | $NCH_3$ | |
| Q-11 | H | $CH_3$ | H | $CH_3$ | $OCH_3$ | N | O | |
| Q-11 | H | H | $CH_3$ | $OCH_3$ | $OCH_3$ | N | O | |
| Q-11 | H | $CH_3$ | $CH_3$ | $CH_3$ | $OCH_3$ | CH | O | |
| Q-11 | $CH_3$ | H | H | $CH_3$ | $OCH_3$ | CH | O | |
| Q-11 | H | H | H | $CH_3$ | $C_2H_5$ | N | O | |
| Q-11 | H | H | H | $CH_3$ | $CH_2OCH_3$ | CH | O | |
| Q-11 | H | $CH_3$ | H | $CH_3$ | $OCH_3$ | N | S | |
| Q-11 | H | H | $CH_3$ | $OCH_3$ | $OCH_3$ | N | S | |
| Q-11 | H | $CH_3$ | $CH_3$ | $CH_3$ | $OCH_3$ | CH | S | |
| Q-11 | $CH_3$ | H | H | $CH_3$ | $OCH_3$ | CH | S | |
| Q-11 | H | H | H | $CH_3$ | $C_2H_5$ | N | S | |
| Q-11 | $CH_3$ | $CH_3$ | $CH_3$ | $CH_3$ | $OCH_3$ | N | S | |
| Q-11 | H | H | H | Cl | $OCH_3$ | CH | S | |
| Q-11 | H | $CH_3$ | H | $CH_3$ | $CH_3$ | CH | $NCH_3$ | |
| Q-11 | H | H | $CH_3$ | $CH_3$ | $OCH_3$ | N | $NCH_3$ | |
| Q-11 | H | $CH_3$ | $CH_3$ | $CH_3$ | $OCH_3$ | CH | $NCH_3$ | |
| Q-11 | $CH_3$ | H | H | $OCH_3$ | $OCH_3$ | N | NH | |
| Q-11 | H | H | H | $CH_3$ | $OCH_3$ | CH | NH | |
| Q-11 | H | H | H | $OCH_3$ | $OCH_3$ | CH | O | * |
| Q-11 | H | H | H | $OCH_3$ | CH | N | O | * |

Table 7b (continued)

| Q | R | $R_{3'}$ | $R_4$ | X | Y | Z | W" | m.p. °C |
|---|---|---|---|---|---|---|---|---|
| Q-11 | H | H | H | $CH_3$ | $OCH_3$ | N | $NCH_3$ | |
| Q-11 | H | H | H | $CH_3$ | $OCF_2H$ | CH | $NCH_3$ | |
| Q-11 | H | $CH_3$ | H | $OCH_3$ | $OCH_2CF_3$ | N | $NCH_3$ | |
| Q-12 | H | H | H | $CH_3$ | $OCH_3$ | CH | O | |
| Q-12 | H | H | H | $CH_3$ | $OCH_3$ | N | O | |
| Q-12 | H | H | H | $OCH_3$ | $OCH_3$ | CH | O | |
| Q-12 | H | H | H | $OCH_3$ | $OCH_3$ | N | O | |
| Q-12 | H | H | H | $CH_3$ | $OCH_3$ | CH | S | |
| Q-12 | H | H | H | $CH_3$ | $OCH_3$ | N | S | |
| Q-12 | H | H | H | $OCH_3$ | $OCH_3$ | CH | S | |
| Q-12 | H | H | H | $OCH_3$ | $OCH_3$ | N | S | |
| Q-12 | H | H | H | $CH_3$ | $CH_3$ | N | $NCH_3$ | |
| Q-12 | H | H | H | $CH_3$ | $OCH_3$ | CH | $NCH_3$ | |
| Q-12 | H | H | H | $CH_3$ | $OCH_3$ | N | $NCH_3$ | |
| Q-12 | H | H | H | $OCH_3$ | $OCH_3$ | CH | $NCH_3$ | |
| Q-12 | H | H | H | $OCH_3$ | $OCH_3$ | N | $NCH_3$ | |
| Q-12 | H | H | H | $OCH_3$ | $OCH_3$ | N | NH | |
| Q-12 | H | $CH_3$ | H | $CH_3$ | $CH_3$ | CH | O | |
| Q-12 | H | H | $CH_3$ | $CH_3$ | $OCH_3$ | N | O | |
| Q-12 | H | $CH_3$ | $CH_3$ | $CH_3$ | $OCH_3$ | CH | O | |
| Q-12 | $CH_3$ | H | H | $OCH_3$ | $OCH_3$ | N | O | |
| Q-12 | $CH_3$ | H | H | $CH_3$ | $CF_3$ | CH | O | |
| Q-12 | H | H | H | $OCH_3$ | $OCH_2CF_3$ | N | | |
| Q-12 | H | $CH_3$ | H | $CH_3$ | $OCH_3$ | CH | S | |
| Q-12 | H | H | $CH_3$ | $CH_3$ | $OCH_3$ | N | S | |
| Q-12 | H | $CH_3$ | $CH_3$ | $OCH_3$ | $OCH_3$ | CH | S | |
| Q-12 | $CH_3$ | H | H | $OCH_3$ | $OCH_3$ | N | S | |
| Q-12 | $CH_3$ | $CH_3$ | H | $CH_3$ | $NHCH_3$ | CH | S | |
| Q-12 | H | H | H | Br | $OC_2H_5$ | CH | S | |
| Q-12 | H | $CH_3$ | H | $CH_3$ | $CH_3$ | CH | NH | |
| Q-12 | H | $CH_3$ | H | $CH_3$ | $CH_3$ | CH | $NCH_3$ | |
| Q-12 | H | H | $CH_3$ | $CH_3$ | $OCH_3$ | N | $NCH_3$ | |
| Q-12 | H | $CH_3$ | $CH_3$ | $CH_3$ | $CH_3$ | $CH_3$ | $NCH_3$ | |
| Q-12 | $CH_3$ | $CH_3$ | $CH_3$ | $CH_3$ | $OCH_3$ | N | $NCH_3$ | |

Table 7b (continued)

| Q | R | $R_3'$ | $R_4$ | X | Y | Z | W" | m.p. °C |
|---|---|---|---|---|---|---|---|---|
| Q-12 | $CH_3$ | $CH_3$ | $CH_3$ | $CH_3$ | $OCH_3$ | CH | $NCH_3$ | |
| Q-12 | H | H | H | Br | $OCH_3$ | CH | $NCH_3$ | |
| Q-12 | H | H | H | $OCH_3$ | $N(CH_3)_2$ | CH | $NCH_3$ | |
| Q-13 | H | H | H | $CH_3$ | $OCH_3$ | CH | O | |
| Q-13 | H | H | H | $CH_3$ | $OCH_3$ | CH | O | |
| Q-13 | H | H | H | $CH_3$ | $CH_3$ | CH | O | |
| Q-13 | H | H | H | $CH_3$ | $OCH_3$ | N | O | |
| Q-13 | H | H | H | $OCH_3$ | $OCH_3$ | CH | O | |
| Q-13 | H | H | H | $CH_3$ | $CH_3$ | N | O | |
| Q-13 | H | H | H | $OCH_3$ | $OCH_3$ | N | O | |
| Q-13 | H | H | H | $CH_3$ | $OCH_3$ | CH | S | |
| Q-13 | H | H | H | $CH_3$ | $OCH_3$ | N | S | |
| Q-13 | H | H | H | $OCH_3$ | $OCH_3$ | CH | S | |
| Q-13 | H | H | H | $OCH_3$ | $OCH_3$ | N | S | |
| Q-13 | H | H | H | $CH_3$ | $OCH_3$ | CH | NH | |
| Q-13 | H | H | H | $CH_3$ | $OCH_3$ | N | NH | |
| Q-13 | H | H | H | $OCH_3$ | $OCH_3$ | CH | $NCH_3$ | |
| Q-13 | H | H | H | $OCH_3$ | $OCH_3$ | N | NH | |
| Q-13 | H | $CH_3$ | H | $CH_3$ | $CH_3$ | CH | O | |
| Q-13 | H | H | $CH_3$ | $CH_3$ | $CH_3$ | N | O | |
| Q-13 | H | $CH_3$ | $CH_3$ | $CH_3$ | $OCH_3$ | CH | O | |
| Q-13 | $CH_3$ | H | H | $CH_3$ | $OCH_3$ | N | O | |
| Q-13 | H | H | H | $CH_3$ | $CH(OCH_3)_2$ | CH | O | |
| Q-13 | H | $CH_3$ | H | $CH_3$ | $OCH_3$ | CH | S | |
| Q-13 | H | H | $CH_3$ | $CH_3$ | $OCH_3$ | CH | S | |
| Q-13 | H | $CH_3$ | $CH_3$ | $CH_3$ | $OCH_3$ | N | S | |
| Q-13 | $CH_3$ | H | $CH_3$ | $OCH_3$ | $OCH_3$ | N | S | |
| Q-13 | $CH_3$ | $CH_3$ | $CH_3$ | $CH_3$ | $CH_3$ | N | S | |
| Q-13 | H | H | H | $OCH_3$ | 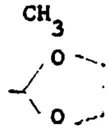 | CH | S | |
| Q-13 | H | $CH_3$ | H | $CH_3$ | $OCH_3$ | N | $NCH_3$ | |
| Q-13 | H | H | $CH_3$ | $CH_3$ | $OCH_3$ | N | $NCH_3$ | |
| Q-13 | H | $CH_3$ | $CH_3$ | $CH_3$ | $OCH_3$ | CH | $NCH_3$ | |

Table 7b (continued)

| Q | R | $R_3$' | $R_4$ | X | Y | Z | W" | m.p. °C |
|---|---|---|---|---|---|---|---|---|
| Q-13 | $CH_3$ | H | H | $OCH_3$ | $OCH_3$ | CH | $NCH_3$ | |
| Q-13 | H | H | H | $CH_3$ | $OCH_3$ | N | NH | |
| Q-13 | H | H | H | Br | $OC_2H_5$ | CH | NH | |
| Q-13 | H | H | H | $OCH_3$ | $C_2H_5$ | N | NH | |
| Q-13 | H | H | H | $CH_3$ | $CH_3$ | CH | $NCH_3$ | |
| Q-13 | H | H | H | $CH_3$ | $CH_3$ | N | $NCH_3$ | |
| Q-13 | H | H | H | Cl | $OCH_3$ | CH | $NCH_3$ | |
| Q-14 | H | H | H | $CH_3$ | $CH_3$ | N | O | |
| Q-14 | H | H | H | $CH_3$ | $CH_3$ | CH | O | |
| Q-14 | H | H | H | $CH_3$ | $OCH_3$ | CH | O | |
| Q-14 | H | H | H | $CH_3$ | $OCH_3$ | CH | O | |
| Q-14 | H | H | H | $CH_3$ | $OCH_3$ | N | O | |
| Q-14 | H | H | H | $OCH_3$ | $OCH_3$ | CH | O | |
| Q-14 | H | H | H | $CH_3$ | $OCH_3$ | CH | S | |
| Q-14 | H | H | H | $CH_3$ | $OCH_3$ | N | S | |
| Q-14 | H | H | H | $OCH_3$ | $OCH_3$ | CH | S | |
| Q-14 | H | H | H | $OCH_3$ | $OCH_3$ | CH | S | |
| Q-14 | H | H | H | $OCH_3$ | $OCH_3$ | N | S | |
| Q-14 | H | H | H | $CH_3$ | $OCH_3$ | CH | $NCH_3$ | |
| Q-14 | H | H | H | $CH_3$ | $OCH_3$ | N | $NCH_3$ | |
| Q-14 | H | H | H | $OCH_3$ | $OCH_3$ | CH | NH | |
| Q-14 | H | H | H | $OCH_3$ | $OCH_3$ | N | $NCH_3$ | |
| Q-14 | H | $CH_3$ | H | $CH_3$ | $CH_3$ | CH | O | |
| Q-14 | H | H | $CH_3$ | $CH_3$ | $CH_3$ | N | O | |
| Q-14 | H | $CH_3$ | $CH_3$ | $CH_3$ | $OCH_3$ | CH | O | |
| Q-14 | $CH_3$ | $CH_3$ | $CH_3$ | $CH_3$ | $OCH_3$ | N | O | |
| Q-14 | H | H | H | $CH_3$ | $C_2H_5$ | N | O | |
| Q-14 | H | H | H | $CH_3$ | $OC_2H_5$ | N | O | |
| Q-14 | H | $CH_3$ | H | $CH_3$ | $OCH_3$ | CH | S | |
| Q-14 | H | H | $CH_3$ | $CH_3$ | $OCH_3$ | N | S | |
| Q-14 | H | $CH_3$ | $CH_3$ | $OCH_3$ | $OCH_3$ | CH | S | |
| Q-14 | $CH_3$ | $CH_3$ | $CH_3$ | $OCH_3$ | $OCH_3$ | CH | S | |
| Q-14 | H | H | H | $OCH_3$ | $C_2H_5$ | N | S | |
| Q-14 | H | H | H | $OCH_3$ | $OC_2H_5$ | N | S | |

Table 7b(continued)

| Q | R | $R_{3'}$ | $R_4$ | X | Y | Z | W'' | m.p. °C |
|---|---|---|---|---|---|---|---|---|
| Q-14 | H | $CH_3$ | H | $CH_3$ | $OCH_3$ | CH | $NCH_3$ | |
| Q-14 | H | H | $CH_3$ | $CH_3$ | $OCH_3$ | N | $NCH_3$ | |
| Q-14 | H | $CH_3$ | $CH_3$ | $OCH_3$ | $OCH_3$ | CH | $NCH_3$ | |
| Q-14 | $CH_3$ | $CH_3$ | H | $OCH_3$ | $OCH_3$ | N | $NCH_3$ | |
| Q-14 | H | $CH_3$ | $CH_3$ | $CH_3$ | $OCH_3$ | CH | NH | |
| Q-14 | H | H | H | $CH_3$ | $OCH_3$ | N | NH | |
| Q-14 | H | H | H | $CH_3$ | $CH_2OCH_3$ | CH | $NCH_3$ | |
| Q-15 | H | H | H | $CH_3$ | $OCH_3$ | CH | O | |
| Q-15 | H | H | H | $CH_3$ | $OCH_3$ | N | O | |
| Q-15 | H | H | H | $OCH_3$ | $OCH_3$ | CH | O | |
| Q-15 | H | H | H | $OCH_3$ | $OCH_3$ | N | O | |
| Q-15 | H | $C_2H_5$ | H | $OCH_3$ | $OCH_3$ | CH | O | |
| Q-15 | H | $CH_3$ | H | $CH_3$ | $OCH_3$ | CH | O | |
| Q-15 | H | $CH_3$ | H | $CH_3$ | $CH_3$ | CH | O | |
| Q-15 | H | $CH_3$ | H | $OCH_3$ | $OCH_3$ | CH | O | |
| Q-15 | H | H | $CH_3$ | $CH_3$ | $CH_3$ | N | O | |
| Q-15 | H | $CH_3$ | H | $CH_3$ | $OCH_3$ | N | O | |
| Q-15 | H | $CH_3$ | $CH_3$ | $CH_3$ | $OCH_3$ | CH | O | |
| Q-15 | H | $CH_3$ | H | $OCH_3$ | $OCH_3$ | N | O | |
| Q-15 | $CH_3$ | H | H | $CH_3$ | $OCH_3$ | N | O | |
| Q-15 | H | $CH_3$ | H | $OCH_3$ | Cl | CH | O | |
| Q-15 | $CH_3$ | $CH_3$ | $CH_3$ | $OCH_3$ | $OCH_3$ | CH | O | |
| Q-15 | H | H | H | $CH_3$ | $CF_3$ | CH | O | |
| Q-15 | H | H | H | $CH_3$ | $CF_3$ | N | O | |
| Q-15 | H | H | H | $OCH_3$ | $OCF_2H$ | CH | O | |
| Q-15 | $CH_3$ | H | H | $OCH_3$ | $OCF_2H$ | CH | O | |
| Q-15 | H | H | H | $CH_3$ | $CH_3$ | CH | O | |
| Q-15 | H | H | H | $CH_3$ | $CH_3$ | N | O | |
| Q-15 | H | H | H | $OCH_3$ | $OCH_3$ | CH | O | * |
| Q-15 | H | H | H | $OCH_3$ | $CH_3$ | N | O | * |
| Q-15 | H | H | H | $CH_3$ | $CH_3$ | N | S | |
| Q-15 | H | $CH_3$ | H | $CH_3$ | $OCH_3$ | CH | S | |
| Q-15 | H | $CH_3$ | H | $CH_3$ | $OCH_3$ | N | S | |

Table 7b (continued)

| Q | R | $R_3$' | $R_4$ | X | Y | Z | W" | m.p. °C |
|---|---|---|---|---|---|---|---|---|
| Q-15 | H | $CH_3$ | H | $OCH_3$ | $OCH_3$ | CH | S | |
| Q-15 | H | $CH_3$ | H | $OCH_3$ | $OCH_3$ | N | S | |
| Q-15 | H | $CH_3$ | H | Cl | $OCH_3$ | CH | S | |
| Q-15 | H | H | H | $CH_3$ | $OCH_3$ | CH | $NCH_3$ | |
| Q-15 | H | H | H | $CH_3$ | $OCH_3$ | N | $NCH_3$ | |
| Q-15 | H | H | H | $OCH_3$ | $OCH_3$ | CH | $NCH_3$ | |
| Q-15 | H | H | H | $OCH_3$ | $OCH_3$ | N | $NCH_3$ | |
| Q-15 | H | $CH_3$ | H | $CH_3$ | $CH_3$ | CH | S | |
| Q-15 | H | H | $CH_3$ | $CH_3$ | $CH_3$ | N | S | |
| Q-15 | H | $CH_3$ | $CH_3$ | $CH_3$ | $OCH_3$ | CH | S | |
| Q-15 | $CH_3$ | H | H | $CH_3$ | $OCH_3$ | N | S | |
| Q-15 | $CH_3$ | H | $CH_3$ | $OCH_3$ | $OCH_3$ | CH | S | |
| Q-15 | H | H | H | Cl | $OC_2H_5$ | CH | S | |
| Q-15 | H | H | H | $OCH_3$ | $OCH_2CF_3$ | CH | S | |
| Q-15 | H | $CH_3$ | H | $CH_3$ | $CH_3$ | CH | $NCH_3$ | |
| Q-15 | H | H | $CH_3$ | $CH_3$ | $OCH_3$ | CH | $NCH_3$ | |
| Q-15 | H | $CH_3$ | $CH_3$ | $CH_3$ | $OCH_3$ | N | $NCH_3$ | |
| Q-15 | $CH_3$ | $CH_3$ | $CH_3$ | $CH_3$ | $OCH_3$ | N | $NCH_3$ | |
| Q-15 | $CH_3$ | H | H | $CH_3$ | $OCH_3$ | CH | NH | |
| Q-15 | H | H | H | $OCH_3$ | $OCH_3$ | N | NH | |
| Q-15 | H | H | H | $CH_3$ | $NHCH_3$ | CH | $NCH_3$ | |
| Q-15 | H | H | H | $OCH_3$ | $N(CH_3)_2$ | CH | $NCH_3$ | |
| Q-15 | H | H | H | $CH_3$ | $OCH_2C{\equiv}CH$ | N | S | |
| Q-15 | H | H | H | $CH_3$ | $CH_2C{\equiv}CH_3$ | CH | S | |
| Q-15 | H | H | H | $CH_3$ | $C{\equiv}CH$ | N | S | |
| Q-15 | H | $C_2H_5$ | H | $OCH_3$ | $OCH_3$ | CH | S | |
| Q-15 | H | H | H | $OCH_3$ | $(CH_2)_3CH_2F$ | CH | S | |
| Q-15 | H | H | H | $OCH_3$ | 2-methyl-1,3-dioxolan-2-yl | N | S | |
| Q-15 | H | H | H | $OCH_3$ | 2-methyl-1,3-dithiolan-2-yl | CH | S | |
| Q-15 | H | H | H | $OCH_3$ | 2-methyl-1,3-dithian-2-yl | N | S | |
| Q-15 | H | H | H | $OCH_3$ | $OCH_3$ | CH | S | * |
| Q-15 | H | H | H | $OCH_3$ | $CH_3$ | N | S | * |

**Table 7e**

**General Structure 3**

| Q | R | $R_3$ | $R_4$ | $R_5$ | X | Y | Z | m.p. °C |
|---|---|---|---|---|---|---|---|---|
| Q-18 | H | H | H | H | $CH_3$ | $CH_3$ | CH | |
| Q-18 | H | H | H | H | $CH_3$ | $OCH_3$ | CH | |
| Q-18 | H | H | H | H | $CH_3$ | $OCH_3$ | N | |
| Q-18 | $CH_3$ | H | H | H | $CH_3$ | $OCH_3$ | N | |
| Q-18 | H | H | H | H | $OCH_3$ | $OCH_3$ | CH | |
| Q-18 | H | H | H | H | $CH_3$ | $CH_3$ | CH | |
| Q-18 | H | H | H | H | $OCH_3$ | $OCH_3$ | N | |
| Q-18 | H | H | H | H | Cl | $OCH_3$ | CH | |
| Q-18 | H | $CH_3$ | H | H | $CH_3$ | $OCH_3$ | CH | |
| Q-18 | H | H | $CH_3$ | H | $CH_3$ | $OCH_3$ | N | |
| Q-18 | H | H | H | $CH_3$ | $CH_3$ | $CH_3$ | CH | |
| Q-18 | H | $CH_3$ | $CH_3$ | H | $CH_3$ | $CH_3$ | N | |
| Q-18 | H | H | $CH_3$ | $CH_3$ | $OCH_3$ | $OCH_3$ | CH | |
| Q-18 | H | $CH_3$ | H | $CH_3$ | $OCH_3$ | $OCH_3$ | N | |
| Q-18 | H | $CH_3$ | $CH_3$ | $CH_3$ | $CH_3$ | $OCH_3$ | CH | |
| Q-18 | H | H | H | H | $OCH_3$ | $CH(OCH_3)_2$ | CH | |
| Q-19 | $CH_3$ | H | H | H | Cl | $OCH_3$ | CH | |
| Q-19 | H | H | H | H | $CH_3$ | $OCH_3$ | CH | |
| Q-19 | H | H | H | H | $CH_3$ | $OCH_3$ | N | |
| Q-19 | H | H | H | H | $OCH_3$ | $OCH_3$ | CH | |
| Q-19 | H | H | H | H | $OCH_3$ | $OCH_3$ | N | |
| Q-19 | H | $CH_3$ | H | H | $CH_3$ | $CH_3$ | CH | |
| Q-19 | H | H | $CH_3$ | H | $CH_3$ | $CH_3$ | N | |
| Q-19 | H | H | H | $CH_3$ | $CH_3$ | $OCH_3$ | CH | |
| Q-19 | H | $CH_3$ | $CH_3$ | H | $CH_3$ | $OCH_3$ | N | |
| Q-19 | H | H | $CH_3$ | $CH_3$ | $OCH_3$ | $OCH_3$ | CH | |
| Q-19 | H | $CH_3$ | H | $CH_3$ | $OCH_3$ | $OCH_3$ | N | |
| Q-19 | H | H | H | H | $CH_3$ | $CH_3$ | CH | |
| Q-19 | H | $CH_3$ | $CH_3$ | $CH_3$ | $CH_3$ | $OCH_3$ | CH | |
| Q-19 | $CH_3$ | H | H | H | $CH_3$ | $OCH_3$ | N | |
| Q-19 | H | H | H | H | $OCH_3$ | 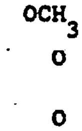 | CH | |

Table 7d

General Structure 3

| Q | R | $R_3'$," | X | Y | Z | W' | m.p. °C |
|---|---|---|---|---|---|---|---|
| Q-21 | H | H | $CH_3$ | $OCH_3$ | CH | O | |
| Q-21 | H | H | $CH_3$ | $OCH_3$ | N | O | |
| Q-21 | H | H | $OCH_3$ | $OCH_3$ | CH | O | |
| Q-21 | $CH_3$ | H | $OCH_3$ | $OCH_3$ | CH | O | |
| Q-21 | H | H | $OCH_3$ | $NHCH_3$ | CH | O | |
| Q-21 | H | H | $OCH_3$ | $OCH_3$ | N | O | |
| Q-21 | H | $CH_3$ | $CH_3$ | $OCH_3$ | CH | O | |
| Q-21 | $CH_3$ | $CH_3$ | $CH_3$ | $OCH_3$ | CH | O | |
| Q-21 | H | H | $CH_3$ | $CH_3$ | CH | O | |
| Q-21 | H | H | $CH_3$ | $CH_3$ | N | O | |
| Q-21 | H | H | $CH_3$ | $OCH_3$ | CH | S | |
| Q-21 | H | H | $CH_3$ | $OCH_3$ | N | S | |
| Q-21 | H | H | $OCH_3$ | $OCH_3$ | CH | S | |
| Q-21 | H | H | $OCH_3$ | $OCH_3$ | N | S | |
| Q-21 | H | $CH_3$ | $CH_3$ | $OCH_3$ | CH | S | |
| Q-21 | $CH_3$ | H | $CH_3$ | $OCH_3$ | CH | S | |
| Q-21 | H | H | $OCH_3$ | $N(CH_3)_2$ | N | S | |
| Q-22 | H | H | $CH_3$ | $OCH_3$ | CH | O | |
| Q-22 | H | H | $CH_3$ | $OCH_3$ | N | O | |
| Q-22 | H | H | $OCH_3$ | $OCH_3$ | CH | O | |
| Q-22 | H | H | $OCH_3$ | $OCH_3$ | N | O | |
| Q-22 | H | $CH_3$ | $CH_3$ | $OCH_3$ | CH | O | |
| Q-22 | H | $CH_3$ | $CH_3$ | $OCH_3$ | N | O | |
| Q-22 | $CH_3$ | $CH_3$ | $CH_3$ | $CH_3$ | CH | O | |
| Q-22 | H | H | $CH_3$ | $CH(OCH_3)_2$ | CH | O | |
| Q-22 | H | H | $CH_3$ | $C_2H_5$ | N | O | |
| Q-22 | H | H | $CH_3$ | $CH_3$ | CH | S | |
| Q-22 | H | H | $CH_3$ | $CH_3$ | N | S | |
| Q-22 | H | H | $CH_3$ | $OCH_3$ | CH | S | |
| Q-22 | H | H | $CH_3$ | $OCH_3$ | N | S | |

$R_{11}$ is -, unless indicated by ** where $R_{11}$ is $CH_3$ in all Tables.

**Table 7d (continued)**

| Q | R | $R_3'$," | X | Y | Z | W' | m.p. °C |
|---|---|---|---|---|---|---|---|
| Q-22 | H | H | $OCH_3$ | $OCH_3$ | CH | S | |
| Q-22 | H | H | $OCH_3$ | $OCH_3$ | N | S | |
| Q-22 | H | $CH_3$ | $CH_3$ | $OCH_3$ | CH | S | |
| Q-22 | $CH_3$ | $CH_3$ | $CH_3$ | $OCH_3$ | CH | S | |
| Q-22 | $CH_3$ | H | $CH_3$ | $OCH_3$ | N | S | |
| Q-22 | H | H | $CH_3$ | 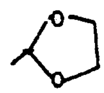 | CH | S | |
| Q-22 | H | H | $OCH_3$ | $C_2H_5$ | N | S | |
| Q-23 | H | H | $CH_3$ | $OCH_3$ | CH | - | |
| Q-23 | H | H | $CH_3$ | $OCH_3$ | N | - | |
| Q-23 | H | H | $OCH_3$ | $OCH_3$ | CH | - | |
| Q-23 | H | H | $OCH_3$ | $OCH_3$ | N | - | |
| Q-23 | H | $CH_3$ | $CH_3$ | $CH_3$ | CH | - | |
| Q-23 | H | $CH_3$ | $CH_3$ | $OCH_3$ | N | - | |
| Q-23 | $CH_3$ | H | $CH_3$ | $OCH_3$ | N | - | |
| Q-23 | $CH_3$ | $CH_3$ | $CH_3$ | $OCH_3$ | CH | - | |
| Q-23 | H | H | $CH_3$ | $OC_2H_5$ | N | - | |
| Q-23 | H | H | $CH_3$ | $CH_2OCH_3$ | CH | - | |
| Q-24 | H | H | $CH_3$ | $OCH_3$ | CH | - | |
| Q-24 | H | H | $CH_3$ | $OCH_3$ | N | - | |
| Q-24 | H | H | $OCH_3$ | $OCH_3$ | CH | - | |
| Q-24 | H | H | $OCH_3$ | $OCH_3$ | N | - | |
| Q-24 | H | $CH_3$ | $CH_3$ | $OCH_3$ | CH | - | |
| Q-24 | H | $CH_3$ | $CH_3$ | $CH_3$ | CH | - | |
| Q-24 | $CH_3$ | H | $CH_3$ | $CH_3$ | N | - | |
| Q-24 | H | H | $CH_3$ | $CF_3$ | CH | - | |
| Q-24 | H | H | $OCH_3$ | $OCF_2H$ | N | - | |
| Q-25 | H | H | $CH_3$ | $OCH_3$ | CH | - | ** |
| Q-25 | H | H | $CH_3$ | $OCH_3$ | N | - | ** |
| Q-25 | H | H | $OCH_3$ | $OCH_3$ | CH | - | ** |

Table 7d (continued)

| Q | R | $R'_3$'' | X | Y | Z | W' | m.p. °C |
|---|---|---|---|---|---|---|---|
| Q-25 | H | H | $OCH_3$ | $OCH_3$ | N | - | ** |
| Q-25 | $CH_3$ | H | $OCH_3$ | $OCH_3$ | N | - | ** |
| Q-25 | H | H | $CH_3$ | $CH_3$ | CH | - | ** |
| Q-25 | H | H | $CH_3$ | $CH_3$ | N | - | ** |
| Q-25 | H | $CH_3$ | $CH_3$ | $OCH_3$ | CH | - | ** |
| Q-25 | H | $CH_3$ | $CH_3$ | $OCH_3$ | N | - | ** |
| Q-25 | $CH_3$ | $CH_3$ | $OCH_3$ | $OCH_3$ | N | - | ** |
| Q-25 | H | H | $CH_3$ | $OCH_2CF_3$ | CH | - | ** |
| Q-25 | H | H | $CH_3$ | $NHCH_3$ | N | - | ** |
| Q-26 | H | H | $CH_3$ | $OCH_3$ | CH | - | ** |
| Q-26 | H | H | $CH_3$ | $OCH_3$ | N | - | ** |
| Q-26 | H | H | $OCH_3$ | $OCH_3$ | CH | - | ** |
| Q-26 | H | $CH_3$ | $CH_3$ | $CH_3$ | N | - | ** |
| Q-26 | H | $CH_3$ | $CH_3$ | $CH_3$ | CH | - | ** |
| Q-26 | H | $CH_3$ | $CH_3$ | $OCH_3$ | N | - | ** |
| Q-26 | H | $CH_3$ | $CH_3$ | $OCH_3$ | CH | - | ** |
| Q-26 | H | $CH_3$ | $CH_3$ | $OCH_3$ | N | - | ** |
| Q-26 | H | H | $CH_3$ | $CH_3$ | CH | - | ** |
| Q-26 | H | H | $CH_3$ | $CH_3$ | N | - | ** |
| Q-26 | H | $CH_3$ | $OCH_3$ | $OCH_3$ | CH | - | ** |
| Q-26 | H | $CH_3$ | $OCH_3$ | $OCH_3$ | N | - | ** |
| Q-26 | $CH_3$ | H | $CH_3$ | $OCH_3$ | CH | - | ** |
| Q-26 | H | H | $CH_3$ | $N(CH_3)_2$ | CH | - | ** |
| Q-26 | H | H | $CH_3$ | $N(CH_3)_2$ | N | - | ** |
| Q-27 | H | H | $CH_3$ | $OCH_3$ | CH | - | ** |
| Q-27 | H | H | $CH_3$ | $OCH_3$ | N | - | ** |
| Q-27 | H | H | $OCH_3$ | $OCH_3$ | CH | - | ** |
| Q-27 | H | H | $OCH_3$ | $OCH_3$ | N | - | ** |
| Q-27 | H | H | $CH_3$ | $CH_3$ | CH | - | ** |
| Q-27 | H | H | $CH_3$ | $CH_3$ | N | - | ** |
| Q-27 | H | $CH_3$ | $CH_3$ | $CH_3$ | CH | - | ** |

Table 7d (continued)

| Q | R | $R'_3$," | X | Y | Z | W' | m.p. °C |
|---|---|---|---|---|---|---|---|
| Q-27 | H | H | Cl | $OC_2H_5$ | CH | - | ** |
| Q-27 | H | $CH_3$ | $CH_3$ | $CH_3$ | N | - | ** |
| Q-27 | H | $CH_3$ | $CH_3$ | $OCH_3$ | CH | - | ** |
| Q-27 | H | $CH_3$ | $CH_3$ | $OCH_3$ | N | - | ** |
| Q-27 | H | $CH_3$ | $OCH_3$ | $OCH_3$ | CH | - | ** |
| Q-27 | H | $CH_3$ | $OCH_3$ | $OCH_3$ | N | - | ** |
| Q-27 | $CH_3$ | $CH_3$ | $CH_3$ | $OCH_3$ | N | - | ** |
| Q-27 | $CH_3$ | H | $CH_3$ | $OCH_3$ | N | - | ** |
| Q-27 | H | H | $CH_3$ | $CH(OCH_3)_2$ | CH | - | ** |
| Q-27 | H | H | $CH_3$ | $O(CH_2)_3CH_2Cl$ | CH | - | ** |
| Q-25 | H | SH | $CH_3$ | $CH_3$ | CH | - | ** |
| Q-25 | H | SH | $CH_3$ | $OCH_3$ | CH | - | ** |
| Q-25 | H | SH | $OCH_3$ | $OCH_3$ | CH | - | ** |
| Q-25 | H | SH | $OCH_3$ | Cl | CH | - | ** |
| Q-25 | H | SH | $CH_3$ | $OCH_3$ | N | - | ** |
| Q-25 | H | SH | $OCH_3$ | $OCH_3$ | N | - | ** |
| Q-25 | H | $SCH_3$ | $CH_3$ | $CH_3$ | CH | - | ** |
| Q-25 | H | $SCH_3$ | $CH_3$ | $OCH_3$ | CH | - | ** |
| Q-25 | H | $SCH_3$ | $OCH_3$ | $OCH_3$ | CH | - | ** |
| Q-25 | H | $SCH_3$ | $OCH_3$ | Cl | CH | - | ** |
| Q-25 | H | $SCH_3$ | CH | $OCH_3$ | N | - | ** |
| Q-25 | H | $SCH_3$ | $OCH_3$ | $OCH_3$ | N | - | ** |
| Q-25 | H | $SCH_2CH=CH_2$ | $CH_3$ | $CH_3$ | CH | - | ** |
| Q-25 | H | $SCH_2CH=CH_2$ | $CH_3$ | $OCH_3$ | CH | - | ** |
| Q-25 | H | $SCH_2CH=CH_2$ | $OCH_3$ | $OCH_3$ | CH | - | ** |
| Q-25 | H | $SCH_2CH=CH_2$ | $OCH_3$ | Cl | CH | - | ** |
| Q-25 | H | $SCH_2CH=CH_2$ | $CH_3$ | $OCH_3$ | N | - | ** |
| Q-25 | H | $SCH_2CH=CH_2$ | $OCH_3$ | $OCH_3$ | N | - | ** |
| Q-25 | $CH_3$ | $SCH_3$ | $OCH_3$ | $OCH_3$ | CH | - | ** |
| Q-25 | $CH_3$ | SH | $OCH_3$ | $OCH_3$ | CH | - | ** |
| Q-25 | $CH_3$ | $SCH_2CH=CH_2$ | $OCH_3$ | $OCH_3$ | CH | - | ** |

Table 7e

General Structure 3

| Q | R | $R_3$ | $R_4$ | $R_5$ | X | Y | Z | m.p. °C |
|---|---|---|---|---|---|---|---|---|
| Q-29 | H | H | H | - | $CH_3$ | $OCH_3$ | CH | |
| Q-29 | H | H | H | - | $CH_3$ | $OCH_3$ | N | |
| Q-29 | H | H | H | - | $OCH_3$ | $OCH_3$ | CH | |
| Q-29 | H | H | H | - | $OCH_3$ | $OCH_3$ | N | |
| Q-29 | H | H | H | - | $CH_3$ | $CH_3$ | CH | |
| Q-29 | H | H | H | - | $CH_3$ | $CH_3$ | N | |
| Q-29 | H | $CH_3$ | H | - | $CH_3$ | $OCH_3$ | CH | |
| Q-29 | H | $CH_3$ | H | - | $CH_3$ | $CH_3$ | CH | |
| Q-29 | H | $CH_3$ | $CH_3$ | - | $CH_3$ | $OCH_3$ | N | |
| Q-29 | $CH_3$ | $CH_3$ | $CH_3$ | - | $CH_3$ | $CH_3$ | N | |
| Q-29 | $CH_3$ | H | H | - | $OCH_3$ | $OCH_3$ | CH | |
| Q-29 | H | H | H | - | $CH_3$ | $OC_2H_5$ | N | |
| Q-29 | H | H | H | - | Cl | $OCH_3$ | CH | |
| Q-36 | H | H | H | H | $CH_3$ | $OCH_3$ | CH | |
| Q-36 | $CH_3$ | H | H | H | $CH_3$ | $OCH_3$ | CH | |
| Q-36 | H | H | H | H | $CH_3$ | $OCH_3$ | N | |
| Q-36 | H | H | H | H | $OCH_3$ | $OCH_3$ | CH | |
| Q-36 | H | H | H | H | $OCH_3$ | $OCH_3$ | N | |
| Q-36 | H | H | H | H | $OCH_3$ | $C_2H_5$ | CH | |
| Q-36 | H | H | H | H | $CH_3$ | $CH_3$ | CH | |
| Q-36 | H | H | H | H | $CH_3$ | $CH_3$ | N | |
| Q-36 | H | $CH_3$ | H | H | $CH_3$ | $CH_3$ | CH | |
| Q-36 | H | H | $CH_3$ | H | $CH_3$ | $CH_3$ | N | |
| Q-36 | H | $CH_3$ | H | $CH_3$ | $CH_3$ | $OCH_3$ | CH | |
| Q-36 | H | $CH_3$ | $CH_3$ | H | $CH_3$ | $OCH_3$ | N | |
| Q-36 | H | $CH_3$ | $CH_3$ | $CH_3$ | $OCH_3$ | $OCH_3$ | CH | |
| Q-36 | $CH_3$ | H | H | H | $OCH_3$ | $OCH_3$ | N | |
| Q-36 | H | H | H | H | $CH_3$ | $NHCH_3$ | CH | |
| Q-36 | H | H | H | H | $CH_3$ | $N(CH_3)_2$ | CH | |
| Q-37 | H | H | H | - | $CH_3$ | $OCH_3$ | CH | |

wherein W' is O.

Table 7e (continued)

| Q | R | $R_3$ | $R_4$ | $R_5$ | X | Y | Z | m.p. °C |
|---|---|---|---|---|---|---|---|---|
| Q-37 | H | H | H | - | $CH_3$ | $OCH_3$ | N | |
| Q-37 | H | H | H | - | $OCH_3$ | $OCH_3$ | CH | |
| Q-37 | H | H | H | - | $OCH_3$ | $OCH_3$ | N | |
| Q-37 | H | H | H | - | $CH_3$ | $CH_3$ | CH | |
| Q-37 | H | 2-$CH_3$ | H | - | $CH_3$ | $CH_3$ | CH | |
| Q-37 | H | 4-$CH_3$ | H | - | $CH_3$ | $CH_3$ | N | |
| Q-37 | H | 5-$CH_3$ | H | - | $CH_3$ | OCH | CH | |
| Q-37 | H | H | H | - | $CH_3$ | $CH_3$ | N | |
| Q-37 | H | 2-$CH_3$ | 4-$CH_3$ | - | $CH_3$ | $OCH_3$ | N | |
| Q-37 | H | 2-$CH_3$ | 5-$CH_3$ | - | $OCH_3$ | $OCH_3$ | CH | |
| Q-37 | H | 4-$CH_3$ | 5-$CH_3$ | - | $OCH_3$ | $OCH_3$ | N | |
| Q-37 | $CH_3$ | H | H | - | $CH_3$ | $OCH_3$ | CH | |
| Q-37 | H | H | H | - | $CH_3$ | $CH(OCH_3)_2$ | CH | |
| Q-37 | H | H | H | - | Cl | $OCH_3$ | CH | |
| Q-38 | H | H | H | - | $CH_3$ | $OCH_3$ | CH | |
| Q-38 | H | H | H | - | $CH_3$ | $OCH_3$ | N | |
| Q-38 | H | H | H | - | $OCH_3$ | $OCH_3$ | CH | |
| Q-38 | H | H | H | - | $OCH_3$ | $OCH_3$ | N | |
| Q-38 | H | 2-$CH_3$ | H | - | $CH_3$ | $CH_3$ | CH | |
| Q-38 | H | 4-$CH_3$ | H | - | $CH_3$ | $CH_3$ | N | |
| Q-38 | H | 2-$CH_3$ | 4-$CH_3$ | - | $CH_3$ | $OCH_3$ | CH | |
| Q-38 | H | 4-$CH_3$ | 4-$CH_3$ | - | $CH_3$ | $OCH_3$ | N | |
| Q-38 | H | H | H | - | $CH_3$ | $CH_3$ | N | |
| Q-38 | $CH_3$ | 4-$CH_3$ | 6-$CH_3$ | - | $OCH_3$ | $OCH_3$ | CH | |
| Q-38 | H | H | H | - | $CH_3$ | 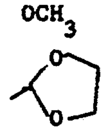 | CH | |
| Q-38 | H | H | H | - | $CH_3$ | $CH_3$ | CH | |
| Q-38 | H | H | H | - | $OCH_3$ | $C_2H_5$ | N | |
| Q-39 | H | H | H | - | $CH_3$ | $OCH_3$ | CH | |
| Q-39 | H | H | H | - | $CH_3$ | $OCH_3$ | N | |
| Q-39 | H | H | H | - | $CH_3$ | $CH_3$ | N | |

Table 7e (continued)

| Q | R | $R_3$ | $R_4$ | $R_5$ | X | Y | Z | m.p. °C |
|---|---|---|---|---|---|---|---|---|
| Q-39 | H | H | H | - | $OCH_3$ | $OCH_3$ | CH | |
| Q-39 | H | H | H | - | $OCH_3$ | $OCH_3$ | N | |
| Q-39 | $CH_3$ | H | H | - | $OCH_3$ | $OCH_3$ | N | |
| Q-39 | H | 3-$CH_3$ | H | - | $CH_3$ | $CH_3$ | CH | |
| Q-39 | H | H | H | - | $CH_3$ | $CH_3$ | CH | |
| Q-39 | H | 5-$CH_3$ | H | - | $CH_3$ | $CH_3$ | N | |
| Q-39 | H | H | H | - | Cl | $OC_2H_5$ | CH | |
| Q-39 | H | 6-$CH_3$ | H | - | $CH_3$ | $OCH_3$ | CH | |
| Q-39 | H | 3-$CH_3$ | 5-$CH_3$ | - | $CH_3$ | $OCH_3$ | N | |
| Q-39 | H | 3-$CH_3$ | 6-$CH_3$ | - | $OCH_3$ | $OCH_3$ | CH | |
| Q-39 | H | 5-$CH_3$ | 6-$CH_3$ | - | $OCH_3$ | $OCH_3$ | N | |
| Q-39 | H | H | H | - | $OCH_3$ | $OC_2H_5$ | N | |
| Q-39 | H | H | H | - | $OCH_3$ | $CH_2OCH_3$ | N | |
| Q-40 | H | H | H | - | $CH_3$ | $CH_3$ | CH | |
| Q-40 | H | H | H | - | $CH_3$ | $OCH_3$ | CH | |
| Q-40 | H | H | H | - | $CH_3$ | $OCH_3$ | N | |
| Q-40 | H | H | H | - | $OCH_3$ | $OCH_3$ | CH | |
| Q-40 | H | H | H | - | $OCH_3$ | $OCH_3$ | N | |
| Q-40 | H | H | H | - | $CH_3$ | $CH_3$ | N | |
| Q-40 | H | 4-$CH_3$ | H | - | $CH_3$ | $CH_3$ | CH | |
| Q-40 | H | 5-$CH_3$ | H | - | $CH_3$ | $CH_3$ | N | |
| Q-40 | H | 6-$CH_3$ | H | - | $CH_3$ | $OCH_3$ | CH | |
| Q-40 | H | 4-$CH_3$ | 5-$CH_3$ | - | $CH_3$ | $OCH_3$ | N | |
| Q-40 | H | 4-$CH_3$ | 6-$CH_3$ | - | $OCH_3$ | $OCH_3$ | CH | |
| Q-40 | H | 5-$CH_3$ | 6-$CH_3$ | - | $OCH_3$ | $OCH_3$ | N | |
| Q-40 | $CH_3$ | H | H | - | $CH_3$ | $CH_3$ | CH | |
| Q-40 | H | H | H | - | $OCH_3$ | $CF_3$ | N | |
| Q-43 | H | H | H | - | $CH_3$ | $CH_3$ | CH | |
| Q-43 | H | H | H | - | $CH_3$ | $OCH_3$ | CH | |
| Q-43 | H | H | H | - | $CH_3$ | $OCH_3$ | N | |

Table 7e (continued)

| Q | R | $R_3$ | $R_4$ | $R_5$ | X | Y | Z | m.p. °C |
|---|---|---|---|---|---|---|---|---|
| Q-43 | H | H | H | - | $OCH_3$ | $OCH_3$ | CH | |
| Q-43 | H | H | H | - | $OCH_3$ | $OCH_3$ | N | |
| Q-43 | $CH_3$ | H | H | - | $OCH_3$ | $OCH_3$ | CH | |
| Q-43 | H | $CH_3$ | $CH_3$ | - | $CH_3$ | $OCH_3$ | CH | |
| Q-43 | H | $CH_3$ | H | - | $CH_3$ | $CH_3$ | CH | |
| Q-43 | H | H | H | - | $CH_3$ | $CH_3$ | N | |
| Q-43 | H | H | H | - | $OCH_3$ | $OCH_2CF_3$ | N | |
| Q-43 | H | H | H | - | $OCH_3$ | $NHCH_3$ | N | |
| Q-44 | H | H | H | - | $CH_3$ | $OCH_3$ | CH | |
| Q-44 | H | H | H | - | $CH_3$ | $OCH_3$ | N | |
| Q-44 | H | H | H | - | $OCH_3$ | $OCH_3$ | CH | |
| Q-44 | H | H | H | - | $OCH_3$ | $OCH_3$ | N | |
| Q-44 | H | $CH_3$ | H | - | $CH_3$ | $CH_3$ | CH | |
| Q-44 | H | H | $CH_3$ | - | $CH_3$ | $CH_3$ | N | |
| Q-44 | H | $CH_3$ | $CH_3$ | - | $CH_3$ | $OCH_3$ | N | |
| Q-44 | $CH_3$ | H | H | - | $CH_3$ | $OCH_3$ | N | |
| Q-44 | H | H | H | - | $OCH_3$ | $N(CH_3)_2$ | N | |
| Q-44 | H | H | H | - | Br | $OCH_3$ | CH | |
| Q-44 | H | H | H | - | $CH_3$ | $CH_3$ | CH | |
| Q-44 | H | H | H | - | $CH_3$ | $CH_3$ | N | |
| Q-45 | H | H | - | - | $CH_3$ | $OCH_3$ | CH | |
| Q-45 | H | H | - | - | $CH_3$ | $OCH_3$ | N | |
| Q-45 | H | H | - | - | $OCH_3$ | $OCH_3$ | CH | |
| Q-45 | H | H | - | - | $OCH_3$ | $OCH_3$ | N | |
| Q-45 | H | $CH_3$ | - | - | $CH_3$ | $CH_3$ | CH | |
| Q-45 | $CH_3$ | H | - | - | $CH_3$ | $CH_3$ | N | |
| Q-45 | H | H | - | - | $OCH_3$ | $CH(OCH_3)_2$ | N | |
| Q-45 | H | H | - | - | $CH_3$ | $CH_3$ | CH | |
| Q-45 | H | H | - | - | $CH_3$ | $CH_3$ | N | |
| Q-45 | H | H | - | - | Cl | $OCH_3$ | CH | |

Table 8a

General Structure 3 wherein Q is Q-5.

| R | $R_3$ | $R_4$ | $R_5$ | $R_6$ | $R_7$ | $R_8$ | X | Y | Z | m.p. °C |
|---|---|---|---|---|---|---|---|---|---|---|
| H | H | H | H | H | H | H | $CH_3$ | $CH_3$ | N | |
| H | H | H | H | H | H | H | $CH_3$ | $OCH_3$ | CH | |
| H | H | H | H | H | H | H | $CH_3$ | $OCH_3$ | N | |
| H | H | H | H | H | H | H | $OCH_3$ | $OCH_3$ | CH | |
| H | H | H | H | H | H | H | $OCH_3$ | $OCH_3$ | N | |
| $CH_3$ | H | H | H | H | H | H | $OCH_3$ | $OCH_3$ | CH | |
| H | $CH_3$ | H | H | H | H | H | $CH_3$ | $CH_3$ | CH | |
| H | H | H | $CH_3$ | H | H | H | $CH_3$ | $CH_3$ | N | |
| H | H | H | H | H | $CH_3$ | H | $CH_3$ | $OCH_3$ | CH | |
| H | $CH_3$ | $CH_3$ | H | H | $CH_3$ | H | $CH_3$ | $OCH_3$ | N | |
| H | $CH_3$ | H | $CH_3$ | H | H | H | $OCH_3$ | $OCH_3$ | CH | |
| H | $CH_3$ | H | H | H | $CH_3$ | H | $OCH_3$ | $OCH_3$ | N | |
| H | H | H | $CH_3$ | H | $CH_3$ | H | $CH_3$ | $CH_3$ | CH | |
| H | $CH_3$ | H | $CH_3$ | H | $CH_3$ | H | $CH_3$ | $CH_3$ | N | |
| H | $CH_3$ | $CH_3$ | H | H | H | H | $CH_3$ | $OCH_3$ | CH | |
| H | H | H | H | $CH_3$ | H | H | $CH_3$ | $OCH_3$ | N | |
| H | H | H | H | H | $CH_3$ | $CH_3$ | $OCH_3$ | $OCH_3$ | CH | |
| H | $CH_3$ | $CH_3$ | $CH_3$ | H | H | H | $OCH_3$ | $OCH_3$ | N | |
| H | $CH_3$ | $CH_3$ | H | H | $CH_3$ | H | $CH_3$ | $CH_3$ | CH | |
| H | H | H | $CH_3$ | $CH_3$ | $CH_3$ | H | $CH_3$ | $CH_3$ | N | |
| H | $CH_3$ | H | $CH_3$ | $CH_3$ | $CH_3$ | H | $CH_3$ | $OCH_3$ | CH | |
| H | $CH_3$ | $CH_3$ | $CH_3$ | H | $CH_3$ | H | $CH_3$ | $OCH_3$ | N | |
| H | $CH_3$ | H | $CH_3$ | H | $CH_3$ | $CH_3$ | $OCH_3$ | $OCH_3$ | CH | |
| $CH_3$ | H | H | H | H | H | H | $CH_3$ | $OCH_3$ | N | |

Wherein W" is O.

Table 8a (continued)

| R | $R_3$ | $R_4$ | $R_5$ | $R_6$ | $R_7$ | $R_8$ | X | Y | Z | m.p. °C |
|---|---|---|---|---|---|---|---|---|---|---|
| H | H | H | H | H | H | H | $OCH_3$ | $CH(OCH_3)_2$ | N | |
| H | H | H | H | H | H | H | $OCH_3$ | 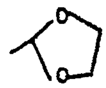 | N | |
| H | H | H | H | H | H | H | Cl | $OCH_3$ | CH | |
| H | H | H | H | H | H | H | $CH_3$ | $CH_3$ | CH | |

Wherein W" is O.

Table 8b

General Structure 3 wherein Q is Q-6.

| Q | R | $R_3$ | $R_4$ | $R_5$ | $R_6$ | $R_7$ | $R_8$ | X | Y | Z | W" | m.p. °C |
|---|---|---|---|---|---|---|---|---|---|---|---|---|
| Q-6 | H | H | H | H | H | H | H | $CH_3$ | $CH_3$ | CH | O | |
| Q-6 | H | H | H | H | H | H | H | $CH_3$ | $CH_3$ | N | O | |
| Q-6 | H | $CH_3$ | H | H | H | H | H | $CH_3$ | $OCH_3$ | CH | O | |
| Q-6 | H | H | H | $CH_3$ | H | H | H | $CH_3$ | $OCH_3$ | N | O | |
| Q-6 | H | H | H | H | H | $CH_3$ | H | $OCH_3$ | $OCH_3$ | CH | O | |
| Q-6 | H | $CH_3$ | H | $CH_3$ | H | H | H | $OCH_3$ | $OCH_3$ | N | O | |
| Q-6 | H | $CH_3$ | H | H | H | $CH_3$ | H | $CH_3$ | $CH_3$ | CH | O | |
| Q-6 | H | H | H | $CH_3$ | H | $CH_3$ | H | $CH_3$ | $CH_3$ | N | O | |
| Q-6 | H | $CH_3$ | $CH_3$ | H | H | H | H | $CH_3$ | $OCH_3$ | CH | O | |
| Q-6 | H | H | H | $CH_3$ | $CH_3$ | H | H | $CH_3$ | $OCH_3$ | N | O | |
| Q-6 | H | H | H | H | H | $CH_3$ | $CH_3$ | $OCH_3$ | $OCH_3$ | CH | O | |
| Q-6 | H | $CH_3$ | H | H | H | $CH_3$ | $CH_3$ | $OCH_3$ | $OCH_3$ | N | O | |
| Q-6 | H | $CH_3$ | $CH_3$ | H | H | $CH_3$ | H | $CH_3$ | $CH_3$ | CH | O | |
| Q-6 | H | $CH_3$ | H | $CH_3$ | $CH_3$ | H | H | $CH_3$ | $CH_3$ | N | O | |
| Q-6 | H | H | H | $CH_3$ | $CH_3$ | $CH_3$ | H | $CH_3$ | $OCH_3$ | CH | O | |
| Q-6 | H | $CH_3$ | $CH_3$ | $CH_3$ | H | H | H | $CH_3$ | $OCH_3$ | N | O | |
| Q-6 | H | H | H | $CH_3$ | $CH_3$ | $CH_3$ | H | $OCH_3$ | $OCH_3$ | CH | O | |
| Q-6 | H | $CH_3$ | H | $CH_3$ | $CH_3$ | $CH_3$ | H | $OCH_3$ | $OCH_3$ | N | O | |
| Q-6 | H | $CH_3$ | $CH_3$ | $CH_3$ | H | $CH_3$ | H | $CH_3$ | $CH_3$ | CH | O | |
| Q-6 | H | $CH_3$ | H | $CH_3$ | H | $CH_3$ | $CH_3$ | $CH_3$ | $CH_3$ | N | O | |
| Q-6 | H | $CH_3$ | $CH_3$ | $CH_3$ | $CH_3$ | H | H | $CH_3$ | $OCH_3$ | CH | O | |
| Q-6 | H | $CH_3$ | $CH_3$ | H | H | $CH_3$ | $CH_3$ | $CH_3$ | $OCH_3$ | N | O | |
| Q-6 | H | H | H | $CH_3$ | $CH_3$ | $CH_3$ | $CH_3$ | $OCH_3$ | $OCH_3$ | CH | O | |
| Q-6 | $CH_3$ | H | H | H | H | H | H | $CH_3$ | $OCH_3$ | N | O | |
| Q-6 | H | H | H | H | H | H | H | Br | $OCH_3$ | CH | O | |
| Q-6 | H | H | H | H | H | H | H | Cl | $OC_2H_5$ | CH | O | |

Table 8b (continued)

| Q | R | $R_3$ | $R_4$ | $R_5$ | $R_6$ | $R_7$ | $R_8$ | X | Y | Z | W" | m.p. °C |
|---|---|---|---|---|---|---|---|---|---|---|---|---|
| Q-6 | H | H | H | H | H | H | H | $OCH_3$ | $C_2H_5$ | CH | O | |
| Q-6 | H | H | H | H | H | H | H | $OCH_3$ | $OC_2H_5$ | CH | O | |
| Q-6 | H | H | H | H | H | H | H | $CH_3$ | $OCH_3$ | CH | O | |
| Q-6 | H | H | H | H | H | H | H | $CH_3$ | $OCH_3$ | N | O | |
| Q-6 | H | H | H | H | H | H | H | $OCH_3$ | $OCH_3$ | CH | O | |
| Q-6 | H | H | H | H | H | H | H | $OCH_3$ | $OCH_3$ | N | O | |
| Q-6 | $CH_3$ | H | H | H | H | H | H | $OCH_3$ | $OCH_3$ | CH | O | |
| Q-6 | H | H | H | H | H | H | H | $CH_3$ | $OCH_3$ | CH | S | |
| Q-6 | H | H | H | H | H | H | H | $CH_3$ | $OCH_3$ | N | S | |
| Q-6 | H | H | H | H | H | H | H | $OCH_3$ | $OCH_3$ | CH | S | |
| Q-6 | H | H | H | H | H | H | H | $OCH_3$ | $OCH_3$ | N | S | |
| Q-6 | H | H | H | H | H | H | H | $OCH_3$ | $C_2H_5$ | CH | S | |
| Q-6 | H | H | H | H | H | H | H | $CH_3$ | $CH_3$ | CH | S | |
| Q-6 | H | H | H | H | H | H | H | $CH_3$ | $CH_3$ | N | S | |
| Q-6 | H | $CH_3$ | H | H | H | H | H | $CH_3$ | $OCH_3$ | CH | S | |
| Q-6 | H | H | H | $CH_3$ | H | H | H | $CH_3$ | $OCH_3$ | N | S | |
| Q-6 | H | H | H | H | H | $CH_3$ | H | $OCH_3$ | $OCH_3$ | CH | S | |
| Q-6 | H | $CH_3$ | H | $CH_3$ | H | H | H | $OCH_3$ | $OCH_3$ | N | S | |
| Q-6 | H | $CH_3$ | H | H | H | $CH_3$ | H | $CH_3$ | $CH_3$ | CH | S | |
| Q-6 | H | H | H | $CH_3$ | H | $CH_3$ | H | $CH_3$ | $CH_3$ | N | S | |
| Q-6 | H | $CH_3$ | $CH_3$ | H | H | H | H | $CH_3$ | $OCH_3$ | CH | S | |
| Q-6 | H | H | H | $CH_3$ | $CH_3$ | H | H | $CH_3$ | $OCH_3$ | N | S | |
| Q-6 | H | H | H | H | H | H | H | $OCH_3$ | $CH_2OCH_3$ | CH | S | |
| Q-6 | H | H | H | H | H | H | H | $OCH_3$ | $CF_3$ | CH | S | |
| Q-6 | H | H | H | H | H | H | H | $OCH_3$ | $OCF_2H$ | CH | S | |
| Q-6 | H | H | H | H | H | $CH_3$ | $CH_3$ | $OCH_3$ | $OCH_3$ | CH | S | |
| Q-6 | H | $CH_3$ | H | H | H | $CH_3$ | $CH_3$ | $OCH_3$ | $OCH_3$ | N | S | |
| Q-6 | H | $CH_3$ | $CH_3$ | H | H | $CH_3$ | H | $CH_3$ | $CH_3$ | CH | S | |
| Q-6 | H | $CH_3$ | H | $CH_3$ | $CH_3$ | H | H | $CH_3$ | $CH_3$ | N | S | |
| Q-6 | H | H | H | $CH_3$ | $CH_3$ | $CH_3$ | H | $CH_3$ | $OCH_3$ | CH | S | |
| Q-6 | H | $CH_3$ | $CH_3$ | $CH_3$ | H | H | H | $CH_3$ | $OCH_3$ | N | S | |

Table 8b (continued)

| Q | R | $R_3$ | $R_4$ | $R_5$ | $R_6$ | $R_7$ | $R_8$ | X | Y | Z | W" | m.p. °C |
|---|---|---|---|---|---|---|---|---|---|---|---|---|
| Q-6 | H | H | H | $CH_3$ | $CH_3$ | $CH_3$ | H | $OCH_3$ | $OCH_3$ | CH | S | |
| Q-6 | H | $CH_3$ | H | $CH_3$ | $CH_3$ | $CH_3$ | H | $OCH_3$ | $OCH_3$ | N | S | |
| Q-6 | H | $CH_3$ | $CH_3$ | $CH_3$ | H | $CH_3$ | H | $CH_3$ | $CH_3$ | CH | S | |
| Q-6 | H | $CH_3$ | H | $CH_3$ | H | $CH_3$ | $CH_3$ | $CH_3$ | $CH_3$ | N | S | |
| Q-6 | H | $CH_3$ | $CH_3$ | $CH_3$ | $CH_3$ | H | H | $CH_3$ | $OCH_3$ | CH | S | |
| Q-6 | H | $CH_3$ | $CH_3$ | H | H | $CH_3$ | $CH_3$ | $CH_3$ | $OCH_3$ | N | S | |
| Q-6 | H | H | H | $CH_3$ | $CH_3$ | $CH_3$ | $CH_3$ | $OCH_3$ | $OCH_3$ | CH | S | |
| Q-6 | $CH_3$ | H | H | H | H | H | H | $CH_3$ | $OCH_3$ | N | S | |

Table 8c

General Structure 3

| Q | R | $R_3$ | $R_4$ | $R_5$ | $R_6$ | X | Y | Z | W' | m.p. °C |
|---|---|---|---|---|---|---|---|---|---|---|
| Q-7 | H | H | H | H | H | $CH_3$ | $OCH_3$ | CH | - | |
| Q-7 | H | H | H | H | H | $CH_3$ | $OCH_3$ | N | - | |
| Q-7 | H | H | H | H | H | $OCH_3$ | $OCH_3$ | CH | - | |
| Q-7 | H | H | H | H | H | $OCH_3$ | $OCH_3$ | N | - | |
| Q-7 | H | H | H | H | H | $CH_3$ | $CH_3$ | CH | - | |
| Q-7 | H | H | H | H | H | $CH_3$ | $CH_3$ | N | - | |
| Q-7 | H | $CH_3$ | H | H | H | $CH_3$ | $OCH_3$ | CH | - | |
| Q-7 | H | H | $CH_3$ | H | H | $CH_3$ | $OCH_3$ | N | - | |
| Q-7 | H | $CH_3$ | H | H | $CH_3$ | $OCH_3$ | $OCH_3$ | CH | - | |
| Q-7 | H | H | $CH_3$ | $CH_3$ | H | $OCH_3$ | $OCH_3$ | N | - | |
| Q-7 | H | $CH_3$ | $CH_3$ | H | H | $CH_3$ | $CH_3$ | CH | - | |
| Q-7 | H | $CH_3$ | $CH_3$ | $CH_3$ | H | $CH_3$ | $CH_3$ | N | - | |
| Q-7 | H | $CH_3$ | H | $CH_3$ | $CH_3$ | $CH_3$ | $OCH_3$ | CH | - | |
| Q-7 | H | $CH_3$ | $CH_3$ | $CH_3$ | $CH_3$ | $CH_3$ | $OCH_3$ | N | - | |
| Q-7 | $CH_3$ | H | H | H | H | $OCH_3$ | $OCH_3$ | CH | - | |
| Q-7 | $CH_3$ | $CH_3$ | $CH_3$ | $CH_3$ | $CH_3$ | $OCH_3$ | $OCH_3$ | CH | - | |
| Q-7 | H | H | H | H | H | $OCH_3$ | $OCH_2CF_3$ | CH | - | |
| Q-7 | H | H | H | H | H | $OCH_3$ | $NHCH_3$ | CH | - | |
| Q-7 | H | H | H | H | H | Cl | $OCH_3$ | CH | - | |
| Q-7 | H | H | H | H | H | $OCH_3$ | $OCH_3$ | CH | - | * |
| Q-7 | H | H | H | H | H | $OCH_3$ | $CH_3$ | N | - | * |
| Q-7 | H | H | H | H | H | $OCH_3$ | CH | CH | - | * |
| Q-16 | H | $CH_3$ | $CH_3$ | H | H | $CH_3$ | $OCH_3$ | CH | O | |
| Q-16 | H | $CH_3$ | $CH_3$ | H | H | $CH_3$ | $OCH_3$ | N | O | |
| Q-16 | H | $CH_3$ | $CH_3$ | H | H | $OCH_3$ | $OCH_3$ | CH | O | |
| Q-16 | H | $CH_3$ | $CH_3$ | H | H | $OCH_3$ | $OCH_3$ | N | O | |
| Q-16 | $CH_3$ | $CH_3$ | $CH_3$ | H | H | $CH_3$ | $OCH_3$ | N | O | |
| Q-16 | $CH_3$ | $CH_3$ | $CH_3$ | H | H | Cl | $OCH_3$ | CH | O | |
| Q-16 | H | $CH_3$ | $CH_3$ | H | H | $CH_3$ | $CH_3$ | CH | O | |
| Q-16 | H | $CH_3$ | $CH_3$ | H | H | $CH_3$ | $CH_3$ | N | O | |
| Q-16 | H | H | H | H | H | $CH_3$ | $OCH_3$ | CH | O | |
| Q-16 | H | H | H | H | H | $CH_3$ | $OCH_3$ | N | O | |

Table 8c (continued)

| Q | R | $R_3$ | $R_4$ | $R_5$ | $R_6$ | X | Y | Z | W' | °C |
|---|---|---|---|---|---|---|---|---|---|---|
| Q-16 | H | H | H | H | H | $OCH_3$ | $OCH_3$ | CH | O | |
| Q-16 | H | H | H | H | H | $OCH_3$ | $OCH_3$ | N | O | |
| Q-16 | H | $CH_3$ | $CH_3$ | H | H | $OCH_3$ | $N(CH_3)_2$ | CH | O | |
| Q-16 | H | $CH_3$ | $CH_3$ | H | H | $OCH_3$ | $CH(OCH_3)_2$ | CH | O | |
| Q-16 | H | $CH_3$ | $CH_3$ | H | H | Cl | $OCH_3$ | CH | O | |
| Q-16 | H | $CH_3$ | $CH_3$ | H | H | $CH_3$ | $CH_2OCH_3$ | N | O | |
| Q-16 | H | $CH_3$ | H | H | H | $CH_3$ | $CH_3$ | CH | O | |
| Q-16 | H | H | H | $CH_3$ | H | $CH_3$ | $CH_3$ | N | O | |
| Q-16 | H | $CH_3$ | H | $CH_3$ | H | $CH_3$ | $OCH_3$ | N | O | |
| Q-16 | H | H | H | $CH_3$ | $CH_3$ | $CH_3$ | $OCH_3$ | CH | O | |
| Q-16 | H | $CH_3$ | H | $CH_3$ | $CH_3$ | $OCH_3$ | $OCH_3$ | CH | O | |
| Q-16 | H | $CH_3$ | $CH_3$ | $CH_3$ | H | $OCH_3$ | $OCH_3$ | N | O | |
| Q-16 | H | $CH_3$ | $CH_3$ | $CH_3$ | H | $CH_3$ | $CH_3$ | CH | O | |
| Q-16 | H | $CH_3$ | $CH_3$ | $CH_3$ | $CH_3$ | $CH_3$ | $CH_3$ | N | O | |
| Q-16 | H | $CH_3$ | $CH_3$ | H | H | $CH_3$ | $CH_3$ | CH | S | |
| Q-16 | H | $CH_3$ | $CH_3$ | H | H | $CH_3$ | $CH_3$ | N | S | |
| Q-16 | H | $CH_3$ | $CH_3$ | H | H | $CH_3$ | $OCH_3$ | CH | S | |
| Q-16 | H | $CH_3$ | $CH_3$ | H | H | $CH_3$ | $OCH_3$ | N | S | |
| Q-16 | H | $CH_3$ | $CH_3$ | H | H | $OCH_3$ | $OCH_3$ | CH | S | |
| Q-16 | H | $CH_3$ | $CH_3$ | H | H | $OCH_3$ | $OCH_3$ | N | S | |
| Q-16 | H | $CH_3$ | $CH_3$ | H | H | Cl | $OCH_3$ | CH | S | |
| Q-16 | H | $CH_3$ | $CH_3$ | H | H | $CH_3$ | $NHCH_3$ | N | S | |
| Q-16 | H | $CH_3$ | $CH_3$ | H | H | $CH_3$ | $N(CH_3)_2$ | N | S | |
| Q-16 | H | H | H | H | H | $CH_3$ | $OCH_3$ | CH | S | |
| Q-16 | H | H | H | H | H | $CH_3$ | $OCH_3$ | N | S | |
| Q-16 | H | H | H | H | H | $OCH_3$ | $OCH_3$ | CH | S | |
| Q-16 | H | H | H | H | H | $OCH_3$ | $OCH_3$ | N | S | |
| Q-16 | $CH_3$ | H | H | H | H | $CH_3$ | $CH_3$ | CH | S | |
| Q-16 | H | $CH_3$ | H | H | H | $CH_3$ | $CH_3$ | N | S | |
| Q-16 | H | H | H | $CH_3$ | H | $CH_3$ | $OCH_3$ | N | S | |
| Q-16 | H | $CH_3$ | H | $CH_3$ | H | $CH_3$ | $OCH_3$ | CH | S | |
| Q-16 | H | H | H | $CH_3$ | $CH_3$ | $OCH_3$ | $OCH_3$ | CH | S | |

Table 8c (continued)

| Q | R | $R_3$ | $R_4$ | $R_5$ | $R_6$ | X | Y | Z | W' | m.p. °C |
|---|---|---|---|---|---|---|---|---|---|---|
| Q-16 | H | $CH_3$ | $CH_3$ | $CH_3$ | H | $OCH_3$ | $OCH_3$ | N | S | |
| Q-16 | H | $CH_3$ | $CH_3$ | $CH_3$ | H | $CH_3$ | $CH_3$ | CH | S | |
| Q-16 | H | $CH_3$ | H | $CH_3$ | $CH_3$ | $CH_3$ | $CH_3$ | N | S | |
| Q-16 | H | $CH_3$ | $CH_3$ | $CH_3$ | $CH_3$ | $CH_3$ | $OCH_3$ | CH | S | |
| Q-17 | H | H | H | H | H | Cl | $OCH_3$ | CH | - | |
| Q-17 | H | H | H | H | H | $CH_3$ | $CH_3$ | CH | - | |
| Q-17 | H | H | H | H | H | Cl | $OC_2H_5$ | CH | - | |
| Q-17 | H | H | H | H | H | $CH_3$ | $CH_3$ | N | - | |
| Q-17 | H | H | H | H | H | $OCH_3$ | $OCH_3$ | CH | - | |
| Q-17 | H | H | H | H | H | $OCH_3$ | $OCH_3$ | N | - | |
| Q-17 | H | H | H | H | H | $OCH_3$ | $OC_2H_5$ | CH | - | |
| Q-17 | H | H | H | H | H | $OCH_3$ | $OC_2H_5$ | N | - | |
| Q-17 | H | $CH_3$ | H | H | H | $CH_3$ | $CH_3$ | CH | - | |
| Q-17 | H | H | H | H | H | $CH_3$ | $CH(OCH_3)_2$ | N | - | |
| Q-17 | H | H | H | H | H | $CH_3$ | 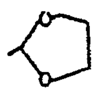 | N | - | |
| Q-17 | H | H | H | $CH_3$ | H | $CH_3$ | $CH_3$ | N | - | |
| Q-17 | H | $CH_3$ | $CH_3$ | H | H | $CH_3$ | $OCH_3$ | CH | - | |
| Q-17 | H | $CH_3$ | $CH_3$ | H | H | $CH_3$ | $OCH_3$ | N | - | |
| Q-17 | H | H | H | $CH_3$ | $CH_3$ | $OCH_3$ | $OCH_3$ | CH | - | |
| Q-17 | H | $CH_3$ | H | $CH_3$ | $CH_3$ | $OCH_3$ | $OCH_3$ | N | - | |
| Q-17 | H | $CH_3$ | $CH_3$ | $CH_3$ | H | $CH_3$ | $CH_3$ | CH | - | |
| Q-17 | H | $CH_3$ | $CH_3$ | $CH_3$ | H | $CH_3$ | $CH_3$ | N | - | |
| Q-17 | H | $CH_3$ | $CH_3$ | $CH_3$ | $CH_3$ | $CH_3$ | $OCH_3$ | CH | - | |
| Q-17 | $CH_3$ | H | H | H | H | $CH_3$ | $OCH_3$ | N | - | |

**Table 8d**

**General Structure 3 wherein Q is Q-30**

| R | $R_3$ | $R_4$ | $R_5$ | $R_6$ | $R_7$ | $R_8$ | $R_9$ | X | Y | Z | m.p. °C |
|---|---|---|---|---|---|---|---|---|---|---|---|
| H | H | H | H | H | H | H | H | $CH_3$ | $OCH_3$ | CH | |
| H | H | H | H | H | H | H | H | $CH_3$ | $OCH_3$ | N | |
| H | H | H | H | H | H | H | H | $OCH_3$ | $OCH_3$ | CH | |
| H | H | H | H | H | H | H | H | $OCH_3$ | $OCH_3$ | N | |
| H | H | H | H | H | H | H | H | $CH_3$ | $CH_3$ | N | |
| H | H | H | H | H | H | H | H | $CH_3$ | $CH_3$ | CH | |
| $CH_3$ | H | H | H | H | H | H | H | $CH_3$ | $OCH_3$ | CH | |
| H | $CH_3$ | H | H | H | H | H | H | $CH_3$ | $OCH_3$ | N | |
| H | H | H | H | H | H | H | H | Cl | $OCH_3$ | CH | |
| H | H | $CH_3$ | H | H | H | H | H | $OCH_3$ | $OCH_3$ | CH | |
| H | H | H | H | $CH_3$ | H | H | H | $OCH_3$ | $OCH_3$ | N | |
| H | H | H | H | H | H | $CH_3$ | H | $CH_3$ | $CH_3$ | CH | |
| H | $CH_3$ | $CH_3$ | H | H | H | H | H | $CH_3$ | $CH_3$ | N | |
| H | $CH_3$ | H | H | $CH_3$ | H | H | H | $CH_3$ | $OCH_3$ | CH | |
| H | $CH_3$ | H | H | H | H | $CH_3$ | H | $CH_3$ | $OCH_3$ | N | |
| H | H | $CH_3$ | $CH_3$ | H | H | H | H | $OCH_3$ | $OCH_3$ | CH | |
| H | H | $CH_3$ | H | $CH_3$ | H | H | H | $OCH_3$ | $OCH_3$ | N | |
| H | H | $CH_3$ | H | H | H | $CH_3$ | H | $CH_3$ | $CH_3$ | CH | |
| H | H | H | H | $CH_3$ | $CH_3$ | H | H | $CH_3$ | $CH_3$ | N | |
| H | H | H | H | $CH_3$ | H | $CH_3$ | H | $CH_3$ | $OCH_3$ | CH | |
| H | H | H | H | H | H | $CH_3$ | $CH_3$ | $CH_3$ | $OCH_3$ | N | |
| H | $CH_3$ | $CH_3$ | $CH_3$ | H | H | H | H | $OCH_3$ | $OCH_3$ | CH | |
| H | $CH_3$ | $CH_3$ | H | $CH_3$ | H | H | H | $OCH_3$ | $OCH_3$ | N | |
| H | $CH_3$ | $CH_3$ | H | H | H | $CH_3$ | H | $CH_3$ | $CH_3$ | CH | |
| H | $CH_3$ | H | H | H | $CH_3$ | H | H | $CH_3$ | $CH_3$ | N | |
| H | $CH_3$ | H | H | $CH_3$ | H | $CH_3$ | H | $CH_3$ | $OCH_3$ | CH | |
| H | $CH_3$ | H | H | H | H | $CH_3$ | $CH_3$ | $CH_3$ | $OCH_3$ | N | |
| H | H | $CH_3$ | $CH_3$ | $CH_3$ | H | H | H | $OCH_3$ | $OCH_3$ | CH | |
| H | H | $CH_3$ | $CH_3$ | H | H | $CH_3$ | H | $OCH_3$ | $OCH_3$ | N | |
| H | H | $CH_3$ | H | $CH_3$ | $CH_3$ | H | H | $CH_3$ | $CH_3$ | CH | |
| H | H | $CH_3$ | H | $CH_3$ | H | $CH_3$ | H | $CH_3$ | $CH_3$ | N | |
| H | H | $CH_3$ | H | H | H | $CH_3$ | $CH_3$ | $CH_3$ | $OCH_3$ | CH | |

Table 8d (continued)

| R | $R_3$ | $R_4$ | $R_5$ | $R_6$ | $R_7$ | $R_8$ | $R_9$ | X | Y | Z | m.p. °C |
|---|---|---|---|---|---|---|---|---|---|---|---|
| H | H | H | H | $CH_3$ | $CH_3$ | $CH_3$ | H | $CH_3$ | $OCH_3$ | N | |
| H | H | H | H | $CH_3$ | H | $CH_3$ | $CH_3$ | $OCH_3$ | $OCH_3$ | CH | |
| H | H | H | H | H | H | H | H | $CH_3$ | $OC_2H_5$ | CH | |
| H | H | H | H | H | H | H | H | $CH_3$ | $CH_2OCH_3$ | CH | |

**Table 8e**

**General Structure 3 wherein Q is Q-31.**

| R | $R_3$ | $R_4$ | $R_5$ | $R_6$ | $R_7$ | $R_8$ | $R_9$ | $R_{10}$ | X | Y | Z | m.p. °C |
|---|---|---|---|---|---|---|---|---|---|---|---|---|
| H | H | H | H | H | H | H | H | H | $CH_3$ | $OCH_3$ | CH | |
| H | H | H | H | H | H | H | H | H | $CH_3$ | $OCH_3$ | N | |
| H | H | H | H | H | H | H | H | H | $OCH_3$ | $OCH_3$ | CH | |
| H | H | H | H | H | H | H | H | H | $OCH_3$ | $OCH_3$ | N | |
| H | H | H | H | H | H | H | H | H | $CH_3$ | $CH_3$ | CH | |
| H | H | H | H | H | H | H | H | H | $CH_3$ | $CH_3$ | N | |
| H | H | H | H | H | H | H | H | H | Cl | $OCH_3$ | CH | |
| H | H | H | H | H | H | H | H | H | $CH_3$ | $C_2H_5$ | CH | |
| H | H | H | H | H | H | H | H | H | $CH_3$ | $CF_3$ | CH | |
| H | H | H | H | H | H | H | H | H | $CH_3$ | $OCF_2H$ | CH | |
| H | H | H | H | H | H | H | H | H | $CH_3$ | $OCH_2CF_3$ | CH | |
| H | $CH_3$ | H | H | H | H | H | H | H | $CH_3$ | $CH_3$ | CH | |
| H | H | H | $CH_3$ | H | H | H | H | H | $CH_3$ | $CH_3$ | N | |
| H | H | H | H | H | $CH_3$ | H | H | H | $CH_3$ | $OCH_3$ | CH | |
| H | H | H | H | H | H | H | $CH_3$ | H | $CH_3$ | $OCH_3$ | N | |
| H | $CH_3$ | $CH_3$ | H | H | H | H | H | H | $OCH_3$ | $OCH_3$ | CH | |
| H | H | H | $CH_3$ | $CH_3$ | H | H | H | H | $OCH_3$ | $OCH_3$ | N | |
| H | H | H | H | H | $CH_3$ | $CH_3$ | H | H | $CH_3$ | $CH_3$ | CH | |
| H | H | H | H | H | H | H | $CH_3$ | $CH_3$ | $CH_3$ | $CH_3$ | N | |
| H | H | H | $CH_3$ | H | H | H | $CH_3$ | H | $CH_3$ | $OCH_3$ | CH | |
| H | $CH_3$ | H | H | H | $CH_3$ | H | H | H | $CH_3$ | $OCH_3$ | N | |
| H | $CH_3$ | H | $CH_3$ | H | H | H | H | H | $OCH_3$ | $OCH_3$ | CH | |
| H | H | H | H | H | $CH_3$ | H | $CH_3$ | H | $OCH_3$ | $OCH_3$ | N | |
| H | $CH_3$ | $CH_3$ | $CH_3$ | H | H | H | H | H | $CH_3$ | $CH_3$ | CH | |
| H | $CH_3$ | H | H | H | $CH_3$ | $CH_3$ | H | H | $CH_3$ | $CH_3$ | N | |
| H | H | H | $CH_3$ | $CH_3$ | H | H | $CH_3$ | H | $CH_3$ | $OCH_3$ | CH | |
| H | H | H | $CH_3$ | $CH_3$ | $CH_3$ | H | H | H | $CH_3$ | $OCH_3$ | N | |
| H | $CH_3$ | H | H | H | $CH_3$ | H | $CH_3$ | H | $OCH_3$ | $OCH_3$ | CH | |

Table 8e (continued)

| R | $R_3$ | $R_4$ | $R_5$ | $R_6$ | $R_7$ | $R_8$ | $R_9$ | $R_{10}$ | X | Y | Z | m.p. °C |
|---|---|---|---|---|---|---|---|---|---|---|---|---|
| $CH_3$ | H | H | H | H | H | H | H | H | $OCH_3$ | $OCH_3$ | N | |
| H | H | H | H | H | H | H | H | H | $CH_3$ | $OCH_3$ | CH | * |
| H | H | H | H | H | H | H | H | H | $CH_3$ | $OCH_3$ | N | * |
| H | H | H | H | H | H | H | H | H | $OCH_3$ | $OCH_3$ | CH | * |
| H | H | H | H | H | H | H | H | H | $OCH_3$ | $OCH_3$ | N | * |
| H | H | H | H | H | H | H | H | H | $CH_3$ | $CH_3$ | CH | * |
| H | H | H | H | H | H | H | H | H | $CH_3$ | $CH_3$ | N | * |
| H | H | H | H | H | H | H | H | H | Cl | $OCH_3$ | CH | * |

***Wherein W" is O, unless indicated by **** where W" is S in all Tables.

**Table 8f**

**General Structure 3 wherein Q is Q-32.**

| R | $R_3$ | $R_4$ | $R_5$ | $R_6$ | $R_7$ | $R_8$ | $R_9$ | $R_{10}$ | X | Y | Z | W" |
|---|---|---|---|---|---|---|---|---|---|---|---|---|
| H | H | H | H | H | H | H | H | H | $CH_3$ | $CH_3$ | CH | O |
| H | H | H | H | H | H | H | H | H | $CH_3$ | $CH_3$ | N | O |
| H | H | H | H | H | H | H | H | H | Cl | $OCH_3$ | CH | O |
| H | H | H | H | H | H | H | H | H | $CH_3$ | $NHCH_3$ | CH | O |
| H | H | H | H | H | H | H | H | H | $CH_3$ | $N(CH_3)_2$ | CH | O |
| H | H | H | H | H | H | H | H | H | $CH_3$ | $CH(OCH_3)_2$ | CH | O |
| H | H | H | H | H | H | H | H | H | Br | $OCH_3$ | CH | O |
| H | $CH_3$ | H | H | H | H | H | H | H | $CH_3$ | $CH_3$ | CH | O |
| H | H | H | $CH_3$ | H | H | H | H | H | $CH_3$ | $CH_3$ | N | O |
| H | H | H | H | H | $CH_3$ | H | H | H | $CH_3$ | $OCH_3$ | CH | O |
| H | H | H | H | H | H | H | $CH_3$ | H | $CH_3$ | $OCH_3$ | N | O |
| H | $CH_3$ | $CH_3$ | H | H | H | H | H | H | $OCH_3$ | $OCH_3$ | CH | O |
| H | H | H | $CH_3$ | $CH_3$ | H | H | H | H | $OCH_3$ | $OCH_3$ | N | O |
| H | H | H | H | H | $CH_3$ | $CH_3$ | H | H | $CH_3$ | $CH_3$ | CH | O |
| H | H | H | H | H | H | H | $CH_3$ | $CH_3$ | $CH_3$ | $CH_3$ | N | O |
| H | H | H | $CH_3$ | H | H | H | $CH_3$ | H | $CH_3$ | $OCH_3$ | CH | O |
| H | $CH_3$ | H | H | H | $CH_3$ | H | H | H | $CH_3$ | $OCH_3$ | N | O |
| H | $CH_3$ | H | $CH_3$ | H | H | H | H | H | $OCH_3$ | $OCH_3$ | CH | O |
| H | H | H | H | H | $CH_3$ | H | $CH_3$ | H | $OCH_3$ | $OCH_3$ | N | O |
| H | $CH_3$ | $CH_3$ | $CH_3$ | H | H | H | H | H | $CH_3$ | $CH_3$ | CH | O |
| H | $CH_3$ | H | H | H | $CH_3$ | $CH_3$ | H | H | $CH_3$ | $CH_3$ | N | O |
| H | H | H | $CH_3$ | $CH_3$ | H | H | $CH_3$ | H | $CH_3$ | $OCH_3$ | CH | O |
| H | H | H | $CH_3$ | $CH_3$ | $CH_3$ | H | H | H | $CH_3$ | $OCH_3$ | N | O |
| H | $CH_3$ | H | H | H | $CH_3$ | H | $CH_3$ | H | $OCH_3$ | $OCH_3$ | CH | O |

**Table 8f (continued)**

| R | $R_3$ | $R_4$ | $R_5$ | $R_6$ | $R_7$ | $R_8$ | $R_9$ | $R_{10}$ | X | Y | Z | W" |
|---|---|---|---|---|---|---|---|---|---|---|---|---|
| $CH_3$ | H | H | H | H | H | H | H | H | $OCH_3$ | $OCH_3$ | N | O |
| H | $CH_3$ | H | H | H | H | H | $CH_3$ | H | $CH_3$ | $OCH_3$ | CH | O |
| H | H | H | H | H | H | H | H | H | $CH_3$ | $OCH_3$ | CH | O |
| H | H | H | H | H | H | H | H | H | $CH_3$ | $OCH_3$ | N | O |
| H | H | H | H | H | H | H | H | H | $OCH_3$ | $OCH_3$ | CH | O |
| H | H | H | H | H | H | H | H | H | $OCH_3$ | $OCH_3$ | N | O |
| H | H | H | H | H | H | H | H | H | $CH_3$ | $OCH_3$ | CH | S |
| H | H | H | H | H | H | H | H | H | $CH_3$ | $OCH_3$ | N | S |
| H | H | H | H | H | H | H | H | H | $OCH_3$ | $OCH_3$ | CH | S |
| H | H | H | H | H | H | H | H | H | $OCH_3$ | $OCH_3$ | N | S |
| H | H | H | H | H | H | H | H | H | $CH_3$ | $CH_3$ | CH | S |
| H | H | H | H | H | H | H | H | H | $CH_3$ | $CH_3$ | N | S |
| H | H | H | H | H | H | H | H | H | Cl | $OCH_3$ | CH | S |
| H | H | H | H | H | H | H | H | H | $CH_3$ | 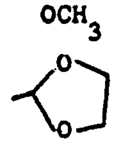 | CH | S |
| H | H | H | H | H | H | H | H | H | Cl | $OC_2H_5$ | CH | S |
| H | H | H | H | H | H | H | H | H | $CH_3$ | $C_2H_5$ | N | S |
| H | H | H | H | H | H | H | H | H | $CH_3$ | $OC_2H_5$ | N | S |
| H | $CH_3$ | H | H | H | H | H | H | H | $CH_3$ | $CH_3$ | CH | S |
| H | H | H | $CH_3$ | H | H | H | H | H | $CH_3$ | $CH_3$ | N | S |
| H | H | H | H | H | $CH_3$ | H | H | H | $CH_3$ | $OCH_3$ | CH | S |
| H | H | H | H | H | H | H | $CH_3$ | H | $CH_3$ | $OCH_3$ | N | S |
| H | $CH_3$ | $CH_3$ | H | H | H | H | H | H | $OCH_3$ | $OCH_3$ | CH | S |
| H | H | H | $CH_3$ | $CH_3$ | H | H | H | H | $OCH_3$ | $OCH_3$ | N | S |
| H | H | H | H | H | $CH_3$ | $CH_3$ | H | H | $CH_3$ | $CH_3$ | CH | S |

Table 8f (continued)

| R | $R_3$ | $R_4$ | $R_5$ | $R_6$ | $R_7$ | $R_8$ | $R_9$ | $R_{10}$ | X | Y | Z | W" |
|---|---|---|---|---|---|---|---|---|---|---|---|---|
| H | H | H | H | H | H | H | $CH_3$ | $CH_3$ | $CH_3$ | $CH_3$ | N | S |
| H | H | H | $CH_3$ | H | H | H | $CH_3$ | H | $CH_3$ | $OCH_3$ | CH | S |
| H | $CH_3$ | H | H | H | $CH_3$ | H | H | H | $CH_3$ | $OCH_3$ | N | S |
| H | $CH_3$ | H | $CH_3$ | H | H | H | H | H | $OCH_3$ | $OCH_3$ | CH | S |
| H | H | H | H | H | $CH_3$ | H | $CH_3$ | H | $OCH_3$ | $OCH_3$ | N | S |
| H | $CH_3$ | $CH_3$ | $CH_3$ | H | H | H | H | H | $CH_3$ | $CH_3$ | CH | S |
| H | $CH_3$ | H | H | H | $CH_3$ | $CH_3$ | H | H | $CH_3$ | $CH_3$ | N | S |
| H | H | H | $CH_3$ | $CH_3$ | H | H | $CH_3$ | H | $CH_3$ | $OCH_3$ | CH | S |
| H | H | H | $CH_3$ | $CH_3$ | $CH_3$ | H | H | H | $CH_3$ | $OCH_3$ | N | S |
| H | $CH_3$ | H | H | H | $CH_3$ | H | $CH_3$ | H | $OCH_3$ | $OCH_3$ | CH | S |
| $CH_3$ | H | H | H | H | H | H | H | H | $OCH_3$ | $OCH_3$ | N | S |
| H | $CH_3$ | H | H | H | H | H | $CH_3$ | H | $CH_3$ | $OCH_3$ | CH | S |

Table 8g

General Structure 3

| Q | R | $R_3$ | $R_4$ | $R_5$ | $R_6$ | $R_7$ | X | Y | Z | m.p. °C |
|---|---|---|---|---|---|---|---|---|---|---|
| Q-41 | H | H | H | H | H | H | Cl | $OCH_3$ | CH | |
| Q-41 | H | H | H | H | H | H | Br | $OCH_3$ | CH | |
| Q-41 | H | H | H | H | H | H | Cl | $OC_2H_5$ | CH | |
| Q-41 | H | H | H | H | H | H | $CH_3$ | $CH_3$ | N | |
| Q-41 | H | H | H | H | H | H | $OCH_3$ | $OCH_3$ | CH | |
| Q-41 | H | H | H | H | H | H | $OCH_3$ | $OCH_3$ | N | |
| Q-41 | H | H | H | H | H | H | $OCH_3$ | $OC_2H_5$ | CH | |
| Q-41 | H | H | H | H | H | H | $OCH_3$ | $OC_2H_5$ | N | |
| Q-41 | H | H | H | H | H | H | $CH_3$ | $CH_3$ | CH | |
| Q-41 | H | H | H | H | H | H | $CH_3$ | $OCH_3$ | N | |
| Q-41 | H | H | H | H | H | H | $CH_3$ | $OCH_3$ | CH | |
| Q-41 | H | H | H | H | H | H | $CH_3$ | $CH_2OCH_3$ | CH | |
| Q-41 | H | H | H | H | H | H | $CH_3$ | $CF_3$ | N | |
| Q-41 | H | $CH_3$ | H | H | H | H | $CH_3$ | $CH_3$ | CH | |
| Q-41 | H | H | H | $CH_3$ | H | H | $CH_3$ | $CH_3$ | N | |
| Q-41 | H | H | H | H | $CH_3$ | H | $CH_3$ | $OCH_3$ | CH | |
| Q-41 | H | $CH_3$ | $CH_3$ | H | H | H | $CH_3$ | $OCH_3$ | N | |
| Q-41 | H | $CH_3$ | $CH_3$ | H | H | H | $OCH_3$ | $OCH_3$ | CH | |
| Q-41 | H | $CH_3$ | $CH_3$ | H | H | H | $OCH_3$ | $OCH_3$ | N | |
| Q-41 | H | $CH_3$ | H | $CH_3$ | H | H | $CH_3$ | $CH_3$ | CH | |
| Q-41 | H | $CH_3$ | H | H | $CH_3$ | H | $CH_3$ | $CH_3$ | N | |
| Q-41 | H | H | H | $CH_3$ | $CH_3$ | H | $CH_3$ | $OCH_3$ | CH | |
| Q-41 | H | H | H | H | $CH_3$ | $CH_3$ | $CH_3$ | $OCH_3$ | N | |
| Q-41 | H | $CH_3$ | $CH_3$ | $CH_3$ | H | H | $OCH_3$ | $OCH_3$ | CH | |
| Q-41 | H | $CH_3$ | $CH_3$ | H | $CH_3$ | H | $OCH_3$ | $OCH_3$ | N | |
| Q-41 | H | $CH_3$ | H | $CH_3$ | $CH_3$ | H | $CH_3$ | $CH_3$ | CH | |
| Q-41 | H | $CH_3$ | H | H | $CH_3$ | $CH_3$ | $CH_3$ | $CH_3$ | N | |
| Q-41 | H | $CH_3$ | $CH_3$ | $CH_3$ | $CH_3$ | H | $CH_3$ | $OCH_3$ | CH | |
| Q-41 | H | $CH_3$ | $CH_3$ | H | $CH_3$ | $CH_3$ | $CH_3$ | $OCH_3$ | N | |

Table 8g (continued)

| Q | R | $R_3$ | $R_4$ | $R_5$ | $R_6$ | $R_7$ | X | Y | Z | m.p. •C |
|---|---|---|---|---|---|---|---|---|---|---|
| Q-41 | H | $CH_3$ | H | $CH_3$ | $CH_3$ | $CH_3$ | $OCH_3$ | $OCH_3$ | CH | |
| Q-41 | $CH_3$ | H | H | H | H | H | $CH_3$ | $CH_3$ | CH | |
| Q-41 | $CH_3$ | H | H | H | H | H | $CH_3$ | $OCH_3$ | CH | |

**Table 9a**

**General Structure 3 wherein Q is Q-20.**

| R | $R'_3$ | X | Y | Z | W' | m.p. °C |
|---|---|---|---|---|---|---|
| H | H | $CH_3$ | $OCH_3$ | CH | O | |
| H | H | $CH_3$ | $OCH_3$ | N | O | |
| H | H | $OCH_3$ | $OCH_3$ | CH | O | |
| H | H | $OCH_3$ | Cl | CH | O | |
| H | H | $OCH_3$ | $OCH_3$ | N | O | |
| H | H | $CH_3$ | $CH_3$ | CH | O | |
| H | $CH_3$ | $CH_3$ | $OCH_3$ | CH | O | |
| $CH_3$ | H | $CH_3$ | $OCH_3$ | N | O | |
| $CH_3$ | $CH_3$ | $OCH_3$ | $OCH_3$ | CH | O | |
| H | H | $CH_3$ | $OC_2H_5$ | N | O | |
| H | H | $CH_3$ | $CH_3$ | CH | S | |
| H | H | $CH_3$ | $CH_3$ | N | S | |
| H | H | $CH_3$ | $OCH_3$ | CH | S | |
| H | H | $CH_3$ | $OCH_3$ | N | S | |
| H | H | $OCH_3$ | $OCH_3$ | CH | S | |
| H | H | $OCH_3$ | $OCH_3$ | N | S | |
| $CH_3$ | H | $CH_3$ | $OCH_3$ | CH | S | |
| H | $CH_3$ | $CH_3$ | $OCH_3$ | N | S | |
| H | $CH_3$ | $CH_3$ | $CH_2OCH_3$ | N | S | |
| H | $C_2H_5$ | $CH_3$ | $CH_3$ | CH | O | |
| H | $C_2H_5$ | $CH_3$ | $CH_3$ | N | O | |
| H | $C_2H_5$ | $CH_3$ | $OCH_3$ | CH | O | |
| H | $C_2H_5$ | $CH_3$ | $OCH_3$ | N | O | |
| H | $C_2H_5$ | $OCH_3$ | $OCH_3$ | CH | O | |
| H | $C_2H_5$ | $OCH_3$ | $OCH_3$ | N | O | |
| H | $C_2H_5$ | $OCH_3$ | Cl | CH | O | |
| H | $C_2H_5$ | $CH_3$ | $CH_3$ | CH | S | |
| H | $C_2H_5$ | $CH_3$ | $CH_3$ | N | S | |
| H | $C_2H_5$ | $CH_3$ | $OCH_3$ | CH | S | |
| H | $C_2H_5$ | $CH_3$ | $OCH_3$ | N | S | |
| H | $C_2H_5$ | $OCH_3$ | $OCH_3$ | CH | S | |
| H | $C_2H_5$ | $OCH_3$ | $OCH_3$ | N | S | |

Table 9a (continued)

| R | R'3 | X | Y | Z | W' | m.p. °C |
|---|---|---|---|---|---|---|
| H | $C_2H_5$ | $OCH_3$ | Cl | CH | S | |
| H | $C_2H_5$ | $OCH_3$ | $N(CH_3)_2$ | N | S | |
| $CH_3$ | n-$C_3H_7$ | $OCH_3$ | $OCH_3$ | CH | O | |
| $CH_3$ | n-$C_3H_7$ | $CH_3$ | $CH_3$ | CH | O | |
| $CH_3$ | n-$C_3H_7$ | $CH_3$ | $OCH_3$ | CH | O | |
| $CH_3$ | n-$C_3H_7$ | $OCH_3$ | $OCH_3$ | CH | O | |
| $CH_3$ | n-$C_3H_7$ | $OCH_3$ | $OCH_3$ | N | O | |
| $CH_3$ | n-$C_3H_7$ | $OCH_3$ | Cl | CH | O | |
| $CH_3$ | n-$C_3H_7$ | $CH_3$ | $OCH_2CF_3$ | CH | O | |
| H | n-$C_3H_7$ | $CH_3$ | $CH_3$ | CH | S | |
| H | n-$C_3H_7$ | $CH_3$ | $OCH_3$ | CH | S | |
| H | n-$C_3H_7$ | $CH_3$ | $OCH_3$ | N | S | |
| H | n-$C_3H_7$ | $OCH_3$ | $OCH_3$ | CH | S | |
| H | n-$C_3H_7$ | $OCH_3$ | $OCH_3$ | N | S | |
| H | n-$C_3H_7$ | $OCH_3$ | Cl | CH | S | |
| H | n-$C_3H_7$ | $CH_3$ | $CH(OCH_3)_2$ | CH | S | |
| H | i-$C_3H_7$ | $CH_3$ | $CH_3$ | CH | O | |
| H | i-$C_3H_7$ | $CH_3$ | $OCH_3$ | CH | O | |
| H | i-$C_3H_7$ | $CH_3$ | $OCH_3$ | N | O | |
| H | i-$C_3H_7$ | $OCH_3$ | $OCH_3$ | CH | O | |
| H | i-$C_3H_7$ | $OCH_3$ | $OCH_3$ | N | O | |
| H | i-$C_3H_7$ | $OCH_3$ | Cl | CH | O | |
| H | i-$C_3H_7$ | $CH_3$ | $CH_3$ | N | O | |
| H | i-$C_3H_7$ | $OCH_3$ | 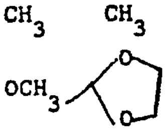 | CH | O | |
| $CH_3$ | i-$C_3H_7$ | $OCH_3$ | $OCH_3$ | CH | S | |
| H | i-$C_3H_7$ | $CH_3$ | $CH_3$ | CH | S | |
| H | i-$C_3H_7$ | $CH_3$ | $OCH_3$ | CH | S | |
| H | i-$C_3H_7$ | $CH_3$ | $OCH_3$ | N | S | |
| H | i-$C_3H_7$ | $OCH_3$ | $OCH_3$ | CH | S | |
| H | i-$C_3H_7$ | $OCH_3$ | $OCH_3$ | N | S | |

Table 9a (continued)

| R | R'3 | X | Y | Z | W' | m.p. °C |
|---|---|---|---|---|---|---|
| H | i-$C_3H_7$ | $OCH_3$ | Cl | CH | S | |
| H | i-$C_3H_7$ | $CH_3$ | $OC_2H_5$ | N | S | |
| H | $SCH_3$ | $CH_3$ | $CH_3$ | CH | O | |
| H | $SCH_3$ | $CH_3$ | $OCH_3$ | CH | O | |
| H | $SCH_3$ | $CH_3$ | $OCH_3$ | N | O | |
| H | $SCH_3$ | $OCH_3$ | $OCH_3$ | CH | O | |
| H | $SCH_3$ | $OCH_3$ | $OCH_3$ | N | O | |
| H | $SCH_3$ | $OCH_3$ | Cl | CH | O | |
| H | $SCH_3$ | $OCH_3$ | $NHCH_3$ | CH | O | |
| $CH_3$ | $SCH_3$ | $OCH_3$ | $OCH_3$ | CH | S | |
| H | $SCH_3$ | $CH_3$ | $CH_3$ | CH | S | |
| H | $SCH_3$ | $CH_3$ | $CH_3$ | N | S | |
| H | $SCH_3$ | $CH_3$ | $OCH_3$ | CH | S | |
| H | $SCH_3$ | $CH_3$ | $OCH_3$ | N | S | |
| H | $SCH_3$ | $OCH_3$ | $OCH_3$ | CH | S | |
| H | $SCH_3$ | $OCH_3$ | $OCH_3$ | N | S | |
| H | $SCH_3$ | $OCH_3$ | Cl | CH | S | |
| $CH_3$ | $SC_2H_5$ | $OCH_3$ | $OCH_3$ | CH | O | |
| H | $SC_2H_5$ | $CH_3$ | $CH_3$ | CH | O | |
| H | $SC_2H_5$ | $CH_3$ | $CH_3$ | N | O | |
| H | $SC_2H_5$ | $CH_3$ | $OCH_3$ | CH | O | |
| H | $SC_2H_5$ | $CH_3$ | $OCH_3$ | N | O | |
| H | $SC_2H_5$ | $OCH_3$ | $OCH_3$ | CH | O | |
| H | $SC_2H_5$ | $OCH_3$ | $OCH_3$ | N | O | |
| H | $SC_2H_5$ | $OCH_3$ | Cl | CH | O | |
| H | $SC_2H_5$ | $CH_3$ | $CH_3$ | CH | S | |
| H | $SC_2H_5$ | $CH_3$ | $OCH_3$ | CH | S | |
| H | $SC_2H_5$ | $CH_3$ | $OCH_3$ | N | S | |
| H | $SC_2H_5$ | $OCH_3$ | $OCH_3$ | CH | S | |
| H | $SC_2H_5$ | $CH_3$ | $OCH_3$ | N | S | |
| H | $SC_2H_5$ | $OCH_3$ | Cl | CH | S | |

Table 9a (continued)

| R | $R'_3$ | X | Y | Z | W' | m.p. °C |
|---|---|---|---|---|---|---|
| H | $SC_2H_5$ | $CH_3$ | $OC_2H_5$ | N | S | |
| H | S-n-$C_3H_7$ | $CH_3$ | $CH_3$ | CH | O | |
| H | S-n-$C_3H_7$ | $CH_3$ | $CH_2OCH_3$ | CH | O | |
| H | S-n-$C_3H_7$ | $CH_3$ | $OCH_3$ | CH | O | |
| H | S-n-$C_3H_7$ | $CH_3$ | $OCH_3$ | N | O | |
| H | S-n-$C_3H_7$ | $OCH_3$ | $OCH_3$ | CH | O | |
| H | S-n-$C_3H_7$ | $OCH_3$ | $OCH_3$ | N | O | |
| H | S-n-$C_3H_7$ | $OCH_3$ | Cl | CH | O | |
| $CH_3$ | S-n-$C_3H_7$ | $OCH_3$ | $OCH_3$ | CH | S | |
| H | S-n-$C_3H_7$ | $CH_3$ | $CH_3$ | CH | S | |
| H | S-n-$C_3H_7$ | $CH_3$ | $CH_3$ | N | S | |
| H | S-n-$C_3H_7$ | $CH_3$ | $OCH_3$ | CH | S | |
| H | S-n-$C_3H_7$ | $OCH_3$ | $OCH_3$ | CH | S | |
| H | S-n-$C_3H_7$ | $OCH_3$ | $OCH_3$ | N | S | |
| H | S-n-$C_3H_7$ | $CH_3$ | Cl | CH | S | |
| H | S-n-$C_3H_7$ | $CH_3$ | $OCH_3$ | CH | O | |
| H | S-n-$C_3H_7$ | $OCH_3$ | $OCH_3$ | CH | S | |
| H | S-n-$C_3H_7$ | $CH_3$ | $OCH_3$ | N | S | |
| $CH_3$ | S-n-$C_4H_9$ | $OCH_3$ | $OCH_3$ | CH | O | |
| H | S-n-$C_4H_9$ | $CH_3$ | $CH_3$ | CH | O | |
| H | S-n-$C_4H_9$ | $CH_3$ | $CH_3$ | N | O | |
| H | S-n-$C_4H_9$ | $CH_3$ | $OCH_3$ | CH | O | |
| H | S-n-$C_4H_9$ | $CH_3$ | $OCH_3$ | N | O | |
| H | S-n-$C_4H_9$ | $OCH_3$ | $OCH_3$ | CH | O | |
| H | S-n-$C_4H_9$ | $OCH_3$ | $OCH_3$ | N | O | |
| H | S-n-$C_4H_9$ | $OCH_3$ | Cl | CH | O | |
| H | S-n-$C_4H_9$ | $CH_3$ | $CH_3$ | CH | S | |
| H | S-n-$C_4H_9$ | $CH_3$ | $OCH_3$ | N | S | |
| H | S-n-$C_4H_9$ | $CH_3$ | $OCH_3$ | CH | S | |
| H | S-n-$C_4H_9$ | $OCH_3$ | $OCH_3$ | N | S | |
| H | S-n-$C_4H_9$ | $OCH_3$ | $OCH_3$ | CH | S | |
| H | S-n-$C_4H_9$ | $OCH_3$ | $OCH_3$ | N | S | |

Table 9a (continued)

| R | $R'_3$ | X | Y | Z | W' | m.p. °C |
|---|---|---|---|---|---|---|
| H | S-n-$C_4H_9$ | $OCH_3$ | $OCH_3$ | CH | O | |
| H | S-n-$C_4H_9$ | $CH_3$ | $OCH_3$ | N | O | |
| H | S-n-$C_4H_9$ | $OCH_3$ | $OCH_3$ | CH | S | |
| H | S-n-$C_4H_9$ | $CH_3$ | $OCH_3$ | CH | S | |
| H | S-n-$C_4H_9$ | $CH_3$ | $OCH_3$ | N | O | |
| H | S-n-$C_4H_9$ | $CH_3$ | $OCH_3$ | CH | O | |
| H | S-n-$C_4H_9$ | $OCH_3$ | $OCH_3$ | CH | S | |
| H | S-n-$C_4H_9$ | $OCH_3$ | $OCH_3$ | N | S | |
| H | S-n-$C_4H_9$ | $CH_3$ | $OCH_3$ | CH | O | |
| H | S-n-$C_4H_9$ | $OCH_3$ | $OCH_3$ | N | O | |
| H | S-n-$C_4H_9$ | $OCH_3$ | $OCH_3$ | CH | S | |
| H | S-n-$C_4H_9$ | $CH_3$ | $OCH_3$ | N | S | |
| H | $SCH_2CH{=}CH_2$ | $CH_3$ | $CH_3$ | CH | O | |
| H | $SCH_2CH{=}CH_2$ | $CH_3$ | $OCH_3$ | N | O | |
| H | $SCH_2CH{=}CH_2$ | $CH_3$ | $OCH_3$ | CH | O | |
| H | $SCH_2CH{=}CH_2$ | $OCH_3$ | $OCH_3$ | N | O | |
| H | $SCH_2CH{=}CH_2$ | $OCH_3$ | $OCH_3$ | CH | O | |
| H | $SCH_2CH{=}CH_2$ | $OCH_3$ | $OCH_3$ | N | O | |
| H | $SCH_2CH{=}CH_2$ | $OCH_3$ | Cl | CH | O | |
| H | $SCH_2CH{=}CH_2$ | $CH_3$ | $OCH_2CF_3$ | CH | O | |
| $CH_3$ | $SCH_2CH{=}CH_2$ | $OCH_3$ | $OCH_3$ | CH | S | |
| H | $SCH_2CH{=}CH_2$ | $CH_3$ | $CH_3$ | CH | S | |
| H | $SCH_2CH{=}CH_2$ | $CH_3$ | $CH_3$ | N | S | |
| H | $SCH_2CH{=}CH_2$ | $CH_3$ | $OCH_3$ | CH | S | |
| H | $SCH_2CH{=}CH_2$ | $CH_3$ | $OCH_3$ | N | S | |
| H | $SCH_2CH{=}CH_2$ | $OCH_3$ | $OCH_3$ | CH | S | |
| H | $SCH_2CH{=}CH_2$ | $OCH_3$ | $OCH_3$ | N | S | |
| H | $SCH_2CH{=}CH_2$ | $OCH_3$ | Cl | CH | S | |
| H | E-$SCH{=}CHCH_3$ | $CH_3$ | $OCH_3$ | CH | O | |
| H | E-$SCH{=}CHCH_3$ | $CH_3$ | $OCH_3$ | N | O | |

Table 9a (continued)

| R | $R'_3$ | X | Y | Z | W' | m.p. °C |
|---|---|---|---|---|---|---|
| H | E-$SCH=CHCH_3$ | $OCH_3$ | $OCH_3$ | CH | O | |
| H | E-$SCH=CHCH_3$ | $OCH_3$ | $OCH_3$ | N | O | |
| H | E-$SCH=CHCH_3$ | $CH_3$ | $OCH_3$ | N | S | |
| H | E-$SCH=CHCH_3$ | $CH_3$ | $OCH_3$ | CH | S | |
| H | E-$SCH=CHCH_3$ | $OCH_3$ | $OCH_3$ | CH | S | |
| H | E-$SCH=CHCH_3$ | $OCH_3$ | Cl | CH | S | |
| H | Z-$SCH=CHCH_3$ | $CH_3$ | $OCH_3$ | N | O | |
| H | Z-$SCH=CHCH_3$ | $OCH_3$ | $OCH_3$ | CH | O | |
| H | Z-$SCH=CHCH_3$ | $OCH_3$ | $OCH_3$ | N | O | |
| H | Z-$SCH=CHCH_3$ | $OCH_3$ | Cl | CH | O | |
| H | Z-$SCH=CHCH_3$ | $CH_3$ | $CH_3$ | CH | S | |
| H | Z-$SCH=CHCH_3$ | $OCH_3$ | $OCH_3$ | CH | S | |
| H | Z-$SCH=CHCH_3$ | $CH_3$ | $OCH_3$ | N | S | |
| H | Z-$SCH=CHCH_3$ | $OCH_3$ | $OCH_3$ | N | S | |
| H | $S(CH_2)_2CH=CH_2$ | $OCH_3$ | $OCH_3$ | CH | O | |
| H | $S(CH_2)_2CH=CH_2$ | $OCH_3$ | $OCH_3$ | N | O | |
| H | $S(CH_2)_2CH=CH_2$ | $CH_3$ | $OCH_3$ | CH | O | |
| H | $S(CH_2)_2CH=CH_2$ | $OCH_3$ | Cl | CH | O | |
| H | $S(CH_2)_2CH=CH_2$ | $OCH_3$ | $OCH_3$ | CH | S | |
| H | $S(CH_2)_2CH=CH_2$ | $CH_3$ | $OCH_3$ | N | S | |
| H | $S(CH_2)_2CH=CH_2$ | $OCH_3$ | $OCH_3$ | N | S | |
| H | $S(CH_2)_2CH=CH_2$ | $CH_3$ | $CH_3$ | CH | S | |
| H | E-$SCH_2CH=CHCH_3$ | $OCH_3$ | $OCH_3$ | CH | O | |
| H | E-$SCH_2CH=CHCH_3$ | $OCH_3$ | $OCH_3$ | N | O | |
| H | E-$SCH_2CH=CHCH_3$ | $CH_3$ | $OCH_3$ | CH | O | |
| H | E-$SCH_2CH=CHCH_3$ | $OCH_3$ | Cl | CH | O | |
| H | E-$SCH_2CH=CHCH_3$ | $OCH_3$ | $OCH_3$ | CH | S | |
| H | E-$SCH_2CH=CHCH_3$ | $CH_3$ | $OCH_3$ | N | S | |
| H | E-$SCH_2CH=CHCH_3$ | $CH_3$ | $OCH_3$ | CH | S | |
| H | E-$SCH_2CH=CHCH_3$ | $CH_3$ | $CH_3$ | CH | S | |
| H | Z-$SCH_2CH=CHCH_3$ | $CH_3$ | $OCH_3$ | CH | O | |

Table 9a (continued)

| R | R'3 | X | Y | Z | W' | m.p. °C |
|---|---|---|---|---|---|---|
| H | Z-$SCH_2CH=CHCH_3$ | $CH_3$ | $OCH_3$ | N | O | |
| H | Z-$SCH_2CH=CHCH_3$ | $OCH_3$ | $OCH_3$ | CH | O | |
| H | Z-$SCH_2CH=CHCH_3$ | $OCH_3$ | Cl | CH | O | |
| H | Z-$SCH_2CH=CHCH_3$ | $CH_3$ | $OCH_3$ | CH | S | |
| H | Z-$SCH_2CH=CHCH_3$ | $CH_3$ | $OCH_3$ | N | S | |
| H | Z-$SCH_2CH=CHCH_3$ | $OCH_3$ | $OCH_3$ | CH | S | |
| H | Z-$SCH_2CH=CHCH_3$ | $OCH_3$ | $OCH_3$ | N | S | |
| H | E-$SCH=CHC_2H_5$ | $OCH_3$ | $OCH_3$ | CH | O | |
| H | E-$SCH=CHC_2H_5$ | $OCH_3$ | $OCH_3$ | N | O | |
| H | E-$SCH=CHC_2H_5$ | $CH_3$ | $OCH_3$ | CH | O | |
| H | E-$SCH=CHC_2H_5$ | $OCH_3$ | Cl | CH | O | |
| H | E-$SCH=CHC_2H_5$ | $OCH_3$ | $OCH_3$ | CH | S | |
| H | E-$SCH=CHC_2H_5$ | $CH_3$ | $CH_3$ | CH | S | |
| H | E-$SCH=CHC_2H_5$ | $CH_3$ | $OCH_3$ | N | S | |
| H | E-$SCH=CHC_2H_5$ | $CH_3$ | $OCH_3$ | CH | S | |
| H | Z-$SCH=CHC_2H_5$ | $OCH_3$ | $OCH_3$ | CH | O | |
| H | Z-$SCH=CHC_2H_5$ | $OCH_3$ | $OCH_3$ | N | O | |
| H | Z-$SCH=CHC_2H_5$ | $CH_3$ | $CH_3$ | CH | O | |
| H | Z-$SCH=CHC_2H_5$ | $CH_3$ | $OCH_3$ | N | O | |
| H | Z-$SCH=CHC_2H_5$ | $OCH_3$ | $OCH_3$ | CH | S | |
| H | Z-$SCH=CHC_2H_5$ | $CH_3$ | $OCH_3$ | CH | S | |
| H | Z-$SCH=CHC_2H_5$ | $CH_3$ | $OCH_3$ | N | S | |
| H | Z-$SCH=CHC_2H_5$ | $OCH_3$ | Cl | CH | S | |
| H | $SCH_2C≡CH$ | $CH_3$ | $OCH_3$ | CH | O | |
| H | $SCH_2C≡CH$ | $CH_3$ | $OCH_3$ | N | O | |
| H | $SCH_2C≡CH$ | $OCH_3$ | $OCH_3$ | CH | O | |
| H | $SCH_2C≡CH$ | $OCH_3$ | $OCH_3$ | N | O | |
| H | $SCH_2C≡CH$ | $CH_3$ | $OCH_3$ | CH | S | |
| H | $SCH_2C≡CH$ | $OCH_3$ | $OCH_3$ | N | S | |
| H | $SCH_2C≡CH$ | $OCH_3$ | $OCH_3$ | CH | S | |
| H | $SCH_2C≡CH$ | $OCH_3$ | Cl | CH | S | |

Table 9a (continued)

| R | R'3 | X | Y | Z | W' | m.p. °C |
|---|---|---|---|---|---|---|
| $CH_3$ | $SCH_2C{\equiv}CH$ | $OCH_3$ | $OCH_3$ | CH | S | |
| H | $SC{\equiv}CCH_3$ | $CH_3$ | $CH_3$ | CH | O | |
| H | $SC{\equiv}CCH_3$ | $CH_3$ | $OCH_3$ | N | O | |
| H | $SC{\equiv}CCH_3$ | $CH_3$ | $OCH_3$ | CH | O | |
| H | $SC{\equiv}CCH_3$ | $OCH_3$ | $OCH_3$ | N | O | |
| H | $SC{\equiv}CCH_3$ | $OCH_3$ | $OCH_3$ | CH | S | |
| H | $SC{\equiv}CCH_3$ | $CH_3$ | $OCH_3$ | N | S | |
| H | $SC{\equiv}CCH_3$ | $CH_3$ | $OCH_3$ | CH | S | |
| H | $SC{\equiv}CCH_3$ | $OCH_3$ | $OCH_3$ | N | S | |
| H | $S(CH_2)_2C{\equiv}CH$ | $CH_3$ | $OCH_3$ | CH | O | |
| H | $S(CH_2)_2C{\equiv}CH$ | $CH_3$ | $OCH_3$ | N | O | |
| H | $S(CH_2)_2C{\equiv}CH$ | $OCH_3$ | $OCH_3$ | CH | O | |
| H | $S(CH_2)_2C{\equiv}CH$ | $OCH_3$ | $OCH_3$ | N | O | |
| H | $S(CH_2)_2C{\equiv}CH$ | $CH_3$ | $OCH_3$ | CH | S | |
| H | $S(CH_2)_2C{\equiv}CH$ | $CH_3$ | $OCH_3$ | N | S | |
| H | $S(CH_2)_2C{\equiv}CH$ | $OCH_3$ | $OCH_3$ | CH | S | |
| H | $S(CH_2)_2C{\equiv}CH$ | $OCH_3$ | $OCH_3$ | N | S | |
| H | $SCH_2C{\equiv}CCH_3$ | $CH_3$ | $CH_3$ | CH | O | |
| H | $SCH_2C{\equiv}CCH_3$ | $CH_3$ | $OCH_3$ | N | O | |
| H | $SCH_2C{\equiv}CCH_3$ | $CH_3$ | $OCH_3$ | CH | O | |
| H | $SCH_2C{\equiv}CCH_3$ | $OCH_3$ | $OCH_3$ | CH | O | |
| H | $SCH_2C{\equiv}CCH_3$ | $OCH_3$ | $OCH_3$ | CH | S | |
| H | $SCH_2C{\equiv}CCH_3$ | $OCH_3$ | $OCH_3$ | N | S | |
| H | $SCH_2C{\equiv}CCH_3$ | $CH_3$ | $OCH_3$ | CH | S | |
| H | $SCH_2C{\equiv}CCH_3$ | $OCH_3$ | Cl | CH | S | |
| H | $SC{\equiv}CC_2H_5$ | $CH_3$ | $OCH_3$ | N | O | |
| H | $SC{\equiv}CC_2H_5$ | $OCH_3$ | $OCH_3$ | CH | O | |
| H | $SC{\equiv}CC_2H_5$ | $OCH_3$ | $OCH_3$ | CH | S | |
| H | $SC{\equiv}CC_2H_5$ | $CH_3$ | $CH_3$ | CH | S | |
| $CH_3$ | $SC{\equiv}CC_2H_5$ | $CH_3$ | $OCH_3$ | CH | O | |
| H | $CH_2CN$ | $CH_3$ | $CH_3$ | CH | O | |

Table 9a (continued)

| R | R'3 | X | Y | Z | W' | m.p. °C |
|---|---|---|---|---|---|---|
| H | $CH_2CN$ | $CH_3$ | $OCH_3$ | N | O | |
| H | $CH_2CN$ | $CH_3$ | $OCH_3$ | CH | O | |
| H | $CH_2CN$ | $OCH_3$ | $OCH_3$ | N | O | |
| H | $CH_2CN$ | $OCH_3$ | $OCH_3$ | CH | O | |
| H | $CH_2CN$ | $OCH_3$ | Cl | CH | O | |
| H | $CH_2CN$ | $CH_3$ | $NHCH_3$ | N | O | |
| $CH_3$ | $CH_2CN$ | $OCH_3$ | $OCH_3$ | CH | S | |
| H | $CH_2CN$ | $CH_3$ | $CH_3$ | CH | S | |
| H | $CH_2CN$ | $CH_3$ | $CH_3$ | N | S | |
| H | $CH_2CN$ | $CH_3$ | $OCH_3$ | CH | S | |
| H | $CH_2CN$ | $CH_3$ | $OCH_3$ | N | S | |
| H | $CH_2CN$ | $OCH_3$ | $OCH_3$ | CH | S | |
| H | $CH_2CN$ | $OCH_3$ | $OCH_3$ | N | S | |
| H | $CH_2CN$ | $OCH_3$ | Cl | CH | S | |
| $CH_3$ | $CH_2CO_2CH_3$ | $CH_3$ | $OCH_3$ | CH | O | |
| H | $CH_2CO_2CH_3$ | $CH_3$ | $CH_3$ | CH | O | |
| H | $CH_2CO_2CH_3$ | $CH_3$ | $OCH_3$ | CH | O | |
| H | $CH_2CO_2CH_3$ | $CH_3$ | $OCH_3$ | N | O | |
| H | $CH_2CO_2CH_3$ | $OCH_3$ | $OCH_3$ | CH | O | |
| H | $CH_2CO_2CH_3$ | $OCH_3$ | $OCH_3$ | N | O | |
| H | $CH_2CO_2CH_3$ | $OCH_3$ | Cl | CH | O | |
| H | $CH_2CO_2CH_3$ | $CH_3$ | $CH_3$ | CH | S | |
| H | $CH_2CO_2CH_3$ | $CH_3$ | $OCH_3$ | N | S | |
| H | $CH_2CO_2CH_3$ | $CH_3$ | $OCH_3$ | CH | S | |
| H | $CH_2CO_2CH_3$ | $OCH_3$ | $OCH_3$ | N | S | |
| H | $CH_2CO_2CH_3$ | $OCH_3$ | $OCH_3$ | CH | S | |
| H | $CH_2CO_2CH_3$ | $OCH_3$ | Cl | CH | S | |
| H | $CH_2CO_2CH_3$ | $CH_3$ | $N(CH_3)_2$ | N | S | |
| H | $CH_2CO_2C_2H_5$ | $CH_3$ | $CH_3$ | CH | O | |
| H | $CH_2CO_2C_2H_5$ | $CH_3$ | $OCH_3$ | N | O | |
| H | $CH_2CO_2C_2H_5$ | $CH_3$ | $OCH_3$ | CH | O | |

Table 9a (continued)

| R | $R'_3$ | X | Y | Z | W' | m.p. °C |
|---|---|---|---|---|---|---|
| H | $CH_2CO_2C_2H_5$ | $OCH_3$ | $OCH_3$ | N | O | |
| H | $CH_2CO_2C_2H_5$ | $OCH_3$ | $OCH_3$ | CH | O | |
| H | $CH_2CO_2C_2H_5$ | $OCH_3$ | Cl | CH | O | |
| H | $CH_2CO_2C_2H_5$ | $OC_2H_5$ | Cl | CH | O | |
| $CH_3$ | $CH_2CO_2C_2H_5$ | $OCH_3$ | $OCH_3$ | CH | S | |
| H | $CH_2CO_2C_2H_5$ | $CH_3$ | $CH_3$ | CH | S | |
| H | $CH_2CO_2C_2H_5$ | $CH_3$ | $CH_3$ | N | S | |
| H | $CH_2CO_2C_2H_5$ | $CH_3$ | $OCH_3$ | CH | S | |
| H | $CH_2CO_2C_2H_5$ | $CH_3$ | $OCH_3$ | N | S | |
| H | $CH_2CO_2C_2H_5$ | $OCH_3$ | $OCH_3$ | CH | S | |
| H | $CH_2CO_2C_2H_5$ | $OCH_3$ | $OCH_3$ | N | S | |
| H | $CH_2CO_2C_2H_5$ | $OCH_3$ | Cl | CH | S | |
| $CH_3$ | $CH_2OCH_3$ | $OCH_3$ | $OCH_3$ | CH | O | |
| H | $CH_2OCH_3$ | $CH_3$ | $CH_3$ | CH | O | |
| H | $CH_2OCH_3$ | $CH_3$ | $CH_3$ | N | O | |
| H | $CH_2OCH_3$ | $CH_3$ | $OCH_3$ | CH | O | |
| H | $CH_2OCH_3$ | $CH_3$ | $OCH_3$ | N | O | |
| H | $CH_2OCH_3$ | $OCH_3$ | $OCH_3$ | CH | O | |
| H | $CH_2OCH_3$ | $OCH_3$ | $OCH_3$ | N | O | |
| H | $CH_2OCH_3$ | $OCH_3$ | Cl | CH | O | |
| H | $CH_2OCH_3$ | $CH_3$ | $CH_3$ | CH | S | |
| H | $CH_2OCH_3$ | $CH_3$ | $OCH_3$ | N | S | |
| H | $CH_2OCH_3$ | $CH_3$ | $OCH_3$ | CH | S | |
| H | $CH_2OCH_3$ | $OCH_3$ | $OCH_3$ | N | S | |
| H | $CH_2OCH_3$ | $OCH_3$ | $OCH_3$ | CH | S | |
| H | $CH_2OCH_3$ | $OCH_3$ | Cl | CH | S | |
| H | $CH_2OCH_3$ | $CH_3$ | $CH(OCH_3)_2$ | CH | S | |
| H | $CH_2OC_2H_5$ | $CH_3$ | $CH_3$ | CH | O | |
| H | $CH_2OC_2H_5$ | $CH_3$ | $OCH_3$ | N | O | |
| H | $CH_2OC_2H_5$ | $CH_3$ | $OCH_3$ | CH | O | |
| H | $CH_2OC_2H_5$ | $OCH_3$ | $OCH_3$ | N | O | |

Table 9a (continued)

| R | $R'_3$ | X | Y | Z | W' | m.p. °C |
|---|---|---|---|---|---|---|
| H | $CH_2OC_2H_5$ | $OCH_3$ | $OCH_3$ | CH | O | |
| H | $CH_2OC_2H_5$ | $OCH_3$ | Cl | CH | O | |
| H | $CH_2OC_2H_5$ | $OCH_3$ | Br | CH | O | |
| $CH_3$ | $CH_2OC_2H_5$ | $CH_3$ | $OCH_3$ | CH | S | |
| H | $CH_2OC_2H_5$ | $CH_3$ | $CH_3$ | CH | S | |
| H | $CH_2OC_2H_5$ | $CH_3$ | $CH_3$ | N | S | |
| H | $CH_2OC_2H_5$ | $CH_3$ | $OCH_3$ | CH | S | |
| H | $CH_2OC_2H_5$ | $CH_3$ | $OCH_3$ | N | S | |
| H | $CH_2OC_2H_5$ | $OCH_3$ | $OCH_3$ | CH | S | |
| H | $CH_2OC_2H_5$ | $OCH_3$ | $OCH_3$ | N | S | |
| H | $CH_2OC_2H_5$ | $OCH_3$ | Cl | CH | S | |
| $CH_3$ | $(CH_2)_2COCH_3$ | $OCH_3$ | $OCH_3$ | CH | O | |
| H | $(CH_2)_2COCH_3$ | $CH_3$ | $CH_3$ | CH | O | |
| H | $(CH_2)_2COCH_3$ | $CH_3$ | $CH_3$ | N | O | |
| H | $(CH_2)_2COCH_3$ | $CH_3$ | $OCH_3$ | CH | O | |
| H | $(CH_2)_2COCH_3$ | $CH_3$ | $OCH_3$ | N | O | |
| H | $(CH_2)_2COCH_3$ | $OCH_3$ | $OCH_3$ | CH | O | |
| H | $(CH_2)_2COCH_3$ | $OCH_3$ | $OCH_3$ | N | O | |
| H | $(CH_2)_2COCH_3$ | $OCH_3$ | Cl | CH | O | |
| H | $(CH_2)_2COCH_3$ | $CH_3$ | $CH_3$ | CH | S | |
| H | $(CH_2)_2COCH_3$ | $CH_3$ | $OCH_3$ | N | S | |
| H | $(CH_2)_2COCH_3$ | $CH_3$ | $OCH_3$ | CH | S | |
| H | $(CH_2)_2COCH_3$ | $OCH_3$ | $OCH_3$ | N | S | |
| H | $(CH_2)_2COCH_3$ | $OCH_3$ | $OCH_3$ | CH | S | |
| H | $(CH_2)_2COCH_3$ | $OCH_3$ | Cl | CH | S | |
| H | $(CH_2)_2COCH_3$ | $CH_3$ | 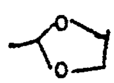 | CH | S | |
| H | $(CH_2)_2CN$ | $CH_3$ | $OCH_3$ | CH | O | |
| H | $(CH_2)_2CN$ | $CH_3$ | $OCH_3$ | N | O | |
| H | $(CH_2)_2CN$ | $OCH_3$ | $OCH_3$ | CH | O | |
| H | $(CH_2)_2CN$ | $OCH_3$ | $OCH_3$ | N | O | |

Table 9a (continued)

| R | $R'_3$ | X | Y | Z | W' | m.p. °C |
|---|---|---|---|---|---|---|
| H | $(CH_2)_2CN$ | $CH_3$ | $CH_3$ | CH | S | |
| H | $(CH_2)_2CN$ | $CH_3$ | $OCH_3$ | N | S | |
| H | $(CH_2)_2CN$ | $OCH_3$ | $OCH_3$ | CH | S | |
| H | $(CH_2)_2CN$ | $OCH_3$ | Cl | CH | S | |
| H | $CH(CH_3)CN$ | $OCH_3$ | $OCH_3$ | CH | O | |
| H | $CH(CH_3)CN$ | $OCH_3$ | $OCH_3$ | N | O | |
| H | $CH(CH_3)CN$ | $CH_3$ | $OCH_3$ | CH | S | |
| H | $CH(CH_3)CN$ | $CH_3$ | $OCH_3$ | N | S | |
| H | $(CH_2)_3CN$ | $CH_3$ | $OCH_3$ | CH | O | |
| H | $(CH_2)_3CN$ | $CH_3$ | $OCH_3$ | N | O | |
| H | $(CH_2)_3CN$ | $OCH_3$ | $OCH_3$ | CH | O | |
| H | $(CH_2)_3CN$ | $OCH_3$ | $OCH_3$ | N | O | |
| H | $(CH_2)_3CN$ | $CH_3$ | $CH_3$ | CH | S | |
| H | $(CH_2)_3CN$ | $CH_3$ | $OCH_3$ | N | S | |
| H | $(CH_2)_3CN$ | $OCH_3$ | $OCH_3$ | $CH_3$ | S | |
| H | $(CH_2)_3CN$ | $OCH_3$ | $OCH_3$ | N | S | |
| H | $CH_2CH(CH_3)CN$ | $CH_3$ | $OCH_3$ | CH | O | |
| H | $CH_2CH(CH_3)CN$ | $CH_3$ | $OCH_3$ | N | O | |
| H | $CH_2CH(CH_3)CN$ | $OCH_3$ | $OCH_3$ | CH | S | |
| H | $CH_2CH(CH_3)CN$ | $OCH_3$ | Cl | CH | S | |
| H | $CH_2CH(CH_3)CN$ | $CH_3$ | $CH_3$ | CH | O | |
| H | $CH_2CH(CH_3)CN$ | $CH_3$ | $OCH_3$ | N | O | |
| H | $CH_2CH(CH_3)CN$ | $OCH_3$ | $OCH_3$ | CH | S | |
| H | $CH_2CH(CH_3)CN$ | $OCH_3$ | $CH_3$ | CH | S | |
| $CH_3$ | $CH_2CH(CH_3)CN$ | $OCH_3$ | $OCH_3$ | CH | S | |
| H | $CH(CN)C_2H_5$ | $CH_3$ | $OCH_3$ | N | O | |
| H | $CH(CN)C_2H_5$ | $OCH_3$ | $OCH_3$ | CH | O | |
| H | $CH(CN)C_2H_5$ | $OCH_3$ | $OCH_3$ | N | S | |
| H | $CH(CN)C_2H_5$ | $OCH_3$ | Cl | CH | S | |
| H | $C(CH_3)_2CN$ | $CH_3$ | $CH_3$ | CH | O | |
| H | $C(CH_3)_2CN$ | $OCH_3$ | $OCH_3$ | CH | O | |

Table 9a (continued)

| R | $R'_3$ | X | Y | Z | W' | m.p. °C |
|---|---|---|---|---|---|---|
| H | $C(CH_3)_2CN$ | $OCH_3$ | $OCH_3$ | N | S | |
| H | $C(CH_3)_2CN$ | $OCH_3$ | $OCH_3$ | CH | S | |
| H | $CH(CH_3)CO_2CH_3$ | $OCH_3$ | $OCH_3$ | CH | O | |
| H | $CH(CH_3)CO_2CH_3$ | $OCH_3$ | $OCH_3$ | N | O | |
| H | $CH(CH_3)CO_2CH_3$ | $CH_3$ | $OCH_3$ | CH | S | |
| H | $CH(CH_3)CO_2CH_3$ | $CH_3$ | $OCH_3$ | N | S | |
| H | $(CH_2)_3CO_2CH_3$ | $CH_3$ | $OCH_3$ | CH | O | |
| H | $(CH_2)_3CO_2CH_3$ | $CH_3$ | $OCH_3$ | N | O | |
| H | $(CH_2)_3CO_2CH_3$ | $OCH_3$ | $OCH_3$ | CH | O | |
| H | $(CH_2)_3CO_2CH_3$ | $OCH_3$ | $OCH_3$ | N | O | |
| H | $(CH_2)_3CO_2CH_3$ | $CH_3$ | $OCH_3$ | CH | S | |
| H | $(CH_2)_3CO_2CH_3$ | $CH_3$ | $OCH_3$ | N | S | |
| H | $(CH_2)_3CO_2CH_3$ | $OCH_3$ | $OCH_3$ | CH | S | |
| H | $(CH_2)_3CO_2CH_3$ | $OCH_3$ | $OCH_3$ | N | S | |
| H | $CH_2CH(CH_3)CO_2CH_3$ | $CH_3$ | $OCH_3$ | CH | O | |
| H | $CH_2CH(CH_3)CO_2CH_3$ | $CH_3$ | $OCH_3$ | N | O | |
| H | $CH_2CH(CH_3)CO_2CH_3$ | $OCH_3$ | $OCH_3$ | CH | S | |
| H | $CH_2CH(CH_3)CO_2CH_3$ | $OCH_3$ | $OCH_3$ | N | S | |
| H | $CH_2CH(CH_3)CO_2CH_3$ | $CH_3$ | $OCH_3$ | CH | O | |
| H | $CH_2CH(CH_3)CO_2CH_3$ | $CH_3$ | $OCH_3$ | N | O | |
| H | $CH_2CH(CH_3)CO_2CH_3$ | $OCH_3$ | $OCH_3$ | CH | S | |
| H | $CH_2CH(CH_3)CO_2CH_3$ | $OCH_3$ | $OCH_3$ | N | S | |
| H | $CH(CO_2CH_3)C_2H_5$ | $CH_3$ | $OCH_3$ | N | O | |
| H | $CH(CO_2CH_3)C_2H_5$ | $OCH_3$ | $OCH_3$ | CH | O | |
| H | $CH(CO_2CH_3)C_2H_5$ | $OCH_3$ | $OCH_3$ | N | S | |
| H | $CH(CO_2CH_3)C_2H_5$ | $OCH_3$ | $OCH_3$ | CH | S | |
| H | $C(CH_3)_2CO_3CH_3$ | $CH_3$ | $OCH_3$ | N | O | |
| H | $C(CH_3)_2CO_3CH_3$ | $OCH_3$ | $OCH_3$ | CH | O | |
| H | $C(CH_3)_2CO_3CH_3$ | $CH_3$ | $OCH_3$ | N | S | |
| H | $C(CH_3)_2CO_3CH_3$ | $OCH_3$ | $OCH_3$ | CH | S | |

Table 9a (continued)

| R | R'3 | X | Y | Z | W' | m.p. °C |
|---|---|---|---|---|---|---|
| H | $(CH_2)_2CO_2C_2H_5$ | $CH_3$ | $CH_3$ | CH | O | |
| H | $(CH_2)_2CO_2C_2H_5$ | $CH_3$ | $OCH_3$ | N | O | |
| H | $(CH_2)_2CO_2C_2H_5$ | $OCH_3$ | $OCH_3$ | CH | O | |
| H | $(CH_2)_2CO_2C_2H_5$ | $OCH_3$ | $OCH_3$ | N | O | |
| H | $(CH_2)_2CO_2C_2H_5$ | $OCH_3$ | $OCH_3$ | CH | S | |
| H | $(CH_2)_2CO_2C_2H_5$ | $CH_3$ | $OCH_3$ | N | S | |
| H | $(CH_2)_2CO_2C_2H_5$ | $OCH_3$ | $OCH_3$ | CH | S | |
| H | $(CH_2)_2CO_2C_2H_5$ | $OCH_3$ | $OCH_3$ | N | S | |
| H | $CH(CH_3)CO_2C_2H_5$ | $OCH_3$ | $OCH_3$ | CH | O | |
| H | $CH(CH_3)CO_2C_2H_5$ | $OCH_3$ | $OCH_3$ | N | O | |
| H | $CH(CH_3)CO_2C_2H_5$ | $CH_3$ | $OCH_3$ | CH | S | |
| H | $CH(CH_3)CO_2C_2H_5$ | $CH_3$ | $OCH_3$ | N | S | |
| H | $(CH_2)_3CO_2C_2H_5$ | $CH_3$ | $OCH_3$ | CH | O | |
| H | $(CH_2)_3CO_2C_2H_5$ | $CH_3$ | $OCH_3$ | N | O | |
| H | $(CH_2)_3CO_2C_2H_5$ | $OCH_3$ | $OCH_3$ | CH | O | |
| H | $(CH_2)_3CO_2C_2H_5$ | $OCH_3$ | $OCH_3$ | N | O | |
| H | $(CH_2)_3CO_2C_2H_5$ | $CH_3$ | $OCH_3$ | CH | S | |
| H | $(CH_2)_3CO_2C_2H_5$ | $CH_3$ | $OCH_3$ | N | S | |
| H | $(CH_2)_3CO_2C_2H_5$ | $OCH_3$ | $OCH_3$ | CH | S | |
| H | $(CH_2)_3CO_2C_2H_5$ | $OCH_3$ | $OCH_3$ | N | S | |
| H | $CH_2CH(CH_3)CO_2C_2H_5$ | $CH_3$ | $OCH_3$ | CH | O | |
| H | $CH_2CH(CH_3)CO_2C_2H_5$ | $CH_3$ | $OCH_3$ | N | O | |
| H | $CH_2CH(CH_3)CO_2C_2H_5$ | $OCH_3$ | $OCH_3$ | CH | S | |
| H | $CH_2CH(CH_3)CO_2C_2H_5$ | $OCH_3$ | $OCH_3$ | N | S | |
| H | $CH_2CH(CH_3)CO_2C_2H_5$ | $CH_3$ | $OCH_3$ | CH | O | |
| H | $CH_2CH(CH_3)CO_2C_2H_5$ | $CH_3$ | $OCH_3$ | N | O | |
| H | $CH_2CH(CH_3)CO_2C_2H_5$ | $CH_3$ | $OCH_3$ | CH | S | |
| H | $CH_2CH(CH_3)CO_2C_2H_5$ | $OCH_3$ | $OCH_3$ | N | S | |
| H | $CH(CO_2C_2H_5)C_2H_5$ | $CH_3$ | $OCH_3$ | N | O | |
| H | $CH(CO_2C_2H_5)C_2H_5$ | $OCH_3$ | $OCH_3$ | CH | O | |
| H | $CH(CO_2C_2H_5)C_2H_5$ | $OCH_3$ | $OCH_3$ | N | S | |

302

**Table 9a (continued)**

| R | $R'_3$ | X | Y | Z | W' | m.p. °C |
|---|---|---|---|---|---|---|
| H | $CH(CO_2C_2H_5)C_2H_5$ | $OCH_3$ | $OCH_3$ | CH | S | |
| H | $C(CH_3)_2CO_2C_2H_5$ | $CH_3$ | $OCH_3$ | N | O | |
| H | $C(CH_3)_2CO_2C_2H_5$ | $OCH_3$ | $OCH_3$ | CH | O | |
| H | $C(CH_3)_2CO_2C_2H_5$ | $CH_3$ | $OCH_3$ | N | O | |
| H | $C(CH_3)_2CO_2C_2H_5$ | $OCH_3$ | $OCH_3$ | CH | S | |
| H | $(CH_2)_2OCH_3$ | $CH_3$ | $CH_3$ | CH | O | |
| H | $(CH_2)_2OCH_3$ | $CH_3$ | $OCH_3$ | N | O | |
| H | $(CH_2)_2OCH_3$ | $OCH_3$ | $OCH_3$ | CH | O | |
| H | $(CH_2)_2OCH_3$ | $OCH_3$ | $OCH_3$ | N | O | |
| H | $(CH_2)_2OCH_3$ | $OCH_3$ | $OCH_3$ | CH | S | |
| H | $(CH_2)_2OCH_3$ | $CH_3$ | $OCH_3$ | N | S | |
| H | $(CH_2)_2OCH_3$ | $OCH_3$ | $OCH_3$ | CH | S | |
| H | $(CH_2)_2OCH_3$ | $OCH_3$ | $OCH_3$ | N | S | |
| H | $CH(CH_3)OCH_3$ | $OCH_3$ | $OCH_3$ | CH | O | |
| H | $CH(CH_3)OCH_3$ | $OCH_3$ | $OCH_3$ | N | O | |
| H | $CH(CH_3)OCH_3$ | $CH_3$ | $OCH_3$ | CH | S | |
| H | $CH(CH_3)OCH_3$ | $CH_3$ | $OCH_3$ | N | S | |
| H | $(CH_2)_3OCH_3$ | $CH_3$ | $OCH_3$ | CH | O | |
| H | $(CH_2)_3OCH_3$ | $CH_3$ | $OCH_3$ | N | O | |
| H | $(CH_2)_3OCH_3$ | $OCH_3$ | $OCH_3$ | CH | O | |
| H | $(CH_2)_3OCH_3$ | $OCH_3$ | $OCH_3$ | N | O | |
| H | $(CH_2)_3OCH_3$ | $CH_3$ | $OCH_3$ | CH | S | |
| H | $(CH_2)_3OCH_3$ | $CH_3$ | $OCH_3$ | N | S | |
| H | $(CH_2)_3OCH_3$ | $OCH_3$ | $OCH_3$ | CH | S | |
| H | $(CH_2)_3OCH_3$ | $OCH_3$ | $OCH_3$ | N | S | |
| H | $CH_2CH(CH_3)OCH_3$ | $CH_3$ | $OCH_3$ | CH | O | |
| H | $CH_2CH(CH_3)OCH_3$ | $CH_3$ | $OCH_3$ | N | O | |
| H | $CH_2CH(CH_3)OCH_3$ | $OCH_3$ | $OCH_3$ | CH | S | |
| H | $CH_2CH(CH_3)OCH_3$ | $OCH_3$ | $OCH_3$ | N | S | |
| H | $CH_2CH(CH_3)OCH_3$ | $CH_3$ | $OCH_3$ | CH | O | |
| H | $CH_2CH(CH_3)OCH_3$ | $CH_3$ | $OCH_3$ | N | O | |

Table 9a (continued)

| R | $R'_3$ | X | Y | Z | W' | m.p. °C |
|---|---|---|---|---|---|---|
| H | $CH_2CH(CH_3)OCH_3$ | $CH_3$ | $OCH_3$ | CH | S | |
| H | $CH_2CH(CH_3)OCH_3$ | $OCH_3$ | $OCH_3$ | N | S | |
| H | $CH(OCH_3)C_2H_5$ | $CH_3$ | $OCH_3$ | N | O | |
| H | $CH(OCH_3)C_2H_5$ | $OCH_3$ | $OCH_3$ | CH | O | |
| H | $CH(OCH_3)C_2H_5$ | $OCH_3$ | $OCH_3$ | N | S | |
| H | $CH(OCH_3)C_2H_5$ | $OCH_3$ | $OCH_3$ | CH | S | |
| H | $C(CH_3)_2OCH_3$ | $CH_3$ | $OCH_3$ | N | O | |
| H | $C(CH_3)_2OCH_3$ | $OCH_3$ | $OCH_3$ | CH | O | |
| H | $C(CH_3)_2OCH_3$ | $CH_3$ | $OCH_3$ | N | O | |
| H | $C(CH_3)_2OCH_3$ | $OCH_3$ | $OCH_3$ | CH | S | |
| H | $(CH_2)_2OC_2H_5$ | $CH_3$ | $CH_3$ | CH | O | |
| H | $(CH_2)_2OC_2H_5$ | $CH_3$ | $OCH_3$ | N | O | |
| H | $(CH_2)_2OC_2H_5$ | $OCH_3$ | $OCH_3$ | CH | O | |
| H | $(CH_2)_2OC_2H_5$ | $OCH_3$ | $OCH_3$ | N | O | |
| H | $(CH_2)_2OC_2H_5$ | $OCH_3$ | $OCH_3$ | CH | S | |
| H | $(CH_2)_2OC_2H_5$ | $CH_3$ | $OCH_3$ | N | S | |
| H | $(CH_2)_2OC_2H_5$ | $OCH_3$ | $OCH_3$ | CH | S | |
| H | $(CH_2)_2OC_2H_5$ | $OCH_3$ | $OCH_3$ | N | S | |
| H | $CH(CH_3)OC_2H_5$ | $OCH_3$ | $OCH_3$ | CH | O | |
| H | $CH(CH_3)OC_2H_5$ | $OCH_3$ | $OCH_3$ | N | O | |
| H | $CH(CH_3)OC_2H_5$ | $CH_3$ | $OCH_3$ | CH | S | |
| H | $CH(CH_3)OC_2H_5$ | $CH_3$ | $OCH_3$ | N | S | |
| H | $(CH_2)_3OC_2H_5$ | $CH_3$ | $OCH_3$ | CH | O | |
| H | $(CH_2)_3OC_2H_5$ | $CH_3$ | $OCH_3$ | N | O | |
| H | $(CH_2)_3OC_2H_5$ | $OCH_3$ | $OCH_3$ | CH | O | |
| H | $(CH_2)_3OC_2H_5$ | $OCH_3$ | $OCH_3$ | N | O | |
| H | $(CH_2)_3OC_2H_5$ | $CH_3$ | $OCH_3$ | CH | S | |
| H | $(CH_2)_3OC_2H_5$ | $CH_3$ | $OCH_3$ | N | S | |
| H | $(CH_2)_3OC_2H_5$ | $OCH_3$ | $OCH_3$ | CH | S | |
| H | $(CH_2)_3OC_2H_5$ | $OCH_3$ | $OCH_3$ | N | S | |
| H | $CH_2CH(CH_3)OC_2H_5$ | $CH_3$ | $OCH_3$ | CH | O | |

**Table 9a (continued)**

| R | $R'_3$ | X | Y | Z | W' | m.p. °C |
|---|---|---|---|---|---|---|
| H | $CH_2CH(CH_3)OC_2H_5$ | $CH_3$ | $OCH_3$ | N | O | |
| H | $CH_2CH(CH_3)OC_2H_5$ | $OCH_3$ | $OCH_3$ | CH | S | |
| H | $CH_2CH(CH_3)OC_2H_5$ | $OCH_3$ | $OCH_3$ | N | S | |
| H | $CH(CH_3)CH_2OC_2H_5$ | $CH_3$ | $OCH_3$ | CH | O | |
| H | $CH(CH_3)CH_2OC_2H_5$ | $CH_3$ | $OCH_3$ | N | O | |
| H | $CH(CH_3)CH_2OC_2H_5$ | $CH_3$ | $OCH_3$ | CH | S | |
| H | $CH(CH_3)CH_2OC_2H_5$ | $OCH_3$ | $OCH_3$ | N | S | |
| H | $CH(OC_2H_5)C_2H_5$ | $CH_3$ | $OCH_3$ | N | O | |
| H | $CH(OC_2H_5)C_2H_5$ | $OCH_3$ | $OCH_3$ | CH | O | |
| H | $CH(OC_2H_5)C_2H_5$ | $OCH_3$ | $OCH_3$ | N | S | |
| H | $CH(OC_2H_5)C_2H_5$ | $OCH_3$ | $OCH_3$ | CH | S | |
| H | $CH(CH_3)_2OC_2H_5$ | $CH_3$ | $OCH_3$ | N | O | |
| H | $CH(CH_3)_2OC_2H_5$ | $OCH_3$ | $OCH_3$ | CH | O | |
| H | $CH(CH_3)_2OC_2H_5$ | $CH_3$ | $OCH_3$ | N | O | |
| H | $C(CH_3)_2OC_2H_5$ | $OCH_3$ | $OCH_3$ | CH | S | |
| H | $(CH_2)CH_2COCH_3$ | $CH_3$ | $CH_3$ | CH | O | |
| H | $(CH_2)CH_2COCH_3$ | $CH_3$ | $OCH_3$ | N | O | |
| H | $(CH_2)CH_2COCH_3$ | $OCH_3$ | $OCH_3$ | CH | O | |
| H | $(CH_2)CH_2COCH_3$ | $OCH_3$ | $OCH_3$ | N | O | |
| H | $(CH_2)CH_2COCH_3$ | $OCH_3$ | $OCH_3$ | CH | S | |
| H | $(CH_2)CH_2COCH_3$ | $CH_3$ | $OCH_3$ | N | S | |
| H | $(CH_2)CH_2COCH_3$ | $OCH_3$ | $OCH_3$ | CH | S | |
| H | $(CH_2)CH_2COCH_3$ | $OCH_3$ | $OCH_3$ | N | S | |
| H | $CH(CH_3)CH_2COCH_3$ | $OCH_3$ | $OCH_3$ | CH | O | |
| H | $CH(CH_3)CH_2COCH_3$ | $OCH_3$ | $OCH_3$ | N | O | |
| H | $CH(CH_3)CH_2COCH_3$ | $CH_3$ | $OCH_3$ | CH | S | |
| H | $CH(CH_3)CH_2COCH_3$ | $CH_3$ | $OCH_3$ | N | S | |
| H | $(CH_2)_3CH_2COCH_3$ | $CH_3$ | $OCH_3$ | CH | O | |
| H | $(CH_2)_3CH_2COCH_3$ | $CH_3$ | $OCH_3$ | N | O | |
| H | $(CH_2)_3CH_2COCH_3$ | $OCH_3$ | $OCH_3$ | CH | O | |
| H | $(CH_2)_3CH_2COCH_3$ | $OCH_3$ | $OCH_3$ | N | O | |

Table 9a (continued)

| R | $R'_3$ | X | Y | Z | W' | m.p. °C |
|---|---|---|---|---|---|---|
| H | $(CH_2)_3CH_2COCH_3$ | $CH_3$ | $OCH_3$ | CH | S | |
| H | $(CH_2)_3CH_2COCH_3$ | $CH_3$ | $OCH_3$ | N | S | |
| H | $(CH_2)_3CH_2COCH_3$ | $OCH_3$ | $OCH_3$ | CH | S | |
| H | $(CH_2)_3CH_2COCH_3$ | $OCH_3$ | $OCH_3$ | N | S | |
| H | $CH_2CH(CH_3)CH_2COCH_3$ | $CH_3$ | $OCH_3$ | CH | O | |
| H | $CH_2CH(CH_3)CH_2COCH_3$ | $CH_3$ | $OCH_3$ | N | O | |
| H | $CH_2CH(CH_3)CH_2COCH_3$ | $OCH_3$ | $OCH_3$ | CH | S | |
| H | $CH_2CH(CH_3)CH_2COCH_3$ | $OCH_3$ | $OCH_3$ | N | S | |
| H | $CH(CH_3)CH_2CH_2COCH_3$ | $CH_3$ | $OCH_3$ | CH | O | |
| H | $CH(CH_3)CH_2CH_2COCH_3$ | $CH_3$ | $OCH_3$ | N | O | |
| H | $CH(CH_3)CH_2CH_2COCH_3$ | $CH_3$ | $OCH_3$ | CH | S | |
| H | $CH(CH_3)CH_2CH_2COCH_3$ | $OCH_3$ | $OCH_3$ | N | S | |
| H | $CH(C_2H_5)CH_2COCH_3$ | $CH_3$ | $OCH_3$ | N | O | |
| H | $CH(C_2H_5)CH_2OCH_3$ | $OCH_3$ | $OCH_3$ | CH | O | |
| H | $CH(C_2H_5)CH_2OCH_3$ | $OCH_3$ | $OCH_3$ | N | S | |
| H | $CH(C_2H_5)CH_2OCH_3$ | $OCH_3$ | $OCH_3$ | CH | S | |
| H | $CH(CH_3)_2CH_2COCH_3$ | $CH_3$ | $OCH_3$ | N | O | |
| H | $CH(CH_3)_2CH_2COCH_3$ | $OCH_3$ | $OCH_3$ | CH | O | |
| H | $CH(CH_3)_2CH_2COCH_3$ | $CH_3$ | $OCH_3$ | N | O | |
| H | $C(CH_3)_2CH_2COCH_3$ | $OCH_3$ | $OCH_3$ | CH | S | |
| H | H | $OCH_3$ | $OCH_3$ | CH | O | * |
| H | H | $OCH_3$ | $OCH_3$ | CH | S | * |
| H | $CH_3$ | $OCH_3$ | $OCH_3$ | CH | O | * |
| H | $CH_3$ | $OCH_3$ | $OCH_3$ | CH | S | * |
| H | $SCH_3$ | $OCH_3$ | $OCH_3$ | CH | O | * |
| H | $SCH_3$ | $OCH_3$ | $OCH_3$ | CH | S | * |
| H | $SCH_2CH=CH_2$ | $OCH_3$ | $OCH_3$ | CH | O | * |
| H | $SCH_3CH=CH_2$ | $OCH_3$ | $OCH_3$ | CH | S | * |
| H | SH | $OCH_3$ | $OCH_3$ | CH | O | * |
| H | SH | $OCH_3$ | $OCH_3$ | CH | S | * |

W is O, unless indicated by * where W is S in all Tables.

**Table 9b**

**General Structure 3 wherein Q is Q-28.**

| R | $R_3'$ | $R_4$ | X | Y | Z | m.p. °C |
|---|---|---|---|---|---|---|
| H | H | H | $CH_3$ | $OCH_3$ | CH | 172-174 |
| H | H | H | $CH_3$ | $OCH_3$ | N | 206-208 |
| H | H | H | $OCH_3$ | $OCH_3$ | CH | 207-209 |
| H | H | H | $OCH_3$ | $OCH_3$ | N | 169-174 |
| H | H | H | $CH_3$ | $CH_3$ | CH | 231-232 |
| H | H | H | $CH_3$ | $CH_3$ | N | |
| H | $CH_3$ | H | $CH_3$ | $OCH_3$ | CH | |
| H | H | H | Cl | $OCH_3$ | CH | 208-210 |
| H | H | $CH_3$ | $CH_3$ | $OCH_3$ | N | |
| H | $CH_3$ | $CH_3$ | $OCH_3$ | $OCH_3$ | CH | |
| $CH_3$ | $CH_3$ | $CH_3$ | $OCH_3$ | $OCH_3$ | N | |
| H | H | H | $CH_3$ | 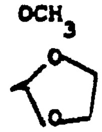 | CH | |
| H | H | H | $CH_3$ | $C_2H_5$ | CH | |
| H | $CH_3$ | H | $CH_3$ | $CH_3$ | CH | |
| H | $CH_3$ | H | $CH_3$ | $OCH_3$ | N | |
| H | $CH_3$ | H | $OCH_3$ | $OCH_3$ | CH | |
| H | $CH_3$ | H | $OCH_3$ | $OCH_3$ | N | |
| H | $CH_3$ | H | $OCH_3$ | Cl | CH | |
| H | $CH_3$ | H | $CH_3$ | $CH_3$ | N | |
| H | Cl | H | $CH_3$ | $CH_3$ | CH | |
| H | Cl | H | $CH_3$ | $CH_3$ | N | |
| H | Cl | H | $CH_3$ | $OCH_3$ | CH | |
| H | Cl | H | $CH_3$ | $OCH_3$ | N | |
| H | Cl | H | $OCH_3$ | $OCH_3$ | CH | |
| H | Cl | H | $OCH_3$ | $OCH_3$ | N | |
| H | Cl | H | $OCH_3$ | Cl | CH | |
| H | Cl | $CH_3$ | $CH_3$ | $OCH_3$ | CH | |
| H | Cl | $CH_3$ | $CH_3$ | $OCH_3$ | N | |
| H | Cl | $CH_3$ | $OCH_3$ | $OCH_3$ | CH | |

Table 9b (continued)

| R | $R_3'$ | $R_4$ | X | Y | Z | m.p. °C |
|---|---|---|---|---|---|---|
| H | Cl | $CH_3$ | $OCH_3$ | $OCH_3$ | N | |
| H | Br | H | $OCH_3$ | $OCH_3$ | CH | |
| H | Br | H | $CH_3$ | $CH_3$ | CH | |
| H | Br | H | $CH_3$ | $OCH_3$ | N | |
| H | Br | H | $CH_3$ | $OCH_3$ | CH | |
| H | Br | H | $OCH_3$ | $OCH_3$ | N | |
| H | Br | H | $OCH_3$ | $OCH_3$ | CH | |
| H | Br | H | $OCH_3$ | Cl | CH | |
| H | H | H | $OCH_3$ | $OCH_3$ | CH * | |
| H | $CH_3$ | H | $OCH_3$ | $OCH_3$ | CH * | |
| H | H | $CH_3$ | $OCH_3$ | $OCH_3$ | CH * | |
| H | $CH_3$ | $CH_3$ | $OCH_3$ | $OCH_3$ | CH * | |
| H | Cl | H | $OCH_3$ | $OCH_3$ | CH * | |
| H | Cl | $CH_3$ | $OCH_3$ | $OCH_3$ | CH * | |
| H | Br | H | $OCH_3$ | $OCH_3$ | CH * | |
| H | Br | $CH_3$ | $OCH_3$ | $OCH_3$ | CH * | |

*Wherein W is O, unless indicated by * where W is S in all Tables.

Table 9c

General Structure 3

| Q | R | $R_3$ | $R_4$ | X | Y | Z | n' | m.p. °C |
|---|---|---|---|---|---|---|---|---|
| Q-33 | H | H | H | $CH_3$ | $OCH_3$ | CH | 0 | |
| Q-33 | H | H | H | $CH_3$ | $OCH_3$ | N | 0 | |
| Q-33 | H | H | H | $OCH_3$ | $OCH_3$ | CH | 0 | |
| Q-33 | H | H | H | $OCH_3$ | $OCH_3$ | N | 0 | |
| Q-33 | H | H | H | $CH_3$ | $CH_3$ | CH | 0 | |
| Q-33 | H | 3-$CH_3$ | H | $CH_3$ | $OCH_3$ | CH | 0 | |
| Q-33 | H | 4-$CH_3$ | H | $CH_3$ | $OCH_3$ | CH | 0 | |
| Q-33 | H | 5-$CH_3$ | H | $CH_3$ | $OCH_3$ | CH | 0 | |
| Q-33 | H | H | H | $CH_3$ | $CH_3$ | N | 0 | |
| Q-33 | H | 6-$CH_3$ | H | $CH_3$ | $OCH_3$ | CH | 0 | |
| Q-33 | H | 3-$CH_3$ | 5-$CH_3$ | $CH_3$ | $OCH_3$ | N | 0 | |
| Q-33 | H | 4-$CH_3$ | 6-$CH_3$ | $CH_3$ | $OCH_3$ | N | 0 | |
| Q-33 | $CH_3$ | H | H | $OCH_3$ | $OCH_3$ | CH | 0 | |
| Q-33 | H | H | H | $CH_3$ | $CH_2OCH_3$ | CH | 0 | |
| Q-33 | H | H | H | Cl | $OC_2H_5$ | CH | 0 | |
| Q-33 | H | H | H | $CH_3$ | $CF_3$ | CH | 0 | |
| Q-33 | H | H | H | Cl | $OCH_3$ | CH | 1 | |
| Q-33 | H | H | H | $CH_3$ | $OCH_3$ | CH | 1 | |
| Q-33 | H | H | H | $CH_3$ | $OCH_3$ | N | 1 | |
| Q-33 | H | H | H | $OCH_3$ | $OCH_3$ | CH | 1 | |
| Q-33 | H | H | H | $OCH_3$ | $OCH_3$ | N | 1 | |
| Q-33 | H | H | H | $CH_3$ | $CH_3$ | CH | 1 | |
| Q-33 | H | 3-$CH_3$ | H | $CH_3$ | $OCH_3$ | CH | 1 | |
| Q-33 | H | 4-$CH_3$ | H | $CH_3$ | $OCH_3$ | CH | 1 | |
| Q-33 | H | 5-$CH_3$ | H | $CH_3$ | $OCH_3$ | CH | 1 | |
| Q-33 | H | H | H | $CH_3$ | $CH_3$ | N | 1 | |
| Q-33 | H | 6-$CH_3$ | H | $CH_3$ | $OCH_3$ | CH | 1 | |
| Q-33 | H | 3-$CH_3$ | 5-$CH_3$ | $CH_3$ | $OCH_3$ | N | 1 | |
| Q-33 | H | 4-$CH_3$ | 6-$CH_3$ | $CH_3$ | $OCH_3$ | N | 1 | |
| Q-33 | $CH_3$ | H | H | $OCH_3$ | $OCH_3$ | CH | 1 | |
| Q-33 | H | H | H | $CH_3$ | $CH_2OCH_3$ | CH | 1 | |

Table 9c (continued)

| Q | R | $R_3$ | $R_4$ | X | Y | Z | n' | m.p. °C |
|---|---|---|---|---|---|---|---|---|
| Q-33 | H | H | H | Cl | $OC_2H_5$ | CH | 1 | |
| Q-33 | H | H | H | $CH_3$ | $CF_3$ | CH | 1 | |
| Q-34 | H | H | H | $CH_3$ | $OCH_3$ | CH | 0 | |
| Q-34 | H | H | H | $CH_3$ | $OCH_3$ | N | 0 | |
| Q-34 | H | H | H | $OCH_3$ | $OCH_3$ | CH | 0 | |
| Q-34 | H | H | H | $OCH_3$ | $OCH_3$ | N | 0 | |
| Q-34 | H | 2-$CH_3$ | H | $CH_3$ | $CH_3$ | CH | 0 | |
| Q-34 | H | 4-$CH_3$ | H | $CH_3$ | $CH_3$ | N | 0 | |
| Q-34 | H | 5-$CH_3$ | H | $CH_3$ | $OCH_3$ | CH | 0 | |
| Q-34 | H | 6-$CH_3$ | H | $CH_3$ | $OCH_3$ | N | 0 | |
| Q-34 | H | 2-$CH_3$ | 6-$CH_3$ | $OCH_3$ | $OCH_3$ | CH | 0 | |
| Q-34 | H | 4-$CH_3$ | 6-$CH_3$ | $OCH_3$ | $OCH_3$ | N | 0 | |
| Q-34 | H | H | H | $CH_3$ | $CH_3$ | N | 0 | |
| Q-34 | $CH_3$ | H | H | $CH_3$ | $CH_3$ | CH | 0 | |
| Q-34 | H | H | H | $CH_3$ | $CH_3$ | CH | 0 | |
| Q-34 | H | H | H | $CH_3$ | $OCF_2H$ | CH | 0 | |
| Q-34 | H | H | H | $CH_3$ | $OCH_3$ | CH | 1 | |
| Q-34 | H | H | H | $CH_3$ | $OCH_3$ | N | 1 | |
| Q-34 | H | H | H | $OCH_3$ | $OCH_3$ | CH | 1 | |
| Q-34 | H | H | H | $OCH_3$ | $OCH_3$ | N | 1 | |
| Q-34 | H | 2-$CH_3$ | H | $CH_3$ | $CH_3$ | CH | 1 | |
| Q-34 | H | 4-$CH_3$ | H | $CH_3$ | $CH_3$ | N | 1 | |
| Q-34 | H | 5-$CH_3$ | H | $CH_3$ | $OCH_3$ | CH | 1 | |
| Q-34 | H | 6-$CH_3$ | H | $CH_3$ | $OCH_3$ | N | 1 | |
| Q-34 | H | 2-$CH_3$ | 6-$CH_3$ | $OCH_3$ | $OCH_3$ | CH | 1 | |
| Q-34 | H | 4-$CH_3$ | 6-$CH_3$ | $OCH_3$ | $OCH_3$ | N | 1 | |
| Q-34 | H | H | H | $CH_3$ | $CH_3$ | N | 1 | |
| Q-34 | $CH_3$ | H | H | $CH_3$ | $CH_3$ | CH | 1 | |
| Q-34 | H | H | H | $CH_3$ | $CH_3$ | CH | 1 | |
| Q-34 | H | H | H | $CH_3$ | $OCF_2H$ | CH | 1 | |
| Q-35 | H | H | H | $CH_3$ | $OCH_3$ | CH | 0 | |

Table 9c (continued)

| Q | R | $R_3$ | $R_4$ | X | Y | Z | n' | m.p. °C |
|---|---|---|---|---|---|---|---|---|
| Q-35 | H | H | H | $CH_3$ | $OCH_3$ | N | 0 | |
| Q-35 | H | H | H | $OCH_3$ | $OCH_3$ | CH | 0 | |
| Q-35 | H | H | H | $OCH_3$ | $OCH_3$ | N | 0 | |
| Q-35 | H | 2-$CH_3$ | H | $CH_3$ | $CH_3$ | CH | 0 | |
| Q-35 | H | H | H | Cl | $OCH_3$ | CH | 0 | |
| Q-35 | H | 3-$CH_3$ | H | $CH_3$ | $CH_3$ | N | 0 | |
| Q-35 | H | 2-$CH_3$ | 6-$CH_3$ | $CH_3$ | $OCH_3$ | CH | 0 | |
| Q-35 | H | 2-$CH_3$ | 3-$CH_3$ | $CH_3$ | $OCH_3$ | N | 0 | |
| Q-35 | H | 3-$CH_3$ | 5-$CH_3$ | $CH_3$ | $OCH_3$ | N | 0 | |
| Q-35 | H | H | H | $CH_3$ | $CH_3$ | N | 0 | |
| Q-35 | $CH_3$ | H | H | $OCH_3$ | $OCH_3$ | CH | 0 | |
| Q-35 | H | H | H | $CH_3$ | $OCH_2CF_3$ | CH | 0 | |
| Q-35 | H | H | H | $CH_3$ | $CH_3$ | CH | 0 | |
| Q-35 | H | H | H | $CH_3$ | $OCH_3$ | CH | 1 | |
| Q-35 | H | H | H | $CH_3$ | $OCH_3$ | N | 1 | |
| Q-35 | H | H | H | $OCH_3$ | $OCH_3$ | CH | 1 | |
| Q-35 | H | H | H | $OCH_3$ | $OCH_3$ | N | 1 | |
| Q-35 | H | 2-$CH_3$ | H | $CH_3$ | $CH_3$ | CH | 1 | |
| Q-35 | H | H | H | Cl | $OCH_3$ | CH | 1 | |
| Q-35 | H | 3-$CH_3$ | H | $CH_3$ | $CH_3$ | N | 1 | |
| Q-35 | H | 2-$CH_3$ | 6-$CH_3$ | $CH_3$ | $OCH_3$ | CH | 1 | |
| Q-35 | H | 2-$CH_3$ | 3-$CH_3$ | $CH_3$ | $OCH_3$ | N | 1 | |
| Q-35 | H | 3-$CH_3$ | 5-$CH_3$ | $CH_3$ | $OCH_3$ | N | 1 | |
| Q-35 | H | H | H | $CH_3$ | $CH_3$ | N | 1 | |
| Q-35 | $CH_3$ | H | H | $OCH_3$ | $OCH_3$ | CH | 1 | |
| Q-35 | H | H | H | $CH_3$ | $OCH_2CF_3$ | CH | 1 | |
| Q-35 | H | H | H | $CH_3$ | $CH_3$ | CH | 1 | |

Table 10

General Structure 4

| | Q | R | W' | W" | A |
|---|---|---|---|---|---|
| Q-2 | ($R_3$, $R_4$, $R_5$= H) | H | S | - | A-2 ($X_1$= $CH_3$, $Y_1$= O) |
| Q-2 | ($R_3$, $R_4$, $R_5$= H) | H | S | - | A-3 ($X_1$= $OCH_3$) |
| Q-2 | ($R_3$, $R_4$, $R_5$= H) | H | O | - | A-4 ($X_1$, $Y_2$= $CH_3$) |
| Q-3 | ($R_3$, $R_4$, $R_5$= H) | H | S | - | A-2 ($X_1$= $OCH_3$, $Y_1$= $CH_2$) |
| Q-3 | ($R_3$, $R_4$, $R_5$= H) | H | S | - | A-3 ($X_1$= $CH_3$) |
| Q-3 | ($R_3$, $R_4$, $R_5$= H) | H | O | - | A-4 ($X_1$= $CH_3$, $Y_2$= H) |
| Q-4 | ($R_3$, $R_4$, $R_5$= H) | H | - | - | A-2 ($X_1$= $OCH_3$, $Y_1$= O) |
| Q-4 | ($R_3$, $R_4$, $R_5$= H) | H | - | - | A-5 ($X_2$= $OCH_3$, $Y_3$= $CH_3$) |
| Q-4 | ($R_3$, $R_4$, $R_5$= H) | H | - | - | A-6 ($X_3$= $OCH_3$) |
| Q-7 | ($R_3$, $R_4$, $R_5$, $R_6$ = H) | H | - | - | A-2 ($X_1$ = $OCF_2H$, $Y_1$ =O) |
| Q-7 | ($R_3$, $R_4$, $R_5$, $R_6$ = H) | H | - | - | A-3 ($X_1$ = $OCH_2CH_3$) |
| Q-7 | ($R_3$, $R_4$, $R_5$, $R_6$ = H) | H | - | - | A-4 ($X_1$ = $OCH_3$, $Y_2$ = $CH_3$) |
| Q-7 | ($R_3$, $R_4$, $R_5$, $R_6$ = H) | H | - | - | A-5 ($X_2$ = $SCH_3$, $Y_3$ = $CH_3$) |
| Q-7 | ($R_3$, $R_4$, $R_5$, $R_6$ = H) | H | - | - | A-6 ($X_3$ = $OCH_3$) |
| Q-10 | ($R_3$, $R_4$ = H) | H | - | O | A-2 ($X_1$ = $OCH_3$, $Y_1$ = O) |
| Q-10 | ($R_3$, $R_4$ = H) | H | - | O | A-3 ($X_1$ = $OCH_3$) |
| Q-10 | ($R_3$, $R_4$ = H) | H | - | S | A-4 ($X_1$ = $CH_3$, $Y_2$ = $CH_3$) |
| Q-11 | ($R_3$, $R_4$ = H) | H | - | O | A-2 ($X_1$ = $OCH_3$, $Y_1$ = $CH_2$) |
| Q-11 | ($R_3$, $R_4$ = H) | H | - | O | A-3 ($X_1$ = $CH_3$) |
| Q-11 | ($R_3$, $R_4$ = H) | H | - | S | A-4 ($X_1$ = $OCH_3$, $Y_2$ = H) |
| Q-16 | ($R_3$, $R_4$, $R_5$, $R_6$ = H) | H | O | - | A-2 ($X_1$ = $CH_3$, $Y_1$ = O) |
| Q-16 | ($R_3$, $R_4$, $R_5$, $R_6$ = H) | H | O | - | A-3 ($X_1$ = $CH_3$) |
| Q-16 | ($R_3$, $R_4$, $R_5$, $R_6$ = H) | H | O | - | A-5 ($X_2$ = $OCH_3$, $Y_3$= $CH_3$) |
| Q-16 | ($R_3$, $R_4$, $R_5$, $R_6$ = H) | H | S | - | A-4 ($X_1$ = $CH_3$, $Y_2$= $CH_3$) |

Table 10 (continued)

| Q | | R | W' | W" | A |
|---|---|---|---|---|---|
| Q-16 | ($R_3$, $R_4$, $R_5$, $R_6$ = H) | H | S | - | A-5 ($X_2$ = $OCH_3$, $Y_3$= $CH_2CH_3$) |
| Q-16 | ($R_3$, $R_4$, $R_5$, $R_6$ = H) | H | S | - | A-6 ($X_3$ = $OCH_3$) |
| Q-20 | ($R_3$' = H) | H | S | - | A-2 ($X_1$ = $OCH_3$, $Y_1$ = O) |
| Q-20 | ($R_3$' = H) | H | O | - | A-2 ($X_1$ = $OCH_3$) |
| Q-20 | ($R_3$' = $CH_3$) | H | S | - | A-4 ($X_1$ = $OCH_3$, $Y_2$ = H) |
| Q-20 | ($R_3$' = $CH_3$) | H | O | - | A-2 ($X_1$ = $OCH_3$, $Y_1$ = O) |
| Q-20 | ($R_3$' = $SCH_3$) | H | S | - | A-3 ($X_1$ = $OCH_3$) |
| Q-20 | ($R_3$' = $SCH_3$) | H | O | - | A-4 ($X_1$ = $OCH_3$, $Y_2$ = $CH_3$) |
| Q-23 | ($R_3$ = H) | H | - | - | A-3 ($X_1$ = $CH_3$) |
| Q-23 | ($R_3$ = H) | H | - | - | A-4 ($X_1$ = $OCH_3$, $Y_2$ = $CH_3$) |
| Q-23 | ($R_3$ = $CH_3$) | H | - | - | A-6 ($X_3$ = $OCH_3$) |
| Q-28 | ($R_3$" = H, $R_4$ = H) | H | - | - | A-2 ($X_1$= $CH_3$, $Y_1$ = O) |
| Q-28 | ($R_3$" = Cl, $R_4$ = H) | H | - | - | A-3 ($X_1$= $OCH_3$) |
| Q-28 | ($R_3$" = $CH_3$, $R_4$ = H) | H | - | - | A-4 ($X_1$= $OCH_3$, $Y_2$ = $CH_3$) |
| Q-28 | ($R_3$" = H, $R_4$ = H) | H | - | - | A-5 ($X_2$= $CH_3$, $Y_2$ = $CH_3$) |
| Q-28 | ($R_3$" = H, $R_4$ = H) | H | - | - | A-6 ($X_3$= $CH_3$) |
| Q-33 | ($R_3$, $R_4$ = H, n = O) | H | - | - | A-2 ($X_1$= $OCH_3$, $Y_1$ = $CH_2$) |
| Q-33 | ($R_3$, $R_4$ = H, n = O) | H | - | - | A-3 ($X_1$= $CH_3$) |
| Q-33 | ($R_3$, $R_4$ = H, n = O) | H | - | - | A-4 ($X_1$= $OCH_3$, $Y_2$ = $CH_3$) |
| Q-36 | ($R_3$, $R_4$, $R_5$ = H) | H | - | - | A-2 ($X_1$ = $OCH_3$, $Y_1$ = O) |
| Q-36 | ($R_3$, $R_4$, $R_5$ = H) | H | - | - | A-3 ($X_1$ = $OCH_3$) |
| Q-36 | ($R_3$, $R_4$, $R_5$ = H) | H | - | - | A-4 ($X_1$ = $OCH_3$, $Y_2$ = $CH_3$) |
| Q-39 | ($R_3$, $R_4$ = H) | H | - | - | A-2 ($X_1$= $CH_3$, $Y_1$ = $CH_2$) |
| Q-39 | ($R_3$, $R_4$ = H) | H | - | - | A-3 ($X_1$= $CH_3$) |
| Q-39 | ($R_3$, $R_4$ = H) | H | - | - | A-4 ($X_1$= $OCH_3$, $Y_2$ = H) |

W is O, unless indicated by * where W is S in all Tables.

Table 11

General Structure 5

| | Q | R | W' | W" | A |
|---|---|---|---|---|---|
| Q-2 | ($R_3$, $R_4$, $R_5$= H) | H | S | - | A-2 ($X_1$= $CH_3$, $Y_1$= O) |
| Q-2 | ($R_3$, $R_4$, $R_5$= H) | H | S | - | A-3 ($X_1$= $OCH_3$) |
| Q-2 | ($R_3$, $R_4$, $R_5$= H) | H | O | - | A-4 ($X_1$, $Y_2$= $CH_3$) |
| Q-3 | ($R_3$, $R_4$, $R_5$= H) | H | S | - | A-2 ($X_1$= $OCH_3$, $Y_1$= $CH_2$) |
| Q-3 | ($R_3$, $R_4$, $R_5$= H) | H | S | - | A-3 ($X_1$= $CH_3$) |
| Q-3 | ($R_3$, $R_4$, $R_5$= H) | H | O | - | A-4 ($X_1$= $CH_3$, $Y_2$= H) |
| Q-4 | ($R_3$, $R_4$, $R_5$= H) | H | - | - | A-2 ($X_1$= $OCH_3$, $Y_1$= O) |
| Q-4 | ($R_3$, $R_4$, $R_5$= H) | H | - | - | A-5 ($X_2$= $OCH_3$, $Y_3$= $CH_3$) |
| Q-4 | ($R_3$, $R_4$, $R_5$= H) | H | - | - | A-6 ($X_3$= $OCH_3$) |
| Q-7 | ($R_3$, $R_4$, $R_5$, $R_6$ = H) | H | - | - | A-2 ($X_1$ = $OCF_2H$, $Y_1$ =O) |
| Q-7 | ($R_3$, $R_4$, $R_5$, $R_6$ = H) | H | - | - | A-3 ($X_1$ = $OCH_2CH_3$) |
| Q-7 | ($R_3$, $R_4$, $R_5$, $R_6$ = H) | H | - | - | A-4 ($X_1$ = $OCH_3$, $Y_2$ = $CH_3$) |
| Q-7 | ($R_3$, $R_4$, $R_5$, $R_6$ = H) | H | - | - | A-5 ($X_2$ = $SCH_3$, $Y_3$ = $CH_3$) |
| Q-7 | ($R_3$, $R_4$, $R_5$, $R_6$ = H) | H | - | - | A-6 ($X_3$ = $OCH_3$) |
| Q-10 | ($R_3$, $R_4$ = H) | H | - | O | A-2 ($X_1$ = $OCH_3$, $Y_1$ = O) |
| Q-10 | ($R_3$, $R_4$ = H) | H | - | O | A-3 ($X_1$ = $OCH_3$) |
| Q-10 | ($R_3$, $R_4$ = H) | H | - | S | A-4 ($X_1$ = $CH_3$, $Y_2$ = $CH_3$) |
| Q-11 | ($R_3$, $R_4$ = H) | H | - | O | A-2 ($X_1$ = $OCH_3$, $Y_1$ = $CH_2$) |
| Q-11 | ($R_3$, $R_4$ = H) | H | - | O | A-3 ($X_1$ = $CH_3$) |
| Q-11 | ($R_3$, $R_4$ = H) | H | - | S | A-4 ($X_1$ = $OCH_3$, $Y_2$ = H) |
| Q-16 | ($R_3$, $R_4$, $R_5$, $R_6$ = H) | H | O | - | A-2 ($X_1$ = $CH_3$, $Y_1$ = O) |
| Q-16 | ($R_3$, $R_4$, $R_5$, $R_6$ = H) | H | O | - | A-3 ($X_1$ = $CH_3$) |
| Q-16 | ($R_3$, $R_4$, $R_5$, $R_6$ = H) | H | O | - | A-5 ($X_2$ = $OCH_3$, $Y_3$= $CH_3$) |
| Q-16 | ($R_3$, $R_4$, $R_5$, $R_6$ = H) | H | S | - | A-4 ($X_1$ = $CH_3$, $Y_2$= $CH_3$) |

Table 11 (continued)

| Q | | R | W' | W" | A |
|---|---|---|---|---|---|
| Q-16 | ($R_3$, $R_4$, $R_5$, $R_6$ = H) | H | S | - | A-5 ($X_2$ = $OCH_3$, $Y_3$= $CH_2CH_3$) |
| Q-16 | ($R_3$, $R_4$, $R_5$, $R_6$ = H) | H | S | - | A-6 ($X_3$ = $OCH_3$) |
| Q-20 | ($R_3$' = H) | H | S | - | A-2 ($X_1$ = $OCH_3$, $Y_1$ = O) |
| Q-20 | ($R_3$' = H) | H | O | - | A-2 ($X_1$ = $OCH_3$) |
| Q-20 | ($R_3$' = $CH_3$) | H | S | - | A-4 ($X_1$ = $OCH_3$, $Y_2$ = H) |
| Q-20 | ($R_3$' = $CH_3$) | H | O | - | A-2 ($X_1$ = $OCH_3$, $Y_1$ = O) |
| Q-20 | ($R_3$' = $SCH_3$) | H | S | - | A-3 ($X_1$ = $OCH_3$) |
| Q-20 | ($R_3$' = $SCH_3$) | H | O | - | A-4 ($X_1$ = $OCH_3$, $Y_2$ = $CH_3$) |
| Q-23 | ($R_3$ = H) | H | - | - | A-3 ($X_1$ = $CH_3$) |
| Q-23 | ($R_3$ = H) | H | - | - | A-4 ($X_1$ = $OCH_3$, $Y_2$ = $CH_3$) |
| Q-23 | ($R_3$ = $CH_3$) | H | - | - | A-6 ($X_3$ = $OCH_3$) |
| Q-28 | ($R_3$" = H, $R_4$ = H) | H | - | - | A-2 ($X_1$= $CH_3$, $Y_1$ = O) |
| Q-28 | ($R_3$" = Cl, $R_4$ = H) | H | - | - | A-3 ($X_1$= $OCH_3$) |
| Q-28 | ($R_3$" = $CH_3$, $R_4$ = H) | H | - | - | A-4 ($X_1$= $OCH_3$, $Y_2$ = $CH_3$) |
| Q-28 | ($R_3$" = H, $R_4$ = H) | H | - | - | A-5 ($X_2$= $CH_3$, $Y_2$ = $CH_3$) |
| Q-28 | ($R_3$" = H, $R_4$ = H) | H | - | - | A-6 ($X_3$= $CH_3$) |
| Q-33 | ($R_3$, $R_4$ = H, n = O) | H | - | - | A-2 ($X_1$= $OCH_3$, $Y_1$ = $CH_2$) |
| Q-33 | ($R_3$, $R_4$ = H, n = O) | H | - | - | A-3 ($X_1$= $CH_3$) |
| Q-33 | ($R_3$, $R_4$ = H, n = O) | H | - | - | A-4 ($X_1$= $OCH_3$, $Y_2$ = $CH_3$) |
| Q-36 | ($R_3$, $R_4$, $R_5$ = H) | H | - | - | A-2 ($X_1$ = $OCH_3$, $Y_1$ = O) |
| Q-36 | ($R_3$, $R_4$, $R_5$ = H) | H | - | - | A-3 ($X_1$ = $OCH_3$) |
| Q-36 | ($R_3$, $R_4$, $R_5$ = H) | H | - | - | A-4 ($X_1$ = $OCH_3$, $Y_2$ = $CH_3$) |
| Q-39 | ($R_3$, $R_4$ = H) | H | - | - | A-2 ($X_1$= $CH_3$, $Y_1$ = $CH_2$) |
| Q-39 | ($R_3$, $R_4$ = H) | H | - | - | A-3 ($X_1$= $CH_3$) |
| Q-39 | ($R_3$, $R_4$ = H) | H | - | - | A-4 ($X_1$= $OCH_3$, $Y_2$ = H) |

Table 12

General Structure 6

| | Q | R | W' | W'' | A |
|---|---|---|---|---|---|
| Q-2 | ($R_3$, $R_4$, $R_5$= H) | H | S | - | A-2 ($X_1$= $CH_3$, $Y_1$= O) |
| Q-2 | ($R_3$, $R_4$, $R_5$= H) | H | S | - | A-3 ($X_1$= $OCH_3$) |
| Q-2 | ($R_3$, $R_4$, $R_5$= H) | H | O | - | A-4 ($X_1$, $Y_2$= $CH_3$) |
| Q-3 | ($R_3$, $R_4$, $R_5$= H) | H | S | - | A-2 ($X_1$= $OCH_3$, $Y_1$= $CH_2$) |
| Q-3 | ($R_3$, $R_4$, $R_5$= H) | H | S | - | A-3 ($X_1$= $CH_3$) |
| Q-3 | ($R_3$, $R_4$, $R_5$= H) | H | O | - | A-4 ($X_1$= $CH_3$, $Y_2$= H) |
| Q-4 | ($R_3$, $R_4$, $R_5$= H) | H | - | - | A-2 ($X_1$= $OCH_3$, $Y_1$= O) |
| Q-4 | ($R_3$, $R_4$, $R_5$= H) | H | - | - | A-5 ($X_2$= $OCH_3$, $Y_3$= $CH_3$) |
| Q-4 | ($R_3$, $R_4$, $R_5$= H) | H | - | - | A-6 ($X_3$= $OCH_3$) |
| Q-7 | ($R_3$, $R_4$, $R_5$, $R_6$ = H) | H | - | - | A-2 ($X_1$ = $OCF_2H$, $Y_1$ =O) |
| Q-7 | ($R_3$, $R_4$, $R_5$, $R_6$ = H) | H | - | - | A-3 ($X_1$ = $OCH_2CH_3$) |
| Q-7 | ($R_3$, $R_4$, $R_5$, $R_6$ = H) | H | - | - | A-4 ($X_1$ = $OCH_3$, $Y_2$ = $CH_3$) |
| Q-7 | ($R_3$, $R_4$, $R_5$, $R_6$ = H) | H | - | - | A-5 ($X_2$ = $SCH_3$, $Y_3$ = $CH_3$) |
| Q-7 | ($R_3$, $R_4$, $R_5$, $R_6$ = H) | H | - | - | A-6 ($X_3$ = $OCH_3$) |
| Q-10 | ($R_3$, $R_4$ = H) | H | - | O | A-2 ($X_1$ = $OCH_3$, $Y_1$ = O) |
| Q-10 | ($R_3$, $R_4$ = H) | H | - | O | A-3 ($X_1$ = $OCH_3$) |
| Q-10 | ($R_3$, $R_4$ = H) | H | - | S | A-4 ($X_1$ = $CH_3$, $Y_2$ = $CH_3$) |
| Q-11 | ($R_3$, $R_4$ = H) | H | - | O | A-2 ($X_1$ = $OCH_3$, $Y_1$ = $CH_2$) |
| Q-11 | ($R_3$, $R_4$ = H) | H | - | O | A-3 ($X_1$ = $CH_3$) |
| Q-11 | ($R_3$, $R_4$ = H) | H | - | S | A-4 ($X_1$ = $OCH_3$, $Y_2$ = H) |
| Q-16 | ($R_3$, $R_4$, $R_5$, $R_6$ = H) | H | O | - | A-2 ($X_1$ = $CH_3$, $Y_1$ = O) |
| Q-16 | ($R_3$, $R_4$, $R_5$, $R_6$ = H) | H | O | - | A-3 ($X_1$ = $CH_3$) |
| Q-16 | ($R_3$, $R_4$, $R_5$, $R_6$ = H) | H | O | - | A-5 ($X_2$ = $OCH_3$, $Y_3$= $CH_3$) |
| Q-16 | ($R_3$, $R_4$, $R_5$, $R_6$ = H) | H | S | - | A-4 ($X_1$ = $CH_3$, $Y_2$= $CH_3$) |

Table 12 (continued)

| Q | | R | W' | W" | A |
|---|---|---|---|---|---|
| Q-16 | ($R_3$, $R_4$, $R_5$, $R_6$ = H) | H | S | - | A-5 ($X_2$ = $OCH_3$, $Y_3$= $CH_2CH_3$) |
| Q-16 | ($R_3$, $R_4$, $R_5$, $R_6$ = H) | H | S | - | A-6 ($X_3$ = $OCH_3$) |
| Q-20 | ($R_3$' = H) | H | S | - | A-2 ($X_1$ = $OCH_3$, $Y_1$ = O) |
| Q-20 | ($R_3$' = H) | H | O | - | A-2 ($X_1$ = $OCH_3$) |
| Q-20 | ($R_3$' = $CH_3$) | H | S | - | A-4 ($X_1$ = $OCH_3$, $Y_2$ = H) |
| Q-20 | ($R_3$' = $CH_3$) | H | O | - | A-2 ($X_1$ = $OCH_3$, $Y_1$ = O) |
| Q-20 | ($R_3$' = $SCH_3$) | H | S | - | A-3 ($X_1$ = $OCH_3$) |
| Q-20 | ($R_3$' = $SCH_3$) | H | O | - | A-4 ($X_1$ = $OCH_3$, $Y_2$ = $CH_3$) |
| Q-23 | ($R_3$ = H) | H | - | - | A-3 ($X_1$ = $CH_3$) |
| Q-23 | ($R_3$ = H) | H | - | - | A-4 ($X_1$ = $OCH_3$, $Y_2$ = $CH_3$) |
| Q-23 | ($R_3$ = $CH_3$) | H | - | - | A-6 ($X_3$ = $OCH_3$) |
| Q-28 | ($R_3$" = H, $R_4$ = H) | H | - | - | A-2 ($X_1$= $CH_3$, $Y_1$ = O) |
| Q-28 | ($R_3$" = Cl, $R_4$ = H) | H | - | - | A-3 ($X_1$= $OCH_3$) |
| Q-28 | ($R_3$" = $CH_3$, $R_4$ = H) | H | - | - | A-4 ($X_1$= $OCH_3$, $Y_2$ = $CH_3$) |
| Q-28 | ($R_3$" = H, $R_4$ = H) | H | - | - | A-5 ($X_2$= $CH_3$, $Y_2$ = $CH_3$) |
| Q-28 | ($R_3$" = H, $R_4$ = H) | H | - | - | A-6 ($X_3$= $CH_3$) |
| Q-33 | ($R_3$, $R_4$ = H, n = O) | H | - | - | A-2 ($X_1$= $OCH_3$, $Y_1$ = $CH_2$) |
| Q-33 | ($R_3$, $R_4$ = H, n = O) | H | - | - | A-3 ($X_1$= $CH_3$) |
| Q-33 | ($R_3$, $R_4$ = H, n = O) | H | - | - | A-4 ($X_1$= $OCH_3$, $Y_2$ = $CH_3$) |
| Q-36 | ($R_3$, $R_4$, $R_5$ = H) | H | - | - | A-2 ($X_1$ = $OCH_3$, $Y_1$ = O) |
| Q-36 | ($R_3$, $R_4$, $R_5$ = H) | H | - | - | A-3 ($X_1$ = $OCH_3$) |
| Q-36 | ($R_3$, $R_4$, $R_5$ = H) | H | - | - | A-4 ($X_1$ = $OCH_3$, $Y_2$ = $CH_3$) |
| Q-39 | ($R_3$, $R_4$ = H) | H | - | - | A-2 ($X_1$= $CH_3$, $Y_1$ = $CH_2$) |
| Q-39 | ($R_3$, $R_4$ = H) | H | - | - | A-3 ($X_1$= $CH_3$) |
| Q-39 | ($R_3$, $R_4$ = H) | H | - | - | A-4 ($X_1$= $OCH_3$, $Y_2$ = H) |

Table 13

General Structure 7

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-7 | 1 | H | - | - | H | - | H | H | H | O | - | $CH_3$ | $CH_3$ | CH |
| Q-7 | 1 | H | - | - | H | - | H | H | H | O | - | $CH_3$ | $OCH_3$ | CH |
| Q-7 | 1 | H | - | - | H | - | H | H | H | O | - | $OCH_3$ | $OCH_3$ | CH |
| Q-7 | 1 | H | - | - | H | - | H | H | H | O | - | $CH_3$ | $OCH_3$ | N |
| Q-7 | 1 | H | - | - | H | - | H | H | H | O | - | $OCH_3$ | $OCH_3$ | N |
| Q-7 | 1 | H | - | - | H | - | H | H | H | O | - | $OCH_3$ | Cl | CH |
| Q-7 | 1 | H | - | - | H | - | H | H | H | S | - | $OCH_3$ | $OCH_3$ | CH |
| Q-7 | 2 | H | - | - | H | - | H | H | H | O | - | $CH_3$ | $CH_3$ | CH |
| Q-7 | 2 | H | - | - | H | - | H | H | H | O | - | $OCH_3$ | $OCH_3$ | CH |
| Q-7 | 2 | H | - | - | H | - | H | H | H | O | - | $OCH_3$ | $OCH_3$ | CH |
| Q-7 | 2 | H | - | - | H | - | H | H | H | O | - | $OCH_3$ | CH | N |
| Q-7 | 2 | H | - | - | H | - | H | H | H | O | - | $OCH_3$ | $OCH_3$ | N |
| Q-7 | 2 | H | - | - | H | - | H | H | H | O | - | $OCH_3$ | Cl | CH |
| Q-7 | 2 | H | - | - | H | - | H | H | H | S | - | $OCH_3$ | $OCH_3$ | CH |
| Q-11 | 1 | H | - | - | H | - | H | - | - | O | O | $CH_3$ | $CH_3$ | CH |
| Q-11 | 1 | H | - | - | H | - | H | - | - | O | O | $CH_3$ | $OCH_3$ | CH |

Table 13 (continued)

| Q | n | R | R1 | R2 | R3 | R3' | R4 | R5 | R6 | W | W' or W" | X | Y | Z |
|---|---|---|---|---|---|---|---|---|---|---|---|---|---|---|
| Q-11 | 1 | H | - | - | H | - | H | - | - | O | O | $OCH_3$ | $OCH_3$ | CH |
| Q-11 | 1 | H | - | - | H | - | H | - | - | O | O | $CH_3$ | $OCH_3$ | N |
| Q-11 | 1 | H | - | - | H | - | H | - | - | O | O | $OCH_3$ | $OCH_3$ | N |
| Q-11 | 1 | H | - | - | H | - | H | - | - | O | O | $OCH_3$ | Cl | CH |
| Q-11 | 1 | H | - | - | H | - | H | - | - | S | O | $OCH_3$ | $OCH_3$ | CH |
| Q-11 | 2 | H | - | - | H | - | H | - | - | O | O | $CH_3$ | $CH_3$ | CH |
| Q-11 | 2 | H | - | - | H | - | H | - | - | O | O | $CH_3$ | $OCH_3$ | CH |
| Q-11 | 2 | H | - | - | H | - | H | - | - | O | O | $OCH_3$ | $OCH_3$ | CH |
| Q-11 | 2 | H | - | - | H | - | H | - | - | O | O | $OCH_3$ | $CH_3$ | N |
| Q-11 | 2 | H | - | - | H | - | H | - | - | O | O | $OCH_3$ | $OCH_3$ | N |
| Q-11 | 2 | H | - | - | H | - | H | - | - | O | O | $OCH_3$ | Cl | CH |
| Q-11 | 2 | H | - | - | H | - | H | - | - | S | O | $OCH_3$ | $OCH_3$ | CH |
| Q-15 | 1 | H | - | - | - | $CH_3$ | H | - | - | O | O | $CH_3$ | $CH_3$ | CH |
| Q-15 | 1 | H | - | - | - | $CH_3$ | H | - | - | O | O | $CH_3$ | $OCH_3$ | CH |
| Q-15 | 1 | H | - | - | - | $CH_3$ | H | - | - | O | O | $OCH_3$ | $OCH_3$ | CH |
| Q-15 | 1 | H | - | - | - | $CH_3$ | H | - | - | O | O | $CH_3$ | $OCH_3$ | N |
| Q-15 | 1 | H | - | - | - | $CH_3$ | H | - | - | O | O | $OCH_3$ | $OCH_3$ | N |
| Q-15 | 1 | H | - | - | - | $CH_3$ | H | - | - | O | O | $OCH_3$ | Cl | CH |
| Q-15 | 1 | H | - | - | - | $CH_3$ | H | - | - | S | O | $OCH_3$ | $OCH_3$ | CH |
| Q-15 | 2 | H | - | - | - | $CH_3$ | H | - | - | O | O | $CH_3$ | $CH_3$ | CH |
| Q-15 | 2 | H | - | - | - | $CH_3$ | H | - | - | O | O | $OCH_3$ | $CH_3$ | CH |
| Q-15 | 2 | H | - | - | - | $CH_3$ | H | - | - | O | O | $OCH_3$ | $OCH_3$ | CH |
| Q-15 | 2 | H | - | - | - | $CH_3$ | H | - | - | O | O | $OCH_3$ | $CH_3$ | N |
| Q-15 | 2 | H | - | - | - | $CH_3$ | H | - | - | O | O | $OCH_3$ | $OCH_3$ | N |
| Q-15 | 2 | H | - | - | - | $CH_3$ | H | - | - | O | O | $OCH_3$ | Cl | CH |
| Q-15 | 2 | H | - | - | - | $CH_3$ | H | - | - | S | O | $OCH_3$ | $OCH_3$ | CH |
| Q-15 | 1 | H | - | - | - | $CH_3$ | H | - | - | O | S | $CH_3$ | $CH_3$ | CH |
| Q-15 | 1 | H | - | - | - | $CH_3$ | H | - | - | O | S | $CH_3$ | $OCH_3$ | CH |
| Q-15 | 1 | H | - | - | - | $CH_3$ | H | - | - | O | S | $OCH_3$ | $OCH_3$ | CH |
| Q-15 | 1 | H | - | - | - | $CH_3$ | H | - | - | O | S | $CH_3$ | $OCH_3$ | N |
| Q-15 | 1 | H | - | - | - | $CH_3$ | H | - | - | O | S | $OCH_3$ | $OCH_3$ | N |

Table 13 (continued)

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-15 | 1 | H | – | – | – | $CH_3$ | H | – | – | O | S | $OCH_3$ | Cl | CH |
| Q-15 | 1 | H | – | – | – | $CH_3$ | H | – | – | S | S | $OCH_3$ | $OCH_3$ | CH |
| Q-15 | 2 | H | – | – | – | $CH_3$ | H | – | – | O | S | $CH_3$ | $CH_3$ | CH |
| Q-15 | 2 | H | – | – | – | $CH_3$ | H | – | – | O | S | $CH_3$ | $OCH_3$ | CH |
| Q-15 | 2 | H | – | – | – | $CH_3$ | H | – | – | O | S | $OCH_3$ | $OCH_3$ | CH |
| Q-15 | 2 | H | – | – | – | $CH_3$ | H | – | – | O | S | $CH_3$ | $OCH_3$ | N |
| Q-15 | 2 | H | – | – | – | $CH_3$ | H | – | – | O | S | $OCH_3$ | $OCH_3$ | N |
| Q-15 | 2 | H | – | – | – | $CH_3$ | H | – | – | O | S | $OCH_3$ | Cl | CH |
| Q-15 | 2 | H | – | – | – | $CH_3$ | H | – | – | S | S | $OCH_3$ | $OCH_3$ | CH |
| Q-20 | 1 | H | – | – | – | $SCH_3$ | H | – | – | O | O | $CH_3$ | $CH_3$ | CH |
| Q-20 | 1 | H | – | – | – | $SCH_3$ | H | – | – | O | O | $CH_3$ | $OCH_3$ | CH |
| Q-20 | 1 | H | – | – | – | $SCH_3$ | H | – | – | O | O | $OCH_3$ | $OCH_3$ | CH |
| Q-20 | 1 | H | – | – | – | $SCH_3$ | H | – | – | O | O | $CH_3$ | $OCH_3$ | N |
| Q-20 | 1 | H | – | – | – | $SCH_3$ | H | – | – | O | O | $OCH_3$ | $OCH_3$ | N |
| Q-20 | 1 | H | – | – | – | $SCH_3$ | H | – | – | O | O | $OCH_3$ | Cl | CH |
| Q-20 | 1 | H | – | – | – | $SCH_3$ | H | – | – | S | O | $OCH_3$ | $OCH_3$ | CH |
| Q-20 | 2 | H | – | – | – | $SCH_3$ | H | – | – | O | O | $CH_3$ | $CH_3$ | CH |
| Q-20 | 2 | H | – | – | – | $SCH_3$ | H | – | – | O | O | $CH_3$ | $OCH_3$ | CH |
| Q-20 | 2 | H | – | – | – | $SCH_3$ | H | – | – | O | O | $OCH_3$ | $OCH_3$ | CH |
| Q-20 | 2 | H | – | – | – | $SCH_3$ | H | – | – | O | O | $CH_3$ | $OCH_3$ | N |
| Q-20 | 2 | H | – | – | – | $SCH_3$ | H | – | – | O | O | $OCH_3$ | $OCH_3$ | N |
| Q-20 | 2 | H | – | – | – | $SCH_3$ | H | – | – | O | O | $OCH_3$ | Cl | CH |
| Q-20 | 2 | H | – | – | – | $SCH_3$ | H | – | – | S | O | $OCH_3$ | $OCH_3$ | CH |
| Q-28 | 1 | H | – | – | – | Cl | H | – | – | O | – | $CH_3$ | $CH_3$ | CH |
| Q-28 | 1 | H | – | – | – | Cl | H | – | – | O | – | $CH_3$ | $OCH_3$ | CH |
| Q-28 | 1 | H | – | – | – | Cl | H | – | – | O | – | $OCH_3$ | $OCH_3$ | CH |
| Q-28 | 1 | H | – | – | – | Cl | H | – | – | O | – | $OCH_3$ | CH | N |
| Q-28 | 1 | H | – | – | – | Cl | H | – | – | O | – | $OCH_3$ | $OCH_3$ | N |
| Q-28 | 1 | H | – | – | – | Cl | H | – | – | O | – | $OCH_3$ | Cl | CH |
| Q-28 | 1 | H | – | – | – | Cl | H | – | – | S | – | $OCH_3$ | $OCH_3$ | CH |
| Q-28 | 2 | H | – | – | – | Cl | H | – | – | O | – | $CH_3$ | $CH_3$ | CH |

Table 13 (continued)

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-28 | 2 | H | - | - | - | Cl | H | - | - | O | - | $CH_3$ | $OCH_3$ | CH |
| Q-28 | 2 | H | - | - | - | Cl | H | - | - | O | - | $OCH_3$ | $OCH_3$ | CH |
| Q-28 | 2 | H | - | - | - | Cl | H | - | - | O | - | $OCH_3$ | CH | N |
| Q-28 | 2 | H | - | - | - | Cl | H | - | - | O | - | $OCH_3$ | $OCH_3$ | N |
| Q-28 | 2 | H | - | - | - | Cl | H | - | - | O | - | $OCH_3$ | Cl | CH |
| Q-28 | 2 | H | - | - | - | Cl | H | - | - | S | - | $OCH_3$ | $OCH_3$ | CH |
| Q-1 | 1 | $CH_3$ | H | H | - | - | - | - | - | O | - | $OCH_3$ | $OCH_3$ | CH |
| Q-1 | 2 | $CH_3$ | H | H | - | - | - | - | - | O | - | $OCH_3$ | $OCH_3$ | CH |
| Q-7 | 1 | $CH_3$ | - | - | H | - | H | H | H | O | - | $OCH_3$ | $OCH_3$ | CH |
| Q-7 | 2 | $CH_3$ | - | - | H | - | H | H | H | O | - | $OCH_3$ | $OCH_3$ | CH |
| Q-11 | 1 | $CH_3$ | - | - | H | - | H | - | - | O | O | $OCH_3$ | $OCH_3$ | CH |
| Q-11 | 2 | $CH_3$ | - | - | H | - | H | - | - | O | O | $OCH_3$ | $OCH_3$ | CH |
| Q-15 | 1 | $CH_3$ | - | - | - | $CH_3$ | H | - | - | O | O | $OCH_3$ | $OCH_3$ | CH |
| Q-15 | 2 | $CH_3$ | - | - | - | $CH_3$ | H | - | - | O | O | $OCH_3$ | $OCH_3$ | CH |
| Q-15 | 1 | $CH_3$ | - | - | - | $CH_3$ | H | - | - | O | S | $OCH_3$ | $OCH_3$ | CH |
| Q-15 | 2 | $CH_3$ | - | - | - | $CH_3$ | H | - | - | O | S | $OCH_3$ | $OCH_3$ | CH |
| Q-20 | 1 | $CH_3$ | - | - | - | $SCH_3$ | H | - | - | O | O | $OCH_3$ | $OCH_3$ | CH |
| Q-20 | 2 | $CH_3$ | - | - | - | $SCH_3$ | H | - | - | O | O | $OCH_3$ | $OCH_3$ | CH |
| Q-28 | 1 | $CH_3$ | - | - | - | Cl | H | - | - | O | - | $OCH_3$ | $OCH_3$ | CH |
| Q-28 | 2 | $CH_3$ | - | - | - | Cl | H | - | - | O | - | $OCH_3$ | $OCH_3$ | CH |

Table 14

General Structure 8

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-7 | 1 | H | - | - | H | - | H | H | H | O | - | $CH_3$ | $CH_3$ | CH |
| Q-7 | 1 | H | - | - | H | - | H | H | H | O | - | $CH_3$ | $OCH_3$ | CH |
| Q-7 | 1 | H | - | - | H | - | H | H | H | O | - | $OCH_3$ | $OCH_3$ | CH |
| Q-7 | 1 | H | - | - | H | - | H | H | H | O | - | $CH_3$ | $OCH_3$ | N |
| Q-7 | 1 | H | - | - | H | - | H | H | H | O | - | $OCH_3$ | $OCH_3$ | N |
| Q-7 | 1 | H | - | - | H | - | H | H | H | O | - | $OCH_3$ | Cl | CH |
| Q-7 | 1 | H | - | - | H | - | H | H | H | S | - | $OCH_3$ | $OCH_3$ | CH |
| Q-7 | 2 | H | - | - | H | - | H | H | H | O | - | $CH_3$ | $CH_3$ | CH |
| Q-7 | 2 | H | - | - | H | - | H | H | H | O | - | $OCH_3$ | $OCH_3$ | CH |
| Q-7 | 2 | H | - | - | H | - | H | H | H | O | - | $OCH_3$ | $OCH_3$ | CH |
| Q-7 | 2 | H | - | - | H | - | H | H | H | O | - | $OCH_3$ | CH | N |
| Q-7 | 2 | H | - | - | H | - | H | H | H | O | - | $OCH_3$ | $OCH_3$ | N |
| Q-7 | 2 | H | - | - | H | - | H | H | H | O | - | $OCH_3$ | Cl | CH |
| Q-7 | 2 | H | - | - | H | - | H | H | H | S | - | $OCH_3$ | $OCH_3$ | CH |
| Q-11 | 1 | H | - | - | H | - | H | - | - | O | O | $CH_3$ | $CH_3$ | CH |
| Q-11 | 1 | H | - | - | H | - | H | - | - | O | O | $CH_3$ | $OCH_3$ | CH |

Table 14 (continued)

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-11 | 1 | H | - | - | H | - | H | - | - | O | O | $OCH_3$ | $OCH_3$ | CH |
| Q-11 | 1 | H | - | - | H | - | H | - | - | O | O | $CH_3$ | $OCH_3$ | N |
| Q-11 | 1 | H | - | - | H | - | H | - | - | O | O | $OCH_3$ | $OCH_3$ | N |
| Q-11 | 1 | H | - | - | H | - | H | - | - | O | O | $OCH_3$ | Cl | CH |
| Q-11 | 1 | H | - | - | H | - | H | - | - | S | O | $OCH_3$ | $OCH_3$ | CH |
| Q-11 | 2 | H | - | - | H | - | H | - | - | O | O | $CH_3$ | $CH_3$ | CH |
| Q-11 | 2 | H | - | - | H | - | H | - | - | O | O | $CH_3$ | $OCH_3$ | CH |
| Q-11 | 2 | H | - | - | H | - | H | - | - | O | O | $OCH_3$ | $OCH_3$ | CH |
| Q-11 | 2 | H | - | - | H | - | H | - | - | O | O | $OCH_3$ | $CH_3$ | N |
| Q-11 | 2 | H | - | - | H | - | H | - | - | O | O | $OCH_3$ | $OCH_3$ | N |
| Q-11 | 2 | H | - | - | H | - | H | - | - | O | O | $OCH_3$ | Cl | CH |
| Q-11 | 2 | H | - | - | H | - | H | - | - | S | O | $OCH_3$ | $OCH_3$ | CH |
| Q-15 | 1 | H | - | - | - | $CH_3$ | H | - | - | O | O | $CH_3$ | $CH_3$ | CH |
| Q-15 | 1 | H | - | - | - | $CH_3$ | H | - | - | O | O | $CH_3$ | $OCH_3$ | CH |
| Q-15 | 1 | H | - | - | - | $CH_3$ | H | - | - | O | O | $OCH_3$ | $OCH_3$ | CH |
| Q-15 | 1 | H | - | - | - | $CH_3$ | H | - | - | O | O | $CH_3$ | $OCH_3$ | N |
| Q-15 | 1 | H | - | - | - | $CH_3$ | H | - | - | O | O | $OCH_3$ | $OCH_3$ | N |
| Q-15 | 1 | H | - | - | - | $CH_3$ | H | - | - | O | O | $OCH_3$ | Cl | CH |
| Q-15 | 1 | H | - | - | - | $CH_3$ | H | - | - | S | O | $OCH_3$ | $OCH_3$ | CH |
| Q-15 | 2 | H | - | - | - | $CH_3$ | H | - | - | O | O | $CH_3$ | $CH_3$ | CH |
| Q-15 | 2 | H | - | - | - | $CH_3$ | H | - | - | O | O | $OCH_3$ | $CH_3$ | CH |
| Q-15 | 2 | H | - | - | - | $CH_3$ | H | - | - | O | O | $OCH_3$ | $OCH_3$ | CH |
| Q-15 | 2 | H | - | - | - | $CH_3$ | H | - | - | O | O | $OCH_3$ | $CH_3$ | N |
| Q-15 | 2 | H | - | - | - | $CH_3$ | H | - | - | O | O | $OCH_3$ | $OCH_3$ | N |
| Q-15 | 2 | H | - | - | - | $CH_3$ | H | - | - | O | O | $OCH_3$ | Cl | CH |
| Q-15 | 2 | H | - | - | - | $CH_3$ | H | - | - | S | O | $OCH_3$ | $OCH_3$ | CH |
| Q-15 | 1 | H | - | - | - | $CH_3$ | H | - | - | O | S | $CH_3$ | $CH_3$ | CH |
| Q-15 | 1 | H | - | - | - | $CH_3$ | H | - | - | O | S | $CH_3$ | $OCH_3$ | CH |
| Q-15 | 1 | H | - | - | - | $CH_3$ | H | - | - | O | S | $OCH_3$ | $OCH_3$ | CH |
| Q-15 | 1 | H | - | - | - | $CH_3$ | H | - | - | O | S | $CH_3$ | $OCH_3$ | N |
| Q-15 | 1 | H | - | - | - | $CH_3$ | H | - | - | O | S | $OCH_3$ | $OCH_3$ | N |

Table 14 (continued)

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-15 | 1 | H | - | - | - | $CH_3$ | H | - | - | O | S | $OCH_3$ | Cl | CH |
| Q-15 | 1 | H | - | - | - | $CH_3$ | H | - | - | S | S | $OCH_3$ | $OCH_3$ | CH |
| Q-15 | 2 | H | - | - | - | $CH_3$ | H | - | - | O | S | $CH_3$ | $CH_3$ | CH |
| Q-15 | 2 | H | - | - | - | $CH_3$ | H | - | - | O | S | $CH_3$ | $OCH_3$ | CH |
| Q-15 | 2 | H | - | - | - | $CH_3$ | H | - | - | O | S | $OCH_3$ | $OCH_3$ | CH |
| Q-15 | 2 | H | - | - | - | $CH_3$ | H | - | - | O | S | $CH_3$ | $OCH_3$ | N |
| Q-15 | 2 | H | - | - | - | $CH_3$ | H | - | - | O | S | $OCH_3$ | $OCH_3$ | N |
| Q-15 | 2 | H | - | - | - | $CH_3$ | H | - | - | O | S | $OCH_3$ | Cl | CH |
| Q-15 | 2 | H | - | - | - | $CH_3$ | H | - | - | S | S | $OCH_3$ | $OCH_3$ | CH |
| Q-20 | 1 | H | - | - | - | $SCH_3$ | H | - | - | O | O | $CH_3$ | $CH_3$ | CH |
| Q-20 | 1 | H | - | - | - | $SCH_3$ | H | - | - | O | O | $CH_3$ | $OCH_3$ | CH |
| Q-20 | 1 | H | - | - | - | $SCH_3$ | H | - | - | O | O | $OCH_3$ | $OCH_3$ | CH |
| Q-20 | 1 | H | - | - | - | $SCH_3$ | H | - | - | O | O | $CH_3$ | $OCH_3$ | N |
| Q-20 | 1 | H | - | - | - | $SCH_3$ | H | - | - | O | O | $OCH_3$ | $OCH_3$ | N |
| Q-20 | 1 | H | - | - | - | $SCH_3$ | H | - | - | O | O | $OCH_3$ | Cl | CH |
| Q-20 | 1 | H | - | - | - | $SCH_3$ | H | - | - | S | O | $OCH_3$ | $OCH_3$ | CH |
| Q-20 | 2 | H | - | - | - | $SCH_3$ | H | - | - | O | O | $CH_3$ | $CH_3$ | CH |
| Q-20 | 2 | H | - | - | - | $SCH_3$ | H | - | - | O | O | $CH_3$ | $OCH_3$ | CH |
| Q-20 | 2 | H | - | - | - | $SCH_3$ | H | - | - | O | O | $OCH_3$ | $OCH_3$ | CH |
| Q-20 | 2 | H | - | - | - | $SCH_3$ | H | - | - | O | O | $CH_3$ | $OCH_3$ | N |
| Q-20 | 2 | H | - | - | - | $SCH_3$ | H | - | - | O | O | $OCH_3$ | $OCH_3$ | N |
| Q-20 | 2 | H | - | - | - | $SCH_3$ | H | - | - | O | O | $OCH_3$ | Cl | CH |
| Q-20 | 2 | H | - | - | - | $SCH_3$ | H | - | - | S | O | $OCH_3$ | $OCH_3$ | CH |
| Q-28 | 1 | H | - | - | - | Cl | H | - | - | O | - | $CH_3$ | $CH_3$ | CH |
| Q-28 | 1 | H | - | - | - | Cl | H | - | - | O | - | $CH_3$ | $OCH_3$ | CH |
| Q-28 | 1 | H | - | - | - | Cl | H | - | - | O | - | $OCH_3$ | $OCH_3$ | CH |
| Q-28 | 1 | H | - | - | - | Cl | H | - | - | O | - | $OCH_3$ | CH | N |
| Q-28 | 1 | H | - | - | - | Cl | H | - | - | O | - | $OCH_3$ | $OCH_3$ | N |
| Q-28 | 1 | H | - | - | - | Cl | H | - | - | O | - | $OCH_3$ | Cl | CH |
| Q-28 | 1 | H | - | - | - | Cl | H | - | - | S | - | $OCH_3$ | $OCH_3$ | CH |
| Q-28 | 2 | H | - | - | - | Cl | H | - | - | O | - | $CH_3$ | $CH_3$ | CH |

Table 14 (continued)

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-28 | 2 | H | - | - | - | Cl | H | - | - | O | - | $CH_3$ | $OCH_3$ | CH |
| Q-28 | 2 | H | - | - | - | Cl | H | - | - | O | - | $OCH_3$ | $OCH_3$ | CH |
| Q-28 | 2 | H | - | - | - | Cl | H | - | - | O | - | $OCH_3$ | CH | N |
| Q-28 | 2 | H | - | - | - | Cl | H | - | - | O | - | $OCH_3$ | $OCH_3$ | N |
| Q-28 | 2 | H | - | - | - | Cl | H | - | - | O | - | $OCH_3$ | Cl | CH |
| Q-28 | 2 | H | - | - | - | Cl | H | - | - | S | - | $OCH_3$ | $OCH_3$ | CH |
| Q-1 | 1 | $CH_3$ | H | H | - | - | - | - | - | O | - | $OCH_3$ | $OCH_3$ | CH |
| Q-1 | 2 | $CH_3$ | H | H | - | - | - | - | - | O | - | $OCH_3$ | $OCH_3$ | CH |
| Q-7 | 1 | $CH_3$ | - | - | H | - | H | H | H | O | - | $OCH_3$ | $OCH_3$ | CH |
| Q-7 | 2 | $CH_3$ | - | - | H | - | H | H | H | O | - | $OCH_3$ | $OCH_3$ | CH |
| Q-11 | 1 | $CH_3$ | - | - | H | - | H | - | - | O | O | $OCH_3$ | $OCH_3$ | CH |
| Q-11 | 2 | $CH_3$ | - | - | H | - | H | - | - | O | O | $OCH_3$ | $OCH_3$ | CH |
| Q-15 | 1 | $CH_3$ | - | - | - | $CH_3$ | H | - | - | O | O | $OCH_3$ | $OCH_3$ | CH |
| Q-15 | 2 | $CH_3$ | - | - | - | $CH_3$ | H | - | - | O | O | $OCH_3$ | $OCH_3$ | CH |
| Q-15 | 1 | $CH_3$ | - | - | - | $CH_3$ | H | - | - | O | S | $OCH_3$ | $OCH_3$ | CH |
| Q-15 | 2 | $CH_3$ | - | - | - | $CH_3$ | H | - | - | O | S | $OCH_3$ | $OCH_3$ | CH |
| Q-20 | 1 | $CH_3$ | - | - | - | $SCH_3$ | H | - | - | O | O | $OCH_3$ | $OCH_3$ | CH |
| Q-20 | 2 | $CH_3$ | - | - | - | $SCH_3$ | H | - | - | O | O | $OCH_3$ | $OCH_3$ | CH |
| Q-28 | 1 | $CH_3$ | - | - | - | Cl | H | - | - | O | - | $OCH_3$ | $OCH_3$ | CH |
| Q-28 | 2 | $CH_3$ | - | - | - | Cl | H | - | - | O | - | $OCH_3$ | $OCH_3$ | CH |

Table 15

General Structure 9

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-7 | 1 | H | - | - | H | - | H | H | H | O | - | $CH_3$ | $CH_3$ | CH |
| Q-7 | 1 | H | - | - | H | - | H | H | H | O | - | $CH_3$ | $OCH_3$ | CH |
| Q-7 | 1 | H | - | - | H | - | H | H | H | O | - | $OCH_3$ | $OCH_3$ | CH |
| Q-7 | 1 | H | - | - | H | - | H | H | H | O | - | $CH_3$ | $OCH_3$ | N |
| Q-7 | 1 | H | - | - | H | - | H | H | H | O | - | $OCH_3$ | $OCH_3$ | N |
| Q-7 | 1 | H | - | - | H | - | H | H | H | O | - | $OCH_3$ | Cl | CH |
| Q-7 | 1 | H | - | - | H | - | H | H | H | S | - | $OCH_3$ | $OCH_3$ | CH |
| Q-7 | 2 | H | - | - | H | - | H | H | H | O | - | $CH_3$ | $CH_3$ | CH |
| Q-7 | 2 | H | - | - | H | - | H | H | H | O | - | $OCH_3$ | $OCH_3$ | CH |
| Q-7 | 2 | H | - | - | H | - | H | H | H | O | - | $OCH_3$ | $OCH_3$ | CH |
| Q-7 | 2 | H | - | - | H | - | H | H | H | O | - | $OCH_3$ | CH | N |
| Q-7 | 2 | H | - | - | H | - | H | H | H | O | - | $OCH_3$ | $OCH_3$ | N |
| Q-7 | 2 | H | - | - | H | - | H | H | H | O | - | $OCH_3$ | Cl | CH |
| Q-7 | 2 | H | - | - | H | - | H | H | H | S | - | $OCH_3$ | $OCH_3$ | CH |
| Q-11 | 1 | H | - | - | H | - | H | - | - | O | O | $CH_3$ | $CH_3$ | CH |
| Q-11 | 1 | H | - | - | H | - | H | - | - | O | O | $CH_3$ | $OCH_3$ | CH |

Table 15 (continued)

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-11 | 1 | H | - | - | H | - | H | - | - | O | O | $OCH_3$ | $OCH_3$ | CH |
| Q-11 | 1 | H | - | - | H | - | H | - | - | O | O | $CH_3$ | $OCH_3$ | N |
| Q-11 | 1 | H | - | - | H | - | H | - | - | O | O | $OCH_3$ | $OCH_3$ | N |
| Q-11 | 1 | H | - | - | H | - | H | - | - | O | O | $OCH_3$ | Cl | CH |
| Q-11 | 1 | H | - | - | H | - | H | - | - | S | O | $OCH_3$ | $OCH_3$ | CH |
| Q-11 | 2 | H | - | - | H | - | H | - | - | O | O | $CH_3$ | $CH_3$ | CH |
| Q-11 | 2 | H | - | - | H | - | H | - | - | O | O | $CH_3$ | $OCH_3$ | CH |
| Q-11 | 2 | H | - | - | H | - | H | - | - | O | O | $OCH_3$ | $OCH_3$ | CH |
| Q-11 | 2 | H | - | - | H | - | H | - | - | O | O | $OCH_3$ | $CH_3$ | N |
| Q-11 | 2 | H | - | - | H | - | H | - | - | O | O | $OCH_3$ | $OCH_3$ | N |
| Q-11 | 2 | H | - | - | H | - | H | - | - | O | O | $OCH_3$ | Cl | CH |
| Q-11 | 2 | H | - | - | H | - | H | - | - | S | O | $OCH_3$ | $OCH_3$ | CH |
| Q-15 | 1 | H | - | - | - | $CH_3$ | H | - | - | O | O | $CH_3$ | $CH_3$ | CH |
| Q-15 | 1 | H | - | - | - | $CH_3$ | H | - | - | O | O | $CH_3$ | $OCH_3$ | CH |
| Q-15 | 1 | H | - | - | - | $CH_3$ | H | - | - | O | O | $OCH_3$ | $OCH_3$ | CH |
| Q-15 | 1 | H | - | - | - | $CH_3$ | H | - | - | O | O | $CH_3$ | $OCH_3$ | N |
| Q-15 | 1 | H | - | - | - | $CH_3$ | H | - | - | O | O | $OCH_3$ | $OCH_3$ | N |
| Q-15 | 1 | H | - | - | - | $CH_3$ | H | - | - | O | O | $OCH_3$ | Cl | CH |
| Q-15 | 1 | H | - | - | - | $CH_3$ | H | - | - | S | O | $OCH_3$ | $OCH_3$ | CH |
| Q-15 | 2 | H | - | - | - | $CH_3$ | H | - | - | O | O | $CH_3$ | $CH_3$ | CH |
| Q-15 | 2 | H | - | - | - | $CH_3$ | H | - | - | O | O | $OCH_3$ | $CH_3$ | CH |
| Q-15 | 2 | H | - | - | - | $CH_3$ | H | - | - | O | O | $OCH_3$ | $OCH_3$ | CH |
| Q-15 | 2 | H | - | - | - | $CH_3$ | H | - | - | O | O | $OCH_3$ | $CH_3$ | N |
| Q-15 | 2 | H | - | - | - | $CH_3$ | H | - | - | O | O | $OCH_3$ | $OCH_3$ | N |
| Q-15 | 2 | H | - | - | - | $CH_3$ | H | - | - | O | O | $OCH_3$ | Cl | CH |
| Q-15 | 2 | H | - | - | - | $CH_3$ | H | - | - | S | O | $OCH_3$ | $OCH_3$ | CH |
| Q-15 | 1 | H | - | - | - | $CH_3$ | H | - | - | O | S | $CH_3$ | $CH_3$ | CH |
| Q-15 | 1 | H | - | - | - | $CH_3$ | H | - | - | O | S | $CH_3$ | $OCH_3$ | CH |
| Q-15 | 1 | H | - | - | - | $CH_3$ | H | - | - | O | S | $OCH_3$ | $OCH_3$ | CH |
| Q-15 | 1 | H | - | - | - | $CH_3$ | H | - | - | O | S | $CH_3$ | $OCH_3$ | N |
| Q-15 | 1 | H | - | - | - | $CH_3$ | H | - | - | O | S | $OCH_3$ | $OCH_3$ | N |

Table 15 (continued)

| Q | n | R | $R_1$ | $R_2$ | $R_3$ | $R_{3'}$ | $R_4$ | $R_5$ | $R_6$ | W | W' or W'' | X | Y | Z |
|---|---|---|---|---|---|---|---|---|---|---|---|---|---|---|
| Q-15 | 1 | H | - | - | - | $CH_3$ | H | - | - | O | S | $OCH_3$ | Cl | CH |
| Q-15 | 1 | H | - | - | - | $CH_3$ | H | - | - | S | S | $OCH_3$ | $OCH_3$ | CH |
| Q-15 | 2 | H | - | - | - | $CH_3$ | H | - | - | O | S | $CH_3$ | $CH_3$ | CH |
| Q-15 | 2 | H | - | - | - | $CH_3$ | H | - | - | O | S | $CH_3$ | $OCH_3$ | CH |
| Q-15 | 2 | H | - | - | - | $CH_3$ | H | - | - | O | S | $OCH_3$ | $OCH_3$ | CH |
| Q-15 | 2 | H | - | - | - | $CH_3$ | H | - | - | O | S | $CH_3$ | $OCH_3$ | N |
| Q-15 | 2 | H | - | - | - | $CH_3$ | H | - | - | O | S | $OCH_3$ | $OCH_3$ | N |
| Q-15 | 2 | H | - | - | - | $CH_3$ | H | - | - | O | S | $OCH_3$ | Cl | CH |
| Q-15 | 2 | H | - | - | - | $CH_3$ | H | - | - | S | S | $OCH_3$ | $OCH_3$ | CH |
| Q-20 | 1 | H | - | - | - | $SCH_3$ | H | - | - | O | O | $CH_3$ | $CH_3$ | CH |
| Q-20 | 1 | H | - | - | - | $SCH_3$ | H | - | - | O | O | $CH_3$ | $OCH_3$ | CH |
| Q-20 | 1 | H | - | - | - | $SCH_3$ | H | - | - | O | O | $OCH_3$ | $OCH_3$ | CH |
| Q-20 | 1 | H | - | - | - | $SCH_3$ | H | - | - | O | O | $CH_3$ | $OCH_3$ | N |
| Q-20 | 1 | H | - | - | - | $SCH_3$ | H | - | - | O | O | $OCH_3$ | $OCH_3$ | N |
| Q-20 | 1 | H | - | - | - | $SCH_3$ | H | - | - | O | O | $OCH_3$ | Cl | CH |
| Q-20 | 1 | H | - | - | - | $SCH_3$ | H | - | - | S | O | $OCH_3$ | $OCH_3$ | CH |
| Q-20 | 2 | H | - | - | - | $SCH_3$ | H | - | - | O | O | $CH_3$ | $CH_3$ | CH |
| Q-20 | 2 | H | - | - | - | $SCH_3$ | H | - | - | O | O | $CH_3$ | $OCH_3$ | CH |
| Q-20 | 2 | H | - | - | - | $SCH_3$ | H | - | - | O | O | $OCH_3$ | $OCH_3$ | CH |
| Q-20 | 2 | H | - | - | - | $SCH_3$ | H | - | - | O | O | $CH_3$ | $OCH_3$ | N |
| Q-20 | 2 | H | - | - | - | $SCH_3$ | H | - | - | O | O | $OCH_3$ | $OCH_3$ | N |
| Q-20 | 2 | H | - | - | - | $SCH_3$ | H | - | - | O | O | $OCH_3$ | Cl | CH |
| Q-20 | 2 | H | - | - | - | $SCH_3$ | H | - | - | S | O | $OCH_3$ | $OCH_3$ | CH |
| Q-28 | 1 | H | - | - | - | Cl | H | - | - | O | - | $CH_3$ | $CH_3$ | CH |
| Q-28 | 1 | H | - | - | - | Cl | H | - | - | O | - | $CH_3$ | $OCH_3$ | CH |
| Q-28 | 1 | H | - | - | - | Cl | H | - | - | O | - | $OCH_3$ | $OCH_3$ | CH |
| Q-28 | 1 | H | - | - | - | Cl | H | - | - | O | - | $OCH_3$ | CH | N |
| Q-28 | 1 | H | - | - | - | Cl | H | - | - | O | - | $OCH_3$ | $OCH_3$ | N |
| Q-28 | 1 | H | - | - | - | Cl | H | - | - | O | - | $OCH_3$ | Cl | CH |
| Q-28 | 1 | H | - | - | - | Cl | H | - | - | S | - | $OCH_3$ | $OCH_3$ | CH |
| Q-28 | 2 | H | - | - | - | Cl | H | - | - | O | - | $CH_3$ | $CH_3$ | CH |

Table 15 (continued)

| Q | n | R | $R_1$ | $R_2$ | $R_3$ | $R_{3'}$ | $R_4$ | $R_5$ | $R_6$ | W | W' or W" | X | Y | Z |
|---|---|---|---|---|---|---|---|---|---|---|---|---|---|---|
| Q-28 | 2 | H | - | - | - | Cl | H | - | - | O | - | $CH_3$ | $OCH_3$ | CH |
| Q-28 | 2 | H | - | - | - | Cl | H | - | - | O | - | $OCH_3$ | $OCH_3$ | CH |
| Q-28 | 2 | H | - | - | - | Cl | H | - | - | O | - | $OCH_3$ | CH | N |
| Q-28 | 2 | H | - | - | - | Cl | H | - | - | O | - | $OCH_3$ | $OCH_3$ | N |
| Q-28 | 2 | H | - | - | - | Cl | H | - | - | O | - | $OCH_3$ | Cl | CH |
| Q-28 | 2 | H | - | - | - | Cl | H | - | - | S | - | $OCH_3$ | $OCH_3$ | CH |
| Q-1 | 1 | $CH_3$ | H | H | - | - | - | - | - | O | - | $OCH_3$ | $OCH_3$ | CH |
| Q-1 | 2 | $CH_3$ | H | H | - | - | - | - | - | O | - | $OCH_3$ | $OCH_3$ | CH |
| Q-7 | 1 | $CH_3$ | - | - | H | - | H | H | H | O | - | $OCH_3$ | $OCH_3$ | CH |
| Q-7 | 2 | $CH_3$ | - | - | H | - | H | H | H | O | - | $OCH_3$ | $OCH_3$ | CH |
| Q-11 | 1 | $CH_3$ | - | - | H | - | H | - | - | O | O | $OCH_3$ | $OCH_3$ | CH |
| Q-11 | 2 | $CH_3$ | - | - | H | - | H | - | - | O | O | $OCH_3$ | $OCH_3$ | CH |
| Q-15 | 1 | $CH_3$ | - | - | - | $CH_3$ | H | - | - | O | O | $OCH_3$ | $OCH_3$ | CH |
| Q-15 | 2 | $CH_3$ | - | - | - | $CH_3$ | H | - | - | O | O | $OCH_3$ | $OCH_3$ | CH |
| Q-15 | 1 | $CH_3$ | - | - | - | $CH_3$ | H | - | - | O | S | $OCH_3$ | $OCH_3$ | CH |
| Q-15 | 2 | $CH_3$ | - | - | - | $CH_3$ | H | - | - | O | S | $OCH_3$ | $OCH_3$ | CH |
| Q-20 | 1 | $CH_3$ | - | - | - | $SCH_3$ | H | - | - | O | O | $OCH_3$ | $OCH_3$ | CH |
| Q-20 | 2 | $CH_3$ | - | - | - | $SCH_3$ | H | - | - | O | O | $OCH_3$ | $OCH_3$ | CH |
| Q-28 | 1 | $CH_3$ | - | - | - | Cl | H | - | - | O | - | $OCH_3$ | $OCH_3$ | CH |
| Q-28 | 2 | $CH_3$ | - | - | - | Cl | H | - | - | O | - | $OCH_3$ | $OCH_3$ | CH |

Table 16a

General Structure 9a

| Q | n | R | $E_1$ | $X_4$ | $Y_4$ | Z | m.p.°C |
|---|---|---|---|---|---|---|---|
| Q-46($R_{12}$,$R_{13}$=H) | 0 | $CH_3$ | H | $OCH_3$ | $OCH_3$ | CH | |
| Q-46($R_{12}$,$R_{13}$=H) | 0 | H | H | $CH_3$ | $CH_3$ | CH | |
| Q-46($R_{12}$,$R_{13}$=H) | 0 | H | H | $CH_3$ | $OCH_3$ | CH | |
| Q-46($R_{12}$,$R_{13}$=H) | 0 | H | H | $OCH_3$ | $OCH_3$ | CH | |
| Q-46($R_{12}$,$R_{13}$=H) | 0 | H | H | Cl | $OCH_3$ | CH | |
| Q-46($R_{12}$=H,$R_{13}$=$CH_3$) | 0 | H | H | $OCH_3$ | $OCH_3$ | CH | |
| Q-46($R_{12}$=$CH_3$,$R_{13}$=H) | 0 | H | H | $OCH_3$ | $OCH_3$ | CH | |
| Q-47($R_{12}$,$R_{13}$=H) | 0 | H | H | $CH_3$ | $CH_3$ | CH | |
| Q-47($R_{12}$,$R_{13}$=H) | 0 | H | H | $OCH_3$ | $OCH_3$ | CH | |
| Q-47($R_{12}$,$R_{13}$=H) | 0 | H | H | $CH_3$ | $OCH_3$ | CH | |
| Q-47($R_{12}$,$R_{13}$=H) | 0 | H | H | Cl | $OCH_3$ | CH | |
| Q-47($R_{12}$=$CH_3$,$R_{13}$=H) | 0 | H | H | $OCH_3$ | $OCH_3$ | CH | |
| Q-47($R_{12}$=H,$R_{13}$=$CH_3$) | 0 | H | H | $OCH_3$ | $OCH_3$ | CH | |
| Q-48($R_{12}$,$R_{13}$=H) | 0 | H | H | $CH_3$ | $CH_3$ | CH | |
| Q-48($R_{12}$,$R_{13}$=H) | 0 | H | H | $CH_3$ | $OCH_3$ | CH | |
| Q-48($R_{12}$,$R_{13}$=H) | 0 | H | H | $OCH_3$ | $OCH_3$ | CH | |
| Q-48($R_{12}$,$R_{13}$=H) | 0 | H | H | Cl | $OCH_3$ | CH | |
| Q-48($R_{12}$=$CH_3$;$R_{13}$=H) | 0 | H | H | $OCH_3$ | $OCH_3$ | CH | |
| Q-49($R_{12}$,$R_{13}$=H) | 0 | H | H | $CH_3$ | $CH_3$ | CH | |
| Q-49($R_{12}$,$R_{13}$=H) | 0 | H | H | $OCH_3$ | $CH_3$ | CH | |
| Q-49($R_{12}$,$R_{13}$=H) | 0 | H | H | $OCH_3$ | $OCH_3$ | CH | |
| Q-49($R_{12}$,$R_{13}$=H) | 0 | H | H | Cl | $OCH_3$ | CH | |
| Q-50($R_{12}$,$R_{13}$=H) | 0 | H | H | $OCH_3$ | $OCH_3$ | CH | |
| Q-50($R_{12}$,$R_{13}$=H) | 0 | H | H | $CH_3$ | $CH_3$ | CH | |
| Q-50($R_{12}$,$R_{13}$=H) | 0 | H | H | $CH_3$ | $OCH_3$ | CH | |
| Q-50($R_{12}$,$R_{13}$=H) | 0 | H | H | Cl | $OCH_3$ | CH | |

Table 16a Continued

| Q | n | R | $E_1$ | $X_4$ | $Y_4$ | Z | m.p.°C |
|---|---|---|---|---|---|---|---|
| Q-51($R_{12}$=H) | 0 | H | H | $OCH_3$ | $OCH_3$ | CH | |
| Q-51($R_{12}$=H) | 0 | H | H | $CH_3$ | $OCH_3$ | CH | |
| Q-51($R_{12}$=H) | 0 | H | H | $CH_3$ | $CH_3$ | CH | |
| Q-51($R_{12}$=H) | 0 | H | H | Cl | $OCH_3$ | CH | |
| Q-52($R_{12}$,$R_{13}$,$R_{14}$=H) | 0 | H | H | $CH_3$ | $CH_3$ | CH | |
| Q-52($R_{12}$,$R_{13}$,$R_{14}$=H) | 0 | H | H | $OCH_3$ | $OCH_3$ | CH | |
| Q-52($R_{12}$,$R_{13}$,$R_{14}$=H) | 0 | H | H | $CH_3$ | $OCH_3$ | CH | |
| Q-52($R_{12}$,$R_{13}$,$R_{14}$=H) | 0 | H | H | Cl | $OCH_3$ | CH | |
| Q-52($R_{12}$,$R_{13}$=$CH_3$;$R_{14}$=H) | 0 | H | H | $OCH_3$ | $OCH_3$ | CH | 204-207 |
| Q-53($R_{15}$=H) | 0 | H | H | $CH_3$ | $CH_3$ | CH | |
| Q-53($R_{15}$=H) | 0 | H | H | $OCH_3$ | $OCH_3$ | CH | |
| Q-53($R_{15}$=H) | 0 | H | H | $CH_3$ | $OCH_3$ | CH | |
| Q-53($R_{15}$=H) | 0 | H | H | Cl | $OCH_3$ | CH | |
| Q-53($R_{15}$=$CH_3$) | 0 | H | H | $OCH_3$ | $OCH_3$ | CH | |
| Q-53($R_{15}$=$CH_3$) | 0 | H | H | $CH_3$ | $OCH_3$ | CH | |
| Q-53($R_{15}$=$CH_3$) | 0 | H | H | $CH_3$ | $CH_3$ | CH | |
| Q-53($R_{15}$=$CH_3$) | 0 | H | H | Cl | $OCH_3$ | CH | |
| Q-53($R_{15}$=$CH_3$) | 0 | $CH_3$ | H | $OCH_3$ | $OCH_3$ | CH | |
| Q-53($R_{15}$=$C_2H_5$) | 0 | H | H | $CH_3$ | $CH_3$ | CH | |
| Q-53($R_{15}$=$C_2H_5$) | 0 | H | H | $OCH_3$ | $OCH_3$ | CH | |
| Q-53($R_{15}$=$C_2H_5$) | 0 | H | H | $CH_3$ | $OCH_3$ | CH | |
| Q-53($R_{15}$=$C_2H_5$) | 0 | H | H | Cl | $OCH_3$ | CH | |
| Q-53($R_{15}$=$SCH_3$) | 0 | H | H | $OCH_3$ | $OCH_3$ | CH | |
| Q-53($R_{15}$=$SCH_3$) | 0 | H | H | $CH_3$ | $OCH_3$ | CH | |
| Q-53($R_{15}$=$SCH_3$) | 0 | H | H | $CH_3$ | $CH_3$ | CH | |
| Q-53($R_{15}$=$SCH_3$) | 0 | H | H | Cl | $OCH_3$ | CH | |
| Q-53($R_{15}$=$SC_2H_5$) | 0 | H | H | $CH_3$ | $CH_3$ | CH | |
| Q-53($R_{15}$=$SC_2H_5$) | 0 | H | H | $OCH_3$ | $OCH_3$ | CH | |
| Q-53($R_{15}$=$SC_2H_5$) | 0 | H | H | $CH_3$ | $OCH_3$ | CH | |

Table 16a Continued

| Q | n | R | $E_1$ | $X_4$ | $Y_4$ | Z | m.p.°C |
|---|---|---|---|---|---|---|---|
| Q-53($R_{15}$=$SC_2H_5$) | 0 | H | H | Cl | $OCH_3$ | CH | |
| Q-53($R_{15}$=$SCH_2CH$=$CH_2$) | 0 | H | H | $OCH_3$ | $OCH_3$ | CH | |
| Q-53($R_{15}$=$SCH_2CH$=$CH_2$) | 0 | H | H | $CH_3$ | $OCH_3$ | CH | |
| Q-53($R_{15}$=$SCH_2CH$=$CH_2$) | 0 | H | H | $CH_3$ | $CH_3$ | CH | |
| Q-53($R_{15}$=$SCH_2CH$=$CH_2$) | 0 | H | H | Cl | $OCH_3$ | CH | |
| Q-53($R_{15}$=$SCF_2H$) | 0 | H | H | $OCH_3$ | $OCH_3$ | CH | |
| Q-53($R_{15}$=$SCF_2H$) | 0 | H | H | $CH_3$ | $CH_3$ | CH | |
| Q-53($R_{15}$=$SCF_2H$) | 0 | H | H | $OCH_3$ | $CH_3$ | CH | |
| Q-53($R_{15}$=$SCF_2H$) | 0 | H | H | Cl | $OCH_3$ | CH | |
| Q-53($R_{15}$=$SC_3H_7$) | 0 | H | H | $OCH_3$ | $OCH_3$ | CH | |
| Q-53($R_{15}$=$SC_3H_7$) | 0 | H | H | $CH_3$ | $OCH_3$ | CH | |
| Q-53($R_{15}$=$SC_3H_7$) | 0 | H | H | Cl | $OCH_3$ | CH | |
| Q-53($R_{15}$=$OCH_3$) | 0 | H | H | $OCH_3$ | $OCH_3$ | CH | |
| Q-53($R_{12}$=$OC_2H_5$) | 0 | H | H | $OCH_3$ | $OCH_3$ | CH | |
| Q-54($R_{12}$=H) | 0 | H | H | $OCH_3$ | $OCH_3$ | CH | |
| Q-54($R_{12}$=H) | 0 | H | H | $CH_3$ | $OCH_3$ | CH | |
| Q-54($R_{12}$=H) | 0 | H | H | $CH_3$ | $CH_3$ | CH | |
| Q-54($R_{12}$=H) | 0 | H | H | Cl | $OCH_3$ | CH | |
| Q-54($R_{12}$=$CH_3$) | 0 | H | H | $OCH_3$ | $OCH_3$ | CH | |
| Q-54($R_{12}$=$CH_3$) | 0 | H | H | $CH_3$ | $OCH_3$ | CH | |
| Q-54($R_{12}$=$CH_3$) | 0 | H | H | $CH_3$ | $CH_3$ | CH | |
| Q-54($R_{12}$=$CH_3$) | 0 | H | H | Cl | $OCH_3$ | CH | |
| Q-55($R_{12}$=H) | 0 | H | H | $CH_3$ | $CH_3$ | CH | |
| Q-55($R_{12}$=H) | 0 | H | H | $OCH_3$ | $OCH_3$ | CH | |
| Q-55($R_{12}$=H) | 0 | H | H | $CH_3$ | $OCH_3$ | CH | |
| Q-55($R_{12}$=H) | 0 | H | H | Cl | $OCH_3$ | CH | |
| Q-55($R_{12}$=$CH_3$) | 0 | H | H | $CH_3$ | $CH_3$ | CH | |
| Q-55($R_{12}$=$CH_3$) | 0 | H | H | $OCH_3$ | $OCH_3$ | CH | |
| Q-55($R_{12}$=$CH_3$) | 0 | H | H | $CH_3$ | $OCH_3$ | CH | |
| Q-55($R_{12}$=$CH_3$) | 0 | H | H | Cl | $OCH_3$ | CH | |
| Q-56($R_{12}$=H) | 0 | H | H | $OCH_3$ | $OCH_3$ | CH | |
| Q-57($R_{16}$=H) | 0 | H | H | $OCH_3$ | $OCH_3$ | CH | |

Table 16a Continued

| Q | n | R | $E_1$ | $X_4$ | $Y_4$ | Z | m.p.°C |
|---|---|---|---|---|---|---|---|
| Q-57($R_{16}$=Cl) | 0 | H | H | $OCH_3$ | $OCH_3$ | CH | |
| Q-58($R_{12}$=$CH_3$) | 0 | H | H | $OCH_3$ | $OCH_3$ | CH | |
| Q-59($R_{12}$=H) | 0 | H | H | $OCH_3$ | $OCH_3$ | CH | |
| Q-59($R_{12}$=H) | 0 | H | H | $CH_3$ | $OCH_3$ | CH | |
| Q-59($R_{12}$=H) | 0 | H | H | Cl | $OCH_3$ | CH | |
| Q-60($R_{12}$=H) | 0 | H | H | $OCH_3$ | $OCH_3$ | CH | |
| Q-61($R_{12}$,$R_{13}$=H) | 0 | H | H | $CH_3$ | $CH_3$ | CH | 240-243 |
| Q-61($R_{12}$,$R_{13}$=H) | 0 | H | H | $OCH_3$ | $OCH_3$ | CH | 235-238 |
| Q-61($R_{12}$,$R_{13}$=H) | 0 | H | H | $CH_3$ | $OCH_3$ | CH | 225-228 |
| Q-61($R_{12}$,$R_{13}$=H) | 0 | H | H | Cl | $OCH_3$ | CH | |
| Q-62($R_{12}$=$CH_3$,$R_{13}$=$CH_3$) | 0 | H | H | $OCH_3$ | $OCH_3$ | CH | |
| Q-62($R_{12}$=$CH_3$,$R_{13}$=$CH_3$) | 0 | H | H | $CH_3$ | $OCH_3$ | CH | |
| Q-62($R_{12}$=H,$R_{13}$=$CH_3$) | 0 | H | H | $CH_3$ | $CH_3$ | CH | |
| Q-62($R_{12}$=H,$R_{13}$=$CH_3$) | 0 | H | H | Cl | $OCH_3$ | CH | |
| Q-63($R_{12}$=H,$R_{13}$=$CH_3$) | 0 | H | H | $OCH_3$ | $OCH_3$ | CH | |
| Q-63($R_{12}$=$CH_3$,$R_{13}$=$CH_3$) | 0 | H | H | $CH_3$ | $OCH_3$ | CH | |
| Q-63($R_{12}$=H,$R_{13}$=$CH_3$) | 0 | H | H | $CH_3$ | $CH_3$ | CH | |
| Q-63($R_{12}$=H,$R_{13}$=$CH_3$) | 0 | H | H | Cl | $OCH_3$ | CH | |
| Q-64($R_{12}$=$CH_3$,$R_{13}$=H) | 0 | H | H | $OCH_3$ | $OCH_3$ | CH | |
| Q-65($R_{12}$,$R_{13}$=$CH_3$) | 0 | H | H | $OCH_3$ | $OCH_3$ | CH | |
| Q-66($R_{12}$,$R_{13}$,$R_{14}$=H) | 0 | H | H | $OCH_3$ | $OCH_3$ | CH | |
| Q-67($R_{12}$,$R_{13}$,$R_{14}$=H) | 0 | H | H | $OCH_3$ | $OCH_3$ | CH | |
| Q-68($R_{12}$,$R_{13}$,$R_{14}$=H) | 0 | H | H | $OCH_3$ | $OCH_3$ | CH | |
| Q-69($R_{12}$,$R_{13}$,$R_{14}$=H) | 0 | H | H | $OCH_3$ | $CH_3$ | CH | |
| Q-70($R_{12}$,$R_{13}$,$R_{14}$=H) | 0 | H | H | $OCH_3$ | $OCH_3$ | CH | |
| Q-71(W'=O) | 0 | H | H | $OCH_3$ | $OCH_3$ | CH | |
| Q-72($R_{12}$,$R_{13}$=H,n'=0) | 0 | H | H | $OCH_3$ | $OCH_3$ | CH | |
| Q-73($R_{12}$,$R_{13}$=H,n'=0) | 0 | H | H | $OCH_3$ | $OCH_3$ | CH | |
| Q-74($R_{12}$,$R_{13}$=H,n'=0) | 0 | H | H | $OCH_3$ | $OCH_3$ | CH | |
| Q-75($R_{12}$=H;$R_{17}$,$R_{18}$=$OCH_3$) | 0 | H | H | $OCH_3$ | $OCH_3$ | CH | |
| Q-75($R_{12}$=H;$R_{17}$,$R_{18}$=$CH_3$) | 0 | H | H | $OCH_3$ | $OCH_3$ | CH | |
| Q-76($R_{17}$,$R_{18}$=H) | 0 | H | H | $OCH_3$ | $OCH_3$ | CH | |

Table 16a Continued

| Q | n | R | $E_1$ | $X_4$ | $Y_4$ | Z | m.p.°C |
|---|---|---|---|---|---|---|---|
| Q-77($R_{17}$,$R_{18}$ =H) | 0 | H | H | $OCH_3$ | $OCH_3$ | CH | |
| Q-78($R_{12}$,$R_{13}$=H) | 0 | H | H | $OCH_3$ | $OCH_3$ | CH | |
| Q-79($R_{12}$,$R_{13}$=H) | 0 | H | H | $OCH_3$ | $OCH_3$ | CH | |
| Q-80($R_{19}$ ,$R_{20}$=$OCH_3$) | 0 | H | H | $OCH_3$ | $OCH_3$ | CH | |
| Q-81($R_{19}$ ,$R_{20}$ =$CH_3$) | 0 | H | H | $OCH_3$ | $OCH_3$ | CH | |
| $C_6H_5$ | 0 | H | H | $CH_3$ | $CH_3$ | CH | |
| $C_6H_5$ | 0 | H | H | $OCH_3$ | $CH_3$ | CH | |
| $C_6H_5$ | 0 | H | H | $OCH_3$ | $OCH_3$ | CH | |
| $C_6H_5$ | 0 | H | H | Cl | $OCH_3$ | CH | |
| Q-46($R_{12}$,$R_{13}$=H) | 0 | H | H | $CH_3$ | $OCH_3$ | N | |
| Q-47($R_{12}$,$R_{13}$=H) | 0 | H | H | $OCH_3$ | $OCH_3$ | N | |
| Q-48($R_{12}$=$CH_3$,$R_{13}$=H) | 0 | H | H | $OCH_3$ | $OCH_3$ | N | |
| Q-53($R_{15}$=$CH_3$) | 0 | H | H | $CH_3$ | $OCH_3$ | N | |
| Q-53($R_{15}$=$SCH_3$) | 0 | H | H | $OCH_3$ | $OCH_3$ | N | |
| Q-54($R_{12}$=$CH_3$) | 0 | H | H | $CH_3$ | $OCH_3$ | N | |
| Q-55($R_{12}$=$CH_3$) | 0 | H | H | $CH_3$ | $OCH_3$ | N | |
| Q-57($R_{16}$=Cl) | 0 | H | H | $CH_3$ | $OCH_3$ | N | |
| Q-62($R_{12}$=H,$R_{13}$=$CH_3$) | 0 | H | H | $OCH_3$ | $CH_3$ | N | |
| Q-63($R_{12}$=H,$R_{13}$=$CH_3$) | 0 | H | H | $CH_3$ | $OCH_3$ | N | |
| Q-63($R_{12}$=H,$R_{13}$=$CH_3$) | 0 | H | H | $OCH_3$ | $OCH_3$ | N | |
| Q-67($R_{12}$,$R_{13}$,$R_{14}$=H) | 0 | H | H | $CH_3$ | $OCH_3$ | N | |
| Q-71(W'=O) | 0 | H | H | $CH_3$ | $OCH_3$ | N | |
| Q-72($R_{12}$,$R_{13}$=H,n'=0) | 0 | H | H | $CH_3$ | $OCH_3$ | N | |
| Q-72($R_{12}$,$R_{13}$=H,n'=0) | 0 | H | H | $OCH_3$ | $OCH_3$ | N | |
| Q-75($R_{12}$,$R_{17}$,$R_{18}$=H) | 0 | H | H | $CH_3$ | $OCH_3$ | N | |
| Q-75($R_{12}$,$R_{17}$,$R_{18}$=H) | 0 | H | H | $OCH_3$ | $OCH_3$ | N | |
| Q-78($R_{12}$,$R_{13}$=H) | 0 | H | H | $CH_3$ | $OCH_3$ | N | |
| $C_6H_5$ | 0 | H | H | $CH_3$ | $OCH_3$ | N | |
| $C_6H_5$ | 0 | H | H | $OCH_3$ | $OCH_3$ | N | |
| $C_6H_5$ | 0 | H | H | $CH_3$ | $CH_3$ | N | |
| Q-47($R_{12}$,$R_{13}$=H) | 0 | H | 5-Cl | $OCH_3$ | $OCH_3$ | CH | |
| Q-61($R_{12}$=H,$R_{13}$=$CH_3$) | 0 | H | 6-Br | $OCH_3$ | $OCH_3$ | CH | |

Table 16a Continued

| Q | n | R | $E_1$ | $X_4$ | $Y_4$ | Z | m.p.°C |
|---|---|---|---|---|---|---|---|
| Q-53($R_{15}$=$SCH_3$) | 0 | H | 5-$CH_3$ | $OCH_3$ | $OCH_3$ | CH | |
| Q-53($R_{15}$=$SCH_3$) | 0 | H | 5-Cl | $OCH_3$ | $CH_3$ | N | |
| Q-46($R_{12}$,$R_{13}$=H) | 0 | H | 5-$CH_3$ | $OCH_3$ | $CH_3$ | N | |
| Q-46($R_{12}$,$R_{13}$=H) | 0 | $CH_3$ | H | $OCH_3$ | $CH_3$ | N | |
| Q-53($R_{15}$=$SCH_3$) | 0 | H | H | $OC_2H_5$ | $CH_3$ | CH | |
| Q-46($R_{12}$,$R_{13}$=H) | 0 | H | H | F | $OCH_3$ | CH | |
| Q-47($R_{12}$,$R_{13}$=H) | 0 | H | H | Br | $OCH_3$ | CH | |
| Q-48($R_{12}$,$R_{13}$=H) | 0 | H | H | I | $OCH_3$ | CH | |
| Q-53($R_{15}$=$SCH_3$) | 0 | H | H | $OCF_2H$ | $OCH_3$ | CH | |
| Q-55($R_{12}$=$CH_3$) | 0 | H | H | $CH_2F$ | $OCH_3$ | CH | |
| Q-63($R_{12}$=H,$R_{13}$=$CH_3$) | 0 | H | H | $OCH_2CH_2F$ | $CH_3$ | CH | |
| Q-62($R_{12}$=H,$R_{13}$=$CH_3$) | 0 | H | H | $OCH_2CHF_2$ | $CH_3$ | CH | |
| Q-53($R_{15}$=$SCH_3$) | 0 | H | H | $OCH_2CF_3$ | $OCH_3$ | CH | |
| Q-55($R_{12}$=$CH_3$) | 0 | H | H | $CF_3$ | $OCH_3$ | CH | |
| Q-47($R_{12}$,$R_{13}$=H) | 0 | H | H | $OCH_2CH_3$ | $OCH_3$ | N | |
| Q-46($R_{12}$,$R_{13}$=H) | 0 | H | H | $OCH_2CH_2F$ | $CH_3$ | N | |
| Q-53($R_{12}$=$SCH_3$) | 0 | H | H | $OCH_2CF_3$ | $OCH_3$ | N | |
| Q-54($R_{12}$=$CH_3$) | 0 | H | H | $OCH_3$ | H | CH | |
| Q-61($R_{12}$=H,$R_{13}$=$CH_3$) | 0 | H | H | $CH_3$ | $OC_2H_5$ | CH | |
| Q-53($R_{15}$=$SCH_3$) | 0 | H | H | $CH_3$ | $CH_2OCH_3$ | CH | |
| Q-53($R_{15}$=$CH_3$) | 0 | H | H | $OC_2H_5$ | $NHCH_3$ | N | |
| Q-62($R_{12}$=H,$R_{13}$=$CH_3$) | 0 | H | H | $OCH_3$ | $N(OCH_3)CH_3$ | N | |
| Q-53($R_{12}$=$SCH_3$) | 0 | H | H | $OCH_3$ | $N(CH_3)_2$ | N | |
| Q-63($R_{12}$=H,$R_{13}$=$CH_3$) | 0 | H | H | $OCH_3$ | $C_2H_5$ | CH | |
| Q-57($R_{16}$=Cl) | 0 | H | H | $OCH_3$ | $CF_3$ | CH | |
| Q-56($R_{12}$=$CH_3$) | 0 | H | H | $OCH_3$ | $SCH_3$ | N | |
| Q-63($R_{12}$=H,$R_{13}$=$CH_3$) | 0 | H | H | $CH_3$ | $OCH_2CH$=$CH_2$ | N | |
| Q-55($R_{12}$=H) | 0 | H | H | $CH_3$ | $OCH_2C{\equiv}CH$ | N | |
| Q-58($R_{12}$=$CH_3$) | 0 | H | H | $OCH_3$ | $CH_2OCH_2CH_3$ | CH | |
| Q-62($R_{12}$=H,$R_{13}$=$CH_3$) | 0 | H | H | $CH_3$ | $OCH_2CH_2OCH_3$ | CH | |
| Q-59($R_{17}$=H) | 0 | H | H | $CH_3$ | $CH_2SCH_3$ | CH | |
| Q-46($R_{12}$,$R_{13}$=H) | 0 | H | H | $OCH_3$ | i-$C_3H_7$ | CH | |

Table 16a Continued

| Q | n | R | $E_1$ | $X_4$ | $Y_4$ | Z | m.p.°C |
|---|---|---|---|---|---|---|---|
| Q-55($R_{12}$=$CH_3$) | 0 | H | H | $CH_3$ | $\overset{O}{\overset{\parallel}{CH}}$ | CH | |
| Q-46($R_{12}$,$R_{13}$=H) | 0 | H | H | $CH_3$ | $\overset{O}{\overset{\parallel}{C}}CH_3$ | CH | |
| Q-53($R_{15}$=$SCH_3$) | 0 | H | H | $OCH_3$ | $CH(OCH_3)_2$ | CH | |
| Q-46($R_{12}$,$R_{13}$=H) | 0 | H | H | $CH_3$ | $CH(OCH_3)_2$ | N | |
| Q-53($R_{12}$=$SCH_3$) | 0 | H | H | $OCH_3$ | $CH(OC_2H_5)_2$ | CH | |
| Q-47($R_{12}$,$R_{13}$=H) | 0 | H | H | $OCH_3$ | $CH(SCH_3)_2$ | CH | |
| Q-48($R_{12}$,$R_{13}$=H) | 0 | H | H | $OCH_3$ | $C(CH_3)(OCH_3)_2$ | CH | |
| Q-53($R_{12}$=$SCH_3$) | 0 | H | H | $OCH_3$ | 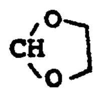 | CH | |
| Q-63($R_{12}$=H,$R_{13}$=$CH_3$) | 0 | H | H | $CH_3$ | 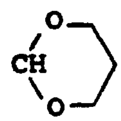 | CH | |
| Q-62($R_{12}$=H,$R_{13}$=$CH_3$) | 0 | H | H | $OCH_3$ | 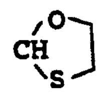 | CH | |
| Q-53($R_{15}$=$CH_3$) | 0 | H | H | $OCH_3$ | 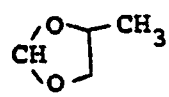 | CH | |
| Q-55($R_{15}$=H) | 0 | H | H | $OCH_3$ | $OCF_2H$ | CH | |
| Q-59 | 0 | H | H | $OCH_3$ | $SCF_2H$ | CH | |
| Q-53($R_{15}$=$SCH_3$) | 0 | H | H | $OCH_3$ | 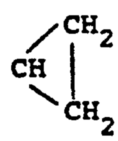 | N | |
| Q-46($R_{12}$,$R_{13}$=H) | 0 | H | H | $OCH_3$ | 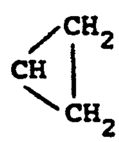 | CH | |

**Table 16a Continued**

| Q | n | R | $E_1$ | $X_4$ | $Y_4$ | Z | m.p. °C |
|---|---|---|---|---|---|---|---|
| Q-46($R_{12}$,$R_{13}$=H) | 1 | $CH_3$ | H | OCH | $OCH_3$ | CH 3 | |
| Q-46($R_{12}$,$R_{13}$=H) | 1 | H | H | $CH_3$ | $CH_3$ | CH | |
| Q-46($R_{12}$,$R_{13}$=H) | 1 | H | H | $CH_3$ | $OCH_3$ | CH | |
| Q-46($R_{12}$,$R_{13}$=H) | 1 | H | H | $OCH_3$ | $OCH_3$ | CH | |
| Q-46($R_{12}$,$R_{13}$=H) | 1 | H | H | Cl | $OCH_3$ | CH | |
| Q-46($R_{12}$=H,$R_{13}$=$CH_3$) | 1 | H | H | $OCH_3$ | $OCH_3$ | CH | |
| Q-46($R_{12}$=H,$CH_3$,$R_{13}$=H) | 1 | H | H | $OCH_3$ | $OCH_3$ | CH | |
| Q-47($R_{12}$,$R_{13}$=H) | 1 | H | H | CH | CH | CH | |
| Q-47($R_{12}$,$R_{13}$=H) | 1 | H | H | $OCH_3$ | $OCH_3$ | CH | |
| Q-47($R_{12}$,$R_{13}$=H) | 1 | H | H | $CH_3$ | $OCH_3$ | CH | |
| Q-47($R_{12}$,$R_{13}$=H) | 1 | H | H | Cl | $OCH_3$ | CH | |
| Q-47($R_{12}$=$CH_3$,$R_{13}$=H) | 1 | H | H | $OCH_3$ | $OCH_3$ | CH | |
| Q-47($R_{12}$=H,$R_{13}$=$CH_3$) | 1 | H | H | $OCH_3$ | $OCH_3$ | CH | |
| Q-48($R_{12}$,$R_{13}$=H) | 1 | H | H | $CH_3$ | $CH_3$ | CH | |
| Q-48($R_{12}$,$R_{13}$=H) | 1 | H | H | $CH_3$ | $OCH_3$ | CH | |
| Q-48($R_{12}$,$R_{13}$=H) | 1 | H | H | $OCH_3$ | $OCH_3$ | CH | |
| Q-48($R_{12}$,$R_{13}$=H) | 1 | H | H | Cl | $OCH_3$ | CH | |
| Q-48($R_{12}$=$CH_3$;$R_{13}$=H) | 1 | H | H | $OCH_3$ | $OCH_3$ | CH | |
| Q-49($R_{12}$,$R_{13}$=H) | 1 | H | H | $CH_3$ | $CH_3$ | CH | |
| Q-49($R_{12}$,$R_{13}$=H) | 1 | H | H | $OCH_3$ | $CH_3$ | CH | |
| Q-49($R_{12}$,$R_{13}$=H) | 1 | H | H | $OCH_3$ | $OCH_3$ | CH | |
| Q-49($R_{12}$,$R_{13}$=H) | 1 | H | H | Cl | $OCH_3$ | CH | |
| Q-50($R_{12}$,$R_{13}$=H) | 1 | H | H | $OCH_3$ | $OCH_3$ | CH | |
| Q-50($R_{12}$,$R_{13}$=H) | 1 | H | H | $CH_3$ | $CH_3$ | CH | |
| Q-50($R_{12}$,$R_{13}$=H) | 1 | H | H | $CH_3$ | $OCH_3$ | CH | |
| Q-50($R_{12}$,$R_{13}$=H) | 1 | H | H | Cl | $OCH_3$ | CH | |
| Q-51($R_{12}$=H) | 1 | H | H | $OCH_3$ | $OCH_3$ | CH | |
| Q-51($R_{12}$=H) | 1 | H | H | $CH_3$ | $OCH_3$ | CH | |
| Q-51($R_{12}$=H) | 1 | H | H | $CH_3$ | $CH_3$ | CH | |
| Q-51($R_{12}$=H) | 1 | H | H | Cl | $OCH_3$ | CH | |
| Q-53($R_{15}$=H) | 1 | H | H | $CH_3$ | $CH_3$ | CH | |
| Q-53($R_{15}$=H) | 1 | H | H | $OCH_3$ | $OCH_3$ | CH | |

Table 16a Continued

| Q | n | R | $E_1$ | $X_4$ | $Y_4$ | Z | m.p.°C |
|---|---|---|---|---|---|---|---|
| Q-53($R_{15}$=H) | 1 | H | H | $CH_3$ | $OCH_3$ | CH | |
| Q-53($R_{15}$=H) | 1 | H | H | Cl | $OCH_3$ | CH | |
| Q-54($R_{12}$=H) | 1 | H | H | $OCH_3$ | $OCH_3$ | CH | |
| Q-54($R_{12}$=H) | 1 | H | H | $CH_3$ | $CH_3$ | CH | |
| Q-55($R_{12}$=H) | 1 | H | H | $OCH_3$ | $OCH_3$ | CH | |
| Q-55($R_{12}$=H) | 1 | H | H | $CH_3$ | $OCH_3$ | CH | |
| Q-55($R_{12}$=H) | 1 | H | H | Cl | $OCH_3$ | CH | |
| Q-56($R_{12}$=H) | 1 | H | H | $OCH_3$ | $OCH_3$ | CH | |
| Q-56($R_{12}$=H) | 1 | H | H | $CH_3$ | $OCH_3$ | CH | |
| Q-57($R_{16}$=H) | 1 | H | H | $CH_3$ | $CH_3$ | CH | |
| Q-57($R_{16}$=H) | 1 | H | H | Cl | $OCH_3$ | CH | |
| Q-58($R_{12}$=$CH_3$) | 1 | H | H | $OCH_3$ | $OCH_3$ | CH | |
| Q-59($R_{12}$=H) | 1 | H | H | $CH_3$ | $CH_3$ | CH | |
| Q-59($R_{12}$=H) | 1 | H | H | $OCH_3$ | $OCH_3$ | CH | |
| Q-59($R_{12}$=H) | 1 | H | H | $CH_3$ | $OCH_3$ | CH | |
| Q-59($R_{12}$=H) | 1 | H | H | Cl | $OCH_3$ | CH | |
| Q-60($R_{12}$=H) | 1 | H | H | $OCH_3$ | $OCH_3$ | CH | |
| Q-61($R_{12}$=H,$R_{13}$=H) | 1 | H | H | $CH_3$ | $OCH_3$ | CH | |
| Q-61($R_{12}$=H,$R_{13}$=H) | 1 | H | H | $CH_3$ | $CH_3$ | CH | |
| Q-61($R_{12}$=H,$R_{13}$=H) | 1 | H | H | Cl | $OCH_3$ | CH | |
| Q-62($R_{12}$=H,$R_{13}$=H) | 1 | H | H | $CH_3$ | $CH_3$ | CH | |
| Q-62($R_{12}$=H,$R_{13}$=H) | 1 | H | H | $OCH_3$ | $OCH_3$ | CH | |
| Q-63($R_{12}$=H,$R_{13}$=H) | 1 | H | H | $CH_3$ | $OCH_3$ | CH | |
| Q-63($R_{12}$=H,$R_{13}$=H) | 1 | H | H | Cl | $OCH_3$ | CH | |
| Q-63($R_{12}$=H,$R_{13}$=H) | 1 | H | H | $OCH_3$ | $OCH_3$ | CH | |
| Q-64($R_{12}$=$CH_3$,$R_{13}$=H) | 1 | H | H | $OCH_3$ | $OCH_3$ | CH | |
| Q-65($R_{12}$,$R_{13}$=$CH_3$) | 1 | H | H | OCH | OCH | CH | |
| Q-67($R_{12}$,$R_{13}$,$R_{14}$=H) | 1 | H | H | $OCH_3$ | $OCH_3$ | CH | |
| Q-69($R_{12}$,$R_{13}$,$R_{14}$=H) | 1 | H | H | $OCH_3$ | $CH_3$ | CH | |
| Q-73($R_{12}$,$R_{13}$=H,n'=0) | 1 | H | H | $OCH_3$ | $OCH_3$ | CH | |
| Q-75($R_{12}$=H,$R_{17}$,$R_{18}$ =$CH_3$) | 1 | H | H | $OCH_3$ | $OCH_3$ | CH | |
| Q-78($R_{12}$,$R_{13}$=H) | 1 | H | H | $OCH_3$ | $OCH_3$ | CH | |

Table 16a Continued

| Q | n | R | $E_1$ | $X_4$ | $Y_4$ | Z | m.p.°C |
|---|---|---|---|---|---|---|---|
| Q-80($R_{19}$ ,$R_{20}$ =$OCH_3$) | 1 | H | H | $OCH_3$ | $OCH_3$ | CH | |
| $C_6H_5$ | 1 | H | H | $OCH_3$ | $OCH_3$ | CH | |
| $C_6H_5$ | 1 | H | H | $OCH_3$ | $CH_3$ | CH | |
| Q-82($R_{12}$=H | 1 | H | H | $OCH_3$ | $OCH_3$ | CH | |
| Q-83($R_{12}$=H) | 1 | H | H | $OCH_3$ | $OCH_3$ | CH | |
| Q-84($R_{12}$,$R_{13}$=H) | 1 | H | H | $OCH_3$ | $OCH_3$ | CH | |
| Q-46($R_{12}$,$R_{13}$=H) | 1 | H | H | $OCH_3$ | $OCH_3$ | N | |
| Q-47($R_{12}$,$R_{13}$=H) | 1 | H | H | $CH_3$ | $OCH_3$ | N | |
| Q-53($R_{15}$=$CH_3$) | 1 | H | H | $CH_3$ | $OCH_3$ | N | |
| Q-53($R_{15}$,=$CH_3$) | 1 | H | H | $OCH_3$ | $OCH_3$ | N | |
| Q-57($R_{16}$=Cl) | 1 | H | H | $OCH_3$ | $OCH_3$ | N | |
| Q-62($R_{12}$=H,$R_{13}$=$CH_3$) | 1 | H | H | $OCH_3$ | $OCH_3$ | N | |
| Q-63($R_{12}$=H,$R_{13}$=$CH_3$) | 1 | H | H | $OCH_3$ | $OCH_3$ | N | |
| Q-67($R_{12}$,$R_{13}$,$R_{14}$=H) | 1 | H | H | $OCH_3$ | $CH_3$ | N | |
| Q-71(W'=S) | 1 | H | H | $OCH_3$ | $OCH_3$ | N | |
| Q-72($R_{12}$,$R_{13}$=H,n'=1) | 1 | H | H | $OCH_3$ | $OCH_3$ | N | |
| Q-75($R_{12}$,$R_{17}$,$R_{18}$=H) | 1 | H | H | $OCH_3$ | $OCH_3$ | N | |
| Q-78($R_{12}$,$R_{13}$=H) | 1 | H | H | $OCH_3$ | $OCH_3$ | N | |
| Q-47($R_{12}$,$R_{13}$=H) | 1 | H | 5-Cl | $OCH_3$ | $OCH_3$ | N | |
| Q-61($R_{12}$=H,$R_{13}$=$CH_3$) | 1 | H | 5-Br | $OCH_3$ | $OCH_3$ | CH | |
| Q-53($R_{15}$=$SCH_3$) | 1 | H | 5-$CH_3$ | $OCH_3$ | $OCH_3$ | CH | |
| Q-53($R_{15}$=$SCH_3$) | 1 | H | 5-Cl | $OCH_3$ | $CH_3$ | N | |
| Q-46($R_{12}$,$R_{13}$=H) | 1 | $CH_3$ | 5-CH | OCH 3 | CH 3 | N 3 | |
| Q-46($R_{12}$,$R_{13}$=H) | 1 | $CH_3$ | H | OCH | CH 3 | N 3 | |
| Q-53($R_{15}$=$SCH_3$) | 1 | H | H | $OC_2H_5$ | $CH_3$ | CH | |
| Q-46($R_{12}$,$R_{13}$=H) | 1 | H | H | F | $OCH_3$ | CH | |
| Q-47($R_{12}$,$R_{13}$=H) | 1 | H | H | Br | $OCH_3$ | CH | |
| Q-48($R_{12}$,$R_{13}$=H) | 1 | H | H | I | $OCH_3$ | CH | |
| Q-53($R_{15}$,R =$SCH_3$) | 1 | H | H | $OCF_2H$ | $OCH_3$ | CH | |
| Q-55($R_{12}$=$CH_3$) | 1 | H | H | $CH_2F$ | $OCH_3$ | CH | |

**Table 16a Continued**

| Q | n | R | $E_1$ | $X_4$ | $Y_4$ | Z | m.p.°C |
|---|---|---|---|---|---|---|---|
| Q-63($R_{12}$=H,$R_{13}$=$CH_3$) | 1 | H | H | $OCH_2CH_2F$ | $CH_3$ | CH | |
| Q-62($R_{12}$=H,$R_{13}$=$CH_3$) | 1 | H | H | $OCH_2CHF_2$ | $CH_3$ | CH | |
| Q-53($R_{15}$=$SCH_3$) | 1 | H | H | $OCH_2CF_3$ | $OCH_3$ | CH | |
| Q-55($R_{12}$=$CH_3$) | 1 | H | H | $CF_3$ | $OCH_3$ | CH | |
| Q-47($R_{12}$,$R_{13}$=H) | 1 | H | H | $OCH_2CH_3$ | $OCH_3$ | N | |
| Q-46($R_{12}$,$R_{13}$=H) | 1 | H | H | $OCH_2CH_2F$ | $CH_3$ | N | |
| Q-53($R_{15}$=$SCH_3$) | 1 | H | H | $OCH_2CF_3$ | $OCH_3$ | N | |
| Q-54($R_{12}$=$CH_3$) | 1 | H | H | $OCH_3$ | H | CH | |
| Q-61($R_{12}$,$R_{13}$=H) | 1 | H | H | $CH_3$ | $OC_2H_5$ | CH | |
| Q-53($R_{15}$=$SCH_3$) | 1 | H | H | $CH_3$ | $CH_2OCH_3$ | CH | |
| Q-53($R_{15}$=$CH_3$) | 1 | H | H | $OC_2H_5$ | $NHCH_3$ | N | |
| Q-62 | 1 | H | H | $OCH_3$ | $N(OCH_3)CH_3$ | N | |
| Q-53($R_{15}$=$SCH_3$) | 1 | H | H | $OCH_3$ | $N(CH_3)_2$ | N | |
| Q-63($R_{12}$=H,$R_{13}$=$CH_3$) | 1 | H | H | $OCH_3$ | $C_2H_5$ | CH | |
| Q-57($R_{16}$=Cl) | 1 | H | H | $OCH_3$ | $CF_3$ | CH | |
| Q-56($R_{12}$=$CH_3$) | 1 | H | H | $OCH_3$ | $SCH_3$ | N | |
| Q-63($R_{12}$=H,$R_{13}$=$CH_3$) | 1 | H | H | $CH_3$ | $OCH_2CH=CH_2$ | N | |
| Q-55($R_{12}$=$CH_3$) | 1 | H | H | $CH_3$ | $OCH_2C\equiv CH$ | N | |
| Q-58($R_{12}$=$CH_3$) | 1 | H | H | $OCH_3$ | $CH_2OCH_2CH_3$ | CH | |
| Q-62($R_{12}$=H,$R_{13}$=$CH_3$) | 1 | H | H | $CH_3$ | $OCH_2CH_2OCH_3$ | CH | |
| Q-59($R_{12}$=H) | 1 | H | H | $CH_3$ | $CH_2SCH_3$ | CH | |
| Q-46($R_{12}$,$R_{13}$=H) | 1 | H | H | $OCH_3$ | $i-C_3H_7$ | CH | |
| Q-55($R_{12}$=$CH_3$) | 1 | H | H | $CH_3$ | $\overset{O}{\parallel}{CH}$ | CH | |
| Q-46($R_{12}$,$R_{13}$=H) | 1 | H | H | $CH_3$ | $\overset{O}{\parallel}{CCH_3}$ | CH | |
| Q-53($R_{15}$=$SCH_3$) | 1 | H | H | $OCH_3$ | $CH(OCH_3)_2$ | CH | |
| Q-46($R_{12}$,$R_{13}$=H) | 1 | H | H | $CH_3$ | $CH(OCH_3)_2$ | N | |
| Q-53($R_{15}$=$SCH_3$) | 1 | H | H | $OCH_3$ | $CH(OC_2H_5)_2$ | CH | |
| Q-47($R_{12}$,$R_{13}$=H) | 1 | H | H | $OCH_3$ | $CH(SCH_3)_2$ | CH | |
| Q-48($R_{12}$,$R_{13}$=H) | 1 | H | H | $OCH_3$ | $C(CH_3)(OCH_3)_2$ | CH | |

## Table 16a Continued

| Q | n | R | $E_1$ | $X_4$ | $Y_4$ | Z | m.p.°C |
|---|---|---|---|---|---|---|---|
| Q-53($R_{15}$=$SCH_3$) | 1 | H | H | $OCH_3$ | 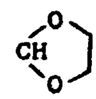 | CH | |
| Q-63($R_{12}$=H,$R_{13}$=$CH_3$) | 1 | H | H | $CH_3$ | 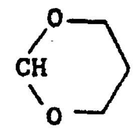 | CH | |
| Q-62($R_{12}$=H,$R_{13}$=$CH_3$) | 1 | H | H | $OCH_3$ | 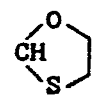 | | |
| Q-53($R_{15}$=$CH_3$) | 1 | H | H | $OCH_3$ | 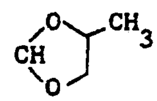 | CH | |
| Q-55($R_{12}$=H) | 1 | H | H | $OCH_3$ | $OCF_2H$ | CH | |
| Q-59($R_{12}$=H) | 1 | H | H | $OCH_3$ | $SCF_2H$ | CH | |
| Q-53($R_{15}$=$SCH_3$) | 1 | H | H | $OCH_3$ | 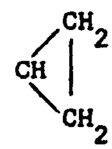 | N | |
| Q-46($R_{12}$,$R_{13}$=H) | 1 | H | H | $OCH_3$ | 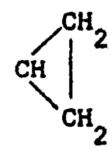 | CH | |
| Q-80($R_{11}$ =$R_{12}$ =$OCH_3$) | 1 | H | H | $OCH_3$ | 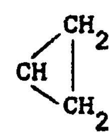 | CH | |
| Q-84($R_{12}$=$R_{13}$=H) | 1 | H | H | $OCH_3$ | 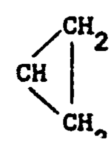 | CH | |

## Table 16a Continued

| Q | n | R | $E_1$ | $X_4$ | $Y_4$ | Z | m.p.°C |
|---|---|---|---|---|---|---|---|
| Q-46($R_{12}$,$R_{13}$=H) | 2 | H | H | $OCH_3$ | $OCH_3$ | CH | |
| Q-50($R_{12}$,$R_{13}$=H) | 2 | H | H | $OCH_3$ | $OCH_3$ | CH | |
| Q-53($R_{15}$=H) | 2 | H | H | $OCH_3$ | $OCH_3$ | CH | |
| Q-59($R_{12}$=H) | 2 | H | H | $OCH_3$ | $OCH_3$ | CH | |
| Q-61($R_{12}$=H,$R_{13}$=$CH_3$) | 2 | H | H | $OCH_3$ | $OCH_3$ | CH | |
| Q-46($R_{12}$,$R_{13}$=H) | 2 | H | H | $OCH_3$ | $OCH_3$ | N | |
| Q-55($R_{12}$=H) | 2 | H | H | $OCH_3$ | $OCH_3$ | N | |
| Q-57($R_{16}$=H) | 2 | H | H | $CH_3$ | $OCH_3$ | N | |
| Q-63($R_{12}$=H,$R_{13}$=$CH_3$) | 2 | H | H | $OCH_3$ | $OCH_3$ | N | |
| Q-69($R_{12}$,$R_{13}$,$R_{14}$=H) | 2 | H | H | $OCH_3$ | $OCH_3$ | N | |
| Q-72($R_{12}$,$R_{13}$=H,n'=1) | 2 | H | H | $OCH_3$ | $CH_3$ | N | |
| Q-78($R_{12}$,$R_{13}$,=H) | 2 | H | H | $OCH_3$ | $OCH_3$ | N | |
| Q-82($R_{12}$,$R_{13}$=H) | 2 | H | H | $OCH_3$ | $OCH_3$ | CH | |
| Q-52($R_{12}$,$R_{14}$=$CH_3$,$R_{13}$=H) | 0 | H | H | $CH_3$ | $OCH_3$ | CH | 220-224 |
| Q-52($R_{12}$,$R_{14}$=$CH_3$,$R_{13}$=H) | 0 | H | H | $CH_3$ | $CH_3$ | CH | 212-216 |
| Q-52($R_{12}$,$R_{14}$=$CH_3$,$R_{13}$=H) | 0 | H | H | Cl | $OCH_3$ | CH | 193-195 |
| Q-52($R_{12}$,$R_{14}$=$CH_3$,$R_{13}$=H) | 0 | H | H | $OCH_3$ | $OCH_3$ | N | 190-193 |

**Table 16b**

**General Structure 9b**

| Q | n | R | $E_1$ | $X_1$ | $Y_1$ | m.p.°C |
|---|---|---|---|---|---|---|
| Q-46($R_{12}$,$R_{13}$=H) | O | H | H | $CH_3$ | O | |
| Q-47($R_{12}$,$R_{13}$=H) | O | H | H | $CH_3$ | O | |
| Q-48($R_{12}$,$R_{13}$=H) | O | H | H | $CH_3$ | O | |
| Q-49($R_{12}$,$R_{13}$=H) | O | H | H | $CH_3$ | O | |
| Q-52($R_{12}$,$R_{13}$,$R_{14}$=H) | O | H | H | $CH_3$ | O | |
| Q-53($R_{15}$=$SCH_3$) | O | H | H | $CH_3$ | O | |
| Q-53($R_{15}$=$CH_3$) | O | H | H | $CH_3$ | O | |
| Q-53($R_{15}$=H) | O | H | H | $CH_3$ | O | |
| Q-54($R_{12}$=$CH_3$) | O | H | H | $CH_3$ | O | |
| Q-55($R_{12}$=$CH_3$) | O | H | H | $CH_3$ | O | |
| Q-59($R_{12}$=H) | O | H | H | $CH_3$ | O | |
| Q-61($R_{12}$,$R_{13}$=H) | O | H | H | $CH_3$ | O | |
| Q-62($R_{12}$,$R_{13}$=H) | O | H | H | $CH_3$ | O | |
| Q-63($R_{12}$,$R_{13}$=H) | O | H | H | $CH_3$ | O | |
| Q-66($R_{12}$,$R_{13}$,$R_{14}$=H) | O | H | H | $CH_3$ | O | |
| Q-67($R_{12}$,$R_{13}$,$R_{14}$=H) | O | H | H | $CH_3$ | O | |
| Q-69($R_{12}$,$R_{13}$,$R_{14}$=H) | O | H | H | $CH_3$ | O | |
| Q-72($R_{12}$,$R_{13}$=H,n'=0) | O | H | H | $CH_3$ | O | |
| Q-75($R_{12}$,$R_{17}$,$R_{18}$ =H) | O | H | H | $CH_3$ | O | |
| Q-78($R_{12}$,$R_{13}$=H) | O | H | H | $CH_3$ | O | |
| Q-53($R_{15}$=$SCH_3$) | O | H | H | $OCH_3$ | O | |
| Q-46($R_{12}$,$R_{13}$=H) | O | H | H | $OC_2H_5$ | O | |
| Q-54($R_{12}$=$CH_3$) | O | H | H | $OCF_2H$ | O | |
| Q-53($R_{15}$=$CH_3$) | O | H | H | $CH_3$ | $CH_2$ | |
| Q-53($R_{15}$=$SCH_3$) | O | H | H | $OCH_3$ | $CH_2$ | |

Table 16b Continued

| Q | n | R | $E_1$ | $X_1$ | $Y_1$ | m.p.°C |
|---|---|---|---|---|---|---|
| Q-46($R_{12}$,$R_{13}$=H) | 1 | H | H | $CH_3$ | O | |
| Q-47($R_{12}$,$R_{13}$=H) | 1 | H | H | $CH_3$ | O | |
| Q-53($R_{15}$=$SCH_3$) | 1 | H | H | $CH_3$ | O | |
| Q-53($R_{15}$=$CH_3$) | 1 | H | H | $CH_3$ | O | |
| Q-59($R_{12}$=H) | 1 | H | H | $CH_3$ | O | |
| Q-61($R_{12}$,$R_{13}$=H) | 1 | H | H | $CH_3$ | O | |
| Q-62($R_{12}$,$R_{13}$=H) | 1 | H | H | $CH_3$ | O | |
| Q-63($R_{12}$,$R_{13}$=H) | 1 | H | H | $CH_3$ | O | |
| Q-78($R_{12}$,$R_{13}$=H) | 1 | H | H | $CH_3$ | O | |
| Q-46($R_{12}$,$R_{13}$=H) | 1 | H | H | $OC_2H_5$ | O | |
| Q-53($R_{15}$=$CH_3$) | 1 | H | H | $OCF_2H$ | O | |
| Q-53($R_{15}$=$SCH_3$) | 1 | H | H | $OCH_3$ | $CH_2$ | |
| Q-53($R_{15}$=$CH_3$) | 2 | H | H | $CH_3$ | O | |
| Q-59($R_{12}$=H) | 2 | H | H | $CH_3$ | O | |
| Q-53($R_{15}$=$SCH_3$) | 2 | H | H | $OC_2H_5$ | O | |
| Q-78,($R_{12}$,$R_{13}$=H) | 2 | H | H | $CH_3$ | O | |

Table 16c

General Structure 9c

| Q | n | R | $E_1$ | $X_1$ | m.p.°C |
|---|---|---|---|---|---|
| Q-46($R_{12}$,$R_{13}$=H) | 0 | H | H | $CH_3$ | |
| Q-47($R_{12}$,$R_{13}$=H) | 0 | H | H | $OCH_3$ | |
| Q-48($R_{12}$,$R_{13}$=H) | 0 | H | H | $OC_2H_5$ | |
| Q-49($R_{12}$,$R_{13}$=H) | 0 | H | H | $CH_3$ | |
| Q-52($R_{12}$,$R_{13}$,$R_{14}$=H) | 0 | H | H | $CH_3$ | |
| Q-53($R_{15}$=$SCH_3$) | 0 | H | H | $OCH_3$ | |
| Q-53($R_{15}$=$CH_3$) | 0 | H | H | $OCH_3$ | |
| Q-53($R_{15}$=H) | 0 | H | H | $CH_3$ | |
| Q-54($R_{12}$=$CH_3$) | 0 | H | H | $OCF_2H$ | |
| Q-55($R_{12}$=$CH_3$) | 0 | H | H | $CH_3$ | |
| Q-59($R_{12}$=H) | 0 | H | H | $OCH_3$ | |
| Q-61($R_{12}$=H,$R_{13}$=$CH_3$) | 0 | H | H | $CH_3$ | |
| Q-62($R_{12}$=H,$R_{13}$=$CH_3$) | 0 | H | H | $OCF_2H$ | |
| Q-63($R_{12}$=H,$R_{13}$=$CH_3$) | 0 | H | H | $OCH_3$ | |
| Q-66($R_{12}$,$R_{13}$,$R_{14}$=H) | 0 | H | H | $OCH_3$ | |
| Q-67($R_{12}$,$R_{13}$,$R_{14}$=H) | 0 | H | H | $CH_3$ | |
| Q-69($R_{12}$,$R_{13}$,$R_{14}$=H) | 0 | H | H | $CH_3$ | |
| Q-72($R_{12}$,$R_{13}$=H,n'=0) | 0 | H | H | $OCH_3$ | |
| Q-75($R_{12}$,$R_{13}$,$R_{18}$=H) | 0 | H | H | $OCH_3$ | |
| Q-78($R_{12}$,$R_{13}$=H) | 0 | H | H | $OCH_3$ | |
| Q-46($R_{12}$,$R_{13}$=H) | 1 | H | H | $CH_3$ | |
| Q-47($R_{12}$,$R_{13}$=H) | 1 | H | H | $OCH_3$ | |
| Q-53($R_{15}$=$CH_3$) | 1 | H | H | $OCH_3$ | |
| Q-53($R_{15}$=$SCH_3$) | 1 | H | H | $CH_3$ | |
| Q-55($R_{12}$=$CH_3$) | 1 | H. | H | $OC_2H_5$ | |

Table 16c Continued

| Q | n | R | $E_1$ | $X_1$ | m.p.°C |
|---|---|---|---|---|---|
| Q-59($R_{12}$=H) | 1 | H | H | $OCH_3$ | |
| Q-62($R_{12}$=H,$R_{13}$=$CH_3$) | 1 | H | H | $OCF_2H$ | |
| Q-66($R_{12}$,$R_{13}$,$R_{14}$=H) | 1 | H | H | $OCH_3$ | |
| Q-78($R_{12}$,$R_{13}$=H) | 1 | H | H | $OCH_3$ | |
| Q-53($R_{15}$=$CH_3$) | 2 | H | H | $CH_3$ | |
| Q-72(R,$R_{13}$=H,n'=1) | 2 | H | H | $OCH_3$ | |
| Q-69($R_{12}$,$R_{13}$=H) | 2 | H | H | $OCH_3$ | |
| Q-46($R_{12}$,$R_{13}$=H) | 2 | H | H | $CH_3$ | |
| Q-49($R_{12}$,$R_{13}$=H) | 2 | H | H | $OC_2H_5$ | |

Table 16d

General Structure 9d

| Q | n | R | $E_1$ | $X_1$ | $Y_2$ | m.p.°C |
|---|---|---|---|---|---|---|
| Q-46($R_{12}$,$R_{13}$=H) | 0 | H | H | $CH_3$ | $CH_3$ | |
| Q-47($R_{12}$,$R_{13}$=H) | 0 | H | H | $OCH_3$ | $CH_3$ | |
| Q-48($R_{12}$,$R_{13}$=H) | 0 | H | H | $OC_2H_5$ | H | |
| Q-49($R_{12}$,$R_{13}$=H) | 0 | H | H | $CH_3$ | $CH_3$ | |
| Q-52($R_{12}$,$R_{13}$,$R_{14}$=H) | 0 | H | H | $CH_3$ | $CH_3$ | |
| Q-53($R_{15}$=$SCH_3$) | 0 | H | H | $CH_3$ | $CH_3$ | |
| Q-53($R_{15}$=$CH_3$) | 0 | H | H | $CH_3$ | $CH_3$ | |
| Q-53($R_{15}$=H) | 0 | H | H | $CH_3$ | $CH_3$ | |
| Q-54($R_{12}$=$CH_3$) | 0 | H | H | $OCF_2H$ | $CH_3$ | |
| Q-55($R_{12}$=$CH_3$) | 0 | H | H | $CH_3$ | $CH_3$ | |
| Q-59($R_{12}$=H) | 0 | H | H | $CH_3$ | $CH_3$ | |
| Q-61($R_{12}$,$R_{13}$=H) | 0 | H | H | $CH_3$ | $CH_3$ | |
| Q-62($R_{12}$,$R_{13}$=H) | 0 | H | H | $OCF_2H$ | $CH_3$ | |
| Q-63($R_{12}$,$R_{13}$=H) | 0 | H | H | $OC_2H_5$ | $CH_3$ | |
| Q-66($R_{12}$,$R_{13}$,$R_{14}$=H) | 0 | H | H | $CH_3$ | $CH_3$ | |
| Q-67($R_{12}$,$R_{13}$,$R_{14}$=H) | 0 | H | H | $OCH_3$ | H | |
| Q-69($R_{12}$,$R_{13}$,$R_{14}$=H) | 0 | H | H | $OCF_2H$ | $CH_3$ | |
| Q-72($R_{12}$,$R_{13}$=H,n'=0) | 0 | H | H | $CH_3$ | $CH_3$ | |
| Q-75($R_{12}$,$R_{17}$,$R_{18}$ =H) | 0 | H | H | $CH_3$ | $CH_3$ | |
| Q-78($R_{12}$,$R_{13}$=H) | 0 | H | H | $CH_3$ | $CH_3$ | |
| Q-46($R_{12}$,$R_{13}$=H) | 1 | H | H | $CH_3$ | $CH_3$ | |
| Q-47($R_{12}$,$R_{13}$=H) | 1 | H | H | $OCH_3$ | $CH_3$ | |
| Q-48($R_{12}$,$R_{13}$=H) | 1 | H | H | $OC_2H_5$ | H | |
| Q-53($R_{15}$=$SCH_3$) | 1 | H | H | $CH_3$ | $CH_3$ | |
| Q-53($R_{15}$=$CH_3$) | 1 | H | H | $CH_3$ | $CH_3$ | |

Table 16d Continued

| Q | n | R | $E_1$ | $X_1$ | $Y_2$ | m.p.°C |
|---|---|---|---|---|---|---|
| Q-59($R_{12}$=H) | 1 | H | H | $CH_3$ | $CH_3$ | |
| Q-61($R_{12}$,$R_{13}$=H) | 1 | H | H | $CH_3$ | $CH_3$ | |
| Q-63($R_{12}$,$R_{13}$=H) | 1 | H | H | $OC_2H_5$ | $CH_3$ | |
| Q-66($R_{12}$,$R_{13}$,$R_{14}$=H) | 1 | H | H | $CH_3$ | $CH_3$ | |
| Q-72($R_{12}$,$R_{13}$=H) | 1 | H | H | $CH_3$ | $CH_3$ | |
| Q-78($R_{12}$,$R_{13}$=H) | 1 | H | H | $CH_3$ | $CH_3$ | |
| Q-83($R_{12}$,$R_{13}$=H) | 1 | H | H | $CH_3$ | $CH_3$ | |
| Q-46($R_{12}$,$R_{13}$=H) | 2 | H | H | $CH_3$ | $CH_3$ | |
| Q-53($R_{15}$=$SCH_3$) | 2 | H | H | $CH_3$ | $CH_3$ | |
| Q-53($R_{15}$=$CH_3$) | 2 | H | H | $CH_3$ | $CH_3$ | |
| Q-59 | 2 | H | H | $OCH_3$ | $CH_3$ | |
| Q-78($R_{12}$,$R_{13}$=H) | 2 | H | H | $CH_3$ | $CH_3$ | |

**Table 16e**

**General Structure 9e**

| Q | n | R | $E_1$ | $X_2$ | $Y_3$ | m.p.°C |
|---|---|---|---|---|---|---|
| Q-46($R_{12}$,$R_{13}$=H) | 0 | H | H | $CH_3$ | $CH_3$ | |
| Q-47($R_{12}$,$R_{13}$=H) | 0 | H | H | $OCH_3$ | $CH_3$ | |
| Q-48($R_{12}$,$R_{13}$=H) | 0 | H | H | $OCH_3$ | $C_2H_5$ | |
| Q-49($R_{12}$,$R_{13}$=H) | 0 | H | H | $CH_3$ | $CH_3$ | |
| Q-52($R_{12}$,$R_{13}$,$R_{14}$=H) | 0 | H | H | $CH_3$ | $CH_3$ | |
| Q-53($R_{15}$=$SCH_3$) | 0 | H | H | $CH_3$ | $CH_2CF_3$ | |
| Q-53($R_{15}$=$CH_3$) | 0 | H | H | $CH_3$ | $CH_2CF_3$ | |
| Q-53($R_{15}$=H) | 0 | H | H | $CH_3$ | $CH_3$ | |
| Q-54($R_{12}$=$CH_3$) | 0 | H | H | $OCH_3$ | $CH_3$ | |
| Q-55($R_{12}$=$CH_3$) | 0 | H | H | $CH_3$ | $CH_3$ | |
| Q-59($R_{12}$=H) | 0 | H | H | $CH_3$ | $C_2H_5$ | |
| Q-61($R_{12}$=H,$R_{13}$=$CH_3$) | 0 | H | H | $CH_3$ | $CH_3$ | |
| Q-62($R_{12}$=H,$R_{13}$=$CH_3$) | 0 | H | H | $OCH_3$ | $CH_2CF_3$ | |
| Q-63($R_{12}$=H,$R_{13}$=$CH_3$) | 0 | H | H | $OCH_3$ | $CH_3$ | |
| Q-66($R_{12}$,$R_{13}$,$R_{14}$=H) | 0 | H | H | $CH_3$ | $CH_3$ | |
| Q-67($R_{12}$,$R_{13}$,$R_{14}$=H) | 0 | H | H | $OCH_3$ | $C_2H_5$ | |
| Q-69($R_{12}$,$R_{13}$,$R_{14}$=H) | 0 | H | H | $OCH_3$ | $CH_3$ | |
| Q-72($R_{12}$,$R_{13}$=H, n'=1) | 0 | H | H | $CH_3$ | $CH_3$ | |
| Q-75($R_{12}$,$R_{17}$,$R_{18}$ =H) | 0 | H | H | $CH_3$ | $CH_3$ | |
| Q-78($R_{12}$,$R_{13}$=H) | 0 | H | H | $CH_3$ | $CH_3$ | |
| Q-46($R_{12}$,$R_{13}$=H) | 1 | H | H | $CH_3$ | $CH_3$ | |
| Q-47($R_{12}$,$R_{13}$=H) | 1 | H | H | $OCH_3$ | $CH_3$ | |
| Q-53($R_{15}$=$CH_3$) | 1 | H | H | $CH_3$ | $C_2H_5$ | |
| Q-53($R_{15}$=$SCH_3$) | 1 | H | H | $CH_3$ | $CH_2CF_3$ | |
| Q-54($R_{12}$=$CH_3$) | 1 | H | H | $OCH_3$ | $CH_3$ | |
| Q-55($R_{12}$=$CH_3$) | 1 | H | H | $CH_3$ | $CH_3$ | |

Table Ie Continued

| Q | n | R | $R_1$ | $X_2$ | $Y_3$ | m.p.°C |
|---|---|---|---|---|---|---|
| Q-59($R_{12}$=H) | 1 | H | H | $CH_3$ | $C_2H_5$ | |
| Q-61($R_{12}$=H, $R_{13}$=$CH_3$) | 1 | H | H | $CH_3$ | $CH_3$ | |
| Q-72($R_{12}$, $R_{13}$=H) | 1 | H | H | $CH_3$ | $CH_3$ | |
| Q-78($R_{12}$, $R_{13}$=H) | 1 | H | H | $CH_3$ | $CH_3$ | |
| Q-46($R_{12}$, $R_{13}$=H) | 2 | H | H | $CH_3$ | $CH_3$ | |
| Q-53($R_{15}$=$CH_3$) | 2 | H | H | $OCH_3$ | $CH_3$ | |
| Q-59 | 2 | H | H | $CH_3$ | $CH_3$ | |
| Q-63 | 2 | H | H | $OCH_3$ | $CH_2CF_3$ | |

## Table 16f

### General Structure 9f

| Q | n | R | $E_1$ | $X_3$ | m.p.°C |
|---|---|---|---|---|---|
| Q-46($R_{12}$,$R_{13}$=H) | 0 | H | H | $CH_3$ | |
| Q-47($R_{12}$,$R_{13}$=H) | 0 | H | H | $CH_3$ | |
| Q-48($R_{12}$,$R_{13}$=H) | 0 | H | H | $CH_3$ | |
| Q-49($R_{12}$,$R_{13}$=H) | 0 | H | H | $OCH_3$ | |
| Q-52($R_{12}$,$R_{13}$,$R_{14}$=H) | 0 | H | H | $OCH_3$ | |
| Q-54($R_{15}$=$CH_3$) | 0 | H | H | $CH_3$ | |
| Q-53($R_{15}$=$SCH_3$) | 0 | H | H | $OCH_3$ | |
| Q-53($R_{15}$=$CH_3$) | 0 | H | H | $CH_3$ | |
| Q-54($R_{12}$=$CH_3$) | 0 | H | H | $CH_3$ | |
| Q-55($R_{12}$=$CH_3$) | 0 | H | H | $OCH_3$ | |
| Q-59($R_{12}$=H) | 0 | H | H | $OCH_3$ | |
| Q-61($R_{12}$,$R_{13}$=H) | 0 | H | H | $CH_3$ | |
| Q-62($R_{12}$,$R_{13}$=H) | 0 | H | H | $CH_3$ | |
| Q-63($R_{12}$,$R_{13}$=H) | 0 | H | H | $OCH_3$ | |
| Q-66($R_{12}$,$R_{13}$,$R_{14}$=H) | 0 | H | H | $CH_3$ | |
| Q-67($R_{12}$,$R_{13}$,$R_{14}$=H) | 0 | H | H | $OCH_3$ | |
| Q-69($R_{12}$,$R_{13}$,$R_{14}$=H) | 0 | H | H | $CH_3$ | |
| Q-72($R_{12}$,$R_{13}$=H,n'=0) | 0 | H | H | $CH_3$ | |
| Q-75($R_{12}$,$R_{17}$,$R_{18}$ =H) | 0 | H | H | $OCH_3$ | |
| Q-78($R_{12}$,$R_{13}$=H) | 0 | H | H | $CH_3$ | |
| Q-83($R_{12}$,$R_{13}$=H) | 0 | H | H | $CH_3$ | |
| Q-46($R_{12}$,$R_{13}$=H) | 1 | H | H | $OCH_3$ | |
| Q-47($R_{12}$,$R_{13}$=H) | 1 | H | H | $CH_3$ | |
| Q-53($R_{15}$=$CH_3$) | 1 | H | H | $CH_3$ | |
| Q-53($R_{15}$=$SCH_3$) | 1 | H | H | $OCH_3$ | |

## Table If Continued

| Q | n | R | $R_1$ | $X_3$ | m.p.°C |
|---|---|---|---|---|---|
| Q-55($R_{12}$=$CH_3$) | 1 | H | H | $CH_3$ | |
| Q-59($R_{12}$=H) | 1 | H | H | $CH_3$ | |
| Q-61($R_{12}$,$R_{13}$=H) | 1 | H | H | $OCH_3$ | |
| Q-63($R_{12}$,$R_{13}$=H) | 1 | H | H | $OCH_3$ | |
| Q-72($R_{12}$,$R_{13}$=H) | 1 | H | H | $CH_3$ | |
| Q-83($R_{12}$,$R_{13}$=H) | 1 | H | H | $OCH_3$ | |
| Q-46($R_{12}$,$R_{13}$=H) | 2 | H | H | $OCH_3$ | |
| Q-53($R_{15}$=$CH_3$) | 2 | H | H | $OCH_3$ | |

## Table 16g

### General Structure 9g

| Q | n | R | $E_1$ | X | Y | Z | m.p.°C |
|---|---|---|---|---|---|---|---|
| Q-46($R_{12}$,$R_{13}$=H) | 0 | H | H | $OCH_3$ | $OCH_3$ | CH | |
| Q-46($R_{12}$,$R_{13}$=H) | 1 | H | H | $OCH_3$ | $OCH_3$ | CH | |
| Q-47($R_{12}$,$R_{13}$=H) | 0 | H | H | $OCH_3$ | $CH_3$ | CH | |
| Q-48($R_{12}$,$R_{13}$=H) | 0 | H | H | $OCH_3$ | $OCH_3$ | CH | |
| Q-49($R_{12}$,$R_{13}$=H) | 0 | H | H | $OCH_3$ | $OCH_3$ | CH | |
| Q-46($R_{12}$,$R_{13}$=H) | 0 | H | H | $CH_3$ | $OCH_3$ | N | |
| Q-53($R_{15}$=$CH_3$) | 0 | H | H | $OCH_3$ | $CH_3$ | CH | |
| Q-54($R_{12}$=$CH_3$) | 0 | H | H | $OCH_3$ | $OCH_3$ | CH | |
| Q-55($R_{12}$=$CH_3$) | 0 | H | H | $CH_3$ | $OCH_3$ | CH | |
| Q-59($R_{12}$=H) | 0 | H | H | $OCH_3$ | $OCH_3$ | CH | |
| Q-61($R_{12}$,$R_{13}$=H) | 0 | H | H | $OCH_3$ | $OCH_3$ | CH | |
| Q-62($R_{12}$=H,$R_{13}$=$CH_3$) | 0 | H | H | $OCH_3$ | $OCH_3$ | CH | |
| Q-66($R_{12}$,$R_{13}$,$R_{14}$=H) | 0 | H | H | $OCH_3$ | $OCH_3$ | CH | |
| Q-67($R_{12}$,$R_{13}$,$R_{14}$=H) | 0 | H | H | $OCH_3$ | $OCH_3$ | CH | |
| Q-69($R_{12}$,$R_{13}$,$R_{14}$=H) | 0 | H | H | $OCH_3$ | $OCH_3$ | CH | |
| Q-78($R_{12}$,$R_{13}$=H) | 0 | H | H | $OCH_3$ | $OCH_3$ | CH | |
| Q-53($R_{15}$=$CH_3$) | 1 | H | H | $OCH_3$ | $OCH_3$ | CH | |
| Q-53($R_{15}$=$CH_3$) | 1 | H | H | $OCH_3$ | $CH_3$ | N | |
| Q-53($R_{15}$=$CH_3$) | 0 | H | H | $CH_3$ | $OCH_3$ | N | |
| Q-53($R_{15}$=$CH_3$) | 2 | H | H | $OCH_3$ | $OCH_3$ | CH | |

**Table 17a**

**General Structure 10a**

| Q | n | R | $E_1$ | $X_4$ | $Y_4$ | Z | m.p.°C |
|---|---|---|---|---|---|---|---|
| Q-46($R_{12}$,$R_{13}$=H) | 0 | $CH_3$ | H | $OCH_3$ | $OCH_3$ | CH | |
| Q-46($R_{12}$,$R_{13}$=H) | 0 | H | H | $CH_3$ | $CH_3$ | CH | |
| Q-46($R_{12}$,$R_{13}$=H) | 0 | H | H | $CH_3$ | $OCH_3$ | CH | |
| Q-46($R_{12}$,$R_{13}$=H) | 0 | H | H | $OCH_3$ | $OCH_3$ | CH | |
| Q-46($R_{12}$,$R_{13}$=H) | 0 | H | H | Cl | $OCH_3$ | CH | |
| Q-46($R_{12}$=H,$R_{13}$=$CH_3$) | 0 | H | H | $OCH_3$ | $OCH_3$ | CH | |
| Q-46($R_{12}$=$CH_3$,$R_{13}$=H) | 0 | H | H | $OCH_3$ | $OCH_3$ | CH | |
| Q-47($R_{12}$,$R_{13}$=H) | 0 | H | H | $CH_3$ | $CH_3$ | CH | |
| Q-47($R_{12}$,$R_{13}$=H) | 0 | H | H | $OCH_3$ | $OCH_3$ | CH | |
| Q-47($R_{12}$,$R_{13}$=H) | 0 | H | H | $CH_3$ | $OCH_3$ | CH | |
| Q-47($R_{12}$,$R_{13}$=H) | 0 | H | H | Cl | $OCH_3$ | CH | |
| Q-47($R_{12}$=$CH_3$,$R_{13}$=H) | 0 | H | H | $OCH_3$ | $OCH_3$ | CH | |
| Q-47($R_{12}$=H,$R_{13}$=$CH_3$) | 0 | H | H | $OCH_3$ | $OCH_3$ | CH | |
| Q-48($R_{12}$,$R_{13}$=H) | 0 | H | H | $CH_3$ | $CH_3$ | CH | |
| Q-48($R_{12}$,$R_{13}$=H) | 0 | H | H | $CH_3$ | $OCH_3$ | CH | |
| Q-48($R_{12}$,$R_{13}$=H) | 0 | H | H | $OCH_3$ | $OCH_3$ | CH | |
| Q-48($R_{12}$,$R_{13}$=H) | 0 | H | H | Cl | $OCH_3$ | CH | |
| Q-48($R_{12}$=$CH_3$,$R_{13}$=H) | 0 | H | H | $OCH_3$ | $OCH_3$ | CH | |
| Q-49($R_{12}$,$R_{13}$=H) | 0 | H | H | $CH_3$ | $CH_3$ | CH | |
| Q-49($R_{12}$,$R_{13}$=H) | 0 | H | H | $OCH_3$ | $CH_3$ | CH | |
| Q-49($R_{12}$,$R_{13}$=H) | 0 | H | H | $OCH_3$ | $OCH_3$ | CH | |
| Q-49($R_{12}$,$R_{13}$=H) | 0 | H | H | Cl | $OCH_3$ | CH | |
| Q-50($R_{12}$,$R_{13}$=H) | 0 | H | H | $OCH_3$ | $OCH_3$ | CH | |
| Q-50($R_{12}$,$R_{13}$=H) | 0 | H | H | $CH_3$ | $CH_3$ | CH | |
| Q-50($R_{12}$,$R_{13}$=H) | 0 | H | H | $CH_3$ | $OCH_3$ | CH | |
| Q-50($R_{12}$,$R_{13}$=H) | 0 | H | H | Cl | $OCH_3$ | CH | |

Table 17a Continued

| Q | n | R | $E_1$ | $X_4$ | $Y_4$ | Z | m.p.°C |
|---|---|---|---|---|---|---|---|
| Q-51($R_{12}$=H) | 0 | H | H | $OCH_3$ | $OCH_3$ | CH | |
| Q-51($R_{12}$=H) | 0 | H | H | $CH_3$ | $OCH_3$ | CH | |
| Q-51($R_{12}$=H) | 0 | H | H | $CH_3$ | $CH_3$ | CH | |
| Q-51($R_{12}$=H) | 0 | H | H | Cl | $OCH_3$ | CH | |
| Q-52($R_{12}$,$R_{13}$,$R_{14}$=H) | 0 | H | H | $CH_3$ | $CH_3$ | CH | |
| Q-52($R_{12}$,$R_{13}$,$R_{14}$=H) | 0 | H | H | $OCH_3$ | $OCH_3$ | CH | |
| Q-52($R_{12}$,$R_{13}$,$R_{14}$=H) | 0 | H | H | $CH_3$ | $OCH_3$ | CH | |
| Q-52($R_{12}$,$R_{13}$,$R_{14}$=H) | 0 | H | H | Cl | $OCH_3$ | CH | |
| Q-52($R_{12}$,$R_{14}$=$CH_3$;$R_{13}$=H) | 0 | H | H | $OCH_3$ | $OCH_3$ | CH | |
| Q-53($R_{15}$=H) | 0 | H | H | $CH_3$ | $CH_3$ | CH | |
| Q-53($R_{15}$=H) | 0 | H | H | $OCH_3$ | $OCH_3$ | CH | |
| Q-53($R_{15}$=H) | 0 | H | H | $CH_3$ | $OCH_3$ | CH | |
| Q-53($R_{15}$=H) | 0 | H | H | Cl | $OCH_3$ | CH | |
| Q-53($R_{15}$=$CH_3$) | 0 | H | H | $OCH_3$ | $OCH_3$ | CH | |
| Q-53($R_{15}$=$CH_3$) | 0 | H | H | $CH_3$ | $OCH_3$ | CH | |
| Q-53($R_{15}$=$CH_3$) | 0 | H | H | $CH_3$ | $CH_3$ | CH | |
| Q-53($R_{15}$=$CH_3$) | 0 | H | H | Cl | $OCH_3$ | CH | |
| Q-53($R_{15}$=$CH_3$) | 0 | $CH_3$ | H | $OCH_3$ | $OCH_3$ | CH | |
| Q-53($R_{15}$=$C_2H_5$) | 0 | H | H | $CH_3$ | $CH_3$ | CH | |
| Q-53($R_{15}$=$C_2H_5$) | 0 | H | H | $OCH_3$ | $OCH_3$ | CH | |
| Q-53($R_{15}$=$C_2H_5$) | 0 | H | H | $CH_3$ | $OCH_3$ | CH | |
| Q-53($R_{15}$=$C_2H_5$) | 0 | H | H | Cl | $OCH_3$ | CH | |
| Q-53($R_{15}$=$SCH_3$) | 0 | H | H | $OCH_3$ | $OCH_3$ | CH | |
| Q-53($R_{15}$=$SCH_3$) | 0 | H | H | $CH_3$ | $OCH_3$ | CH | |
| Q-53($R_{15}$=$SCH_3$) | 0 | H | H | $CH_3$ | $CH_3$ | CH | |
| Q-53($R_{15}$=$SCH_3$) | 0 | H | H | Cl | $OCH_3$ | CH | |
| Q-53($R_{15}$=$SC_2H_5$) | 0 | H | H | $CH_3$ | $CH_3$ | CH | |
| Q-53($R_{15}$=$SC_2H_5$) | 0 | H | H | $OCH_3$ | $OCH_3$ | CH | |
| Q-53($R_{15}$=$SC_2H_5$) | 0 | H | H | $CH_3$ | $OCH_3$ | CH | |
| Q-53($R_{15}$=$SC_2H_5$) | 0 | H | H | Cl | $OCH_3$ | CH | |
| Q-53($R_{15}$=$SCH_2CH$=$CH_2$) | 0 | H | H | $OCH_3$ | $OCH_3$ | CH | |

Table 17a Continued

| Q | n | R | $E_1$ | $X_4$ | $Y_4$ | Z | m.p.°C |
|---|---|---|---|---|---|---|---|
| Q-53($R_{15}$=$SCH_2CH$=$CH_2$) | 0 | H | H | $CH_3$ | $OCH_3$ | CH | |
| Q-53($R_{15}$=$SCH_2CH$=$CH_2$) | 0 | H | H | $CH_3$ | $CH_3$ | CH | |
| Q-53($R_{15}$=$SCH_2CH$=$CH_2$) | 0 | H | H | Cl | $OCH_3$ | CH | |
| Q-53($R_{15}$=$SCF_2H$) | 0 | H | H | $OCH_3$ | $OCH_3$ | CH | |
| Q-53($R_{15}$=$SCF_2H$) | 0 | H | H | $CH_3$ | $CH_3$ | CH | |
| Q-53($R_{15}$=$SCF_2H$) | 0 | H | H | $OCH_3$ | $CH_3$ | CH | |
| Q-53($R_{15}$=$SCF_2H$) | 0 | H | H | Cl | $OCH_3$ | CH | |
| Q-53($R_{15}$=$SC_3H_7$) | 0 | H | H | $OCH_3$ | $OCH_3$ | CH | |
| Q-53($R_{15}$=$SC_3H_7$) | 0 | H | H | $CH_3$ | $OCH_3$ | CH | |
| Q-53($R_{15}$=$SC_3H_7$) | 0 | H | H | Cl | $OCH_3$ | CH | |
| Q-53($R_{15}$=$OCH_3$) | 0 | H | H | $OCH_3$ | $OCH_3$ | CH | |
| Q-53($R_{15}$=$OC_2H_5$) | 0 | H | H | $OCH_3$ | $OCH_3$ | CH | |
| Q-54($R_{12}$=H) | 0 | H | H | $OCH_3$ | $OCH_3$ | CH | |
| Q-54($R_{12}$=H) | 0 | H | H | $CH_3$ | $OCH_3$ | CH | |
| Q-54($R_{12}$=H) | 0 | H | H | $CH_3$ | $CH_3$ | CH | |
| Q-54($R_{12}$=H) | 0 | H | H | Cl | $OCH_3$ | CH | |
| Q-54($R_{12}$=$CH_3$) | 0 | H | H | $OCH_3$ | $OCH_3$ | CH | |
| Q-54($R_{12}$=$CH_3$) | 0 | H | H | $CH_3$ | $OCH_3$ | CH | |
| Q-54($R_{12}$=$CH_3$) | 0 | H | H | $CH_3$ | $CH_3$ | CH | |
| Q-54($R_{12}$=$CH_3$) | 0 | H | H | Cl | $OCH_3$ | CH | |
| Q-55($R_{12}$=H) | 0 | H | H | $CH_3$ | $CH_3$ | CH | |
| Q-55($R_{12}$=H) | 0 | H | H | $OCH_3$ | $OCH_3$ | CH | |
| Q-55($R_{12}$=H) | 0 | H | H | $CH_3$ | $OCH_3$ | CH | |
| Q-55($R_{12}$=H | 0 | H | H | Cl | $OCH_3$ | CH | |
| Q-55($R_{12}$=$CH_3$) | 0 | H | H | $CH_3$ | $CH_3$ | CH | 160-165 |
| Q-55($R_{12}$=$CH_3$) | 0 | H | H | $OCH_3$ | $OCH_3$ | CH | 169-173 |
| Q-55($R_{12}$=$CH_3$) | 0 | H | H | $CH_3$ | $OCH_3$ | CH | |
| Q-55($R_{12}$=$CH_3$) | 0 | H | H | Cl | $OCH_3$ | CH | 162-166 |
| Q-54($R_{12}$=H) | 0 | H | H | $OCH_3$ | $OCH_3$ | CH | |
| Q-55($R_{16}$=H) | 0 | H | H | $OCH_3$ | $OCH_3$ | CH | |
| Q-55($R_{16}$=Cl) | 0 | H | H | $OCH_3$ | $OCH_3$ | CH | |

Table 17a Continued

| Q | n | R | $E_1$ | $X_4$ | $Y_4$ | Z | m.p.°C |
|---|---|---|---|---|---|---|---|
| Q-58($R_{12}$=$CH_3$) | 0 | H | H | $OCH_3$ | $OCH_3$ | CH | |
| Q-59($R_{12}$=H) | 0 | H | H | $OCH_3$ | $OCH_3$ | CH | |
| Q-59($R_{12}$=H) | 0 | H | H | $CH_3$ | $OCH_3$ | CH | |
| Q-59($R_{12}$=H) | 0 | H | H | Cl | $OCH_3$ | CH | |
| Q-60($R_{12}$=H) | 0 | H | H | $OCH_3$ | $OCH_3$ | CH | |
| Q-61($R_{12}$,$R_{13}$=H) | 0 | H | H | $CH_3$ | $CH_3$ | CH | |
| Q-61($R_{12}$,$R_{13}$=H) | 0 | H | H | $OCH_3$ | $OCH_3$ | CH | |
| Q-61($R_{12}$,$R_{13}$=H) | 0 | H | H | $CH_3$ | $OCH_3$ | CH | |
| Q-61($R_{12}$,$R_{13}$=H) | 0 | H | H | Cl | $OCH_3$ | CH | |
| Q-62($R_{12}$,$R_{13}$=H) | 0 | H | H | $OCH_3$ | $OCH_3$ | CH | |
| Q-62($R_{12}$,$R_{13}$=H) | 0 | H | H | $CH_3$ | $OCH_3$ | CH | |
| Q-62($R_{12}$,$R_{13}$=H) | 0 | H | H | $CH_3$ | $CH_3$ | CH | |
| Q-62($R_{12}$,$R_{13}$=H) | 0 | H | H | Cl | $OCH_3$ | CH | |
| Q-63($R_{12}$,$R_{13}$=H) | 0 | H | H | $OCH_3$ | $OCH_3$ | CH | |
| Q-63($R_{12}$,$R_{13}$=H) | 0 | H | H | $CH_3$ | $OCH_3$ | CH | |
| Q-63($R_{12}$,$R_{13}$=H) | 0 | H | H | $CH_3$ | $CH_3$ | CH | |
| Q-63($R_{12}$,$R_{13}$=H) | 0 | H | H | Cl | $OCH_3$ | CH | |
| Q-64($R_{12}$=$CH_3$,$R_{13}$=H) | 0 | H | H | $OCH_3$ | $OCH_3$ | CH | |
| Q-65($R_{12}$,$R_{13}$=$CH_3$) | 0 | H | H | $OCH_3$ | $OCH_3$ | CH | |
| Q-66($R_{12}$,$R_{13}$,$R_{14}$=H) | 0 | H | H | $OCH_3$ | $OCH_3$ | CH | |
| Q-67($R_{12}$,$R_{13}$,$R_{14}$=H) | 0 | H | H | $OCH_3$ | $OCH_3$ | CH | |
| Q-68($R_{12}$,$R_{13}$,$R_{14}$=H) | 0 | H | H | $OCH_3$ | $OCH_3$ | CH | |
| Q-69($R_{12}$,$R_{13}$,$R_{14}$=H) | 0 | H | H | $OCH_3$ | $CH_3$ | CH | |
| Q-70($R_{12}$,$R_{13}$,$R_{14}$=H) | 0 | H | H | $OCH_3$ | $OCH_3$ | CH | |
| Q-71(W'=O) | 0 | H | H | $OCH_3$ | $OCH_3$ | CH | |
| Q-72($R_{12}$,$R_{13}$=H,n'=0) | 0 | H | H | $OCH_3$ | $OCH_3$ | CH | |
| Q-73($R_{12}$,$R_{13}$=H,n'=0) | 0 | H | H | $OCH_3$ | $OCH_3$ | CH | |
| Q-74($R_{12}$,$R_{13}$=H,n'=0) | 0 | H | H | $OCH_3$ | $OCH_3$ | CH | |
| Q-75($R_{12}$=H;$R_{17}$,$R_{18}$=$OCH_3$) | 0 | H | H | $OCH_3$ | $OCH_3$ | CH | |
| Q-75($R_{12}$=H;$R_{17}$,$R_{18}$ =$CH_3$) | 0 | H | H | $OCH_3$ | $OCH_3$ | CH | |
| Q-76($R_{17}$,$R_{18}$=H) | 0 | H | H | $OCH_3$ | $OCH_3$ | CH | |

Table 17a Continued

| Q | n | R | $E_1$ | $X_4$ | $Y_4$ | Z | m.p.°C |
|---|---|---|---|---|---|---|---|
| Q-77($R_{17}$,$R_{18}$ =H) | 0 | H | H | $OCH_3$ | $OCH_3$ | CH | |
| Q-78($R_{12}$,$R_{13}$=H) | 0 | H | H | $OCH_3$ | $OCH_3$ | CH | |
| Q-79($R_{12}$,$R_{13}$=H) | 0 | H | H | $OCH_3$ | $OCH_3$ | CH | |
| Q-80($R_{19}$,$R_{20}$=$OCH_3$) | 0 | H | H | $OCH_3$ | $OCH_3$ | CH | |
| Q-81($R_{19}$,$R_{20}$ =$CH_3$) | 0 | H | H | $OCH_3$ | $OCH_3$ | CH | |
| Q-46($R_{12}$,$R_{13}$=H) | 0 | H | H | $CH_3$ | $OCH_3$ | N | |
| Q-47($R_{12}$,$R_{13}$=H) | 0 | H | H | $OCH_3$ | $OCH_3$ | N | |
| Q-48($R_{12}$=$CH_3$,$R_{13}$=H) | 0 | H | H | $OCH_3$ | $OCH_3$ | N | |
| Q-53($R_{15}$=$CH_3$) | 0 | H | H | $CH_3$ | $OCH_3$ | N | |
| Q-53($R_{15}$=$SCH_3$) | 0 | H | H | $OCH_3$ | $OCH_3$ | N | |
| Q-54($R_{12}$=$CH_3$) | 0 | H | H | $CH_3$ | $OCH_3$ | N | |
| Q-55($R_{12}$=$CH_3$) | 0 | H | H | $CH_3$ | $OCH_3$ | N | |
| Q-57($R_{16}$=Cl) | 0 | H | H | $CH_3$ | $OCH_3$ | N | |
| Q-62 | 0 | H | H | $OCH_3$ | $CH_3$ | N | |
| Q-63 | 0 | H | H | $CH_3$ | $OCH_3$ | N | |
| Q-63 | 0 | H | H | $OCH_3$ | $OCH_3$ | N | |
| Q-67($R_{12}$,$R_{13}$,$R_{14}$=H) | 0 | H | H | $CH_3$ | $OCH_3$ | N | |
| Q-71(W'=S) | 0 | H | H | $CH_3$ | $OCH_3$ | N | |
| Q-72($R_{12}$,$R_{13}$=H,n'=0) | 0 | H | H | $CH_3$ | $OCH_3$ | N | |
| Q-72($R_{12}$,$R_{13}$=H,n'=0) | 0 | H | H | $OCH_3$ | $OCH_3$ | N | |
| Q-75($R_{12}$,$R_{17}$,$R_{18}$=H) | 0 | H | H | $CH_3$ | $OCH_3$ | N | |
| Q-75($R_{12}$,$R_{17}$,$R_{18}$=H) | 0 | H | H | $OCH_3$ | $OCH_3$ | N | |
| Q-78($R_{12}$,$R_{13}$=H) | 0 | H | H | $CH_3$ | $OCH_3$ | N | |
| Q-47($R_{12}$,$R_{13}$=H) | 0 | H | 5-Cl | $OCH_3$ | $OCH_3$ | CH | |

**Table 17a Continued**

| Q | n | R | $E_1$ | $X_4$ | $Y_4$ | Z | m.p.°C |
|---|---|---|---|---|---|---|---|
| Q-61($R_{12}$,$R_{13}$=H) | 0 | H | 6-Br | $OCH_3$ | $OCH_3$ | CH | |
| Q-53($R_{15}$=$SCH_3$) | 0 | H | 5-$CH_3$ | $OCH_3$ | $OCH_3$ | CH | |
| Q-53($R_{15}$=$SCH_3$) | 0 | H | 5-Cl | $OCH_3$ | $CH_3$ | N | |
| Q-46($R_{12}$,$R_{13}$=H) | 0 | H | 5-$CH_3$ | $OCH_3$ | $CH_3$ | N | |
| Q-46($R_{12}$,$R_{13}$=H) | 0 | $CH_3$ | H | $OCH_3$ | $CH_3$ | N | |
| Q-53($R_{15}$=$SCH_3$) | 0 | H | H | $OC_2H_5$ | $CH_3$ | CH | |
| Q-46($R_{12}$,$R_{13}$=H) | 0 | H | H | F | $OCH_3$ | CH | |
| Q-47($R_{12}$,$R_{13}$=H) | 0 | H | H | Br | $OCH_3$ | CH | |
| Q-48($R_{12}$,$R_{13}$=H) | 0 | H | H | I | $OCH_3$ | CH | |
| Q-53($R_{15}$=$SCH_3$) | 0 | H | H | $OCF_2H$ | $OCH_3$ | CH | |
| Q-55($R_{12}$=$CH_3$) | 0 | H | H | $CH_2F$ | $OCH_3$ | CH | |
| Q-63($R_{12}$=H,$R_{13}$=$CH_3$) | 0 | H | H | $OCH_2CH_2F$ | $CH_3$ | CH | |
| Q-62($R_{12}$=H,$R_{13}$=$CH_3$) | 0 | H | H | $OCH_2CHF_2$ | $CH_3$ | CH | |
| Q-53($R_{15}$=$SCH_3$) | 0 | H | H | $OCH_2CF_3$ | $OCH_3$ | CH | |
| Q-55($R_{12}$=$CH_3$) | 0 | H | H | $CF_3$ | $OCH_3$ | CH | |
| Q-47($R_{12}$,$R_{13}$=H) | 0 | H | H | $OCH_2CH_3$ | $OCH_3$ | N | |
| Q-46($R_{12}$,$R_{13}$=H) | 0 | H | H | $OCH_2CH_2F$ | $CH_3$ | N | |
| Q-53($R_{15}$=$SCH_3$) | 0 | H | H | $OCH_2CF_3$ | $OCH_3$ | N. | |
| Q-54($R_{12}$=$CH_3$) | 0 | H | H | $OCH_3$ | H | CH | |
| Q-61 | 0 | H | H | $CH_3$ | $OC_2H_5$ | CH | |
| Q-53($R_{15}$=$SCH_3$) | 0 | H | H | $CH_3$ | $CH_2OCH_3$ | CH | |
| Q-53($R_{15}$=$CH_3$) | 0 | H | H | $OC_2H_5$ | $NHCH_3$ | N | |
| Q-62($R_{12}$=H,$R_{13}$=$CH_3$) | 0 | H | H | $OCH_3$ | $N(OCH_3)CH_3$ | N | |
| Q-53($R_{15}$=$SCH_3$) | 0 | H | H | $OCH_3$ | $N(CH_3)_2$ | N | |
| Q-63($R_{12}$=H,$R_{13}$=$CH_3$) | 0 | H | H | $OCH_3$ | $C_2H_5$ | CH | |
| Q-57($R_{16}$=Cl) | 0 | H | H | $OCH_3$ | $CF_3$ | CH | |
| Q-56($R_{12}$=$CH_3$) | 0 | H | H | $OCH_3$ | $SCH_3$ | N | |
| Q-63($R_{12}$=H,$R_{13}$=$CH_3$) | 0 | H | H | $CH_3$ | $OCH_2CH=CH_2$ | N | |
| Q-55($R_{12}$=H) | 0 | H | H | $CH_3$ | $OCH_2C\equiv CH$ | N | |
| Q-58($R_{12}$=$CH_3$) | 0 | H | H | $OCH_3$ | $CH_2OCH_2CH_3$ | CH | |
| Q-62($R_{12}$=H,$R_{13}$=$CH_3$) | 0 | H | H | $CH_3$ | $OCH_2CH_2OCH_3$ | CH | |

Table 17a Continued

| Q | n | R | $E_1$ | $X_4$ | $Y_4$ | Z | m.p.°C |
|---|---|---|---|---|---|---|---|
| Q-59 | 0 | H | H | $CH_3$ | $CH_2SCH_3$ | CH | |
| Q-55($R_{12}$=$CH_3$) | 0 | H | H | $CH_3$ | $\overset{O}{\overset{\|}{C}}H$ | CH | |
| Q-46($R_{12}$,$R_{13}$=H) | 0 | H | H | $CH_3$ | $\overset{O}{\overset{\|}{C}}CH_3$ | CH | |
| Q-53($R_{15}$=$SCH_3$) | 0 | H | H | $OCH_3$ | $CH(OCH_3)_2$ | CH | |
| Q-46($R_{12}$,$R_{13}$=H) | 0 | H | H | $CH_3$ | $CH(OCH_3)_2$ | N | |
| Q-53($R_{12}$=$SCH_3$) | 0 | H | H | $OCH_3$ | $CH(OC_2H_5)_2$ | CH | |
| Q-47($R_{12}$,$R_{13}$=H) | 0 | H | H | $OCH_3$ | $CH(SCH_3)_2$ | CH | |
| Q-48($R_{12}$,$R_{13}$=H) | 0 | H | H | $OCH_3$ | $C(CH_3)(OCH_3)_2$ | CH | |
| Q-53($R_{15}$=$SCH_3$) | 0 | H | H | $OCH_3$ | 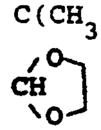 | CH | |
| Q-63 | 0 | H | H | $CH_3$ | 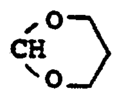 | CH | |
| Q-62 | 0 | H | H | $OCH_3$ | 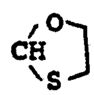 | CH | |
| Q-53($R_{15}$=$CH_3$) | 0 | H | H | $OCH_3$ | 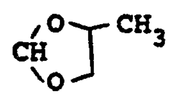 | CH | |
| Q-55($R_{12}$=H) | 0 | H | H | $OCH_3$ | $OCF_2H$ | CH | |
| Q-59 | 0 | H | H | $OCH_3$ | $SCF_2H$ | CH | |
| Q-53($R_{15}$=$SCH_3$) | 0 | H | H | $OCH_3$ | 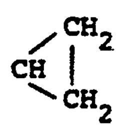 | N | |
| Q-46($R_{12}$,$R_{13}$=H) | 0 | H | H | $OCH_3$ | 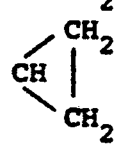 | CH | |

Table 17a Continued

| Q | n | R | $E_1$ | $X_4$ | $Y_4$ | Z | m.p.°C |
|---|---|---|---|---|---|---|---|
| Q-46($R_{12}$,$R_{13}$=H) | 1 | $CH_3$ | H | $OCH_3$ | $OCH_3$ | CH | |
| Q-46($R_{12}$,$R_{13}$=H) | 1 | H | H | $CH_3$ | $CH_3$ | CH | |
| Q-46($R_{12}$,$R_{13}$=H) | 1 | H | H | $CH_3$ | $OCH_3$ | CH | |
| Q-46($R_{12}$,$R_{13}$=H) | 1 | H | H | $OCH_3$ | $OCH_3$ | CH | |
| Q-46($R_{12}$,$R_{13}$=H) | 1 | H | H | Cl | $OCH_3$ | CH | |
| Q-46($R_{12}$=H,$R_{13}$=$CH_3$) | 1 | H | H | $OCH_3$ | $OCH_3$ | CH | |
| Q-46($R_{12}$=$CH_3$,$R_{13}$=H) | 1 | H | H | $OCH_3$ | $OCH_3$ | CH | |
| Q-47($R_{12}$,$R_{13}$=H) | 1 | H | H | $CH_3$ | $CH_3$ | CH | |
| Q-47($R_{12}$,$R_{13}$=H) | 1 | H | H | $OCH_3$ | $OCH_3$ | CH | |
| Q-47($R_{12}$,$R_{13}$=H) | 1 | H | H | $CH_3$ | $OCH_3$ | CH | |
| Q-47($R_{12}$,$R_{13}$=H) | 1 | H | H | Cl | $OCH_3$ | CH | |
| Q-47($R_{12}$=$CH_3$,$R_{13}$=H) | 1 | H | H | $OCH_3$ | $OCH_3$ | CH | |
| Q-47($R_{12}$=H,$R_{13}$=$CH_3$) | 1 | H | H | $OCH_3$ | $OCH_3$ | CH | |
| Q-48($R_{12}$,$R_{13}$=H) | 1 | H | H | $CH_3$ | $CH_3$ | CH | |
| Q-48($R_{12}$,$R_{13}$=H) | 1 | H | H | $CH_3$ | $OCH_3$ | CH | |
| Q-48($R_{12}$,$R_{13}$=H) | 1 | H | H | $OCH_3$ | $OCH_3$ | CH | |
| Q-48($R_{12}$,$R_{13}$=H) | 1 | H | H | Cl | $OCH_3$ | CH | |
| Q-48($R_{12}$=$CH_3$: $R_{13}$=H) | 1 | H | H | $OCH_3$ | $OCH_3$ | CH | |
| Q-49($R_{12}$,$R_{13}$=H) | 1 | H | H | $CH_3$ | $CH_3$ | CH | |
| Q-49($R_{12}$,$R_{13}$=H) | 1 | H | H | $OCH_3$ | $CH_3$ | CH | |
| Q-49($R_{12}$,$R_{13}$=H) | 1 | H | H | $OCH_3$ | $OCH_3$ | CH | |
| Q-49($R_{12}$,$R_{13}$=H) | 1 | H | H | Cl | $OCH_3$ | CH | |
| Q-50($R_{12}$,$R_{13}$=H) | 1 | H | H | $OCH_3$ | $OCH_3$ | CH | |
| Q-50($R_{12}$,$R_{13}$=H) | 1 | H | H | $CH_3$ | $CH_3$ | CH | |
| Q-50($R_{12}$,$R_{13}$=H) | 1 | H | H | $CH_3$ | $OCH_3$ | CH | |
| Q-50($R_{12}$,$R_{13}$=H) | 1 | H | H | Cl | $OCH_3$ | CH | |
| Q-51($R_{12}$=H) | 1 | H | H | $OCH_3$ | $OCH_3$ | CH | |
| Q-51($R_{12}$=H) | 1 | H | H | $CH_3$ | $OCH_3$ | CH | |
| Q-51($R_{12}$=H) | 1 | H | H | $CH_3$ | $CH_3$ | CH | |
| Q-51($R_{12}$=H) | 1 | H | H | Cl | $OCH_3$ | CH | |
| Q-53($R_{15}$=H) | 1 | H | H | $CH_3$ | $CH_3$ | CH | |
| Q-53($R_{15}$=H) | 1 | H | H | $OCH_3$ | $OCH_3$ | CH | |

Table 17a Continued

| Q | n | R | $E_1$ | $X_4$ | $Y_4$ | Z | m.p.°C |
|---|---|---|---|---|---|---|---|
| Q-53($R_{15}$=H) | 1 | H | H | $CH_3$ | $OCH_3$ | CH | |
| Q-53($R_{15}$=H) | 1 | H | H | Cl | $OCH_3$ | CH | |
| Q-54($R_{12}$=H) | 1 | H | H | $OCH_3$ | $OCH_3$ | CH | |
| Q-54($R_{12}$=H) | 1 | H | H | $CH_3$ | $CH_3$ | CH | |
| Q-55($R_{12}$=H) | 1 | H | H | $OCH_3$ | $OCH_3$ | CH | |
| Q-55($R_{12}$=H) | 1 | H | H | $CH_3$ | $OCH_3$ | CH | |
| Q-55($R_{12}$=H) | 1 | H | H | Cl | $OCH_3$ | CH | |
| Q-56($R_{12}$=H) | 1 | H | H | $OCH_3$ | $OCH_3$ | CH | |
| Q-56($R_{12}$=H) | 1 | H | H | $CH_3$ | $OCH_3$ | CH | |
| Q-57($R_{16}$=H) | 1 | H | H | $CH_3$ | $CH_3$ | CH | |
| Q-57($R_{16}$=H) | 1 | H | H | Cl | $OCH_3$ | CH | |
| Q-58($R_{12}$=$CH_3$) | 1 | H | H | $OCH_3$ | $OCH_3$ | CH | |
| Q-59($R_{12}$=H) | 1 | H | H | $CH_3$ | $CH_3$ | CH | |
| Q-59($R_{12}$=H) | 1 | H | H | $OCH_3$ | $OCH_3$ | CH | |
| Q-59($R_{12}$=H) | 1 | H | H | $CH_3$ | $OCH_3$ | CH | |
| Q-59($R_{12}$=H) | 1 | H | H | Cl | $OCH_3$ | CH | |
| Q-60($R_{12}$=H) | 1 | H | H | $OCH_3$ | $OCH_3$ | CH | |
| Q-61($R_{12}$,$R_{13}$=H) | 1 | H | H | $CH_3$ | $OCH_3$ | CH | |
| Q-61($R_{12}$,$R_{13}$=H) | 1 | H | H | $CH_3$ | $CH_3$ | CH | |
| Q-61($R_{12}$,$R_{13}$=H) | 1 | H | H | Cl | $OCH_3$ | CH | |
| Q-62($R_{12}$,$R_{13}$=H) | 1 | H | H | $CH_3$ | $CH_3$ | CH | |
| Q-61($R_{12}$,$R_{13}$=H) | 1 | H | H | $OCH_3$ | $OCH_3$ | CH | |
| Q-63($R_{12}$,$R_{13}$=H) | 1 | H | H | $CH_3$ | $OCH_3$ | CH | |
| Q-63($R_{12}$,$R_{13}$=H) | 1 | H | H | Cl | $OCH_3$ | CH | |
| Q-63($R_{12}$,$R_{13}$=H) | 1 | H | H | $OCH_3$ | $OCH_3$ | CH | |
| Q-64($R_{12}$=$CH_3$,$R_{13}$=H) | 1 | H | H | $OCH_3$ | $OCH_3$ | CH | |
| Q-65($R_{12}$,$R_{13}$=$CH_3$) | 1 | H | H | $OCH_3$ | $OCH_3$ | CH | |
| Q-67($R_{12}$,$R_{13}$,$R_{14}$=H) | 1 | H | H | $OCH_3$ | $OCH_3$ | CH | |
| Q-69($R_{12}$,$R_{13}$,$R_{14}$=H) | 1 | H | H | $OCH_3$ | $CH_3$ | CH | |
| Q-73($R_{12}$,$R_{13}$=H,n'=1) | 1 | H | H | $OCH_3$ | $OCH_3$ | CH | |
| Q-75($R_{12}$=H,$R_{17}$,$R_{18}$=$CH_3$) | 1 | H | H | $OCH_3$ | $OCH_3$ | CH | |
| Q-78($R_{12}$,$R_{13}$=H) | 1 | H | H | $OCH_3$ | $OCH_3$ | CH | |

Table 17a Continued

| Q | n | R | $E_1$ | $X_4$ | $Y_4$ | Z | m.p.°C |
|---|---|---|---|---|---|---|---|
| Q-80($R_{19}$,$R_{20}$=$OCH_3$) | 1 | H | H | $OCH_3$ | $OCH_3$ | CH | |
| $C_6H_5$ | 1 | H | H | $OCH_3$ | $OCH_3$ | CH | |
| $C_6H_5$ | 1 | H | H | $OCH_3$ | $CH_3$ | CH | |
| Q-82($R_{12}$,$R_{13}$=H) | 1 | H | H | $OCH_3$ | $OCH_3$ | CH | |
| Q-83($R_{12}$,$R_{13}$=H) | 1 | H | H | $OCH_3$ | $OCH_3$ | CH | |
| Q-84($R_{12}$,$R_{13}$=H) | 1 | H | H | $OCH_3$ | $OCH_3$ | CH | |
| Q-46($R_{12}$,$R_{13}$=H) | 1 | H | H | $OCH_3$ | $OCH_3$ | N | |
| Q-47($R_{12}$,$R_{13}$=H) | 1 | H | H | $CH_3$ | $OCH_3$ | N | |
| Q-53($R_{15}$=$CH_3$) | 1 | H | H | $CH_3$ | $OCH_3$ | N | |
| Q-53($R_{15}$=$CH_3$) | 1 | H | H | $OCH_3$ | $OCH_3$ | N | |
| Q-57($R_{16}$=Cl) | 1 | H | H | $OCH_3$ | $OCH_3$ | N | |
| Q-62($R_{12}$,$R_{13}$=H) | 1 | H | H | $OCH_3$ | $OCH_3$ | N | |
| Q-63($R_{12}$,$R_{13}$=H) | 1 | H | H | $OCH_3$ | $OCH_3$ | N | |
| Q-67($R_{12}$,$R_{13}$,$R_{14}$=H) | 1 | H | H | $OCH_3$ | $CH_3$ | N | |
| Q-71(W'=O) | 1 | H | H | $OCH_3$ | $OCH_3$ | N | |
| Q-72($R_{12}$,$R_{13}$=H) | 1 | H | H | $OCH_3$ | $OCH_3$ | N | |
| Q-75($R_{12}$,$R_{17}$,$R_{18}$=H) | 1 | H | H | $OCH_3$ | $OCH_3$ | N | |
| Q-78($R_{12}$,$R_{13}$=H) | 1 | H | H | $OCH_3$ | $OCH_3$ | N | |
| Q-47($R_{12}$,$R_{13}$=H) | 1 | H | 5-Cl | $OCH_3$ | $OCH_3$ | N | |
| Q-61($R_{12}$,$R_{13}$=H) | 1 | H | 5-Br | $OCH_3$ | $OCH_3$ | CH | |
| Q-53($R_{15}$=$SCH_3$) | 1 | H | 5-$CH_3$ | $OCH_3$ | $OCH_3$ | CH | |
| Q-53($R_{15}$=$SCH_3$) | 1 | H | 5-Cl | $OCH_3$ | $CH_3$ | N | |
| Q-46($R_{12}$,$R_{13}$=H) | 1 | H | 5-$CH_3$ | $OCH_3$ | $CH_3$ | N | |
| Q-46($R_{12}$,$R_{13}$=H) | 1 | $CH_3$ | H | $OCH_3$ | $CH_3$ | N | |
| Q-53($R_{15}$=$SCH_3$) | 1 | H | H | $OC_2H_5$ | $CH_3$ | CH | |
| Q-46($R_{12}$,$R_{13}$=H) | 1 | H | H | F | $OCH_3$ | CH | |
| Q-47($R_{12}$,$R_{13}$=H) | 1 | H | H | Br | $OCH_3$ | CH | |
| Q-48($R_{12}$,$R_{13}$=H) | 1 | H | H | I | $OCH_3$ | CH | |
| Q-53($R_{15}$=$SCH_3$) | 1 | H | H | $OCF_2H$ | $OCH_3$ | CH | |
| Q-55($R_{12}$=$CH_3$) | 1 | H | H | $CH_2F$ | $OCH_3$ | CH | |

Table 17a Continued

| Q | n | R | $E_1$ | $X_4$ | $Y_4$ | Z | m.p.°C |
|---|---|---|---|---|---|---|---|
| Q-63($R_{12}$,$R_{13}$=H) | 1 | H | H | $OCH_2CH_2F$ | $CH_3$ | CH | |
| Q-62($R_{12}$,$R_{13}$=H) | 1 | H | H | $OCH_2CHF_2$ | $CH_3$ | CH | |
| Q-53($R_{15}$=$SCH_3$) | 1 | H | H | $OCH_2CF_3$ | $OCH_3$ | CH | |
| Q-55($R_{12}$=$CH_3$) | 1 | H | H | $CF_3$ | $OCH_3$ | CH | |
| Q-47($R_{12}$,$R_{13}$=H) | 1 | H | H | $OCH_2CH_3$ | $OCH_3$ | N | |
| Q-46($R_{12}$,$R_{13}$=H) | 1 | H | H | $OCH_2CH_2F$ | $CH_3$ | N | |
| Q-53($R_{15}$=$SCH_3$) | 1 | H | H | $OCH_2CF_3$ | $OCH_3$ | N | |
| Q-54($R_{12}$=$CH_3$) | 1 | H | H | $OCH_3$ | H | CH | |
| Q-61($R_{12}$=H,$R_{13}$=$CH_3$) | 1 | H | H | $CH_3$ | $OC_2H_5$ | CH | |
| Q-53($R_{15}$=$SCH_3$) | 1 | H | H | $CH_3$ | $CH_2OCH_3$ | CH | |
| Q-53($R_{15}$=$CH_3$) | 1 | H | H | $OC_2H_5$ | $NHCH_3$ | N | |
| Q-62($R_{12}$=H,$R_{13}$=$CH_3$) | 1 | H | H | $OCH_3$ | $N(OCH_3)CH_3$ | N | |
| Q-53($R_{15}$=$SCH_3$) | 1 | H | H | $OCH_3$ | $N(CH_3)_2$ | N | |
| Q-63($R_{12}$=H,$R_{13}$=$CH_3$) | 1 | H | H | $OCH_3$ | $C_2H_5$ | CH | |
| Q-57($R_{16}$=Cl) | 1 | H | H | $OCH_3$ | $CF_3$ | CH | |
| Q-56($R_{12}$=$CH_3$) | 1 | H | H | $OCH_3$ | $SCH_3$ | N | |
| Q-63($R_{12}$=H,$R_{13}$=$CH_3$) | 1 | H | H | $CH_3$ | $OCH_2CH{=}CH_2$ | N | |
| Q-55($R_{12}$=$CH_3$) | 1 | H | H | $CH_3$ | $OCH_2C{\equiv}CH$ | N | |
| Q-58($R_{12}$=$CH_3$) | 1 | H | H | $OCH_3$ | $CH_2OCH_2CH_3$ | CH | |
| Q-62($R_{12}$=H,$R_{13}$=$CH_3$) | 1 | H | H | $CH_3$ | $OCH_2CH_2OCH_3$ | CH | |
| Q-59($R_{12}$=H) | 1 | H | H | $CH_3$ | $CH_2SCH_3$ | CH | |
| Q-46($R_{12}$,$R_{13}$=H) | 1 | H | H | $OCH_3$ | i-$C_3H_7$ | CH | |
| Q-55($R_{12}$=$CH_3$) | 1 | H | H | $CH_3$ | CHO | CH | |
| Q-46($R_{12}$,$R_{13}$=H) | 1 | H | H | $CH_3$ | $COCH_3$ | CH | |
| Q-53($R_{15}$=$SCH_3$) | 1 | H | H | $OCH_3$ | $CH(OCH_3)_2$ | CH | |
| Q-46($R_{12}$,$R_{13}$=H) | 1 | H | H | $CH_3$ | $CH(OCH_3)_2$ | N | |
| Q-53($R_{12}$=$SCH_3$) | 1 | H | H | $OCH_3$ | $CH(OC_2H_5)_2$ | CH | |
| Q-47($R_{12}$,$R_{13}$=H) | 1 | H | H | $OCH_3$ | $CH(SCH_3)_2$ | CH | |
| Q-48($R_{12}$,$R_{13}$=H) | 1 | H | H | $OCH_3$ | $C(CH_3)(OCH_3)_2$ | CH | |

## Table 17a Continued

| Q | n | R | $E_1$ | $X_4$ | $Y_4$ | Z | m.p.°C |
|---|---|---|---|---|---|---|---|
| Q-53($R_{15}$=$SCH_3$) | 1 | H | H | $OCH_3$ | 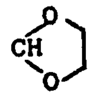 | CH | |
| Q-63($R_{12}$=$R_{13}$=H) | 1 | H | H | $CH_3$ | 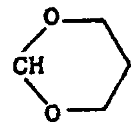 | CH | |
| Q-62($R_{12}$=$R_{13}$=H) | 1 | H | H | $OCH_3$ | 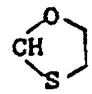 | CH | |
| Q-53($R_{15}$=$CH_3$) | 1 | H | H | $OCH_3$ | 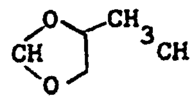 | CH | |
| Q-55($R_{12}$=H) | 1 | H | H | $OCH_3$ | $OCF_2H$ | CH | |
| Q-59($R_{12}$=H) | 1 | H | H | $OCH_3$ | $SCF_2H$ | CH | |
| Q-53($R_{15}$=$SCH_3$) | 1 | H | H | $OCH_3$ | 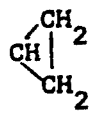 | N | |
| Q-46($R_{12}$,$R_{13}$=H) | 1 | H | H | $OCH_3$ | 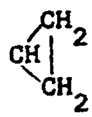 | CH | |
| Q-46($R_{12}$,$R_{13}$=H) | 1 | H | H | $OCH_3$ | 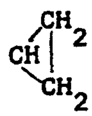 | CH | |
| Q-80($R_{19}$,$R_{20}$=$OCH_3$) | 1 | H | H | $OCH_3$ | 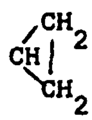 | CH | |
| Q84($R_{12}$,$R_{13}$=H) | 1 | H | H | $OCH_3$ | 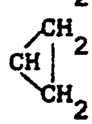 | CH | |
| Q-46($R_{12}$,$R_{13}$=H) | 2 | H | H | $OCH_3$ | $OCH_3$ | CH | |
| Q-50($R_{12}$,$R_{13}$=H) | 2 | H | H | $OCH_3$ | $OCH_3$ | CH | |
| Q-53($R_{15}$=H) | 2 | H | H | $OCH_3$ | $OCH_3$ | CH | |
| Q-59($R_{12}$=H) | 2 | H | H | $OCH_3$ | $OCH_3$ | CH | |

## Table 17a Continued

| Q | n | R | $E_1$ | $X_4$ | $Y_4$ | Z | m.p.°C |
|---|---|---|---|---|---|---|---|
| Q-61($R_{12}$,$R_{13}$=H) | 2 | H | H | $OCH_3$ | $OCH_3$ | CH | |
| Q-46($R_{12}$,$R_{13}$=H) | 2 | H | H | $OCH_3$ | $OCH_3$ | N | |
| Q-55($R_{12}$=H) | 2 | H | H | $OCH_3$ | $OCH_3$ | N | |
| Q-57($R_{16}$=H) | 2 | H | H | $CH_3$ | $OCH_3$ | N | |
| Q-63($R_{12}$=H,$R_{13}$=$CH_3$) | 2 | H | H | $OCH_3$ | $OCH_3$ | N | |
| Q-69($R_{12}$,$R_{13}$,$R_{14}$=H) | 2 | H | H | $OCH_3$ | $OCH_3$ | N | |
| Q-72($R_{12}$,$R_{13}$=H) | 2 | H | H | $OCH_3$ | $CH_3$ | N | |
| Q-78($R_{12}$,$R_{13}$=H) | 2 | H | H | $OCH_3$ | $OCH_3$ | N | |
| Q-82($R_{12}$,$R_{13}$=H) | 2 | H | H | $OCH_3$ | $OCH_3$ | CH | |
| Q-46($R_{12}$,$R_{13}$=H) | 2 | H | H | F | $OCH_3$ | CH | |
| Q-53($R_{15}$=$SCH_3$) | 2 | H | H | $OCF_2H$ | $OCH_3$ | CH | |
| Q-62($R_{12}$=H,$R_{13}$=$CH_3$) | 2 | H | H | $OCH_2CH_2F$ | $CH_3$ | CH | |
| Q-47($R_{12}$,$R_{13}$=H) | 2 | H | H | $OCH_2CH_3$ | $OCH_3$ | N | |
| Q-53($R_{15}$=$SCH_3$) | 2 | H | H | $OCH_2CF_3$ | $OCH_3$ | N | |
| Q-53($R_{15}$=$CH_3$) | 2 | H | H | $OC_2H_5$ | $NHCH_3$ | N | |
| Q-53($R_{15}$=$SCH_3$) | 2 | H | H | $OCH_3$ | 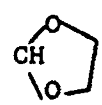 | CH | |
| Q-63($R_{12}$=H,$R_{13}$=$CH_3$) | 2 | H | H | $CH_3$ | 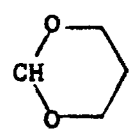 | CH | |
| Q-53($R_{15}$=$CH_3$) | 2 | H | H | $OCH_3$ | 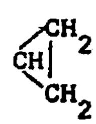 | N | |
| Q-46($R_{12}$,$R_{13}$=H) | 2 | H | H | $OCH_3$ | 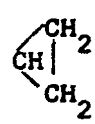 | CH | |
| Q-55($R_{12}$=$CH_3$) | 0 | H | H | $OCH_3$ | $OCH_3$ | N | 185-188 |

## Table 17b

## General Structure 10b

| Q | n | R | $E_1$ | $X_1$ | $Y_1$ | m.p. °C |
|---|---|---|---|---|---|---|
| Q-46($R_{12}$,$R_{13}$=H) | 0 | H | H | $CH_3$ | O | |
| Q-47($R_{12}$,$R_{13}$=H) | 0 | H | H | $CH_3$ | O | |
| Q-48($R_{12}$,$R_{13}$=H) | 0 | H | H | $CH_3$ | O | |
| Q-49($R_{12}$,$R_{13}$=H) | 0 | H | H | $CH_3$ | O | |
| Q-52($R_{12}$,$R_{13}$,$R_{14}$=H) | 0 | H | H | $CH_3$ | O | |
| Q-53($R_{15}$=$SCH_3$) | 0 | H | H | $CH_3$ | O | |
| Q-53($R_{15}$=$CH_3$) | 0 | H | H | $CH_3$ | O | |
| Q-53($R_{15}$=H) | 0 | H | H | $CH_3$ | O | |
| Q-54($R_{12}$=$CH_3$) | 0 | H | H | $CH_3$ | O | |
| Q-55($R_{12}$=$CH_3$) | 0 | H | H | $CH_3$ | O | |
| Q-59($R_{12}$=H) | 0 | H | H | $CH_3$ | O | |
| Q-61($R_{12}$,$R_{13}$=H) | 0 | H | H | $CH_3$ | O | |
| Q-62($R_{12}$,$R_{13}$=H) | 0 | H | H | $CH_3$ | O | |
| Q-63($R_{12}$,$R_{13}$=H) | 0 | H | H | $CH_3$ | O | |
| Q-66($R_{12}$,$R_{13}$,$R_{14}$=H) | 0 | H | H | $CH_3$ | O | |
| Q-67($R_{12}$,$R_{13}$,$R_{14}$=H) | 0 | H | H | $CH_3$ | O | |
| Q-69($R_{12}$,$R_{13}$,$R_{14}$=H) | 0 | H | H | $CH_3$ | O | |
| Q-72($R_{12}$,$R_{13}$=H,n'=0) | 0 | H | H | $CH_3$ | O | |
| Q-75($R_{12}$,$R_{17}$,$R_{18}$=H) | 0 | H | H | $CH_3$ | O | |
| Q-78($R_{12}$,$R_{13}$=H) | 0 | H | H | $CH_3$ | O | |
| Q-53($R_{15}$=$SCH_3$) | 0 | H | H | $OCH_3$ | O | |
| Q-46($R_{12}$,$R_{13}$=H) | 0 | H | H | $OC_2H_5$ | O | |
| Q-54($R_{12}$=$CH_3$) | 0 | H | H | $OCF_2H$ | O | |
| Q-53($R_{15}$=$CH_3$) | 0 | H | H | $CH_3$ | $CH_2$ | |
| Q-53($R_{15}$=$SCH_3$) | 0 | H | H | $OCH_3$ | $CH_2$ | |

Table 17b Continued

| Q | n | R | $E_1$ | $X_1$ | $Y_1$ | m.p.°C |
|---|---|---|---|---|---|---|
| Q-46($R_{12}$,$R_{13}$=H) | 1 | H | H | $CH_3$ | O | |
| Q-47($R_{12}$,$R_{13}$=H) | 1 | H | H | $CH_3$ | O | |
| Q-53($R_{15}$=$SCH_3$) | 1 | H | H | $CH_3$ | O | |
| Q-53($R_{15}$=$CH_3$) | 1 | H | H | $CH_3$ | O | |
| Q-59($R_{12}$=H) | 1 | H | H | $CH_3$ | O | |
| Q-61($R_{12}$,$R_{13}$=H) | 1 | H | H | $CH_3$ | O | |
| Q-62($R_{12}$,$R_{13}$=H) | 1 | H | H | $CH_3$ | O | |
| Q-63($R_{12}$,$R_{13}$=H) | 1 | H | H | $CH_3$ | O | |
| Q-78($R_{12}$,$R_{13}$=H) | 1 | H | H | $CH_3$ | O | |
| Q-46($R_{12}$,$R_{13}$=H) | 1 | H | H | $OC_2H_5$ | O | |
| Q-53($R_{15}$=$CH_3$) | 1 | H | H | $OCF_2H$ | O | |
| Q-53($R_{15}$=$SCH_3$) | 1 | H | H | $OCH_3$ | $CH_2$ | |
| Q-53($R_{15}$=$CH_3$) | 2 | H | H | $CH_3$ | O | |
| Q-59($R_{12}$=H) | 2 | H | H | $CH_3$ | O | |
| Q-53($R_{15}$=$SCH_3$) | 2 | H | H | $OC_2H_5$ | O | |
| Q-78($R_{12}$,$R_{13}$=H) | 2 | H | H | $CH_3$ | O | |

Table 17c

General Structure 10c

| Q | n | R | $E_1$ | $X_1$ | m.p.°C |
|---|---|---|---|---|---|
| Q-46($R_{12}$,$R_{13}$=H) | 0 | H | H | $CH_3$ | |
| Q-47($R_{12}$,$R_{13}$=H) | 0 | H | H | $OCH_3$ | |
| Q-48($R_{12}$,$R_{13}$=H) | 0 | H | H | $OC_2H_5$ | |
| Q-49($R_{12}$,$R_{13}$=H) | 0 | H | H | $CH_3$ | |
| Q-52($R_{12}$,$R_{13}$,$R_{14}$=H) | 0 | H | H | $CH_3$ | |
| Q-53($R_{15}$=$SCH_3$) | 0 | H | H | $OCH_3$ | |
| Q-53($R_{15}$=$CH_3$) | 0 | H | H | $OCH_3$ | |
| Q-53($R_{15}$=H) | 0 | H | H | $CH_3$ | |
| Q-54($R_{12}$=$CH_3$) | 0 | H | H | $OCF_2H$ | |
| Q-55($R_{12}$=$CH_3$) | 0 | H | H | $CH_3$ | |
| Q-59($R_{12}$=H) | 0 | H | H | $OCH_3$ | |
| Q-61($R_{12}$=H,$R_{13}$=$CH_3$) | 0 | H | H | $CH_3$ | |
| Q-62($R_{12}$=H,$R_{13}$=$CH_3$) | 0 | H | H | $OCF_2H$ | |
| Q-63($R_{12}$=H,$R_{13}$=$CH_3$) | 0 | H | H | $OCH_3$ | |
| Q-66($R_{12}$,$R_{13}$,$R_{14}$=H) | 0 | H | H | $OCH_3$ | |
| Q-67($R_{12}$,$R_{13}$,$R_{14}$=H) | 0 | H | H | $CH_3$ | |
| Q-69($R_{12}$,$R_{13}$,$R_{14}$=H) | 0 | H | H | $CH_3$ | |
| Q-72($R_{12}$,$R_{13}$=H,n'=0) | 0 | H | H | $OCH_3$ | |
| Q-75($R_{12}$,$R_{17}$,$R_{18}$ =H) | 0 | H | H | $OCH_3$ | |
| Q-78($R_{12}$,$R_{13}$=H) | 0 | H | H | $OCH_3$ | |
| Q-46($R_{12}$,$R_{13}$=H) | 1 | H | H | $CH_3$ | |
| Q-47($R_{12}$,$R_{13}$=H) | 1 | H | H | $OCH_3$ | |
| Q-53($R_{15}$=$CH_3$) | 1 | H | H | $OCH_3$ | |
| Q-53($R_{15}$=$SCH_3$) | 1 | H | H | $CH_3$ | |
| Q-55($R_{12}$=$CH_3$) | 1 | H | H | $OC_2H_5$ | |

Table 17c Continued

| Q | n | R | $E_1$ | $X_1$ | m.p.°C |
|---|---|---|---|---|---|
| Q-59($R_{12}$=H) | 1 | H | H | $OCH_3$ | |
| Q-62($R_{12}$=H,$R_{13}$=$CH_3$) | 1 | H | H | $OCF_2H$ | |
| Q-66($R_{12}$=H,$R_{13}$=$CH_3$) | 1 | H | H | $OCH_3$ | |
| Q-78($R_{12}$,$R_{13}$=H) | 1 | H | H | $OCH_3$ | |
| Q-53($R_{15}$=$CH_3$) | 2 | H | H | $CH_3$ | |
| Q-72($R_{12}$,$R_{13}$=H,n'=0) | 2 | H | H | $OCH_3$ | |
| Q-73($R_{12}$,$R_{13}$=H,n'=1) | 2 | H | H | $OCH_3$ | |
| Q-46($R_{12}$,$R_{13}$=H) | 2 | H | H | $CH_3$ | |
| Q-49($R_{12}$,$R_{13}$=H) | 2 | H | H | $OC_2H_5$ | |

Table 17d

General Structure 10d

| Q | n | R | $E_1$ | $X_1$ | $Y_2$ | m.p.°C |
|---|---|---|---|---|---|---|
| Q-46($R_{12}$,$R_{13}$=H) | 0 | H | H | $CH_3$ | $CH_3$ | |
| Q-47($R_{12}$,$R_{13}$=H) | 0 | H | H | $OCH_3$ | $CH_3$ | |
| Q-48($R_{12}$,$R_{13}$=H) | 0 | H | H | $OC_2H_5$ | H | |
| Q-49($R_{12}$,$R_{13}$=H) | 0 | H | H | $CH_3$ | $CH_3$ | |
| Q-52($R_{12}$,$R_{13}$,$R_{14}$=H) | 0 | H | H | $CH_3$ | $CH_3$ | |
| Q-53($R_{15}$=$SCH_3$) | 0 | H | H | $CH_3$ | $CH_3$ | |
| Q-53($R_{15}$=$CH_3$) | 0 | H | H | $CH_3$ | $CH_3$ | |
| Q-53($R_{15}$=H) | 0 | H | H | $CH_3$ | $CH_3$ | |
| Q-54($R_{12}$=$CH_3$) | 0 | H | H | $OCF_2H$ | $CH_3$ | |
| Q-55($R_{12}$=$CH_3$) | 0 | H | H | $CH_3$ | $CH_3$ | |
| Q-59($R_{12}$=H) | 0 | H | H | $CH_3$ | $CH_3$ | |
| Q-61($R_{12}$,$R_{13}$=H) | 0 | H | H | $CH_3$ | $CH_3$ | |
| Q-62($R_{12}$,$R_{13}$=H) | 0 | H | H | $OCF_2H$ | $CH_3$ | |
| Q-63($R_{12}$,$R_{13}$=H) | 0 | H | H | $OC_2H_5$ | $CH_3$ | |
| Q-66($R_{12}$,$R_{13}$,$R_{14}$=H) | 0 | H | H | $CH_3$ | $CH_3$ | |
| Q-67($R_{12}$,$R_{13}$,$R_{14}$=H) | 0 | H | H | $OCH_3$ | H | |
| Q-69($R_{12}$,$R_{13}$,$R_{14}$=H) | 0 | H | H | $OCF_2H$ | $CH_3$ | |
| Q-72($R_{12}$,$R_{13}$=H,n'=0) | 0 | H | H | $CH_3$ | $CH_3$ | |
| Q-75($R_{12}$,$R_{17}$,$R_{18}$=H) | 0 | H | H | $CH_3$ | $CH_3$ | |
| Q-78($R_{12}$,$R_{13}$=H) | 0 | H | H | $CH_3$ | $CH_3$ | |
| Q-46($R_{12}$,$R_{13}$=H) | 1 | H | H | $CH_3$ | $CH_3$ | |
| Q-47($R_{12}$,$R_{13}$=H) | 1 | H | H | $OCH_3$ | $CH_3$ | |
| Q-48($R_{12}$,$R_{13}$=H) | 1 | H | H | $OC_2H_5$ | H | |
| Q-53($R_{15}$=$SCH_3$) | 1 | H | H | $CH_3$ | $CH_3$ | |
| Q-53($R_{15}$=$CH_3$) | 1 | H | H | $CH_3$ | $CH_3$ | |
| Q-59($R_{12}$=H) | 1 | H | H | $CH_3$ | $CH_3$ | |

Table 17d Continued

| Q | n | R | $E_1$ | $X_1$ | $Y_2$ | m.p.°C |
|---|---|---|---|---|---|---|
| Q-61($R_{12}$=H,$R_{13}$=$CH_3$) | 1 | H | H | $CH_3$ | $CH_3$ | |
| Q-63($R_{12}$=H,$R_{13}$=$CH_3$) | 1 | H | H | $OC_2H_5$ | $CH_3$ | |
| Q-66($R_{12}$,$R_{13}$,$R_{14}$=H) | 1 | H | H | $CH_3$ | $CH_3$ | |
| Q-72($R_{12}$,$R_{13}$=H,n'=0) | 1 | H | H | $CH_3$ | $CH_3$ | |
| Q-78($R_{12}$,$R_{13}$=H) | 1 | H | H | $CH_3$ | $CH_3$ | |
| Q-83($R_{12}$,$R_{13}$=H | 1 | H | H | $CH_3$ | $CH_3$ | |
| Q-46($R_{12}$,$R_{13}$=H) | 2 | H | H | $CH_3$ | $CH_3$ | |
| Q-53($R_{15}$=$SCH_3$) | 2 | H | H | $CH_3$ | $CH_3$ | |
| Q-53($R_{15}$=$CH_3$) | 2 | H | H | $CH_3$ | $CH_3$ | |
| Q-59($R_{12}$=H) | 2 | H | H | $OCH_3$ | $CH_3$ | |
| Q-78($R_{12}$,$R_{13}$=H) | 2 | H | H | $CH_3$ | $CH_3$ | |

## Table 17e

## General Structure 10e

| Q | n | R | $E_1$ | $X_2$ | $Y_3$ | m.p.°C |
|---|---|---|---|---|---|---|
| Q-46($R_{12}$,$R_{13}$=H) | 0 | H | H | $CH_3$ | $CH_3$ | |
| Q-47($R_{12}$,$R_{13}$=H) | 0 | H | H | $OCH_3$ | $CH_3$ | |
| Q-48($R_{12}$,$R_{13}$=H) | 0 | H | H | $OCH_3$ | $C_2H_5$ | |
| Q-49($R_{12}$,$R_{13}$=H) | 0 | H | H | $CH_3$ | $CH_3$ | |
| Q-52($R_{12}$,$R_{13}$,$R_{14}$=H) | 0 | H | H | $CH_3$ | $CH_3$ | |
| Q-53($R_{15}$=$SCH_3$) | 0 | H | H | $CH_3$ | $CH_2CF_3$ | |
| Q-53($R_{15}$=$CH_3$) | 0 | H | H | $CH_3$ | $CH_2CF_3$ | |
| Q-53($R_{15}$=H) | 0 | H | H | $CH_3$ | $CH_3$ | |
| Q-54($R_{12}$=$CH_3$) | 0 | H | H | $OCH_3$ | $CH_3$ | |
| Q-55($R_{12}$=$CH_3$) | 0 | H | H | $CH_3$ | $CH_3$ | |
| Q-59($R_{12}$=H) | 0 | H | H | $CH_3$ | $C_2H_5$ | |
| Q-61($R_{12}$=H,$R_{13}$=$CH_3$) | 0 | H | H | $CH_3$ | $CH_3$ | |
| Q-62($R_{12}$=H,$R_{13}$=$CH_3$) | 0 | H | H | $OCH_3$ | $CH_2CF_3$ | |
| Q-63($R_{12}$=H,$R_{13}$=$CH_3$) | 0 | H | H | $OCH_3$ | $CH_3$ | |
| Q-66($R_{12}$,$R_{13}$,$R_{14}$=H) | 0 | H | H | $CH_3$ | $CH_3$ | |
| Q-67($R_{12}$,$R_{13}$,$R_{14}$=H) | 0 | H | H | $OCH_3$ | $C_2H_5$ | |
| Q-69($R_{12}$,$R_{13}$,$R_{14}$=H) | 0 | H | H | $OCH_3$ | $CH_3$ | |
| Q-72($R_{12}$,$R_{13}$=H,n'=0) | 0 | H | H | $CH_3$ | $CH_3$ | |
| Q-75($R_{12}$,$R_{17}$,$R_{18}$=H) | 0 | H | H | $CH_3$ | $CH_3$ | |
| Q-78($R_{12}$,$R_{13}$=H) | 0 | H | H | $CH_3$ | $CH_3$ | |
| Q-46($R_{12}$,$R_{13}$=H) | 1 | H | H | $CH_3$ | $CH_3$ | |
| Q-47($R_{12}$,$R_{13}$=H) | 1 | H | H | $OCH_3$ | $CH_3$ | |
| Q-53($R_{15}$=$CH_3$) | 1 | H | H | $CH_3$ | $C_2H_5$ | |
| Q-53($R_{15}$=$SCH_3$) | 1 | H | H | $CH_3$ | $CH_2CF_3$ | |
| Q-54($R_{12}$=$CH_3$) | 1 | H | H | $OCH_3$ | $CH_3$ | |
| Q-55($R_{12}$=$CH_3$) | 1 | H | H | $CH_3$ | $CH_3$ | |
| Q-59($R_{12}$=H) | 1 | H | H | $CH_3$ | $C_2H_5$ | |

Table 17e Continued

| Q | n | R | $E_1$ | $X_2$ | $Y_3$ | m.p.°C |
|---|---|---|---|---|---|---|
| Q-61($R_{12}$=H,$R_{13}$=$CH_3$) | 1 | H | H | $CH_3$ | $CH_3$ | |
| Q-72($R_{12}$,$R_{13}$=H) | 1 | H | H | $CH_3$ | $CH_3$ | |
| Q-78($R_{12}$,$R_{13}$=H) | 1 | H | H | $CH_3$ | $CH_3$ | |
| Q-46($R_{12}$,$R_{13}$=H) | 2 | H | H | $CH_3$ | $CH_3$ | |
| Q-53($R_{15}$=$CH_3$) | 2 | H | H | $OCH_3$ | $CH_3$ | |
| Q-59($R_{12}$=H) | 2 | H | H | $CH_3$ | $CH_3$ | |
| Q-63($R_{12}$=H,$R_{13}$=$CH_3$) | 2 | H | H | $OCH_3$ | $CH_2CF_3$ | |

Table 17f

General Structure 10f

| Q | n | R | $E_1$ | $X_3$ | m.p.°C |
|---|---|---|---|---|---|
| Q-46($R_{12}$,$R_{13}$=H) | 0 | H | H | $CH_3$ | |
| Q-47($R_{12}$,$R_{13}$=H) | 0 | H | H | $CH_3$ | |
| Q-48($R_{12}$,$R_{13}$=H) | 0 | H | H | $CH_3$ | |
| Q-49($R_{12}$,$R_{13}$=H) | 0 | H | H | $OCH_3$ | |
| Q-52($R_{12}$,$R_{13}$,$R_{14}$=H) | 0 | H | H | $OCH_3$ | |
| Q-53($R_{15}$=$CH_3$) | 0 | H | H | $CH_3$ | |
| Q-53($R_{15}$=$SCH_3$) | 0 | H | H | $OCH_3$ | |
| Q-53($R_{15}$=H) | 0 | H | H | $CH_3$ | |
| Q-54($R_{12}$=$CH_3$) | 0 | H | H | $CH_3$ | |
| Q-55($R_{12}$=$CH_3$) | 0 | H | H | $OCH_3$ | |
| Q-59($R_{12}$=H) | 0 | H | H | $OCH_3$ | |
| Q-61($R_{12}$=H,$R_{13}$=$CH_3$) | 0 | H | H | $CH_3$ | |
| Q-62($R_{12}$=H,$R_{13}$=$CH_3$) | 0 | H | H | $CH_3$ | |
| Q-63($R_{12}$=H,$R_{13}$=$CH_3$) | 0 | H | H | $OCH_3$ | |
| Q-66($R_{12}$,$R_{13}$,$R_{14}$=H) | 0 | H | H | $CH_3$ | |
| Q-67($R_{12}$,$R_{13}$,$R_{14}$=H) | 0 | H | H | $OCH_3$ | |
| Q-69($R_{12}$,$R_{13}$,$R_{14}$=H) | 0 | H | H | $CH_3$ | |
| Q-72($R_{12}$,$R_{13}$=H) | 0 | H | H | $CH_3$ | |
| Q-75($R_{12}$,$R_{17}$,$R_{18}$=H) | 0 | H | H | $OCH_3$ | |
| Q-78($R_{12}$,$R_{13}$=H) | 0 | H | H | $CH_3$ | |
| Q-83($R_{12}$,$R_{13}$=H) | 0 | H | H | $CH_3$ | |
| Q-46($R_{12}$,$R_{13}$=H) | 1 | H | H | $OCH_3$ | |
| Q-47($R_{12}$,$R_{13}$=H) | 1 | H | H | $CH_3$ | |
| Q-53($R_{15}$=$CH_3$) | 1 | H | H | $CH_3$ | |
| Q-53($R_{15}$=$SCH_3$) | 1 | H | H | $OCH_3$ | |

Table 17f Continued

| Q | n | R | $E_1$ | $X_3$ | m.p.°C |
|---|---|---|---|---|---|
| Q-55($R_{12}$=$CH_3$) | 1 | H | H | $CH_3$ | |
| Q-59($R_{12}$=H) | 1 | H | H | $CH_3$ | |
| Q-61($R_{12}$=H,$R_{13}$=$CH_3$) | 1 | H | H | $OCH_3$ | |
| Q-63($R_{12}$=H,$R_{13}$=$CH_3$) | 1 | H | H | $OCH_3$ | |
| Q-72($R_{12}$,$R_{13}$=H,n'=0) | 1 | H | H | $CH_3$ | |
| Q-83($R_{12}$,$R_{13}$=H) | 1 | H | H | $OCH_3$ | |
| Q-46($R_{12}$,$R_{13}$=H) | 2 | H | H | $OCH_3$ | |
| Q-53($R_{15}$=$CH_3$) | 2 | H | H | $OCH_3$ | |

Table 17g

General Structure 10g

| Q | n | R | $E_1$ | X | Y | Z | m.p.°C |
|---|---|---|---|---|---|---|---|
| Q-46($R_{12}$,$R_{13}$=H) | 0 | H | H | $OCH_3$ | $OCH_3$ | CH | |
| Q-46($R_{12}$,$R_{13}$=H) | 1 | H | H | $OCH_3$ | $OCH_3$ | CH | |
| Q-47($R_{12}$,$R_{13}$=H) | 0 | H | H | $OCH_3$ | $CH_3$ | CH | |
| Q-48($R_{12}$,$R_{13}$=H) | 0 | H | H | $OCH_3$ | $OCH_3$ | CH | |
| Q-49($R_{12}$,$R_{13}$=H) | 0 | H | H | $OCH_3$ | $OCH_3$ | CH | |
| Q-46($R_{12}$,$R_{13}$=H) | 0 | H | H | $CH_3$ | $OCH_3$ | N | |
| Q-53($R_{15}$=$CH_3$) | 0 | H | H | $OCH_3$ | $CH_3$ | CH | |
| Q-54($R_{12}$=$CH_3$) | 0 | H | H | $OCH_3$ | $OCH_3$ | CH | |
| Q-55($R_{12}$=$CH_3$) | 0 | H | H | $CH_3$ | $OCH_3$ | CH | |
| Q-59($R_{12}$=H) | 0 | H | H | $OCH_3$ | $OCH_3$ | CH | |
| Q-61($R_{12}$,$R_{13}$=H) | 0 | H | H | $OCH_3$ | $OCH_3$ | CH | |
| Q-62($R_{12}$=H,$R_{13}$=$CH_3$) | 0 | H | H | $OCH_3$ | $OCH_3$ | CH | |
| Q-66($R_{12}$,$R_{13}$,$R_{14}$=H) | 0 | H | H | $OCH_3$ | $OCH_3$ | CH | |
| Q-67($R_{12}$,$R_{13}$,$R_{14}$=H) | 0 | H | H | $OCH_3$ | $OCH_3$ | CH | |
| Q-69($R_{12}$,$R_{13}$,$R_{14}$=H) | 0 | H | H | $OCH_3$ | $OCH_3$ | CH | |
| Q-78($R_{12}$,$R_{13}$=H) | 0 | H | H | $OCH_3$ | $OCH_3$ | CH | |
| Q-53($R_{15}$=$CH_3$) | 1 | H | H | $OCH_3$ | $OCH_3$ | CH | |
| Q-53($R_{15}$=$CH_3$) | 1 | H | H | $OCH_3$ | $CH_3$ | N | |
| Q-53($R_{15}$=$CH_3$) | 0 | H | H | $CH_3$ | $OCH_3$ | N | |
| Q-53($R_{15}$=$CH_3$) | 2 | H | H | $OCH_3$ | $OCH_3$ | CH | |

## Formulations

Useful formulations of the compounds of Formula I can be prepared in conventional ways. They include dusts, granules, pellets, solutions, suspensions, emulsions, wettable powders, emulsifiable concentrates and

the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions: Table 18

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

In the following examples, all parts are by weight unless otherwise indicated.

Example 12

Wettable Powder

| | |
|---|---|
| N-[(4,6-dimethylpyrimidin-2-yl)aminocarbonyl]-3-(isoxazol-3-yl)-2-thiophenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 13

Wettable Powder

| | |
|---|---|
| 3-(isoxazol-3-yl)-N-[(4-methoxy-6-methylpyrimidin-2-yl)-aminocarbonyl]-2-thiophenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 14

Granule

| | |
|---|---|
| Wettable Powder of Example 13 | 5% |
| attapulgite granules (U.S.S. 20-40 mesh; 0.84-0.42 mm) | 95% |

A slurry of wettable powder containing 25% solids is sprayed on the surface of attapulgite granules in a double-cone blender. The granules are dried and packaged.

Example 15

Extruded Pellet

| | |
|---|---|
| N-[(4,6-dimethoxypyrimidin-2-yl)aminocarbonyl]-3-(isoxazol-3-yl)-2-thiophenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled. Example 16

Low Strength Granule

| | |
|---|---|
| N-[(4,6-dimethylpyrimidin-2-yl)aminocarbonyl]-3-(isoxazol-3-yl)-2-thiophenesulfonamide | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 17

Granule

| | |
|---|---|
| 3-(isoxazol-3-yl)-N-[(4-methoxy-6-methylpyrimidin-2-yl)-aminocarbonyl]-2-thiophenesulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 18

Low Strength Granule

| | |
|---|---|
| N-[(4,6-dimethoxypyrimidin-2-yl)aminocarbonyl]-3-(isoxazol-3-yl)-2-thiophenesulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 19

Aqueous Suspension

| | |
|---|---|
| N-[(4,6-dimethylpyrimidin-2-yl)aminocarbonyl]-3-(isoxazol-3-yl)-2-thiophenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 20

Solution

| | |
|---|---|
| 3-(isoxazol-3-yl)-N-[(4-methoxy-6-methylpyrimidin-2-yl)-aminocarbonyl]-2-thiophenesulfonamide, sodium salt | 5% |
| water | 95% |

The salt is added directly to the water with stirring to produce the solution, which may then be packaged for use.

Example 21

High Strength Concentrate

| | |
|---|---|
| N-[(4,6-dimethoxypyrimidin-2-yl)aminocarbonyl]-3-(isoxazol-3-yl)-2-thiophenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 22

Wettable Powder

| | |
|---|---|
| N-[(4,6-dimethylpyrimidin-2-yl)aminocarbonyl]-3-(isoxazol-3-yl)-2-thiophenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 23

Wettable Powder

| | |
|---|---|
| 3-(isoxazol-3-yl)-N-[(4-methoxy-6-methylpyrimidin-2-yl)-aminocarbonyl]-2-thiophenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 24

Oil Suspension

| | |
|---|---|
| N-[(4,6-dimethoxypyrimidin-2-yl)aminocarbonyl]-3-(isoxazol-3-yl)-2-thiophenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 25

Dust

| | |
|---|---|
| N-[(4,6-dimethylpyrimidin-2-yl)aminocarbonyl]-3-(isoxazol-3-yl)-2-thiophenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 26

Oil Suspension

| | |
|---|---|
| 3-(isoxazol-3-yl)-N-[(4-methoxy-6-methylpyrimidin-2-yl)-aminocarbonyl]-2-thiophenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 27

Wettable Powder

| | |
|---|---|
| N-[(4,6-dimethoxypyrimidin-2-yl)aminocarbonyl]-3-(isoxazol-3-yl)-2-thiophenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 28

Wettable Powder

| | |
|---|---|
| N-[(4-chloro-6-methoxypyrimidin-2-yl)aminocarbonyl]-3-(1H-pyrrol-1-yl)-2-thiophenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 29

Wettable Powder

| | |
|---|---|
| N-[(4,6-dimethoxy-1,3,5-triazin-2-yl)aminocarbonyl]-3-(1H-pyrrol-1-yl)-2-thiophenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 30

Extruded Pellet

| | |
|---|---|
| N-[(4,6-dimethyl-1,3,5-triazin-2-yl)aminocarbonyl]-3-(1H-pyrrol-1-yl)-2-thiophenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 31

Low Strength Granule

| | |
|---|---|
| N-[(4,6-dimethylpyrimidin-2-yl)aminocarbonyl]-3-(isoxazol-3-yl)-2-thiophenesulfonamide | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 32

Granule

| | |
|---|---|
| N-[(4,6-dimethoxypyrimidin-2-yl)aminocarbonyl]-3-(1H-pyrrol-1-yl)-2-thiophenesulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 33

Low Strength Granule

| | |
|---|---|
| N-[(4-methoxy-6-methyl-1,3,5-triazin-2-yl)aminocarbonyl]-3-(1H-pyrrol-1-yl)-2-thiophenesulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 34

Aqueous Suspension

| | |
|---|---|
| N-[(4-methoxy-6-methylpyrimidin-2-yl)aminocarbonyl]-3-(1H-pyrrol-1-yl)-2-thiophenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 35

Solution

| | |
|---|---|
| 3-(5-chloro-1H-1,2,4-triazol-1-yl)-N-[(4,6-dimethoxypyrimidin-2-yl)aminocarbonyl]-2-thiophenesulfonamide, sodium salt | 5% |
| water | 95% |

The salt is added directly to the water with stirring to produce the solution, which may then be packaged for use.

Example 36

Wettable Powder

| | |
|---|---|
| N-[(4,6-dimethoxypyrimidin-2-yl)aminocarbonyl]-2-(1H-1,2,4-triazol-1-yl)-3-pyridinesulfonamide | 80% |
| sodium alkylnaphthalenesulfonate | 2% |
| sodium ligninsulfonate | 2% |
| synthetic amorphous silica | 3% |
| kaolinite | 13% |

The ingredients are thoroughly blended and hammer-milled or air-milled to produce particles averaging below 20 microns in diameter. The product is reblended before packaging.

Example 40

Emulsifiable Concentrate

| | |
|---|---|
| N-[(4,6-dimethoxypyrimidin-2-yl)aminocarbonyl]-2-(1H-1,2,4-triazol-1-yl)-3-pyridinesulfonamide | 5.0% |
| blend of oil soluble sulfonates and polyoxyethylene ethers | 4.0% |
| N-methyl pyrrolidone | 45.5% |
| xylene | 45.5% |

The active ingredients are combined and stirred until dissolved. A fine screen filter is included in packaging operation to insure the absence of any extraneous undissolved material in the product.

Utility

Test results indicate that the compounds of the present invention are active herbicides. They have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Alternatively, many of the subject compounds should be useful for the selective pre- or post-emergence weed control in crops, especially wheat, barley and soybeans.

The rates of application for the compounds of the invention are determined by a number of factors, including their use as selective or general herbicides, the crop species involved, the types of weeds to be controlled, weather and climate, formulations selected, mode of application, amount of foliage present, etc. In general terms, the subject compounds should be applied at levels of around 0.01 to 5 kg/ha, the lower rates being suggested for use on lighter soils and/or those having a low organic matter content, for selective weed control or for situations where only short-term persistence is required.

The compounds of the invention may be used in combination with any other commercial herbicide, examples of which are those of the triazine, triazole, uracil, urea, amide, diphenylether, carbamate and bipyridylium types. The compounds may also be used in combination with mefluidide.

The herbicidal properties of the subject compounds were discovered in a number of greenhouse tests. The test procedures and results follow.

Test A

Seeds of crabgrass (Digitaria sp.), barnyardgrass (Echinochloa crusgalli), wild oats (Avena fatua), sicklepod (Cassia obtusifolia), morningglory (Ipomoea sp.), cocklebur (Xanthium sp.), sorghum, corn, soybean, cotton, sugar beet, rice, wheat and nutsedge tubers (Cyperus rotundus) were planted in a growth medium and treated pre-emergence with the chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings are based on a numerical scale extending from 0 = no injury, to 10 = complete kill. The accompanying descriptive symbols have the following meanings:

C = chlorosis or necrosis;
G = growth retardation;
H = formative effects;
U = unusual pigmentation.

The compounds are highly active herbicides. Wheat exhibits tolerance.

## Compounds

Compound 1

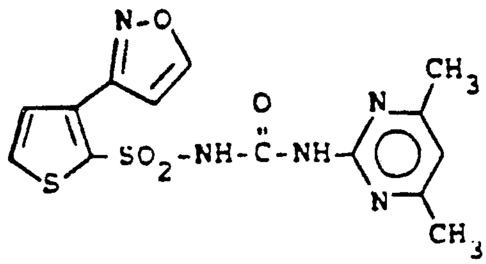

Compound 2

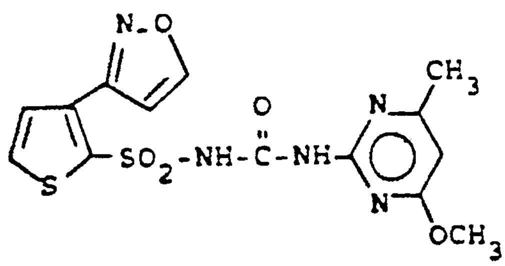

Compound 3

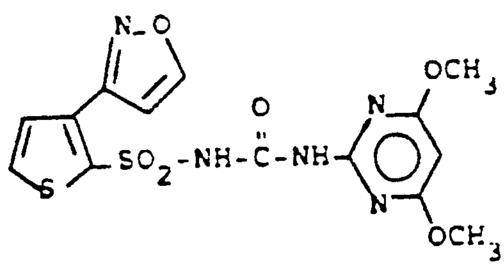

Compound 4

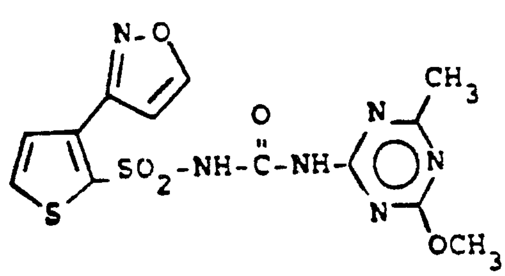

Compounds (continued)

Compound 5

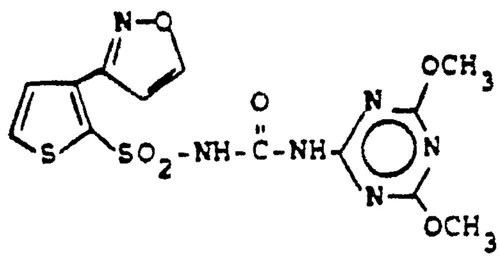

Compound 6

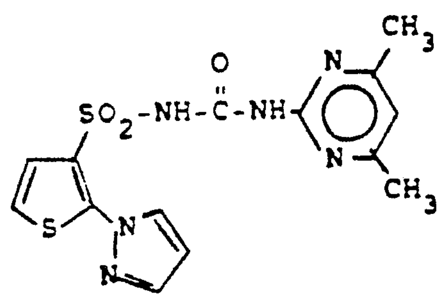

Compound 7

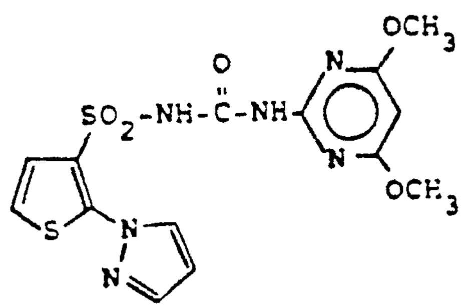

Compound 8

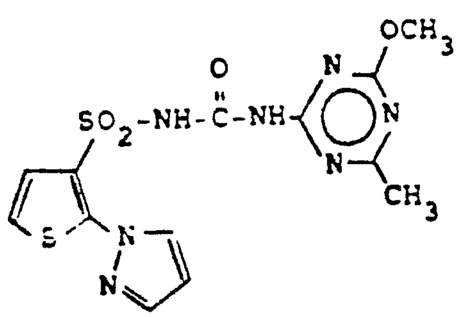

Compounds (continued)

Compound 9

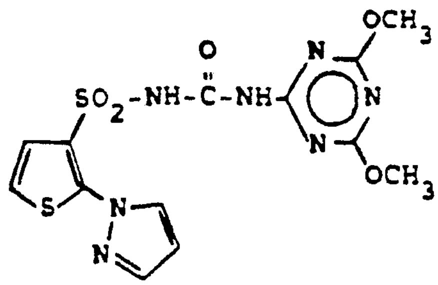

Compound 10

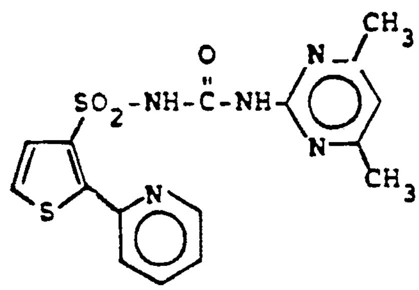

Compound 11

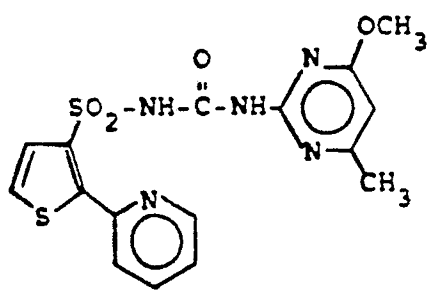

Compound 12

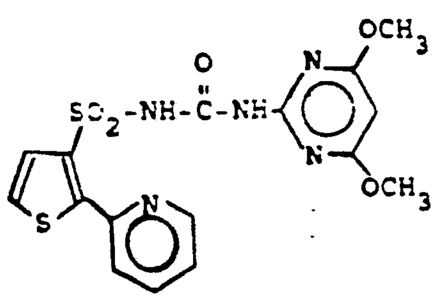

Compounds (continued)

Compound 13

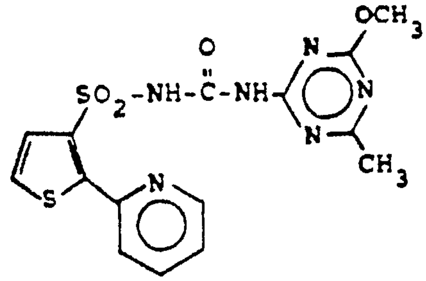

Compound 14

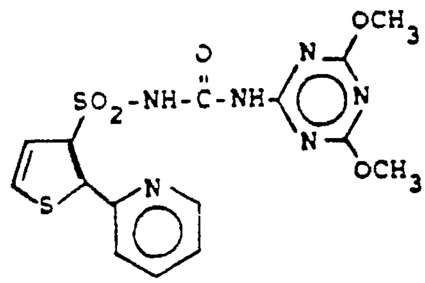

Compound 15

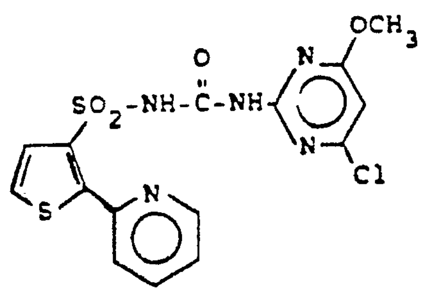

Compound 16

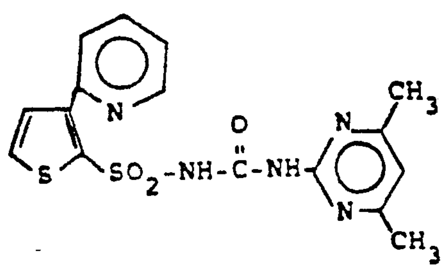

Compounds (continued)

Compound 17

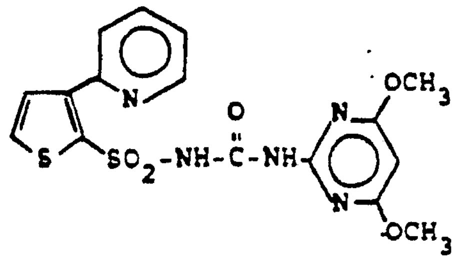

Compound 18

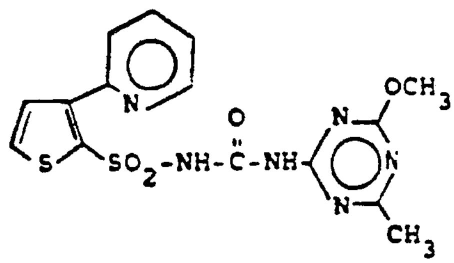

Compound 19

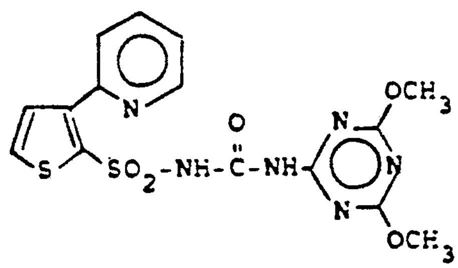

Compound 20

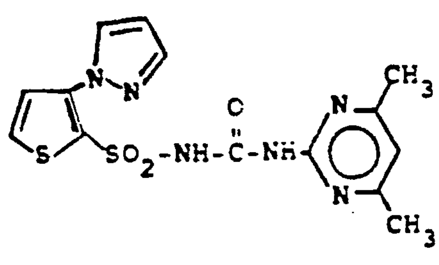

Compounds (continued)

Compound 21

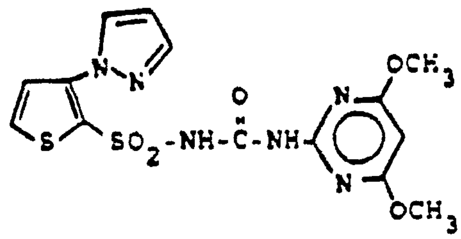

Compound 22

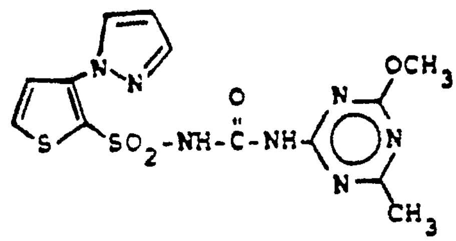

Compound 23

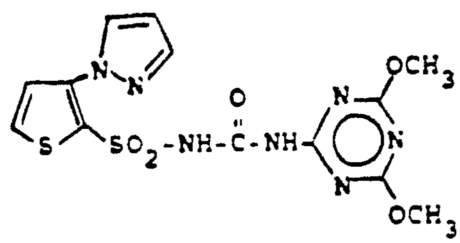

Compound 24

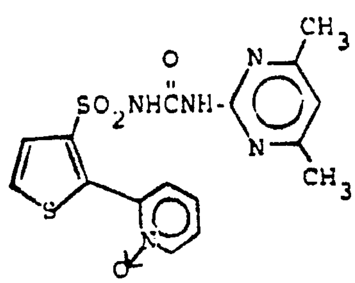

Compounds (continued)

Compound 25

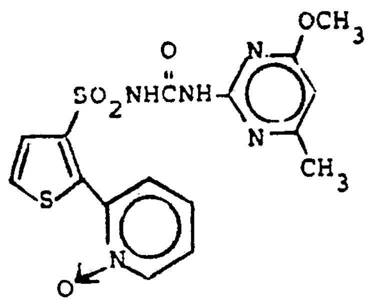

Compound 26

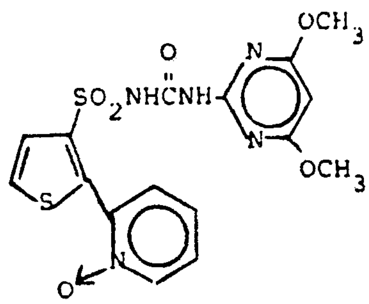

Compound 27

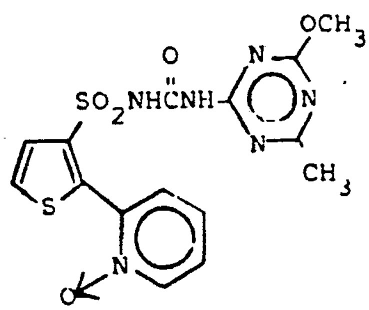

Compounds (continued)

Compound 28

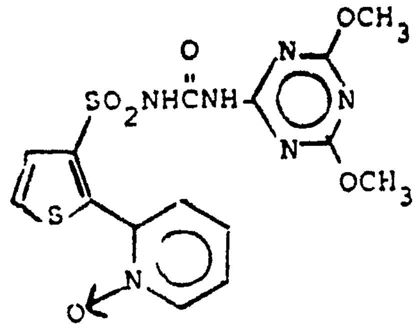

Compound 29

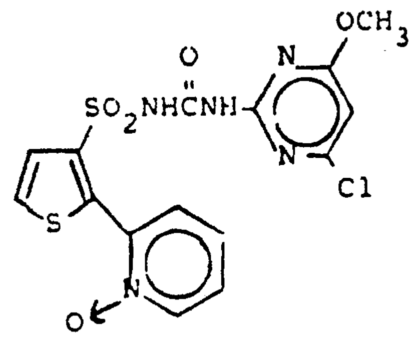

Compound 30

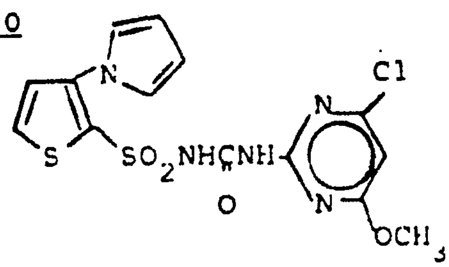

Compound 31

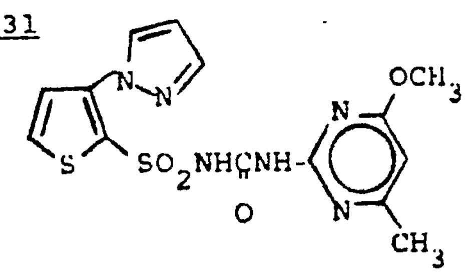

Compounds (continued)

Compound 32

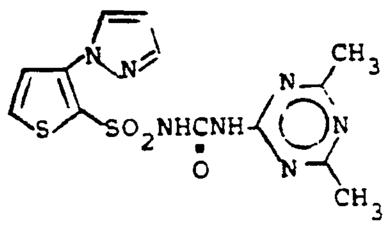

Compound 33

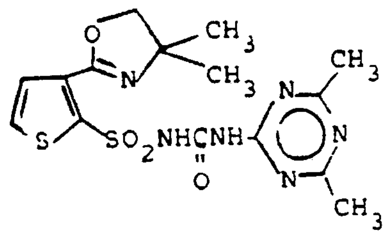

**Compounds (continued)**

Compound 34

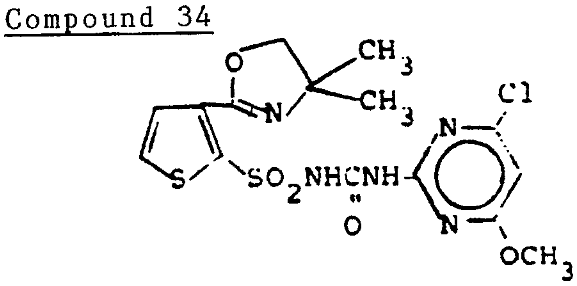

Compound 35

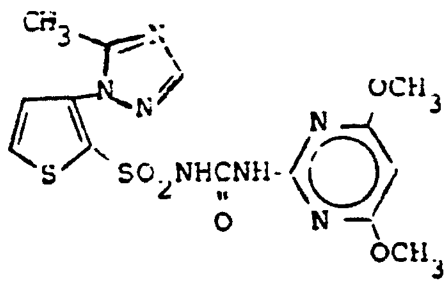

Compound 36

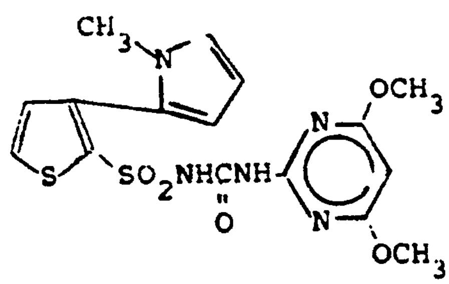

**Compounds (continued)**

Compound 37

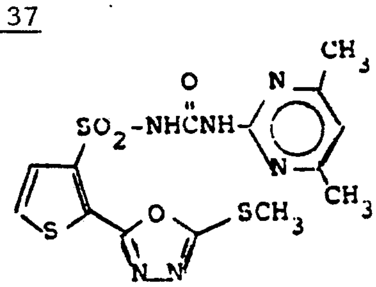

Compound 38

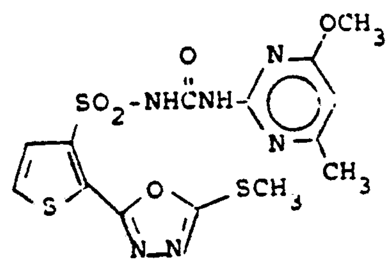

Compound 39

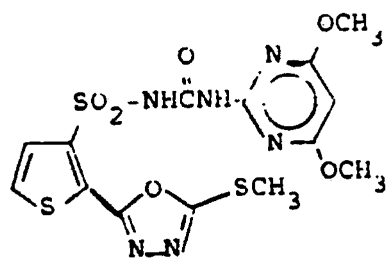

Compound 40

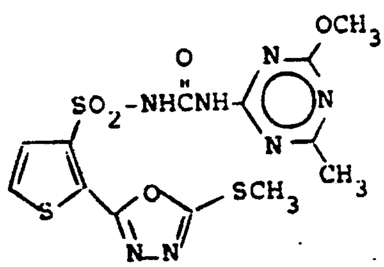

Compounds (continued)

Compound 41

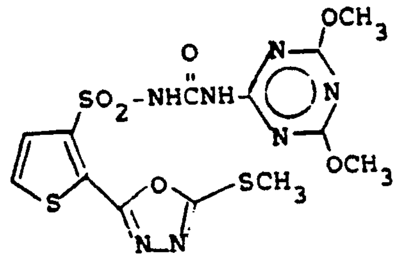

Compound 42

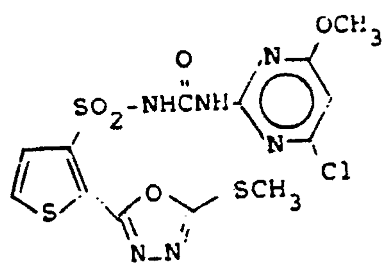

Compound 43

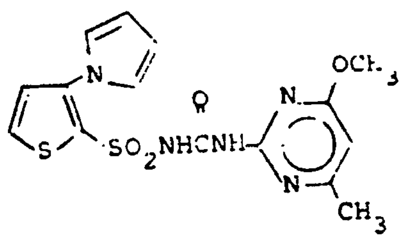

Compound 44

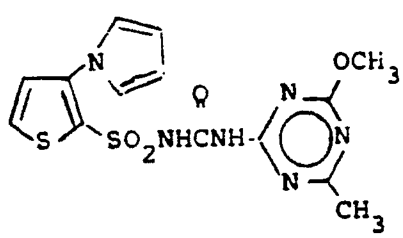

**Compounds (continued)**

Compound 45

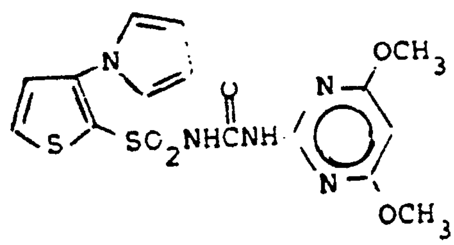

Compound 46

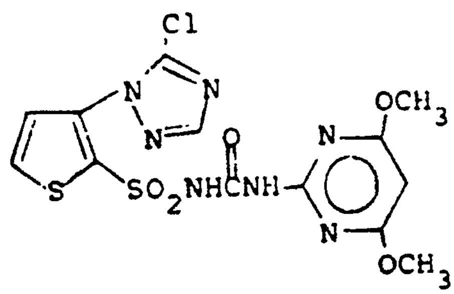

Compound 47

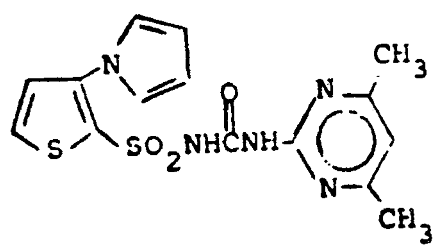

**Compounds (continued)**

Compound 48

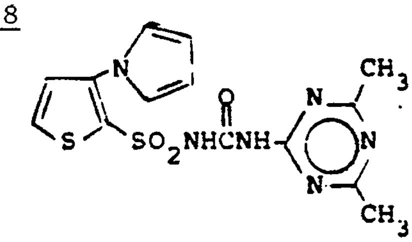

Compound 49

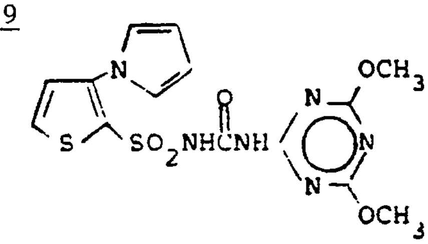

Compound 50

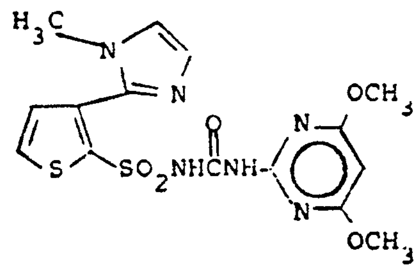

Compound 51

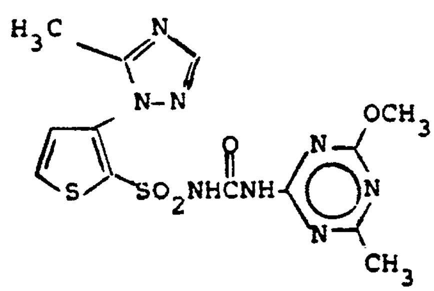

Compounds (continued)

Compound 52

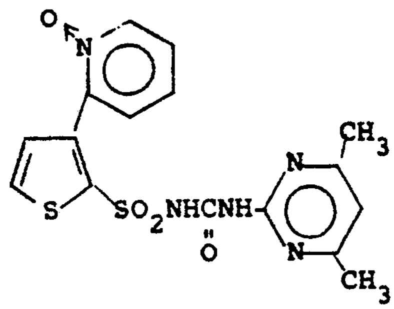

Compound 53

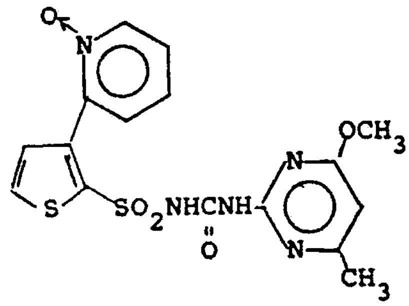

Compound 54

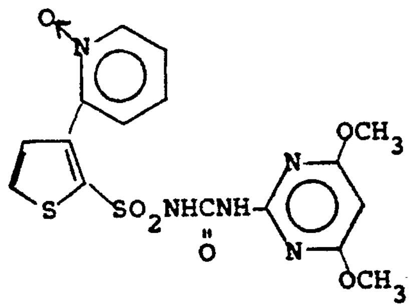

Compound 55

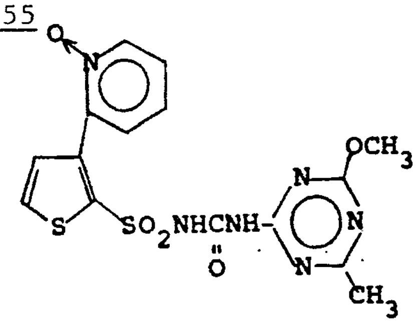

**Compounds (continued)**

Compound 56

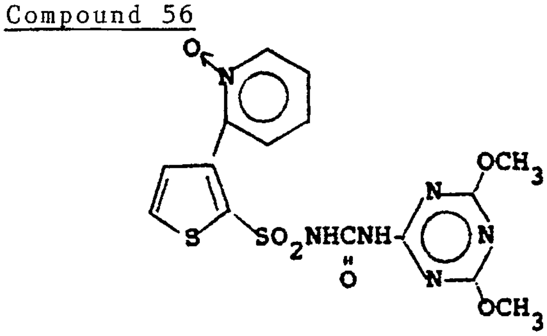

Compound 57

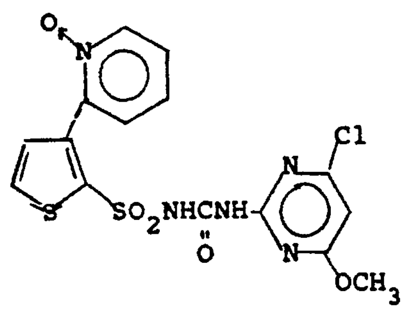

Compound 58

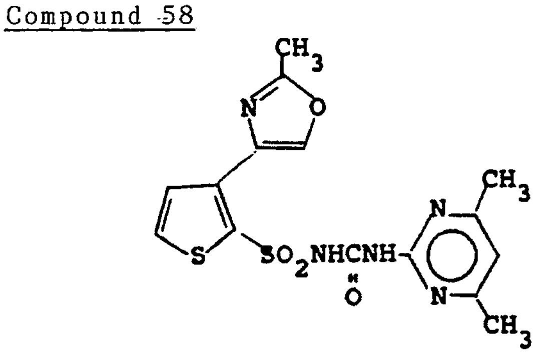

Compound 59

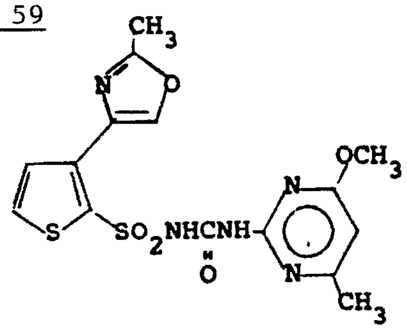

Compounds (continued)

Compound 60

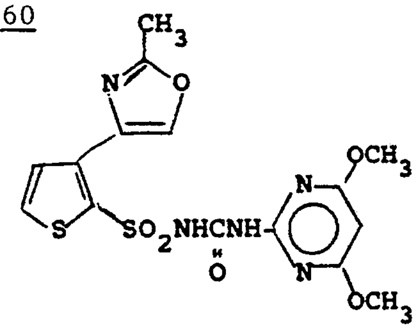

Compound 61

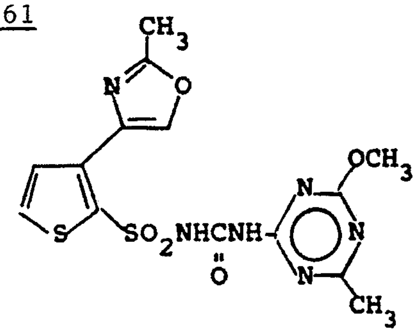

Compound 62

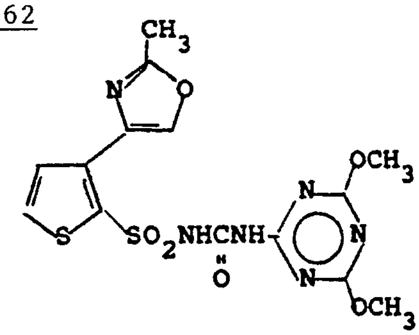

Compound 63

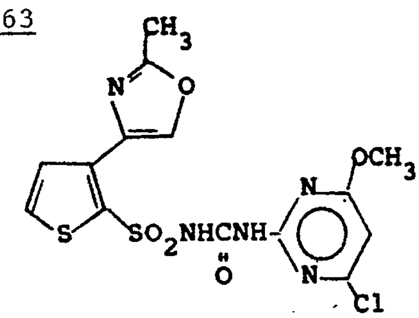

Compounds (continued)

Compound 64

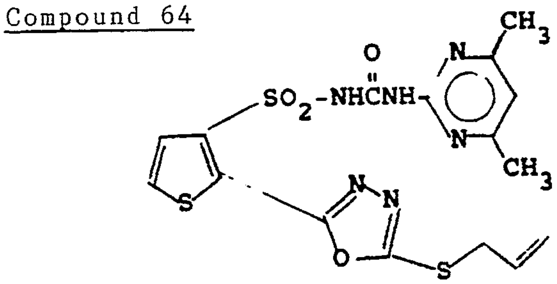

Compound 65

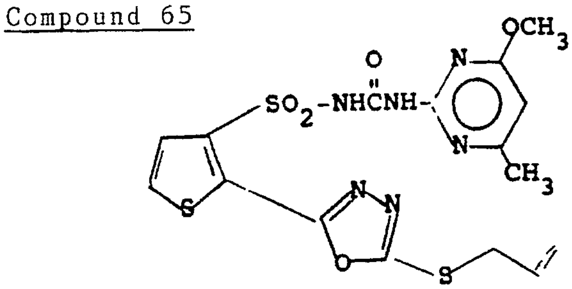

Compound 66

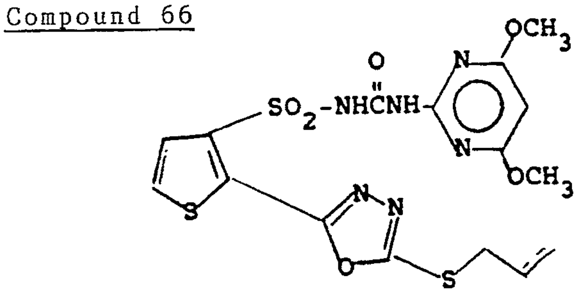

Compound 67

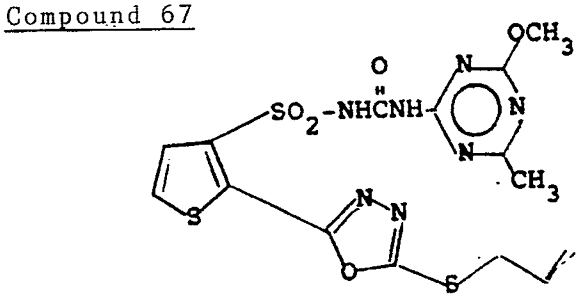

**Compounds (continued)**

Compound 68

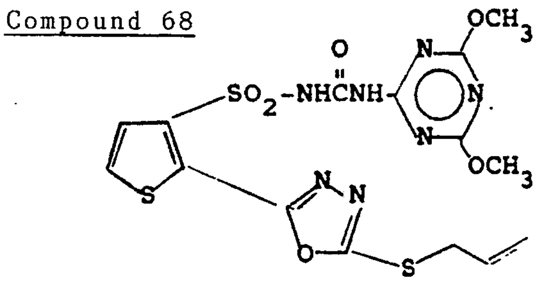

Compound 69

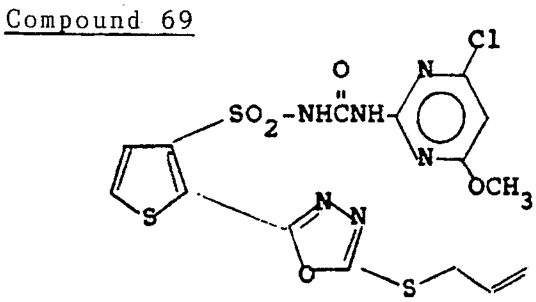

Compound 70

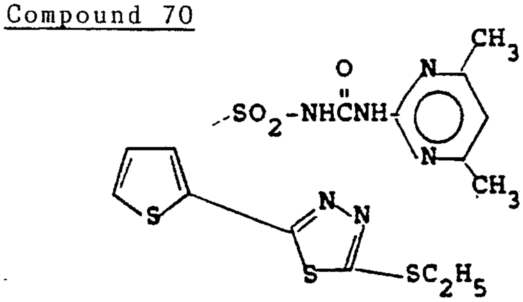

**Compounds (continued)**

Compound 71

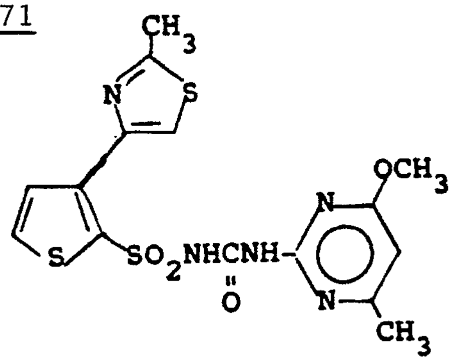

Compound 72

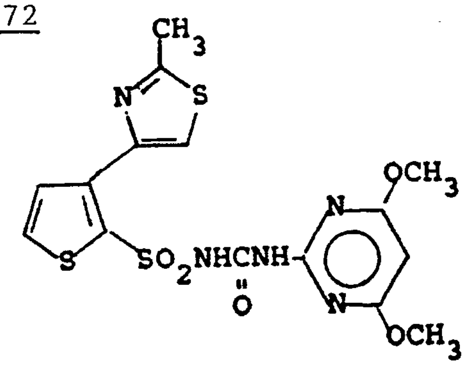

Compound 73

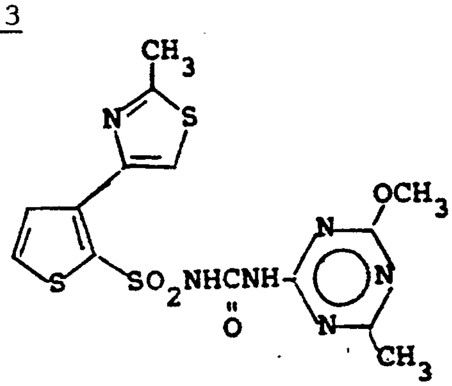

Compounds (continued)

Compound 74

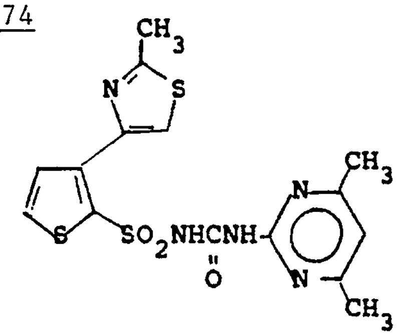

Compound 75

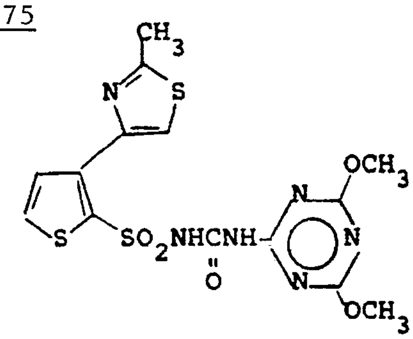

Compound 76

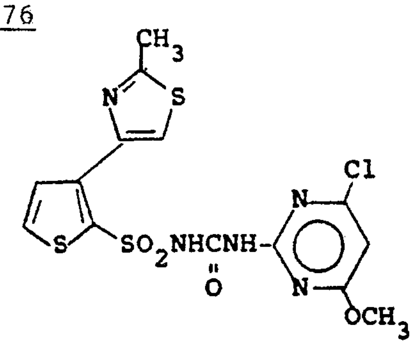

Compounds (continued)

Compound 77

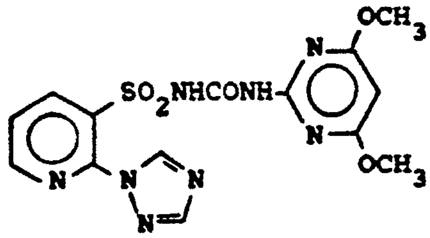

Compound 78

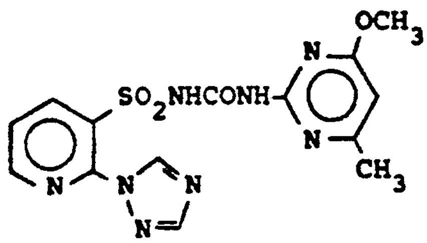

Compound 79

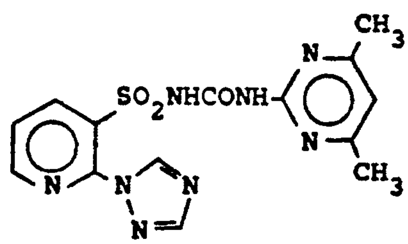

**Table A**

| | Cmpd. 1 | Cmpd. 2 | Cmpd. 3 | Cmpd. 4 | Cmpd.5 |
|---|---|---|---|---|---|
| Rate kg/ha | 0.05 | 0.05 | 0.05 | 0.05 | 0.05 |
| POST-EMERGENCE | | | | | |
| Morningglory | 2C,5G | 4C,9G | 10C | 0 | 0 |
| Cocklebur | 4C,9G | 9C | 10C | 0 | 0 |
| Sicklepod | 2C,8H | 9C | 9C | 0 | 0 |
| Nutsedge | 9C | 9C | 10C | 0 | 0 |
| Crabgrass | 0 | 2C,6G | 2C,6G | 0 | 0 |
| Barnyardgrass | 10C | 9C | 9C | 0 | 0 |
| Wild Oats | 2G | 2C,6G | 0 | 0 | 0 |
| Wheat | 0 | 1C | 0 | 0 | 0 |
| Corn | 5U,9G | 9C | 5C,9G | 0 | 0 |
| Soybean | 5C,9G | 9C | 9C | 3G | 1H |
| Rice | 2C,8G | 3C,9G | 5G | 0 | 0 |
| Sorghum | 9C | 3C,9H | 4C,9G | 0 | 0 |
| Sugar beet | 5C,9G | 9C | 9C | 0 | 0 |
| Cotton | 4C,9G | 6C,9G | 9C | 0 | 0 |
| PRE-EMERGENCE | | | | | |
| Morningglory | 2G | 9C | 9G | 0 | 0 |
| Cocklebur | 9G | 9H | 9H | 0 | 0 |
| Sicklepod | 8G | 9C | 9C | 0 | 0 |
| Nutsedge | 10E | 10E | 10E | 0 | 0 |
| Crabgrass | 0 | 4G | 4G | 0 | 0 |
| Barnyardgrass | 5G | 3C,9H | 3C,9H | 0 | 0 |
| Wild Oats | 2C,7G | 3C,8G | 2C,7G | 0 | 0 |
| Wheat | 5G | 6G | 0 | 0 | 0 |
| Corn | 2C,9H | 3C,9H | 2C,9H | 0 | 0 |
| Soybean | 3C,6H | 9H | 2C,8H | 0 | 0 |
| Rice | 8H | 9G | 8G | 0 | 0 |
| Sorghum | 2C,9G | 6C,9H | 2C,9H | 0 | 0 |
| Sugar beet | 5C,9G | 10C | 9C | 0 | 0 |
| Cotton | 9G | 10C | 9G | 0 | 0 |

Table A (continued)

| | Cmpd. 4 | Cmpd. 5 | Cmpd. 6 | Cmpd. 7 |
|---|---|---|---|---|
| Rate kg/ha | 2 | 2 | 0.05 | 0.05 |
| POST-EMERGENCE | | | | |
| Bush bean | - | - | - | - |
| Cotton | 2C,9G | 1C | 2C,8H | 9C |
| Morningglory | 2C,5G | 1C | 0 | 1C,3G |
| Cocklebur | 1C | 1C | 2C,3H | 6G |
| Sicklepod | 2C,6G | 1C | 1C | 5C,9G |
| Nutsedge | 0 | 0 | 0 | 9G |
| Crabgrass | 0 | 0 | 0 | 0 |
| Barnyardgrass | 0 | 0 | 0 | 4G |
| Wild Oats | 0 | 0 | 0 | 0 |
| Wheat | 0 | 0 | 0 | 0 |
| Corn | 0 | 0 | 0 | 3G |
| Soybean | 2C,7G | 0 | 1C,4H | 5C,9G |
| Rice | 0 | 0 | 2C,4G | 3G |
| Sorghum | 0 | 0 | 2C,4G | 2C,5G |
| Sugar beet | 5C,9G | 3C,5G | 3C,8H | 9C |
| PRE-EMERGENCE | | | | |
| Morningglory | 5G | 0 | 2G | 7G |
| Cocklebur | 5G | 0 | - | 7G |
| Sicklepod | 2C | 0 | 2C | 7G |
| Nutsedge | 0 | 0 | 0 | 10E |
| Crabgrass | 0 | 0 | 0 | 2G |
| Barnyardgrass | 0 | 0 | 0 | 2C,4G |
| Wild Oats | 0 | 0 | 0 | 2C |
| Wheat | 0 | 0 | 0 | 0 |
| Corn | 5G | 0 | 0 | 4G |
| Soybean | 2C,3G | 0 | 3G | 2C,7H |
| Rice | 1C | 0 | 5G | 2C,5G |
| Sorghum | 2G | 0 | 5G | 3C,7H |
| Sugar beet | 4C,9G | 0 | 3C,6G | 9C |
| Cotton | 2C,5G | 0 | 6G | 9G |

Table A (continued)

| | Cmpd. 8 | Cmpd. 9 | Cmpd. 10 | Cmpd. 11 |
|---|---|---|---|---|
| Rate kg/ha | 0.05 | 0.05 | 0.05 | 0.05 |
| POST-EMERGENCE | | | | |
| Bush bean | - | - | - | - |
| Morningglory | 3C,8H | 5C,8H | 2C,5G | 10C |
| Cocklebur | 4C,9G | 2G | 2C,6H | 2C,7G |
| Sicklepod | 3C,3H | 4C,5G | 3C,3H | 2C,7G |
| Nutsedge | 0 | 0 | 7G | 8G |
| Crabgrass | 0 | 0 | 0 | 1H |
| Barnyardgrass | 0 | 0 | 3G | 8H |
| Wild Oats | 0 | 0 | 2C,3G | 0 |
| Wheat | 0 | 0 | 3G | 0 |
| Corn | 0 | 0 | 1C,4H | 3C,9H |
| Soybean | 3C,8H | 3C,7H | 3C,9G | 4C,9G |
| Rice | 4G | 2G | 6G | 2C,5G |
| Sorghum | 3G | 0 | 3C,7G | 5C,9H |
| Sugar beet | 9C | 9C | 2C,7G | 3C,8G |
| Cotton | 4C,9H | 4C,8H | 3C,9H | 9C |
| PRE-EMERGENCE | | | | |
| Morningglory | 8G | 9G | 5G | 9G |
| Cocklebur | 5G | 5H | - | 8H |
| Sicklepod | 3C,8G | 5C,9G | 2C | 7G |
| Nutsedge | 0 | 5G | 0 | 0 |
| Crabgrass | 0 | 0 | 0 | 0 |
| Barnyardgrass | 0 | 0 | 0 | 2C,8H |
| Wild Oats | 0 | 0 | 0 | 2C,4G |
| Wheat | 0 | 2G | 0 | 0 |
| Corn | 3G | 5G | 2G | 4C,9H |
| Soybean | 3C,5H | 4C,7G | 2C,3H | 8H |
| Rice | 3G | 5G | 0 | 7G |
| Sorghum | 2G | 0 | 2C,4H | 3C,9H |
| Sugar beet | 9C | 5C,9G | 4G | 5C,9G |
| Cotton | 6G | 3C,7H | 6G | 8G |

Table A (continued)

| | Cmpd. 12 | Cmpd. 13 | Cmpd. 14 | Cmpd. 15 |
|---|---|---|---|---|
| Rate kg/ha | 0.05 | 0.05 | 0.05 | 0.05 |
| POST-EMERGENCE | | | | |
| Bush bean | - | - | - | - |
| Morningglory | 10C | 9C | 9C | 2C,6G |
| Cocklebur | 4C,9G | 9C | 3C,8G | 2C,5G |
| Sicklepod | 4C,8G | 4C,7G | 4C,8G | 1C |
| Nutsedge | 10C | 3G | 3G | 2C,9G |
| Crabgrass | 0 | 0 | 0 | 0 |
| Barnyardgrass | 3C,8H | 0 | 0 | 0 |
| Wild Oats | 0 | 0 | 0 | 0 |
| Wheat | 0 | 0 | 0 | 0 |
| Corn | 2C,6H | 2G | 3G | 0 |
| Soybean | 4C,9G | 4C,9G | 4C,9G | 1C |
| Rice | 1C,4G | 0 | 0 | 0 |
| Sorghum | 3C,7H | 0 | 0 | 1C |
| Sugar beet | 9C | 9C | 10C | 0 |
| Cotton | 9C | 5C,9G | 4C,9H | 4C,8G |
| PRE-EMERGENCE | | | | |
| Morningglory | 9G | 6G | 9G | 7G |
| Cocklebur | 8H | 4G | 8H | 0 |
| Sicklepod | 8G | 2C,3H | 8G | 0 |
| Nutsedge | 2G | 0 | 0 | 0 |
| Crabgrass | 0 | 0 | 0 | 0 |
| Barnyardgrass | 2C,5G | 0 | 0 | 0 |
| Wild Oats | 0 | 0 | 0 | 0 |
| Wheat | 0 | 0 | 0 | 0 |
| Corn | 2C,7G | 4G | 4G | 0 |
| Soybean | 2C,5H | 3C,6G | 2C,6G | 0 |
| Rice | 1C | 0 | 0 | 0 |
| Sorghum | 5G | 0 | 0 | 0 |
| Sugar beet | 8G | 3C,7G | 4C,7G | 0 |
| Cotton | 8G | 2C,7G | 8G | 6G |

Table A (continued)

| | Cmpd. 16 | Cmpd. 17 | Cmpd. 18 | Cmpd. 19 |
|---|---|---|---|---|
| Rate kg/ha | 0.05 | 0.05 | 0.05 | 0.05 |
| POST-EMERGENCE | | | | |
| Bush bean | - | - | - | - |
| Morningglory | 10C | 10C | 5C,9G | 5C,9G |
| Cocklebur | 5C,9G | 9C | 10C | 9C |
| Sicklepod | 4C,8G | 5C,9G | 5C,9G | 6C,9G |
| Nutsedge | 3C,9G | 9C | 0 | 2C,3G |
| Crabgrass | 0 | 3G | 0 | 0 |
| Barnyardgrass | 4C,9H | 4C,8H | 2H | 0 |
| Wild Oats | 3C,9G | 0 | 0 | 0 |
| Wheat | 0 | 0 | 0 | 0 |
| Corn | 3C,9H | 1C,3H | 0 | 0 |
| Soybean | 5C,9G | 5C,9G | 5C,9G | 5C,9G |
| Rice | 4C,9G | 3G | 0 | 0 |
| Sorghum | 3C,9H | 2C,7G | 0 | 0 |
| Sugar beet | 3C,8G | 9C | 9C | 9C |
| Cotton | 5C,9H | 9C | 5C,9H | 9C |
| PRE-EMERGENCE | | | | |
| Morningglory | 8G | 9G | 4C,9G | 9G |
| Cocklebur | 7H | 8H | 7H | 3C,7H |
| Sicklepod | 5G | 7G | 4C,7G | 4C,8G |
| Nutsedge | 0 | 6G | 0 | 0 |
| Crabgrass | 0 | 0 | 0 | 0 |
| Barnyardgrass | 5G | 3C,9H | 0 | 0 |
| Wild Oats | 4G | 0 | 0 | 0 |
| Wheat | 0 | 0 | 0 | 0 |
| Corn | 2G | 3G | 0 | 0 |
| Soybean | 2H | 3C,6H | 2C,5G | 3C,6H |
| Rice | 5G | 4G | 0 | 0 |
| Sorghum | 2C,5G | 3C,7H | 0 | 0 |
| Sugar beet | 8G | 5C,9G | 5C,9G | 5C,9G |
| Cotton | 4G | 9G | 5G | 2C,6G |

Table A (continued)

| | Cmpd. 20 | Cmpd. 21 | Cmpd. 22 | Cmpd. 23 |
|---|---|---|---|---|
| Rate kg/ha | 0.05 | 0.05 | 0.05 | 0.05 |
| POST-EMERGENCE | | | | |
| Bush bean | - | - | - | - |
| Morningglory | 10C | 9C | 4C,9G | 10C |
| Cocklebur | 9C | 9C | 9C | 10C |
| Sicklepod | 2C,4G | 9C | 2C | 5C,8H |
| Nutsedge | 2C,8G | 9C | 0 | 0 |
| Crabgrass | 0 | 0 | 0 | 0 |
| Barnyardgrass | 3C,8H | 3H | 3G | 0 |
| Wild Oats | 3C,3G | 0 | 0 | 0 |
| Wheat | 2G | 0 | 0 | 0 |
| Corn | 2C,7H | 2G | 2C,3H | 0 |
| Soybean | 4C,9H | 4C,9G | 4C,9G | 4C,9G |
| Rice | 2C,8G | 0 | 0 | 0 |
| Sorghum | 3C,9H | 3C,5H | 0 | 0 |
| Sugar beet | 9C | 9C | 9C | 9C |
| Cotton | 9C | 9C | 4C,9H | 4C,9H |
| PRE-EMERGENCE | | | | |
| Morningglory | 8G | 9G | 8H | 8G |
| Cocklebur | 5H | 8H | 2C | 5H |
| Sicklepod | 5G | 8G | 2C,4G | 5G |
| Nutsedge | 0 | 10E | 0 | 0 |
| Crabgrass | 0 | 0 | 0 | 0 |
| Barnyardgrass | 0 | 2H | 0 | 0 |
| Wild Oats | 0 | 0 | 0 | 0 |
| Wheat | 0 | 0 | 0 | 0 |
| Corn | 2C,8H | 7G | 2C,5G | 2C,4G |
| Soybean | 4C,5G | 2C,6H | 4C,5H | 4C,6G |
| Rice | 2C,5G | 5G | 3G | 0 |
| Sorghum | 3C,9H | 5H | 0 | 0 |
| Sugar beet | 5C,9G | 4C,9G | 4C,9G | 4C,9G |
| Cotton | 8G | 9G | 2C,7G | 6G |

Table A (continued)

| | Cmpd. 24 | Cmpd. 25 | Cmpd. 26 | Cmpd. 27 |
|---|---|---|---|---|
| Rate kg/ha | 0.05 | 0.05 | 0.05 | 0.05 |
| POST-EMERGENCE | | | | |
| Morningglory | 2C,8H | 10C | 4C,9G | 4C,7H |
| Cocklebur | 5G | 5C,9G | 5C,9G | 4C,9H |
| Velvetleaf | 2C,5H | 9C | 5C,9G | 3C,7H |
| Nutsedge | 0 | 5G | 3C,9G | 0 |
| Crabgrass | 0 | 5G | 3G | 0 |
| Barnyardgrass | 0 | 3C,8H | 3C,9H | 0 |
| Wild Oats | 0 | 0 | 0 | 0 |
| Wheat | 0 | 0 | 2G | 0 |
| Corn | 0 | 4C,9G | 3C,9H | 0 |
| Soybean | 2C,7G | 4C,9G | 4C,9G | 3C,6H |
| Rice | 2G | 3C,8G | 3C,8G | 0 |
| Sorghum | 2C | 4C,9H | 3C,8H | 0 |
| Cheatgrass | 0 | 3C,8G | 5G | 0 |
| Sugar beet | 0 | 9C | 9C | 4C,8H |
| Cotton | 2G | 5C,9H | 5C,9G | 3C,6G |
| PRE-EMERGENCE | | | | |
| Morningglory | 0 | 9G | 8G | 3C,3H |
| Cocklebur | 0 | 3C,5H | 9H | 0 |
| Velvetleaf | 0 | 3C,6H | 5H | 0 |
| Nutsedge | 0 | 0 | 0 | 0 |
| Crabgrass | 0 | 1C | 0 | 0 |
| Barnyardgrass | 0 | 1C | 3H | 0 |
| Wild Oats | 0 | 0 | 0 | 0 |
| Wheat | 0 | 0 | 0 | 0 |
| Corn | 0 | 5C,8G | 4C,3G | 0 |
| Soybean | 0 | 4C,7H | 3C,4G | 0 |
| Rice | 0 | 5G | 2C | 2H |
| Sorghum | 0 | 4C,7G | 3C,7G | 2G |
| Cheatgrass | 0 | 4G | 0 | 0 |
| Sugar beet | 0 | 4C,9G | 4C,8G | 0 |
| Cotton | 0 | 3C,7H | 7G | 0 |

Table A (continued)

| | Cmpd. 28 | Cmpd. 29 | Cmpd. 30 | Cmpd. 31 |
|---|---|---|---|---|
| Rate kg/ha | 0.05 | 0.05 | 0.05 | 0.05 |
| POST-EMERGENCE | | | | |
| Morningglory | 4C,9G | 1C,2H | 2C,7G | 9C |
| Cocklebur | 4C,8H | 2C,5H | 2C,7H | 9C |
| Velvetleaf | 0 | 3G | 2G | 10C |
| Nutsedge | 0 | 0 | 2G | 6C,9G |
| Crabgrass | 0 | 0 | 0 | 3G |
| Barnyardgrass | 0 | 2C,5H | 7H | 3C,7G |
| Wild Oats | 0 | 0 | 0 | 0 |
| Wheat | 0 | 0 | 0 | 0 |
| Corn | 0 | 0 | 0 | 3C,8H |
| Soybean | 3C,8G | 0 | 2H | 4C,9G |
| Rice | 0 | 0 | 5C,9G | 5G |
| Sorghum | 0 | 1C,2H | 2C,6H | 3C,9H |
| Cheatgrass | 0 | 0 | 0 | 6G |
| Sugar beet | 4C,9H | 0 | 5C,9H | 10C |
| Cotton | 3C,7G | 0 | 5C,9G | 9C |
| PRE-EMERGENCE | | | | |
| Morningglory | 0 | 0 | 7G | 9G |
| Cocklebur | 0 | 0 | 4H | 9H |
| Velvetleaf | 0 | 0 | 8G | 10E |
| Nutsedge | 0 | 0 | 7G | 8G |
| Crabgrass | 0 | 0 | 0 | 0 |
| Barnyardgrass | 0 | 0 | 0 | 2C,5H |
| Wild Oats | 0 | 0 | 0 | 2G |
| Wheat | 0 | 0 | 0 | 0 |
| Corn | 0 | 0 | 2C,7G | 3C,9H |
| Soybean | 2C | 0 | 1H | 4C,8H |
| Rice | 4G | 0 | 2C,4G | 4C,8G |
| Sorghum | 0 | 0 | 3C,6H | 4C,9H |
| Cheatgrass | 0 | 0 | 2G | 9G |
| Sugar beet | 0 | 0 | 8G | 9C |
| Cotton | 0 | 0 | 9G | 9G |

Table A (continued)

| | Cmpd. 32 | Cmpd. 33 |
|---|---|---|
| Rate kg/ha | 0.05 | 0.05 |
| POST-EMERGENCE | | |
| Morningglory | 4C,8G | 0 |
| Cocklebur | 4C,9H | 0 |
| Velvetleaf | 3C,9G | 0 |
| Nutsedge | 4G | 0 |
| Crabgrass | 0 | 0 |
| Barnyardgrass | 2H | 0 |
| Wild Oats | 0 | 0 |
| Wheat | 0 | 0 |
| Corn | 0 | 0 |
| Soybean | 3C,7H | 2G |
| Rice | 0 | 0 |
| Sorghum | 0 | 0 |
| Cheatgrass | 0 | 0 |
| Sugar beet | 4C,8H | 2H |
| Cotton | 4C,8G | 2G |
| PRE-EMERGENCE | | |
| Morningglory | 8G | 0 |
| Cocklebur | 3G | 0 |
| Velvetleaf | 4G | 0 |
| Nutsedge | 0 | 0 |
| Crabgrass | 0 | 0 |
| Barnyardgrass | 0 | 0 |
| Wild Oats | 0 | 0 |
| Wheat | 0 | 0 |
| Corn | 0 | 0 |
| Soybean | 6H | 0 |
| Rice | 0 | 0 |
| Sorghum | 1C | 0 |
| Cheatgrass | 0 | 0 |
| Sugar beet | 9C | 4G |
| Cotton | 8G | 0 |

Table A (continued)

| | Cmpd. 34 | Cmpd. 35 | Cmpd. 36 |
|---|---|---|---|
| Rate kg/ha | 0.05 | 0.05 | 0.05 |
| POST-EMERGENCE | | | |
| Morningglory | 5G | 9C | 4H |
| Cocklebur | 2C,8G | 10C | 0 |
| Velvetleaf | 7G | 9C | 0 |
| Nutsedge | 0 | 5C,9G | 0 |
| Crabgrass | 0 | 4C,5G | 0 |
| Barnyardgrass | 0 | 9C | 0 |
| Wild Oats | 0 | 2C,2G | 0 |
| Wheat | 0 | 8G | 0 |
| Corn | 0 | 5C,9H | 0 |
| Soybean | 2G | 5C,9G | 0 |
| Rice | 0 | 9C | 0 |
| Sorghum | 0 | 4C,9G | 0 |
| Cheatgrass | 0 | 9C | 0 |
| Sugar beet | 4G | 9C | 2H |
| Cotton | 5G | 9C | 0 |
| PRE-EMERGENCE | | | |
| Morningglory | 0 | 9C | 0 |
| Cocklebur | 0 | 9H | 0 |
| Velvetleaf | 0 | 9H,9G | 0 |
| Nutsedge | 0 | 5C,9H | 0 |
| Crabgrass | 0 | 5G | 0 |
| Barnyardgrass | 0 | 4C,9H | 0 |
| Wild Oats | 0 | 3G | 0 |
| Wheat | 0 | 8H | 0 |
| Corn | 0 | 9H | 0 |
| Soybean | 0 | 4C,8H | 0 |
| Rice | 0 | 10E | 0 |
| Sorghum | 0 | 5C,9H | 0 |
| Cheatgrass | 0 | 5C,9H | 0 |
| Sugar beet | 4G | 5C,9G | 0 |
| Cotton | 0 | 2C,9G | 0 |

Table A (continued)

| | Cmpd. 37 | Cmpd. 38 | Cmpd. 39 | Cmpd. 40 |
|---|---|---|---|---|
| Rate kg/ha | 0.05 | 0.05 | 0.05 | 0.05 |
| POST-EMERGENCE | | | | |
| Morningglory | 5C,9G | 10C | 9C | 4C,9G |
| Cocklebur | 4C,9G | 9C | 9C | 5C,9G |
| Velvetleaf | 9C | 10C | 10C | 5C,8G |
| Nutsedge | 3C,5G | 4C,8G | 5C,9G | 0 |
| Crabgrass | 0 | 2G | 2G | 0 |
| Barnyardgrass | 4G | 4C,9H | 4C,9H | 0 |
| Cheatgrass | 2C,6G | 3C,5G | 8G | 0 |
| Wild Oats | 0 | 0 | 0 | 0 |
| Wheat | 0 | 0 | 0 | 0 |
| Corn | 1H | 3C,7H | 3H | 0 |
| Soybean | 4H | 4C,8H | 2C,7G | 2G |
| Rice | 8G | 8G | 6G | 0 |
| Sorghum | 2C,8G | 4C,8H | 3C,8H | 0 |
| Sugar beet | 3C,8H | 9C | 9C | 9C |
| Cotton | 4C,9H | 10C | 10C | 3C,9G |
| PRE-EMERGENCE | | | | |
| Morningglory | 3C | 6G | 7G | 2C,6G |
| Cocklebur | 4H | 4G | 5G | 2G |
| Velvetleaf | 3G | 8H | - | 3G |
| Nutsedge | 0 | 0 | 0 | 0 |
| Crabgrass | 0 | 0 | 2G | 0 |
| Barnyardgrass | 0 | 1C,3H | 4C,7G | 0 |
| Cheatgrass | 0 | 5G | 8G | 0 |
| Wild Oats | 0 | 0 | 0 | 0 |
| Wheat | 0 | 0 | 0 | 0 |
| Corn | 0 | 3G | 6G | 0 |
| Soybean | 0 | 4G | 2C,5G | 2G |
| Rice | 0 | 5G | 8G | 0 |
| Sorghum | 0 | 2C,5G | 4C,9G | 0 |
| Sugar beet | 4G | 8G | 5C,9G | 4C,8G |
| Cotton | 4G | 8G | 8G | 7G |

Table A (continued)

| | Cmpd. 41 | Cmpd. 42 | Cmpd. 43 | Cmpd. 44 |
|---|---|---|---|---|
| Rate kg/ha | 0.05 | 0.05 | 0.05 | 0.05 |
| POST-EMERGENCE | | | | |
| Morningglory | 4C,9G | 2C,4G | 7G | 5C,9G |
| Cocklebur | 5C,9G | 2C,5G | 5C,9H | 10C |
| Velvetleaf | 2C,5G | 3C,8G | 5C,9H | 5C,9G |
| Nutsedge | 0 | 0 | 4C,9G,8X | 0 |
| Crabgrass | 0 | 0 | 0 | 2C,6H |
| Barnyardgrass | 0 | 0 | 3C,9H | 3C,9H |
| Cheatgrass | 0 | 0 | 4C,9G | 4G |
| Wild Oats | 0 | 0 | 2G | 0 |
| Wheat | 0 | 0 | 2G | 0 |
| Corn | 0 | 0 | 3C,9H | 5C,8G |
| Soybean | 0 | 0 | 4C,9G | 5C,9G |
| Rice | 0 | 0 | 9C | 5C,9G |
| Sorghum | 0 | 0 | 3C,9H | 2C,8H |
| Sugar beet | 9C | 2C,3H | 10C | 4C,9H |
| Cotton | 4C,9G | 6G | 10C | 9C |
| PRE-EMERGENCE | | | | |
| Morningglory | 7H | 5G | 8H | 7G |
| Cocklebur | 0 | 0 | 8H | 8G |
| Velvetleaf | 2H | 3G | 9G | 7G |
| Nutsedge | 0 | 0 | 0 | 0 |
| Crabgrass | 0 | 0 | 0 | 0 |
| Barnyardgrass | 0 | 0 | 2C,6H | 5G |
| Cheatgrass | 0 | 0 | 2C,8G | 0 |
| Wild Oats | 0 | 0 | 6G | 0 |
| Wheat | 0 | 0 | 5G | 0 |
| Corn | 0 | 0 | 2C,9H | 5G |
| Soybean | 0 | 0 | 2C,6G | 5G |
| Rice | 2C | 0 | 3C,5G | 5G |
| Sorghum | 1C | 2C,4G | 3C,8H | 2G |
| Sugar beet | 7G | 8G | 9C | 5C,9G |
| Cotton | 3G | 5G | 8G | 6G |

Table A (continued)

| | Cmpd. 45 | Cmpd. 46 | Cmpd. 47 |
|---|---|---|---|
| Rate kg/ha | 0.05 | 0.05 | 0.05 |
| POST-EMERGENCE | | | |
| Morningglory | 4C,9H | 10C | 3C,7H |
| Cocklebur | 10C | 10C | 4C,9H |
| Velvetleaf | 4C,9G | 10C | 5C,9G |
| Nutsedge | 2C,9G | 5C,9G | 2C,5G |
| Crabgrass | 0 | 8G | 0 |
| Barnyardgrass | 4C,8H | 9C | 3C,9H |
| Cheatgrass | 1C | 9C | 2C,6G |
| Wild Oats | 0 | 3C,5G | 0 |
| Wheat | 0 | 2C,4G | 0 |
| Corn | 2C,7H | 5C,9H | 9G |
| Soybean | 3C,8G | 5C,9G | 2C,9G |
| Rice | 5C,9G | 6C,9G | 2C,9G |
| Sorghum | 3C,7H | 5C,9H | 3C,9H |
| Sugar beet | 5C,9G | 9C | 3C,8H |
| Cotton | 10C | 9C | 3C,7H |
| PRE-EMERGENCE | | | |
| Morningglory | 9G | 9G | 5G |
| Cocklebur | 7G | 9H | 5H |
| Velvetleaf | 8G | 5C,9G | 6G |
| Nutsedge | 9G | 8G | 0 |
| Crabgrass | 0 | 7G | 0 |
| Barnyardgrass | 3C,8H | 2C,9H | 6G |
| Cheatgrass | 3G | 8G | 3C,7G |
| Wild Oats | 0 | 6G | 0 |
| Wheat | 0 | 6G | 0 |
| Corn | 3C,5G | 2C,9H | 2C,8H |
| Soybean | 4G | 9H | 1C,3G |
| Rice | 0 | 10E | 3C,5G |
| Sorghum | 2C,8H | 6C,9G | 3C,8H |
| Sugar beet | 8G | 5C,9G | 2C,9G |
| Cotton | 8G | 9G | 8G |

Table A (continued)

| | Cmpd. 48 | Cmpd. 49 | Cmpd. 50 | Cmpd. 51 |
|---|---|---|---|---|
| Rate kg/ha | 0.05 | 0.05 | 0.05 | 0.05 |
| POST-EMERGENCE | | | | |
| Morningglory | 0 | 5C,9G | 5C,9G | 0 |
| Cocklebur | 2C,7G | 8H | 4C,9G | 0 |
| Velvetleaf | 2C,4G | 4C,9G | 4C,8G | 0 |
| Nutsedge | 0 | 2G | 0 | 0 |
| Crabgrass | 0 | 0 | 4G | 0 |
| Barnyardgrass | 2C,4H | 2C,7H | 4C,8H | 4H |
| Cheatgrass | 0 | 0 | 5G | 0 |
| Wild Oats | 0 | 0 | 0 | 0 |
| Wheat | 0 | 0 | 0 | 0 |
| Corn | 3C,6H | 2C,8G | 0 | 0 |
| Soybean | 3C,5G | 4C,9G | 3C,9G | 0 |
| Rice | 2C,8G | 3C,8G | 2C,3G | 0 |
| Sorghum | 2C,5G | 3C,7H | 2C,6G | 3G |
| Sugar beet | 5C,9H | 9C | 5C,9G | 1H |
| Cotton | 0 | 5C,9G | 5C,9G | 0 |
| PRE-EMERGENCE | | | | |
| Morningglory | 4H | 6G | 7H | 0 |
| Cocklebur | 3H | 2C,5G | 0 | 0 |
| Velvetleaf | 5G | 4G | 0 | 0 |
| Nutsedge | 0 | 5G | 0 | 0 |
| Crabgrass | 4G | 0 | 0 | 0 |
| Barnyardgrass | 1H | 0 | 0 | 0 |
| Cheatgrass | 0 | 0 | 0 | 0 |
| Wild Oats | 0 | 0 | 0 | 0 |
| Wheat | 0 | 0 | 0 | 0 |
| Corn | 2G | 2C,5G | 0 | 0 |
| Soybean | 0 | 2C | 0 | 0 |
| Rice | 0 | 3G | 0 | 0 |
| Sorghum | 0 | 1C | 0 | 0 |
| Sugar beet | 5G | 4C,9G | 7G | 0 |
| Cotton | 3G | 8G | 2G | 0 |

Table A (continued)

| | Cmpd. 52 | Cmpd. 53 | Cmpd. 54 | Cmpd. 55 |
|---|---|---|---|---|
| Rate kg/ha | 0.05 | 0.05 | 0.05 | 0.05 |
| POST-EMERGENCE | | | | |
| Morningglory | 5C,9G | 10C | 9C | 4C,9G |
| Cocklebur | 5C,9G | 9C | 10C | 9C |
| Velvetleaf | 4C,8H | 5C,9G | 9C | 4C,8H |
| Nutsedge | 9G | 2C,9G | 8G | 0 |
| Crabgrass | 0 | 3C,7G | 5G | 2G |
| Barnyardgrass | 4C,9H | 9C | 5C,9H | 4H |
| Cheatgrass | 2C,8G | 2C,9G | 3C,9G | 0 |
| Wild Oats | 3G | 2C,5G | 3C,5G | 0 |
| Wheat | 2G | 2C,8G | 9G | 0 |
| Corn | 3C,9H | 5C,9G | 3C,9H | 5H |
| Soybean | 4C,9G | 9C | 9C | 4C,8G |
| Rice | 2C,8G | 5C,9G | 5C,9G | 0 |
| Sorghum | 3C,9H | 5C,9G | 4C,9H | 0 |
| Sugar beet | 3C,7G | 4C,9H | 5C,9H | 3C,7G |
| Cotton | 3C,5G | 4C,9H | 5C,9G | 3C,4G |
| PRE-EMERGENCE | | | | |
| Morningglory | 2C | 3C,7H | 3C,7H | 2H |
| Cocklebur | 1C | 3C,3H | 3C,6H | 0 |
| Velvetleaf | 0 | 4C,5G | 3C,5G | 0 |
| Nutsedge | 0 | 0 | 4G | 0 |
| Crabgrass | 0 | 0 | 0 | 0 |
| Barnyardgrass | 0 | 0 | 4G | 0 |
| Cheatgrass | 0 | 0 | 0 | 0 |
| Wild Oats | 0 | 0 | 0 | 0 |
| Wheat | 0 | 0 | 0 | 0 |
| Corn | 2C | 3C,5G | 3C,4G | 3G |
| Soybean | 0 | 3C,3H | 3C,5G | 0 |
| Rice | 0 | 0 | 3C | 0 |
| Sorghum | 0 | 3C | 3C,4G | 0 |
| Sugar beet | 0 | 3C,6G | 4C,7G | 0 |
| Cotton | 0 | 2C | 6G | 0 |

**Table A (continued)**

| | Cmpd. 56 | Cmpd. 57 | Cmpd. 58 | Cmpd. 59 |
|---|---|---|---|---|
| Rate kg/ha | 0.05 | 0.05 | 0.05 | 0.05 |
| **POST-EMERGENCE** | | | | |
| Morningglory | 9C | 4C,9G | 2C,8G | 10C |
| Cocklebur | 10C | 5C,9G | 10C | 10C |
| Velvetleaf | 2C,4G | 3C,6G | 5C,9G | 10C |
| Nutsedge | 0 | 3C,8G | 5G | 2C,9G |
| Crabgrass | 0 | 0 | 4G | 5G |
| Barnyardgrass | 2H | 5H | 3C,5H | 3C,9H |
| Cheatgrass | 0 | 2G | 6G | 2C,8G |
| Wild Oats | 0 | 0 | 0 | 0 |
| Wheat | 0 | 0 | 0 | 0 |
| Corn | 0 | 3H | 2C,9G | 2U,9G |
| Soybean | 4C,9G | 2C,3G | 4C,9G | 9C |
| Rice | 0 | 3G | 3C,9G | 4C,9G |
| Sorghum | 0 | 2C,7G | 3C,8H | 4C,9H |
| Sugar beet | 9C | 4G | 9C | 9C |
| Cotton | 3C,7G | 2G | 5C,9G | 10C |
| **PRE-EMERGENCE** | | | | |
| Morningglory | 3H | 2C,5G | 2C,6G | 8G |
| Cocklebur | 0 | 2C | 3C,7H | 6G |
| Velvetleaf | 0 | 0 | 6G | 4C,9G |
| Nutsedge | 0 | 0 | 4G | 10E |
| Crabgrass | 0 | 0 | 3G | 5G |
| Barnyardgrass | 0 | 0 | 0 | 3C,8H |
| Cheatgrass | 0 | 0 | 4G | 8H |
| Wild Oats | 0 | 0 | 0 | 0 |
| Wheat | 0 | 0 | 0 | 0 |
| Corn | 0 | 0 | 2C,7G | 3C,9G |
| Soybean | 0 | 0 | 2C,2H | 3C,8H |
| Rice | 2G | 0 | 3G | 7G |
| Sorghum | 0 | 2C | 3G | 3C,9H |
| Sugar beet | 0 | 0 | 7G | 5C,9G |
| Cotton | 3G | 2C | 8G | 9G |

Table A (continued)

| | Cmpd. 60 | Cmpd. 61 | Cmpd. 62 | Cmpd. 63 |
|---|---|---|---|---|
| Rate kg/ha | 0.05 | 0.05 | 0.05 | 0.05 |
| POST-EMERGENCE | | | | |
| Morningglory | 10C | 9C | 5C,9G | 2C,9G |
| Cocklebur | 10C | 9C | 4C,9G | 5C,9G |
| Velvetleaf | 10C | 9C | 4C,9G | 3C,9G |
| Nutsedge | 9C | 0 | 0 | 3C,9G |
| Crabgrass | 5G | 0 | 2G | 2G |
| Barnyardgrass | 9H | 3H | 2H | 8H |
| Cheatgrass | 8G | 0 | 0 | 0 |
| Wild Oats | 0 | 0 | 0 | 0 |
| Wheat | 0 | 0 | 0 | 0 |
| Corn | 3C,9H | 6H | 6H | 5H |
| Soybean | 9C | 5C,9G | 4C,9G | 2C,7G |
| Rice | 2C,8G | 3G | 0 | 3G |
| Sorghum | 3C,9H | 5G | 3G | 2C,8G |
| Sugar beet | 9C | 9C | 9C | 9C |
| Cotton | 10C | 2C,8G | 2C,3G | 5C,9G |
| PRE-EMERGENCE | | | | |
| Morningglory | 8G | 2C,5G | 2C,5H | 0 |
| Cocklebur | 8H | 2C,5H | 2H | 0 |
| Velvetleaf | 8G | 3C,3H | 2C,4G | 0 |
| Nutsedge | 5G | 0 | 0 | 0 |
| Crabgrass | 0 | 0 | 0 | 0 |
| Barnyardgrass | 5G | 2C | 0 | 2C |
| Cheatgrass | 0 | 0 | 0 | 0 |
| Wild Oats | 0 | 0 | 0 | 0 |
| Wheat | 0 | 0 | 0 | 0 |
| Corn | 2C,5G | 2C,6G | 2G | 2G |
| Soybean | 4H | 2C,4G | 6G | 3G |
| Rice | 2G | 0 | 0 | 2G |
| Sorghum | 2C,9G | 2C,8G | 2G | 3C,6G |
| Sugar beet | 3C,9G | 5C,9G | 4C,9G | 7G |
| Cotton | 9G | 8G | 7G | 6G |

Table A (continued)

| | Cmpd. 64 | Cmpd. 65 | Cmpd. 66 | Cmpd. 67 |
|---|---|---|---|---|
| Rate kg/ha | 0.05 | 0.05 | 0.05 | 0.05 |
| POST-EMERGENCE | | | | |
| Morningglory | 5G | 9C | 2C,7G | 5G |
| Cocklebur | 5G | 6G | 5G | 4G |
| Velvetleaf | 2C,8G | 9C | 9C | 2C,6G |
| Nutsedge | 2C,8G | 2C,9G | 9G | 0 |
| Crabgrass | 0 | 2C,6G | 3C,8G | 0 |
| Barnyardgrass | 2C,5G | 3C,9H | 9H | 0 |
| Cheatgrass | 2C,9G | 3C,9G | 3C,9G | 0 |
| Wild Oats | 0 | 2G | 0 | 0 |
| Wheat | 0 | 2G | 0 | 0 |
| Corn | 1C,2G | 2C,9H | 1C,4G | 0 |
| Soybean | 3H,8G | 4C,9G | 2C,8H | 3H |
| Rice | 2C,6G | 2C,8G | 3C,9G | 0 |
| Sorghum | 2C,8H | 3C,9G | 3C,9G | 0 |
| Sugar beet | 9C | 9C | 9C | 5C,9G |
| Cotton | 3C,9H | 2C,9G | 9C | 2C,9G |
| PRE-EMERGENCE | | | | |
| Morningglory | 2G | 2C,7G | 9G | 5G |
| Cocklebur | 0 | 6G | 7G | 0 |
| Velvetleaf | 5G | 4C,9G | 5C,9G | 0 |
| Nutsedge | 0 | 6G | 2C,9G | 0 |
| Crabgrass | 3G | 4G | 5G | 0 |
| Barnyardgrass | 0 | 3C,7H | 2C,9H | 0 |
| Cheatgrass | 5G | 2C,8G | 9H | 0 |
| Wild Oats | 0 | 2C,7G | 4G | 0 |
| Wheat | 0 | 5G | 2G | 0 |
| Corn | 2C,5G | 2C,8H | 2C,8G | 0 |
| Soybean | 1C | 2C,6G | 6G | 0 |
| Rice | 2C,5G | 3C,8H | 3C,9H | 0 |
| Sorghum | 3C,8G | 3C,9G | 2C,9H | 3G |
| Sugar beet | 2C,8G | 9G | 3C,8G | 5G |
| Cotton | 6G | 8G | 9G | 0 |

Table A (continued)

| | Cmpd. 68 | Cmpd. 69 | Cmpd. 70 |
|---|---|---|---|
| Rate kg/ha | 0.05 | 0.05 | 0.05 |
| POST-EMERGENCE | | | |
| Morningglory | 2C,5G | 2C,6G | 2C,6G |
| Cocklebur | 2G | 0 | 5G |
| Velvetleaf | 2G | 5G | 5C,9G |
| Nutsedge | 0 | 0 | 8G |
| Crabgrass | 0 | 1C | 2G |
| Barnyardgrass | 0 | 2C,8H | 4C,9H |
| Cheatgrass | 0 | 0 | 2C,9G |
| Wild Oats | 0 | 0 | 3C,8G |
| Wheat | 0 | 0 | 3G |
| Corn | 0 | 0 | 9H |
| Soybean | 1H | 3H | 4C,9G |
| Rice | 0 | 5G | 5C,9G |
| Sorghum | 1C | 2C,9H | 5C,9G |
| Sugar beet | 2C,7G | 3C,7G | 5C,9H |
| Cotton | 2C,5G | 3C,7H | 4C,9H |
| PRE-EMERGENCE | | | |
| Morningglory | 2G | 6H | 4G |
| Cocklebur | 0 | 0 | 0 |
| Velvetleaf | 0 | 3G | 2G |
| Nutsedge | 0 | 0 | 0 |
| Crabgrass | 0 | 0 | 0 |
| Barnyardgrass | 0 | 0 | 1C |
| Cheatgrass | 0 | 0 | 6G |
| Wild Oats | 0 | 0 | 0 |
| Wheat | 0 | 0 | 4G |
| Corn | 0 | 0 | 2C,4G |
| Soybean | 0 | 0 | 5G |
| Rice | 0 | 5G | 9H |
| Sorghum | 0 | 2C,8G | 3C,8H |
| Sugar beet | 0 | 3C,6G | 4C,8G |
| Cotton | 0 | 3C,6G | 8G |

Table A (continued)

| | Cmpd. 71 | Cmpd. 72 | Cmpd. 73 |
|---|---|---|---|
| Rate kg/ha | 0.05 | 0.05 | 0.05 |
| POST-EMERGENCE | | | |
| Morningglory | 4C,9G | 10C | 7G |
| Cocklebur | 4C,9H | 10C | 3C,9G |
| Velvetleaf | 10C | 10C | 4C,9G |
| Nutsedge | 2C,5G | 7G | 0 |
| Crabgrass | 0 | 4G | 0 |
| Barnyardgrass | 9H | 4C,9H | 2H |
| Cheatgrass | 2C,5G | 3G | 0 |
| Wild Oats | 0 | 0 | 0 |
| Wheat | 0 | 0 | 0 |
| Corn | 3C,9H | 0 | 0 |
| Soybean | 5C,9G | 4C,9G | 4C,9G |
| Rice | 2G | 2G | 0 |
| Sorghum | 3C,4H | 2C,5H | 2H |
| Sugar beet | 4C,9H | 9C | 4C,9G |
| Cotton | 4C,9G | 9C | 2C,5G |
| PRE-EMERGENCE | | | |
| Morningglory | 3G | 7H | 0 |
| Cocklebur | 3C,6H | - | 0 |
| Velvetleaf | 9C | 4C,8G | 2G |
| Nutsedge | 5G | 0 | 0 |
| Crabgrass | 0 | 0 | 0 |
| Barnyardgrass | 3C,7G | 3H | 0 |
| Cheatgrass | 7G | 0 | 0 |
| Wild Oats | 0 | 0 | 0 |
| Wheat | 0 | 0 | 0 |
| Corn | 3C,9H | 2C,5G | 0 |
| Soybean | 3C,2H | 2C | 0 |
| Rice | 4G | 0 | 0 |
| Sorghum | 3C,8H | 3C,8H | 0 |
| Sugar beet | 4C,9G | 8G | 5G |
| Cotton | 8G | 6G | 0 |

Table A (continued)

| | Cmpd. 74 | Cmpd. 75 | Cmpd. 76 |
|---|---|---|---|
| Rate kg/ha | 0.05 | 0.05 | 0.05 |
| POST-EMERGENCE | | | |
| Morningglory | 2C,5G | 10C | 2C,3G |
| Cocklebur | 3C,9H | 5C,9G | 6G |
| Velvetleaf | 4C,9G | 4C,8H | 4G |
| Nutsedge | 4G | 0 | 5G |
| Crabgrass | 0 | 5G | 3G |
| Barnyardgrass | 5H | 2H | 3C,8H |
| Cheatgrass | 5G | 0 | 0 |
| Wild Oats | 0 | 0 | 0 |
| Wheat | 0 | 0 | 0 |
| Corn | 3C,8H | 0 | 0 |
| Soybean | 4C,8G | 4C,9G | 0 |
| Rice | 4G | 0 | 0 |
| Sorghum | 3C,7G | 2G | 3C,6H |
| Sugar beet | 3C,6G | 5C,9G | 0 |
| Cotton | 2C,5G | 2G | 0 |
| PRE-EMERGENCE | | | |
| Morningglory | 7G | 5H | 5G |
| Cocklebur | 0 | 0 | 0 |
| Velvetleaf | 2C,5G | 0 | 0 |
| Nutsedge | 0 | 0 | 0 |
| Crabgrass | 4G | 0 | 0 |
| Barnyardgrass | 2G | 2G | 0 |
| Cheatgrass | 0 | 0 | 0 |
| Wild Oats | 0 | 0 | 0 |
| Wheat | 0 | 0 | 0 |
| Corn | 2C,5G | 0 | 2G |
| Soybean | 2C,5G | 0 | 0 |
| Rice | 0 | 0 | 0 |
| Sorghum | 3C,7H | 2G | 2C,8G |
| Sugar beet | 7G | 5G | 7G |
| Cotton | 6G | 5G | 7G |

Table A (continued)

| | Compound 77 | | Compound 78 | |
|---|---|---|---|---|
| Rate g/ha | 2000 | 400 | 2000 | 400 |
| POSTEMERGENCE | | | | |
| Cotton | 5C,9G | 4C,9G | 4C,9G | 3C,9H |
| Sorghum | 3C,8H | 2C,2G | 3C,6G | 3C |
| Corn | 3C,8H | 0 | 2C,6H | 0 |
| Soybean | 3C,8H | 3C,8G | 3C,8G | 2C,8G |
| Wheat | 5G | 0 | 5G | 2G |
| Wild Oats | 0 | 0 | 5G | 0 |
| Rice | 2C,8G | 3G | 4C,8G | 3C,5G |
| Barnyardgrass | 3C,9H | 2G | 4C,8H | 0 |
| Morningglory | 3C,8H | 3C,8H | 3C,8H | 3C,7H |
| Cocklebur | 5C,9H | 4C,8H | 3C,9H | 4C,9H |
| Sicklepod | 4C,8H | 3C,7G | 3C,8H | 4C,5G |
| Nutsedge | 2C,9G | 2C,9G | 3C,9G | 3C,8G |
| Sugar beet | 9C | 5C,9G | 9C | 9H |
| PREEMERGENCE | | | | |
| Cotton | 9G | 8G | 9G | 7G |
| Sorghum | 3C,9G | 3C,5G | 3C,8H | 2C,5G |
| Corn | 2C,9G | 3C,7H | 3C,9H | 3C,7G |
| Soybean | 3C,7G | 3C,6G | 4C,8H | 3C,6H |
| Wheat | 2G | 0 | 2C,7G | 3G |
| Wild Oats | 0 | 0 | 3C,8G | 0 |
| Rice | 9H | 5G | 4C,8H | 2C,5G |
| Barnyardgrass | 8H | 3C,6H | 3C,8H | 0 |
| Morningglory | 9G | 9G | 9G | 3C,8H |
| Cocklebur | 9H | 7H | - | 3C,8H |
| Sicklepod | 8G | 4C,8G | 4C,9G | 3C,5G |
| Nutsedge | 10E | 5G | 10E | 0 |
| Sugar beet | 4C,9G | 5C,9G | 5C,9G | 3C,9G |

Table A (continued)

| | Compound 79 | |
|---|---|---|
| Rate g/ha | 2000 | 400 |
| POSTEMERGENCE | | |
| Cotton | 3C,9H | 3C,5H |
| Sorghum | 0 | 0 |
| Corn | 0 | 0 |
| Soybean | 0 | 0 |
| Wheat | 0 | 0 |
| Wild Oats | 0 | 0 |
| Rice | 3C,5G | 0 |
| Barnyardgrass | 0 | 0 |
| Morningglory | 0 | 0 |
| Cocklebur | 0 | 0 |
| Sicklepod | 0 | 0 |
| Nutsedge | 0 | 0 |
| Sugar beet | 0 | 0 |
| PREEMERGENCE | | |
| Cotton | 2G | 0 |
| Sorghum | 0 | 0 |
| Corn | 2G | 0 |
| Soybean | 2C | 0 |
| Wheat | 0 | 0 |
| Wild Oats | 0 | 0 |
| Rice | 2C | 0 |
| Barnyardgrass | 0 | 0 |
| Morningglory | 2C | 0 |
| Cocklebur | 0 | 0 |
| Sicklepod | 2C | 0 |
| Nutsedge | 0 | 0 |
| Sugar beet | 5G | 0 |

Test B

Postemergence

Two round pans (25 cm diameter by 12.5 cm deep) were filled with Woodstown sandy loam soil. One pan was planted with blackgrass (Alopacurus myosuroides), sugar beets, nutsedge (Cyperus rotundus) tubers, crabgrass (Digitaria sanguinalis), sicklepod (Cassia obtusifolia), teaweed (Sida spinosa), jimsonweed (Datura stramonium), velvetleaf (Abutilon theophrasti), and giant foxtail (Setaria faberii). The other pan was planted with wheat, cotton, rice, corn, soybean, wild oats (Avena fatua), cocklebur (Xantium pensylvanicum), morningglory (Ipomoea hederacea), johnsongrass (Sorghum halepense) and barnyardgrass (Echinochloa crusgalli). The plants were grown for approximately fourteen days, then sprayed post-emergence with the chemicals dissolved in a non-phytotoxic solvent.

Pre-emergence

Two round pans (25 cm diameter by 12.5 cm deep) were filled with Woodstown sandy loam soil. One pan was planted with blackgrass (Alopacurus myosuroides), sugar beets, nutsedge, crabgrass, sicklepod, teaweed, jimsonweed, velvetleaf, and giant foxtail. The other pan was planted with wheat, cotton, rice, corn, soybeans, wild oats, cocklebur, morningglory, johnsongrass, and barnyardgrass. The two pans were sprayed pre-emergence with the chemicals dissolved in a non-phytotoxic solvent.

Treated plants and controls were maintained in the greenhouse for 28 days, then all treated plants were

compared to controls and visually rated for plant response utilizing the rating system as described for Test A. The data are summarized in Table B.

The high herbicidal activity and the tolerance of wheat as observed in Test A were confirmed.

**Table B**

**Compound 1**

| POST-EMERGENCE Rate g/ha | 62 | 16 | 4 | 1 |
|---|---|---|---|---|
| Corn | 10G | 8G | 2G | 0 |
| Wheat | 2G | 0 | 0 | 0 |
| Rice | 10G | 7G | 3G | 0 |
| Soybean | 10G | 10G | 8G | 2G |
| Cotton | 10G | 10G | 4G | 0 |
| Sugar beet | 10G | 10G | 7G | 3G |
| Crabgrass | 6G | 5G | 2G | 0 |
| Johnsongrass | 10G | 6G | 3G | 0 |
| Blackgrass | 7G | 4G | 0 | 0 |
| Barnyardgrass | 10G | 9G | 3G | 0 |
| Nutsedge | 10G | 10G | [illegible]G | 0 |
| Giant Foxtail | 10G | 5G | 2G | 0 |
| Wild Oats | 2G | 0 | 0 | 0 |
| Cocklebur | 10G | 9G | 4G | 0 |
| Morningglory | 9G | 2G | 0 | 0 |
| Teaweed | 10G | 10G | 3G | 0 |
| Sicklepod | 10G | 5G | 2G | 0 |
| Jimsonweed | 7G | 4G | 0 | 0 |
| Velvetleaf | 10G | 10G | 4G | 2G |

| PRE-EMERGENCE Rate g/ha | 250 | 62 | 16 | 4 |
|---|---|---|---|---|
| Corn | 10G | 7G | 3G | 0 |
| Wheat | 3G | 0 | 0 | 0 |
| Rice | 10E | 10G | 8G | 7G |
| Soybean | 10G | 8G | 7G | 3G |
| Cotton | 10G | 9G | 5G | 2G |
| Sugar beet | 10G | 10G | 7G | 4G |
| Crabgrass | 6G | 2G | 0 | 0 |
| Johnsongrass | 10G | 9G | 8G | 3G |
| Blackgrass | 10G | 8G | 4G | 2G |
| Barnyardgrass | 9G | 7G | 2G | 0 |
| Nutsedge | 10E | 9G | 6G | 2G |
| Giant Foxtail | - | 6G | 2G | 0 |
| Wild Oats | 5G | 2G | 0 | 0 |
| Cocklebur | 10G | 8G | 3G | 0 |
| Morningglory | 5G | 3G | 0 | 0 |
| Teaweed | 10G | 5G | 2G | 0 |
| Sicklepod | 9G | 4G | 2G | 0 |
| Jimsonweed | 10G | 5G | 2G | 0 |
| Velvetleaf | 10G | 8G | 3G | 0 |

Table B (continued)

Compound 2

| POST-EMERGENCE Rate g/ha | 62 | 16 | 4 | 1 |
|---|---|---|---|---|
| Corn | 10G | 9G | 7G | 2G |
| Wheat | 2G | 0 | 0 | 0 |
| Rice | 9G | 5G | 2G | 0 |
| Soybean | 10G | 10G | 10G | 8G |
| Cotton | 10G | 10G | 10G | 5G |
| Sugar beet | 10G | 10G | 10G | 6G |
| Crabgrass | 5G | 4G | 3G | 0 |
| Johnsongrass | 10G | 8G | 5G | 0 |
| Blackgrass | 5G | 0 | 0 | 0 |
| Barnyardgrass | 9G | 8G | 3G | 0 |
| Nutsedge | 10G | 10G | 10G | 9G |
| Giant Foxtail | 7G | 3G | 0 | 0 |
| Wild Oats | 2G | 0 | 0 | 0 |
| Cocklebur | 10G | 10G | 7G | 2G |
| Morningglory | 10G | 10G | 6G | 2G |
| Teaweed | 6G | 3G | 0 | 0 |
| Sicklepod | 10G | 10G | 6G | 0 |
| Jimsonweed | 9G | 4G | 2G | 0 |
| Velvetleaf | 10G | 10G | 9G | 2G |

| PRE-EMERGENCE Rate g/ha | 250 | 62 | 16 | 4 |
|---|---|---|---|---|
| Corn | 10G | 8G | 5G | 0 |
| Wheat | 3G | 0 | 0 | 0 |
| Rice | 10G | 9G | 7G | 4G |
| Soybean | 10G | 9G | 5G | 2C |
| Cotton | 10G | 10G | 9G | 6G |
| Sugar beet | 10G | 10G | 8G | 4G |
| Crabgrass | 7G | 3G | 0 | 0 |
| Johnsongrass | 10G | 10G | 8G | 3G |
| Blackgrass | 10G | 8G | 4G | 0 |
| Barnyardgrass | 10G | 6G | 5G | 2G |
| Nutsedge | 10E | 9G | 4G | 0 |
| Giant Foxtail | 10G | 6G | - | 2G |
| Wild Oats | 2G | 0 | 0 | 0 |
| Cocklebur | 9G | 8G | 5G | 2G |
| Morningglory | 8G | 4G | 0 | 0 |
| Teaweed | 10G | 7G | 3G | 0 |
| Sicklepod | 8G | 5G | 0 | 0 |
| Jimsonweed | 10G | 8G | 3G | 0 |
| Velvetleaf | 10G | 9G | 6G | 3G |

**Table B (continued)**

**Compound 3**

| POST-EMERGENCE Rate g/ha | 62 | 16 | 4 | 1 |
|---|---|---|---|---|
| Corn | 8G | 4G | 0 | 0 |
| Wheat | 0 | 0 | 0 | 0 |
| Rice | 2G | 0 | 0 | 0 |
| Soybean | 10G | 10G | 10G | 4G |
| Cotton | 10G | 10G | 9G | 2G |
| Sugar beet | 10G | 10G | 10G | 8G |
| Crabgrass | 6G | 2G | 0 | 0 |
| Johnsongrass | 8G | 4G | 2G | 0 |
| Blackgrass | 0 | 0 | 0 | 0 |
| Barnyardgrass | 9G | 4G | 2G | 0 |
| Nutsedge | 10G | 10G | 10G | 9G |
| Giant Foxtail | 5G | 2G | 0 | 0 |
| Wild Oats | 0 | 0 | 0 | 0 |
| Cocklebur | 10G | 6G | 2G | 0 |
| Morningglory | 9G | 7G | 3G | 0 |
| Teaweed | 6G | 4G | 0 | 0 |
| Sicklepod | 10G | 8G | 4G | 0 |
| Jimsonweed | 7G | 4G | 0 | 0 |
| Velvetleaf | 10G | 10G | 9G | 3G |

| PRE-EMERGENCE Rate g/ha | 250 | 62 | 16 | 4 |
|---|---|---|---|---|
| Corn | 10G | 4G | 0 | 0 |
| Wheat | 0 | 0 | 0 | 0 |
| Rice | 10G | 8G | 6G | 3G |
| Soybean | 10G | 8G | 4G | 2C |
| Cotton | 9G | 10G | 8G | 2G |
| Sugar beet | 10G | 10G | 8G | 5G |
| Crabgrass | 6G | 3G | 2G | 0 |
| Johnsongrass | 9G | 10G | 4G | 0 |
| Blackgrass | 5G | 2G | 0 | 0 |
| Barnyardgrass | 9G | 6G | 4G | 0 |
| Nutsedge | 10G | 9G | 8G | 3G |
| Giant Foxtail | 9G | 6G | - | 0 |
| Wild Oats | 0 | 0 | 0 | 0 |
| Cocklebur | 8G | 5G | 4G | 2G |
| Morningglory | 9G | 3G | 0 | 0 |
| Teaweed | 10G | 9G | 5G | 2G |
| Sicklepod | 9G | 3G | 0 | 0 |
| Jimsonweed | 10G | 9G | 6G | 2G |
| Velvetleaf | 10G | 10G | 5G | 0 |

Test C

Two plastic pans with polyethylene liners were filled with prepared Sassafras sandy loam soil. One pan was planted with seeds of wheat (Triticum aestivum), barley (Hordeum vulgare), wild oats (Avena fatua), cheatgrass (Bromus secalinus), blackgrass (Alopecurus myosuroides), annual bluegrass (Poa annua), green foxtail (Setaria viridis), Italian ryegrass (Lolium multiflorum) and rapeseed (Brassia napus). The other pan was planted with seeds of Russian thistle (Salsola kali), cleavers (Galium aparine), speedwell (Veronica persica), kochia (Kochia scoparia), shepherspurse (Capsella bursa-pastoris), (Matricaria inodora), black nightshade (Solanum nigrum), wild buckwheat (Polygonum convolvulus) and sugar beets (Beta vulgaris). The above two pans were treated pre-emergence. At the same time two pans in which the above plant

species were already growing were treated post-emergence. Plant heights at the time of treatment ranged from 1-20 cm depending on plant species.

Compound #3 was diluted with a non-phytotoxic solvent and sprayed over the top of the pans. An untreated control and a solvent alone control were included for comparison. All treatments were maintained in the greenhouse for 19-22 days at which time the treatments were compared to the controls and the effects visually rated. The recorded data are presented in Table C. Compound #3 has high herbicidal activity at rates of application which are non-injurious to wheat and barley.

## Table C

**POST-EMERGENCE ON**

**SASSAFRAS SANDY LOAM SOIL**

| | Compound 3 | | | |
|---|---|---|---|---|
| Rate kg/ha | 0.125 | 0.06 | 0.03 | 0.015 |
| Wheat | 0 | 0 | 0 | 0 |
| Barley | 0 | 0 | 0 | 0 |
| Wild Oats | 0 | 0 | 0 | 0 |
| Cheatgrass | 6G | 3G | 2G | 0 |
| Blackgrass | 0 | 0 | 0 | 0 |
| Annual Bluegrass | 2G | 2G | 0 | 0 |
| Green Foxtail | 8G | 8G | 6G | 6G |
| Italian Ryegrass | 3G | 2G | 0 | 0 |
| Rapeseed | 10C | 10C | 9G | 8G |
| Matricaria inodora | 9G | 9G | 9G | 9G |
| Galium | 10C | 10C | 10C | 10C |
| Russian Thistle | 10C | 10C | 10C | 7G |
| Shepherdspurse | 8G | 8G | 8G | 6G |
| Kochia | 7G | 4G | 0 | 0 |
| Black Nightshade | 4G | 3G | 2G | 0 |
| Speedwell | 7G | 6G | 0 | 0 |
| Wild Buckwheat | 10G | 10C | 10C | 7G |
| Sugarbeet | 10C | 10C | 10C | 9G |

Table C (continued)

PRE-EMERGENCE ON
SASSAFRAS SANDY LOAM SOIL

Compound 3

| Rate kg/ha | 0.125 | 0.06 | 0.03 | 0.015 |
|---|---|---|---|---|
| Wheat | 0 | 0 | 0 | 0 |
| Barley | 1G | 0 | 0 | 0 |
| Wild Oats | 3G | 3G | 0 | 0 |
| Cheatgrass | 9G | 8G | 7G | 5G |
| Blackgrass | 6G | 5G | 5G | 4G |
| Annual Bluegrass | 5G | 4G | 4G | 3G |
| Green Foxtail | 9G | 8G | 7G | 7G |
| Italian Ryegrass | 6G | 6G | 6G | 4G |
| Rapeseed | 10C | 10C | 10C | 9G |
| Matricaria inodora | 9G | 8G,5C | 8G,5C | 8G,5C |
| Galium | 10C | 9G | 8G | 7G |
| Russian Thistle | 10C | 10C | 10C | 10C |
| Shepherdspurse | 9G,8C | 9G,8C | 9G,3C | 9G,3C |
| Kochia | 10C | 10C | 10C | 10C |
| Black Nightshade | 8G | 7G | 7G | 7G |
| Speedwell | 9G | 8G,5C | 7G | 6G,3C |
| Wild Buckwheat | 8G | 8G | 7G | 6G |
| Sugarbeet | 10C | 10C | 10C | 9G,7C |

## Claims

1. A compound of Formula Ia, Ib, Ic, Id or Ie

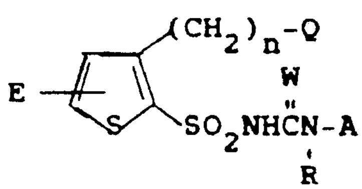

Ia

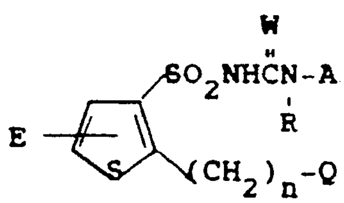

Ib

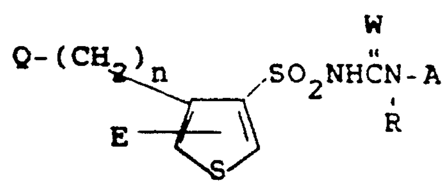

Ic

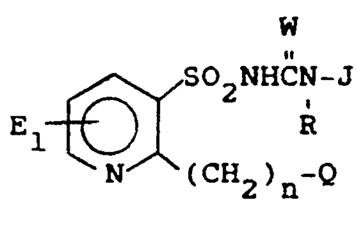

Id

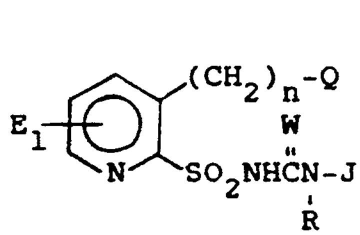

Ie

wherein

R is H or $CH_3$;

n is 0, 1 or 2;

W is O or S;

Q is a saturated 5- or 6-membered ring containing 1 heteroatom selected from sulfur, oxygen or nitrogen, or an unsaturated 5- or 6-membered ring containing 1-3 heteroatoms selected from 0-1 sulfur, 0-1 oxygen or 0-3 nitrogen; in compounds of Formulae Ia-Ic, Q may optionally be substituted by one or more groups selected from SH, $C_1$-$C_4$ alkyl, $C_3$-$C_4$ alkenyl, $C_1$-$C_3$ haloalkyl, halogen, $C_1$-$C_3$ alkoxy, $C_1$-$C_4$ alkylthio, $C_3$-$C_4$ alkenylthio, $C_3$-$C_4$ alkenyloxy, $C_1$-$C_2$ haloalkoxy, $C_1$-$C_2$ haloalkylthio, $C_3$-$C_4$ alkynylthio, $C_1$-$C_4$ cyanoalkylthio, $C_1$-$C_2$ alkoxycarbonylmethylthio or $C_1$-$C_2$ alkylcarbonylmethylthio; in compounds of Formulae Id and Ie, Q may optionally be substituted by one or more groups selected from $C_1$-$C_4$ alkyl, halogen, $C_3$-$C_4$ alkenyl, $C_1$-$C_3$ alkoxy, $C_1$-$C_3$ alkylthio, $C_3$-$C_4$ alkenylthio, $C_1$-$C_2$ haloalkoxy or $C_1$-$C_2$ haloalkylthio;

E is H, $C_1$-$C_2$ alkyl $C_1$-$C_2$ alkoxy, halogen, $NO_2$, $C_1$-$C_2$ haloalkyl, $C_1$-$C_2$ alkylthio, $C_1$-$C_2$ alkylsulfonyl, $C_1$-$C_2$ alkoxycarbonyl, $C_1$-$C_2$ dialkylaminosulfamoyl;

$E_1$ is H, Cl, Br, $CH_3$ or $SCH_3$;

A is

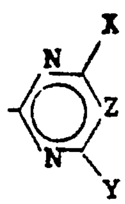 ,

A-1

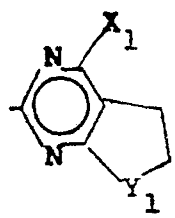 ,

A-2

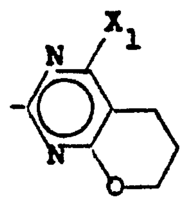 ,

A-3

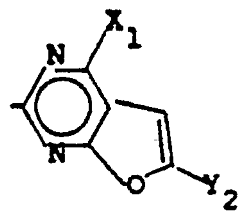

A-4

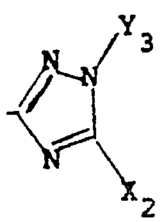

A-5

or

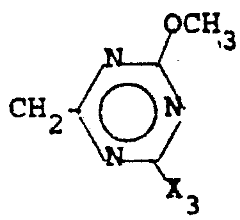 ;

A-6 .

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino or di($C_1$-$C_3$ alkyl)amino;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_2$-$C_5$ alkylsulfinylalkyl, $C_2$-$C_5$ alkylsulfonylalkyl, $C_1$-$C_4$ haloalkyl, $C_3$-$C_5$ cycloalkyl, $C_2$-$C_4$ alkynyl, $C(O)R_c$,

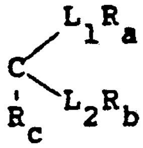 , 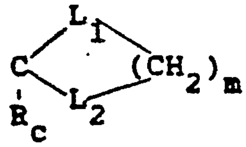 , 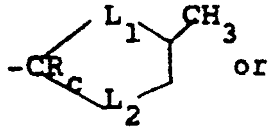 or $N(OCH_3)CH_3$;

m is 2 or 3;

$L_1$ and $L_2$ are independently O or S;

$R_a$ and $R_b$ are independently $C_1$-$C_2$ alkyl;

$R_c$ is H or $CH_3$;
Z is CH or N;
J is

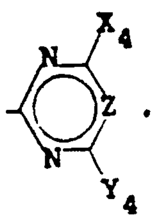

J-1

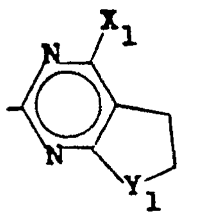

J-2

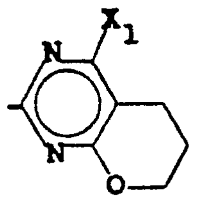

J-3

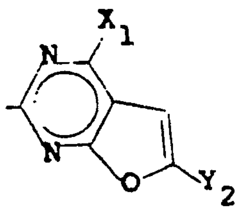

J-4

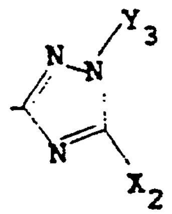

J-5

or

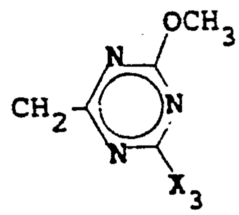

J-6

;

$Y_1$ is O or $CH_2$;
$X_1$ is $CH_3$, $OCH_3$, $OC_2H_5$ or $OCF_2H$;
$Y_2$ is H or $CH_3$;
$X_2$ is $CH_3$, $OCH_3$ or $SCH_3$;
$Y_3$ is $CH_3$, $CH_2CH_3$ or $CH_2CF_3$; and
$X_3$ is $CH_3$ or $OCH_3$;
$X_4$ is $CH_3$, $OCH_3$, $OC_2H_5$, Cl, F, Br, I, $OCF_2H$, $CH_2F$, $OCH_2CH_2F$, $OCH_2CHF_2$, $OCH_2CF_3$ or $CF_3$;
$Y_4$ is H, $CH_3$, $OCH_3$, $OC_2H_5$, $CH_2OCH_3$, $NHCH_3$, $N(OCH_3)CH_3$, $N(CH_3)_2$, $CH_2CH_3$, $CF_3$, $SCH_3$, $OCH_2CH=CH_2$, $OCH_2C{\equiv}CH$, $CH_2OC_2H_5$, $OCH_2CH_2OCH_3$, $CH_2SCH_3$, $C(O)R_c$,

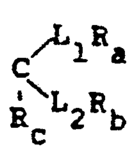,
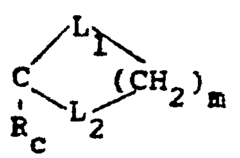,
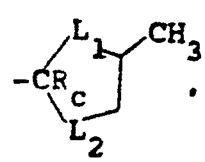, $OCF_2H$,

$SCF_2H$ or cyclopropyl;

and their agriculturally suitable salts; provided that

1) when X or $X_4$ is F, Cl, Br or I, then Z is CH, Y is $OCH_3$, $OC_2H_5$, $OCF_2H$, $NH_2$, $NHCH_3$, $N(OCH_3)$-$CH_3$ or $N(CH_3)_2$, and $Y_4$ is $OCH_3$, $OC_2H_5$, $OCF_2H$, $NHCH_3$, $N(OCH_3)CH_3$ or $N(CH_3)_2$;

2) the total number of carbon atoms of Q must be less than or equal to 8;

3) when X, Y, $X_4$ or $Y_4$ is $OCF_2H$, then Z is CH;

4) when Q is a saturated 5- or 6-membered ring containing one nitrogen atom, it is bonded to the thiophene or pyridine ring through carbon;

5) in compounds of Formulae Id and Ie, when Q is 1H-1,2,4-triazol-1-yl, then Z is CH;

6) when $Y_4$ is cyclopropyl, $X_4$ is other than Cl, F, Br or I; and 7) when W is S, then R is H, A is A-1, J is J-1, and

Y and $Y_4$ are $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$,

$CH(OCH_3)_2$ or

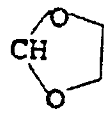 .

2. A compound of Claim 1, Formulae Ia, Ib and Ic, wherein
Q is

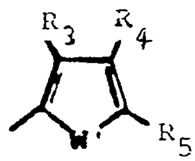

**Q-2**

,

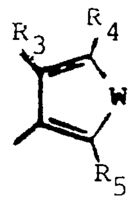

**Q-3**

,

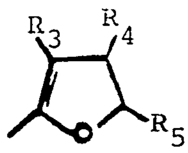

**Q-4**

,

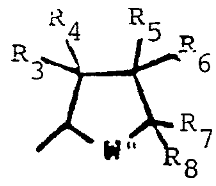

**Q-5**

,

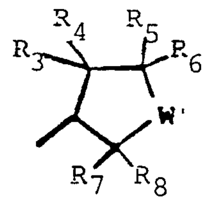

**Q-6**

,

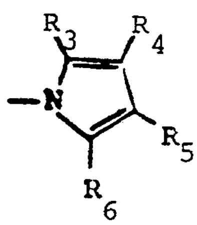

**Q-7**

,

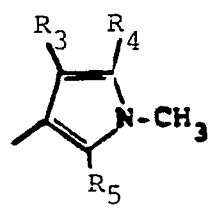

**Q-9**

,

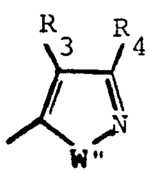

**Q-10**

,

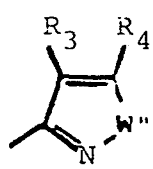

**Q-11**

,

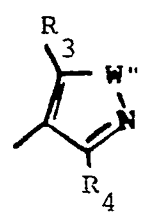

**Q-12**

,

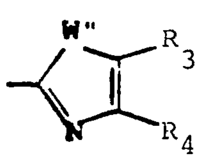

**Q-13**

,

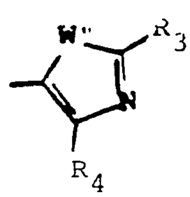

**Q-14**

,

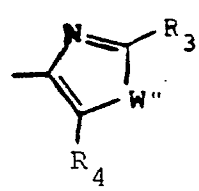

**Q-15**

,

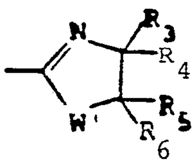

Q-16

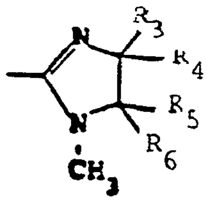

Q-17

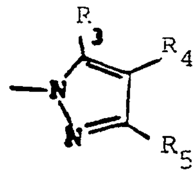

Q-18

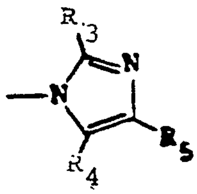

Q-19

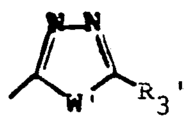

Q-20

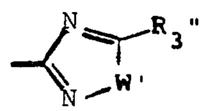

Q-21

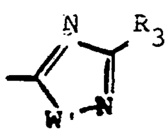

Q-22

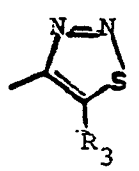

Q-23

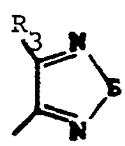

Q-24

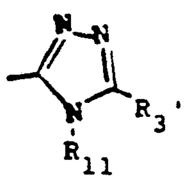

Q-25

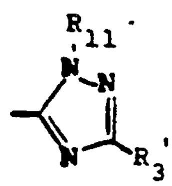

Q-26

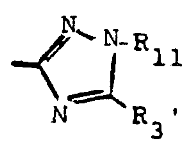

Q-27

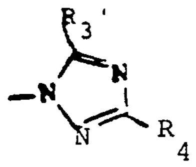 ,

Q-28

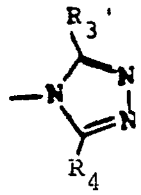 ,

Q-29

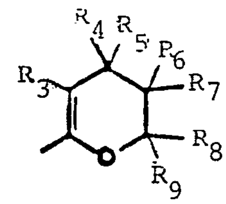

Q-30

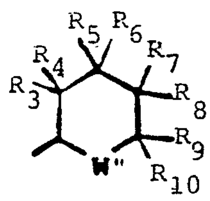 ,

Q-31

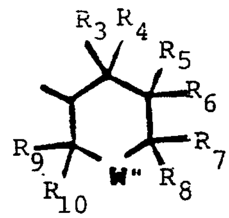 ,

Q-32

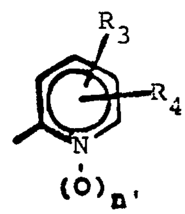 ,

Q-33

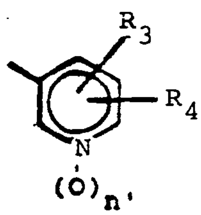 ,

Q-34

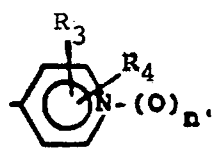 ,

Q-35

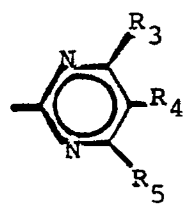

Q-36

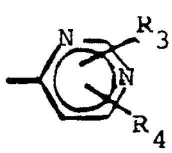 ,

Q-37

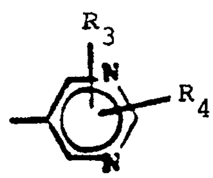 ,

Q-38

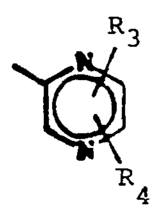

Q-39

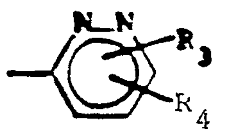 , 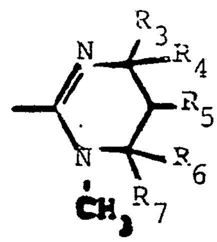 ,

Q-40 Q-41

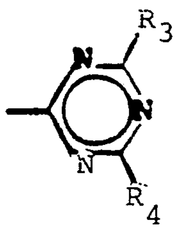 , 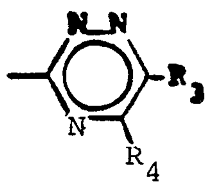 or 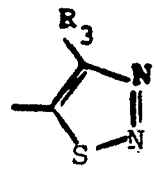 ;

Q-43 Q-44 Q-45

| | |
|---|---|
| $R_3$, $R_4$, $R_5$, $R_6$, $R_7$, $R_8$, $R_9$ and $R_{10}$ | are independently H or $CH_3$; |
| n' | is 0 or 1; |
| $R_3$' | is H, SH, $C_1$-$C_3$ alkyl, $C_1$-$C_4$ alkylthio, $C_3$-$C_4$ alkenylthio, $C_3$-$C_4$ alkynylthio, $C_1$-$C_3$ cyanoalkylthio, $SCH_2CO_2CH_3$, $SCH_2CO_2C_2H_5$, $SCH_2C(O)CH_3$, halogen, $C_1$-$C_3$ alkoxy or $OCH_2CH=CH_2$; |
| $R_3$'' | is H, $CH_3$, Cl or Br; |
| W' | is O or S; |
| W'' | is O, S or $NR_{11}$; |
| $R_{11}$ | is H, $C_1$-$C_3$ alkyl or $CH_2CH=CH_2$. |

**3.** A compound of Claim 2 wherein E is H; A is A-1; X is $CH_3$, $OCH_3$, $OC_2H_5$, Cl or Br; Y is H, $CH_3$, $OCH_3$, $C_2H_5$, $OC_2H_5$, $CH_2OCH_3$, $CF_3$, $OCF_2H$, cyclopropyl, $OCH_2CF_3$, $NHCH_3$, $N(CH_3)_2$, $CH(OCH_3)_2$or

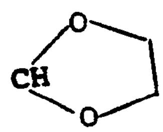

n' is O; and
$R_3$' and $R_3$'' are independently H, $CH_3$ or Cl; R is H; and W is O.

**4.** A compound of Claim 3 wherein n is O.

**5.** A compound of Claim 4 wherein Y is $C_1$-$C_2$ alkyl, $OCH_3$, or $OCF_2H$.

**6.** A compound of Claim 5 of Formula Ia.

**7.** A compound of Claim 5 of Formula Ib.

**8.** A compound of Claim 5 of Formula Ic.

**9.** A compound of Claim 6 where Q is Q-2, Q-3, Q-5, Q-7, Q-10, Q-11, Q-15, Q-16, Q-20, Q-23, Q-28, Q-36 or Q-39.

**10.** A compound of Claim 7 where Q is Q-2, Q-3, Q-5, Q-7, Q-10, Q-11, Q-15, Q-16, Q-20, Q-23, Q-28, Q-36 or Q-39.

**11.** A compound of Claim 8 where Q is Q-2, Q-3, Q-5, Q-7, Q-10, Q-11, Q-15, Q-16, Q-20, Q-23, Q-28, Q-36 or Q-39.

**12.** A compound of Claim 9 where W" is S.

**13.** A compound of Claim 10 where W" is S.

**14.** A compound of Claim 11 where W" is S.

**15.** A compound of Claim 1, Formulae <u>Id</u> and <u>Ie</u>, wherein

R is H;

W is O;

Q is selected from the group consisting of

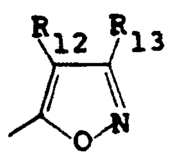

<u>Q-46</u>

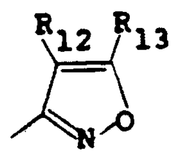

<u>Q-47</u>

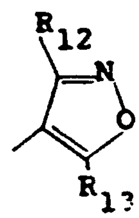

<u>Q-48</u>

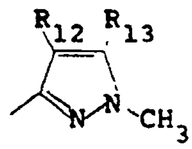,
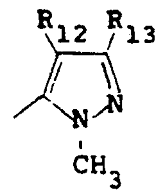,
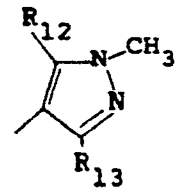,

Q-49 Q-50 Q-51

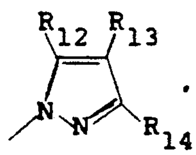,
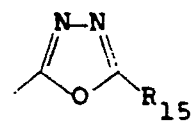,
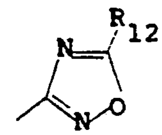,

Q-52 Q-53 Q-54

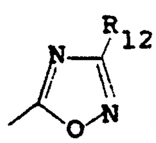,
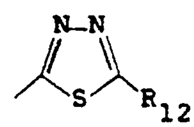,
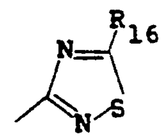,

Q-55 Q-56 Q-57

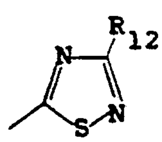,
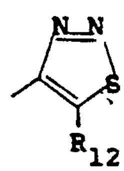,
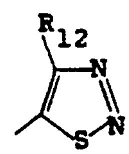,

Q-58 Q-59 Q-60

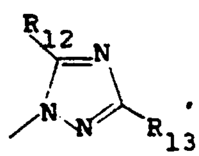,
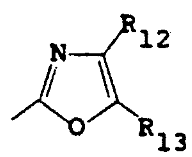,
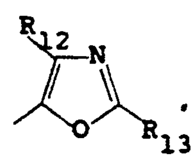,

Q-61 Q-62 Q-63

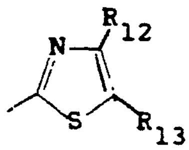, 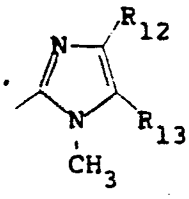, 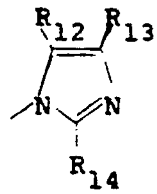, 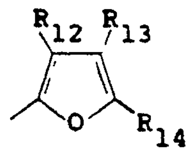,

Q-64 Q-65 Q-66 Q-67

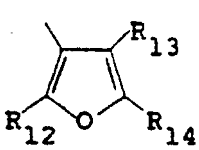, 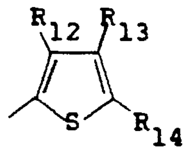, 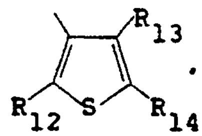, 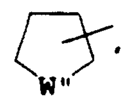,

Q-68 Q-69 Q-70 Q-71

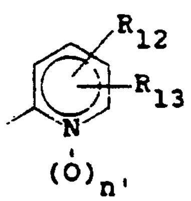, 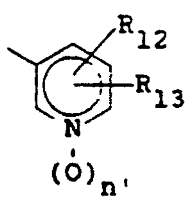, 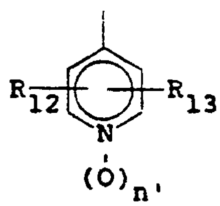, 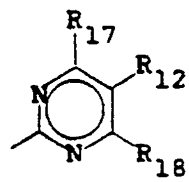,

Q-72 Q-73 Q-74 Q-75

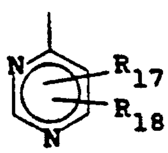, 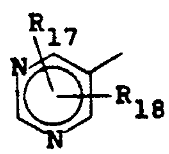, 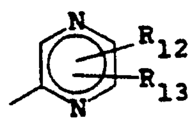,

Q-76 Q-77 Q-78

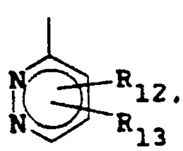, 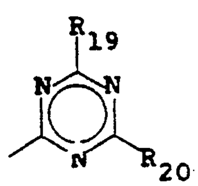, 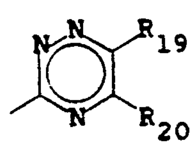,

Q-79 Q-80 Q-81

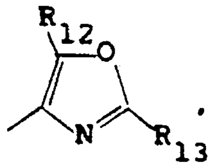 , 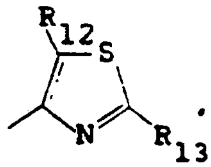 , 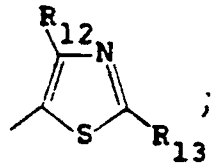 ;

Q-82 Q-83 Q-84

| | |
|---|---|
| n' | is as defined previously; |
| $R_{12}$, $R_{13}$ and $R_{14}$ | are independently H or $CH_3$; |
| $R_{15}$ | is H, $CH_3$, $C_2H_5$, $C_1$-$C_3$ alkylthio, $SCH_2CH=CH_2$, $SCF_2H$, $OCH_3$ or $OCH_2CH_3$; |
| $R_{16}$ | is H or Cl; |
| $R_{17}$ and $R_{18}$ | are independently H, $CH_3$ or $OCH_3$; |
| $R_{19}$ and $R_{20}$ | are independently $CH_3$ or $OCH_3$; |
| W" | is O, S or $NR_{11}$; and |
| $R_{11}$ | is as defined previously. |

**16.** A compound according to Claim 15 where J is J-1; $E_1$ is H; n' is O; and Q is selected from the group consisting of Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82, Q-83 and phenyl.

**17.** A compound according to Claim 16 where $X_4$ is $CH_3$, $OCH_3$, $OCH_2CH_3$ or Cl, and $Y_4$ is $CH_3$, $CH_2CH_3$, $OCH_3$, $CH(OCH_3)_2$ or $CH_2OCH_3$.

**18.** A compound according to Claim 17 where n is O.

**19.** A compound according to Claim 18 where $Y_4$ is $CH_3$, $CH_2CH_3$ or $OCH_3$.

**20.** A compound according to Claim 19 of Formula Id.

**21.** A compound according to Claim 19 of Formula Ie.

**22.** A compound according to Claim 20 where Q is Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82 and Q-83.

**23.** A compound according to Claim 21 where Q is Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82 and Q-83.

**24.** The compound of Claim 1 which is N-[(4,6-dimethylpyrimidin-2-yl)aminocarbonyl]-3-(isoxazol-3-yl)-2-thiophenesulfonamide.

**25.** The compound of Claim 1 which is 3-(isoxazol-3-yl)-N-[(4-methoxy-6-methylpyrimidin-2-yl)-aminocarbonyl]-2-thiophenesulfonamide.

**26.** The compound of Claim 1 which is N-[(4,6-dimethoxypyrimidin-2-yl)aminocarbonyl]-3-(isoxazol-3-yl)-2-thiophenesulfonamide.

**27.** The compound of Claim 1 which is 3-(5-chloro-1H-1,2,4-triazol-1-yl)-N-[(4,6-dimethoxypyrimidin-2-yl)-aminocarbonyl]-2-thiophenesulfonamide.

**28.** The compound of Claim 1 which is N-[(4-methoxy-6-methylpyrimidin-2-yl)aminocarbonyl-3-(1H-pyrrol-1-yl)-2-thiophenesulfonamide.

**29.** The compound of Claim 1 which is N-[(4-methoxy-6-methyl-1,3,5-triazin-2-yl)aminocarbonyl-3-(1H-pyrrol-1-yl)-2-thiophenesulfonamide.

**30.** The compound of Claim 1 which is N-[(4,6-dimethoxypyrimidin-2-yl)aminocarbonyl]-3-(1H-pyrrol-1-yl)-2-thiophenesulfonamide.

**31.** The compound of Claim 1 which is N-[(4,6-dimethylpyrimidin-2-yl)aminocarbonyl]-3-(1H-pyrrol-1-yl)-2-thiophenesulfonamide.

**32.** The compound of Claim 1 which is N-[(4,6-dimethoxypyrimidin-2-yl)aminocarbonyl]-2-(1H-1,2,4-triazol-1-yl)-3-pyridinesulfonamide.

**33.** The compound of Claim 1 which is N-[(4,6-dimethyl-1,3,5-triazin-2-yl)aminocarbonyl]-3-(1H-pyrrol-1-yl)-2-thiophenesulfonamide.

**34.** The compound of Claim 1 which is N-[(4,6-dimethoxy-1,3,5-triazin-2-yl)aminocarbonyl]-3-(1H-pyrrol-1-yl)-2-thiophenesulfonamide.

**35.** The compound of Claim 1 which is N-[(4-chloro-6-methoxypyrimidin-2-yl)aminocarbonyl]-3-(1H-pyrrol-1-yl)-2-thiophenesulfonamide.

**36.** The compound of Claim 1 which is N-[(4-methoxy-6-methylpyrimidin-2-yl)aminocarbonyl]-3-(2-methyl-4-thiazolyl)-2-thiophenesulfonamide.

**37.** The compound of Claim 1 which is N-[(4,6-dimethoxypyrimidin-2-yl)aminocarbonyl]-3-(2-methyl-4-thiazolyl)-2-thiophenesulfonamide.

**38.** A compound of claim 2 wherein:

n' is O;
$R_3$' is H;
$R_3$'' is H;
W'' is O.

**39.** A composition suitable for controlling the growth of undesired vegetation which comprises an effective amount of a compound of any of claims 1 to 38 and at least one of the following: surfactant, solid or liquid diluent.

**40.** A method for controlling the growth of undesired vegetation which comprises applying to the locus to be protected an effective amount of a compound of any of claims 1 to 38.

**41.** A method for regulating the growth of plants, which comprises applying to the locus of such plants an effective but substantially non-phytotoxic amount of a plant growth regulant selected from compounds of any of claims 1 to 38.

**42.** A process for the production of a compound of claim 1, which comprises:

(a) reacting a sulfonamide of formula

$BSO_2NH_2$

wherein B is selected from

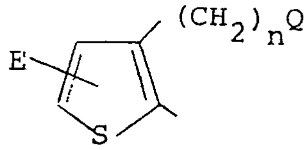
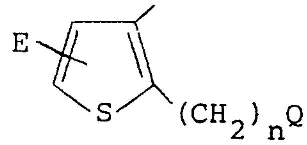
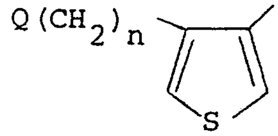

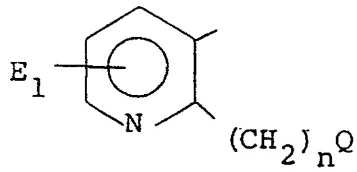
and
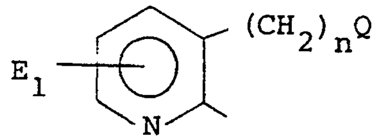

wherein E, $E_1$, Q and n are as defined in claim 1, with an appropriate methyl or phenyl carbamate of formula

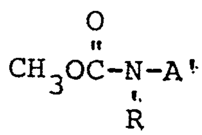

or

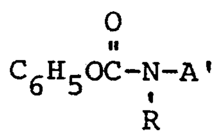

wherein R is H or $CH_3$ and A' is A or J as defined in claim 1; or
(b) reacting a sulfonylcarbamate of formula

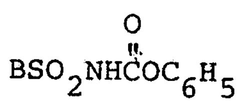

wherein B is as defined above, with a heterocyclic amine of formula

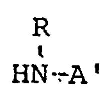

wherein R and A' are as defined above; or
(c) reacting a sulfonylisocyanate or sulfonylisothiocyanate of formula

BNCW

wherein B is as defined above and W is O or S, with said amine of formula

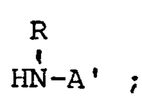

or

(d) replacing a chlorine atom in a compound of formula

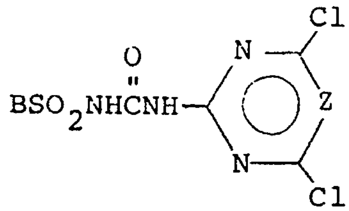

wherein B is as defined above and Z is as defined in claim 1, with $OCH_3$ by reaction with a metal methoxide and/or in either order replacing a chlorine atom by OY', where Y' is $CH_3$, $C_2H_5$ or $CH_2CF_3$, by reaction with an appropriate metal alkoxide.

**43.** Sulfonamides of formula

$BSO_2NH_2$

and phenylcarbamates thereof; and isocyanates and isothiocyanates of formula

BSCW

where B is as defined in claim 42 and W is O or S.

**44.** Compounds of formula

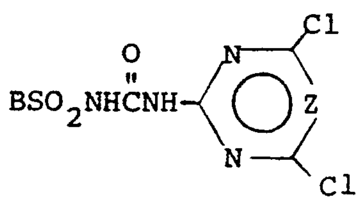

wherein B is as defined in claim 42 and Z is as defined in claim 1.

## Revendications

**1.** Un composé de Formule Ia, Ib, Ic, Id, ou Ie

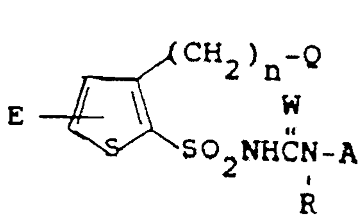

Ia

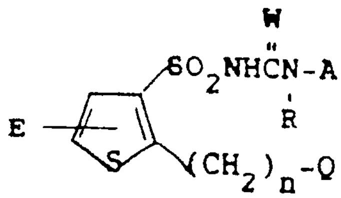

Ib

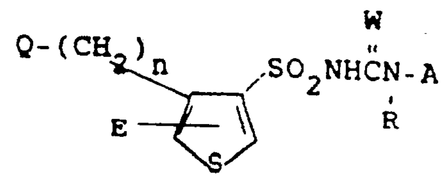

Ic

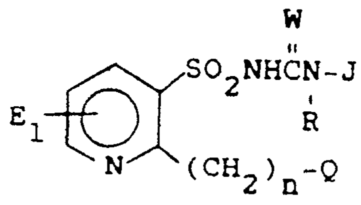

Id

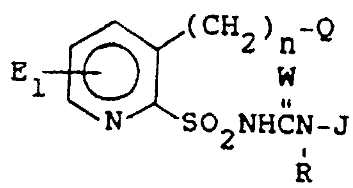

Ie

où

| | |
|---|---|
| R | est H ou $CH_3$; |
| n | est 0, 1 ou 2 ; |
| W | est O ou S ; |
| Q | est un cycle penta- ou hexagonal saturé contenant 1 hétéroatome choisi parmi le soufre, l'oxygène et l'azote, ou un cycle penta- ou hexagonal insaturé contenant 1 à 3 hétéroatomes choisis parmi 0 ou 1 atome de soufre, 0 ou 1 atome d'oxygène et 0 à 3 atomes d'azote ; dans les composés de Formules Ia à Ic, Q peut être facultativement substitué par un ou plusieurs groupes choisis parmi SH, les groupes alkyles en $C_1$-$C_4$, alcényles en $C_3$-$C_4$, halogénalkyles en $C_1$-$C_3$, halogéno, alcoxy en $C_1$-$C_3$, alkylthio en $C_1$-$C_4$, alcénylthio en $C_3$-$C_4$, alcényloxy en $C_3$-$C_4$, halogénalcoxy en $C_1$-$C_2$, halogénalkylthio en $C_1$-$C_2$, alcynylthio en $C_3$-$C_4$, cyanoalkylthio en $C_1$-$C_4$, (alcoxy en $C_1$-$C_2$)-carbonylméthylthio ou (alkyle en $C_1$-$C_2$ )carbonylméthylthio ; dans les composés de Formules Id et Ie, Q peut être facultativement substitué par un ou plusieurs groupes choisis parmi les groupes alkyles en $C_1$-$C_4$, halogéno, alcényles en $C_3$-$C_4$, alcoxy en $C_1$-$C_3$, alkylthio en $C_1$-$C_3$, alcénylthio en $C_3$-$C_4$, halogénalcoxy en $C_1$-$C_2$ ou halogénalkylthio en $C_1$-$C_2$ ; |
| E | est H, un groupe alkyle en $C_1$-$C_2$, alcoxy en $C_1$-$C_2$, halogéno, $NO_2$, halogénalkyle en $C_1$-$C_2$, alkylthio en $C_1$-$C_2$, alkylsulfonyle en $C_1$-$C_2$, alcoxycarbonyle en $C_1$-$C_2$, dialkylaminosulfamyle en $C_1$-$C_2$; |
| $E_1$ | est H, Cl, Br, $CH_3$ ou $SCH_3$ ; |
| A | est |

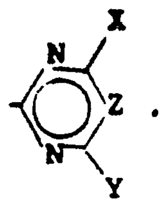, 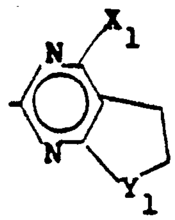, 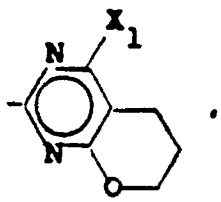, 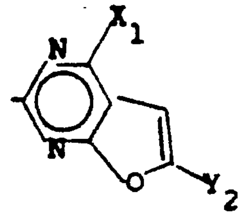,

A-1 A-2 A-3 A-4

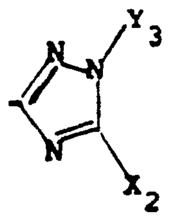 ou 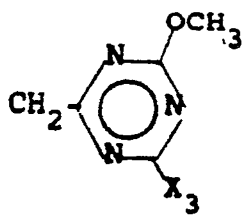 ;

A-5 A-6

X est H, un groupe alkyle en $C_1$-$C_4$, alcoxy en $C_1$-$C_4$, halogénalcoxy en $C_1$-$C_4$, halogénalkyle en $C_1$-$C_4$, halogénalkylthio en $C_1$-$C_4$, alkylthio en $C_1$-$C_4$, halogéno, alcoxyalkyle en $C_2$-$C_5$, alcoxyalcoxy en $C_2$-$C_5$, amino, alkylamino en $C_1$-$C_3$ ou di(alkyle en $C_1$-$C_3$)-amino ;

Y est H, un groupe alkyle en $C_1$-$C_4$, alcoxy en $C_1$-$C_4$, halogénalcoxy en $C_1$-$C_4$, halogénalkylthio en $C_1$-$C_4$, alkylthio en $C_1$-$C_4$, halogéno, alcoxyalkyle en $C_2$-$C_5$, alcoxyalcoxy en $C_2$-$C_5$, amino, alkylamino en $C_1$-$C_3$, di(alkyle en $C_1$-$C_3$ )amino, alcényloxy en $C_3$-$C_4$, alcynyloxy en $C_3$-$C_4$, alkylthioalkyle en $C_2$-$C_5$,alkylsulfinylalkyle en $C_2$-$C_5$, alkylsulfonylalkyle en $C_2$-$C_5$, halogénalkyle en $C_1$-$C_4$, cycloalkyle en $C_3$-$C_5$, alcynyle en $C_2$-$C_4$, C-(O)$R_c$,

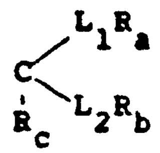, 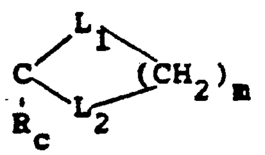, 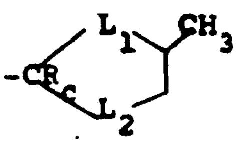 ou $N(OCH_3)CH_3$ ;

m est 2 ou 3 ;
$L_1$ et $L_2$ sont indépendamment O ou S ;
$R_a$ et $R_b$ sont indépendamment un groupe alkyle en $C_1$-$C_2$ ;
$R_c$ est H ou $CH_3$ ;
Z est CH ou N ;
J est

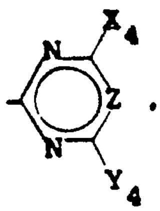 , 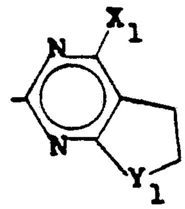 , 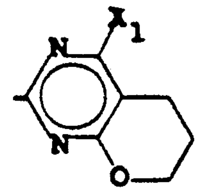 ,

J-1 J-2 J-3

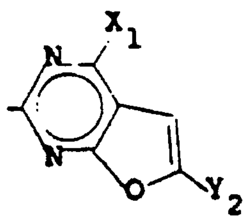 , 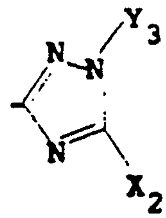 ou 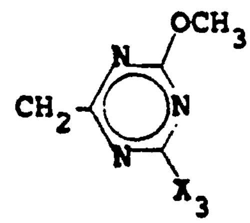 ;

J-4 J-5 J-6

| | |
|---|---|
| $Y_1$ | est O ou $CH_2$ ; |
| $X_1$ | est $CH_3$, $OCH_3$, $OC_2H_5$ ou $OCF_2H$ ; |
| $Y_2$ | est H ou $CH_3$ ; |
| $X_2$ | est $CH_3$, $OCH_3$ ou $SCH_3$ ; |
| $Y_3$ | est $CH_3$, $CH_2CH_3$ ou $CH_2CF_3$ ; et |
| $X_3$ | est $CH_3$ ou $OCH_3$ ; |
| $X_4$ | est $CH_3$, $OCH_3$, $OC_2H_5$, Cl, F, Br, I, $OCF_2H$, $CH_2F$, $OCH_2CH_2F$, $OCH_2CHF_2$, $OCH_2CF_3$ ou $CF_3$ ; |
| $Y_4$ | est H, $CH_3$, $OCH_3$, $OC_2H_5$, $CH_2OCH_3$, $NHCH_3$, $N(OCH_3)CH_3$, $N(CH_3)_2$, $CH_2CH_3$, $CF_3$, $SCH_3$, $OCH_2CH=CH_2$, $OCH_2C\equiv CH$, $CH_2OC_2H_5$, $OCH_2CH_2OCH_3$, $CH_2SCH_3$, $C(O)R_c$, |

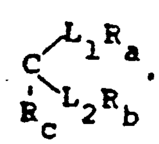 , 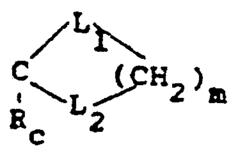 , 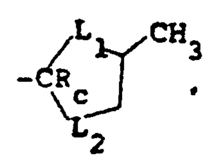 , $OCF_2H$,

$SCF_2H$ ou le groupe cyclopropyle ;

et ses sels utilisables en agriculture ; avec les conditions suivantes

1) si X ou $X_4$ est F, Cl, Br, ou I, alors Z est CH, Y est $OCH_3$, $OC_2H_5$, $OCF_2H$, $NH_2$, $NHCH_3$, $N(OCH_3)CH_3$ ou $N(CH_3)_2$ et $Y_4$ est $OCH_3$, $OC_2H_5$, $OCF_2H$, $NHCH_3$, $N(OCH_3)CH_3$ ou $N(CH_3)_2$ ;

2) le nombre total d'atomes de carbone de Q doit être inférieur ou égal à 8 ;

3) si X, Y, $X_4$ ou $Y_4$ est $OCF_2H$, alors Z est CH ;

4) si Q est un cycle penta- ou hexagonal saturé contenant un atome d'azote, il est relié au noyau de thiophène ou de pyridine par un atome de carbone ;

5) dans les composés de Formules Id et Ie, si Q est un groupe 1H-1,2,4-triazole-1-yle, alors Z est CH ;

6) si $Y_4$ est un groupe cyclopropyle, $X_4$ est autre que Cl, F, Br ou I ; et

7) si W est S, alors R est H, A est A-1, J est J-1 et Y et $Y_4$ sont $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$, $OCH_2CH_2OCH_3$, $CH(OCH_3)_2$ ou

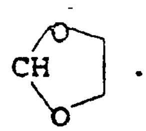

2. Un composé de la revendication 1, Formules Ia, Ib et Ic, dans lequel
Q est

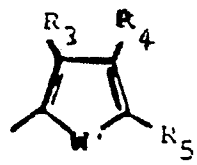 ,

Q-2

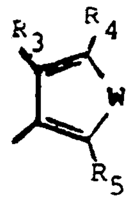 ,

Q-3

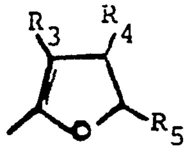 ,

Q-4

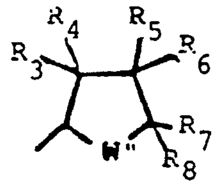 ,

Q-5

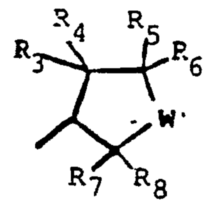 ,

Q-6

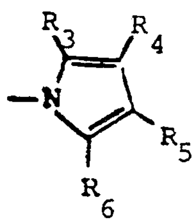 ,

Q-7

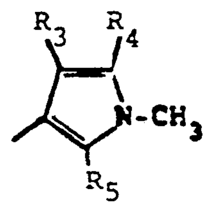 ,

Q-9

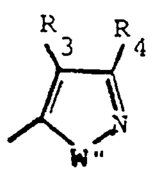 ,

Q-10

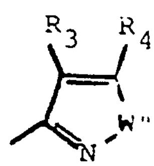 ,

Q-11

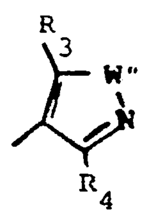 ,

Q-12

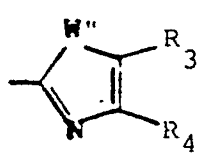 ,

Q-13

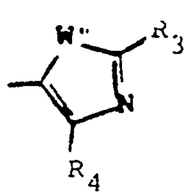 ,

Q-14

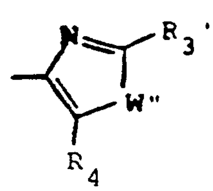 ,

Q-15

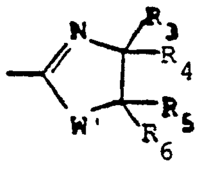 . 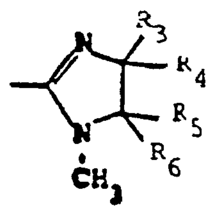 . 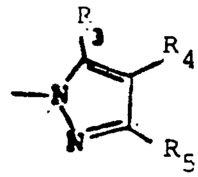 .

Q-16 Q-17 Q-18

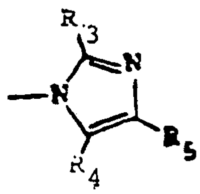 . 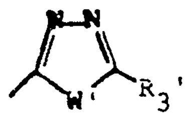 . 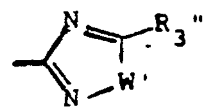 .

Q-19 Q-20 Q-21

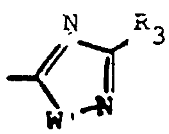 . 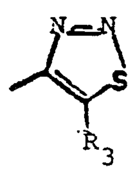 . 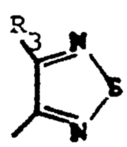

Q-22 Q-23 Q-24

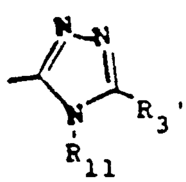 . 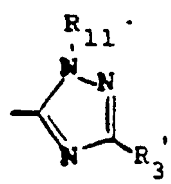 . 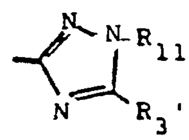 .

Q-25 Q-26 Q-27

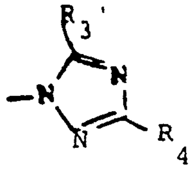 , 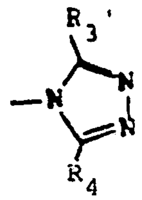 , 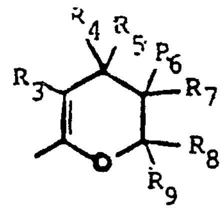

Q-28 Q-29 Q-30

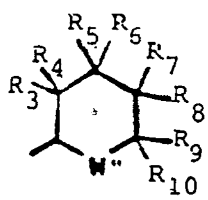 , 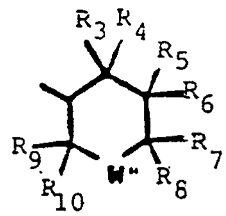 , 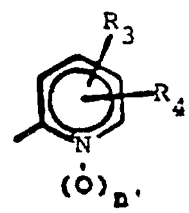 ,

Q-31 Q-32 Q-33

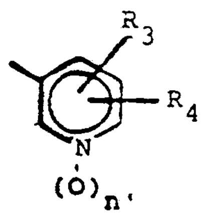 , 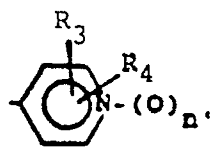 , 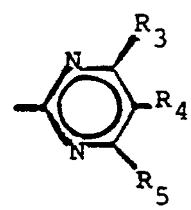

Q-34 Q-35 Q-36

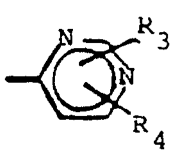 , 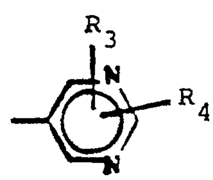 , 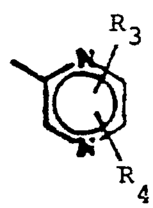

Q-37 Q-38 Q-39

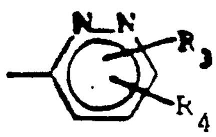 , 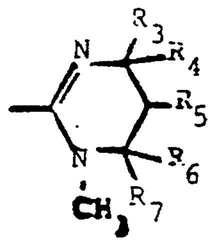 ,

Q-40 Q-41

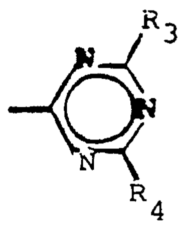 , 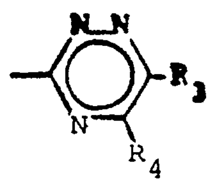 ou 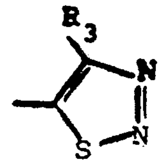 ;

Q-43 Q-44 Q-45

| | |
|---|---|
| $R_3$ , $R_4$ , $R_5$ , $R_6$ , $R_7$ , $R_8$ , $R_9$ et $R_{10}$ | sont indépendamment H ou $CH_3$ ; |
| n' | est 0 ou 1 ; |
| $R_3$' | est H, SH, un groupe alkyle en $C_1$-$C_3$, alkylthio en $C_1$-$C_4$, alcénylthio en $C_3$-$C_4$, alcynylthio en $C_3$-$C_4$, cyanoalkylthio en $C_1$-$C_3$, $SCH_2CO_2CH_3$, $SCH_2CO_2C_2H_5$, $SCH_2C(O)CH_3$, halogéno, alcoxy en $C_1$-$C_3$ ou $OCH_2CH=CH_2$ ; |
| $R_3$'' | est H, $CH_3$, Cl ou Br ; |
| W' | est O ou S ; |
| W'' | est O, S ou $NR_{11}$ ; |
| $R_{11}$ | est H, un groupe alkyle en $C_1$-$C_3$ ou $CH_2CH=CH_2$. |

3. Un composé de la revendication 2, dans lequel E est H ; A est A-1 ; X est $CH_3$, $OCH_3$, $OC_2H_5$, Cl ou Br ; Y est H, $CH_3$, $OCH_3$, $C_2H_5$, $OC_2H_5$, $CH_2OCH_3$, $CF_3$, $OCF_2H$, un groupe cyclopropyle, $OCH_2CF_3$, $NHCH_3$, $N(CH_3)_2$, $CH(OCH_3)_2$ ou

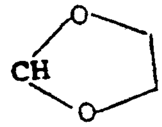

n' est 0 ; et $R_3$' et $R_3$'' sont indépendamment H, $CH_3$ ou Cl ; R est H ; et W est O.

4. Un composé de la revendication 3, dans lequel n est 0.

5. Un composé de la revendication 4, dans lequel Y est un groupe alkyle en $C_1$-$C_2$, $OCH_3$ ou $OCF_2H$.

6. Un composé de la revendication 5, de Formule Ia.

7. Un composé de la revendication 5, de Formule Ib.

8. Un composé de la revendication 5, de Formule Ic.

9. Un composé de la revendication 6, dans lequel Q est Q-2, Q-3, Q-5, Q-7, Q-10, Q-11, Q-15, Q-16, Q-20, Q-23, Q-28, Q-36 ou Q-39.

**10.** Un composé de la revendication 7, dans lequel Q est Q-2, Q-3, Q-5, Q-7, Q-10, Q-11, Q-15, Q-16, Q-20, Q-23, Q-28, Q-36 ou Q-39.

**11.** Un composé de la revendication 8, dans lequel Q est Q-2, Q-3, Q-5, Q-7, Q-10, Q-11, Q-15, Q-16, Q-20, Q-23, Q-28, Q-36 ou Q-39.

**12.** Un composé de la revendication 9, dans lequel W" est S.

**13.** Un composé de la revendication 10, dans lequel W" est S.

**14.** Un composé de la revendication 11, dans lequel W" est S.

**15.** Un composé de la revendication 1, Formules Id et Ie, dans lequel

R est H ;

W est O ;

Q est choisi dans le groupe formé par

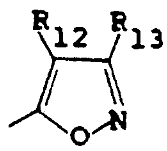

,

Q-46

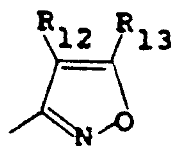

,

Q-47

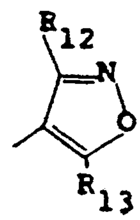

.

Q-48

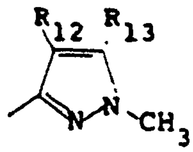
Q-49 ,

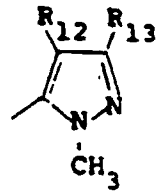
Q-50 ,

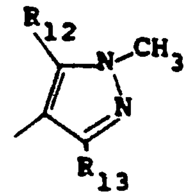
Q-51 ,

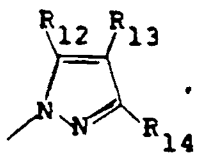
Q-52 ,

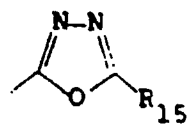
Q-53 ,

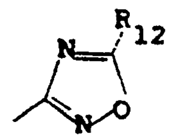
Q-54 ,

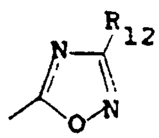
Q-55 ,

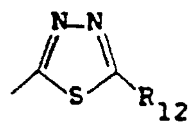
Q-56 ,

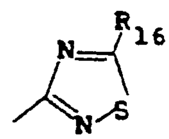
Q-57 ,

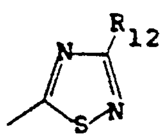
Q-58 ,

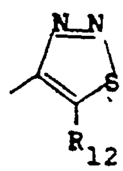
Q-59 ,

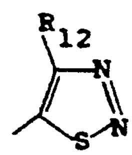
Q-60 ,

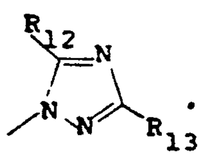
Q-61 ,

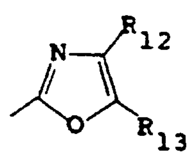
Q-62 ,

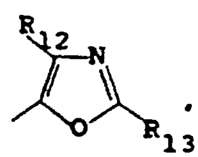
Q-63 ,

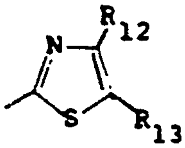
Q-64

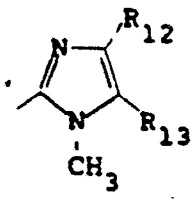
Q-65

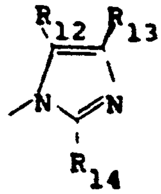
Q-66

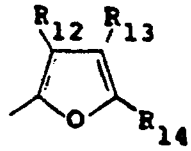
Q-67

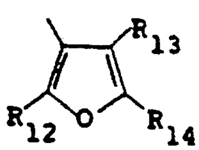
Q-68

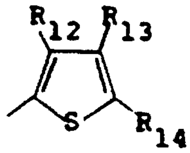
Q-69

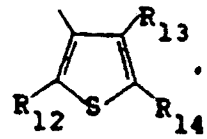
Q-70

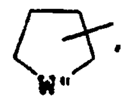
Q-71

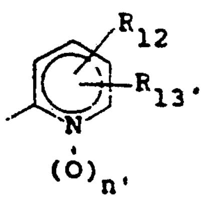
Q-72

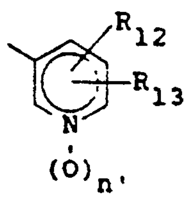
Q-73

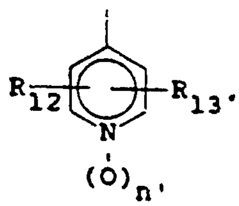
Q-74

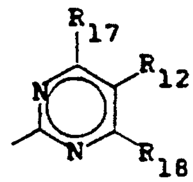
Q-75

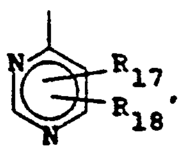
Q-76

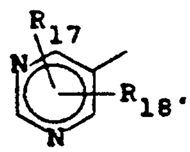
Q-77

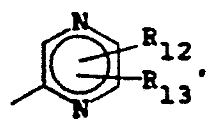
Q-78

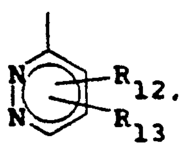
Q-79

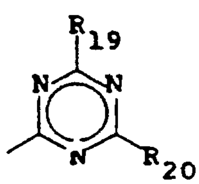
Q-80

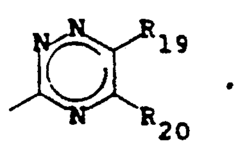
Q-81

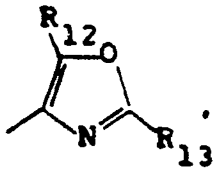 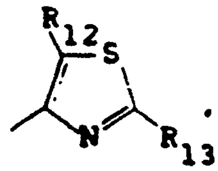 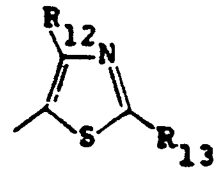

Q-82 Q-83 Q-84

| | |
|---|---|
| n' | est tel que défini précédemment ; |
| $R_{12}$, $R_{13}$ et $R_{14}$ | sont indépendamment H ou $CH_3$ ; |
| $R_{15}$ | est H, $CH_3$, $C_2H_5$, un groupe alkylthio en $C_1$-$C_3$;$SCH_2CH=CH_2$, $SCF_2H$, $OCH_3$ ou $OCH_2CH_3$ ; |
| $R_{16}$ | est H ou Cl ; |
| $R_{17}$ et $R_{18}$ | sont indépendamment H, $CH_3$ ou $OCH_3$ ; |
| $R_{19}$ et $R_{20}$ | sont indépendamment $CH_3$ ou $OCH_3$ ; |
| W" | est O, S ou $NR_{11}$ ; et |
| $R_{11}$ | est tel que défini précédemment. |

**16.** Un composé selon la revendication 15, dans lequel J est J-1 ; $E_1$ est H ; n' est 0 ; et Q est choisi dans le groupe formé par Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82, Q-83 et le groupe phényle.

**17.** Un composé selon la revendication 16, dans lequel $X_4$ est $CH_3$, $OCH_3$, $OCH_2CH_3$ ou Cl, et $Y_4$ est $CH_3$, $CH_2CH_3$, $OCH_3$, $CH(OCH_3)_2$ ou $CH_2OCH_3$.

**18.** Un composé selon la revendication 17, dans lequel n est 0.

**19.** Un composé selon la revendication 18, dans lequel $Y_4$ est $CH_3$, $CH_2CH_3$ ou $OCH_3$.

**20.** Un composé selon la revendication 19, de Formule Id.

**21.** Un composé selon la revendication 19, de Formule Ie.

**22.** Un composé selon la revendication 20, dans lequel Q est Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82 ou Q-83.

**23.** Un composé selon la revendication 21, dans lequel Q est Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82 ou Q-83.

**24.** Le composé de la revendication 1, qui est le N-[(4,6-diméthylpyrimidine-2-yl)aminocarbonyl]-3-(isoxazole-3-yl)-2-thiophène-sulfonamide.

**25.** Le composé de la revendication 1, qui est le 3-(isoxazole-3-yl)-N-[(4-méthoxy-6-méthylpyrimidine-2-yl)-aminocarbonyl]-2-thiophène-sulfonamide.

**26.** Le composé de la revendication 1, qui est le N-[(4,6-diméthoxypyrimidine-2-yl)aminocarbonyl]-3-(isoxazole-3-yl)-2-thiophène-sulfonamide.

**27.** Le composé de la revendication 1, qui est le 3-(5-chloro-1H-1,2,4-triazole-1-yl)-N-[(4,6-diméthoxypyrimidine-2-yl)aminocarbonyl]-2-thiophènesulfonamide.

**28.** Le composé de la revendication 1, qui est le N-[(4-méthoxy-6-méthylpyrimidine-2-yl)aminocarbonyl]-3-(1H-pyrrole-1-yl)-2-thiophène-sulfonamide.

**29.** Le composé de la revendication 1, qui est le N-[(4-méthoxy-6-méthyl-1,3,5-triazine-2-yl)aminocarbonyl]-3-(1H-pyrrole-1-yl)-2-thiophène-sulfonamide.

30. Le composé de la revendication 1, qui est le N-[(4,6-diméthoxypyrimidine-2-yl)aminocarbonyl]-3-(1H-pyrrole-1-yl)-2-thiophène-sulfonamide.

31. Le composé de la revendication 1, qui est le N-[(4,6-diméthylpyrimidine-2-yl)aminocarbonyl]-3-(1H-pyrrole-1-yl)-2-thiophène-sulfonamide.

32. Le composé de la revendication 1, qui est le N-[(4,6-diméthoxypyrimidine-2-yl)aminocarbonyl]-2-(1H-1,2,4-triazole-1-yl)-3-pyridine-sulfonamide.

33. Le composé de la revendication 1, qui est le N-[(4,6-diméthyl-1,3,5-triazine-2-yl)aminocarbonyl]-3-(1H-pyrrole-1-yl)-2-thiophène-sulfonamide.

34. Le composé de la revendication 1, qui est le N-[(4,6-diméthoxy-1,3,5-triazine-2-yl)aminocarbonyl]-3-(1H-pyrrole-1-yl)-2-thiophène-sulfonamide.

35. Le composé de la revendication 1, qui est le N-[(4-chloro-6-méthoxypyrimidine-2-yl)aminocarbonyl]-3-(1H-pyrrole-1-yl)-2-thiophène-sulfonamide.

36. Le composé de la revendication 1, qui est le N-[(4-méthoxy-6-méthylpyrimidine-2-yl)aminocarbonyl]-3-(2-méthyl-4-thiazolyl)-2-thiophène-sulfonamide.

37. Le composé de la revendication 1, qui est le N-[(4,6-diméthoxypyrimidine-2-yl)aminocarbonyl]-3-(2-méthyl-4-thiazolyl)-2-thiophène-sulfonamide.

38. Un composé de la revendication 2, dans lequel :

$n'$ est 0 ;
$R_3'$ est H ;
$R_3''$ est H ;
$W''$ est O.

39. Une composition utilisable pour combattre la croissance de végétation indésirable, qui comprend une quantité efficace d'un composé de l'une quelconque des revendications 1 à 38 et au moins l'un des ingrédients suivants : agent tensio-actif, diluant solide ou liquide.

40. Un procédé pour combattre la croissance de végétation indésirable, qui consiste à appliquer au lieu à protéger une quantité efficace d'un composé de l'une quelconque des revendications 1 à 38.

41. Un procédé pour réguler la croissance de plantes, qui consiste à appliquer au lieu où se trouvent ces plantes une quantité efficace, mais sensiblement non phytotoxique, d'un régulateur de croissance de plantes choisi parmi les composés de l'une quelconque des revendications 1 à 38.

42. Un procédé pour la production d'un composé de la revendication 1, qui consiste à :

(a) faire réagir un sulfonamide de formule

$BSO_2NH_2$

où B est choisi parmi

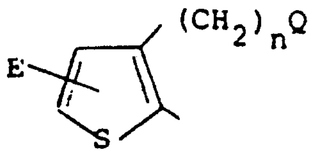
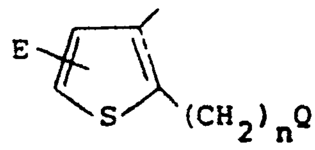
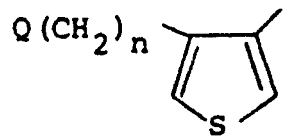

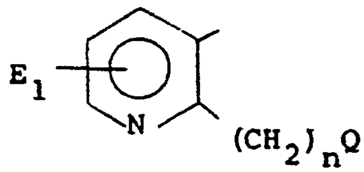

et

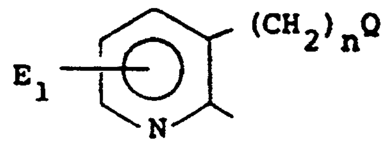

où E, $E_1$, Q et n sont tels que définis dans la revendication 1, avec un carbamate de méthyle ou de phényle approprié de formule

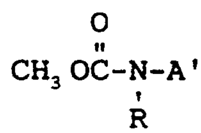

ou

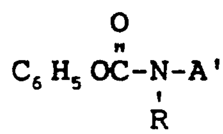

où R est H ou $CH_3$ et A' est A ou J, tels que définis dans la revendication 1 ; ou

(b) faire réagir un sulfonylcarbamate de formule

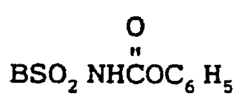

où B est tel que défini ci-dessus, avec une amine hétérocyclique de formule

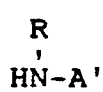

où R et A' sont tels que définis ci-dessus ; ou

(c) faire réagir un isocyanate de sulfonyle ou un isothiocyanate de sulfonyle de formule

BNCW

où B est tel que défini ci-dessus et W est O ou S, avec ladite amine de formule

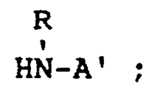

ou

(d) remplacer un atome de chlore dans un composé de formule

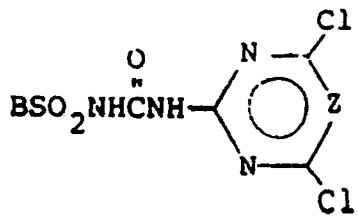

où B est tel que défini ci-dessus et Z est tel que défini dans la revendication 1, par $OCH_3$ par réaction avec un méthylate de métal et/ou, dans cet ordre ou l'autre, remplacer un atome de chlore par OY', où Y' est $CH_3$, $C_2H_5$ ou $CH_2CF_3$, par réaction avec un alcoolate de métal approprié.

**43.** Sulfonamides de formule

$BSO_2NH_2$

et leurs carbamates de phényle ; et isocyanates et isothiocyanates de formule

BSCW

où B est tel que défini dans la revendication 42 et W est O ou S.

**44.** Composés de formule

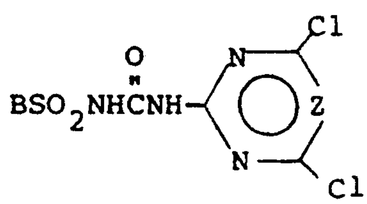

où B est tel que défini dans la revendication 42 et Z est tel que défini dans la revendication 1.

**Patentansprüche**

**1.** Verbindung der Formel Ia, Ib, Ic, Id oder Ie

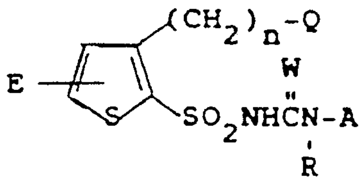

Ia

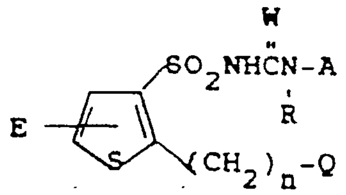

Ib

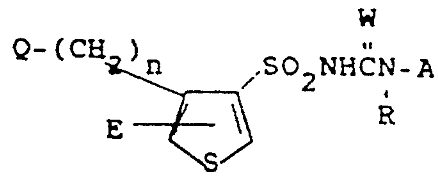

Ic

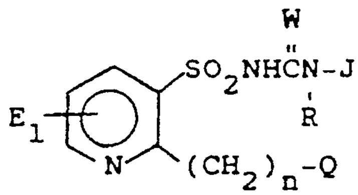

Id

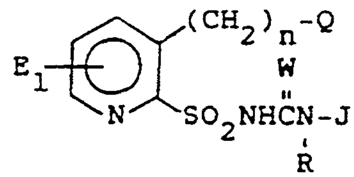

Ie

worin

R H oder $CH_3$ ist;

n 0, 1 oder 2 ist;

W O oder S ist;

Q ein gesättigter 5- oder 6-gliedriger Ring mit einem aus Schwefel, Sauerstoff oder Stickstoff ausgewählten Heteroatom oder ein ungesättigter 5- oder 6-gliedriger Ring mit 1 bis 3 aus 0 bis 1 Schwefel, 0 bis 1 Sauerstoff oder 0 bis 3 Stickstoff ausgewählten Heteroatomen ist, wobei in Verbindungen der Formeln Ia-Ic Q gegebenenfalls durch eine oder mehrere aus SH, $C_1$-$C_4$-Alkyl, $C_3$-$C_4$-Alkenyl, $C_1$-$C_3$-Halogenalkyl, Halogen, $C_1$-$C_3$-Alkoxy, $C_1$-$C_4$-Alkylthio, $C_3$-$C_4$-Alkenylthio, $C_3$-$C_4$-Alkenyloxy, $C_1$-$C_2$-Halogenalkoxy, $C_1$-$C_2$-Halogenalkylthio, $C_3$-$C_4$-Alkinylthio, $C_1$-$C_4$-Cyanalkylthio, $C_1$-$C_2$-Alkoxycarbonylmethylthio oder $C_1$-$C_2$-Alkylcarbonylmethylthioausgewählten Gruppen substituiert sein kann und in Verbindungen der Formeln Id und Ie Q gegebenenfalls durch eine oder mehrere aus $C_1$-$C_4$-Alkyl,Halogen, $C_3$-$C_4$-Alkenyl, $C_1$-$C_3$-Alkoxy, $C_1$-$C_3$-Alkylthio, $C_3$-$C_4$-Alkenylthio, $C_1$-$C_2$-Halogenalkoxy oder $C_1$-$C_2$-Halogenalkylthio ausgewählten Gruppen substituiert sein kann;

E H, $C_1$-$C_2$-Alkyl, $C_1$-$C_2$-Alkoxy, Halogen, $NO_2$, $C_1$-$C_2$-Halogenalkyl, $C_1$-$C_2$-Alkylthio, $C_1$-$C_2$-Alkylsulfonyl, $C_1$-$C_2$-Alkoxycarbonyl, $C_1$-$C_2$-Dialkylaminosulfamoyl ist;

$E_1$ H, Cl, Br, $CH_3$ oder $SCH_3$ ist;

A

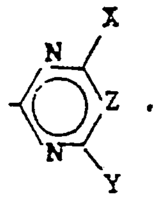 , 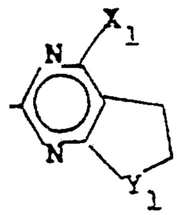 , 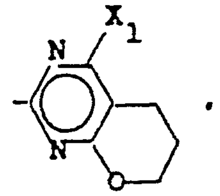 , 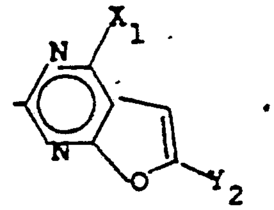 ,

A-1 A-2 A-3 A-4

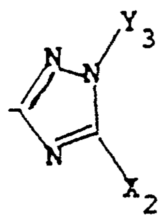 oder 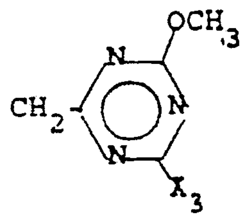 ist;

A-5 A-6

X H, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino oder Di($C_1$-$C_3$-alkyl)amino ist;

Y H, $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino, $C_3$-$C_4$-Alkenyloxy, $C_3$-$C_4$-Alkinyloxy, $C_2$-$C_5$-Alkylthioalkyl, $C_2$-$C_5$-Alkylsulfinylalkyl, $C_2$-$C_5$-Alkylsulfonylalkyl, $C_1$-$C_4$-Halogenalkyl, $C_3$-$C_5$-Cycloalkyl, $C_2$-$C_4$-Alkenyl, $C(O)R_c$,

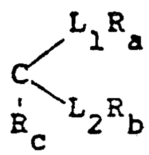 , 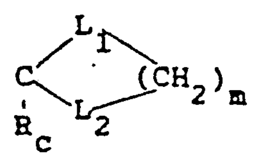 , 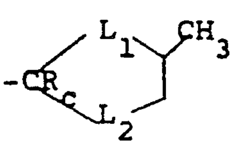

oder $N(OCH_3)CH_3$ ist;

m 2 oder 3 ist;

$L_1$ und $L_2$ unabhängig O oder S sind;

$R_a$ und $R_b$ unabhängig $C_1$-$C_2$-Alkyl sind;

$R_c$ H oder $CH_3$ ist;

Z CH oder N ist;

J

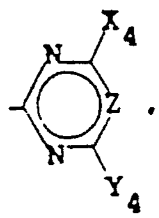, 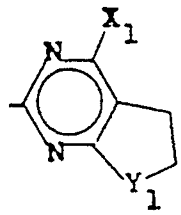, 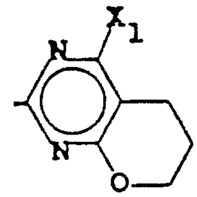,

J-1 J-2 J-3

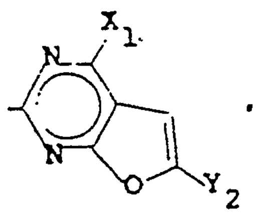, 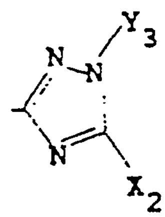 oder 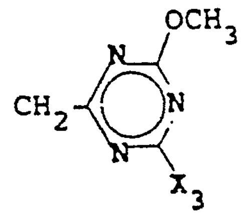 ist:

J-4 J-5 J-6

$Y_1$ O oder $CH_2$ ist;
$X_1$ $CH_3$, $OCH_3$, $OC_2H_5$ oder $OCF_2H$ ist;
$Y_2$ H oder $CH_3$ ist;
$X_2$ $CH_3$, $OCH_3$ oder $SCH_3$ ist;
$Y_3$ $CH_3$, $CH_2CH_3$ oder $CH_2CF_3$ ist; und
$X_3$ $CH_3$ oder $OCH_3$ ist;
$X_4$ $CH_3$, $OCH_3$, $OC_2H_5$, Cl, F, Br, I, $OCF_2H$, $CH_2F$, $OCH_2CH_2F$, $OCH_2CHF_2$, $OCH_2CF_3$ oder $CF_3$ ist;
$Y_4$ H, $CH_3$, $OCH_3$, $OCH_2H_5$, $CH_2OCH_3$, $NHCH_3$, $N(OCH_3)CH_3$, $N(CH_3)_2$, $CH_2CH_3$, $CF_3$, $SCH_3$, $OCH_2CH=CH_2$, $OCH_2C=CH$, $CH_2OC_2H_5$, $OCH_2CH_2OCH_3$, $CH_2SCH_3$, C(O)-$R_c$,

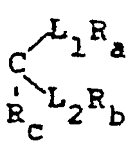, 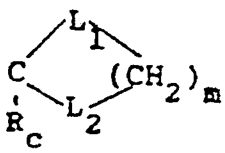, 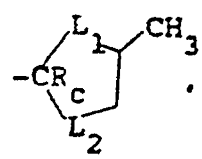, $OCF_2H$,

$SCF_2H$ oder Cyclopropyl ist;

und ihre landwirtschaftlich geeigneten Salze; mit der Maßgabe, daß

1) wenn X oder $X_4$ F, Cl, Br oder I ist, dann Z CH ist, Y $OCH_3$, $OC_2H_5$, $OCF_2H$, $NH_2$, $NHCH_3$, N-$(OCH_3)CH_3$ oder $N(CH_3)_2$ ist und $Y_4$ $OCH_3$, $OC_2H_5$, $OCF_2H$, $NHCH_3$, $N(OCH_3)CH_3$ oder $N(CH_3)_2$ ist;
2) die Gesamtzahl der Kohlenstoffatome von Q kleiner oder gleich 8 ist;
3) wenn X, Y, $X_4$ oder $Y_4$ $OCF_2H$ ist, dann Z CH ist;
4) wenn Q ein gesättigter 5- oder 6-gliedriger Ring mit einem Stickstoffatom ist, er über Kohlenstoff an den Thiophen- oder Pyridinring gebunden ist;
5) in Verbindungen der Formeln Id und Ie, wenn Q 1H-1,2,4-Triazol-1-yl ist, dann Z CH ist;
6) wenn $Y_4$ Cyclopropyl ist, $X_4$ von Cl, F, Br oder I verschieden ist und
7) wenn W S ist, dann R H ist, A A-1 ist, J J-1 ist und Y und $Y_4$ $CH_3$, $OCH_3$, $OC_2H_5$, $CH_2OCH_3$, $C_2H_5$, $CF_3$, $SCH_3$, $OCH_2CH=CH_2$, $OCH_2C≡CH$, $OCH_2CH_2OCH_3$, $CH(OCH_3)_2$ oder

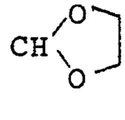

ist.

**2.** Verbindung nach Anspruch 1, Formeln Ia, Ib und Ic, worin Q

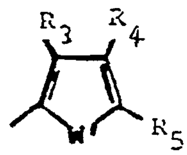

Q-2

.

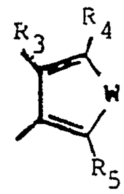

Q-3

.

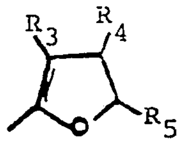

Q-4

.

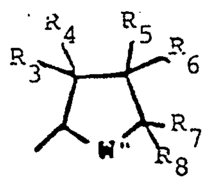

Q-5

.

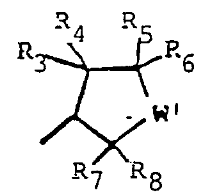

Q-6

.

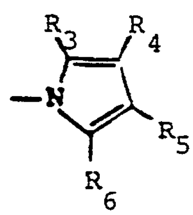

Q-7

.

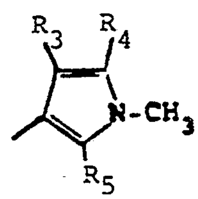

Q-9

.

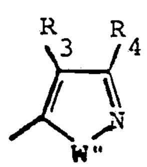

Q-10

.

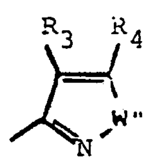

Q-11

.

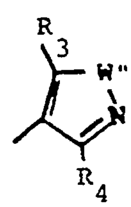

Q-12

.

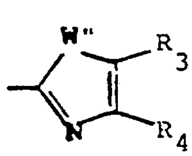

Q-13

.

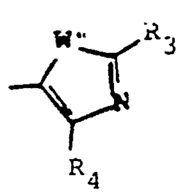

Q-14

.

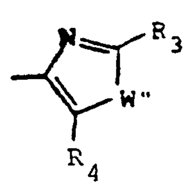

Q-15

.

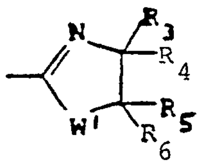 ,

Q-16

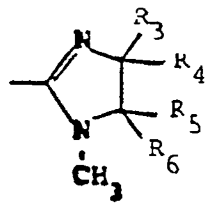 ,

Q-17

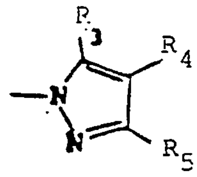 ,

Q-18

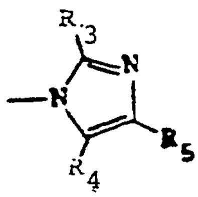 ,

Q-19

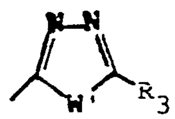 ,

Q-20

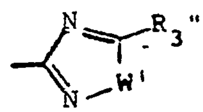 ,

Q-21

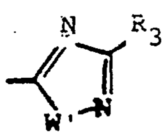 ,

Q-22

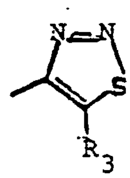 ,

Q-23

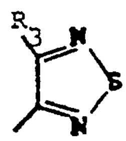

Q-24

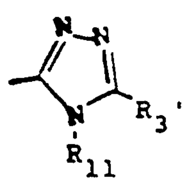 ,

Q-25

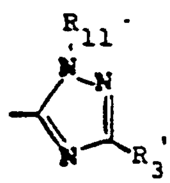 ,

Q-26

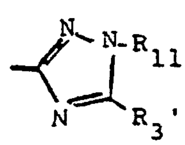 ,

Q-27

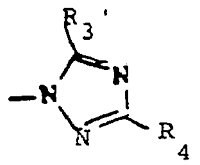 , 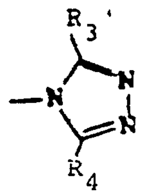 , 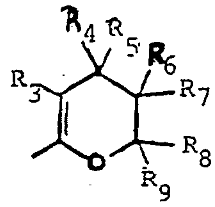

Q-28 Q-29 Q-30

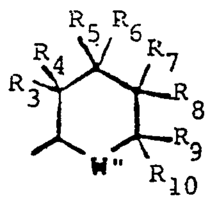 , 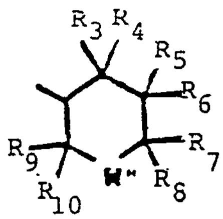 , 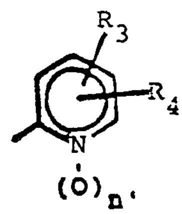 ,

Q-31 Q-32 Q-33

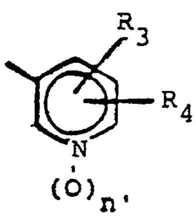 , 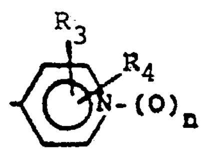 , 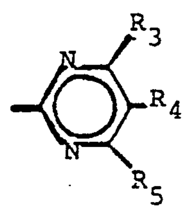

Q-34 Q-35 Q-36

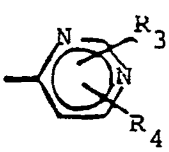 , 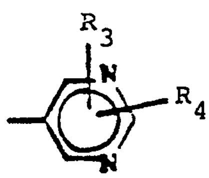 , 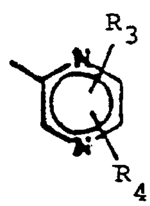

Q-37 Q-38 Q-39

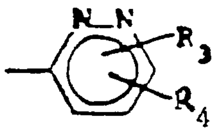

,

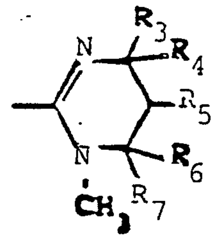

,

Q-40

Q-41

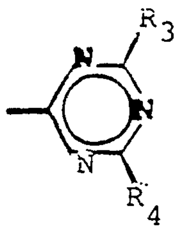

,

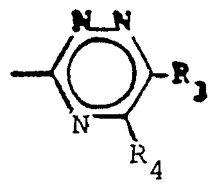

oder

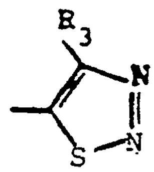

ist;

Q-43

Q-44

Q-45

| | |
|---|---|
| $R_3$, $R_4$, $R_5$, $R_6$, $R_7$, $R_8$, $R_9$ und $R_{10}$ | unabhängig H oder $CH_3$ sind; |
| n' | 0 oder 1 ist; |
| $R_3$' | H, SH, $C_1$-$C_3$-Alkyl, $C_1$-$C_4$-Alkylthio, $C_3$-$C_4$-Alkenylthio, $C_3$-$C_4$-Alkinylthio, $C_1$-$C_3$-Cyanalkylthio, $SCH_2CO_2CH_3$, $SCH_2CO_2C_2H_5$, $SCH_2C(O)CH_3$, Halogen, $C_1$-$C_3$-Alkoxy oder $OCH_2CH=CH_2$ ist; |
| $R_3$" | H, $CH_3$, Cl oder Br ist; |
| W' | O oder S ist; |
| W" | O, S oder $NR_{11}$ ist; |
| $R_{11}$ | H, $C_1$-$C_3$-Alkyl oder $CH_2CH=CH_2$ ist. |

3. Verbindung nach Anspruch 2, worin E H ist; A A-1 ist; X $CH_3$, $OCH_3$, $OC_2H_5$, Cl oder Br ist; Y H, $CH_3$, $OCH_3$, $C_2H_5$, $OC_2H_5$, $CH_2OCH_3$, $CF_3$, $OCF_2H$, Cyclopropyl, $OCH_2CF_3$, $NHCH_3$, $N(CH_3)_2$, $CH(OCH_3)_2$ oder

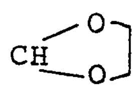

ist, n' 0 ist und $R_3$' und $R_3$" unabhängig H, $CH_3$ oder Cl sind; R H ist; und W O ist.

4. Verbindung nach Anspruch 3, worin n 0 ist.

5. Verbindung nach Anspruch 4, worin Y $C_1$-$C_2$-Alkyl, $OCH_3$ oder $OCF_2H$ ist.

6. Verbindung nach Anspruch 5 der Formel Ia.

7. Verbindung nach Anspruch 5 der Formel Ib.

8. Verbindung nach Anspruch 5 der Formel Ic.

9. Verbindung nach Anspruch 6, worin Q Q-2, Q-3, Q-5, Q-7, Q-10, Q-11, Q-15, Q-16, Q-20, Q-23, Q-28, Q-36 oder Q-39 ist.

10. Verbindung nach Anspruch 7, worin Q Q-2, Q-3, Q-5, Q-7, Q-10, Q-11, Q-15, Q-16, Q-20, Q-23, Q-28,

Q-36 oder Q-39 ist.

**11.** Verbindung nach Anspruch 8, worin Q Q-2, Q-3, Q-5, Q-7, Q-10, Q-11, Q-15, Q-16, Q-20, Q-23, Q-28, Q-36 oder Q-39 ist.

**12.** Verbindung nach Anspruch 9, worin W" S ist.

**13.** Verbindung nach Anspruch 10, worin W" S ist.

**14.** Verbindung nach Anspruch 11, worin W" S ist.

**15.** Verbindung nach Anspruch 1, Formeln Id und Ie,
worin

| | |
|---|---|
| R | H ist; |
| W | O ist; |
| Q | aus der aus |

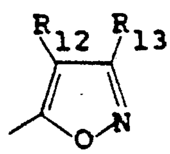 ,

Q-46

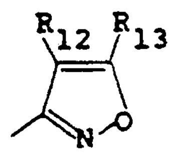 ,

Q-47

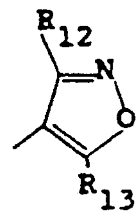 ,

Q-48

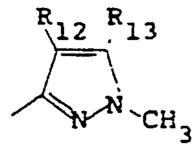
,

Q-49

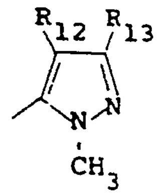
,

Q-50

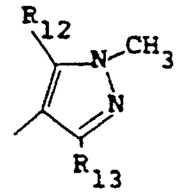
,

Q-51

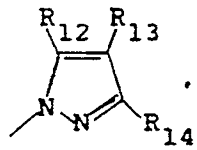
,

Q-52

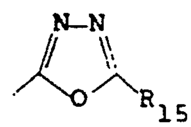
,

Q-53

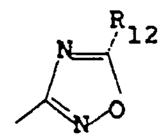
,

Q-54

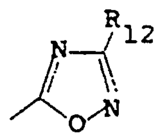
,

Q-55

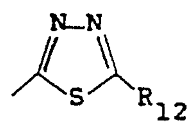
,

Q-56

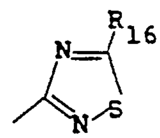
,

Q-57

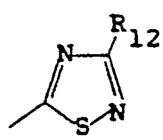
,

Q-58

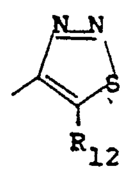
,

Q-59

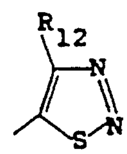
,

Q-60

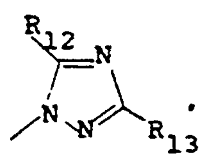
,

Q-61

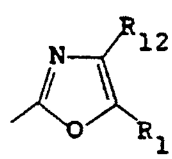
,

Q-62

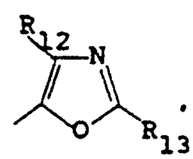
,

Q-63

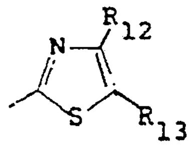

Q-64

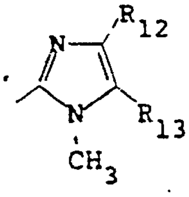

Q-65

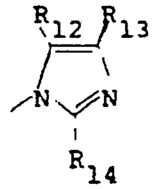

Q-66

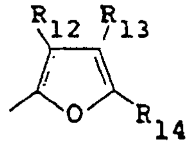

Q-67

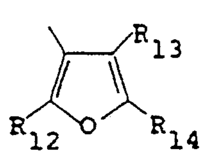

Q-68

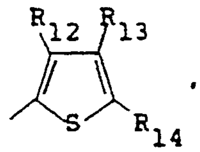

Q-69

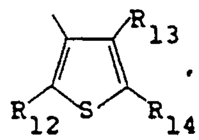

Q-70

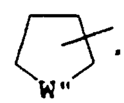

Q-71

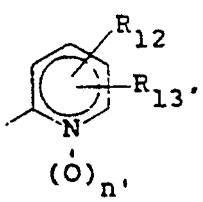

Q-72

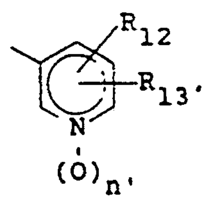

Q-73

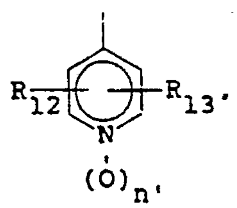

Q-74

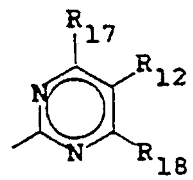

Q-75

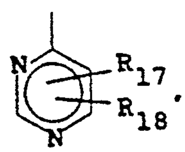

Q-76

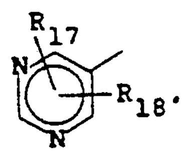

Q-77

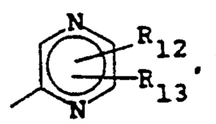

Q-78

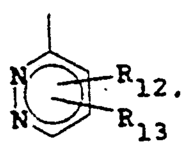

Q-79

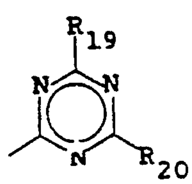

Q-80

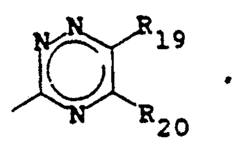

Q-81

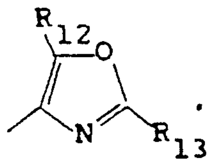 , 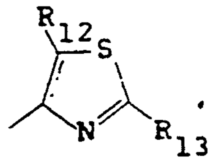 , 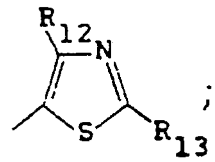 ;

Q-82 Q-83 Q-84

| | |
|---|---|
| | bestehenden Gruppe ausgewählt ist; |
| n' | wie vorstehend definiert ist; |
| $R_{12}$, $R_{13}$ und $R_{14}$ | unabhängig H oder $CH_3$ sind; |
| $R_{15}$ | H, $CH_3$, $C_2H_5$, $C_1$-$C_3$-Alkylthio, $SCH_2CH=CH_2$, $SCF_2H$, $OCH_3$ oder $OCH_2CH_3$ ist; |
| $R_{16}$ | H oder Cl ist; |
| $R_{17}$ und $R_{18}$ | unabhängig H, $CH_3$ oder $OCH_3$ sind; |
| $R_{19}$ und $R_{20}$ | unabhängig $CH_3$ oder $OCH_3$ sind; |
| W" | O, S oder $NR_{11}$ ist; und |
| $R_{11}$ | wie vorstehend definiert ist. |

**16.** Verbindung nach Anspruch 15, worin J J-1 ist; $E_1$ H ist; n' 0 ist; und Q aus der aus Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82, Q-83 und Phenyl bestehenden Gruppe ausgewählt ist.

**17.** Verbindung nach Anspruch 16, worin $X_4$ $CH_3$, $OCH_3$, $OCH_2CH_3$ oder Cl ist und $Y_4$ $CH_3$, $CH_2CH_3$, $OCH_3$, $CH(OCH_3)_2$ oder $CH_2OCH_3$ ist.

**18.** Verbindung nach Anspruch 17, worin n 0 ist.

**19.** Verbindung nach Anspruch 18, worin $Y_4$ $CH_3$, $CH_2CH_3$ oder $OCH_3$ ist.

**20.** Verbindung nach Anspruch 19 der Formel Id.

**21.** Verbindung nach Anspruch 19 der Formel Ie.

**22.** Verbindung nach Anspruch 20, worin Q Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82 oder Q-83 ist.

**23.** Verbindung nach Anspruch 21, worin Q Q-46, Q-47, Q-48, Q-49, Q-52, Q-53, Q-54, Q-55, Q-56, Q-59, Q-61, Q-62, Q-63, Q-66, Q-67, Q-69, Q-72, Q-75, Q-78, Q-82 oder Q-83 ist.

**24.** Verbindung nach Anspruch 1, welche N-[(4,6-Dimethylpyrimidin-2-yl)aminocarbonyl]-3-(isoxazol-3-yl)-2-thiophensulfonamid ist.

**25.** Verbindung nach Anspruch 1, welche 3-(Isoxazol-3-yl)-N-[(4-methoxy-5-methylpyrimidin-2-yl)-aminocarbonyl]-2-thiophensulfonamid ist.

**26.** Verbindung nach Anspruch 1, welche N-[(4,6-Dimethoxypyrimidin-2-yl)aminocarbonyl]-3-(isoxazol-3-yl)-2-thiophensulfonamid ist.

**27.** Verbindung nach Anspruch 1, welche 3-(5-Chlor-1H-1,2,4-triazol-1-yl)-N-[(4,6-dimethoxypyrimidin-2-yl)-aminocarbonyl]-2-thiophensulfonamid ist.

**28.** Verbindung nach Anspruch 1, welche N-[(4-Methoxy-6-methylpyrimidin-2-yl)aminocarbonyl]-3-(1H-pyrrol-1-yl)-2-thiophensulfonamid ist.

**29.** Verbindung nach Anspruch 1, welche N-[(4-Methoxy-6-methyl-1,3,5-triazin-2-yl)aminocarbonyl]-3-(1H-pyrrol-1-yl)-2-thiophensulfonamid ist.

**30.** Verbindung nach Anspruch 1, welche N-[(4,6-Dimethoxypyrimidin-2-yl)aminocarbonyl]-3-(1H-pyrrol-1-yl)-2-thiophensulfonamid ist.

**31.** Verbindung nach Anspruch 1, welche N-[(4,6-Dimethylpyrimidin-2-yl)aminocarbonyl]-3-(1H-pyrrol-1-yl)-2-thiophensulfonamid ist.

**32.** Verbindung nach Anspruch 1, welche N-[(4,6-Dimethoxypyrimidin-2-yl)aminocarbonyl]-2-(1H-1,2,4-triazol-1-yl)-3-pyridinsulfonamid ist.

**33.** Verbindung nach Anspruch 1, welche N-[(4,6-Dimethyl-1,3,5-triazin-2-yl)aminocarbonyl]-3-(1H-pyrrol-1-yl)-2-thiophensulfonamid ist.

**34.** Verbindung nach Anspruch 1, welche N-[(4,6-Dimethoxy-1,3,5-triazin-2-yl)aminocarbonyl]-3-(1H-pyrrol-1-yl)-2-thiophensulfonamid ist.

**35.** Verbindung nach Anspruch 1, welche N-[(4-Chlor-6-methoxypyrimidin-2-yl)aminocarbonyl]-3-(1H-pyrrol-1-yl)-2-thiophensulfonamid ist.

**36.** Verbindung nach Anspruch 1, welche N-[(4-Methoxy-6-methylpyrimidin-2-yl)aminocarbonyl]-3-(2-methyl-4-thiazolyl)-2-thiophensulfonamid ist.

**37.** Verbindung nach Anspruch 1, welche N-[(4,6-Dimethoxypyrimidin-2-yl)aminocarbonyl]-3-(2-methyl-4-thiazolyl)-2-thiophensulfonamid ist.

**38.** Verbindung nach Anspruch 2,
worin

| | |
|---|---|
| n' | 0 ist; |
| $R_3$' | H ist; |
| $R_3$" | H ist; |
| W" | O ist. |

**39.** Zur Bekämpfung des Wachstums unerwünschter Vegetation geeignete Zusammensetzung, welche eine wirksame Menge einer Verbindung nach einem der Ansprüche 1 bis 38 und wenigstens eines der folgenden umfaßt: oberflächenaktives Mittel, festes oder flüssiges Verdünnungsmittel.

**40.** Verfahren zur Bekämpfung des Wachstums unerwünschter Vegetation durch Anwenden einer wirksamen Menge einer Verbindung nach einem der Ansprüche 1 bis 38 auf den zu schützenden Ort.

**41.** Verfahren zur Steuerung des Wachstums von Pflanzen durch Anwenden einer wirksamen, jedoch im wesentlichen nicht phytotoxischen Menge eines aus Verbindungen nach einem der Ansprüche 1 bis 38 ausgewählten Pflanzenwachstumsregulans auf den Ort solcher Pflanzen.

**42.** Verfahren zur Herstellung einer Verbindung nach Anspruch 1 durch
(a) Umsetzen eines Sulfonamids der Formel

$BSO_2NH_2$

worin B aus

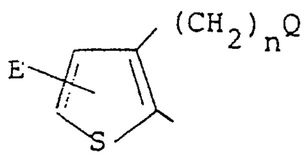 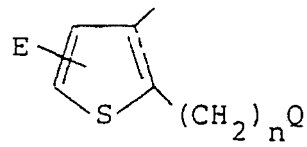 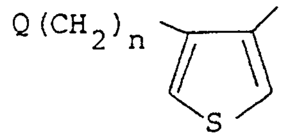

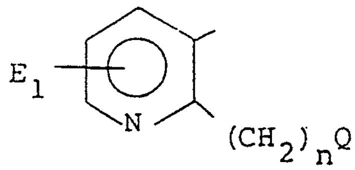 und 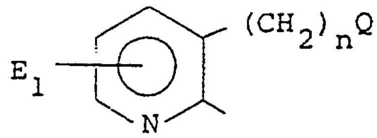

ausgewählt ist, worin E, $E_1$, Q und n wie in Anspruch 1 definiert sind, mit einem geeigneten Methyl- oder Phenylcarbamat der Formel

$$CH_3O\overset{O}{\overset{\|}{C}}-\underset{R}{\underset{|}{N}}-A'$$

oder

$$C_6H_5O\overset{O}{\overset{\|}{C}}-\underset{R}{\underset{|}{N}}-A'$$

worin R H oder $CH_3$ ist und A' A oder J ist, wie in Anspruch 1 definiert; oder

(b) Umsetzen eines Sulfonylcarbamats der Formel

$$BSO_2NH\overset{O}{\overset{\|}{C}}OC_6H_5$$

worin B wie vorstehend definiert ist, mit einem heterocyclischen Amin der Formel

$$H\overset{R}{\overset{|}{N}}-A'$$

worin R und A' wie vorstehend definiert sind; oder

(c) Umsetzen eines Sulfonylisocyanats oder Sulfonylisothiocyanats der Formel

BNCW

worin B wie vorstehend definiert ist und W O oder S ist, mit einem Amin der Formel

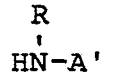 oder

(d) Austausch eines Chloratoms in einer Verbindung der Formel

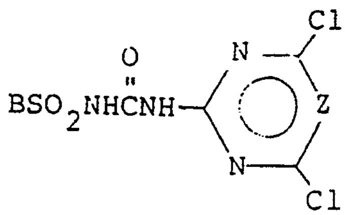

worin B wie vorstehend definiert ist und Z wie in Anspruch 1 definiert ist, gegen $OCH_3$ durch Reaktion mit einem Metallmethoxid und/oder in beliebiger Reihenfolge austauschen eines Chloratoms gegen OY', worin Y' $CH_3$, $C_2H_5$ oder $CH_2CF_3$ ist, durch Reaktion mit einem geeigneten Metallalkoxid.

**43.** Sulfonamide der Formel

$BSO_2NH_2$

und deren Phenylcarbamate; sowie Isocyanate und Isothiocyanate der Formel

BSCW

worin B wie in Anspruch 42 definiert ist und W O oder S ist.

**44.** Verbindungen der Formel

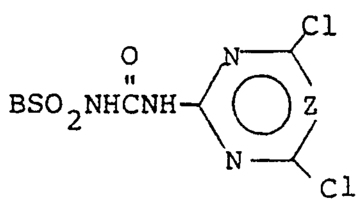

worin B wie in Anspruch 42 definiert ist und Z wie in Anspruch 1 definiert ist.